(19) Europäisches Patentamt
European Patent Office
Office européen des brevets

(11) EP 4 471 037 A1

(12) EUROPEAN PATENT APPLICATION
published in accordance with Art. 153(4) EPC

(43) Date of publication:
**04.12.2024 Bulletin 2024/49**

(21) Application number: **23746500.0**

(22) Date of filing: **30.01.2023**

(51) International Patent Classification (IPC):
***C07D 519/00*** (2006.01) ***C07D 498/22*** (2006.01)
***C07D 403/14*** (2006.01) ***C07D 413/14*** (2006.01)
***C07D 491/107*** (2006.01) ***A61P 35/00*** (2006.01)
***A61K 31/553*** (2006.01)

(52) Cooperative Patent Classification (CPC):
**A61K 31/517; A61K 31/519; A61K 31/5377; A61K 31/551; A61K 31/553; A61K 31/695; A61P 35/00; A61P 35/02; C07D 403/14; C07D 413/14; C07D 487/04; C07D 491/107; C07D 498/22; C07D 519/00; C07F 7/18**

(86) International application number:
**PCT/CN2023/073901**

(87) International publication number:
**WO 2023/143623 (03.08.2023 Gazette 2023/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.01.2022 CN 202210114712**
**09.06.2022 CN 202210654148**
**17.07.2022 CN 202210838120**
**15.09.2022 CN 202211125181**
**29.09.2022 CN 202211204559**

(71) Applicant: **Shanghai Pharmaceuticals Holding Co., Ltd.**
**Shanghai 201203 (CN)**

(72) Inventors:
- **ZHANG, Chaoxin**
  **Shanghai 201203 (CN)**
- **XIA, Guangxin**
  **Shanghai 201203 (CN)**
- **LI, Zhilong**
  **Shanghai 201203 (CN)**
- **PENG, Yayuan**
  **Shanghai 201203 (CN)**
- **KE, Ying**
  **Shanghai 201203 (CN)**
- **MAO, Haibin**
  **Shanghai 201203 (CN)**
- **FU, Yuhong**
  **Shanghai 201203 (CN)**

(74) Representative: **Calysta NV**
**Lambroekstraat 5a**
**1831 Diegem (BE)**

# (54) QUINOLINE COMPOUND AND USE THEREOF

(57) Disclosed are a quinoline compound and the use thereof. The quinoline compound is a compound as represented by formula (I), formula (II) or formula (III), a pharmaceutically acceptable salt thereof, a solvate thereof, a stereoisomer thereof, a tautomer thereof, a prodrug thereof, a metabolite thereof, or an isotope compound thereof.

EP 4 471 037 A1

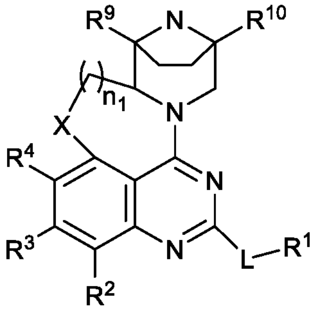

(I)

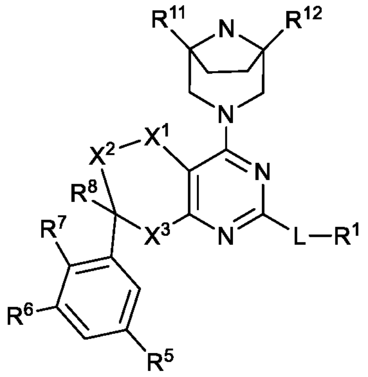

(II)

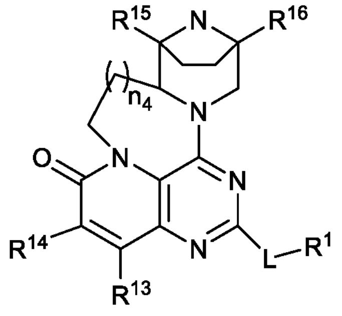

(III)

## Description

The present application claims the priorities of Chinese patent application 2022101147120 filed on January 30, 2022, Chinese patent application 2022106541481 filed on June 09, 2022, Chinese patent application 2022108381203 filed on July 17, 2022, Chinese patent application 2022111251811 filed on September 15, 2022, and Chinese patent application 2022112045597 filed on September 29, 2022. The contents of the above Chinese patent applications are incorporated herein by reference in their entireties.

TECHNICAL FIELD

The present disclosure relates to a quinazoline compound and a use thereof.

BACKGROUND

RAS represents a group of closely related monomeric globular proteins (molecular weight of 21 kDa) with 189 amino acids, and RAS is associated with the plasma membrane and binds to GDP or GTP. RAS acts as a molecular switch. When RAS contains bound GDP, it is in a resting or off position and "inactive". In response to exposure of cells to certain growth-promoting stimuli, RAS is induced to exchange its bound GDP for GTP. Upon binding GTP, RAS is "turned on" and able to interact with and activate other proteins (its "downstream targets"). The RAS protein itself has a very low inherent ability to hydrolyze GTP back to GDP, thereby turning itself into an off state. Turning off RAS requires an exogenous protein known as GTPase-activating protein (GAP), which interacts with RAS and greatly accelerates the conversion of GTP to GDP. Any mutation in RAS that affects its ability to interact with GAP or convert GTP back to GDP will result in prolonged activation of the protein, and therefore prolonged signals transmitted to cells telling them to continue to grow and divide. Since these signals lead to cell growth and division, over-activated RAS signaling can ultimately lead to cancer.

Structurally, the RAS protein contains a G domain responsible for the enzymatic activity of RAS-guanine nucleotide binding and hydrolysis (GTPase reaction). It also contains a C-terminal extension known as CAAX box, which can be post-translationally modified and is responsible for targeting the protein to the membrane. The G domain is approximately 21 to 25 kDa in size and contains a phosphate-binding loop (P-loop). The P-loop represents a nucleotide-binding pocket within the protein, and it is a rigid part of the domain with conserved amino acid residues that are essential for nucleotide binding and hydrolysis (glycine 12, threonine 26, and lysine 16). The G domain also contains the so-called switch I region (residues 30-40) and switch II region (residues 60-76), both of which are dynamic parts of the protein. These dynamic parts are often expressed as a "spring-loaded" mechanism due to their ability to switch between the resting and loaded states. The main interaction is the hydrogen bond formed between threonine-35 and glycine-60 with the γ-phosphate of GTP, which maintains the active conformations of the switch I and switch II regions, respectively. After GTP hydrolysis and phosphate release, these two relax into the inactive GDP conformation.

The most notable members of the RAS subfamily are HRAS, KRAS, and NRAS, which are primarily involved in many types of cancer. However, there are many other members, including DIRAS1; DIRAS2; DIRAS3; ERAS; GEM; MRAS; NKIRAS1; NKIRAS2; NRAS; RALA; RALB; RAP1A; RAP1B; RAP2A; RAP2B; RAP2C; RASD1; RASD2; RASL10A; RASL10B; RASL11A; RASL11B; RASL12; REM1; REM2; RERG; RERGL; RRAD; RRAS, and RRAS2.

Mutations in any of the three major isoforms of the RAS gene (HRAS, NRAS, or KRAS) are one of the most common events in human tumorigenesis. It is found that approximately 30% of all human tumors carry some mutations in the RAS gene. Notably, KRAS mutations are detected in 25% to 30% of tumors. By comparison, the rate of oncogenic mutations in NRAS and HRAS family members is much lower (8% and 3%, respectively). The most common KRAS mutations are found in the P-loop at residues G12 and G13 and at residue Q61. Among tumor-associated KRAS G12 mutations, KRAS G12D has the highest mutation probability, accounting for approximately 40%.

Based on the importance of aberrant KRAS activation in cancer progression and the prevalence of KRAS gene mutations in human cancers, KRAS has been a target of interest for drug developers. Although progress has been made in this field, there remains a need in the art for improved KRAS G12D mutant protein inhibitors.

CONTENT OF THE PRESENT INVENTION

The technical problem to be solved by the present disclosure is to overcome the shortcomings of limited types of KRAS G12D mutant protein inhibitors in the prior art. To this end, a quinazoline compound and a use thereof are provided. The quinazoline compound provided by the present disclosure has good inhibitory effects on KRAS G12D mutant protein.

The present disclosure solves the above technical problem through the following technical solutions.

The present disclosure provides a compound of formula I, formula II, formula III, or formula IV, a pharmaceutically acceptable salt thereof, a solvate thereof, a stereoisomer thereof, a tautomer thereof, a prodrug thereof, a metabolite thereof, or an isotopic compound thereof:

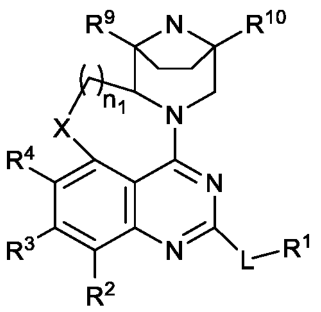

I

,

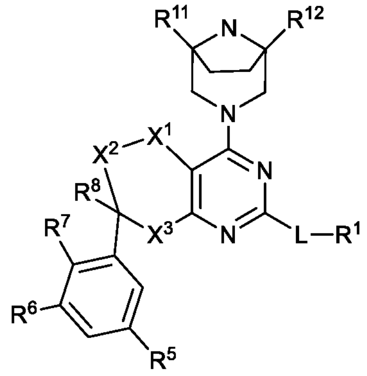

II

,

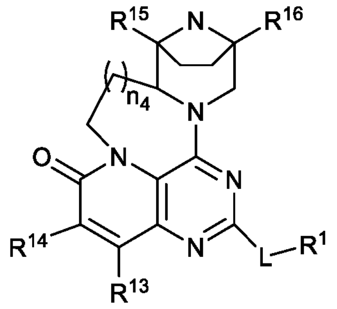

III

,

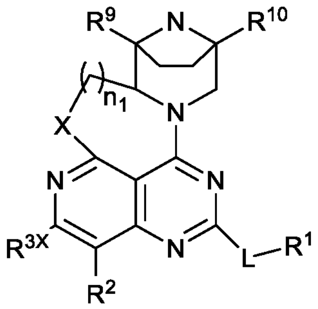

IV

,

wherein formula I, II, or III satisfies the following situation 1 or situation 2:

**situation 1:**

X is N, O, or S;
n1 and n4 are each independently 1, 2, 3, or 4;
each L is independently -O-$(CR^{L-1}R^{L-2})_{n2}$-*, -$(CR^{L-3}R^{L-4})_{n3}$-*, or

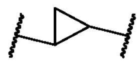
;

* represents one end connected to $R^1$;
n2 and n3 are each independently 0, 1, 2, 3, or 4;
$R^{L-1}$, $R^{L-2}$, $R^{L-3}$, and $R^{L-4}$ are each independently H, $C_1$-$C_6$ alkyl, $C_1$-$C_6$ alkoxy, $C_1$-$C_6$ alkyl substituted by one or more $R^{L-1-1}$, or halogen;
each $R^{L-1-1}$ is independently halogen or $C_1$-$C_6$ alkoxy;
each $R^1$ is 4- to 10-membered heterocycloalkyl substituted by one or more $R^{1-1}$; heteroatoms in the 4- to 10-membered heterocycloalkyl of the 4- to 10-membered heterocycloalkyl substituted by one or more $R^{1-1}$ are independently 1, 2, or 3 kinds of N, O, or S, and the number of heteroatoms is 1, 2, or 3;
each $R^{1-1}$ is independently halogen;
$R^2$ and $R^{13}$ are each independently H or halogen;
$R^3$ and $R^{14}$ are each independently $C_6$-$C_{10}$ aryl, $C_6$-$C_{10}$ aryl substituted by one or more $R^{3-1}$, 5- to 10-membered heteroaryl, or 5- to 10-membered heteroaryl substituted by one or more $R^{3-2}$; heteroatoms in the 5- to 10-membered heteroaryl and the 5- to 10-membered heteroaryl substituted by one or more $R^{3-2}$ are independently 1, 2, or 3 kinds of N, O, or S, and the number of heteroatoms is 1, 2, or 3;
$R^{3-1}$ and $R^{3-2}$ are each independently OH, halogen, $C_1$-$C_6$ alkyl, $C_1$-$C_6$ alkyl substituted by one or more $R^{3-1-1}$, $C_2$-$C_6$ alkynyl, 3- to 8-membered cycloalkyl, -S-$C(R^{3-1-2})_3$, -$S(R^{3-1-3})_5$, amino, $C_1$-$C_6$ alkyl, or 5- to 10-membered heteroaryl;
alternatively, any two adjacent $R^{3-1}$, together with the carbon atom to which they are attached, form a 5- to 10-membered heteroaryl group or a 5- to 10-membered heteroaryl group substituted by one or more $R^{3-1-4}$; heteroatoms in the 5- to 10-membered heteroaryl group and the 5- to 10-membered heteroaryl group substituted by one or more $R^{3-1-4}$ are independently 1, 2, or 3 kinds of N, O, or S, and the number of heteroatoms is 1, 2, or 3;
each $R^{3-1-1}$ is independently oxo (=O), OH, $C_1$-$C_6$ alkoxy, or halogen;
$R^{3-1-2}$ and $R^{3-1-3}$ are each independently halogen;
each $R^{3-1-4}$ is independently $C_1$-$C_6$ alkyl;
$R^4$ is H, OH, $C_1$-$C_6$ alkyl, $C_1$-$C_6$ alkoxy, $C_1$-$C_6$ alkyl substituted by one or more $R^{4-1}$, cyano, or halogen;
each $R^{4-1}$ is independently halogen;
$X^1$ is $C(R^{1a}R^{1b})$ or O;
$X^2$ is $C(R^{2a}R^{2b})$ or O;
$X^3$ is $C(R^{3a}R^{3b})$ or O;
$R^{1a}$, $R^{1b}$, $R^{2a}$, $R^{2b}$, $R^{3a}$, and $R^{3b}$ are each independently H, $C_1$-$C_6$ alkyl, or halogen;
$R^5$ is H or OH;
$R^6$ is H, $C_1$-$C_6$ alkyl, $C_1$-$C_6$ alkoxy, 3- to 8-membered cycloalkyl, halogen, or $C_1$-$C_6$ alkyl substituted by one or more $R^{6-1}$;
each $R^{6-1}$ is independently halogen;
$R^7$ and $R^8$ are connected to form ring A, and ring A is a 5- to 6-membered saturated or unsaturated monocyclic, spirocyclic, or fused carbocyclic ring, a 5- to 6-membered saturated or unsaturated monocyclic, spirocyclic, or fused carbocyclic ring substituted by one or more $R^{7-1}$, a 5- to 6-membered saturated or unsaturated monocyclic, spirocyclic, or fused heterocyclic ring with 1, 2, or 3 heteroatoms selected from 1, 2, or 3 kinds of N, O, and S, or a 5- to 6-membered saturated or unsaturated monocyclic, spirocyclic, or fused heterocyclic ring with 1, 2, or 3 heteroatoms selected from 1, 2, or 3 kinds of N, O, and S substituted by one or more $R^{7-2}$;
$R^{7-1}$ and $R^{7-2}$ are each independently $C_1$-$C_6$ alkyl, oxo, or halogen;
$R^9$, $R^{10}$, $R^{11}$, $R^{12}$, $R^{15}$, and $R^{16}$ are each independently H, $C_1$-$C_6$ alkyl, or halogen;

**situation 2:**

X is N, O, or S;
n1 and n4 are independently 1, 2, 3, or 4;
each L is independently $-O-(CR^{L-1}R^{L-2})_{n2}-*$, $-(CR^{L-3}R^{L-4})_{n3}-*$, or

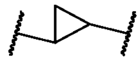;

* represents one end connected to $R^1$;
n2 and n3 are each independently 0, 1, 2, 3, or 4;
$R^{L-1}$, $R^{L-2}$, $R^{L-3}$, and $R^{L-4}$ are each independently H, $C_1$-$C_6$ alkyl, $C_1$-$C_6$ alkoxy, $C_1$-$C_6$ alkyl substituted by one or more $R^{L-1-1}$, or halogen;
each $R^{L-1-1}$ is independently halogen or $C_1$-$C_6$ alkoxy;
each $R^1$ is 4- to 10-membered heterocycloalkyl substituted by one or more $R^{1-1}$; heteroatoms in the 4- to 10-membered heterocycloalkyl of the 4- to 10-membered heterocycloalkyl substituted by one or more $R^{1-1}$ are independently 1, 2, or 3 kinds of N, O, or S, and the number of heteroatoms is 1, 2, or 3;
each $R^{1-1}$ is independently halogen, hydroxyl, -O-$C_1$-$C_6$ alkyl, $C_1$-$C_6$ alkyl, or $C_1$-$C_6$ alkyl substituted by one or more $R^{1-1-1}$.
each $R^{1-1-1}$ is independently hydroxyl, -O-$C_1$-$C_6$ alkyl, 4- to 10-membered heterocycloalkyl, or 4- to 10-membered heterocycloalkyl substituted by one or more $R^{1-1-1-1}$; heteroatoms in the 4- to 10-membered heterocycloalkyl and the 4- to 10-membered heterocycloalkyl substituted by one or more $R^{1-1-1-1}$ are independently 1, 2, or 3 kinds of N, O, or S, and the number of heteroatoms is 1, 2, or 3;
each $R^{1-1-1-1}$ is independently $C_1$-$C_6$ alkyl;
$R^2$ and $R^{13}$ are each independently H or halogen;
$R^3$ is

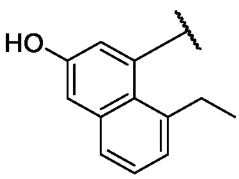 , 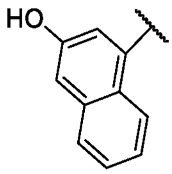 , 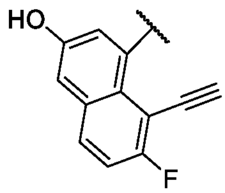 , 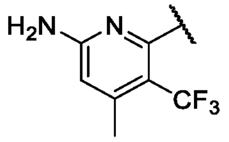 ,

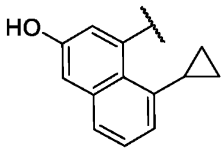 , 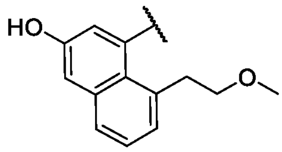 , 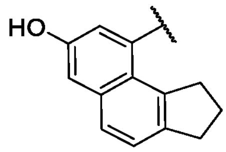 , 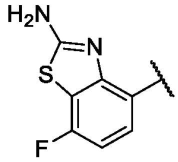 ,

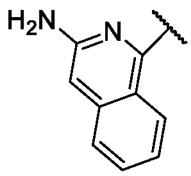 , or 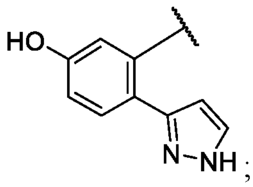 ;

when $R^3$ is

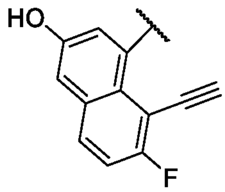 or 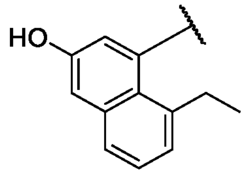 ,

R4 is F;

each $R^{14}$ is independently $C_6$-$C_{10}$ aryl, $C_6$-$C_{10}$ aryl substituted by one or more $R^{3-1}$, 5- to 10-membered heteroaryl, or 5- to 10-membered heteroaryl substituted by one or more $R^{3-2}$; heteroatoms in the 5- to 10-membered heteroaryl and the 5- to 10-membered heteroaryl substituted by one or more $R^{3-2}$ are independently 1, 2, or 3 kinds of N, O, or S, and the number of heteroatoms is 1, 2, or 3;

$R^{3-1}$ and $R^{3-2}$ are each independently OH, halogen, $C_1$-$C_6$ alkyl, $C_1$-$C_6$ alkyl substituted by one or more $R^{3-1-1}$, $C_2$-$C_6$ alkynyl, 3- to 8-membered cycloalkyl, -S-C($R^{3-1-2}$)$_3$, -S($R^{3-1-3}$)$_5$, amino, $C_1$-$C_6$ alkyl, 5- to 10-membered heteroaryl, 5- to 10-membered heteroaryl substituted by one or more $R^{3-1-4}$, or -O-$C_1$-$C_6$ alkyl; heteroatoms in the 5- to 10-membered heteroaryl and the 5- to 10-membered heteroaryl substituted by one or more $R^{3-1-4}$ are independently 1, 2, or 3 kinds of N, O, or S, and the number of heteroatoms is 1, 2, or 3;

alternatively, any two adjacent $R^{3-1}$, together with the carbon atom to which they are attached, form a 5- to 6-membered carbocyclic ring, a 5- to 6-membered carbocyclic ring substituted by one or more $R^{3-1-4}$, a 5- to 6-membered heterocyclic ring, or a 5- to 6-membered heterocyclic ring substituted by one or more $R^{3-1-4}$; heteroatoms in the 5- to 6-membered heterocyclic ring and the 5- to 6-membered heterocyclic ring substituted by one or more $R^{3-1-4}$ are independently 1, 2, or 3 kinds of N, O, or S, and the number of heteroatoms is 1, 2, or 3;

each $R^{3-1-1}$ is independently oxo (=O), OH, $C_1$-$C_6$ alkoxy, or halogen;

$R^{3-1-2}$ and $R^{3-1-3}$ are each independently halogen;

each $R^{3-1-4}$ is independently $C_1$-$C_6$ alkyl;

$R^4$ is H, OH, $C_1$-$C_6$ alkyl, $C_1$-$C_6$ alkoxy, $C_1$-$C_6$ alkyl substituted by one or more $R^{4-1}$, cyano, or F;

each $R^{4-1}$ is independently halogen;

$X^1$ is C($R^{1a}R^{1b}$) or O;

$X^2$ is C($R^{2a}R^{2b}$) or O;

$X^3$ is C($R^{3a}R^{3b}$) or O;

$R^{1a}$, $R^{1b}$, $R^{2a}$, $R^{2b}$, $R^{3a}$, and $R^{3b}$ are each independently H, $C_1$-$C_6$ alkyl, or halogen;

$R^5$ is H or OH;

$R^6$ is H, $C_1$-$C_6$ alkyl, $C_1$-$C_6$ alkoxy, 3- to 8-membered cycloalkyl, halogen, or $C_1$-$C_6$ alkyl substituted by one or more $R^{6-1}$;

each $R^{6-1}$ is independently halogen;

$R^7$ and $R^8$ are connected to form ring A, and ring A is a 5- to 6-membered saturated or unsaturated monocyclic, spirocyclic, or fused carbocyclic ring, a 5- to 6-membered saturated or unsaturated monocyclic, spirocyclic, or fused carbocyclic ring substituted by one or more $R^{7-1}$, a 5- to 6-membered saturated or unsaturated monocyclic, spirocyclic, or fused heterocyclic ring with 1, 2, or 3 heteroatoms selected from 1, 2, or 3 kinds of N, O, and S, or a 5- to 6-membered saturated or unsaturated monocyclic, spirocyclic, or fused heterocyclic ring with 1, 2, or 3 heteroatoms selected from 1, 2, or 3 kinds of N, O, and S substituted by one or more $R^{7-2}$;

$R^{7-1}$ and $R^{7-2}$ are each independently $C_1$-$C_6$ alkyl, oxo, or halogen;

$R^9$, $R^{10}$, $R^{11}$, $R^{12}$, $R^{15}$, and $R^{16}$ are each independently H, $C_1$-$C_6$ alkyl, or halogen;

in formula IV,

X is N, O, or S;

each n1 is independently 1, 2, 3, or 4;

each L is independently -O-$(CR^{L-1}R^{L-2})_{n2}$-*, -$(CR^{L-3}R^{L-4})_{n3}$-*, or

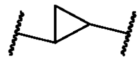;

* represents one end connected to $R^1$;

n2 and n3 are each independently 0, 1, 2, 3, or 4;

$R^{L-1}$, $R^{L-2}$, $R^{L-3}$, and $R^{L-4}$ are each independently H, $C_1$-$C_6$ alkyl, $C_1$-$C_6$ alkoxy, $C_1$-$C_6$ alkyl substituted by one or more $R^{L-1-1}$, or halogen;

each $R^{L-1-1}$ is independently halogen or $C_1$-$C_6$ alkoxy;

each $R^1$ is 4- to 10-membered heterocycloalkyl substituted by one or more $R^{1-1}$; heteroatoms in the 4- to 10-membered heterocycloalkyl of the 4- to 10-membered heterocycloalkyl substituted by one or more $R^{1-1}$ are independently 1, 2, or 3 kinds of N, O, or S, and the number of heteroatoms is 1, 2, or 3;

each $R^{1-1}$ is independently halogen, hydroxyl, -O-$C_1$-$C_6$ alkyl, $C_1$-$C_6$ alkyl, or $C_1$-$C_6$ alkyl substituted by one or more $R^{1-1-1}$;

each $R^{1-1-1}$ is independently hydroxyl, -O-$C_1$-$C_6$ alkyl, 4- to 10-membered heterocycloalkyl, or 4- to 10-membered heterocycloalkyl substituted by one or more $R^{1-1-1-1}$; heteroatoms in the 4- to 10-membered heterocycloalkyl and the 4- to 10-membered heterocycloalkyl substituted by one or more $R^{1-1-1-1}$ are independently 1, 2, or 3 kinds of N, O, or S, and the number of heteroatoms is 1, 2, or 3;

each $R^{1-1-1-1}$ is independently $C_1$-$C_6$ alkyl;

$R^2$ and $R^{13}$ are each independently H or halogen;

$R^{3X}$ is 5- to 10-membered heteroaryl or 5- to 10-membered heteroaryl substituted by one or more $R^{3X-1}$; heteroatoms in the 5- to 10-membered heteroaryl and the 5- to 10-membered heteroaryl substituted by one or more $R^{3X-1}$ are independently 1, 2, or 3 kinds of N, O, or S, and the number of heteroatoms is 1, 2, or 3;

each $R^{3X-1}$ is independently $C_1$-$C_6$ alkyl, amino, or $C_1$-$C_6$ alkyl substituted by one or more halogens;

$R^9$ and $R^{10}$ are each independently H, $C_1$-$C_6$ alkyl, or halogen.

In some embodiments, in situation 1, each $R^1$ is also 11-membered heterocycloalkyl substituted by one or more $R^{1-1}$; heteroatoms in the 11-membered heterocycloalkyl of the 11-membered heterocycloalkyl substituted by one or more $R^{1-1}$ are independently 1, 2, or 3 kinds of N, O, or S, and the number of heteroatoms is 1, 2, or 3.

In some embodiments, in situation 1, each $R^{1-1}$ is also independently hydroxyl, -O-$C_1$-$C_6$ alkyl, $C_1$-$C_6$ alkyl, or $C_1$-$C_6$ alkyl substituted by one or more $R^{1-1-1}$;

each $R^{1-1-1}$ is independently hydroxyl, -O-$C_1$-$C_6$ alkyl, 4- to 10-membered heterocycloalkyl, or 4- to 10-membered heterocycloalkyl substituted by one or more $R^{1-1-1-1}$; heteroatoms in the 4- to 10-membered heterocycloalkyl and the 4- to 10-membered heterocycloalkyl substituted by one or more $R^{1-1-1-1}$ are independently 1, 2, or 3 kinds of N, O, or S, and the number of heteroatoms is 1, 2, or 3;

each $R^{1-1-1-1}$ is independently $C_1$-$C_6$ alkyl.

In some embodiments, in situation 1, $R^{3-1}$ and $R^{3-2}$ are also each independently 5- to 10-membered heteroaryl substituted by one or more $R^{3-1-4}$ or -O-$C_1$-$C_6$ alkyl; heteroatoms in the 5- to 10-membered heteroaryl substituted by one or more $R^{3-1-4}$ are independently 1, 2, or 3 kinds of N, O, or S, and the number of heteroatoms is 1, 2, or 3;

alternatively, any two adjacent $R^{3-1}$, together with the carbon atom to which they are attached, form a 5- to 6-membered carbocyclic ring, a 5- to 6-membered carbocyclic ring substituted by one or more $R^{3-1-4}$ a 5- to 6-membered heterocyclic ring, or a 5- to 6-membered heterocyclic ring substituted by one or more $R^{3-1-4}$; heteroatoms in the 5- to 6-membered heterocyclic ring and the 5- to 6-membered heterocyclic ring substituted by one or more $R^{3-1-4}$ are independently 1, 2, or 3 kinds of N, O, or S, and the number of heteroatoms is 1, 2, or 3.

In some embodiments, X is O.

In some embodiments, n1 is 1 or 2.

In some embodiments, n1 is 1.

In some embodiments, n2 and n3 are each independently 1 or 2.

In some embodiments, $R^{L-1}$, $R^{L-2}$, $R^{L-3}$, and $R^{L-4}$ are each independently H, $C_1$-$C_6$ alkyl substituted by one or more $R^{L-1-1}$, or halogen, preferably H.

In some embodiments, each $R^{L-1-1}$ is independently $C_1$-$C_6$ alkoxy.

In some embodiments, $R^2$ is halogen.

In some embodiments, $R^3$ is $C_6$-$C_{10}$ aryl substituted by one or more $R^{3-1}$ or 5- to 10-membered heteroaryl substituted by one or more $R^{3-2}$.

In some embodiments, $R^3$ is $C_6$-$C_{10}$ aryl substituted by one or more $R^{3-1}$.

In some embodiments, each $R^{3-1}$ is independently 5- to 10-membered heteroaryl, -O-$C_1$-$C_6$ alkyl, $C_1$-$C_6$ alkyl substituted by one or more $R^{3-1-1}$, 3- to 8-membered cycloalkyl, OH, halogen, $C_1$-$C_6$ alkyl, or $C_2$-$C_6$ alkynyl.

In some embodiments, each $R^{3-1}$ is independently OH, halogen, $C_1$-$C_6$ alkyl, or $C_2$-$C_6$ alkynyl.

In some embodiments, $R^4$ is H, halogen, cyano, OH, $C_1$-$C_6$ alkoxy, or $C_1$-$C_6$ alkyl substituted by one or more $R^{4-1}$; preferably H or halogen.

In some embodiments, $X^1$ is $C(R^{1a}R^{1b})$.

In some embodiments, $X^2$ is $C(R^{2a}R^{2b})$.

In some embodiments, $X^3$ is $C(R^{3a}R^{3b})$.

In some embodiments, $R^{1a}$, $R^{1b}$, $R^{2a}$, $R^{2b}$, $R^{3a}$, and $R^{3b}$ are each independently H or halogen; preferably H.

In some embodiments, $R^5$ is OH.

In some embodiments, $R^6$ is H, halogen, $C_1$-$C_6$ alkyl, $C_1$-$C_6$ alkyl substituted by one or more $R^{6-1}$, or 3- to 8-membered cycloalkyl; preferably halogen.

In some embodiments, ring A is a 5- to 6-membered saturated or unsaturated monocyclic, spirocyclic, or fused carbocyclic ring, a 5- to 6-membered saturated or unsaturated monocyclic, spirocyclic, or fused carbocyclic ring substituted by one or more $R^{7-1}$, or a 5- to 6-membered saturated or unsaturated monocyclic, spirocyclic, or fused heterocyclic ring with 1, 2, or 3 heteroatoms selected from 1, 2, or 3 kinds of N, O, and S; preferably a 5- to 6-membered saturated or unsaturated monocyclic carbocyclic ring.

In some embodiments, $R^9$, $R^{10}$, $R^{11}$, and $R^{12}$ are each independently H or $C_1$-$C_6$ alkyl; preferably H.

In some embodiments, each $R^{3-2}$ is independently $C_1$-$C_6$ alkyl, amino, halogen, or $C_1$-$C_6$ alkyl substituted by one or more $R^{3-1-1}$.

In some embodiments, each $R^{3-1-1}$ is independently $C_1$-$C_6$ alkoxy or halogen.

In some embodiments, X is O;

n1 is 1;
$R^1$ is 4- to 10-membered heterocycloalkyl substituted by one or more $R^{1-1}$;
each $R^{1-1}$ is independently halogen;
$R^2$ is halogen;
$R^4$ is H or halogen;
$R^3$ is $C_6$-$C_{10}$ aryl substituted by one or more $R^{3-1}$;
each $R^{3-1}$ is independently OH, halogen, $C_1$-$C_6$ alkyl, or $C_2$-$C_6$ alkynyl;
L is -O-$(CR^{L-1}R^{L-2})_{n2}$-*;
$R^{L-1}$ or $R^{L-2}$ are each independently H;
n2 is 1;
$R^9$ and $R^{10}$ are each independently H.

In some embodiments, $X^1$ is $C(R^{1a}R^{1b})$ or O;

$X^2$ is $C(R^{2a}R^{2b})$ or O;
$X^3$ is $C(R^{3a}R^{3b})$ or O;
$R^{1a}$, $R^{1b}$, $R^{2a}$, $R^{2b}$, $R^{3a}$, and $R^{3b}$ are each independently H or halogen;
L is -O-$(CR^{L-1}R^{L-2})_{n2}$-*, -$(CR^{L-3}R^{L-4})_{n3}$-*, or

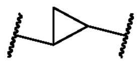
;

* represents one end connected to $R^1$;
n2 and n3 are each independently 1 or 2;
$R^{L-1}$, $R^{L-2}$, $R^{L-3}$, and $R^{L-4}$ are each independently H, $C_1$-$C_6$ alkyl substituted by one or more $R^{L-1-1}$, or halogen;
each $R^{L-1-1}$ is independently $C_1$-$C_6$ alkoxy;
$R^1$ is 4- to 10-membered heterocycloalkyl substituted by one or more $R^{1-1}$;
each $R^{1-1}$ is independently halogen;
$R^5$ is H or OH;
$R^6$ is H, $C_1$-$C_6$ alkyl, $C_1$-$C_6$ alkoxy, 3- to 8-membered cycloalkyl, halogen, or $C_1$-$C_6$ alkyl substituted by one or more $R^{6-1}$;
each $R^{6-1}$ is independently halogen;
$R^7$ and $R^8$ are connected to form ring A, and ring A is a 5- to 6-membered saturated or unsaturated monocyclic, spirocyclic, or fused carbocyclic ring, a 5- to 6-membered saturated or unsaturated monocyclic, spirocyclic, or fused carbocyclic ring substituted by one or more $R^{7-1}$, or a 5- to 6-membered saturated or unsaturated monocyclic, spirocyclic, or fused heterocyclic ring with 1, 2, or 3 heteroatoms selected from 1, 2, or 3 kinds of N, O, and S;
each $R^{7-1}$ is independently $C_1$-$C_6$ alkyl, oxo (=O), or halogen;
$R^{11}$ and $R^{12}$ are each independently H, $C_1$-$C_6$ alkyl, or halogen.

In some embodiments, $X^1$ is $C(R^{1a}R^{1b})$;

$X^2$ is $C(R^{2a}R^{2b})$;
$X^3$ is $C(R^{3a}R^{3b})$;
$R^{1a}$, $R^{1b}$, $R^{2a}$, $R^{2b}$, $R^{3a}$, and $R^{3b}$ are each independently H or halogen;
$R^1$ is 4- to 10-membered heterocycloalkyl substituted by one or more $R^{1-1}$;
each $R^{1-1}$ is independently halogen;
L is $-O-(CR^{L-1}R^{L-2})_{n2}-*$;
$R^{L-1}$ or $R^{L-2}$ are each independently H;
n2 is 1;
$R^5$ is OH;
$R^6$ is halogen;
ring A is a 5-membered saturated monocyclic carbocyclic ring;
$R^{11}$ and $R^{12}$ are each independently H.

In some embodiments, in $R^1$, the "4- to 10-membered heterocycloalkyl" in the "4- to 10-membered heterocycloalkyl substituted by one or more $R^{1-1}$" is 8- to 10-membered heterocycloalkyl containing an N atom, and may also be bicyclo[3.3.0]heterooctyl containing an N atom, for example,

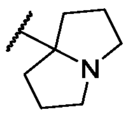

In some embodiments, in $R^{1-1-1}$, the 4- to 10-membered heterocycloalkyl in the 4- to 10-membered heterocycloalkyl and the "4- to 10-membered heterocycloalkyl substituted by one or more $R^{1-1-1-1}$" is independently 5- to 6-membered monocyclic heterocycloalkyl, heteroatoms are N and/or O, and the number is 1 or 2; the 4- to 10-membered heterocycloalkyl may also be piperidinyl, piperazinyl, or morpholinyl, and further may be

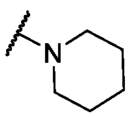 , 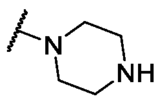 ,

or

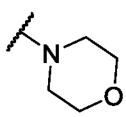

In some embodiments, in $R^4$, $R^6$, $R^{3-1}$, $R^{L-1}$, $R^{L-2}$, $R^{L-3}$, $R^{L-4}$, $R^{7-1}$, $R^{7-2}$, $R^9$, $R^{10}$, $R^{11}$, and $R^{12}$, each "$C_1$-$C_6$ alkyl"

in the "$C_1$-$C_6$ alkyl", "$C_1$-$C_6$ alkyl substituted by one or more $R^{4-1}$", "$C_1$-$C_6$ alkyl substituted by one or more $R^{3-1-1}$", and "$C_1$-$C_6$ alkyl substituted by one or more $R^{L-1-1}$" is independently methyl, ethyl, *n*-propyl, isopropyl, *n*-butyl, isobutyl, or *tert*-butyl; preferably methyl or ethyl.

In some embodiments, in $R^{1-1}$, the $C_1$-$C_6$ alkyl in the -O-$C_1$-$C_6$ alkyl, the $C_1$-$C_6$ alkyl, and the $C_1$-$C_6$ alkyl in the $C_1$-$C_6$ alkyl substituted by one or more $R^{1-1-1}$ are independently methyl, ethyl, *n*-propyl, isopropyl, *n*-butyl, isobutyl, or *tert*-butyl; preferably methyl or ethyl.

In some embodiments, in $R^{3-1}$ and $R^{3-2}$, each $C_1$-$C_6$ alkyl in the -O-$C_1$-$C_6$ alkyl is independently methyl, ethyl, *n*-propyl, isopropyl, *n*-butyl, isobutyl, or *tert*-butyl; preferably methyl or ethyl.

In some embodiments, in $R^{1-1-1}$, $R^{1-1-1-1}$, $R^{3-1-4}$, $R^{1a}$, $R^{1b}$, $R^{2a}$, $R^{2b}$, $R^{3a}$, $R^{3b}$, $R^{15}$, $R^{16}$, $R^{3X-1}$, and $R^{3-2}$, each "$C_1$-$C_6$ alkyl" in the "$C_1$-$C_6$ alkyl", "$C_1$-$C_6$ alkyl substituted by one or more $R^{3-1-1}$", "-O-$C_1$-$C_6$ alkyl", "$C_1$-$C_6$ alkyl substituted by one or more $R^{1-1-1}$", and "$C_1$-$C_6$ alkyl substituted by one or more halogens" is independently methyl, ethyl, *n*-propyl, isopropyl, *n*-butyl, isobutyl, or *tert*-butyl; preferably methyl or ethyl.

In some embodiments, in $R^{1-1-1}$, the two $R^{1-1-1}$ attached to the same carbon atom, together with the carbon atom to which they are attached, form a 3- to 8-membered cycloalkyl group, which is cyclopropyl, cyclobutyl, cyclopentyl, or cyclohexyl.

In some embodiments, in $R^{1a}$, $R^{1b}$, $R^{2a}$, $R^{2b}$, $R^{3a}$, $R^{3b}$, $R^{1-1}$, $R^2$, $R^4$, $R^6$, $R^{3-1}$, $R^{3-1-1}$, $R^{3-1-2}$, $R^{3-1-3}$, $R^{4-1}$, $R^{6-1}$, $R^{L-1}$, $R^{L-2}$, $R^{L-3}$, $R^{L-4}$, $R^{L-1-1}$, $R^{7-1}$, $R^{7-2}$ , $R^9$, $R^{10}$, $R^{11}$, and $R^{12}$, each halogen is independently fluorine, chlorine, bromine, or iodine, preferably fluorine or chlorine.

In some embodiments, in $R^{13}$, $R^{3-2}$, $R^{15}$, $R^{16}$, and $R^{3X-1}$, each halogen is independently fluorine, chlorine, bromine, or iodine, preferably fluorine or chlorine.

In some embodiments, in $R^4$, $R^6$, $R^{3-1-1}$, $R^{L-1}$, $R^{L-2}$, $R^{L-3}$, $R^{L-4}$, and $R^{L-1-1}$, each "$C_1$-$C_6$ alkoxy" is independently methoxy, ethoxy, *n*-propoxy, isopropoxy, *n*-butoxy, isobutoxy, or *tert*-butoxy; preferably methoxy or ethoxy.

In some embodiments, in $R^6$ and $R^{3-1}$, each 3- to 8-membered cycloalkyl is independently cyclopropyl, cyclobutyl, cyclopentyl, or cyclohexyl, preferably cyclopropyl or cyclobutyl.

In some embodiments, in $R^{3-2}$, each 3- to 8-membered cycloalkyl is independently cyclopropyl, cyclobutyl, cyclopentyl, or cyclohexyl, preferably cyclopropyl or cyclobutyl.

In some embodiments, in $R^{3-1}$, the "$C_2$-$C_6$ alkynyl" is $C_2$-$C_4$ alkynyl, preferably ethynyl.

In some embodiments, in $R^{3-2}$, the "$C_2$-$C_6$ alkynyl" is $C_2$-$C_4$ alkynyl, preferably ethynyl.

In some embodiments, in $R^{14}$, each "$C_6$-$C_{10}$ aryl" in the "$C_6$-$C_{10}$ aryl" and "$C_6$-$C_{10}$ aryl substituted by one or more $R^{3-1}$" is independently phenyl or naphthyl, preferably naphthyl.

In some embodiments, in $R^3$, each "$C_6$-$C_{10}$ aryl" in the "$C_6$-$C_{10}$ aryl" and "$C_6$-$C_{10}$ aryl substituted by one or more $R^{3-1}$" is independently phenyl or naphthyl, preferably naphthyl.

In some embodiments, in $R^3$, the "5- to 10-membered heteroaryl" is 9- to 10-membered heteroaryl.

In some embodiments, in $R^{3-1}$, $R^{3-2}$, and $R^1$, each "5- to 10-membered heteroaryl" in the"5- to 10-membered heteroaryl", 5- to 10-membered heteroaryl substituted by $C_1$-$C_6$ alkyl, and "5- to 10-membered heteroaryl substituted by one or more $R^{3-2}$" is independently 9- to 10-membered heteroaryl.

In some embodiments, in $R^{3X}$, each "5- to 10-membered heteroaryl" in the "5- to 10-membered heteroaryl" and the "5- to 10-membered heteroaryl substituted by one or more $R^{3a-1}$" is independently pyridyl.

In some embodiments, when $R^3$ and $R^{14}$ are each independently "$C_6$-$C_{10}$ aryl substituted by one or more $R^{3-1}$", and any two adjacent $R^{3-1}$, together with the carbon atom to which they are attached, form a 5- to 10-membered heteroaryl group or a "5- to 10-membered heteroaryl group substituted by one or more $R^{3-1-4}$", then the $R^3$ and $R^{14}$ are each independently

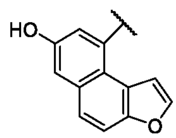

In some embodiments, -L-$R^1$ is

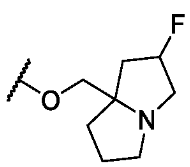 , 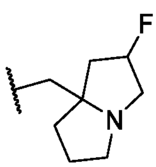 , 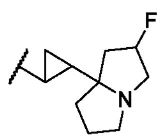 ,

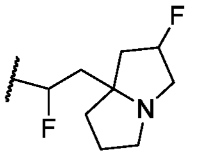, 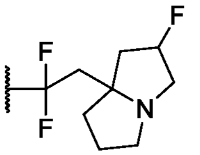, 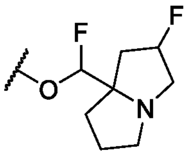, 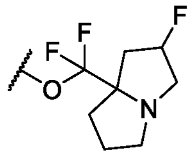, 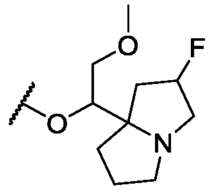,

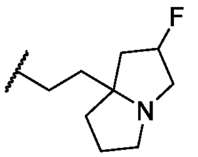, 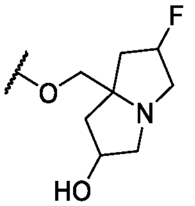, 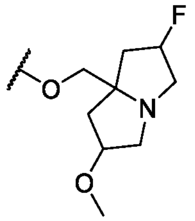, 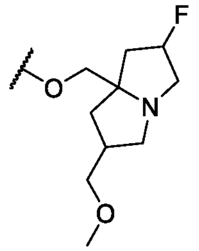, 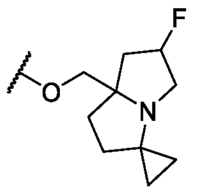,

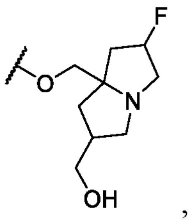, 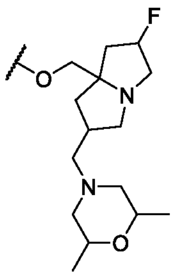, 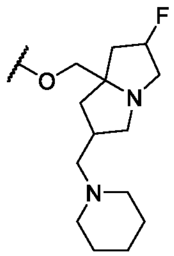, 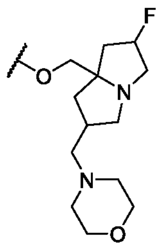, 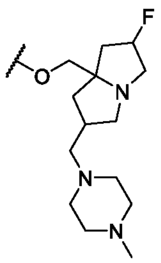, 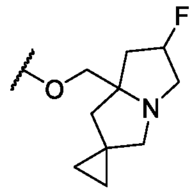,

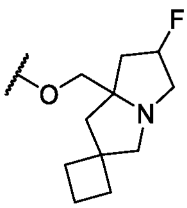, or 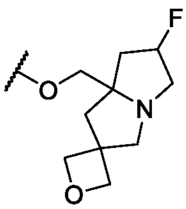,

preferably

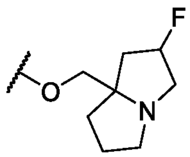, 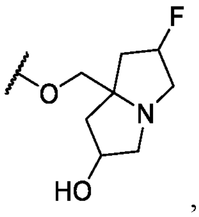, 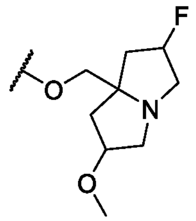,

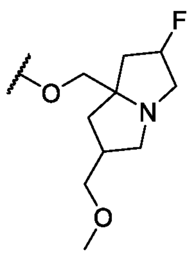, 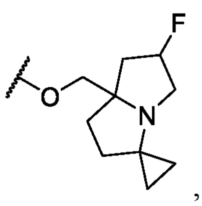, 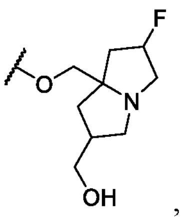, 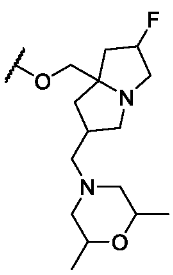, 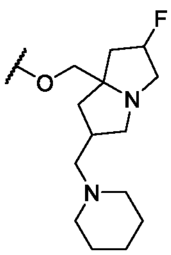, 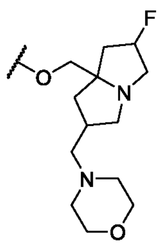,

or

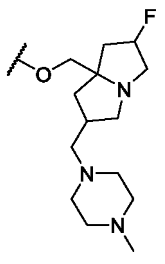,

more preferably

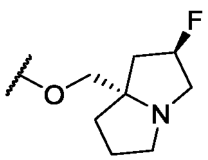, 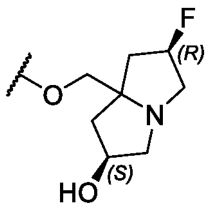, 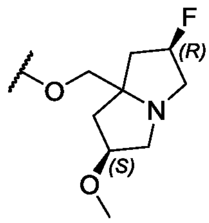,

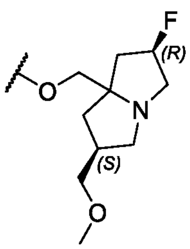, 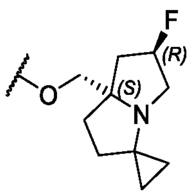, 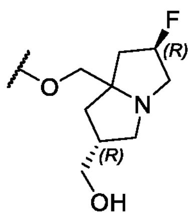, 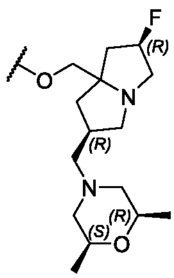, 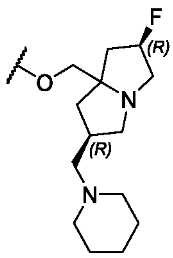, 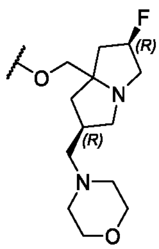,

or

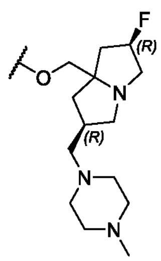

In some embodiments, $R^2$ is fluorine.

In some embodiments, $R^3$ is

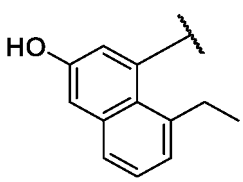, 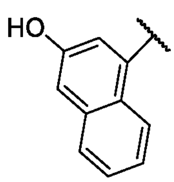, 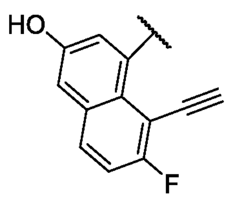,

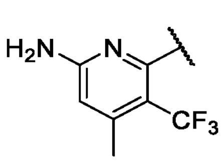, 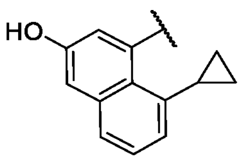, 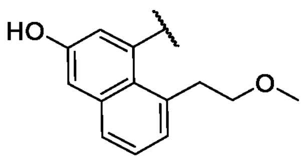, 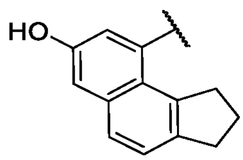,

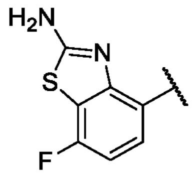, 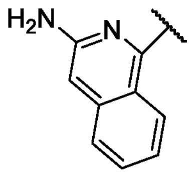, or 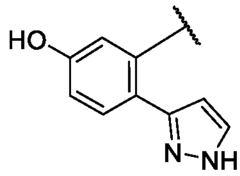,

preferably

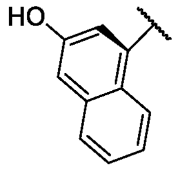, 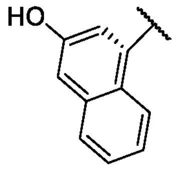,

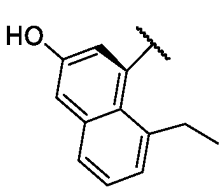, 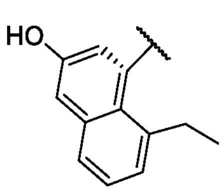, 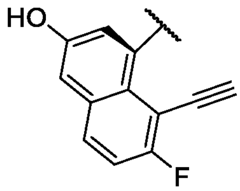, 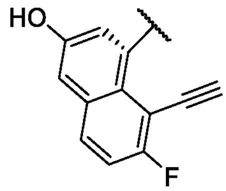, 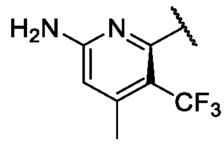,

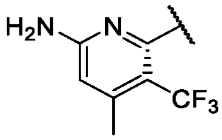, 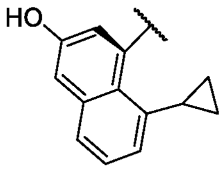, 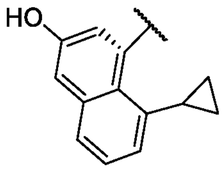, 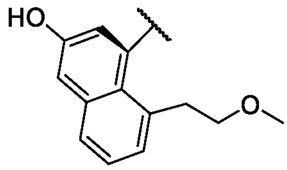,

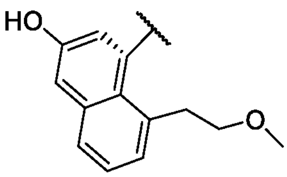, 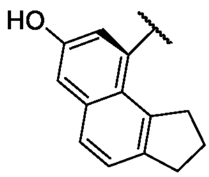, 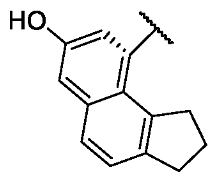, 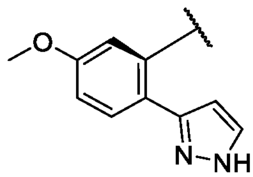,

or

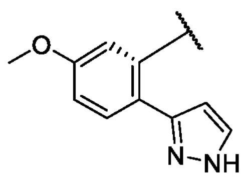

In some embodiments, $R^4$ is H, fluorine, chlorine, cyano, trifluoromethyl, hydroxyl, methoxy, or ethoxy, preferably hydrogen or fluorine.

In some embodiments, $R^9$, $R^{10}$, $R^{11}$, and $R^{12}$ are each independently H or methyl, preferably H.

In some embodiments, $X^1$, $X^2$, and $X^3$ are each independently $CH_2$, O, CHF, or $CF_2$, preferably $CH_2$.

In some embodiments, $R^5$ is OH or H, preferably OH.

In some embodiments, $R^6$ is H, chlorine, fluorine, methyl, trifluoromethyl, or cyclopropyl, preferably chlorine.

In some embodiments, ring A is

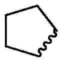, 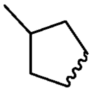, 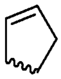, 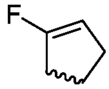, 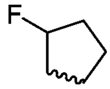, 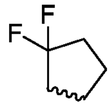,

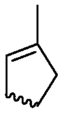, 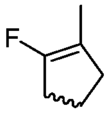, 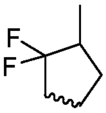, 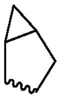, 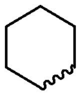, 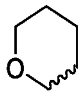, 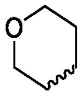, 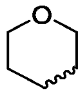, 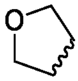, 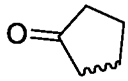, 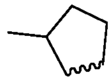,

or

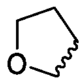

( ~ denoting a bond connected to the benzene ring in which $R^5$ is located), preferably

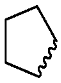, 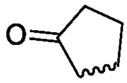, or 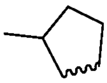,

for example,

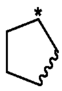, 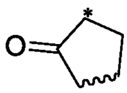, or 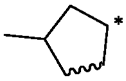

(* denoting a site connected to the ring in which $X^1$, $X^2$, and $X^3$ are located).

In some embodiments, $R^{13}$ is fluorine.

In some embodiments, $R^{14}$ is

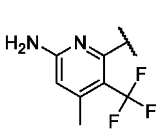, 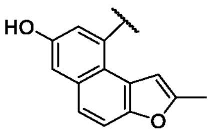, 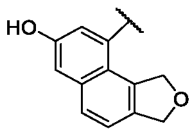,

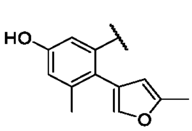, 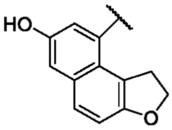, 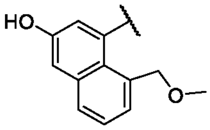, 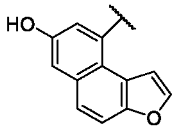, 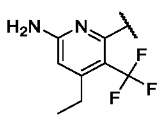,

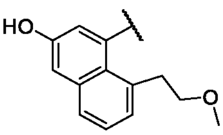, 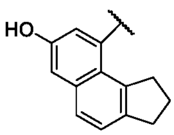, 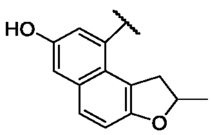, 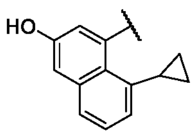, or 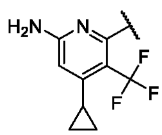

In some embodiments, $R^{3X}$ is

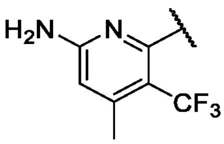

In some embodiments, the compound of formula I, formula II, or formula III, the pharmaceutically acceptable salt

thereof, the solvate thereof, the stereoisomer thereof, the tautomer thereof, the prodrug thereof, the metabolite thereof, or the isotopic compound thereof:

wherein X is N, O, or S;
n1 and n4 are independently 1, 2, 3, or 4;
each L is independently $-O-(CR^{L-1}R^{L-2})_{n2}-*$, $-(CR^{L-3}R^{L-4})_{n3}-*$, or

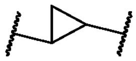
;

* represents one end connected to $R^1$;
n2 and n3 are each independently 0, 1, 2, 3, or 4;
$R^{L-1}$, $R^{L-2}$, $R^{L-3}$, and $R^{L-4}$ are each independently H, $C_1$-$C_6$ alkyl, $C_1$-$C_6$ alkoxy, $C_1$-$C_6$ alkyl substituted by one or more $R^{L-1-1}$, or halogen;
each $R^{L-1-1}$ is independently halogen or $C_1$-$C_6$ alkoxy;
each $R^1$ is 4- to 10-membered heterocycloalkyl substituted by one or more $R^{1-1}$; heteroatoms in the 4- to 10-membered heterocycloalkyl of the 4- to 10-membered heterocycloalkyl substituted by one or more $R^{1-1}$ are independently 1, 2, or 3 kinds of N, O, or S, and the number of heteroatoms is 1, 2, or 3;
each $R^{1-1}$ is independently halogen;
$R^2$ and $R^{13}$ are each independently H or halogen;
$R^3$ and $R^{14}$ are each independently $C_6$-$C_{10}$ aryl, $C_6$-$C_{10}$ aryl substituted by one or more $R^{3-1}$, 5- to 10-membered heteroaryl, or 5- to 10-membered heteroaryl substituted by one or more $R^{3-2}$; heteroatoms in the 5- to 10-membered heteroaryl and the 5- to 10-membered heteroaryl substituted by one or more $R^{3-2}$ are independently 1, 2, or 3 kinds of N, O, or S, and the number of heteroatoms is 1, 2, or 3;
$R^{3-1}$ and $R^{3-2}$ are each independently OH, halogen, $C_1$-$C_6$ alkyl, $C_1$-$C_6$ alkyl substituted by one or more $R^{3-1-1}$, $C_2$-$C_6$ alkynyl, 3- to 8-membered cycloalkyl, $-S-C(R^{3-1-2})_3$, $-S(R^{3-1-3})_5$, amino, $C_1$-$C_6$ alkyl, or 5- to 10-membered heteroaryl; alternatively, any two adjacent $R^{3-1}$, together with the carbon atom to which they are attached, form a 5- to 10-membered heteroaryl group or a 5- to 10-membered heteroaryl group substituted by one or more $R^{3-1-4}$; heteroatoms in the 5- to 10-membered heteroaryl group and the 5- to 10-membered heteroaryl group substituted by one or more $R^{3-1-4}$ are independently 1, 2, or 3 kinds of N, O, or S, and the number of heteroatoms is 1, 2, or 3;
each $R^{3-1-1}$ is independently oxo (=O), OH, $C_1$-$C_6$ alkoxy, or halogen;
$R^{3-1-2}$ and $R^{3-1-3}$ are each independently halogen;
each $R^{3-1-4}$ is independently $C_1$-$C_6$ alkyl;
$R^4$ is H, OH, $C_1$-$C_6$ alkyl, $C_1$-$C_6$ alkoxy, $C_1$-$C_6$ alkyl substituted by one or more $R^{4-1}$, cyano, or halogen;
each $R^{4-1}$ is independently halogen;
$X^1$ is $C(R^{1a}R^{1b})$ or O;
$X^2$ is $C(R^{2a}R^{2b})$ or O;
$X^3$ is $C(R^{3a}R^{3b})$ or O;
$R^{1a}$, $R^{1b}$, $R^{2a}$, $R^{2b}$, $R^{3a}$, and $R^{3b}$ are each independently H, $C_1$-$C_6$ alkyl, or halogen;
$R^5$ is H or OH;
$R^6$ is H, $C_1$-$C_6$ alkyl, $C_1$-$C_6$ alkoxy, 3- to 8-membered cycloalkyl, halogen, or $C_1$-$C_6$ alkyl substituted by one or more $R^{6-1}$;
each $R^{6-1}$ is independently halogen;
$R^7$ and $R^8$ are connected to form ring A, and ring A is a 5- to 6-membered saturated or unsaturated monocyclic, spirocyclic, or fused carbocyclic ring, a 5- to 6-membered saturated or unsaturated monocyclic, spirocyclic, or fused carbocyclic ring substituted by one or more $R^{7-1}$, a 5- to 6-membered saturated or unsaturated monocyclic, spirocyclic, or fused heterocyclic ring with 1, 2, or 3 heteroatoms selected from 1, 2, or 3 kinds of N, O, and S, or a 5- to 6-membered saturated or unsaturated monocyclic, spirocyclic, or fused heterocyclic ring with 1, 2, or 3 heteroatoms selected from 1, 2, or 3 kinds of N, O, and S substituted by one or more $R^{7-2}$;
$R^{7-1}$ and $R^{7-2}$ are each independently $C_1$-$C_6$ alkyl, oxo, or halogen;
$R^9$, $R^{10}$, $R^{11}$, $R^{12}$, $R^{15}$, and $R^{16}$ are each independently H, $C_1$-$C_6$ alkyl, or halogen.

In some embodiments, the compound of formula I, formula II, or formula III, the pharmaceutically acceptable salt thereof, the solvate thereof, the stereoisomer thereof, the tautomer thereof, the prodrug thereof, the metabolite thereof, or the isotopic compound thereof:

in the compound of formula I, formula II, or formula III, X is N, O, or S;

n1 and n4 are independently 1, 2, 3, or 4;
each L is independently -O-($CR^{L-1}R^{L-2}$)$_{n2}$-*, -($CR^{L-3}R^{L-4}$)$_{n3}$-*, or

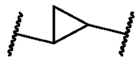;

* represents one end connected to $R^1$;
n2 and n3 are each independently 0, 1, 2, 3, or 4;
$R^{L-1}$, $R^{L-2}$, $R^{L-3}$, and $R^{L-4}$ are each independently H, $C_1$-$C_6$ alkyl, $C_1$-$C_6$ alkoxy, $C_1$-$C_6$ alkyl substituted by one or more $R^{L-1-1}$, or halogen;
each $R^{L-1-1}$ is independently halogen or $C_1$-$C_6$ alkoxy;
each $R^1$ is 4- to 10-membered heterocycloalkyl substituted by one or more $R^{1-1}$; heteroatoms in the 4- to 10-membered heterocycloalkyl of the 4- to 10-membered heterocycloalkyl substituted by one or more $R^{1-1}$ are independently 1, 2, or 3 kinds of N, O, or S, and the number of heteroatoms is 1, 2, or 3;
each $R^{1-1}$ is independently halogen;
$R^2$ and $R^{13}$ are each independently H or halogen;
$R^3$ and $R^{14}$ are each independently $C_6$-$C_{10}$ aryl, $C_6$-$C_{10}$ aryl substituted by one or more $R^{3-1}$, 5- to 10-membered heteroaryl, or 5- to 10-membered heteroaryl substituted by one or more $R^{3-2}$; heteroatoms in the 5- to 10-membered heteroaryl and the 5- to 10-membered heteroaryl substituted by one or more $R^{3-2}$ are independently 1, 2, or 3 kinds of N, O, or S, and the number of heteroatoms is 1, 2, or 3;
$R^{3-1}$ and $R^{3-2}$ are each independently OH, halogen, $C_1$-$C_6$ alkyl, $C_1$-$C_6$ alkyl substituted by one or more $R^{3-1-1}$, $C_2$-$C_6$ alkynyl, 3- to 8-membered cycloalkyl, -S-C($R^{3-1-2}$)$_3$, or -S($R^{3-1-3}$)$_5$, or amino;
alternatively, any two adjacent $R^{3-1}$, together with the carbon atom to which they are attached, form a 5- to 10-membered heteroaryl group or a 5- to 10-membered heteroaryl group substituted by one or more $R^{3-1-4}$; heteroatoms in the 5- to 10-membered heteroaryl group and the 5- to 10-membered heteroaryl group substituted by one or more $R^{3-1-4}$ are independently 1, 2, or 3 kinds of N, O, or S, and the number of heteroatoms is 1, 2, or 3;
each $R^{3-1-1}$ is independently oxo (=O), OH, $C_1$-$C_6$ alkoxy, or halogen;
$R^{3-1-2}$ and $R^{3-1-3}$ are each independently halogen;
each $R^{3-1-4}$ is independently $C_1$-$C_6$ alkyl;
$R^4$ is H, OH, $C_1$-$C_6$ alkyl, $C_1$-$C_6$ alkoxy, $C_1$-$C_6$ alkyl substituted by one or more $R^{4-1}$, cyano, or halogen;
each $R^{4-1}$ is independently halogen;
$X^1$ is C($R^{1a}R^{1b}$) or O;
$X^2$ is C($R^{2a}R^{2b}$) or O;
$X^3$ is C($R^{3a}R^{3b}$) or O;
$R^{1a}$, $R^{1b}$, $R^{2a}$, $R^{2b}$, $R^{3a}$, and $R^{3b}$ are each independently H, $C_1$-$C_6$ alkyl, or halogen;
$R^5$ is H or OH;
$R^6$ is H, $C_1$-$C_6$ alkyl, $C_1$-$C_6$ alkoxy, 3- to 8-membered cycloalkyl, halogen, or $C_1$-$C_6$ alkyl substituted by one or more $R^{6-1}$;
each $R^{6-1}$ is independently halogen;
$R^7$ and $R^8$ are connected to form ring A, and ring A is a 5- to 6-membered saturated or unsaturated monocyclic, spirocyclic, or fused carbocyclic ring, a 5- to 6-membered saturated or unsaturated monocyclic, spirocyclic, or fused carbocyclic ring substituted by one or more $R^{7-1}$, a 5- to 6-membered saturated or unsaturated monocyclic, spirocyclic, or fused heterocyclic ring with 1, 2, or 3 heteroatoms selected from 1, 2, or 3 kinds of N, O, and S, or a 5- to 6-membered saturated or unsaturated monocyclic, spirocyclic, or fused heterocyclic ring with 1, 2, or 3 heteroatoms selected from 1, 2, or 3 kinds of N, O, and S substituted by one or more $R^{7-2}$;
$R^{7-1}$ and $R^{7-2}$ are each independently $C_1$-$C_6$ alkyl, oxo, or halogen;
$R^9$, $R^{10}$, $R^{11}$, $R^{12}$, $R^{15}$, and $R^{16}$ are each independently H, $C_1$-$C_6$ alkyl, or halogen.

In some embodiments, the compound of formula I or formula II, the pharmaceutically acceptable salt thereof, the solvate thereof, the stereoisomer thereof, the tautomer thereof, the prodrug thereof, the metabolite thereof, or the isotopic compound thereof:

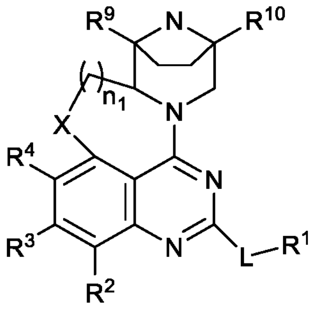

I ,

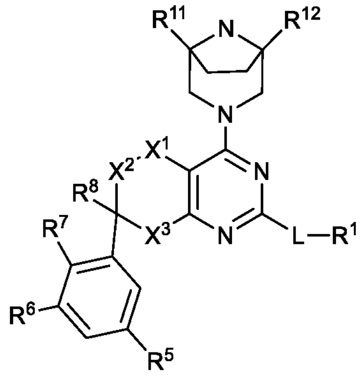

II ,

wherein X is N, O, or S;
n1 is 1, 2, 3, or 4;
L is $-O-(CR^{L-1}R^{L-2})_{n2}-*$, $-(CR^{L-3}R^{L-4})_{n3}-*$, or

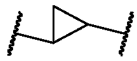 ;

* represents one end connected to $R^1$;
n2 and n3 are each independently 0, 1, 2, 3, or 4;
$R^{L-1}$, $R^{L-2}$, $R^{L-3}$, and $R^{L-4}$ are each independently H, $C_1$-$C_6$ alkyl, $C_1$-$C_6$ alkoxy, $C_1$-$C_6$ alkyl substituted by one or more $R^{L-1-1}$, or halogen;
each $R^{L-1-1}$ is independently halogen or $C_1$-$C_6$ alkoxy;
$R^1$ is 4- to 10-membered heterocycloalkyl substituted by one or more $R^{1-1}$; heteroatoms in the 4- to 10-membered heterocycloalkyl of the 4- to 10-membered heterocycloalkyl substituted by one or more $R^{1-1}$ are independently 1, 2, or 3 kinds of N, O, or S, and the number of heteroatoms is 1, 2, or 3;
each $R^{1-1}$ is independently halogen;
$R^2$ is H or halogen;
$R^3$ is $C_6$-$C_{10}$ aryl, $C_6$-$C_{10}$ aryl substituted by one or more $R^{3-1}$, or 5- to 10-membered heteroaryl; heteroatoms in the 5- to 10-membered heteroaryl are independently 1, 2, or 3 kinds of N, O, or S, and the number of heteroatoms is 1, 2, or 3;
each $R^{3-1}$ is independently OH, halogen, $C_1$-$C_6$ alkyl, $C_1$-$C_6$ alkyl substituted by one or more $R^{3-1-1}$, $C_2$-$C_6$ alkynyl, 3- to 8-membered cycloalkyl, $-S-C(R^{3-1-2})_3$, or $-S(R^{3-1-3})_5$;
each $R^{3-1-1}$ is independently oxo (=O), OH, $C_1$-$C_6$ alkoxy, or halogen;
$R^{3-1-2}$ and $R^{3-1-3}$ are each independently halogen;
$R^4$ is H, OH, $C_1$-$C_6$ alkyl, $C_1$-$C_6$ alkoxy, $C_1$-$C_6$ alkyl substituted by one or more $R^{4-1}$, cyano, or halogen;
each $R^{4-1}$ is independently halogen;
$X^1$ is $C(R^{1a}R^{1b})$ or O;
$X^2$ is $C(R^{2a}R^{2b})$ or O;
$X^3$ is $C(R^{3a}R^{3b})$ or O;
$R^{1a}$, $R^{1b}$, $R^{2a}$, $R^{2b}$, $R^{3a}$, and $R^{3b}$ are each independently H, $C_1$-$C_6$ alkyl, or halogen;
$R^5$ is H or OH;
$R^6$ is H, $C_1$-$C_6$ alkyl, $C_1$-$C_6$ alkoxy, 3- to 8-membered cycloalkyl, halogen, or $C_1$-$C_6$ alkyl substituted by one or more $R^{6-1}$;
each $R^{6-1}$ is independently halogen;
$R^7$ and $R^8$ are connected to form ring A, and ring A is a 5- to 6-membered saturated or unsaturated monocyclic, spirocyclic, or fused carbocyclic ring, a 5- to 6-membered saturated or unsaturated monocyclic, spirocyclic, or fused carbocyclic ring substituted by one or more $R^{7-1}$, a 5- to 6-membered saturated or unsaturated monocyclic, spirocyclic, or fused heterocyclic ring with 1, 2, or 3 heteroatoms selected from 1, 2, or 3 kinds of N, O, and S, or a 5- to 6-membered saturated or unsaturated monocyclic, spirocyclic, or fused heterocyclic ring with 1, 2, or 3 heteroatoms selected from 1, 2, or 3 kinds of N, O, and S substituted by one or more $R^{7-2}$;
$R^{7-1}$ and $R^{7-2}$ are each independently $C_1$-$C_6$ alkyl, oxo (=O), or halogen;
$R^9$, $R^{10}$, $R^{11}$, and $R^{12}$ are each independently H, $C_1$-$C_6$ alkyl, or halogen.
In some embodiments, X is N, O, or S;

n1 is 1, 2, 3, or 4;

L is independently -O-$(CR^{L-1}R^{L-2})_{n2}$-*, -$(CR^{L-3}R^{L-4})_{n3}$-*, or

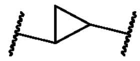;

* represents one end connected to $R^1$;

n2 and n3 are each independently 0, 1, 2, 3, or 4;

$R^{L-1}$, $R^{L-2}$, $R^{L-3}$, and $R^{L-4}$ are each independently H, $C_1$-$C_6$ alkyl, $C_1$-$C_6$ alkoxy, $C_1$-$C_6$ alkyl substituted by one or more $R^{L-1-1}$, or halogen;

each $R^{L-1-1}$ is independently halogen or $C_1$-$C_6$ alkoxy;

$R^1$ is 4- to 10-membered heterocycloalkyl substituted by one or more $R^{1-1}$; heteroatoms in the 4- to 10-membered heterocycloalkyl of the 4- to 10-membered heterocycloalkyl substituted by one or more $R^{1-1}$ are independently 1, 2, or 3 kinds of N, O, or S, and the number of heteroatoms is 1, 2, or 3;

each $R^{1-1}$ is independently halogen;

$R^2$ is H or halogen;

$R^3$ is $C_6$-$C_{10}$ aryl, $C_6$-$C_{10}$ aryl substituted by one or more $R^{3-1}$, or 5- to 10-membered heteroaryl; heteroatoms in the 5- to 10-membered heteroaryl are independently 1, 2, or 3 kinds of N, O, or S, and the number of heteroatoms is 1, 2, or 3;

each $R^{3-1}$ is independently OH, halogen, $C_1$-$C_6$ alkyl, $C_1$-$C_6$ alkyl substituted by one or more $R^{3-1-1}$, $C_2$-$C_6$ alkynyl, 3- to 8-membered cycloalkyl, -S-$C(R^{3-1-2})_3$, or -$S(R^{3-1-3})_5$;

each $R^{3-1-1}$ is independently oxo (=O), OH, $C_1$-$C_6$ alkoxy, or halogen;

$R^{3-1-2}$ and $R^{3-1-3}$ are each independently halogen;

$R^4$ is H, OH, $C_1$-$C_6$ alkyl, $C_1$-$C_6$ alkoxy, $C_1$-$C_6$ alkyl substituted by one or more $R^{4-1}$, cyano, or halogen;

each $R^{4-1}$ is independently halogen;

$X^1$ is $C(R^{1a}R^{1b})$ or O;

$X^2$ is $C(R^{2a}R^{2b})$ or O;

$X^3$ is $C(R^{3a}R^{3b})$ or O;

$R^{1a}$, $R^{1b}$, $R^{2a}$, $R^{2b}$, $R^{3a}$, and $R^{3b}$ are each independently H, $C_1$-$C_6$ alkyl, or halogen;

$R^5$ is H or OH;

$R^6$ is H, $C_1$-$C_6$ alkyl, $C_1$-$C_6$ alkoxy, 3- to 8-membered cycloalkyl, halogen, or $C_1$-$C_6$ alkyl substituted by one or more $R^{6-1}$;

each $R^{6-1}$ is independently halogen;

$R^7$ and $R^8$ are connected to form ring A, and ring A is a 5- to 6-membered saturated or unsaturated monocyclic, spirocyclic, or fused carbocyclic ring, a 5- to 6-membered saturated or unsaturated monocyclic, spirocyclic, or fused carbocyclic ring substituted by one or more $R^{7-1}$, a 5- to 6-membered saturated or unsaturated monocyclic, spirocyclic, or fused heterocyclic ring with 1, 2, or 3 heteroatoms selected from 1, 2, or 3 kinds of N, O, and S, or a 5- to 6-membered saturated or unsaturated monocyclic, spirocyclic, or fused heterocyclic ring with 1, 2, or 3 heteroatoms selected from 1, 2, or 3 kinds of N, O, and S substituted by one or more $R^{7-2}$;

$R^{7-1}$ and $R^{7-2}$ are each independently $C_1$-$C_6$ alkyl or halogen;

$R^9$, $R^{10}$, $R^{11}$, and $R^{12}$ are each independently H, $C_1$-$C_6$ alkyl, or halogen.

In some embodiments, the compound of formula I, formula II, formula III, or formula IV is any one of the following compounds:

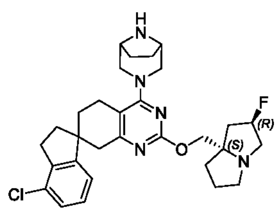

1'

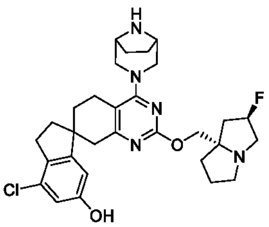

2'

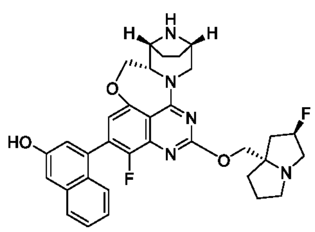

3

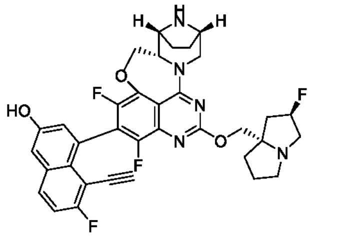

4'

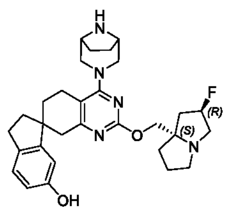
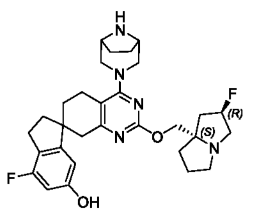
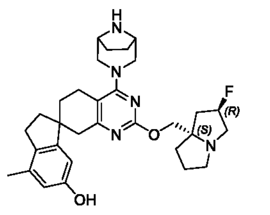

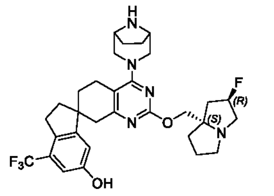
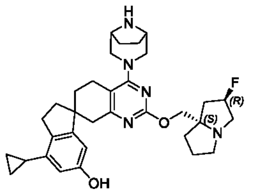
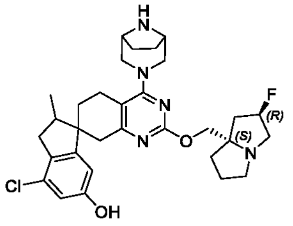

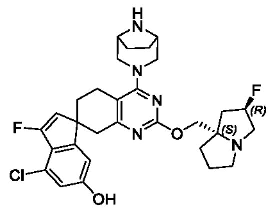
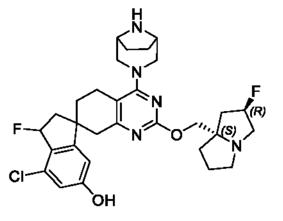
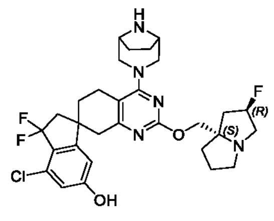
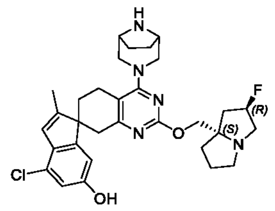

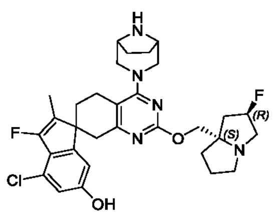
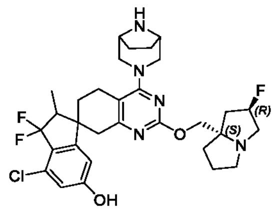
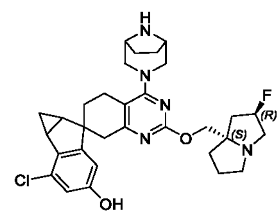
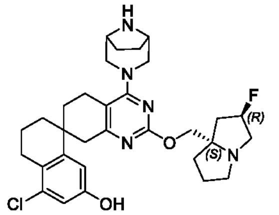

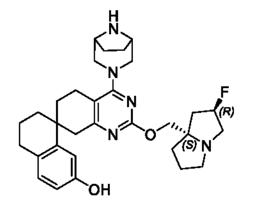
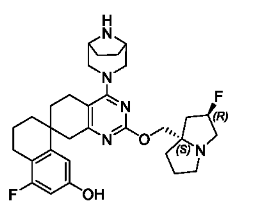
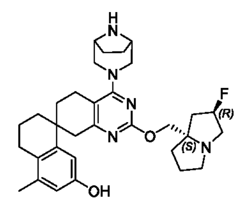
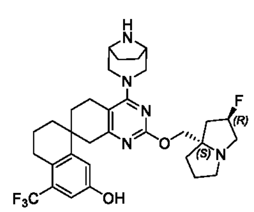

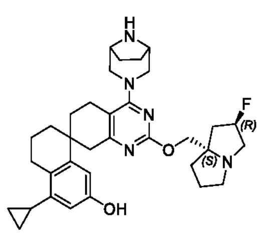
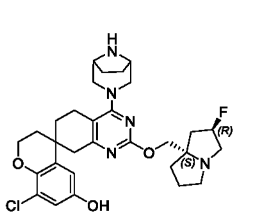
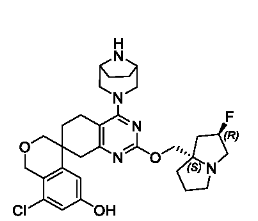
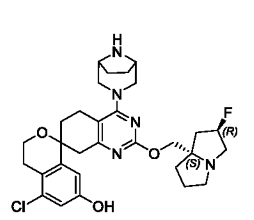

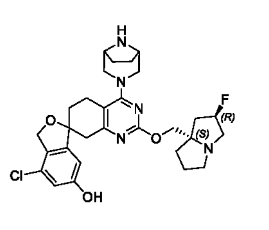
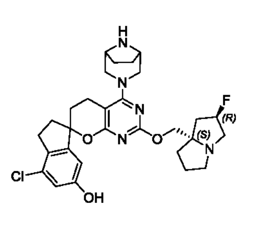
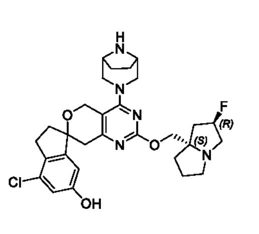
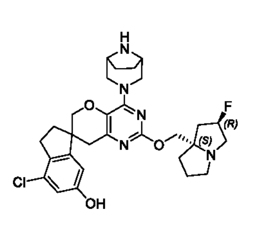

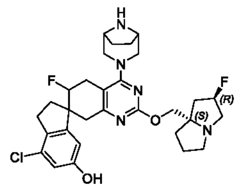
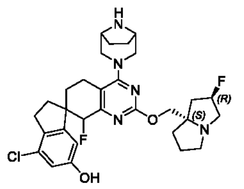
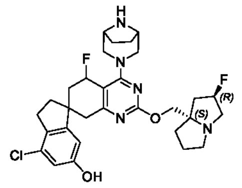
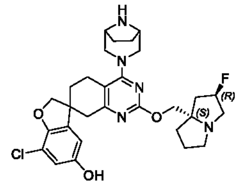

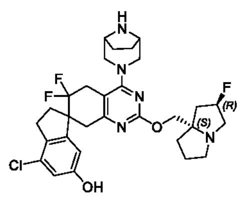
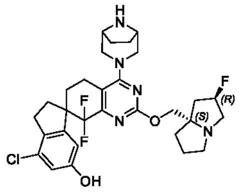
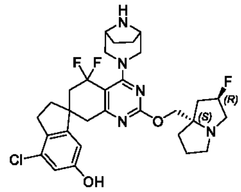
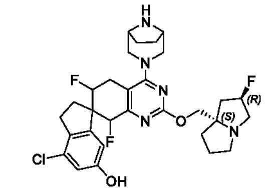

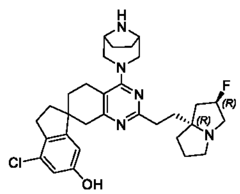
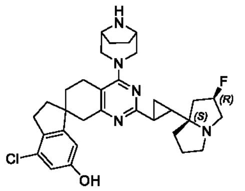
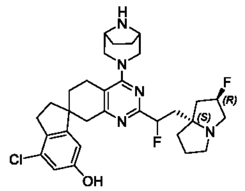
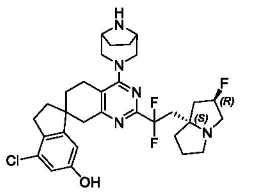

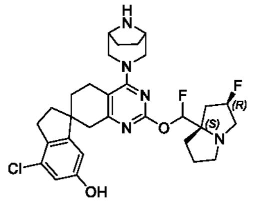
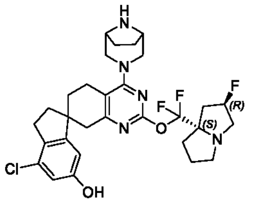
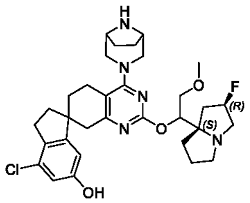
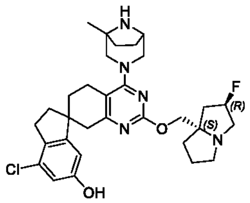

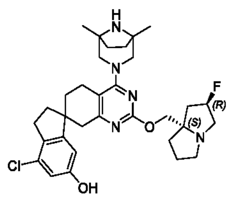
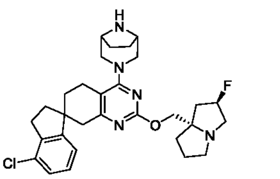
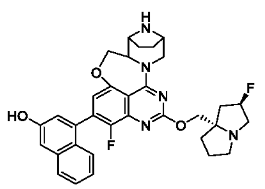
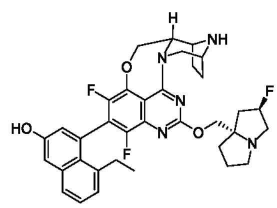

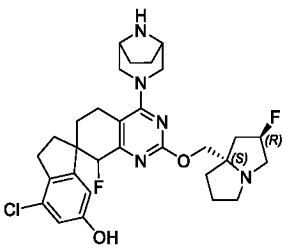
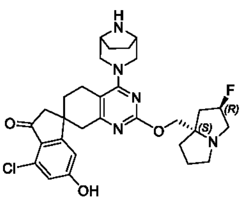
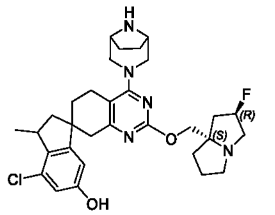
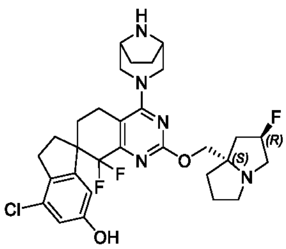

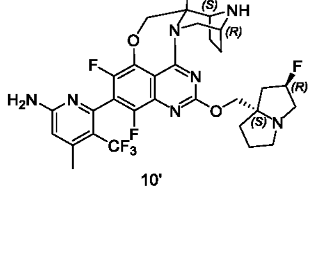
10'

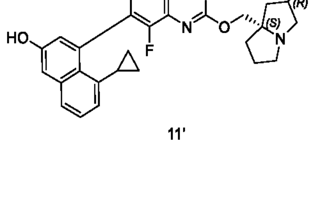
11'

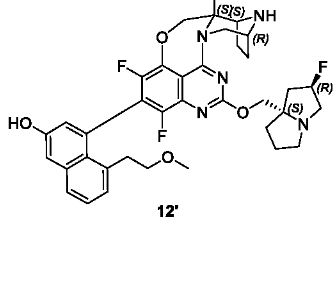
12'

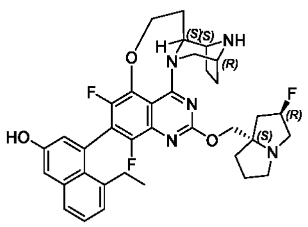

13'

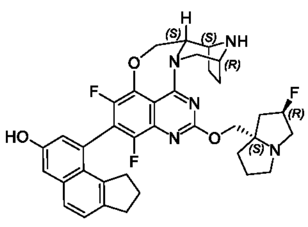

14'

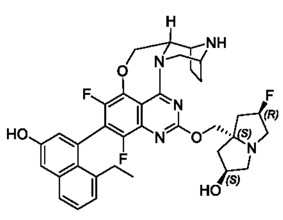

15'

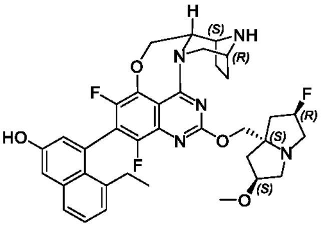

16'

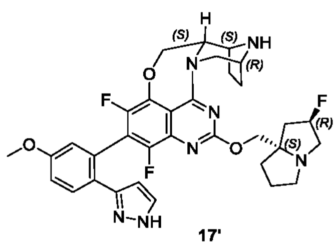

17'

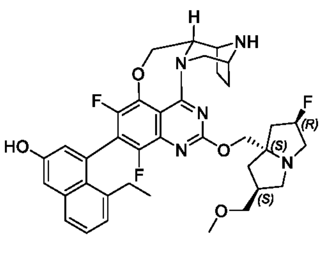

18'

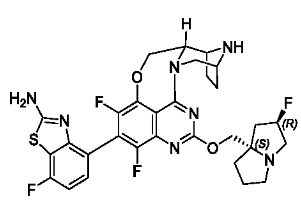

19'

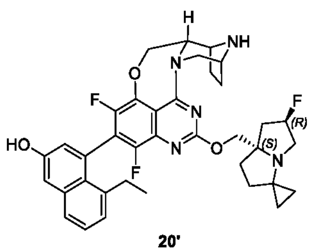

20'

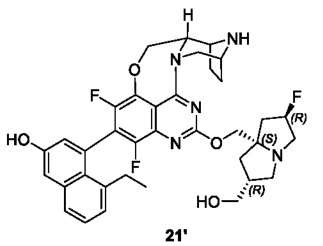

21'

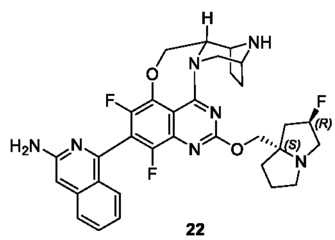

22

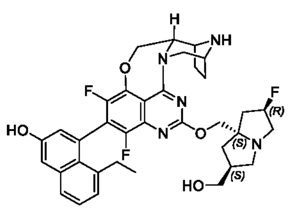

23'

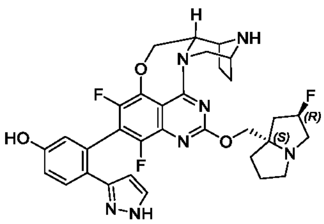

24'

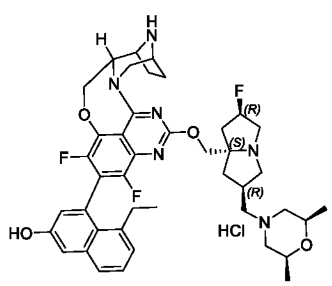

25'

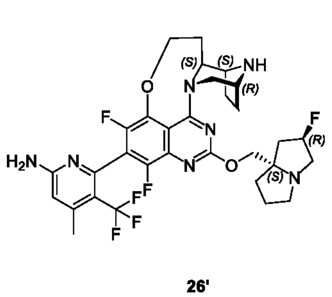

26'

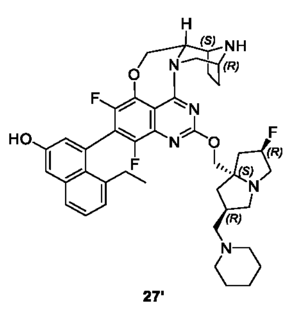

27'

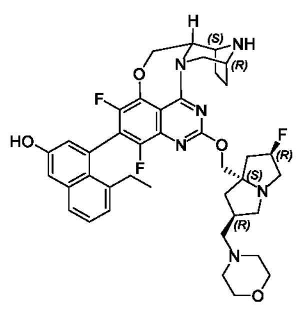

28'

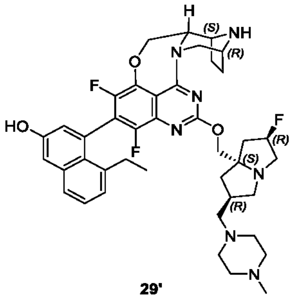

29'

In some embodiments, the stereoisomer of the compound of formula I, formula II, formula III, or formula IV is any one of the following compounds:

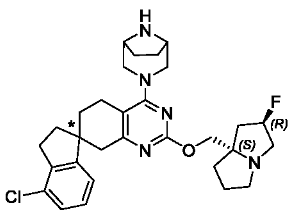

1a'

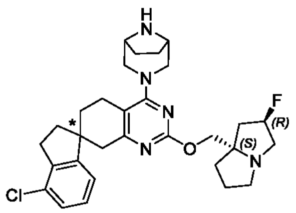

1b'

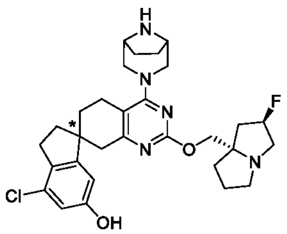

2a'

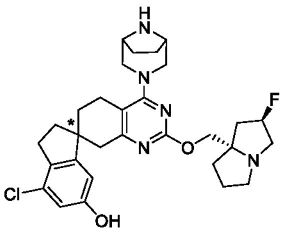

2b'

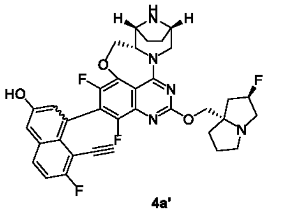

4a'

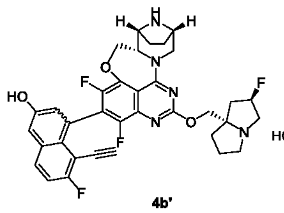

4b'

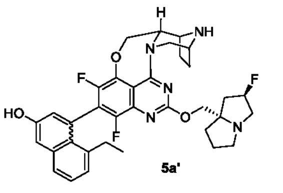

5a'

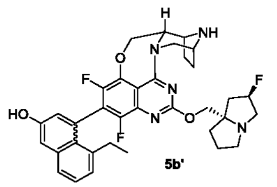

5b'

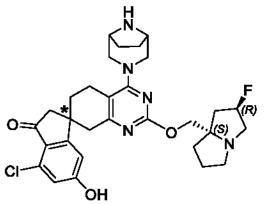

6a'

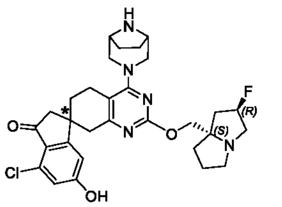

6b'

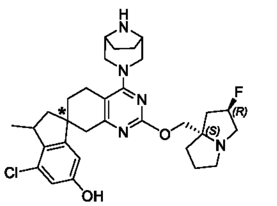

7a'

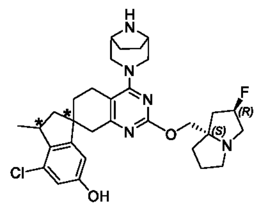

7aa'

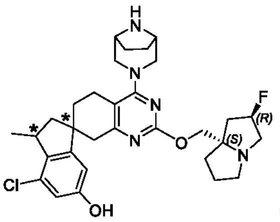

7ab'

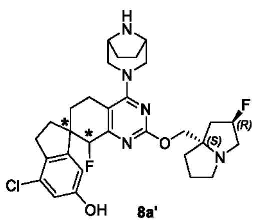

8a'

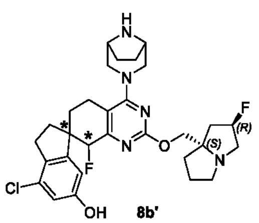

8b'

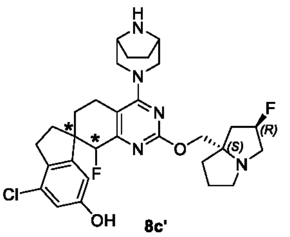

8c'

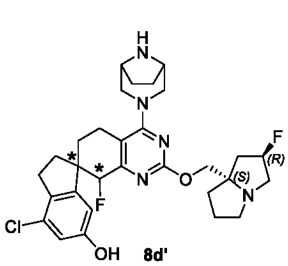
8d'

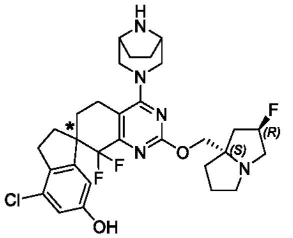
9a'

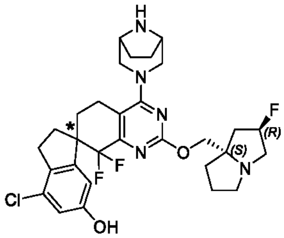
9b'

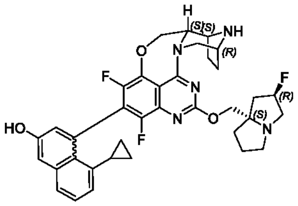
11a'

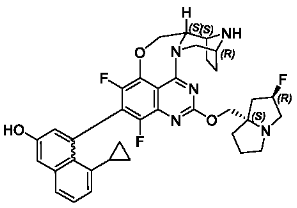
11b'

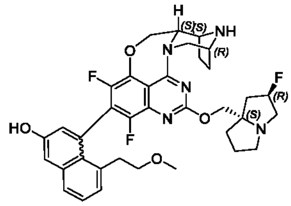
12a'

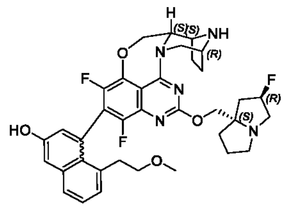
12b'

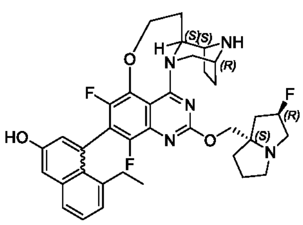
13a'

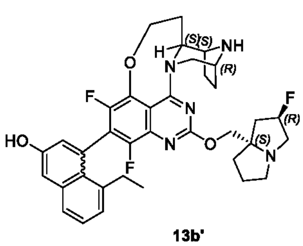
13b'

| Compound | Condition | Retention time |
|---|---|---|
| 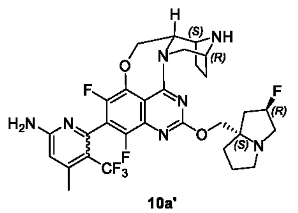 10a' | Chromatographic column YMC-Actus Triart C18 ExRS, 30 × 150 mm, 5 μm; mobile phase A: water (10 mmol/L ammonium bicarbonate), mobile phase B: acetonitrile; flow rate: 60 mL/min; elution with 25% to 45% phase B in 13 minutes; detector: 220 nm | 10.13 minutes |
| 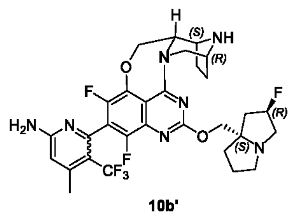 10b' | | 10.98 minutes |

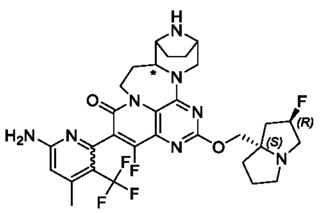

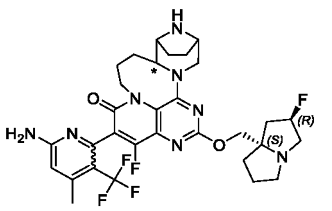

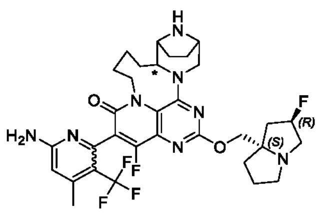

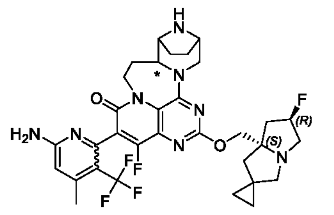

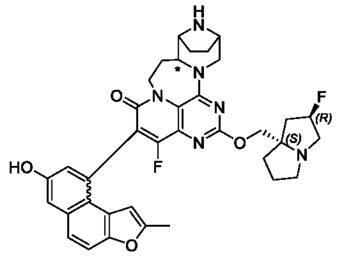

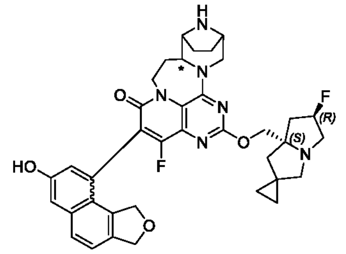

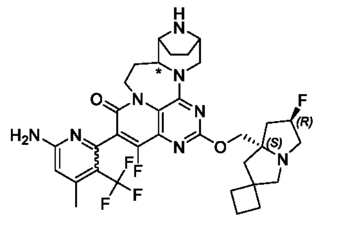

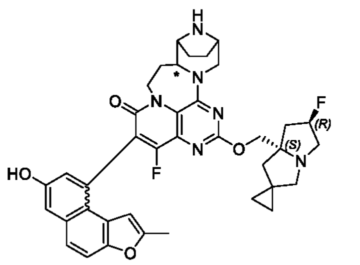

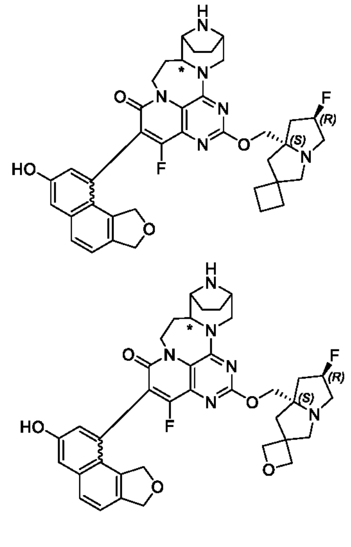

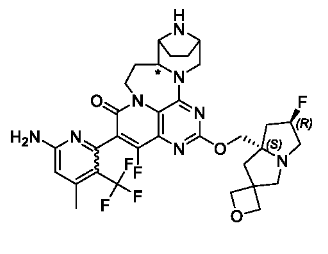

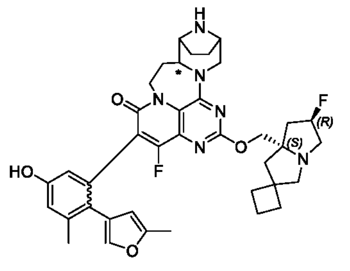

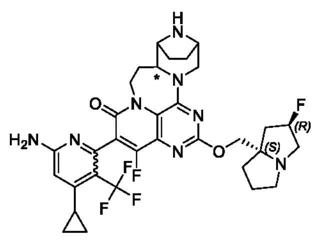

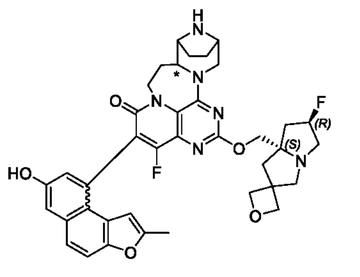

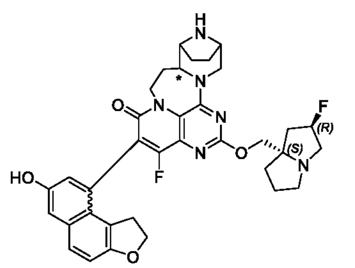

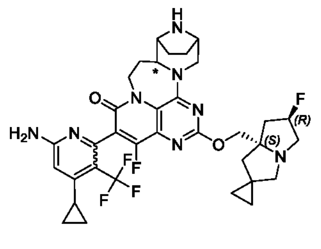

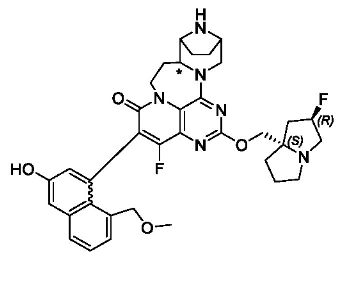

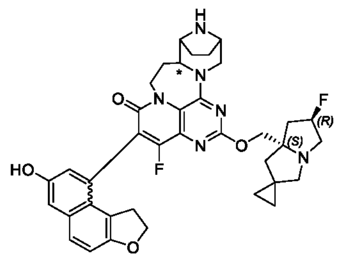

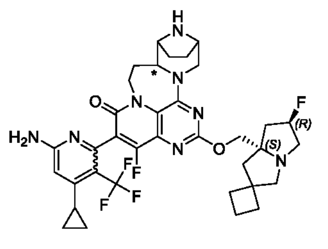

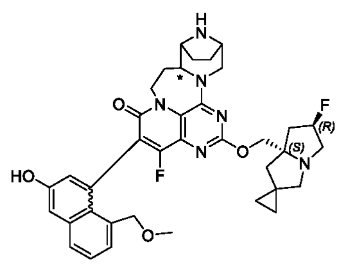

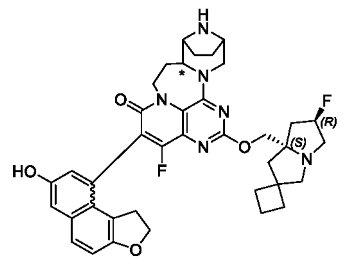

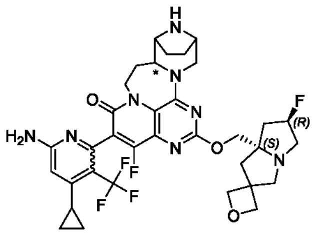
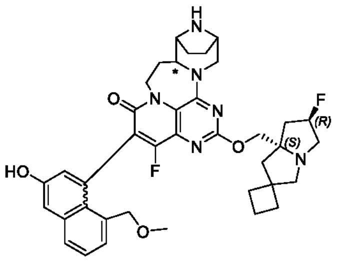
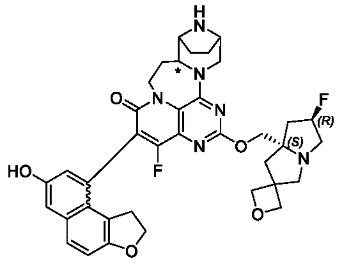

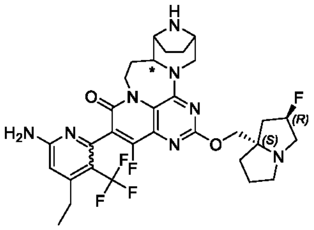
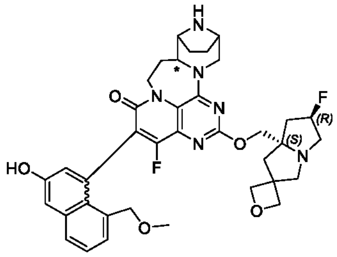
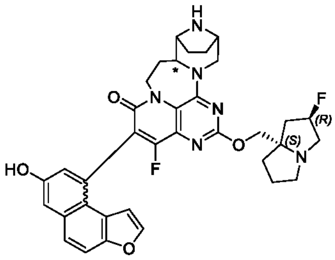

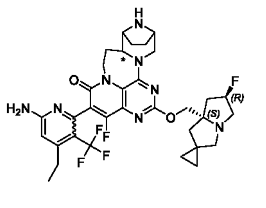
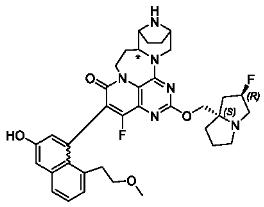
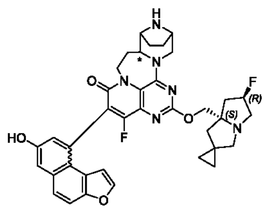

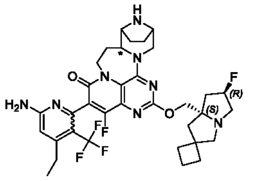
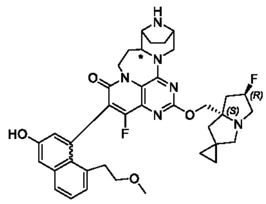
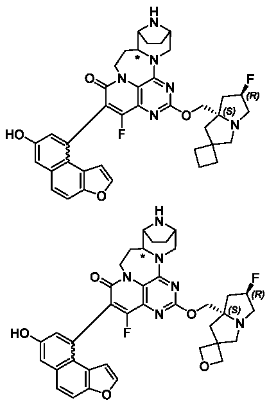

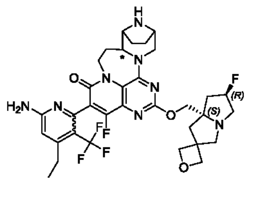
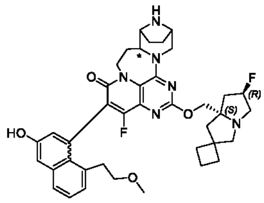

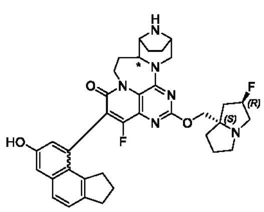
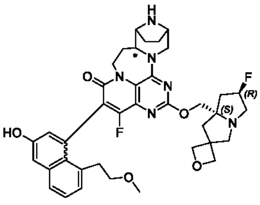
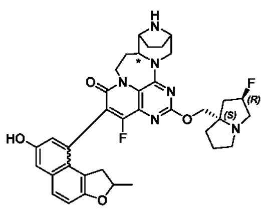

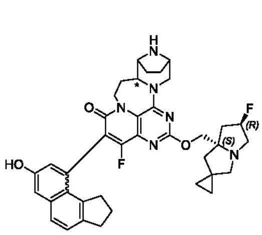

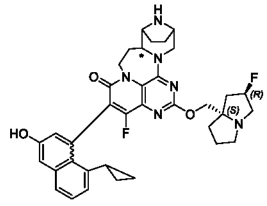

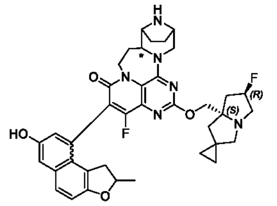

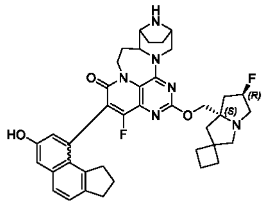

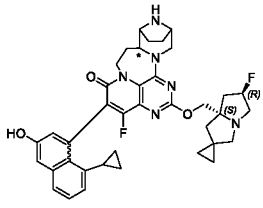

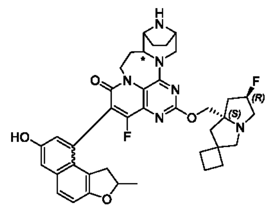

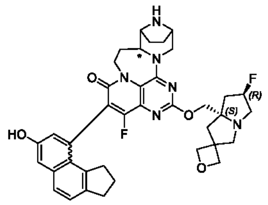

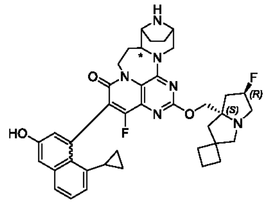

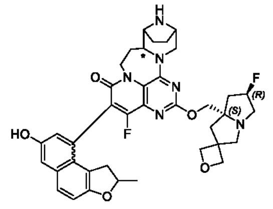

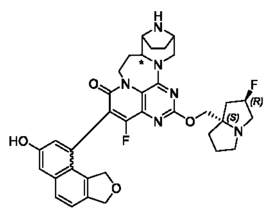

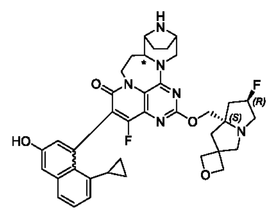

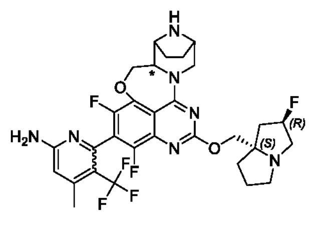

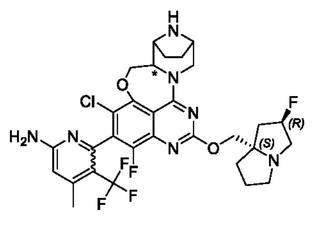

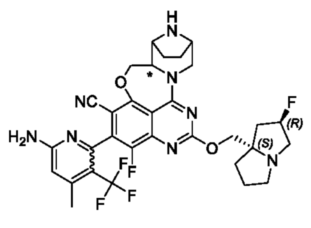

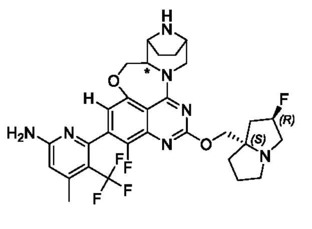

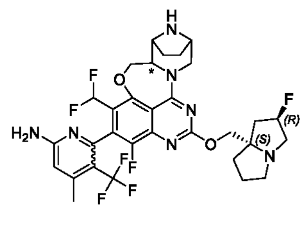

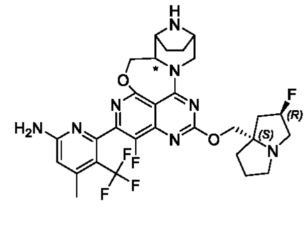

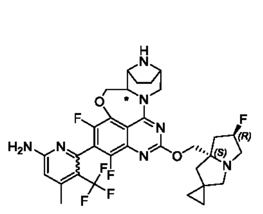

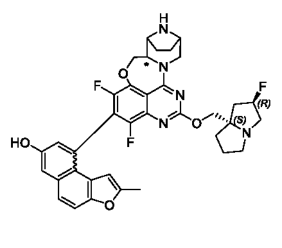

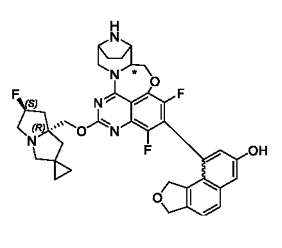

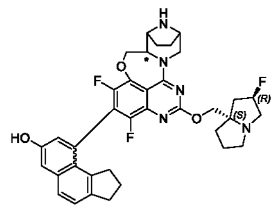

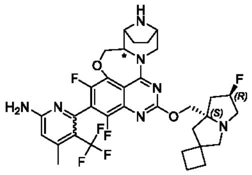
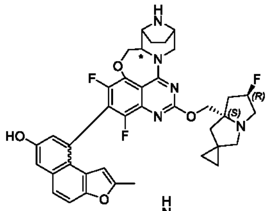
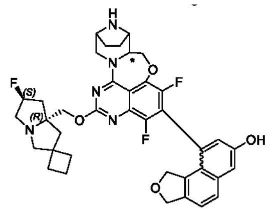
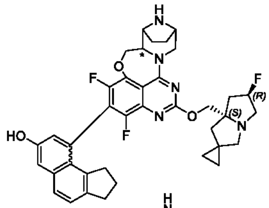
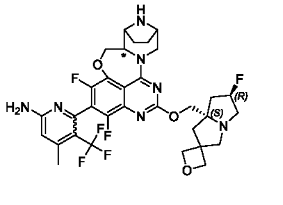
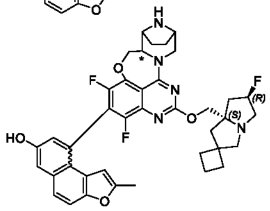
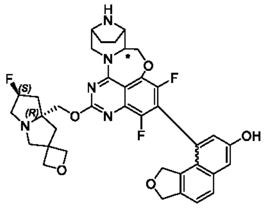
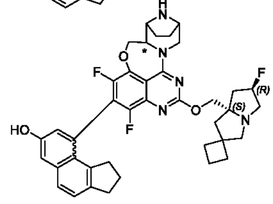
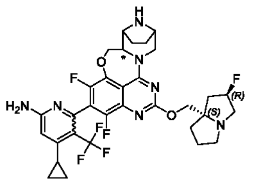
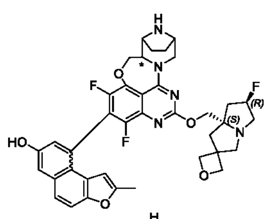
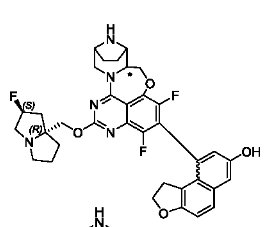
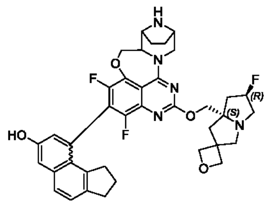
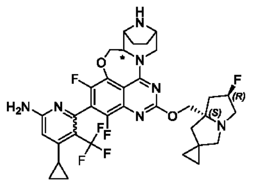
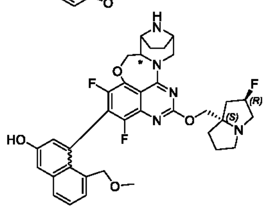
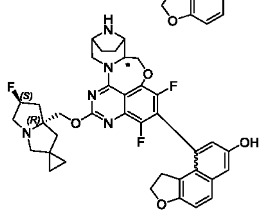
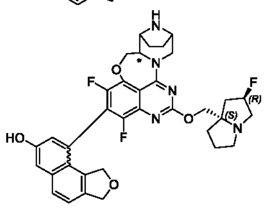
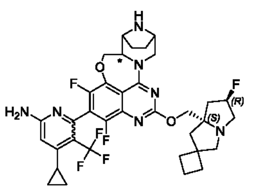
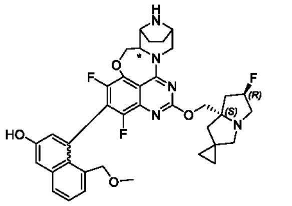
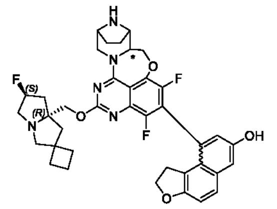
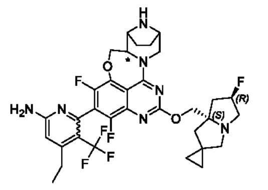
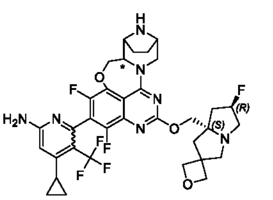
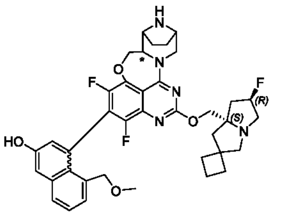
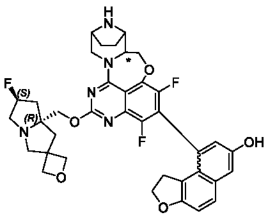
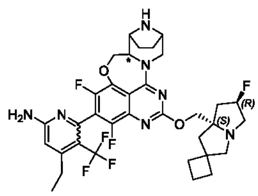
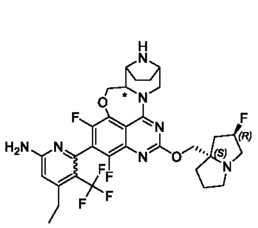
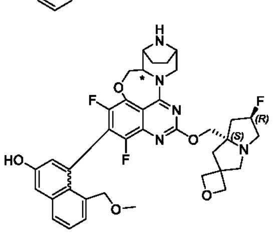
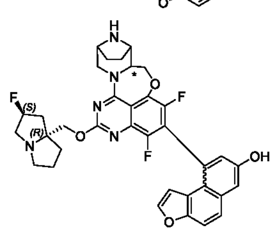
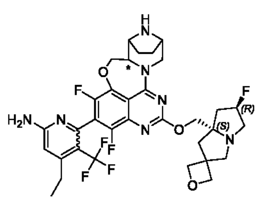

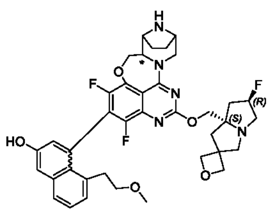
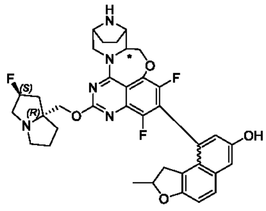
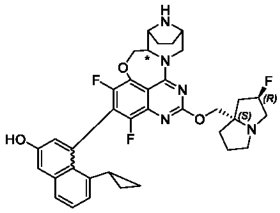
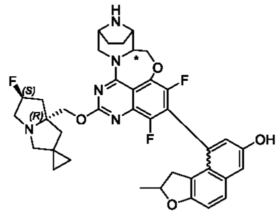

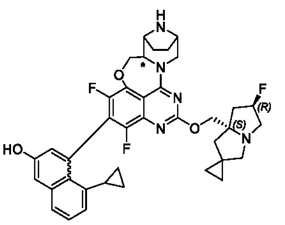
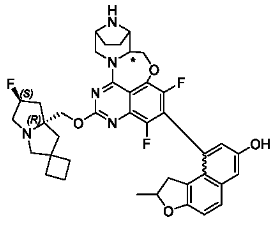
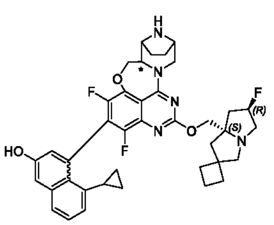
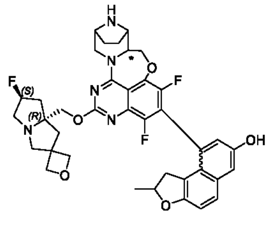

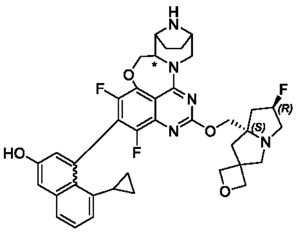
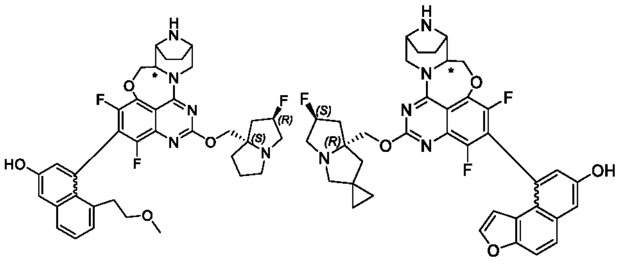

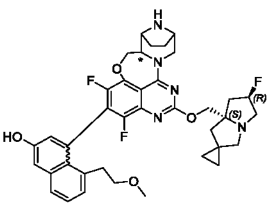
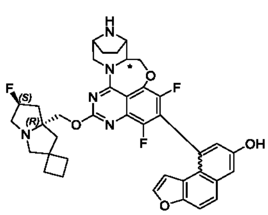
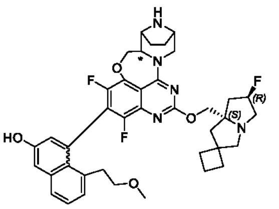
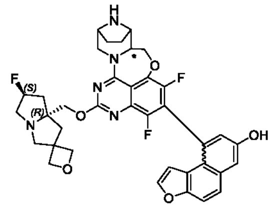

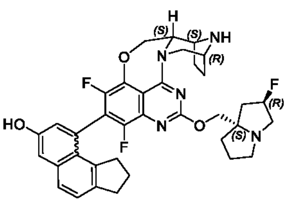
14a'

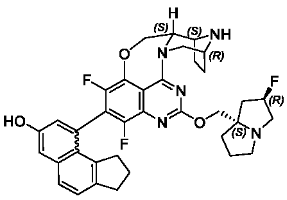
14b'

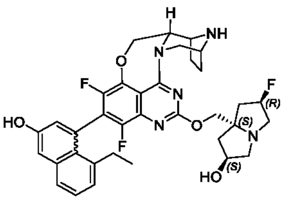
15a'

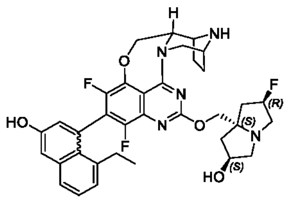
15b'

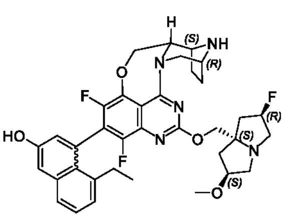
16a'

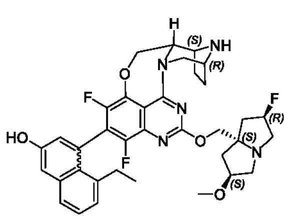
16b'

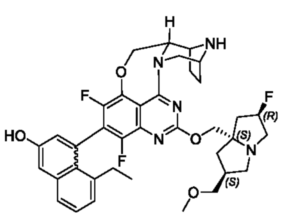
18a'

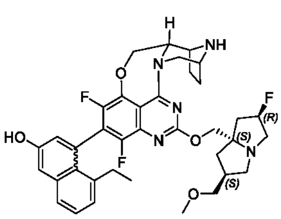
18b'

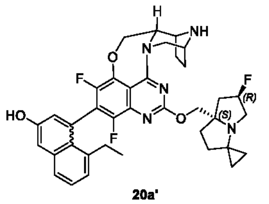
20a'

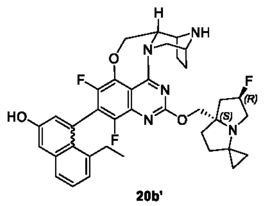
20b'

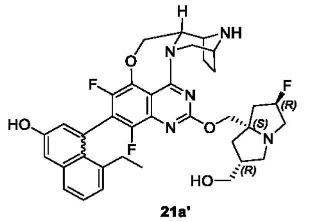
21a'

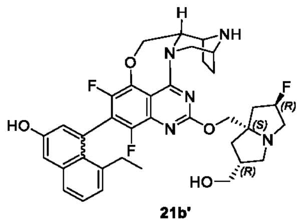
21b'

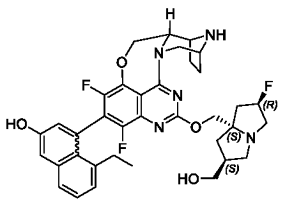
23a'

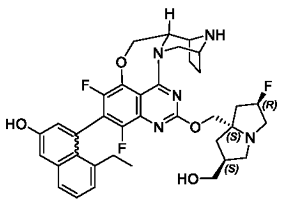
23b'

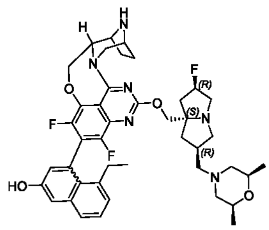
25a'

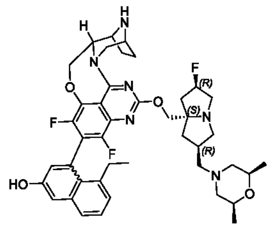
25b'

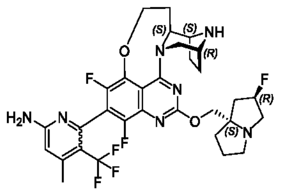
26a

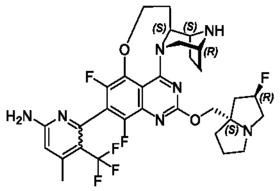
26b

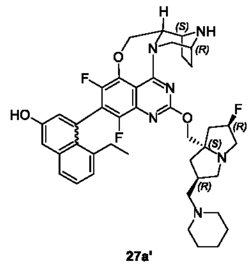
27a'

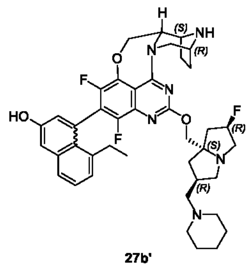
27b'

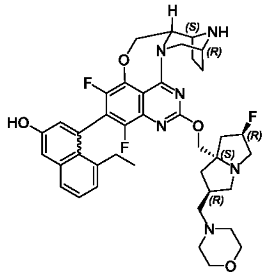
28a'

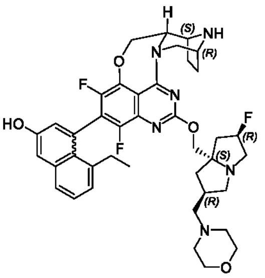
28b'

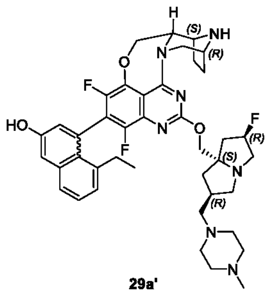
29a'

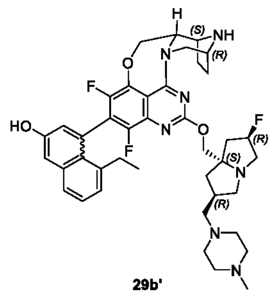
29b' ;

preferably:

a compound prepared by one stereoisomer of

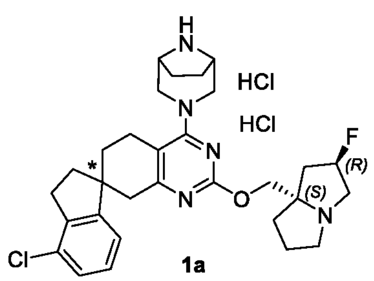
1a

with a retention time of 1.275 minutes under the following conditions: Optichiral C9-3, 3.0 × 100 mm, 3 μm; mobile phase A: supercritical carbon dioxide; mobile phase B: methanol (20 mmol/L ammonia); flow rate: 2 mL/min; isocratic elution with 50% phase B in 1.8 minutes; detector: UV 220 nm;

or, a compound prepared by one stereoisomer of

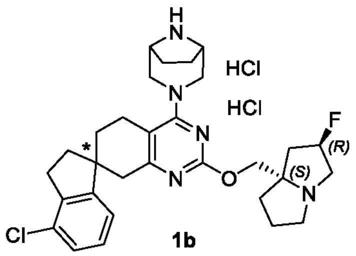

**1b**

with a retention time of 1.082 minutes under the following conditions: Optichiral C9-3, 3.0 × 100 mm, 3 μm; mobile phase A: supercritical carbon dioxide; mobile phase B: methanol (20 mmol/L ammonia); flow rate: 2 mL/min; isocratic elution with 50% phase B in 1.8 minutes; detector: UV 220 nm;

or, a compound prepared by one stereoisomer of

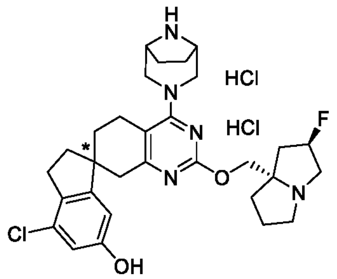

**2a**

with a retention time of 5.009 minutes under the following conditions: (*S*, *S*)-Whelk-O1, 4.6 × 100 mm, 3.5 μm; mobile phase A: supercritical carbon dioxide; mobile phase B: methanol (20 mmol/L ammonia); flow rate: 2 mL/min; isocratic elution with 40% phase B in 6.5 minutes; detector: UV 220 nm;

or, a compound prepared by one stereoisomer of

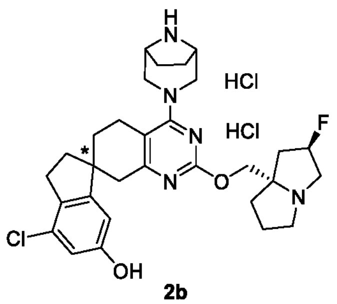

**2b**

with a retention time of 4.193 minutes under the following conditions: (*S*, *S*)-Whelk-O1, 4.6 × 100 mm, 3.5 μm; mobile phase A: supercritical carbon dioxide; mobile phase B: methanol (20 mmol/L ammonia); flow rate: 2 mL/min; isocratic elution with 40% phase B in 6.5 minutes; detector: UV 220 nm;

or, one stereoisomer of

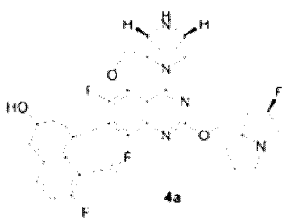

**4a**

with a retention time of 0.836 minutes under the following conditions: Column: Optichiral C9-3, 3 × 100 mm, 3 μm;

mobile phase A: supercritical carbon dioxide fluid; mobile phase B: methanol (20 mmol/L ammonia); flow rate: 2 mL/min; isocratic elution with 50% phase B in 2 minutes; detector: UV 230 nm;

or, one stereoisomer of

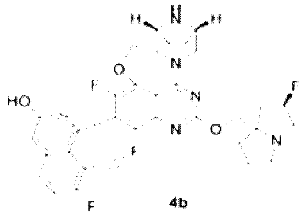

4b

with a retention time of 1.233 minutes under the following conditions: Column: Optichiral C9-3, 3 × 100 mm, 3 μm; mobile phase A: supercritical carbon dioxide fluid; mobile phase B: methanol (20 mmol/L ammonia); flow rate: 2 mL/min; isocratic elution with 50% phase B in 2 minutes; detector: UV 230 nm;

or, a compound prepared by one stereoisomer of

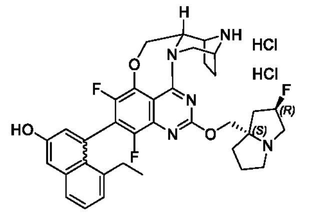

5a

with a retention time of 3.304 minutes under the following conditions: N-CHIRALPAK IC-3 (Lot No. IC3SCK-VK002), 3.0 × 100 mm, 3 μm; mobile phase A: supercritical carbon dioxide fluid; mobile phase B: methanol (20 mmol/L ammonia); flow rate: 2 mL/min; isocratic elution with 50% phase B in 12 minutes; detector: UV 220 nm;

or, a compound prepared by one stereoisomer of

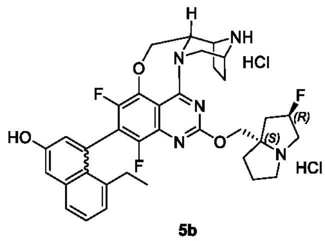

5b

with a retention time of 2.199 minutes under the following conditions: N-CHIRALPAK IC-3 (Lot No. IC3SCK-VK002), 3.0 × 100 mm, 3 μm; mobile phase A: supercritical carbon dioxide fluid; mobile phase B: methanol (20 mmol/L ammonia); flow rate: 2 mL/min; isocratic elution with 50% phase B in 12 minutes; detector: UV 220 nm;

or, a compound prepared by one stereoisomer of

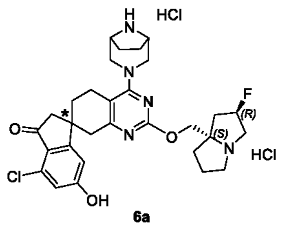

6a

with a retention time of 3.795 minutes under the following conditions: N-CHIRALPAK IC-3 (Lot No. IC30CS-VF008),

4.6 × 100 mm, 3 μm; mobile phase A: supercritical carbon dioxide fluid; mobile phase B: methanol (20 mmol/L ammonia); flow rate: 2 mL/min; isocratic elution with 50% phase B in 6 minutes; detector: UV 220 nm;

or, a compound prepared by one stereoisomer of

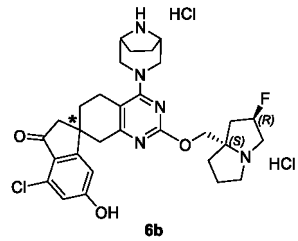

6b

with a retention time of 4.358 minutes under the following conditions: N-CHIRALPAK IC-3 (Lot No. IC30CS-VF008), 4.6 × 100 mm, 3 μm; mobile phase A: supercritical carbon dioxide fluid; mobile phase B: methanol (20 mmol/L ammonia); flow rate: 2 mL/min; isocratic elution with 50% phase B in 6 minutes; detector: UV 220 nm;

or, a compound prepared by one stereoisomer of

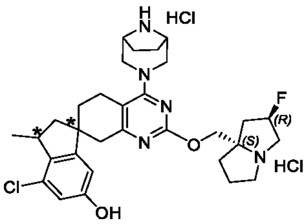

7aa

with a retention time of 1.964 minutes under the following conditions: CHIRALPAK IG-3, 4.6 × 50 mm, 3 μm; mobile phase A: *n*-hexane (0.1% ethylenediamine); mobile phase B: ethanol; flow rate: 1.67 mL/min; isocratic elution with 30% phase B in 7 minutes; detector: UV 290 nm;

or, a compound prepared by one stereoisomer of

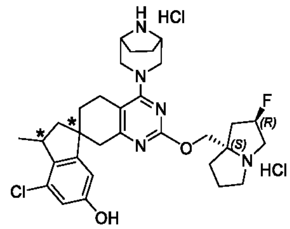

7ab

with a retention time of 4.664 minutes under the following conditions: CHIRALPAK IG-3, 4.6 × 50 mm, 3 μm; mobile phase A: *n*-hexane (0.1% ethylenediamine); mobile phase B: ethanol; flow rate: 1.67 mL/min; isocratic elution with 30% phase B in 7 minutes; detector: UV 290 nm;

or, a compound prepared by one stereoisomer of

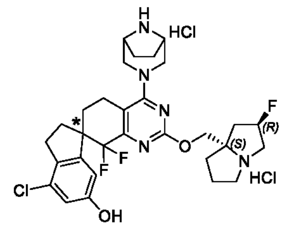

9a

with a retention time of 5.228 minutes under the following conditions: Lux Cellulose-2, 4.6 × 100 mm, 3 μm; mobile phase A: supercritical carbon dioxide fluid; mobile phase B: methanol (0.1% diethylamine); flow rate: 2 mL/min; isocratic elution with 50% phase B in 7.5 minutes; detector: UV 220 nm;

or, a compound prepared by one stereoisomer of

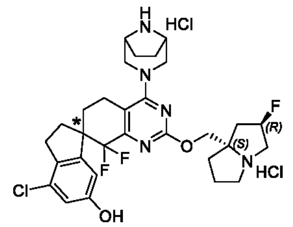

9b

with a retention time of 4.017 minutes under the following conditions: Lux Cellulose-2, 4.6 × 100 mm, 3 μm; mobile phase A: supercritical carbon dioxide fluid; mobile phase B: methanol (0.1% diethylamine); flow rate: 2 mL/min; isocratic elution with 50% phase B in 7.5 minutes; detector: UV 220 nm;

wherein "*" denotes a carbon atom in S configuration or a carbon atom in *R* configuration, and " 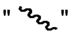 " denotes " 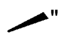 " or " 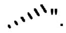 ".

In some embodiments, the pharmaceutically acceptable salt of the compound of formula I, formula II, formula III, or formula IV may be a hydrochloride salt of the compound of formula I, formula II, or formula III.

In some embodiments, the number of the pharmaceutically acceptable salts of the compound of formula I, formula II, formula III, or formula IV may be 1, 2, 3, 4, or 5.

In some embodiments, the pharmaceutically acceptable salt of the compound of formula I, formula II, formula III, or formula IV is any one of the following compounds:

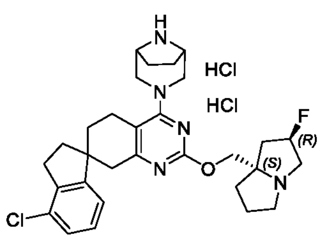

1"

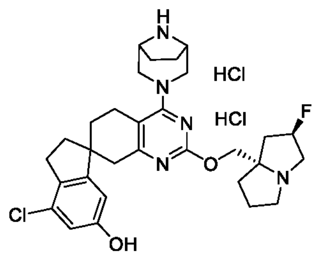

2"

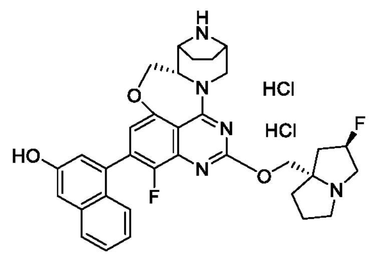

3

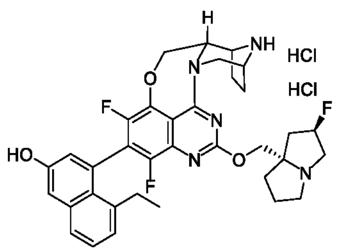

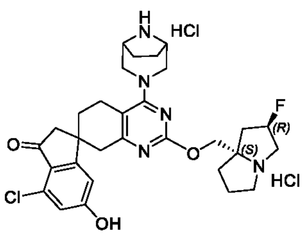

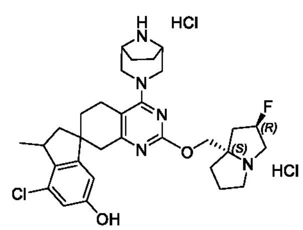

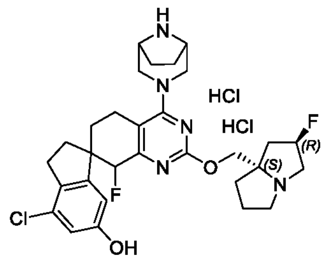

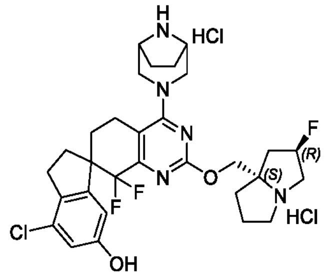

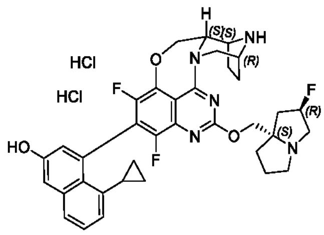

11''

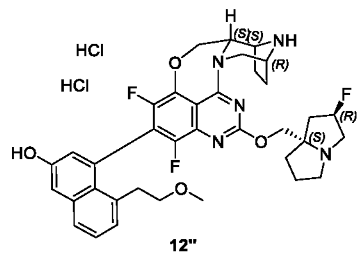

12''

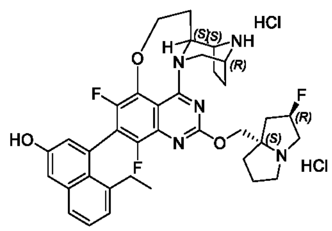

13''

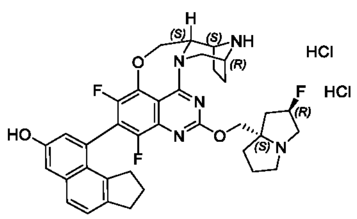

14''

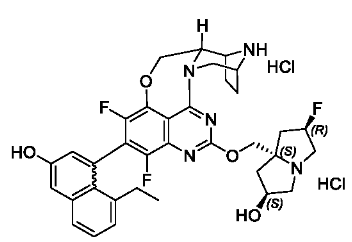

15''

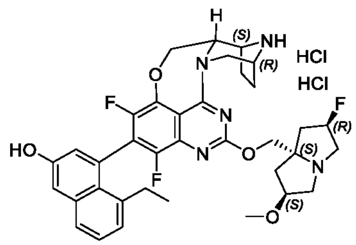

16''

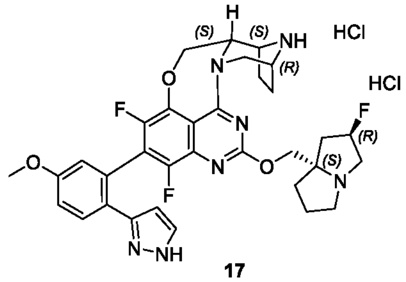

17

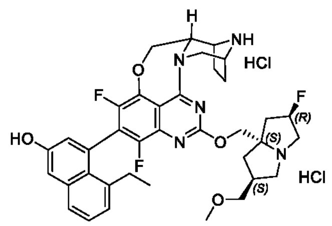

18''

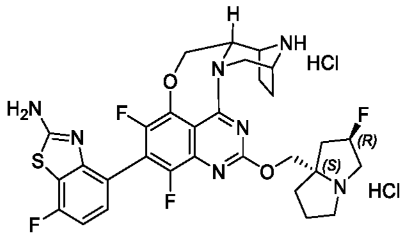

19

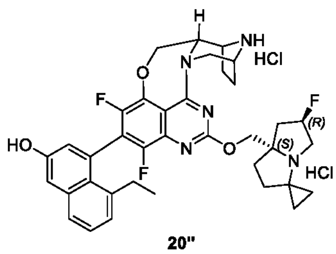

20''

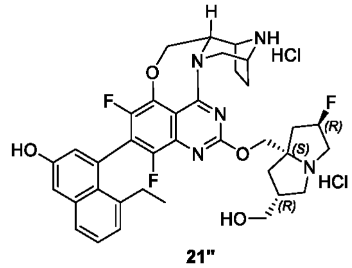

21''

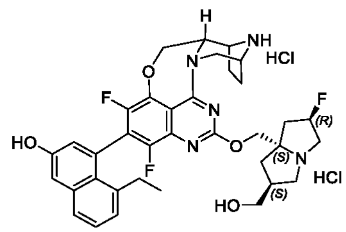

23''

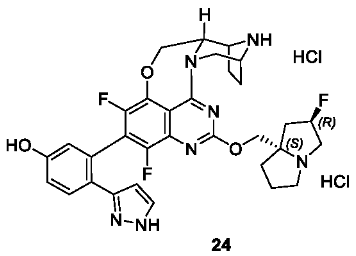
24

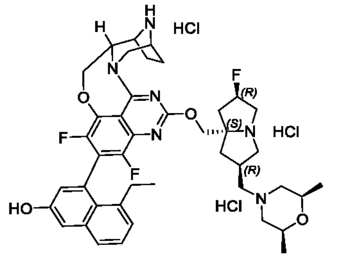
25''

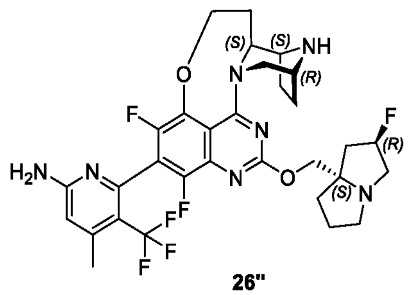
26''

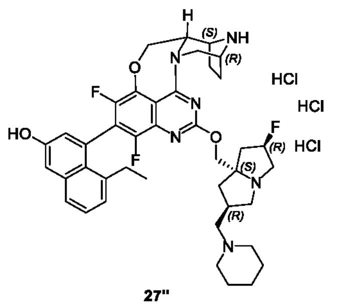
27''

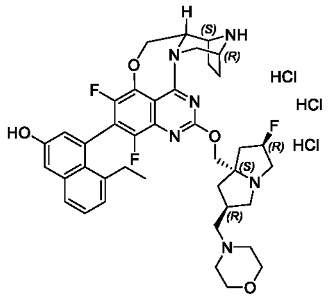
28''

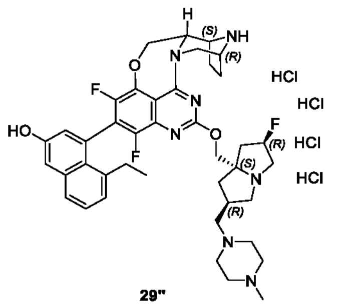
29''

In some embodiments, the pharmaceutically acceptable salt of the compound of formula I, formula II, formula III, or formula IV is any one of the following compounds:

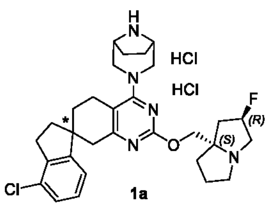
1a

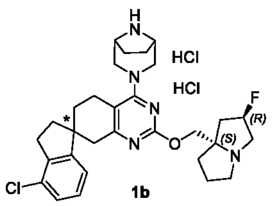
1b

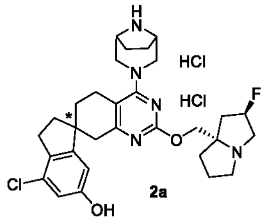
2a

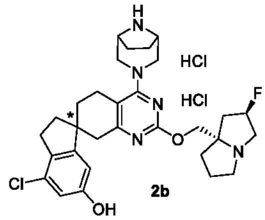
2b

;

preferably:

a compound with a retention time of 1.275 minutes under the following conditions, which is one stereoisomer of

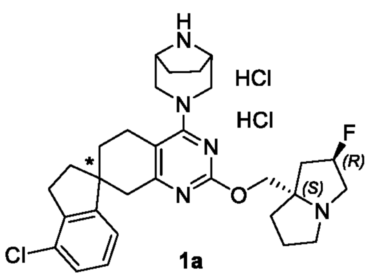
1a

Optichiral C9-3, 3.0 × 100 mm, 3 μm; mobile phase A: supercritical carbon dioxide; mobile phase B: methanol (20 mmol/L ammonia); flow rate: 2 mL/min; isocratic elution with 50% phase B in 1.8 minutes; detector: UV 220 nm;

or, a compound with a retention time of 1.082 minutes under the following conditions, which is one stereoisomer of

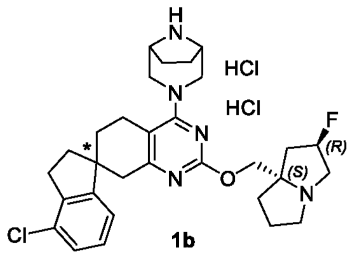

**1b**

Optichiral C9-3, 3.0 × 100 mm, 3 μm; mobile phase A: supercritical carbon dioxide; mobile phase B: methanol (20 mmol/L ammonia); flow rate: 2 mL/min; isocratic elution with 50% phase B in 1.8 minutes; detector: UV 220 nm;

or, a compound with a retention time of 5.009 minutes under the following conditions, which is one stereoisomer of

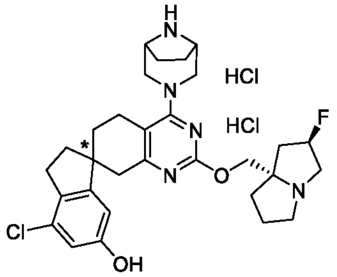

**2a**

: (*S*, *S*)-Whelk-O1, 4.6 × 100 mm, 3.5 μm; mobile phase A: supercritical carbon dioxide; mobile phase B: methanol (20 mmol/L ammonia); flow rate: 2 mL/min; isocratic elution with 40% phase B in 6.5 minutes; detector: UV 220 nm;

or, a compound with a retention time of 4.193 minutes under the following conditions, which is one stereoisomer of

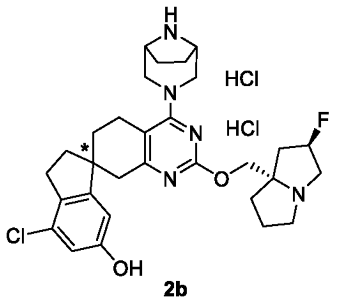

**2b**

(*S*, *S*)-Whelk-O1, 4.6 × 100 mm, 3.5 μm; mobile phase A: supercritical carbon dioxide; mobile phase B: methanol (20 mmol/L ammonia); flow rate: 2 mL/min; isocratic elution with 40% phase B in 6.5 minutes; detector: UV 220 nm;

or, one stereoisomer of

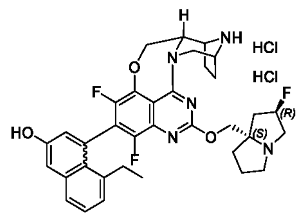

**5a**

with a retention time of 3.304 minutes under the following conditions: N-CHIRALPAK IC-3 (Lot No. IC3SCK-VK002), 3.0 × 100 mm, 3 μm; mobile phase A: supercritical carbon dioxide fluid; mobile phase B: methanol (20 mmol/L ammonia); flow rate: 2 mL/min; isocratic elution with 50% phase B in 12 minutes; detector: UV 220 nm;

or, one stereoisomer of

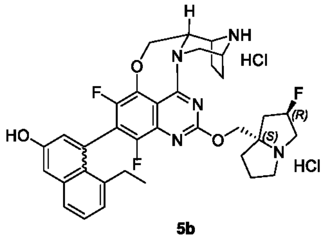

5b

with a retention time of 2.199 minutes under the following conditions: N-CHIRALPAK IC-3 (Lot No. IC3SCK-VK002), 3.0 × 100 mm, 3 μm; mobile phase A: supercritical carbon dioxide fluid; mobile phase B: methanol (20 mmol/L ammonia); flow rate: 2 mL/min; isocratic elution with 50% phase B in 12 minutes; detector: UV 220 nm;

or, one stereoisomer of

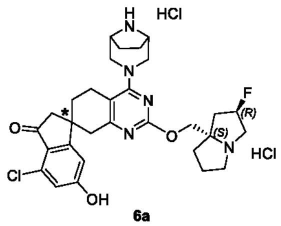

6a

with a retention time of 3.795 minutes under the following conditions: N-CHIRALPAK IC-3 (Lot No. IC30CS-VF008), 4.6 × 100 mm, 3 μm; mobile phase A: supercritical carbon dioxide fluid; mobile phase B: methanol (20 mmol/L ammonia); flow rate: 2 mL/min; isocratic elution with 50% phase B in 6 minutes; detector: UV 220 nm;

or, one stereoisomer of

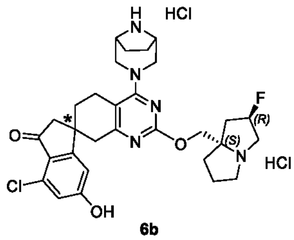

6b

with a retention time of 4.358 minutes under the following conditions: N-CHIRALPAK IC-3 (Lot No. IC30CS-VF008), 4.6 × 100 mm, 3 μm; mobile phase A: supercritical carbon dioxide fluid; mobile phase B: methanol (20 mmol/L ammonia); flow rate: 2 mL/min; isocratic elution with 50% phase B in 6 minutes; detector: UV 220 nm;

or, one stereoisomer of

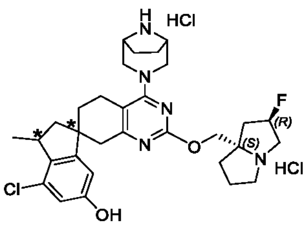

7aa

with a retention time of 1.964 minutes under the following conditions: CHIRALPAK IG-3, 4.6 × 50 mm, 3 μm; mobile phase A: n-hexane (0.1% ethylenediamine); mobile phase B: ethanol; flow rate: 1.67 mL/min; isocratic elution with 30% phase B in 7 minutes; detector: UV 290 nm;

or, one stereoisomer of

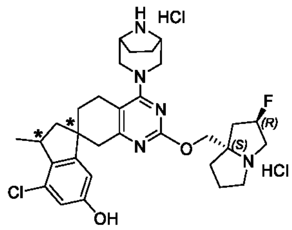

7ab

with a retention time of 4.664 minutes under the following conditions: CHIRALPAK IG-3, 4.6 × 50 mm, 3 μm; mobile phase A: *n*-hexane (0.1% ethylenediamine); mobile phase B: ethanol; flow rate: 1.67 mL/min; isocratic elution with 30% phase B in 7 minutes; detector: UV 290 nm;

or, one stereoisomer of

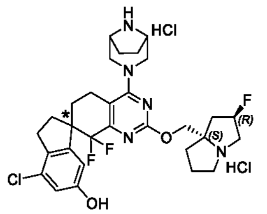

9a

with a retention time of 5.228 minutes under the following conditions: Lux Cellulose-2, 4.6 × 100 mm, 3 μm; mobile phase A: supercritical carbon dioxide fluid; mobile phase B: methanol (0.1% diethylamine); flow rate: 2 mL/min; isocratic elution with 50% phase B in 7.5 minutes; detector: UV 220 nm;

or, one stereoisomer of

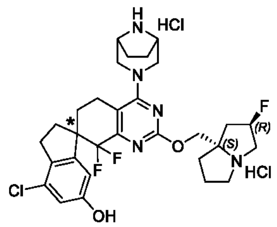

9b

with a retention time of 4.017 minutes under the following conditions: Lux Cellulose-2, 4.6 × 100 mm, 3 μm; mobile phase A: supercritical carbon dioxide fluid; mobile phase B: methanol (0.1% diethylamine); flow rate: 2 mL/min; isocratic elution with 50% phase B in 7.5 minutes; detector: UV 220 nm;

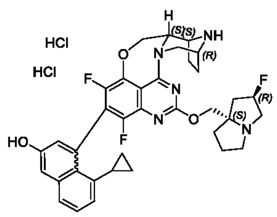

11a

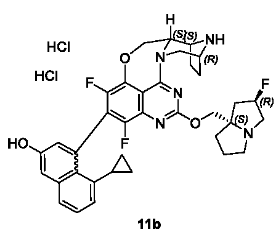

11b

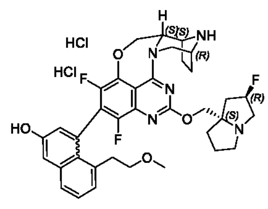

12a

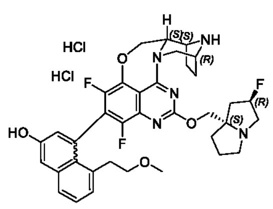

12b

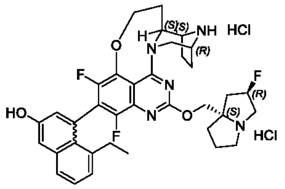

13a

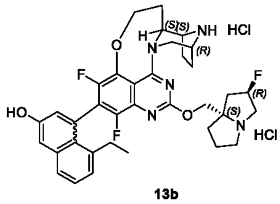

13b

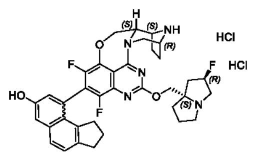

14a

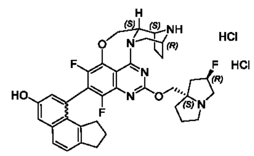

14b

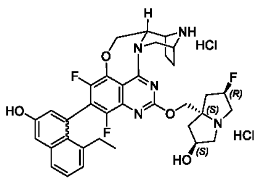

15a

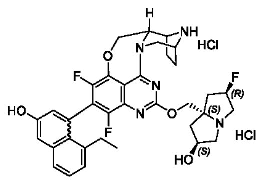

15b

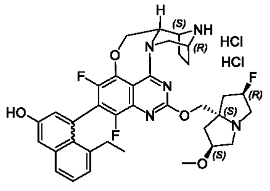

16a

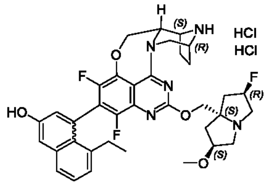

16b

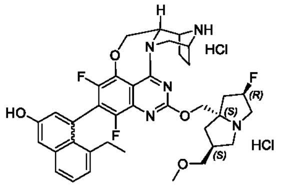

18a

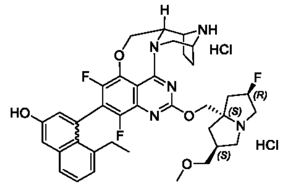

18b

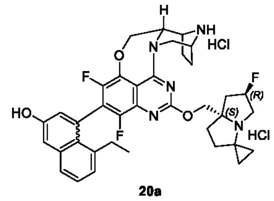

20a

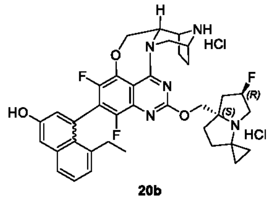

20b

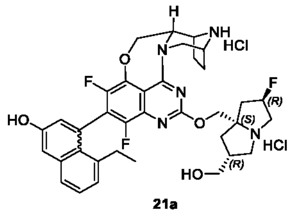

21a

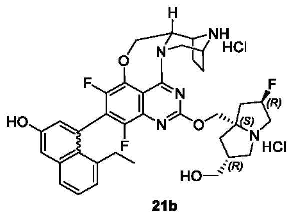

21b

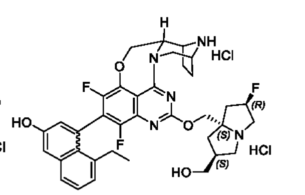

23a

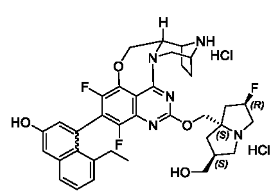

23b

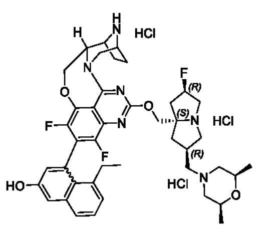

25a

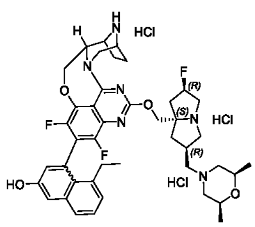

25b

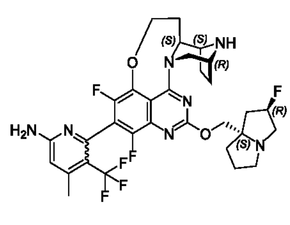

26a

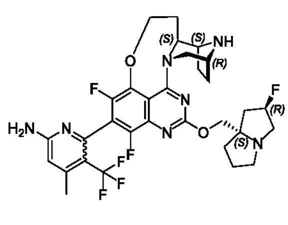

26b

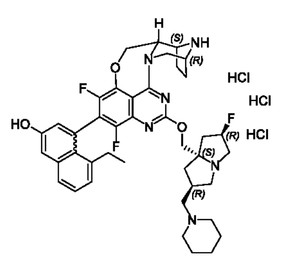

27a

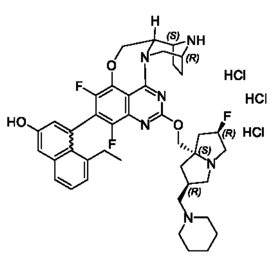

27b

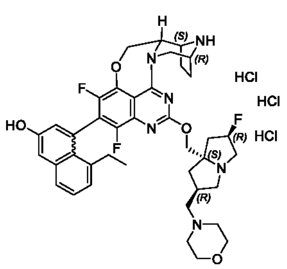

28a

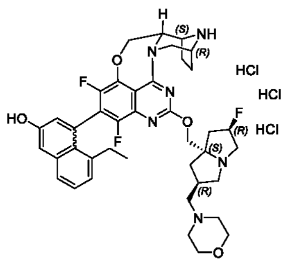

28b

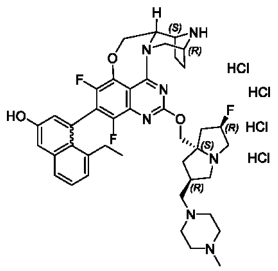

29a

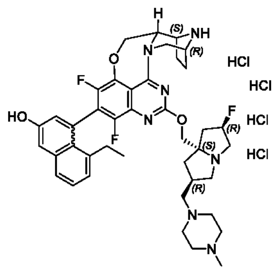

29b ;

wherein "*" denotes a carbon atom in S configuration or a carbon atom in *R* configuration, and " 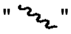 " denotes " 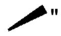 " or " 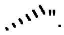 ".

The present disclosure further provides compounds shown below or pharmaceutically acceptable salts thereof:

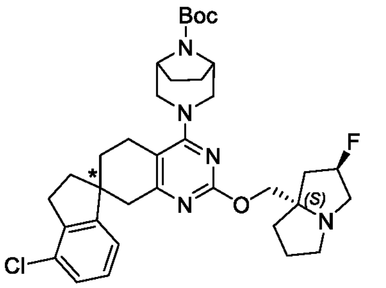

1-10a ,

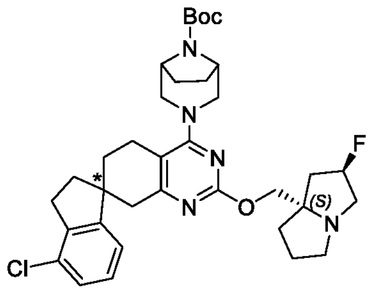

1-10b ,

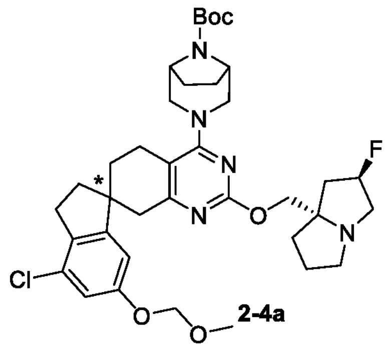

2-4a ,

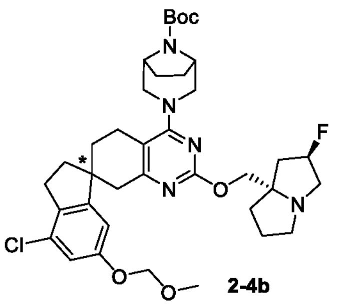

2-4b ,

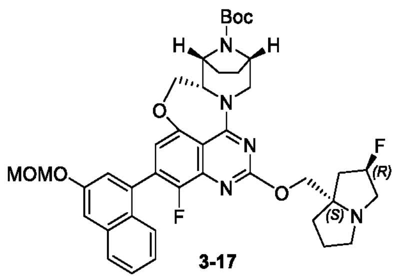

3-17 ,

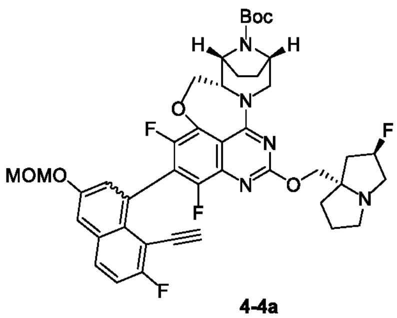

4-4a ,

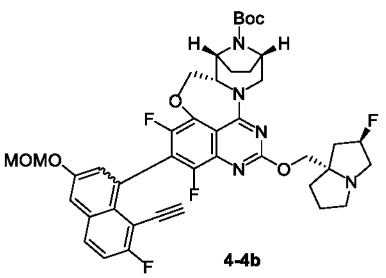

4-4b ,

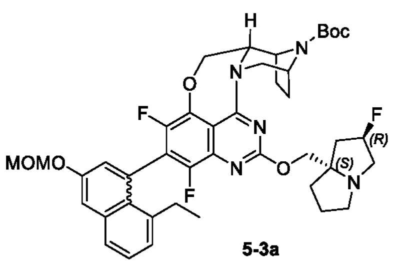

5-3a ,

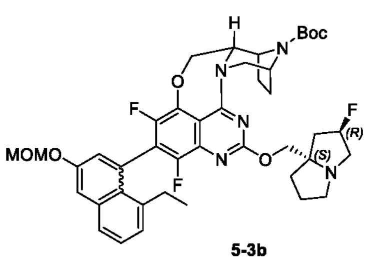

5-3b ,

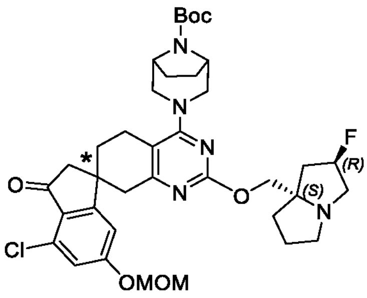
6-2a ,

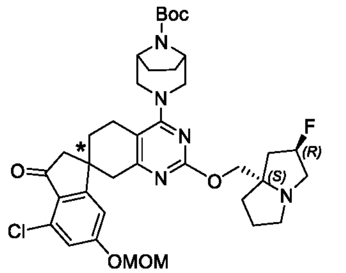
6-2b ,

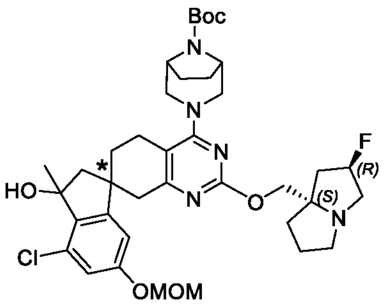
7-1a ,

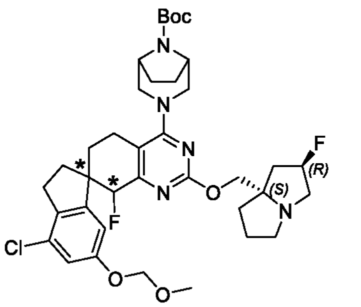
8-5a ,

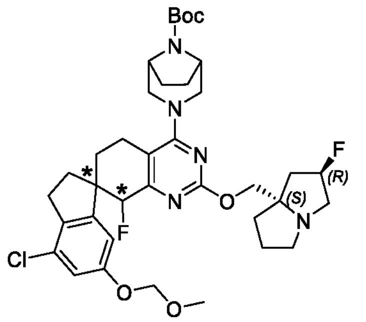
8-5b ,

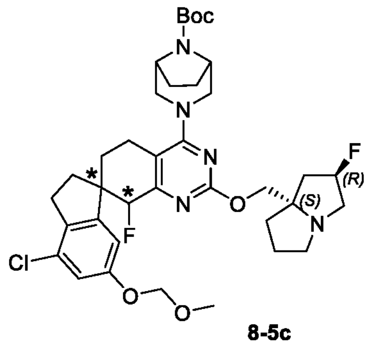
8-5c ,

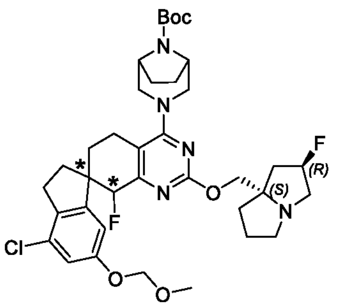
8-5d ,

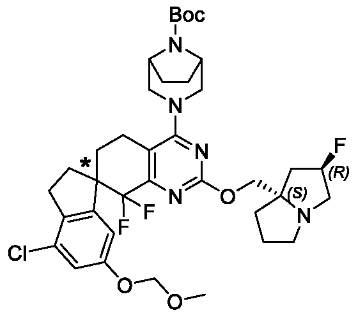
9-5a ,

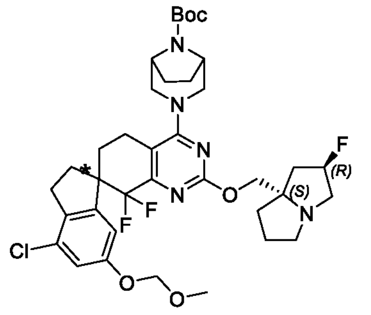
9-5b ,

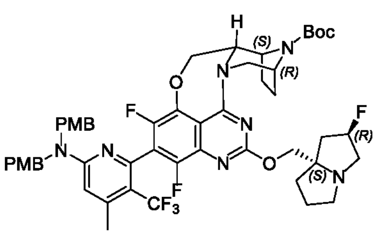
10-3 ,

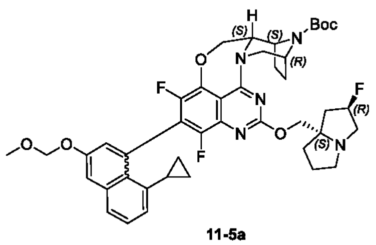
11-5a ,

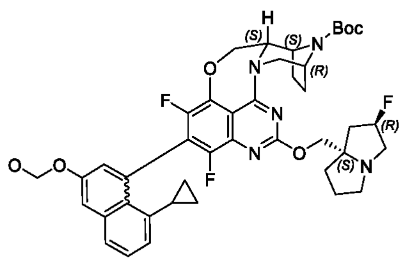
11-5b ,

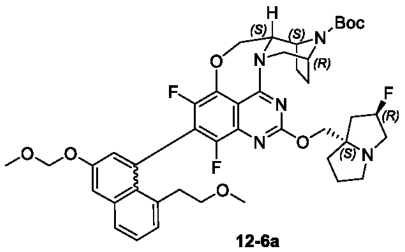
12-6a ,

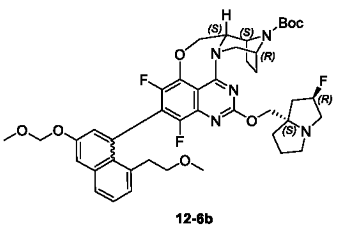
12-6b ,

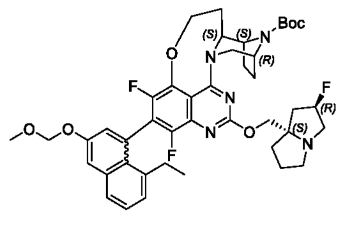
13-17a ,

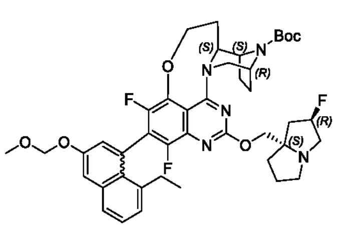

**13-17b** ,

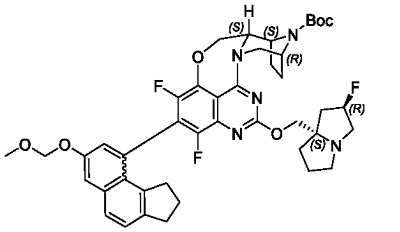

**14-11a** ,

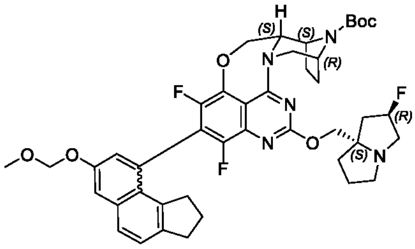

**14-11b** ,

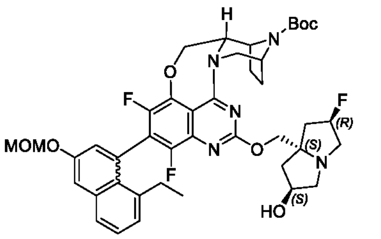

**15-9a** ,

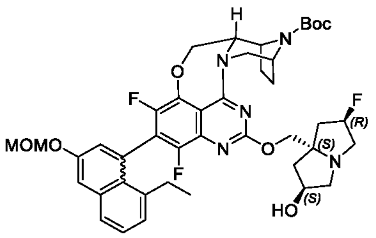

**15-9b** ,

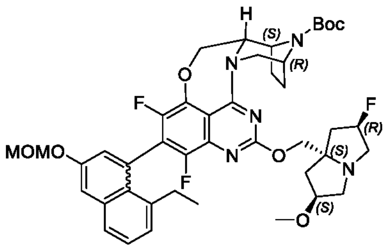

**16-4a** ,

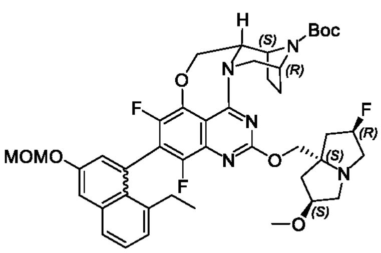

**16-4b** ,

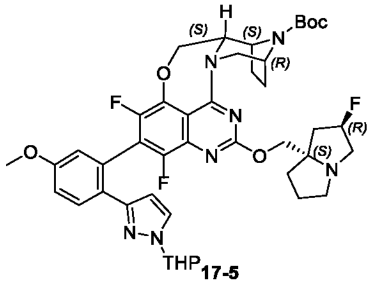

**17-5** ,

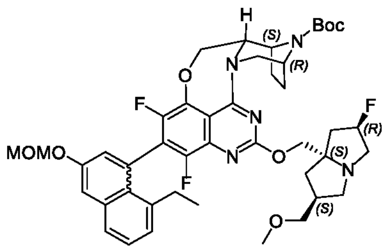

**18-4a** ,

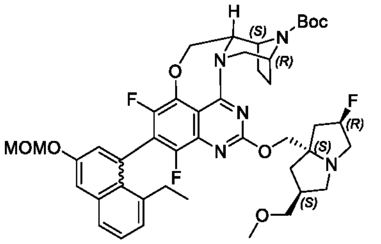

**18-4b** ,

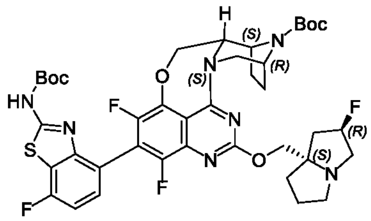

**19-1**

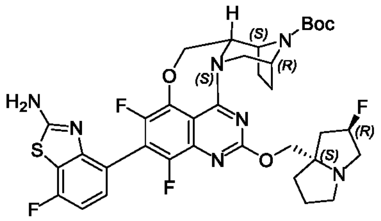

**19-2** ,

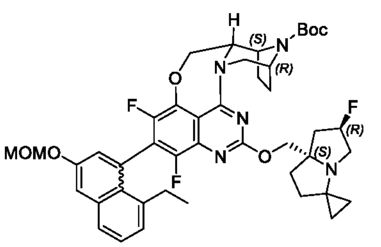

**20-2a** ,

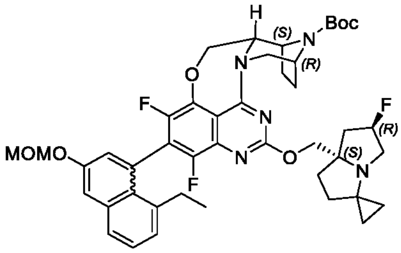

**20-2b** ,

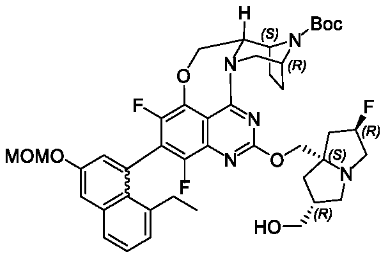

**21-4a** ,

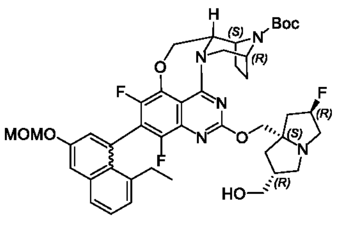

21-4b ,

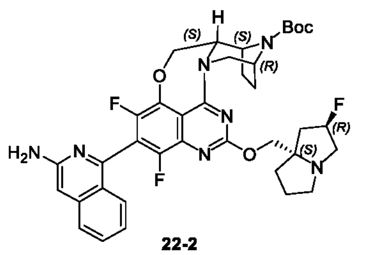

22-2 ,

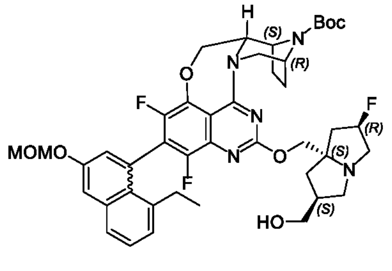

23-4a ,

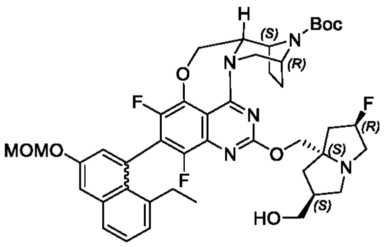

23-4b ,

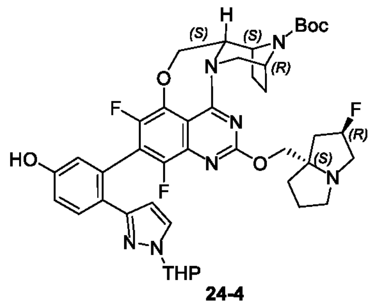

24-4 ,

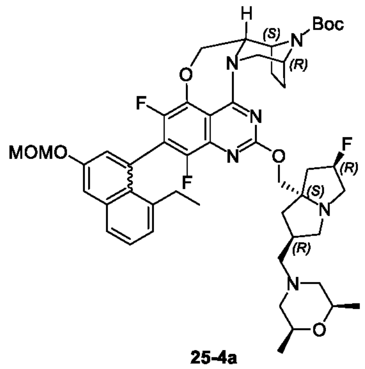

25-4a ,

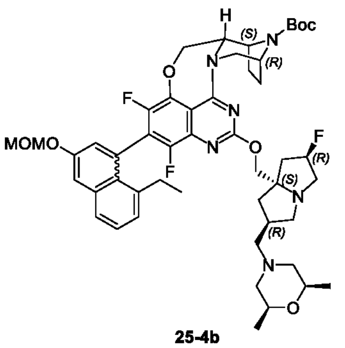

25-4b ,

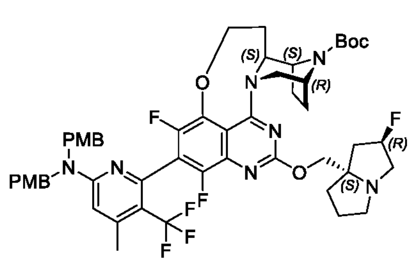

26-1 ,

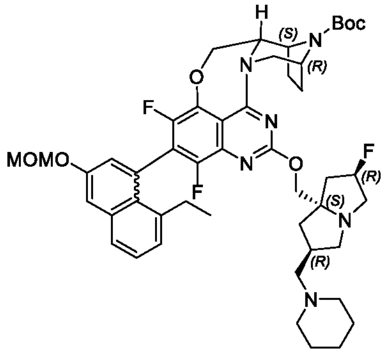

27-1a ,

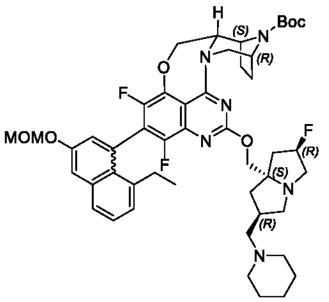

27-1b ,

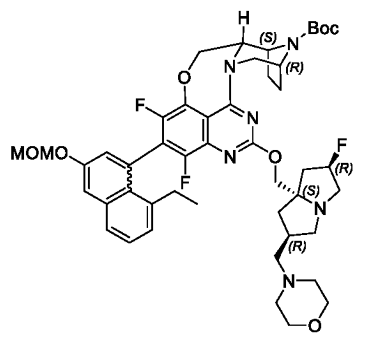

28-1a ,

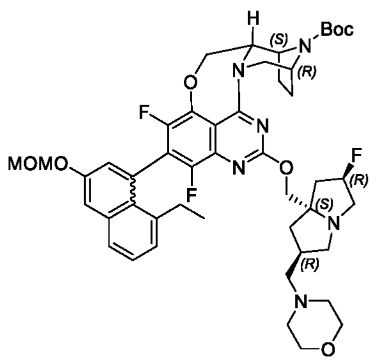

28-1b ,

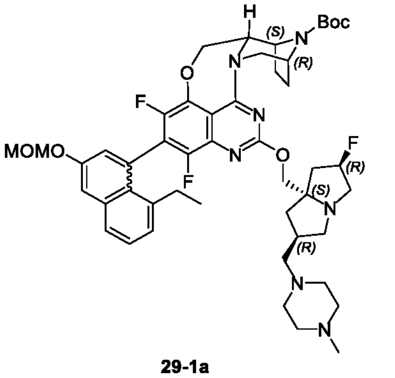

29-1a ,

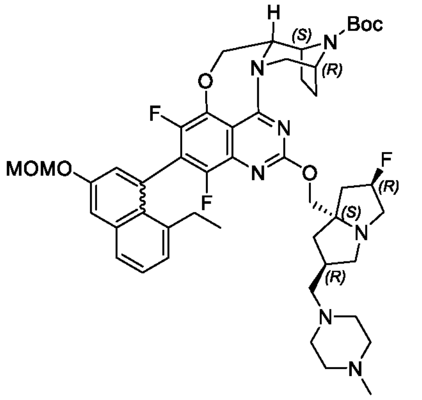

29-1b ,

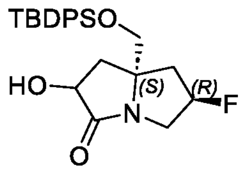

15-3 ,

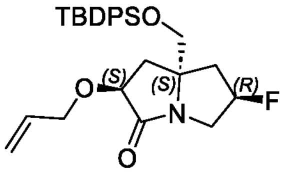

15-4a ,

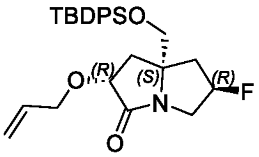

15-4b ,

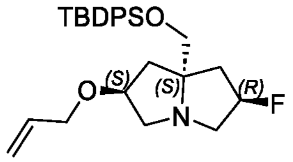

15-5 ,

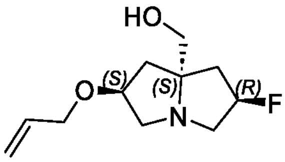

15-6 ,

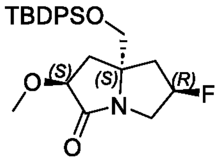

16-1 ,

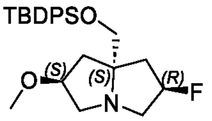

16-2 ,

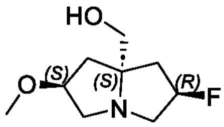

16-3 ,

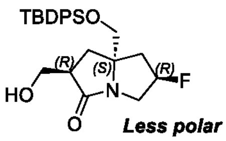

*Less polar*

18-1a ,

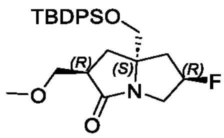

18-2 ,

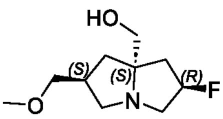

18-3 ,

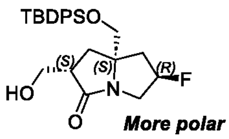

*More polar*

18-1b ,

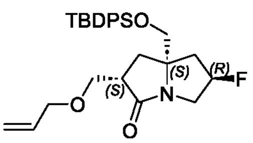

21-1 ,

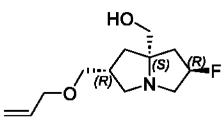

21-2 ,

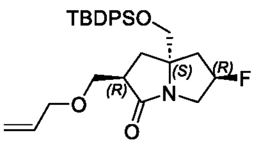

23-1 ,

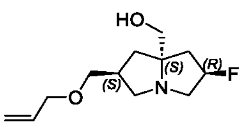

23-2 ,

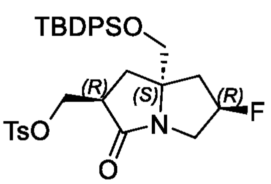

25-1 ,

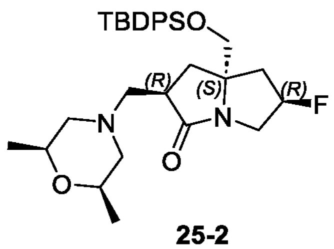

25-2 ,

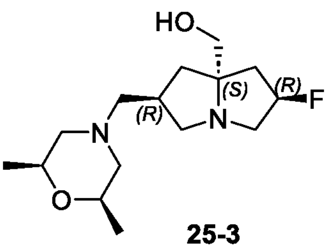

25-3 ,

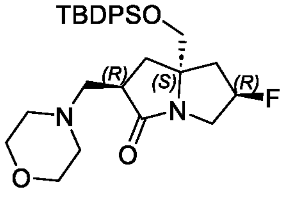
**28-2**
,

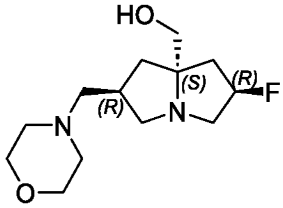
**28-3**
,

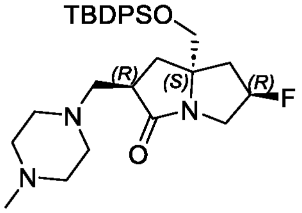
**29-2**
,

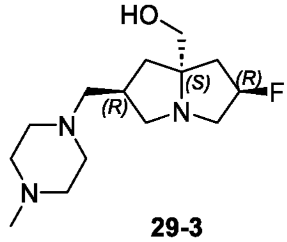
**29-3**
,

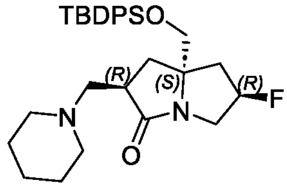
**27-2**
,

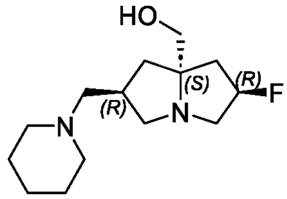
**27-3**
,

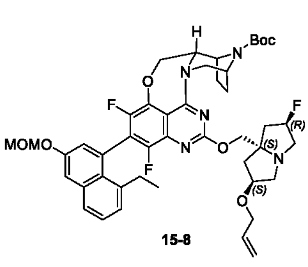
**15-8**
,

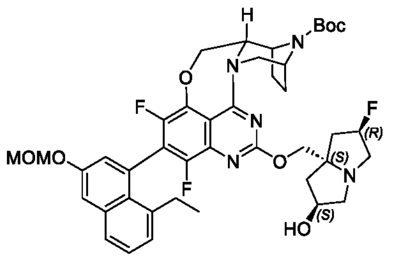
**15-9**
,

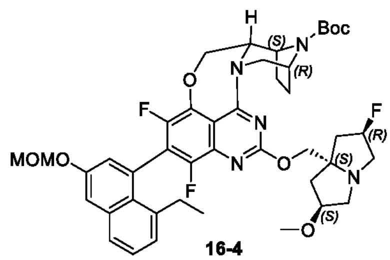
**16-4**
,

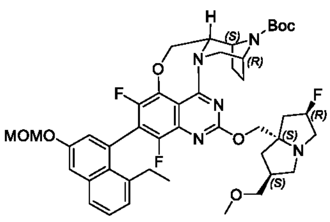
**18-4**
,

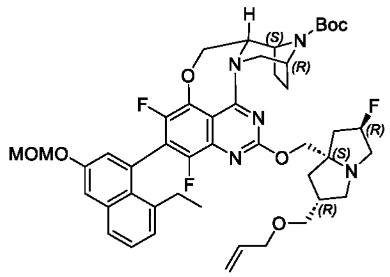
**21-3**
,

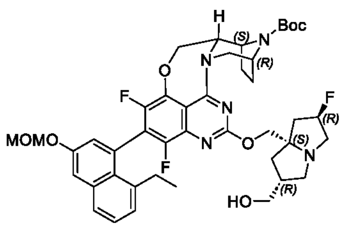
**21-4**
,

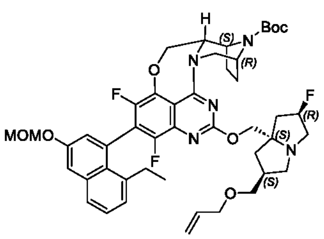
**23-3**
,

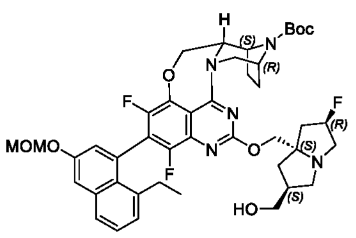
**23-4**
,

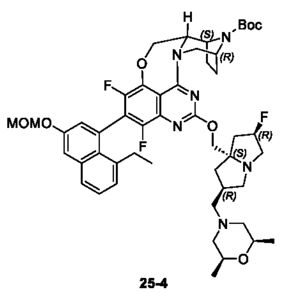
**25-4**
,

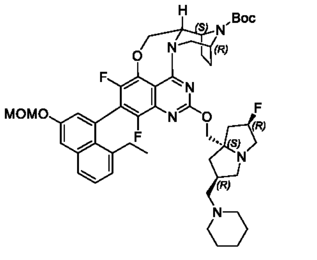

27-1 ,

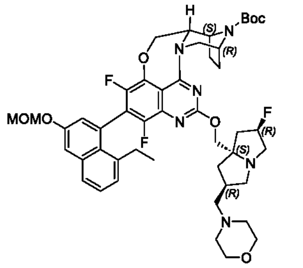

28-1 ,

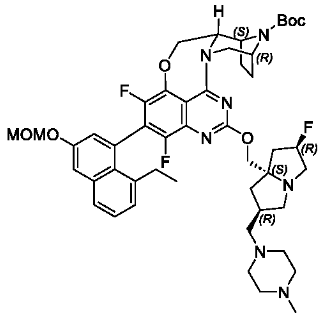

29-1 ,

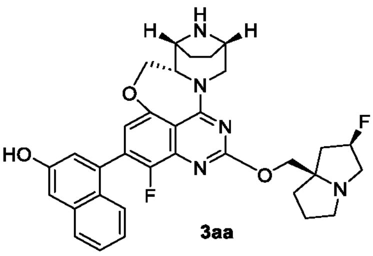

3aa ,

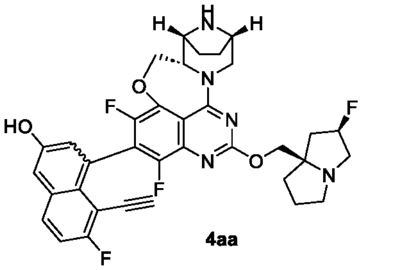

4aa

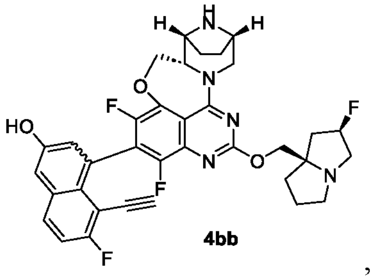

4bb ,

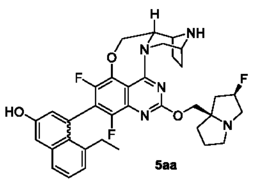

5aa

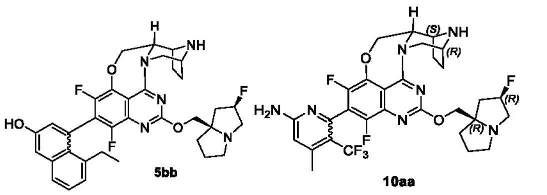

5bb

10aa

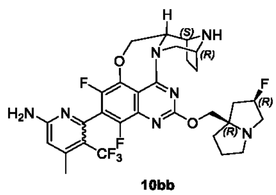

10bb

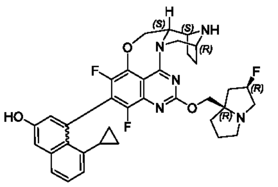

11aa

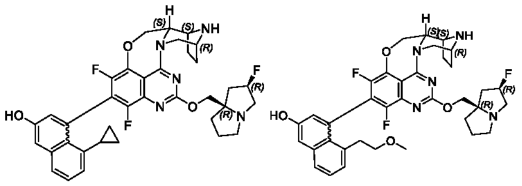

11bb

12aa

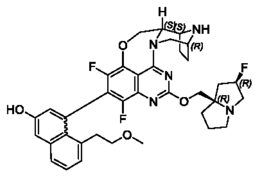

12bb

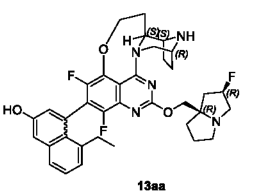

13aa

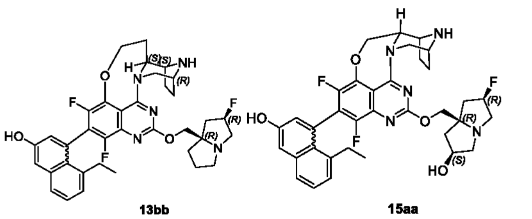

13bb

15aa

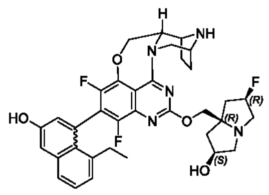

15bb

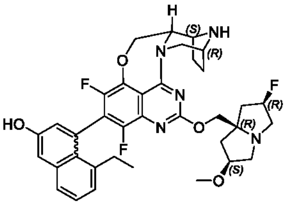

16aa

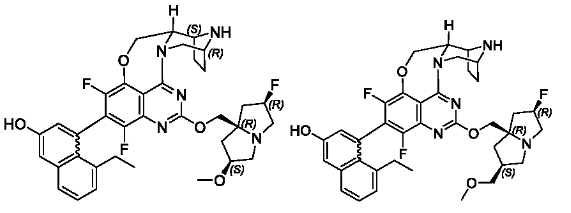

16bb

18aa

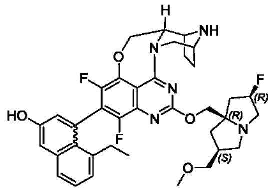

18bb

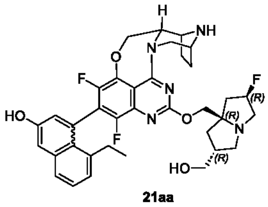
21aa
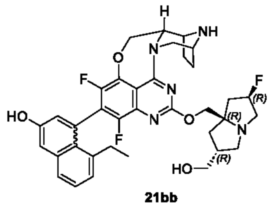
21bb
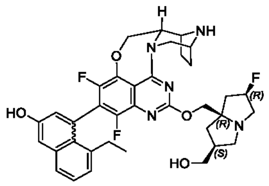
23aa
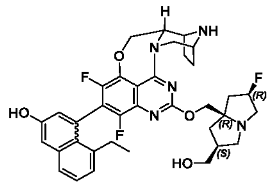
23bb

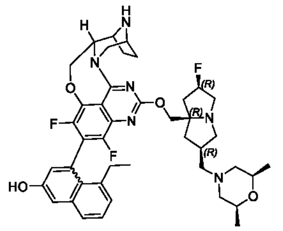
25aa
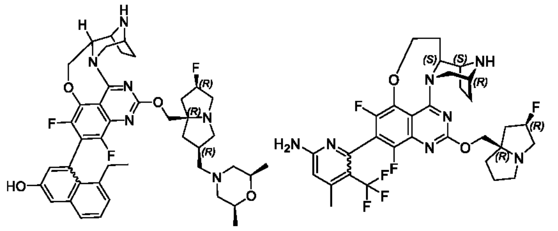
25bb
26aa
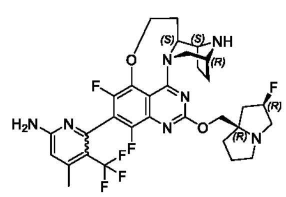
26bb

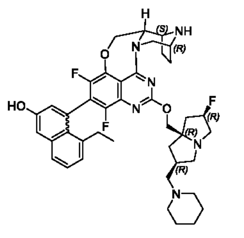
27aa
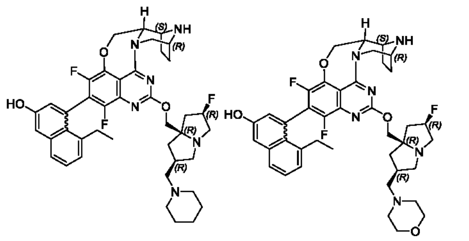
27bb
28aa
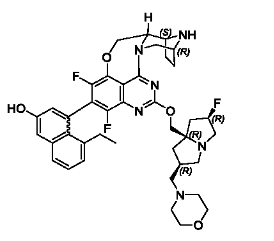
28bb

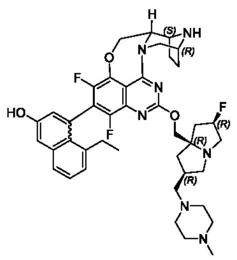
29aa
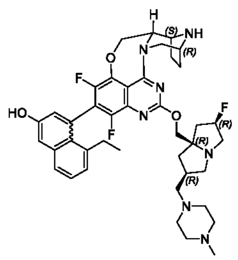
29bb
,

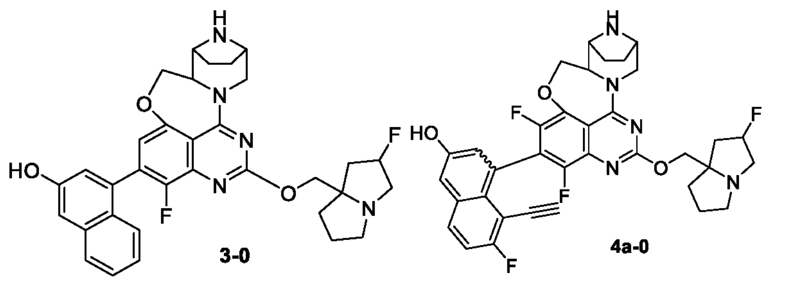
3-0
4a-0
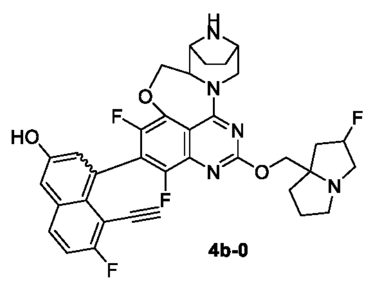
4b-0

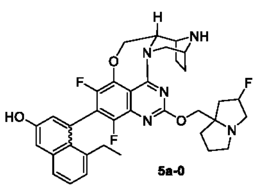
5a-0
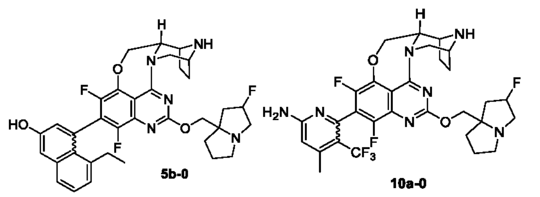
5b-0
10a-0
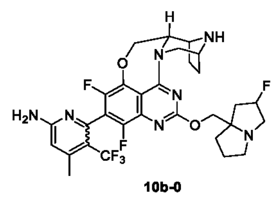
10b-0

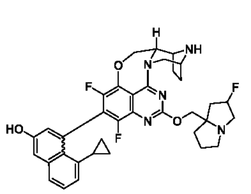
11a-0

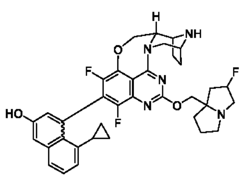
11b-0

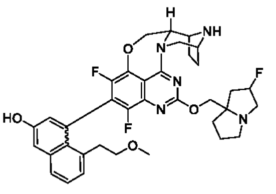
12a-0

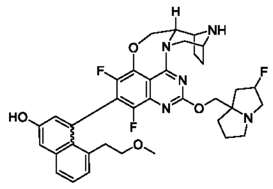
12b-0

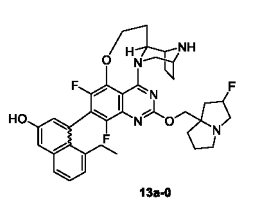
13a-0

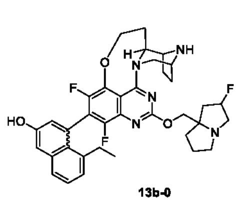
13b-0

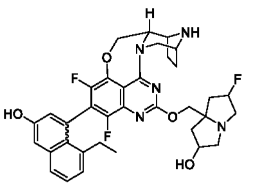
15a-0

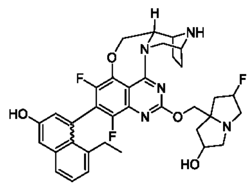
15b-0

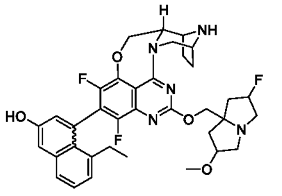
16a-0

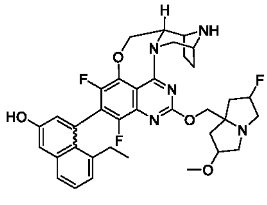
16b-0

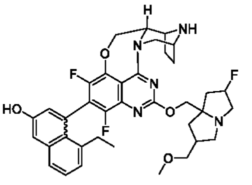
18a-0

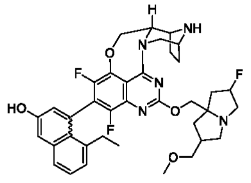
18b-0

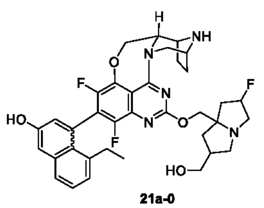
21a-0

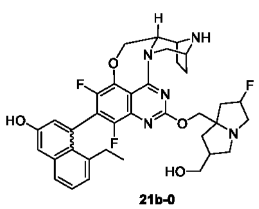
21b-0

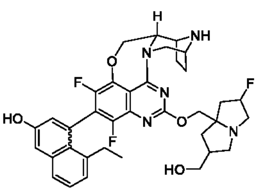
23a-0

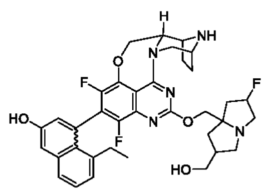
23b-0

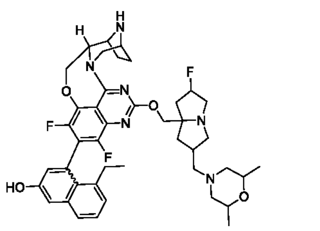
25a-0

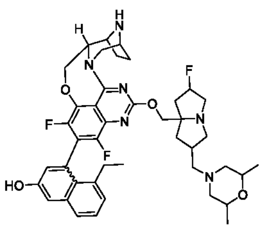
25b-0

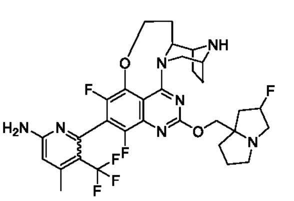
26a-0

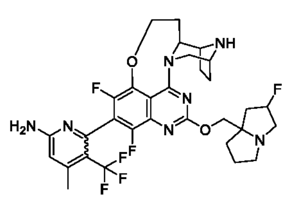
26b-0

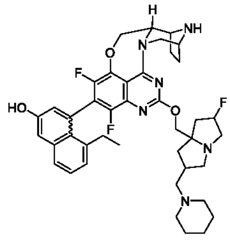
27a-0

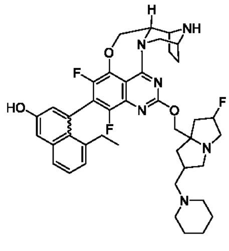
27b-0

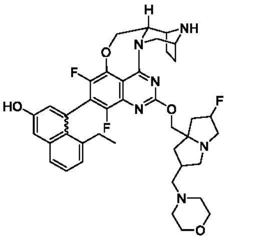
28a-0

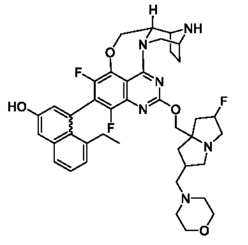
28b-0

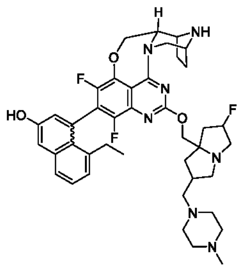

29a-0

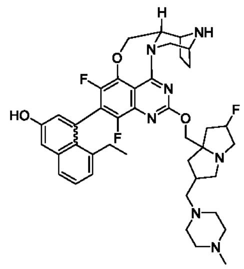

29b-0

wherein "*" denotes a carbon atom in *S* configuration or a carbon atom in *R* configuration, and " 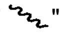 " denotes " 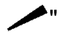 " or " 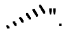 ".

The present disclosure provides a pharmaceutical composition comprising the above compound of formula I, formula II, formula III, or formula IV, the pharmaceutically acceptable salt thereof, the solvate thereof, the stereoisomer thereof, the tautomer thereof, the prodrug thereof, the metabolite thereof, or the isotopic compound thereof, and a pharmaceutical excipient.

In the pharmaceutical composition, the compound of formula I, formula II, formula III, or formula IV, the pharmaceutically acceptable salt thereof, the solvate thereof, the stereoisomer thereof, the tautomer thereof, the prodrug thereof, the metabolite thereof, or the isotopic compound thereof may be present in a therapeutically effective amount.

The present disclosure further provides a use of the above compound of formula I, formula II, formula III, or formula IV, the pharmaceutically acceptable salt thereof, the solvate thereof, the stereoisomer thereof, the tautomer thereof, the prodrug thereof, the metabolite thereof, or the isotopic compound thereof, or the above pharmaceutical composition in the manufacture of an inhibitor of KRAS mutant protein.

In the use, the KRAS mutant protein may be KRAS G12D mutant protein; the KRAS mutant protein inhibitor is used *in vitro,* mainly for experimental purposes, such as serving as a standard sample or a control sample to provide comparison, or making a kit according to conventional methods in the art, to offer rapid detection of effect of the KRAS G12D mutant protein inhibitor.

The present disclosure further provides a use of the above compound of formula I, formula II, formula III, or formula IV, the pharmaceutically acceptable salt thereof, the solvate thereof, the stereoisomer thereof, the tautomer thereof, the prodrug thereof, the metabolite thereof, or the isotopic compound thereof, or the above pharmaceutical composition in the manufacture of a medicament, and the medicament is used for the prevention or treatment of a cancer mediated by KRAS mutation.

The KRAS mutant protein is preferably KRAS G12D mutant protein.

The cancer mediated by the KRAS mutant protein may be blood cancer, pancreatic cancer, MYH-associated polyposis, colorectal cancer, or lung cancer, etc.

The present disclosure further provides a use of the above compound of formula I, formula II, formula III, or formula IV, the pharmaceutically acceptable salt thereof, the solvate thereof, the stereoisomer thereof, the tautomer thereof, the prodrug thereof, the metabolite thereof, or the isotopic compound thereof, or the above pharmaceutical composition in the manufacture of a medicament, and the medicament is used for the prevention or treatment of cancer.

The cancer is, for example, blood cancer, pancreatic cancer, MYH-associated polyposis, colorectal cancer, or lung cancer, etc.

The cancer is a cancer mediated by KRAS mutation. The KRAS mutant protein may be KRAS G12D mutant protein.

The present disclosure further provides a method for therapeutically preventing or treating a cancer mediated by KRAS mutation, which comprises administering to a patient a therapeutically effective amount of the above compound of formula I, the pharmaceutically acceptable salt thereof, the solvate thereof, the stereoisomer thereof, the tautomer thereof, the prodrug thereof, the metabolite thereof, or the isotopic compound thereof, or the above pharmaceutical composition.

The cancer is, for example, blood cancer, pancreatic cancer, MYH-associated polyposis, colorectal cancer, or lung cancer, etc.

The KRAS mutant protein may be KRAS G12D mutant protein.

The present disclosure further provides a method for therapeutically preventing or treating a cancer, which comprises administering to a patient a therapeutically effective amount of the above compound of formula I, formula II, formula III, or formula IV, the pharmaceutically acceptable salt thereof, the solvate thereof, the stereoisomer thereof, the tautomer thereof, the prodrug thereof, the metabolite thereof, or the isotopic compound thereof, or the above pharmaceutical composition.

The cancer is, for example, blood cancer, pancreatic cancer, MYH-associated polyposis, colorectal cancer, or lung cancer, etc.

The present disclosure further relates to a method for treating a hyperproliferative disease in a mammal,

comprising administering to the mammal a therapeutically effective amount of the compound of the present disclosure or a pharmaceutically acceptable salt, ester, prodrug, solvate, hydrate, or derivative thereof.

Ras mutations, include, but are not limited to, Ras mutations of K-Ras, H-Ras, or N-Ras mutations that have been identified in hematological cancers or malignancies (such as cancers affecting the blood, bone marrow, and/or lymph nodes). Accordingly, certain embodiments involve administering the disclosed compounds (e.g., in the form of a pharmaceutical composition) to a patient in need of treatment of a hematological cancer or malignancy.

In certain specific embodiments, the present disclosure relates to a method for treating lung cancer, comprising administering an effective amount of any of the above compounds (or pharmaceutical compositions comprising the compounds) to a subject in need thereof.

In the present disclosure, the cancer or malignancy includes, but is not limited to, leukemia and lymphoma. In certain embodiments, the leukemia is also, for example, acute lymphoblastic leukemia (ALL), acute myelogenous leukemia (AML), chronic lymphocytic leukemia (CLL), small lymphocytic lymphoma (SLL), chronic myelogenous leukemia (CML), acute monocytic leukemia (AMoL), and/or other leukemias. In certain embodiments, the lymphoma is, for example, all subtypes of Hodgkin lymphoma or non-Hodgkin lymphoma.

In certain embodiments of the present disclosure, the lung cancer is non-small cell lung cancer (NSCLC), for example, adenocarcinoma, squamous cell lung cancer, or large cell lung cancer. In other embodiments, the lung cancer is small cell lung cancer. Other lung cancers include, but are not limited to, adenomas, carcinoids, and anaplastic carcinomas.

In some embodiments of the present disclosure, the cancer is, for example, acute myeloid leukemia, adolescent cancer, childhood adrenocortical carcinoma, AIDS-related cancer (such as lymphoma and Kaposi's sarcoma), anal cancer, appendiceal cancer, astrocytoma, atypical teratoid, basal cell carcinoma, cholangiocarcinoma, bladder cancer, bone cancer, brainstem glioma, brain tumor, breast cancer, bronchial tumor, Burkitt lymphoma, carcinoid, atypical teratoid, embryonal tumor, germ cell tumor, primary lymphoma, cervical cancer, childhood cancer, chordoma, cardiac tumor, chronic lymphocytic leukemia (CLL), chronic myelogenous leukemia (CML), chronic myeloproliferative disorder, colon cancer, colorectal cancer, craniopharyngioma, cutaneous T-cell lymphoma, extrahepatic ductal carcinoma *in situ* (DCIS), embryonal tumor, central nervous system cancer, endometrial cancer, ependymoma, esophageal cancer, granulomatous neuroblastoma, Ewing's sarcoma, extracranial germ cell tumor, extragonadal germ cell tumor, eye cancer, bone fibrous histiocytoma, gallbladder cancer, gastric cancer, gastrointestinal carcinoid, gastrointestinal stromal tumor (GIST), germ cell tumor, gestational trophoblastic tumor, hairy cell leukemia, head and neck cancer, heart disease, liver cancer, Hodgkin lymphoma, hypopharyngeal cancer, intraocular melanoma, islet cell tumor, pancreatic neuroendocrine tumor, kidney cancer, laryngeal cancer, lip and oral cancer, liver cancer, lobular carcinoma *in situ* (LCIS), lung cancer, lymphoma, metastatic squamous cell carcinoma, occult primary, midline cancer, oral cancer, multiple endocrine neoplasia syndrome, multiple myeloma/plasmacytoma, mycosis, mycosis fungoides, myelodysplastic syndrome, myelodysplastic/myeloproliferative neoplasm, multiple myeloma, Merkel cell carcinoma, malignant mesothelioma, malignant fibrous histiocytoma of bone and osteosarcoma, nasal cavity and paranasal sinuses, nasal cavity and sinonasal neuroblastoma, non-Hodgkin lymphoma, non-small cell lung cancer (NSCLC), oral cancer, lip and oral cancer, oropharyngeal cancer, ovarian cancer, pancreatic cancer, papillomatosis, paraganglioma, paranasal sinus and nasal cavity cancer, parathyroid cancer, penile cancer, throat cancer, pleuropulmonary blastoma, primary central nervous system (CNS) lymphoma, prostate cancer, rectal cancer, transitional cell carcinoma, retinoblastoma, rhabdomyosarcoma, salivary gland cancer, skin cancer, gastric (stomach) cancer, small cell lung cancer, small intestine cancer, soft tissue sarcoma, T-cell lymphoma, testicular cancer, laryngeal cancer, thymoma and thymic carcinoma, thyroid cancer, transitional cell carcinoma of the renal pelvis and ureter, trophoblastic tumor, uncommon childhood cancer, urethra cancer, uterine sarcoma, vaginal cancer, vulvar cancer, or viral cancer. In some embodiments, the non-cancerous hyperproliferative disease is, for example, benign hyperplasia of skin (such as psoriasis), restenosis, or prostate (such as benign prostatic hypertrophy (BPH)).

In some embodiments of the present disclosure, the cancer is selected from brain cancer, thyroid cancer, head and neck cancer, nasopharyngeal cancer, throat cancer, oral cancer, salivary gland cancer, esophageal cancer, gastric cancer, lung cancer, liver cancer, kidney cancer, pancreatic cancer, gallbladder cancer, cholangiocarcinoma, colorectal cancer, small intestine cancer, gastrointestinal stromal tumor, urothelial carcinoma, urethral cancer, bladder cancer, breast cancer, vaginal cancer, ovarian cancer, endometrial cancer, cervical cancer, fallopian tube cancer, testicular cancer, prostate cancer, hemangioma, leukemia, lymphoma, myeloma, skin cancer, lipoma, bone cancer, soft tissue sarcoma, neurofibroma, glioma, neuroblastoma, and glioblastoma; preferably selected from pancreatic cancer, colorectal cancer, and non-small cell lung cancer.

Definition of terms

The term "pharmaceutically acceptable" means that salts, solvents, excipients, etc. are generally non-toxic, safe, and suitable for use by patients. The "patient" is preferably a mammal, more preferably a human.

The term "pharmaceutically acceptable salt" refers to a pharmaceutically acceptable salt as defined herein and

has all the effects of the parent compound. The pharmaceutically acceptable salt can be prepared by adding a corresponding acid to a suitable organic solvent of an organic base and treating according to conventional methods.

Examples of salt formation include: for a base addition salt, it is possible to prepare salts of alkali metal (e.g., sodium, potassium, or lithium) or alkaline earth metal (e.g., aluminum, magnesium, calcium, zinc, or bismuth) by treating the compounds of the present disclosure with appropriate acidic protons in an aqueous medium using alkali metal, alkaline earth metal hydroxides, alcohol salts (e.g., ethanol salts or methanol salts), or suitable basic organic amines (e.g., diethanolamine, choline, or meglumine).

Alternatively, for an acid addition salt, the salt is formed with an inorganic acid, such as hydrochloric acid, hydrobromic acid, sulfuric acid, nitric acid, and phosphoric acid; and the salt is formed with an organic acid, such as acetic acid, benzenesulfonic acid, benzoic acid, camphorsulfonic acid, citric acid, ethanesulfonic acid, fumaric acid, glucoheptonic acid, glutamic acid, glycolic acid, hydroxynaphthoic acid, 2-hydroxyethanesulfonic acid, lactic acid, maleic acid, malic acid, oxalic acid, pyruvic acid, malonic acid, mandelic acid, methanesulfonic acid, muconic acid, 2-naphthalenesulfonic acid, propionic acid, salicylic acid, succinic acid, tartaric acid, citric acid, cinnamic acid, *p*-toluenesulfonic acid, or trimethylacetic acid.

The term "solvate" refers to a substance formed by combining a compound of the present disclosure with a stoichiometric or non-stoichiometric amount of a solvent. The solvent molecules in the solvate may exist in an ordered or disordered arrangement. The solvent includes, but is not limited to: water, methanol, ethanol, etc.

The term "prodrug" refers to a compound obtained after modification of the chemical structure of a drug, which is inactive or less active *in vitro,* and undergoes an enzymatic or non-enzymatic transformation *in vivo* to release the active drug and exert its therapeutic effect.

The term "metabolite" refers to intermediate and final metabolites of metabolism.

The term "isotopic compound" refers to a compound in which one or more atoms may be present in their non-natural abundance forms. Taking a hydrogen atom as an example, its non-natural abundance form means that about 95% of it is deuterium.

The term "pharmaceutical excipients" may refer to those excipients widely used in the field of pharmaceutical production. Excipients are mainly used to provide a safe, stable, and functional pharmaceutical composition, and can also provide a method to enable the active ingredients to dissolve at a desired rate after administration to the subject, or to facilitate the effective absorption of the active ingredient after the subject receives the composition. The pharmaceutical excipients may be inert fillers, or provide certain functions, such as stabilizing the overall pH value of the composition or preventing degradation of the active ingredients of the composition. The pharmaceutical excipients may include one or more of the following excipients: a binder, a suspending agent, an emulsifier, a diluent, a filler, a granulating agent, an adhesive, a disintegrating agent, a lubricant, an anti-adhesion agents, a glidant, a wetting agent, a gelling agent, an absorption retardant, a dissolution inhibitor, an enhancer, an adsorbent, a buffer, a chelating agent, a preservative, a colorant, a flavoring agent, and a sweetener.

The pharmaceutical composition of the present disclosure can be prepared by any method known to those skilled in the art according to the disclosure. For example, conventional mixing, dissolving, granulating, emulsifying, grinding, encapsulating, embedding, or lyophilizing processes.

The pharmaceutical composition of the present disclosure may be administered in any form, including injection (intravenous), mucosal, oral (solid and liquid formulations), inhalation, ocular, rectal, topical, or parenteral (infusion, injection, implantation, subcutaneous, intravenous, intraarterial, intramuscular) administration. The pharmaceutical composition of the present disclosure may also be in a controlled-release or delayed-release dosage form (such as liposomes or microspheres). Examples of solid oral formulations include, but are not limited to, powders, capsules, caplets, softgels, and tablets. Examples of liquid formulations for oral or mucosal administration include, but are not limited to, suspensions, emulsions, elixirs, and solutions. Examples of topical formulations include, but are not limited to, emulsions, gels, ointments, creams, patches, pastes, foams, lotions, drops, or serum formulations. Examples of formulations for parenteral administration include, but are not limited to, injectable solutions, dry formulations which may be dissolved or suspended in a pharmaceutically acceptable carrier, injectable suspensions, and injectable emulsions. Examples of other suitable formulations of the pharmaceutical composition include, but are not limited to, eye drops and other ophthalmic formulations; aerosols such as nasal sprays or inhalants; liquid dosage forms suitable for parenteral administration; suppositories and lozenges.

"Treatment" means any treatment of a disease in a mammal, including: (1) preventing the disease, that is, causing the symptoms of clinical disease not to develop; (2) inhibiting the disease, that is, preventing the development of clinical symptoms; and (3) alleviating the disease, that is, causing the clinical symptoms to subside.

"Effective amount" refers to an amount of a compound sufficient, when administered to a patient in need of treatment, to (i) treat the relevant disease, (ii) attenuate, ameliorate, or eliminate one or more symptoms of a particular disease or condition, or (iii) delay the onset of one or more symptoms of a specific disease or condition described herein. The amount of the carbonyl heterocyclic compound of formula II or the pharmaceutically acceptable salt thereof, or the amount of the above pharmaceutical composition, corresponding to such amount, will vary based on factors such as the

specific compound, disease condition and its severity, the characteristics (e.g., weight) of the patient in need of treatment, but can nevertheless be routinely determined by those skilled in the art.

The "methanol (20 mmol/L ammonia)" mentioned in the present disclosure means that each liter of methanol contains 20 mmol ammonia.

The "prevention" mentioned in the present disclosure refers to reducing the risk of acquiring or developing a disease or disorder.

The term "alkyl" refers to a straight or branched alkyl group with a specified number of carbon atoms. Examples of alkyl include methyl, ethyl, *n*-propyl, isopropyl, *n*-butyl, *tert*-butyl, isobutyl, *sec*-butyl, *n*-pentyl, *n*-hexyl, *n*-heptyl, *n*-octyl, and similar alkyl groups, preferably methyl. Alkyl is unsubstituted unless a substituent is specifically stated.

The term "heterocycloalkyl" refers to a stable 4- to 10- or 11-membered saturated cyclic group consisting of 3 to 9 carbon atoms and 1 to 3 heteroatoms selected from nitrogen, oxygen, and sulfur. Unless otherwise specifically stated in the specification, a heterocycloalkyl group may be a monocyclic ("monocyclic heterocycloalkyl"), or bicyclic, tricyclic, or polycyclic ring system, which may include fused (fused-ring), bridged (bridged-ring), or spiro (spiro-ring) ring systems (e.g., a bicyclic system ("bicyclic heterocycloalkyl")). The heterocycloalkyl bicyclic ring system may include one or more heteroatoms in one or both rings; and is saturated. Heterocycloalkyl is unsubstituted unless a substituent is specifically stated.

The term "aryl" refers to phenyl or naphthyl.

The term "heteroaryl" refers to an aromatic group containing heteroatoms, preferably aromatic 5- to 10-membered monocyclic rings or 5- to 10-membered bicyclic rings containing 1, 2, or 3 heteroatoms independently selected from nitrogen, oxygen, and sulphur, such as furyl, pyridyl, pyridazinyl, pyrimidinyl, pyrazinyl, thienyl, isoxazolyl, oxazolyl, diazolyl, imidazolyl, pyrrolyl, pyrazolyl, triazolyl, tetrazolyl, thiazolyl, isothiazolyl, thiadiazolyl, benzimidazolyl, indolyl, indazolyl, benzothiazolyl, benzisothiazolyl, benzoxazolyl, benzisoxazolyl, quinolyl, isoquinolyl, etc.

The term "halogen" refers to fluorine, chlorine, bromine, or iodine, preferably fluorine or chlorine.

The term "alkoxy" refers to the group -O-$R^X$, wherein $R^X$ represents the alkyl group as defined above.

The term "alkynyl" refers to a straight or branched hydrocarbon group having one or more triple bonds with a specific number of carbon atoms (for example, $C_2$-$C_6$ alkynyl, for another example, $C_2$-$C_4$ alkynyl). The one or more carbon-carbon triple bonds may be internal or terminal, such as a propynyl group with a triple bond at the internal position

( 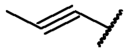 )

or a propynyl group with a triple bond at the terminal position

( 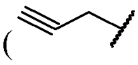 ).

The term "carbocyclic ring" refers to a cyclic, saturated or unsaturated ring with a specified number of carbon atoms (e.g., $C_3$-$C_6$), and the ring (1) is attached to the rest of the molecule by two or more single bonds; or (2) shares two atoms and one bond with the rest of the molecule.

The term "heterocyclic ring" refers to a cyclic, saturated or unsaturated ring with a specified number of ring atoms (e.g., 3- to 10-membered), a specified number of heteroatoms (e.g., 1, 2, or 3), and a specified type of heteroatoms (one or more of N, O, and S), and the ring (1) is attached to the rest of the molecule by two or more single bonds; or (2) shares two atoms and one bond with the rest of the molecule.

The term "cycloalkyl" refers to a cyclic, saturated, monovalent hydrocarbon group with a specified number of carbon atoms (e.g., $C_3$-$C_8$). Cycloalkyl includes, but is not limited to:

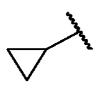, 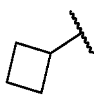, 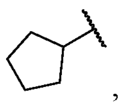, 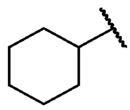,

etc.

The term "oxo" refers to the "=O" group.

The term "-$(CR^{L-3}R^{L-4})_{n3}$-*" means that 0, 1, 2, 3, or 4 -$(CR^{L-3}R^{L-4})_{n3}$- moieties are connected. For example, when n3 is 2, "-$(CR^{L-3}R^{L-4})_{n3}$-*" means -$(CR^{L-3}R^{L-4})$-$(CR^{L-3}R^{L-4})$- *, where each $R^{L-3}$ and $R^{L-4}$ are the same or different.

In the present disclosure, if there is a stereoconfiguration resulting from a chiral axis or a chiral carbon in a compound or its salt, the stereoconfiguration of these compounds is consistent with that of their salts. For example, the stereoconfiguration of compound 1a' is consistent with that of its salt 1a.

On the basis of not violating common sense in the art, the above various preferred conditions can be arbitrarily combined to obtain the various preferred embodiments of the present disclosure.

The positive and progressive effect of the present disclosure lies in that the compound of the present disclosure has a good inhibitory effect on KRAS G12D mutant protein.

DETAILED DESCRIPTION OF THE PREFERRED EMBODIMENT

The present disclosure is further described through examples below, but is not limited to the scope of the examples provided. In the following examples, experimental methods without specified conditions are carried out according to conventional methods and conditions, or are selected according to the product instructions.

In the present disclosure, for the compounds or their salts ultimately prepared in the following examples, if there exists a stereoconfiguration resulting from a chiral axis or chiral carbon in these compounds or their salts, the stereoconfigurationof these compounds or their salts resulting from the chiral axis or chiral carbon is consistent with the configuration of intermediates containing the chiral axis or chiral carbon used to prepare these compounds. For example, the salts 1a and 1b of the compound in Example 1: the salt 1a (1b) of the compound is prepared from intermediate 1-10a (intermediate 1-10b) containing a chiral carbon, then the configuration resulting from the chiral carbon in the salt 1a (1b) of the compound is consistent with the configuration of the intermediate 1-10a (intermediate 1-10b). In other examples of the present disclosure, the configurations resulting from the chiral carbon or chiral axis are consistent with the situation in Example 1.

**Example 1**

(*S* or *R*)-4'-((1*R*,5*S*)-3,8-Diazabicyclo[3.2.1]octan-3-yl)-4-chloro-2'-((2*R*,7aS)-2-fluorotetrahydro-1*H*-pyrrolizin-7*a*(5*H*)-ylmethoxy)-2,3,5',8'-tetrahydro-6'*H*-spiro[indene-1,7'-quinazoline] dihydrochloride **1a**; (*R* or *S*)-4'-((1*R*,5*S*)-3,8-diazabicyclo[3.2.1]octan-3-yl)-4-chloro-2'-((2*R*,7a*S*)-2-fluorotetrahydro-1*H*-pyrrolizin-7*a*(5*H*)-ylmethoxy)-2,3,5',8'-tetrahydro-6'*H*-spiro[indene-1,7'-quinazoline] dihydrochloride **1b**

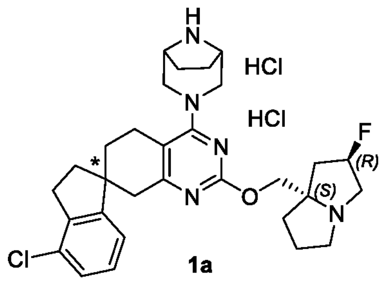
**1a**

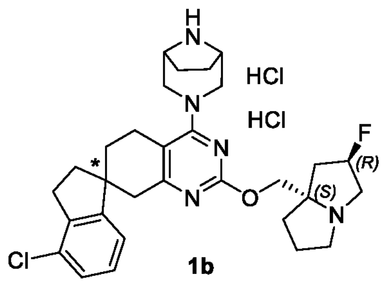
**1b**

The synthetic route is as follows:

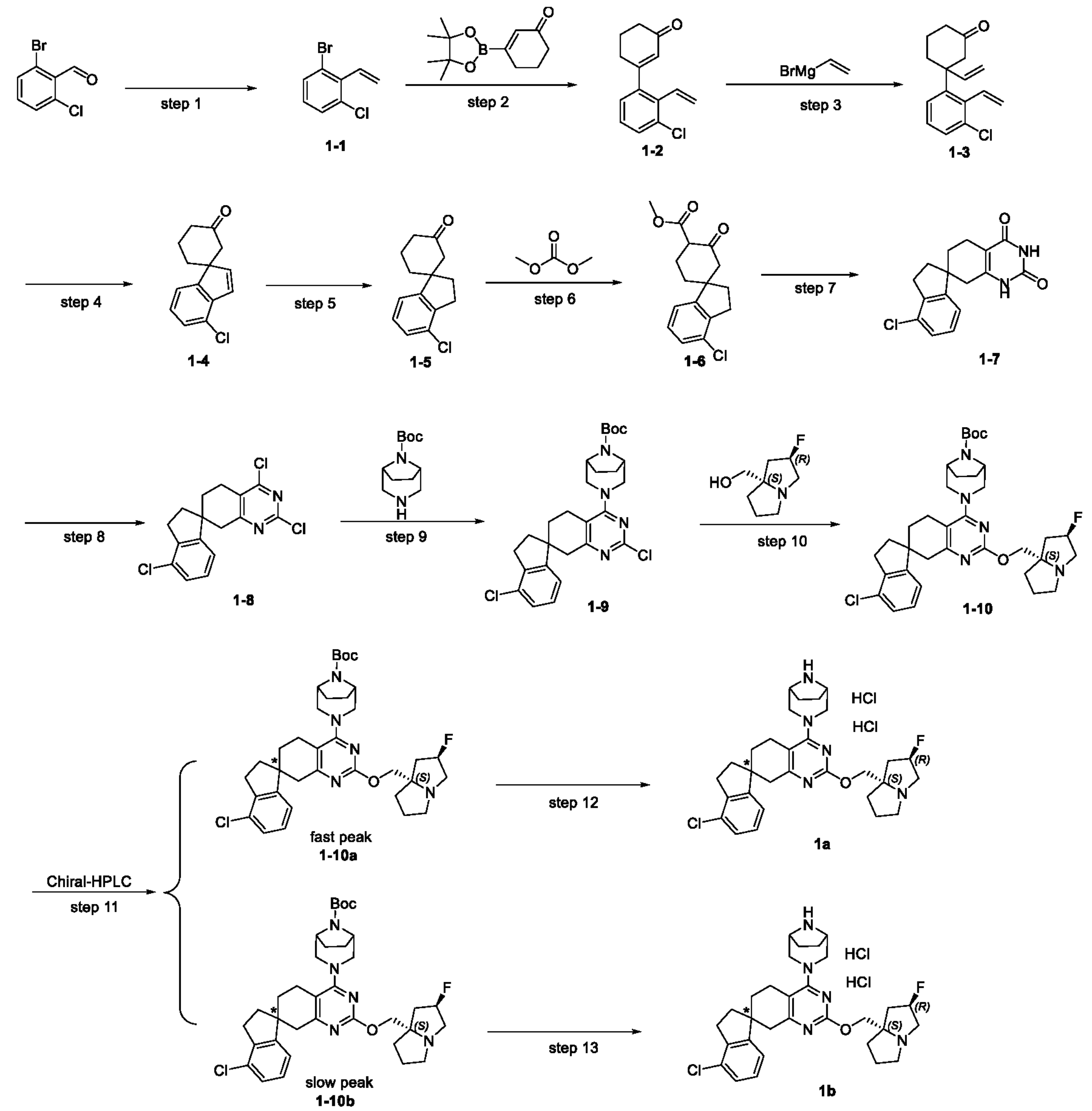


Step 1:

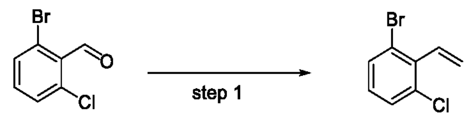


1-1

Under nitrogen atmosphere at 0°C with stirring, a solution of n-butyllithium in n-hexane (2.5 M, 145 mL, 363.6 mmol, 1.2 eq) was slowly added dropwise to a solution of methyltriphenylphosphonium bromide (136.7 g, 363.6 mmol, 1.2 eq) in anhydrous tetrahydrofuran (700 mL). After the mixture was reacted with stirring at 0°C for 1 hour, a solution of 2-chloro-6-bromobenzaldehyde (70 g, 303.0 mmol, 1.0 eq) in anhydrous tetrahydrofuran (300 mL) was slowly added to the reaction mixture at the same temperature. The resulting mixture was reacted with stirring at 25°C for 2 hours, with the reaction progress monitored by TLC. After the reaction was completed, the reaction was quenched by adding saturated ammonium chloride solution (700 mL) at 0°C. The mixture was extracted with ethyl acetate (700 mL × 3). The organic phases were combined, dried over anhydrous sodium sulfate, and filtered to remove the desiccant. The filtrate was concentrated to obtain the crude product. The crude product was purified by silica gel column chromatography, eluting with

petroleum ether. The collected fraction was subjected to rotary evaporation under reduced pressure to remove the solvent, yielding compound **1-1** (colorless liquid, 35 g, yield of 50%). $^1$H NMR (300 MHz, $CDCl_3$) $\delta$ 7.58 - 7.49 (m, 1H), 7.43 - 7.35 (m, 1H), 7.10 - 6.99 (m, 1H), 6.76 - 6.60 (m, 1H), 5.80 - 5.64 (m, 2H).

Step 2:

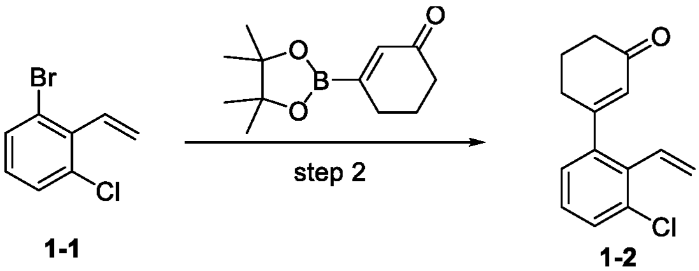


1-1

1-2

Under nitrogen atmosphere at 25°C with stirring, [1,1'-bis(diphenylphosphino)ferrocene]dichloropalladium(II) dichloromethane complex (3.88 g, 4.52 mmol, 0.05 eq) was added to a mixed solution of compound **1-1** (20.7 g, 90.4 mmol, 1.0 eq), 2-cyclohexen-1-one-3-boronic acid pinacol ester (25.37 g, 108.5 mmol, 1.2 eq), and sodium carbonate (30.26 g, 271.25 mmol, 3 eq) in dimethoxyethane (150 mL) and water (30 mL). The mixture was reacted with stirring at 90°C for 2 hours, with the reaction progress monitored by LC-MS and TLC. After the reaction was completed, the mixture was cooled to room temperature. The reaction mixture was diluted by adding water (200 mL) and then extracted with ethyl acetate (200 mL × 3). The organic phases were combined, dried over anhydrous sodium sulfate, and filtered to remove the desiccant. The filtrate was concentrated to obtain the crude product. The crude product was purified by silica gel column chromatography and subjected to a gradient elution with a mobile phase of 0% to 30% ethyl acetate/petroleum ether. The collected fraction was subjected to rotary evaporation under reduced pressure to remove the solvent, yielding compound **1-2** (pale yellow liquid, 20 g, yield of 82%). MS (ESI, m/z): 233.0/235.0 $[M + H]^+$, $^1$H NMR (300 MHz, $CDCl_3$) $\delta$ 7.46 - 7.36 (m, 1H), 7.30 - 7.18 (m, 1H), 7.13 - 7.03 (m, 1H), 6.98 - 6.82 (m, 1H), 6.17 - 6.10 (m, 1H), 5.60 - 5.45 (m, 2H), 2.60 - 2.42 (m, 4H), 2.15 - 2.02 (m, 2H).

Step 3:

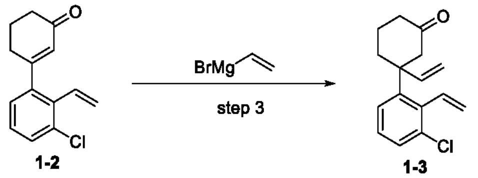


1-2

1-3

Under nitrogen atmosphere at -78°C with stirring, a solution of vinylmagnesium bromide in tetrahydrofuran (1 M, 122 mL, 122.46 mmol, 3 eq) was slowly added to a solution of cuprous iodide (12.28 g, 61.2 mmol, 1.5 eq) and lithium chloride (2.73 g, 61.2 mmol, 1.5 eq) in anhydrous tetrahydrofuran (300 mL). After the mixture was reacted with stirring at -78°C for 1 hour, a solution of compound **1-2** (10 g, 40.82 mmol, 1.0 eq) in anhydrous tetrahydrofuran (300 mL) was slowly added dropwise to the reaction mixture at the same temperature. The mixture was reacted with stirring at -78°C for 1 hour, then slowly warmed to room temperature and allowed to react for an additional 0.5 hours, with the reaction progress monitored by LC-MS and TLC. After the reaction was completed, the reaction was quenched by adding saturated ammonium chloride solution (200 mL) to the mixture at 0°C. The mixture was then extracted with ethyl acetate (200 mL × 3). The organic phases were combined, dried over anhydrous sodium sulfate, and filtered. The filtrate was concentrated to obtain the crude product. The crude product was purified by silica gel column chromatography and subjected to a gradient elution with a mobile phase of 0% to 10% ethyl acetate/petroleum ether. The collected fraction was subjected to rotary evaporation under reduced pressure to remove the solvent, yielding compound **1-3** (pale yellow oil, 5.68 g, yield of 53%). MS (ESI, m/z): 261.0/263.0 $[M + H]^+$; $^1$H NMR (400 MHz, $CDCl_3$) $\delta$ 7.39 - 7.33 (m, 1H), 7.31 - 7.27 (m, 1H), 7.19 - 7.13 (m, 1H), 6.83 - 6.72 (m, 1H), 5.97 - 5.88 (m, 1H), 5.64 - 5.58 (m, 1H), 5.38 - 5.30 (m, 1H), 5.20 - 5.14 (m, 1H), 4.98 - 4.90 (m, 1H), 2.79 (s, 2H), 2.75 - 2.65 (m, 1H), 2.35 - 2.26 (m, 2H), 2.06 - 1.96 (m, 1H), 1.91 - 1.79 (m, 1H), 1.67 - 1.59 (m, 1H).

Step 4:

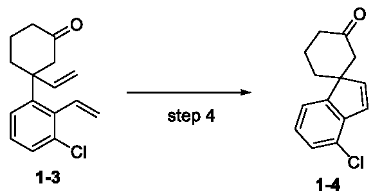


1-3 1-4

Under nitrogen atmosphere at 25°C with stirring, 1,3-bis(2,4,6-trimethylphenyl)-2-imidazolidinylidene)dichloro(phenylmethylene)(tricyclohexylphosphine)ruthenium (2.77 g, 3.10 mmol, 0.15 eq) was added to a solution of compound **1-3** (5.68 g, 20.7 mmol, 1.0 eq) in dichloromethane (60 mL). The mixture was reacted with stirring at 30°C for 16 hours, with the reaction progress monitored by TLC. After the reaction was completed, the mixture was concentrated under reduced pressure to obtain the crude product. The crude product was purified by silica gel column chromatography and subjected to a gradient elution with a mobile phase of 0% to 10% ethyl acetate/petroleum ether. The collected fraction was subjected to rotary evaporation under reduced pressure to remove the solvent, yielding compound **1-4** (pale yellow oil, 3.8 g, yield of 74%). $^1$H NMR (300 MHz, $CDCl_3$) $\delta$ 7.30 - 7.14 (m, 3H), 6.93 - 6.86 (m, 1H), 6.57 - 6.52 (m, 1H), 2.77 - 2.69 (m, 1H), 2.63 - 2.55 (m, 2H), 2.36 - 2.24 (m, 1H), 2.22 - 2.14 (m, 1H), 2.14 - 1.94 (m, 2H), 1.75 - 1.64 (m, 1H).

Step 5:

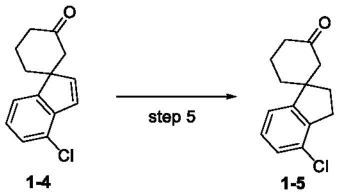


1-4 1-5

Under nitrogen atmosphere at 25°C with stirring, platinum on carbon (10% platinum, 0.35 g) was added to a solution of compound **1-4** (3.5 g, 14.3 mmol, 1.0 eq) in ethyl acetate (50 mL). The nitrogen was replaced with hydrogen through gas displacement. The mixture was reacted with stirring at 25°C under a hydrogen atmosphere at 1 atm for 2 hours, with the reaction progress monitored by TLC. After the reaction was completed, the mixture was filtered to remove the insoluble material, and the filter cake was washed with ethyl acetate (70 mL × 3). The filtrate was concentrated under reduced pressure to obtain the crude product. The crude product was purified by silica gel column chromatography and subjected to a gradient elution with a mobile phase of 0% to 20% methyl *tert*-butyl ether/petroleum ether. The collected fraction was subjected to rotary evaporation under reduced pressure to remove the solvent, yielding compound **1-5** (colorless oil, 3.24 g, yield of 95%). $^1$H NMR (300 MHz, $CDCl_3$) $\delta$ 7.24 - 7.14 (m, 2H), 7.08 - 7.05 (m, 1H), 2.99 - 2.94 (m, 2H), 2.58 - 2.34 (m, 4H), 2.16 - 2.02 (m, 2H), 2.01 - 1.85 (m, 3H), 1.84 - 1.75 (m, 1H).

Step 6:

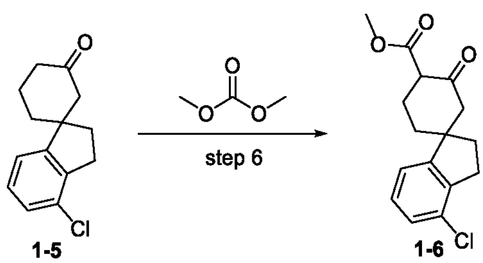


1-5 1-6

Under nitrogen atmosphere at 25°C with stirring, sodium hydride (60%, 2.43 g, 60.7 mmol, 5.0 eq) was added to a

solution of compound **1-5** (3.0 g, 12.14 mmol, 1.0 eq) in tetrahydrofuran (100 mL). After the mixture was reacted with stirring at 25°C for 0.5 hours, dimethyl carbonate (6.91 g, 72.85 mmol, 6.0 eq) was added to the reaction mixture. The mixture was reacted with stirring at 70°C for 2 hours, with the reaction progress monitored by LC-MS and TLC. After the reaction was completed, the reaction was quenched by adding saturated ammonium chloride solution (100 mL) to the system at 0°C. The mixture was then extracted with ethyl acetate (50 mL × 3). The organic phases were combined, dried over anhydrous sodium sulfate, and filtered. The filtrate was concentrated to obtain the crude product. The crude product was purified by silica gel column chromatography and subjected to a gradient elution with a mobile phase of 0% to 20% ethyl acetate/petroleum ether. The collected fraction was subjected to rotary evaporation under reduced pressure to remove the solvent, yielding compound **1-6** (white solid, 3.7 g, yield of 98%). MS (ESI, m/z): 293.1/295.1 $[M + H]^+$; $^1H$ NMR (400 MHz, $CDCl_3$) $\delta$ 12.19 (s, 1H), 7.22 - 6.94 (m, 3H), 3.80 - 3.79 (m, 3H), 2.98 - 2.94 (m, 2H), 2.49 - 2.22 (m, 4H), 2.02 - 1.91 (m, 2H), 1.86 - 1.79 (m, 1H), 1.74 - 1.60 (m, 1H).

Step 7:

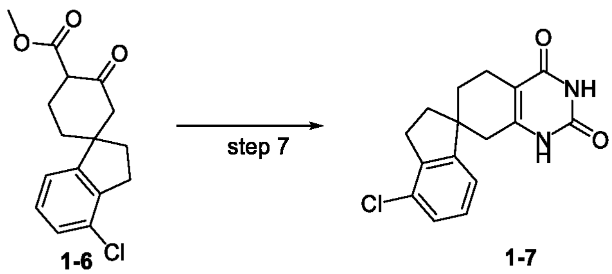


1-6 1-7

With stirring at 25°C, ammonium acetate (0.79 g, 9.73 mmol, 3.0 eq) was added to a solution of compound **1-6** (1.0 g, 3.24 mmol, 1.0 eq) in methanol (30 mL). The mixture was reacted with stirring at 25°C for 16 hours, with the reaction progress monitored by LC-MS and TLC. After the reaction was completed, water (70 mL) was added to dilute the reaction mixture. The mixture was then extracted with dichloromethane (70 mL × 3). The organic phases were combined, dried over anhydrous sodium sulfate, and filtered. The filtrate was concentrated to obtain the crude product.

With stirring at 25°C, trichloroacetyl isocyanate (0.84 g, 4.22 mmol, 1.3 eq) was added to a solution of the crude product in acetonitrile (20 mL). The mixture was reacted with stirring at 25°C for 0.5 hours, with the reaction progress monitored by LC-MS and TLC. After the reaction was completed, the mixture was filtered to obtain an intermediate, which was washed with acetonitrile (20 mL × 3). With stirring at 25°C, the intermediate was then dissolved in a solution of ammonia in methanol (7 M, 30 mL). The mixture was reacted with stirring at 70°C for 2 hours, with the reaction progress monitored by LC-MS and TLC. After the reaction was completed, the mixture was cooled to room temperature and filtered to obtain a solid. The filter cake was washed with methyl *tert*-butyl ether (10 mL × 3). The resulting filter cake was dried to obtain compound **1-7** (white solid, 820 mg, yield of 81%). MS (ESI, m/z): 303.1/305.1 $[M + H]^+$; $^1H$ NMR (400 MHz, DMSO-$d_6$) $\delta$ 10.97 (s, 1H), 10.65 (s, 1H), 7.30 - 7.20 (m, 2H), 7.19 - 7.14 (m, 1H), 2.96 - 2.88 (m, 2H), 2.55 - 2.53 (m, 1H), 2.40 - 2.24 (m, 3H), 2.01 - 1.84 (m, 3H), 1.70 - 1.59 (m, 1H).

Step 8:

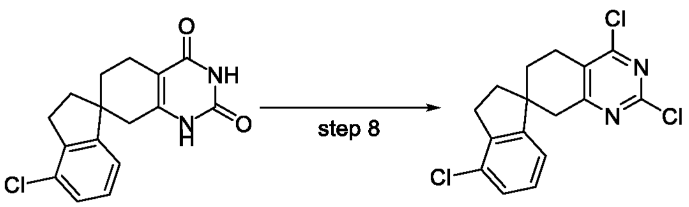


1-7 1-8

Under nitrogen atmosphere at 25°C with stirring, *N*,*N*-diisopropylethylamine (623 mg, 4.83 mmol, 2.0 eq) and *N*,*N*-dimethylformamide (28 mg, 0.384 mmol, 0.16 eq) were added to a solution of compound **1-7** (770 mg, 2.42 mmol, 1.0 eq) in phosphorus oxychloride (10 mL). The mixture was reacted with stirring at 110°C for 2 hours, with the reaction progress monitored by LC-MS and TLC. After the reaction was completed, the system was cooled to room temperature.

The mixture was concentrated under reduced pressure to obtain the crude product. At 0°C, 40 mL of saturated sodium bicarbonate was added to the mixture. The resulting mixture was extracted with ethyl acetate (60 mL × 3). The organic phases were combined, dried over anhydrous sodium sulfate, and filtered. The filtrate was concentrated to obtain the crude product. The crude product was purified by silica gel column chromatography and subjected to a gradient elution with a mobile phase of 0% to 20% ethyl acetate/petroleum ether. The collected fraction was subjected to rotary evaporation under reduced pressure to remove the solvent, yielding compound **1-8** (white solid, 780 mg, yield of 93%). $^1$H NMR (300 MHz, $CDCl_3$) $\delta$ 7.29 - 7.13 (m, 2H), 6.93 - 6.87 (m, 1H), 3.07 - 2.98 (m, 4H), 2.95 - 2.78 (m, 2H), 2.18 - 1.89 (m, 4H).

Step 9:

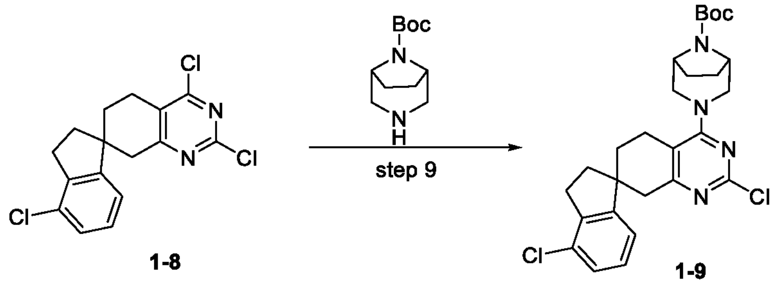


1-8

1-9

Under nitrogen atmosphere at 25°C with stirring, *N*,*N*-diisopropylethylamine (831 mg, 6.44 mmol, 3.0 eq) was added to a mixed solution of compound **1-8** (770 mg, 2.15 mmol, 1.0 eq) and *tert*-butyl 3,8-diazabicyclo[3.2.1]octane-8-carboxylate (481 mg, 2.15 mmol, 1.0 eq) in dimethyl sulfoxide (12 mL) and 1,2-dichloroethane (3 mL). The mixture was reacted with stirring at 55°C for 1 hour, with the reaction progress monitored by LC-MS and TLC. After the reaction was completed, the reaction mixture was cooled to room temperature. At 25°C, water (150 mL) was added to the mixture to dilute the reaction mixture. The resulting mixture was extracted with ethyl acetate (80 mL × 3). The organic phases were combined, further washed with saturated brine (150 mL), then dried over anhydrous sodium sulfate, and filtered to remove the desiccant. The filtrate was concentrated to obtain the crude product. The crude product was purified by silica gel column chromatography and subjected to a gradient elution with a mobile phase of 0% to 20% ethyl acetate/petroleum ether. The collected fraction was subjected to rotary evaporation under reduced pressure to remove the solvent, yielding compound **1-9** (white solid, 1 g, yield of 86%). MS (ESI, m/z): 515.0/517.0/519.0 $[M + H]^+$; $^1$H NMR (400 MHz, $CDCl_3$) $\delta$ 7.23 - 7.18 (m, 1H), 7.16 - 7.09 (m, 1H), 6.95 - 6.87 (m, 1H), 4.44 - 4.20 (m, 2H), 3.96 - 3.86 (m, 1H), 3.78 - 3.69 (m, 1H), 3.38 - 3.26 (m, 1H), 3.25 - 3.17 (m, 1H), 3.06 - 2.95 (m, 2H), 2.94 - 2.89 (m, 2H), 2.74 - 2.49 (m, 2H), 2.10 - 2.01 (m, 2H), 1.99 - 1.88 (m, 4H), 1.85 - 1.70 (m, 2H), 1.49 (s, 9H).

Step 10:

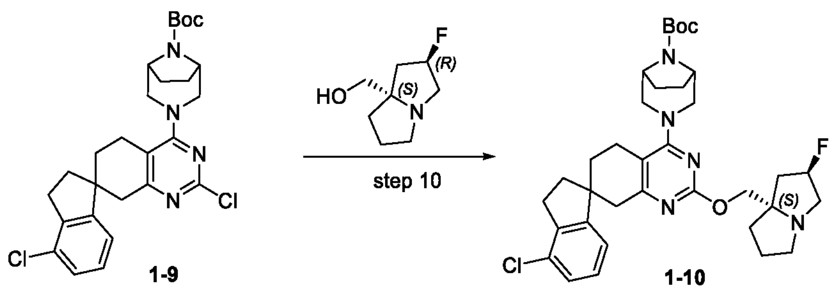


1-9

1-10

Under nitrogen atmosphere at 0°C with stirring, a solution of potassium *tert*-butoxide in tetrahydrofuran (1 M, 1.84 mL, 1.84 mmol, 5.0 eq) was added to a solution of compound **1-9** (200 mg, 0.36 mmol, 1.0 eq) and (2*R*,7a*S*)-2-fluorotetrahydro-1*H*-pyrrolizin-7a(5*H*)-methanol (247 mg, 1.48 mmol, 4.0 eq) in 1,4-dioxane (5 mL). The mixture was reacted with stirring at 100°C for 2 hours, with the reaction progress monitored by LC-MS and TLC. After the reaction was completed, the reaction mixture was cooled to room temperature. At 0°C, water (30 mL) was added to the mixture to quench the reaction. The resulting mixture was extracted with ethyl acetate (30 mL × 3). The organic phases were combined, dried over anhydrous sodium sulfate, and filtered to remove the desiccant. The filtrate was concentrated to

obtain the crude product. The crude product was purified by silica gel column chromatography and subjected to a gradient elution with a mobile phase of 0% to 10% methanol/dichloromethane. The collected fraction was subjected to rotary evaporation under reduced pressure to remove the solvent, yielding compound 1-10 (white solid, 155 mg, yield of 64%). MS (ESI, m/z): 638.4/640.4 [M + H]+.

Step 11:

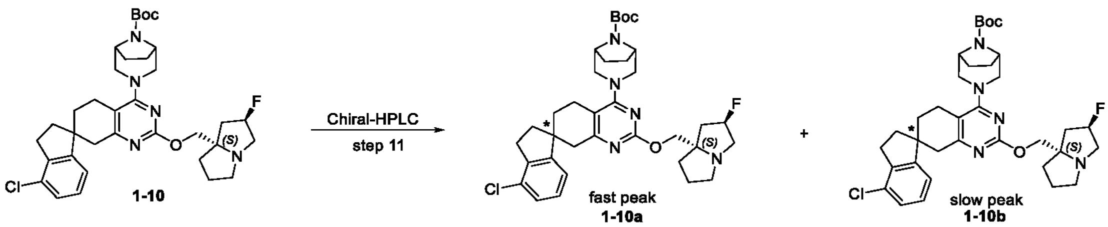


The compound **1-10** (155 mg) obtained from step 10 was subjected to chiral resolution by preparative chiral high-performance liquid chromatography: chiral column CHIRAL ART Cellulose-SB, 2 × 25 cm, 5 μm; mobile phase A: n-hexane (10 mmol/L a solution of ammonia in methanol), mobile phase B: ethanol; flow rate: 20 mL/min; elution with 10% phase B over 10 minutes; detector: UV 222/288 nm, resulting in two products. The product with a shorter retention time (6.2 minutes) was compound **1-10a** (white solid, 64.6 mg, yield of 40%), MS (ESI, m/z): 638.4/640.4 [M + H]+. The product with a longer retention time (7.9 minutes) was compound **1-10b** (white solid, 57.5 mg, yield of 35%), MS (ESI, m/z): 638.4/640.4 [M + H]+.

Step 12:

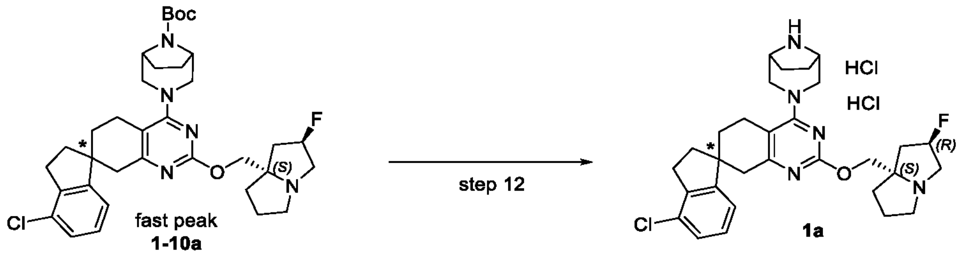


With stirring at 0°C, a solution of hydrogen chloride in 1,4-dioxane (4 M, 2 mL) was slowly added to a solution of compound **1-10a** (58 mg, 0.088 mmol, 1.00 eq) in methanol (2 mL). The reaction mixture was reacted at 25°C for 1 hour, with the reaction progress monitored by LC-MS and TLC. After the reaction was completed, the reaction mixture was concentrated under reduced pressure to obtain the crude product. The crude product was purified by reverse-phase chromatography (C18 column) and eluted with a mobile phase of 0% to 95% acetonitrile/water (0.1% hydrochloric acid) over 20 minutes, with detection at UV 254/220 nm. The product obtained was compound **1a** (white solid, 39.7 mg, yield of 73%). MS (ESI, m/z): 538.2/540.2 [M + H]+; $^1$H NMR (300 MHz, DMSO-$d_6$) $\delta$ 12.18 - 11.99 (m, 1H), 10.20 - 10.07 (m, 1H), 9.88 - 9.71 (m, 1H), 7.34 - 7.19 (m, 3H), 5.73 - 5.47 (m, 1H), 4.72 - 4.57 (m, 4H), 4.41 - 4.26 (m, 1H), 4.18 - 4.00 (m, 2H), 3.97 - 3.62 (m, 5H), 3.32 - 3.19 (m, 1H), 3.01 - 2.79 (m, 5H), 2.67 - 2.56 (m, 1H), 2.48 - 2.37 (m, 1H), 2.30 - 1.89 (m, 10H), 1.87 - 1.70 (m, 2H); $^{19}$F NMR (282 MHz, DMSO-$d_6$) $\delta$ -171.74. The chiral analysis conditions for compound **1a** were as follows: Optichiral C9-3, 3.0 × 100 mm, 3 μm; mobile phase A: supercritical carbon dioxide; mobile phase B: methanol (20 mmol/L ammonia); flow rate: 2 mL/min; isocratic gradient elution with 50% phase B over 1.8 minutes; detector: UV 220 nm; retention time: 1.275 minutes; *dr* > 40:1.

Step 13:

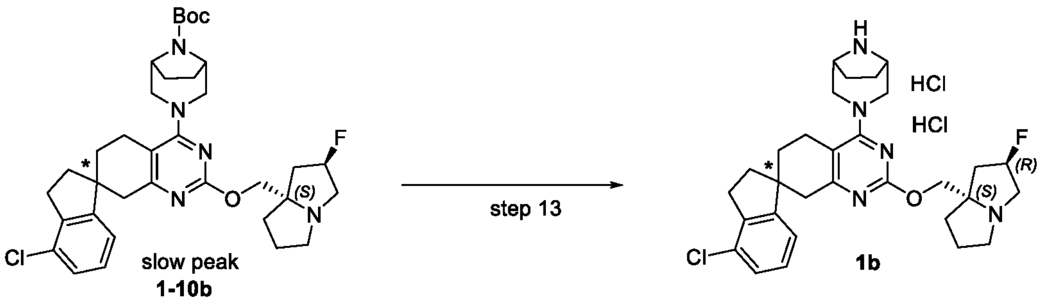


slow peak
**1-10b**

**1b**

Compound **1b** (white solid, 37.7 mg, yield of 69%) could be obtained using the same method above. MS (ESI, m/z): 538.2/540.2 [M + H]$^+$; $^1$H NMR (300 MHz, DMSO-$d_6$) $\delta$ 12.01 - 11.72 (m, 1H), 10.14 - 9.94 (m, 1H), 9.81 - 9.60 (m, 1H), 7.33 - 7.25 (m, 2H), 7.24 - 7.16 (m, 1H), 5.71 - 5.47 (m, 1H), 4.70 - 4.51 (m, 3H), 4.23 - 4.06 (m, 4H), 3.88 - 3.70 (m, 4H), 3.67 - 3.48 (m, 1H), 3.35 - 3.19 (m, 1H), 3.02 - 2.94 (m, 2H), 2.91 - 2.79 (m, 3H), 2.68 - 2.58 (m, 1H), 2.50 - 2.43 (m, 1H), 2.31 - 1.67 (m, 12H); $^{19}$F NMR (282 MHz, DMSO-$d_6$) $\delta$ -172.08. The chiral analysis conditions for compound **1b** were as follows: Optichiral C9-3, 3.0 × 100 mm, 3 μm; mobile phase A: supercritical carbon dioxide; mobile phase B: methanol (20 mmol/L ammonia); flow rate: 2 mL/min; isocratic gradient elution with 50% phase B over 1.8 minutes; detector: UV 220 nm; retention time: 1.082 minutes; *dr* > 40:1.

## Example 2

(*S* or *R*)-4'-((1*R*,5*S*)-3,8-Diazabicyclo[3.2.1]octan-3-yl)-4-chloro-2'-((2*R*,7aS)-2-fluorotetrahydro-1*H*-pyrrolizin-7*a*(5*H*)-ylmethoxy)-2,3,5',8'-tetrahydro-6'*H*-spiro[indene-1,7'-quinazolin]-6-ol dihydrochloride **2a;** (*R* or *S*)-4'-((1*R*,5*S*)-3,8-diazabicyclo[3.2.1]octan-3-yl)-4-chloro-2'-((2*R*,7a*S*)-2-fluorotetrahydro-1*H*-pyrrolizin-7*a*(5*H*)-ylmethoxy)-2,3,5',8'-tetrahydro-6'*H*-spiro[indene-1,7'-quinazolin]-6-ol dihydrochloride **2b**

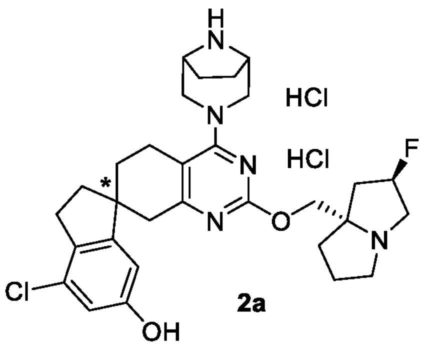

**2a**

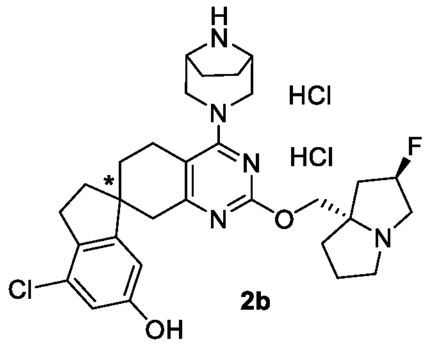

**2b**

The synthetic route is as follows:

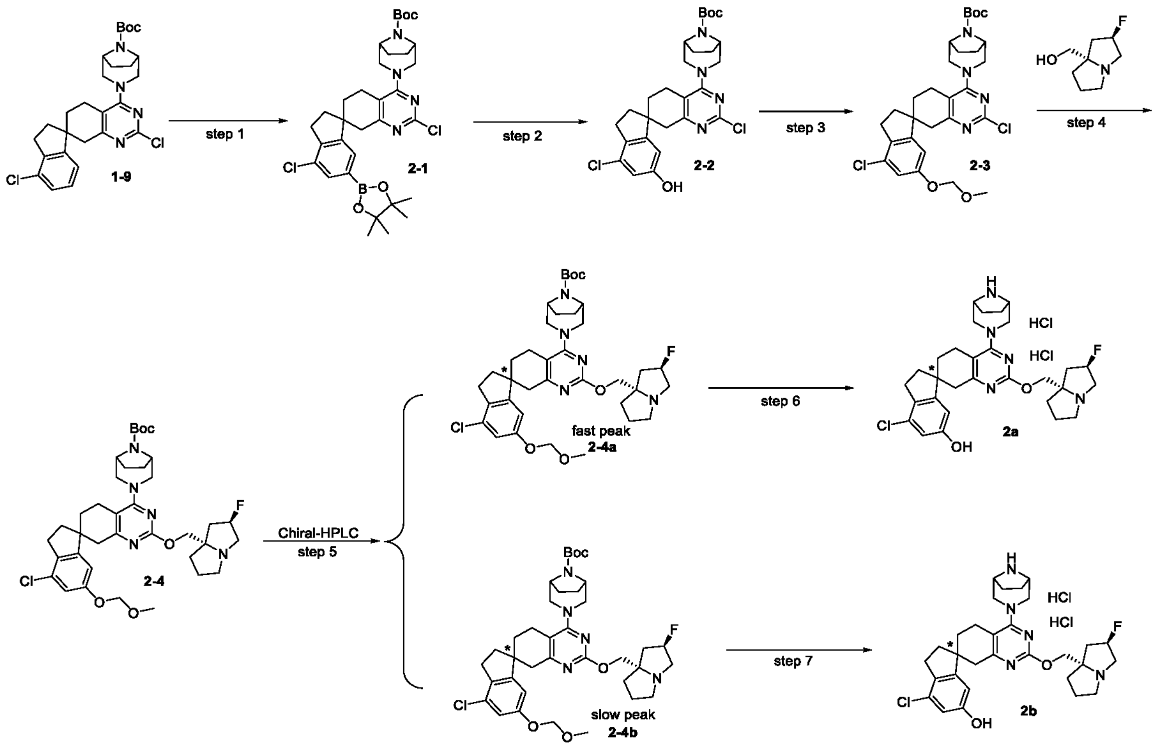


Step 1:

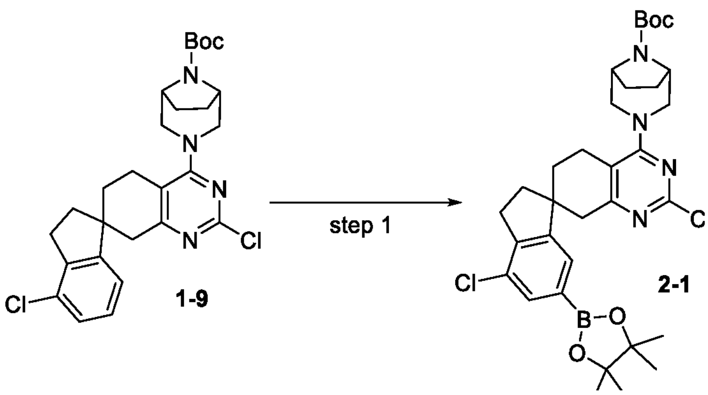


Under nitrogen atmosphere at 25°C with stirring, compound **1-9** (300 mg, 0.553 mmol, 1.0 eq), bis(pinacolato) diboron (591 mg, 2.21 mmol, 4.0 eq), chloro(1,5-cyclooctadiene)iridium(I) dimer (39 mg, 0.055 mmol, 0.1 eq), 4,4'-di-tert-butyl-2,2'-bipyridine (31 mg, 0.111 mmol, 0.2 eq), and 1,4-dioxane (6 mL) were sequentially added to a reaction flask. The mixture was reacted at 120°C for 3 hours, with the reaction progress monitored by LC-MS and TLC. After the reaction was completed, the system was cooled to room temperature. The reaction mixture was concentrated under reduced pressure to obtain the crude product. The crude product was purified by silica gel column chromatography and subjected to a gradient elution with a mobile phase of 0% to 20% ethyl acetate/petroleum ether. The collected fraction was subjected to rotary evaporation under reduced pressure to remove the solvent, yielding compound **2-1** (white solid, 280 mg, yield of 79%). MS (ESI, m/z): 641.2/643.2/645.2 $[M + H]^+$.

Step 2:

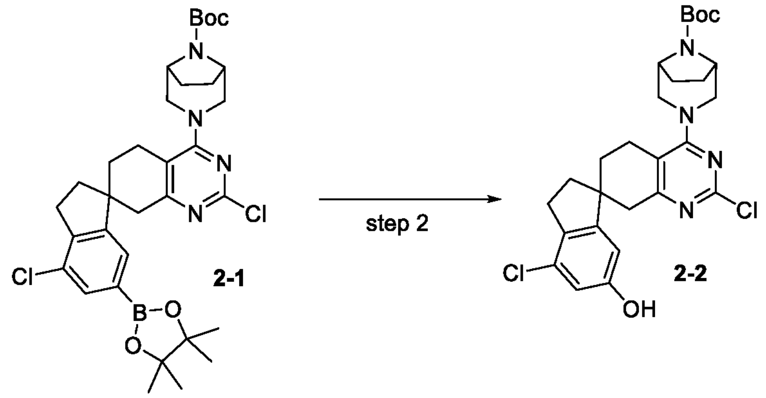


2-1 2-2

With stirring at 25°C, a hydrogen peroxide-urea complex (287.44 mg, 2.905 mmol, 7 eq) was added to a solution of compound **2-1** (280 mg, 0.415 mmol, 1 eq) in methanol (5 mL). The mixture was stirred at 25°C for 1 hour, with the reaction progress monitored by LC-MS and TLC. After the reaction was completed, the reaction mixture was concentrated under reduced pressure to obtain the crude product. The crude product was purified by silica gel column chromatography and subjected to a gradient elution with a mobile phase of 0% to 40% ethyl acetate/petroleum ether. The collected fraction was subjected to rotary evaporation under reduced pressure to remove the solvent, yielding compound **2-2** (white solid, 225 mg, yield of 96%). MS (ESI, m/z): 531.2/533.2/535.2 $[M + H]^+$.

Step 3:

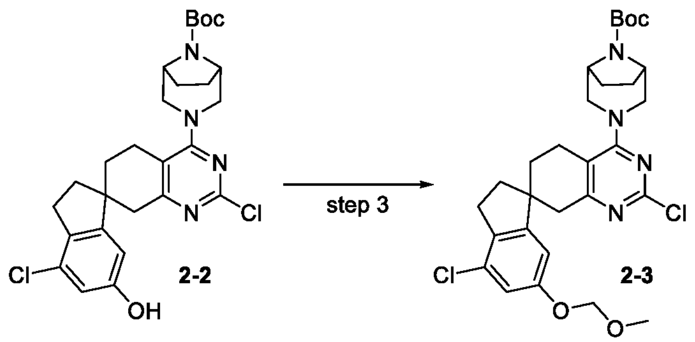


2-2 2-3

Under nitrogen atmosphere at 25°C with stirring, *N*,*N*-diisopropylethylamine (160.50 mg, 1.179 mmol, 3.00 eq) and chloromethyl methyl ether (41.28 mg, 0.590 mmol, 1.50 eq) were sequentially added dropwise to a solution of compound **2-2** (220 mg, 0.393 mmol, 1 eq) in dichloromethane (5 mL). The mixture was stirred at 25°C for 2 hour, with the reaction progress monitored by LC-MS and TLC. After the reaction was completed, the reaction mixture was concentrated under reduced pressure to obtain the crude product. The crude product was purified by silica gel column chromatography and subjected to a gradient elution with a mobile phase of 0% to 40% ethyl acetate/petroleum ether. The collected fraction was subjected to rotary evaporation under reduced pressure to remove the solvent, yielding compound **2-3** (white solid, 170 mg, yield of 71%). MS (ESI, m/z): 575.2/577.2/579.2 $[M + H]^+$.

Step 4:

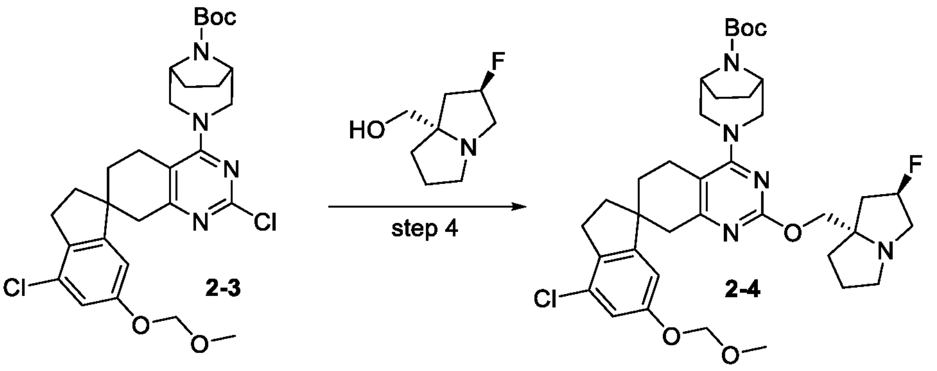


2-3 2-4

Under nitrogen atmosphere at 0°C with stirring, a solution of potassium *tert*-butoxide in tetrahydrofuran (1 M, 1.4 mL, 1.4 mmol, 5.0 eq) was added to a solution of compound **2-3** (170 mg, 0.28 mmol, 1.0 eq) and (2*R*,7a*S*)-2-fluorotetrahydro-1*H*-pyrrolizin-7a(5*H*)-methanol (188 mg, 1.12 mmol, 4.0 eq) in 1,4-dioxane (5 mL). The mixture was reacted with stirring at 90°C for 1 hour, with the reaction progress monitored by LC-MS and TLC. After the reaction was completed, the reaction mixture was cooled to room temperature. At 0°C, water (30 mL) was added to the mixture to quench the reaction. The resulting mixture was extracted with ethyl acetate (30 mL × 3). The organic phases were combined, dried over anhydrous sodium sulfate, and filtered. The filtrate was concentrated to obtain the crude product. The crude product was purified by silica gel column chromatography and subjected to a gradient elution with a mobile phase of 0% to 10% methanol/dichloromethane. The collected fraction was subjected to rotary evaporation under reduced pressure to remove the solvent, yielding compound **2-4** (white solid, 170 mg, yield of 82%). MS (ESI, m/z): 698.3/700.3 $[M+H]^+$.

Step 5:

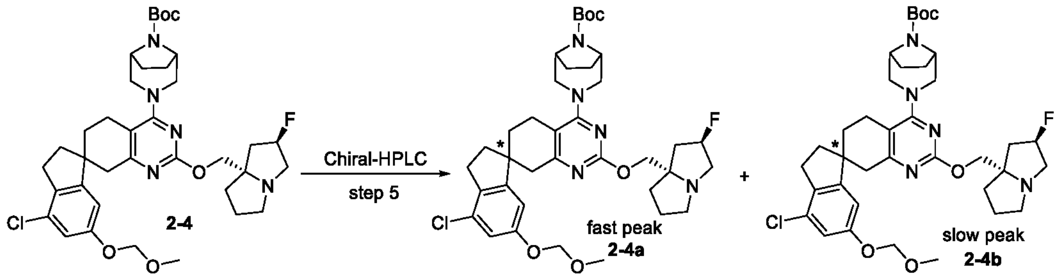


2-4 2-4a 2-4b

The compound **2-4** (170 mg) obtained from step 4 was subjected to chiral resolution by preparative chiral high-performance liquid chromatography: chiral column (*R*, *R*)-WHELK-O1-Kromasil, 3 × 25 cm, 5 μm; mobile phase A: n-hexane (0.1% diethylamine), mobile phase B: isopropanol; flow rate: 40 mL/min; elution with 30% phase B over 79 minutes; detector: UV 210/259 nm, resulting in two products. The product with a shorter retention time (42 minutes) was compound **2-4a** (white solid, 66 mg, yield of 40%), MS (ESI, m/z): 698.3/700.3 $[M+H]^+$. The product with a longer retention time (63 minutes) was compound **2-4b** (white solid, 64 mg, yield of 39%), MS (ESI, m/z): 698.3/700.3 $[M+H]^+$.

Step 6:

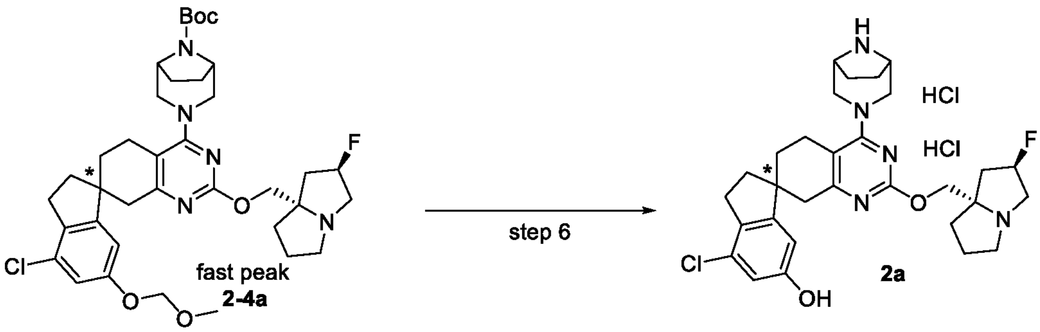


With stirring at 0°C, a solution of hydrogen chloride in 1,4-dioxane (4 M, 2 mL) was slowly added to a solution of compound **2-4a** (66 mg, 0.094 mmol, 1.00 eq) in methanol (2 mL). The reaction mixture was reacted at 25°C for 1.5 hour, with the reaction progress monitored by LC-MS and TLC. After the reaction was completed, the reaction mixture was concentrated under reduced pressure to obtain the crude product. The crude product was purified by reverse-phase chromatography (C18 column) and eluted with a mobile phase of 0% to 95% acetonitrile/water (0.1% hydrochloric acid) over 20 minutes, with detection at UV 254/220 nm. The product obtained was compound **2a** (white solid, 38.8 mg, yield of 63%). MS (ESI, m/z): 554.1/556.1 $[M + H]^+$; $^1H$ NMR (400 MHz, DMSO-$d_6$ + $D_2O$) $\delta$ 6.72 (d, $J$ = 2.0 Hz, 1H), 6.61 (d, $J$ = 2.1 Hz, 1H), 5.68 - 5.50 (m, 1H), 4.72 - 4.60 (m, 3H), 4.36 - 4.27 (m, 1H), 4.18 - 4.09 (m, 2H), 4.03 - 3.93 (m, 1H), 3.92 - 3.74 (m, 4H), 3.33 - 3.23 (m, 1H), 2.92 - 2.77 (m, 5H), 2.70 - 2.58 (m, 2H), 2.50 - 2.41 (m, 1H), 2.35 - 2.25 (m, 1H), 2.24 - 2.12 (m, 2H), 2.10 - 1.93 (m, 6H), 1.92 - 1.80 (m, 2H), 1.78 - 1.68 (m, 1H); $^{19}F$ NMR (377 MHz, DMSO-$d_6$) $\delta$ -171.82. The chiral analysis conditions for compound **2a** were as follows: (*S*, *S*)-Whelk-O1, 4.6 × 100 mm, 3.5 μm; mobile phase A: supercritical carbon dioxide; mobile phase B: methanol (20 mmol/L ammonia); flow rate: 2 mL/min; isocratic gradient elution with 40% phase B over 6.5 minutes; detector: UV 220 nm; retention time: 5.009 minutes; *dr* > 40:1.

Step 7:

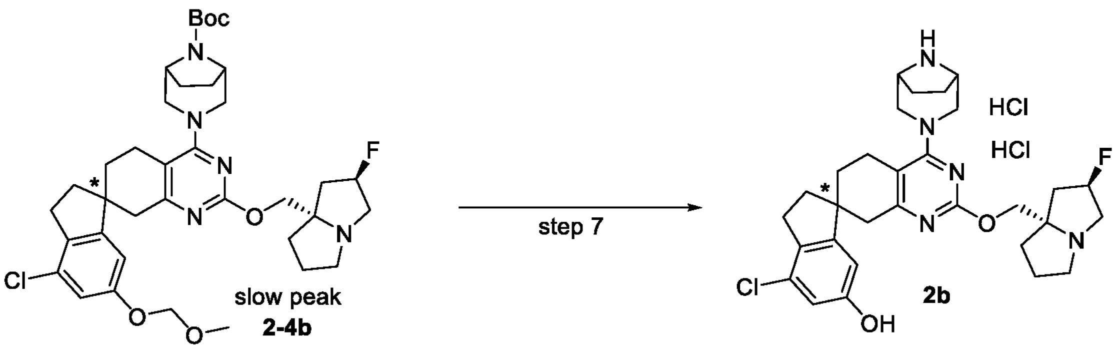


Compound **2b** (white solid, 33.6 mg, yield of 57%) could be obtained using the same method above. MS (ESI, m/z): 554.1/556.1 $[M + H]^+$; $^1H$ NMR (400 MHz, DMSO-$d_6$) $\delta$ 12.13 - 11.99 (m, 1H), 10.18 - 10.08 (m, 1H), 9.82 - 9.73 (m, 1H), 6.72 (d, $J$ = 2.0 Hz, 1H), 6.60 (d, $J$ = 2.0 Hz, 1H), 5.68 - 5.51 (m, 1H), 4.76 - 4.70 (m, 1H), 4.66 - 4.56 (m, 2H), 4.36 - 4.27 (m, 1H), 4.15 - 4.06 (m, 2H), 4.04 - 3.86 (m, 2H), 3.84 - 3.71 (m, 2H), 3.70 - 3.62 (m, 1H), 3.30 - 3.19 (m, 1H), 2.93 - 2.78 (m, 5H), 2.68 - 2.53 (m, 2H), 2.48 - 2.37 (m, 1H), 2.31 - 2.23 (m, 1H), 2.22 - 2.12 (m, 2H), 2.09 - 1.93 (m, 6H), 1.91 - 1.77 (m, 2H), 1.76 - 1.67 (m, 1H); $^{19}F$ NMR (377 MHz, DMSO-$d_6$) $\delta$ -171.76. The chiral analysis conditions for compound **2b** were as follows: (*S*, *S*)-Whelk-O1, 4.6 × 100 mm, 3.5 μm; mobile phase A: supercritical carbon dioxide; mobile phase B: methanol (20 mmol/L ammonia); flow rate: 2 mL/min; isocratic gradient elution with 40% phase B over 6.5 minutes; detector: UV 220 nm; retention time: 4.193 minutes; *dr* > 40:1.

**Example 3**

4-((5a*S*,6*R*,9*S*)-1-Fluoro-13-((*R*)-1-(2-fluoroethyl)pyrrolizin-2-yl)methoxy)-5a,6,7,8,9,10-hexahydro-5*H*-6,9-epiminoazepino[2',1':3,4][1,4]oxazepino[5,6,7-*de*]quinazolin-2-yl)naphthalen-2-ol dihydrochloride **3**

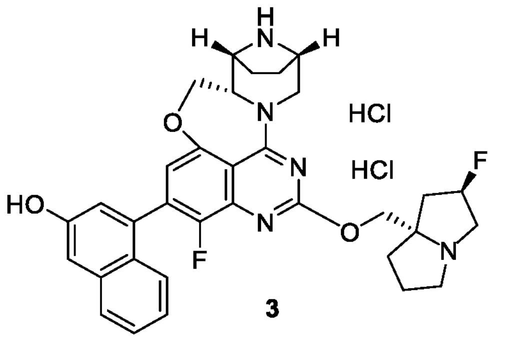

3

The synthetic route is as follows:

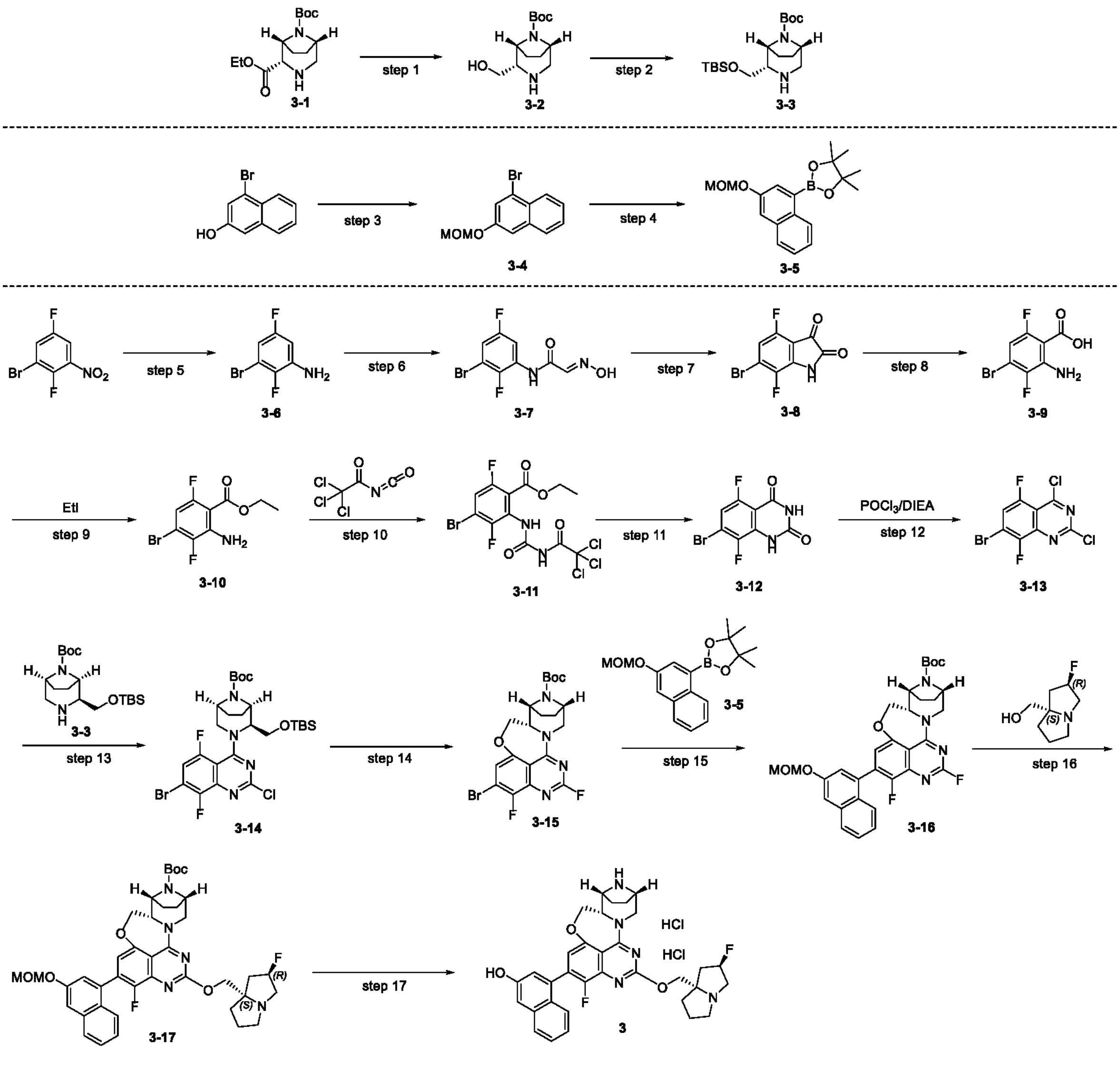


Step 1:

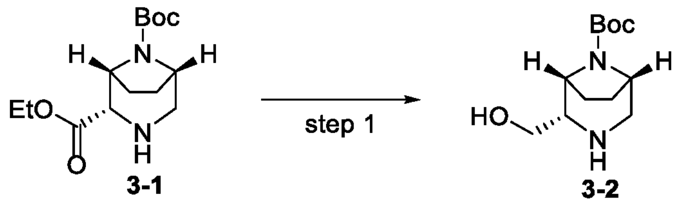


3-1 3-2

Compound **3-1** was synthesized with reference to patent WO2019179515A1.

Under nitrogen atmosphere at -40°C with stirring, a solution of diisobutylaluminum hydride in *n*-hexane (1 M, 13.36 mL, 13.36 mmol, 4.0 eq) was slowly added to a solution of compound **3-1** (1 g, 3.34 mmol, 1.0 eq) in anhydrous tetrahydrofuran (10 mL). The mixture was reacted with stirring at -40°C for 2 hours, with the reaction progress monitored by LC-MS and TLC. After the reaction was completed, the reaction was quenched by adding saturated potassium sodium tartrate solution (50 mL) to the reaction mixture at 0°C. The mixture was extracted with dichloromethane (50 mL × 3). The organic phases were combined, dried over anhydrous sodium sulfate, and filtered to remove the desiccant. The filtrate was concentrated under reduced pressure to obtain the crude product. The resulting crude product was purified by silica gel column chromatography and subjected to a gradient elution with a mobile phase of 0% to 10% ammonia in methanol (7 M)/dichloromethane. The collected fraction was subjected to rotary evaporation under reduced pressure to remove the solvent, yielding compound **3-2** (white solid, 500 mg, yield of 58%). MS (ESI, m/z): 243.3 $[M + H]^+$; $^1H$ NMR (400 MHz, $CDCl_3$) $\delta$ 4.20 - 3.94 (m, 2H), 3.59 - 3.55 (m, 1H), 3.45 - 3.40 (m, 1H), 3.10 - 2.93 (m, 2H), 2.74 - 2.70 (m, 1H), 1.96 - 1.82 (m, 2H), 1.79 - 1.70 (m, 2H), 1.47 (s, 9H).

Step 2:

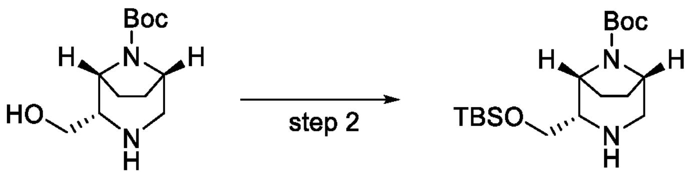


3-2 3-3

Under nitrogen atmosphere at 0°C with stirring, *tert*-butyldimethylsilyl chloride (313 mg, 1.976 mmol, 1.2 eq) was slowly added to a solution of compound **3-2** (420 mg, 1.647 mmol, 1.0 eq) and imidazole (141 mg, 1.976 mmol, 1.2 eq) in dichloromethane (5 mL). The mixture was reacted with stirring at 25°C for 2 hours, with the reaction progress monitored by LC-MS and TLC. After the reaction was completed, the mixture was concentrated under reduced pressure to obtain the crude product. The resulting crude product was purified by silica gel column chromatography and subjected to a gradient elution with a mobile phase of 0% to 5% methanol/dichloromethane. The collected fraction was subjected to rotary evaporation under reduced pressure to remove the solvent, yielding compound **3-3** (white solid, 500 mg, yield of 81%). MS (ESI, m/z): 357.2 $[M + H]^+$; $^1H$ NMR (400 MHz, $CDCl_3$) $\delta$ 4.28 - 4.00 (m, 2H), 3.62 - 3.40 (m, 2H), 3.10 - 2.94 (m, 2H), 2.78 - 2.68 (m, 1H), 1.97 - 1.85 (m, 3H), 1.79 - 1.67 (m, 2H), 1.47 (s, 9H), 0.89 (s, 9H), 0.06 (s, 6H).

Step 3:

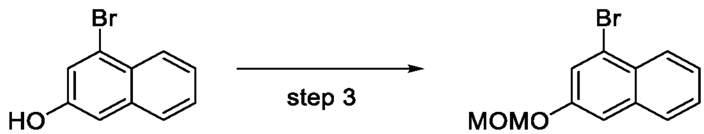


3-4

Under nitrogen atmosphere at 0°C with stirring, *N*,*N*-diisopropylethylamine (8.69 g, 66.57 mmol, 1.5 eq) and chloromethyl methyl ether (4.69 g, 57.69 mmol, 1.3 eq) were added to a solution of 1-bromo-3-hydroxynaphthalene (10 g, 44.38 mmol, 1.0 eq) in dichloromethane (100 mL). The mixture was reacted at 25°C for 3 hours, with the reaction progress monitored by LC-MS and TLC. After the reaction was completed, the reaction mixture was concentrated to obtain the crude product. The crude product was purified by silica gel column chromatography and subjected to a gradient elution with a

mobile phase of 0% to 10% ethyl acetate/petroleum ether. The collected fraction was subjected to rotary evaporation under reduced pressure to remove the solvent, yielding compound **3-4** (white solid, 10.5 g, yield of 87%). MS (ESI, *m*/*z):* 267.1/269.1 [M + H]$^+$; $^1$H NMR (300 MHz, $CDCl_3$) $\delta$ 8.16 - 8.11 (m, 1H), 7.75 - 7.71 (m, 1H), 7.57 (d,*J* = 2.4 Hz, 1H), 7.50 - 7.43 (m, 2H), 7.39 (d, *J* = 2.4 Hz, 1H), 5.28 (s, 2H), 3.52 (s, 3H).

Step 4:

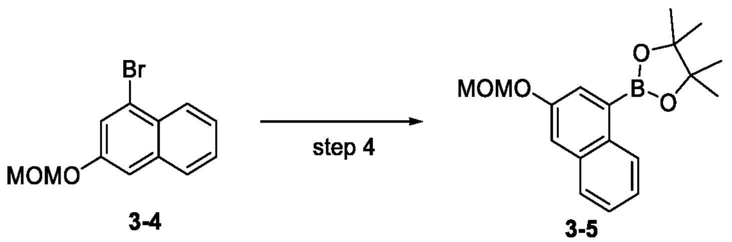


**3-4** **3-5**

Under nitrogen atmosphere at 25°C with stirring, potassium acetate (14.70 g, 142.26 mmol, 4.0 eq), bis(pinacolato)diboron (12.36 g, 46.23 mmol, 1.3 eq), and [1,1'-bis(diphenylphosphino)ferrocene]dichloropalladium(II) (3.05 g, 3.55 mmol, 0.1 eq) were sequentially added to a solution of compound **3-4** (10 g, 35.56 mmol, 1.0 equivalent) in 1,4-dioxane (100 mL). The mixture was reacted under nitrogen atmosphere at 100°C for 1 hour, with the reaction progress monitored by LC-MS and TLC. After the reaction was completed, the mixture was cooled to 25°C. The reaction mixture was concentrated under reduced pressure to obtain the crude product. The crude product was purified by silica gel column chromatography and subjected to a gradient elution with a mobile phase of 0% to 20% ethyl acetate/petroleum ether. The collected fraction was subjected to rotary evaporation under reduced pressure to remove the solvent, yielding compound **3-5** (white solid, 10 g, yield of 85%). MS (ESI, *m*/*z):* 315.2 [M + H]$^+$; $^1$H NMR (300 MHz, $CDCl_3$) $\delta$ 8.72 - 8.66 (m, 1H), 7.82 (d, *J* = 2.7 Hz, 1H), 7.79 - 7.73 (m, 1H), 7.51 (d, *J* = 2.7 Hz, 1H), 7.49 - 7.40 (m, 2H), 5.33 (s, 2H), 3.54 (s, 3H), 1.44 (s, 12H).

Step 5:

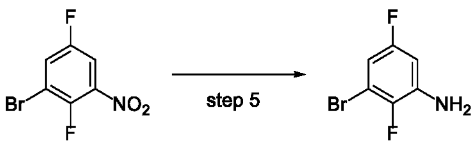


**3-6**

With stirring at 25°C, ammonium chloride (100.02 g, 1776.35 mmol, 5 eq) was added in batches to a mixed solution of 3-bromo-2,5-difluoronitrobenzene (89.0 g, 355.27 mmol, 1.0 eq) and iron powder (104.42 g, 1776.35 mmol, 5 eq) in ethanol (1200 mL) and water (240 mL). The mixture was reacted with stirring at 25°C for 16 hours, with the reaction progress monitored by LC-MS and TLC. After the reaction was completed, the mixture was filtered to remove the insoluble material, and the filter cake was washed with ethanol (500 mL × 3). The filtrate was concentrated under reduced pressure to obtain the crude product. The crude product was purified by silica gel column chromatography and subjected to a gradient elution with a mobile phase of 0% to 10% ethyl acetate/petroleum ether. The collected fraction was subjected to rotary evaporation under reduced pressure to remove the solvent, yielding compound **3-6** (orange oil, 44 g, yield of 56%). MS (ESI, m/z): 208.1/210.1 [M + H]$^+$; $^1$H NMR (400 MHz, DMSO-$d_6$) $\delta$ 6.66 - 6.60 (m, 1H), 6.58 - 6.50 (m, 1H), 5.80 (s, 2H).

Step 6:

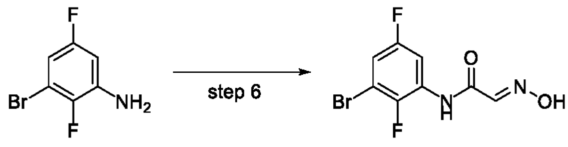


3-6 3-7

With stirring at 25°C, sodium sulfate (390.6 g, 2612.42 mmol, 13 eq) and hydroxylamine hydrochloride (44.1 g, 602.86 mmol, 3 eq) were added to a mixed solution of compound **3-6** (44 g, 200.95 mmol, 1.0 eq) and chloral hydrate (38.48 g, 221.05 mmol, 1.1 eq) in sulfuric acid (220 mL) and water (924 mL). The mixture was reacted with stirring at 70°C for 16 hours, with the reaction progress monitored by LC-MS and TLC. After the reaction was completed, the mixture was cooled to room temperature. The mixture was filtered, and the filter cake was washed with water (500 mL × 3) to obtain the crude product compound **3-7** (orange solid, 54 g). The crude product was directly used in the next reaction without further purification. MS (ESI, m/z): 277.1/279.1 $[M+H]^-$.

Step 7:

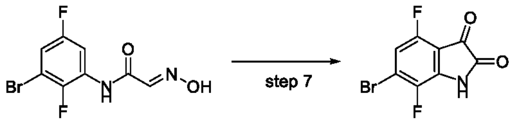


3-7 3-8

At 25°C, compound **3-7** (54 g, 172.23 mmol, 1.0 eq) was dissolved in sulfuric acid (475 mL). The mixture was reacted with stirring at 90°C for 1 hour, with the reaction progress monitored by TLC. After the reaction was completed, the mixture was cooled to room temperature. The mixture was quenched by pouring it into ice water. The mixture was filtered, and the filter cake was washed with water (500 mL × 3) to obtain the crude product of compound **3-8** (brown solid, 38 g). The crude product was directly used in the next reaction without further purification.

Step 8:

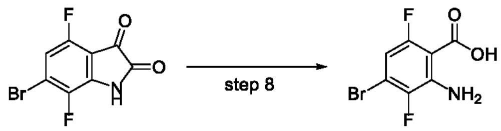


3-8 3-9

With stirring at 25°C, hydrogen peroxide (30%, 646 mL) was slowly added to an aqueous sodium hydroxide solution (2 M, 76 mL) of compound **3-8** (38 g, 137.78 mmol, 1.0 eq. The mixture was reacted with stirring at 25°C for 16 hours, with the reaction progress monitored by LC-MS and TLC. After the reaction was completed, the pH of the reaction mixture was adjusted to 7 using dilute hydrochloric acid. The mixture was then filtered, and the filter cake was washed with water (500 mL × 3). The combined filtrate was adjusted to pH 1 with dilute hydrochloric acid to precipitate a solid, and filtered. The filter cake was washed with water (500 mL × 3), yielding the crude product of compound **3-9** (gray solid, 20.4 g). The crude product was directly used in the next reaction without further purification. MS (ESI, m/z): 250.1/252.1 $[M+H]^-$.

Step 9:

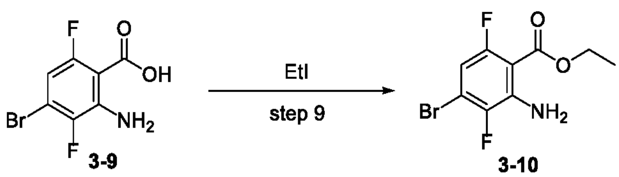


3-9 3-10

Under nitrogen atmosphere at 0°C, iodoethane (3.34 g, 20.35 mmol, 1.2 eq) was slowly added to a solution of compound **3-9** (4.5 g, 16.96 mmol, 1.0 eq) and cesium carbonate (11.64 g, 33.92 mmol, 2 eq) in *N,N*-dimethylformamide (45 mL). The mixture was reacted with stirring at 25°C for 4 hours, with the reaction progress monitored by LC-MS and TLC. After the reaction was completed, water (400 mL) was added to dilute the reaction mixture. The resulting mixture was extracted with ethyl acetate (500 mL × 3). The organic phases were combined, washed with saturated brine (500 mL × 3), dried over anhydrous sodium sulfate, and filtered to remove the desiccant. The filtrate was concentrated under reduced pressure to obtain the crude product. The crude product was purified by silica gel column chromatography and subjected to a gradient elution with a mobile phase of 0% to 50% ethyl acetate/petroleum ether. The collected fraction was subjected to rotary evaporation under reduced pressure to remove the solvent, yielding compound **3-10** (orange oil, 3.92 g, yield of 78%). MS (ESI, m/z): 280.1/282.2 $[M + H]^+$, $^1H$ NMR (300 MHz, $CDCl_3$) $\delta$ 6.62 - 6.53 (m, 1H), 4.49 - 4.34 (m, 2H), 1.52 - 1.32 (m, 3H).

Step 10:

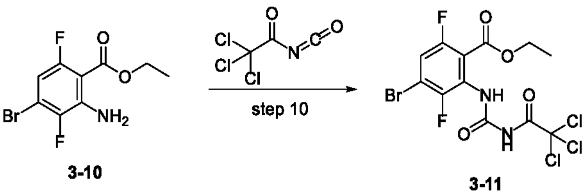


3-10 3-11

Under nitrogen atmosphere at 25°C, trichloroacetyl isocyanate (3.96 g, 19.94 mmol, 1.5 eq) was slowly added to a solution of compound **3-10** (3.92 g, 13.29 mmol, 1.0 eq) in tetrahydrofuran (40 mL). The mixture was reacted with stirring at 25°C for 0.5 hours, with the reaction progress monitored by LC-MS and TLC. After the reaction was completed, the reaction mixture was concentrated under reduced pressure to obtain the crude product. The crude product was slurried with methyl tert-butyl ether (500 mL) to obtain compound **3-11** (white solid, 6.5 g, yield of 99%). MS (ESI, m/z): 467.0/469.0/471.0 $[M + H]^+$; $^1H$ NMR (300 MHz, DMSO-$d_6$) $\delta$ 11.76 (s, 1H), 9.88 (s, 1H), 7.95 - 7.83 (m, 1H), 4.29 (q, $J$ = 7.1 Hz, 2H), 1.26 (t, $J$ = 7.1 Hz, 3H).

Step 11:

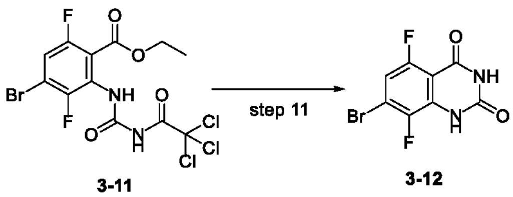


3-11 3-12

With stirring at 25°C, a solution of ammonia in methanol (7 M, 7 mL) was slowly added to a solution of compound **3-11** (6.5 g, 13.18 mmol, 1.0 eq) in methanol (70 mL). The mixture was reacted with stirring at 25°C for 1 hour, with the reaction progress monitored by LC-MS and TLC. After the reaction was completed, the reaction mixture was concentrated under reduced pressure to obtain the crude product. The crude product was slurried with methyl tert-butyl ether (200 mL) to obtain compound **3-12** (white solid, 3.92 g, yield of 99%). MS (ESI, m/z): 277.2/279.2 $[M + H]^+$; $^1H$ NMR (300 MHz, DMSO-$d_6$) $\delta$ 11.67 - 11.41 (m, 2H), 7.47 - 7.36 (m, 1H).

Step 12:

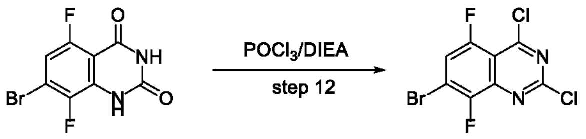


3-12 3-13

With stirring at 0°C, *N,N*-diisopropylethylamine (5 mL, 27.2 mmol, 3.18 eq) was slowly added to a solution of compound **3-12** (2.5 g, 8.57 mmol, 1.0 eq) in phosphorus oxychloride (47.5 mL). The mixture was reacted with stirring at 90°C for 3 hours, with the reaction progress monitored by TLC. After the reaction was completed, the reaction mixture was concentrated under reduced pressure to obtain the crude product. The crude product was dispersed in 50 mL of dichloromethane and then concentrated to remove the solvent, yielding the crude product. The crude product was purified by silica gel column chromatography and subjected to a gradient elution with a mobile phase of 0% to 10% ethyl acetate/petroleum ether. The collected fraction was subjected to rotary evaporation under reduced pressure to remove the solvent, yielding compound **3-13** (white solid, 1 g, yield of 35%). $^1$H NMR (400 MHz, $CDCl_3$) $\delta$ 7.62 - 7.53 (m, 1H).

Step 13:

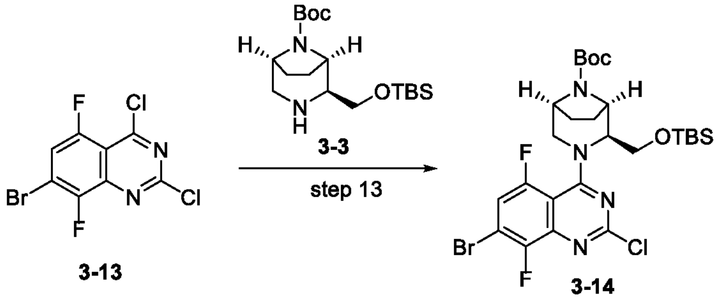


3-13 3-14

With stirring at 0°C, *N,N*-diisopropylethylamine (434 mg, 3.2 mmol, 3 eq) was slowly added to a solution of compound **3-13** (352 mg, 1.06 mmol, 1.0 eq) and compound **3-3** (400 mg, 1.06 mmol, 3.0 eq) in dichloromethane (5 mL). The mixture was reacted with stirring at 25°C for 1 hour, with the reaction progress monitored by LC-MS and TLC. After the reaction was completed, the reaction mixture was concentrated under reduced pressure to obtain the crude product. The crude product was purified by silica gel column chromatography and subjected to a gradient elution with a mobile phase of 0% to 20% ethyl acetate/petroleum ether. The collected fraction was subjected to rotary evaporation under reduced pressure to remove the solvent, yielding compound **3-14** (white solid, 550 mg, yield of 77%). MS (ESI, m/z): 633.2/635.2/637.2 [M + H]$^+$.

Step 14:

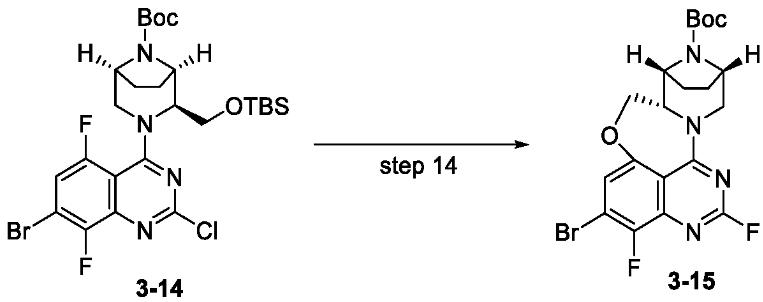


3-14 3-15

With stirring at 25°C, a solution of tetrabutylammonium fluoride in tetrahydrofuran (1 M, 1.75 mL, 1.75 mmol, 2.2 eq) was slowly added to a solution of compound **3-14** (530 mg, 0.794 mmol, 1.0 eq) in anhydrous tetrahydrofuran (6 mL). The mixture was reacted with stirring at 25°C for 1 hour, then heated to 65°C and stirred for an additional 2 hours. The reaction progress was monitored by LC-MS and TLC. After the reaction was completed, the reaction mixture was

concentrated under reduced pressure to obtain the crude product. The crude product was purified by silica gel column chromatography and subjected to a gradient elution with a mobile phase of 0% to 20% ethyl acetate/petroleum ether. The collected fraction was subjected to rotary evaporation under reduced pressure to remove the solvent, yielding compound **3-15** (white solid, 120 mg, yield of 29%). MS (ESI, m/z): 483.0/485.0 $[M + H]^+$; $^1H$ NMR (300 MHz, $CDCl_3$) $\delta$ 7.12 (d, $J$ = 5.8 Hz, 1H), 5.19 - 4.98 (m, 1H), 4.51 - 4.34 (m, 2H), 4.32 - 4.06 (m, 3H), 3.33 - 3.15 (m, 1H), 2.08 - 1.72 (m, 4H), 1.53 (s, 9H).

Step 15:

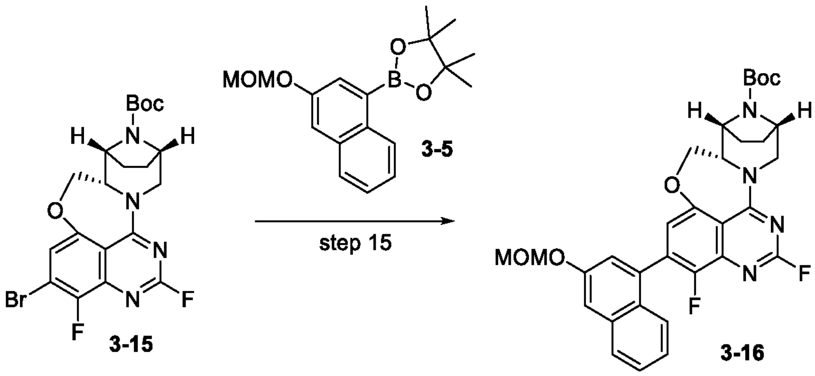


3-15 3-5 3-16

Under nitrogen atmosphere at 25°C with stirring, compound **3-15** (120 mg, 0.236 mmol, 1.0 eq), compound **3-5** (93 mg, 0.283 mmol, 1.2 eq), potassium phosphate (105 mg, 0.472 mmol, 2.0 eq), 3-(*tert*-butyl)-4-(2,6-dimethoxyphenyl)-2,3-dihydrobenzo[*D*][1,3]oxaphosphole (16 mg, 0.047 mmol, 0.2 eq), tris(dibenzylideneacetone)dipalladium (22 mg, 0.024 mmol, 0.1 eq), toluene (2 mL), and water (0.4 mL) were sequentially added to a three-neck flask. The mixture was reacted at 80°C for 2 hours, with the reaction progress monitored by LC-MS and TLC. After the reaction was completed, the system was cooled to room temperature. The reaction mixture was concentrated under reduced pressure to obtain the crude product. The crude product was purified by silica gel column chromatography and subjected to a gradient elution with a mobile phase of 0% to 40% ethyl acetate/petroleum ether. The collected fraction was subjected to rotary evaporation under reduced pressure to remove the solvent, yielding compound **3-16** (white solid, 120 mg, yield of 29%). MS (ESI, m/z): 591.2 $[M + H]^+$.

Step 16:

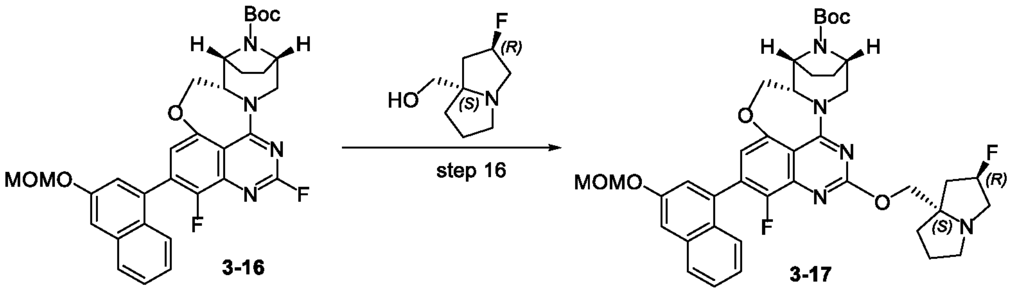


3-16 3-17

Under nitrogen atmosphere at 25°C with stirring, compound **3-16** (100 mg, 0.161 mmol, 1.0 eq), triethylenediamine (3.8 mg, 0.032 mmol, 0.2 eq), cesium carbonate (110 mg, 0.322 mmol, 2.0 eq), (2*R*,7a*S*)-2-fluorotetrahydro-1*H*-pyrrolizin-7a(5*H*)-methanol (40 mg, 0.241 mmol, 1.5 eq), and *N*,*N*-dimethylformamide (2 mL) were sequentially added to a reaction flask. The mixture was reacted at 100°C for 6 hours, with the reaction progress monitored by LC-MS and TLC. After the reaction was completed, the reaction mixture was diluted by adding 20 mL of water, and then extracted with ethyl acetate (20 mL × 3), and the organic phases were combined. The organic phases were further washed with saturated brine (20 mL × 2), dried over anhydrous sodium sulfate, and filtered to remove the desiccant. The filtrate was concentrated under reduced pressure to obtain the crude product. The resulting crude product was purified by silica gel column chromatography and subjected to a gradient elution with a mobile phase of 0% to 10% methanol/dichloromethane. The

collected fraction was subjected to rotary evaporation under reduced pressure to remove the solvent, yielding compound **3-17** (white solid, 60 mg, yield of 48%). MS (ESI, m/z): 730.1 [M + H]$^+$.

Step 17:

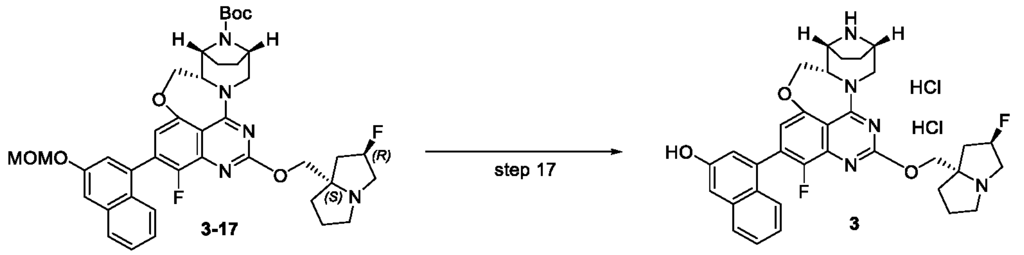


3-17 3

With stirring at 0°C, compound **3-17** (50 mg, 0.065 mmol, 1.0 eq), methanol (1.0 mL), and a solution of hydrochloric acid in 1,4-dioxane (4 M, 1.0 mL) were sequentially added to a reaction flask. The mixture was reacted with stirring at 25°C for 2 hours, with the reaction progress monitored by LC-MS and TLC. After the reaction was completed, the reaction mixture was concentrated under reduced pressure to obtain the crude product. The crude product was purified by high-performance liquid chromatography: XSelect CSH Prep C18 OBD column (19 × 150 mm, 5 μm); mobile phase A: water (0.05% hydrochloric acid), mobile phase B: acetonitrile; flow rate: 25 mL/min; elution with 8% to 26% mobile phase B; detector: UV 220/254 nm. The product obtained was compound **3** (yellow solid, 30 mg, yield of 69%). MS (ESI, m/z): 586.1 [M + H]$^+$; $^1$H NMR (400 MHz, DMSO-*d*$_6$) δ 11.54 - 11.41 (m, 1H), 10.43 - 9.75 (m, 3H), 7.80 (d, *J* = 8.3 Hz, 1H), 7.48 - 7.35 (m, 2H), 7.31 - 7.20 (m, 2H), 7.16 - 7.09 (m, 1H), 6.91 (d, *J* = 6.0 Hz, 1H), 5.69 - 5.45 (m, 1H), 5.07 - 4.92 (m, 1H), 4.72 - 4.48 (m, 5H), 4.33 - 4.21 (m, 2H), 3.88 - 3.83 (m, 1H), 3.81 - 3.74 (m, 2H), 3.71 - 3.63 (m, 1H), 3.36 - 3.23 (m, 1H), 2.69 - 2.53 (m, 1H), 2.48 - 2.42 (m, 1H), 2.37 - 2.28 (m, 1H), 2.23 - 2.10 (m, 3H), 2.09 - 1.84 (m, 4H); $^{19}$F NMR (377 MHz, DMSO-*d*$_6$) δ -133.35, -133.54, -172.72.

**Example 4**

(*S* or *R*)-4-((5a*S*,6*R*,9*S*)-1,3-Difluoro-13-((2*R*,7a*S*)-2-fluorotetrahydro-1*H*-pyrrolizin-7a(5*H*)-ylmethoxy)-5a,6,7,8,9,10-hexahydro-5*H*-6,9-epiminoazepino[2',1':3,4][1,4]oxazepino[5,6,7-*de*]quinazolin-2-yl)-5-ethynyl-6-fluoronaphthalen-2-ol **4a'**; (*R* or*S*)-4-((5a*S*,6*R*,9*S*)-1,3-difluoro-13-((2*R*,7a*S*)-2-fluorotetrahydro-1*H*-pyrrolizin-7a(5*H*)-ylmethoxy)-5a,6,7,8,9,10-hexahydro-5*H*-6,9-epiminoazepino[2',1':3,4][1,4]oxazepino[5,6,7-*de*]quinazolin-2-yl)-5-ethynyl-6-fluoronaphthalen-2-ol **4b'**

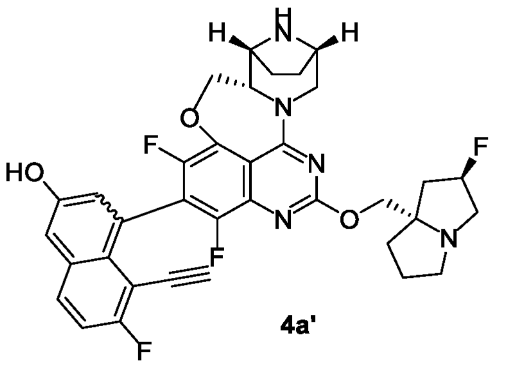

4a'

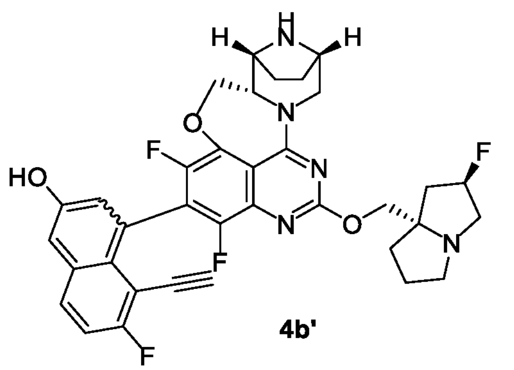

4b'

The synthetic route is as follows:

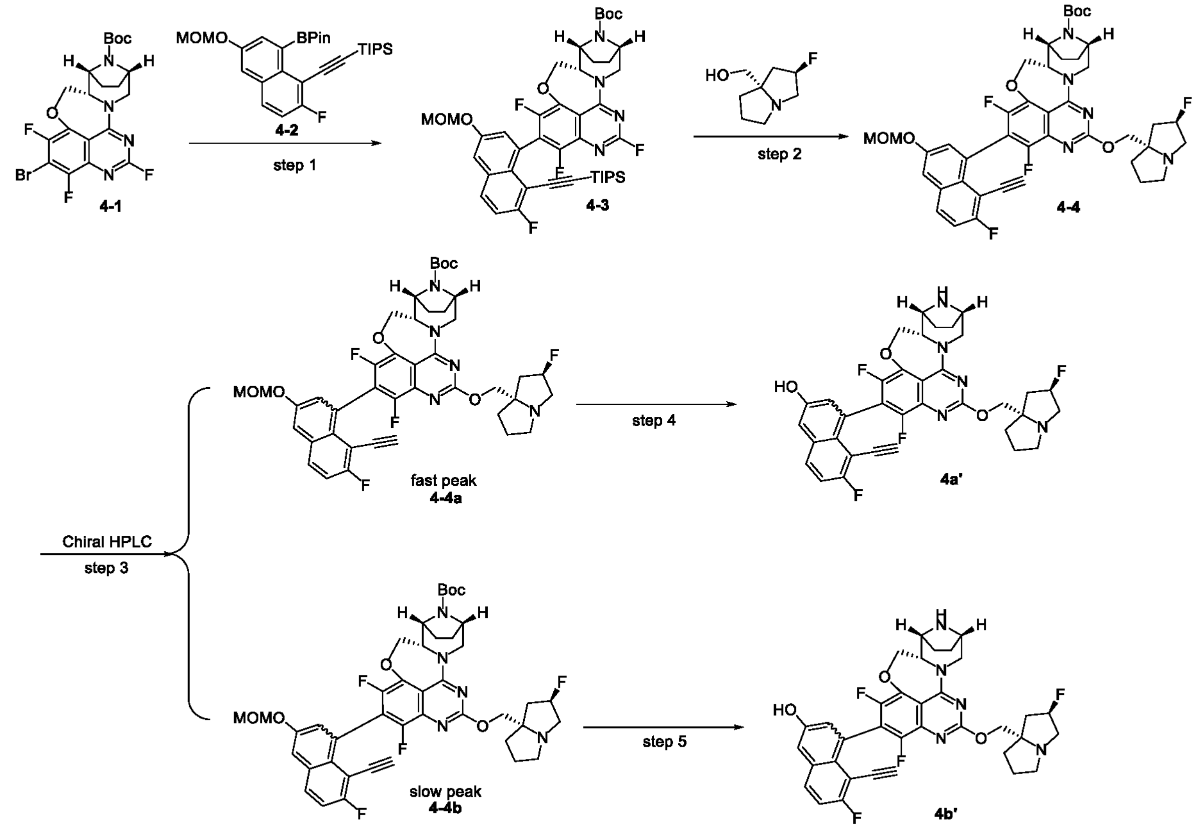


Step 1:

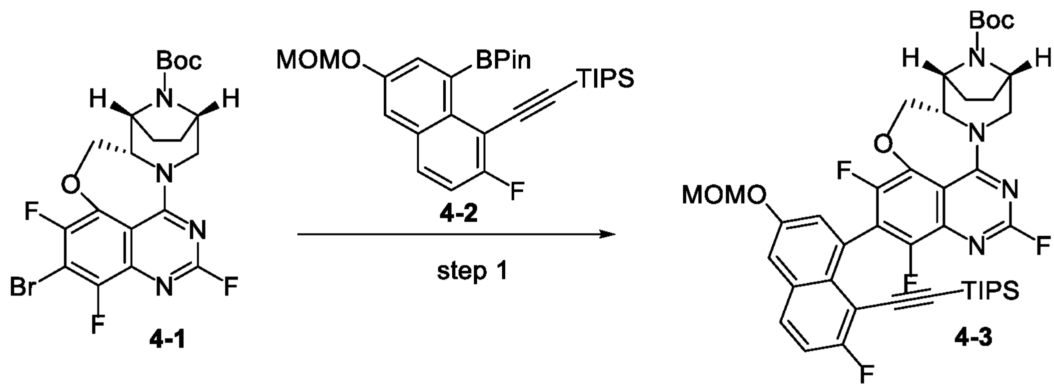


Compound **4-1** was synthesized with reference to Example 3.

Compound **4-2** was synthesized with reference to patent WO2021041671.

Under nitrogen atmosphere at 25°C with stirring, compound **4-1** (240 mg, 0.455 mmol, 1.0 eq), compound **4-2** (294 mg, 0.546 mmol, 1.2 eq), potassium phosphate (203 mg, 0.91 mmol, 2.0 eq), 3-(*tert*-butyl)-4-(2,6-dimethoxyphenyl)-2,3-dihydrobenzo[D][1,3]oxaphosphole (31 mg, 0.091 mmol, 0.2 eq), tris(dibenzylideneacetone)dipalladium (43 mg, 0.046 mmol, 0.1 eq), toluene (4 mL), and water (0.8 mL) were sequentially added to a three-neck flask. The resulting mixture was reacted at 80°C for 1 hour, with the reaction progress monitored by LC-MS and TLC. After the reaction was completed, the system was cooled to room temperature. The reaction mixture was concentrated under reduced pressure to obtain the crude product. The crude product was purified by silica gel column chromatography and subjected to a gradient elution with a mobile phase of 0% to 40% ethyl acetate/petroleum ether. The collected fraction was subjected to rotary evaporation under reduced pressure to remove the solvent, yielding compound **4-3** (white solid, 300 mg, yield of 77%). MS (ESI, m/z): 807.3 $[M + H]^+$.

Step 2:

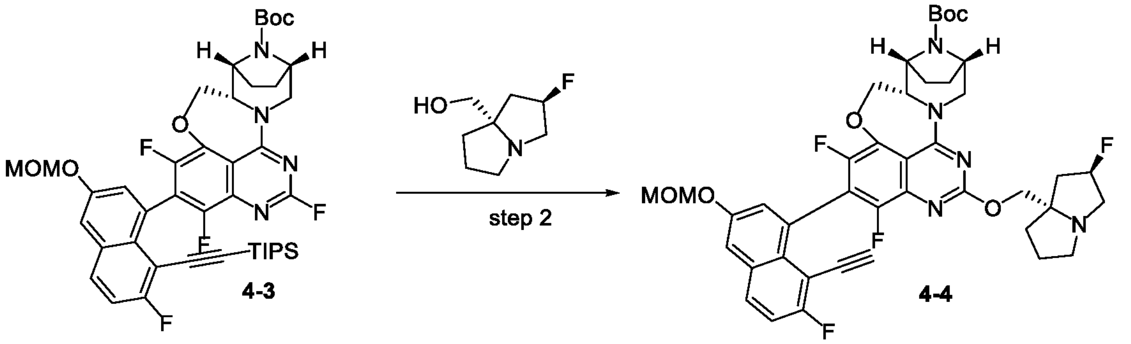


4-3 4-4

Under nitrogen atmosphere at 25°C with stirring, compound **4-3** (270 mg, 0.318 mmol, 1.0 eq), triethylenediamine (7 mg, 0.064 mmol, 0.2 eq), cesium carbonate (218 mg, 0.636 mmol, 2.0 eq), (2*R*,7a*S*)-2-fluorotetrahydro-1*H*-pyrrolizin-7a(5*H*)-methanol (64 mg, 0.382 mmol, 1.2 eq), and *N*,*N*-dimethylformamide (3 mL) were sequentially added to a reaction flask. The mixture was reacted at 100°C for 2 hours, with the reaction progress monitored by LC-MS and TLC. After the reaction was completed, the reaction mixture was diluted with 30 mL of water and then extracted with ethyl acetate (30 mL × 3), and the organic phases were combined. The organic phases were further washed with saturated brine (30 mL × 2), dried over anhydrous sodium sulfate, and filtered to remove the desiccant. The filtrate was concentrated under reduced pressure to obtain the crude product. The crude product was purified by high-performance liquid chromatography: column: XBridge Prep OBD C18, 30 × 150 mm, 5 μm; mobile phase A: water (10 mmol/L ammonium bicarbonate), mobile phase B: acetonitrile; flow rate: 60 mL/min; elution with 51% to 80% mobile phase B; detector: UV 220/254 nm. The product obtained was compound **4-4** (white solid, 70 mg, yield of 27%). MS (ESI, m/z): 790.3 $[M + H]^+$.

Step 3:

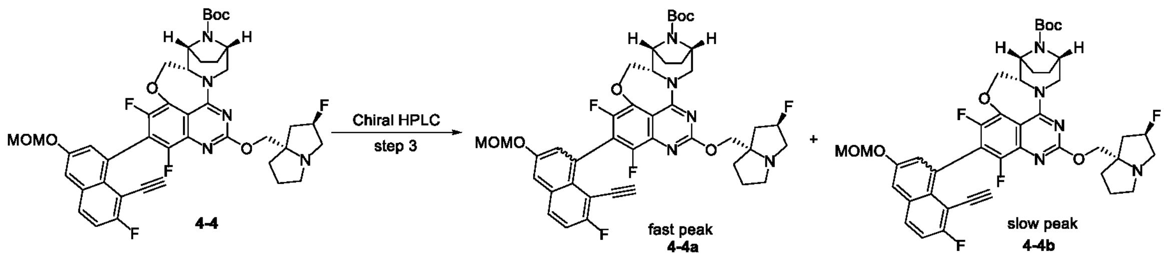


4-4 fast peak 4-4a + slow peak 4-4b

The compound **4-4** (70 mg) obtained from step **2** was subjected to chiral resolution by preparative high-performance liquid chromatography: chiral column (*R*, *R*)-WHELK-O1-Kromasil, 2.11 × 25 cm, 5 μm; mobile phase A: n-hexane (10 mmol/L ammonia in methanol), mobile phase B: isopropanol; flow rate: 25 mL/min; elution with 50% phase B over 35 minutes; detector: UV 206/232 nm, resulting in two products. The product with a shorter retention time (20.5 minutes) was compound **4-4a** (white solid, 23 mg, yield of 32%), MS (ESI, m/z): 790.3 $[M + H]^+$. The product with a longer retention time (26 minutes) was compound **4-4b** (white solid, 30 mg, yield of 42%), MS (ESI, m/z): 790.3 $[M + H]^+$.

Step 4:

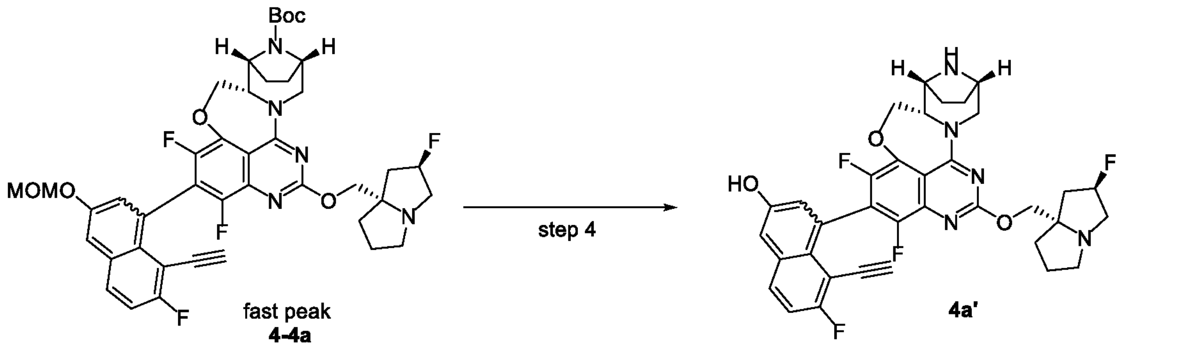


fast peak
4-4a

4a'

With stirring at 0°C, compound **4-4a** (20 mg, 0.024 mmol, 1.0 eq), methanol (1.5 mL), and a solution of hydrochloric acid in 1,4-dioxane (4 M, 1.5 mL) were sequentially added to a reaction flask. The mixture was reacted with stirring at 0°C for 2 hours, with the reaction progress monitored by LC-MS and TLC. After the reaction was completed, the reaction mixture was concentrated under reduced pressure to obtain the crude product.

At 0°C, a solution of ammonium in methanol (7 M, 1 mL) was added to the crude product. The mixture was reacted with stirring at 0°C for 10 minutes, then concentrated under reduced pressure to obtain the free crude product. The crude product was purified by reverse-phase chromatography (C18 column) and eluted with a mobile phase of 5% to 95% acetonitrile/water (0.1% ammonium bicarbonate) over 25 minutes, with detection at UV 254/220 nm. The product obtained was compound **4a'** (white solid, 6 mg, yield of 38%). MS (ESI, *m/z*): 646.0 $[M + H]^+$, $^1H$ NMR (300 MHz, DMSO-$d_6$) $\delta$ 10.24 (s, 1H), 8.04 - 7.94 (m, 1H), 7.54 - 7.44 (m, 1H), 7.41 (d, *J* = 2.6 Hz, 1H), 7.15 (d, *J* = 2.5 Hz, 1H), 5.41 - 5.17 (m, 1H), 4.78 - 4.69 (m, 1H), 4.62 - 4.53 (m, 1H), 4.44 - 4.32 (m, 1H), 4.11 - 4.02 (m, 2H), 4.01 - 3.92 (m, 2H), 3.64 - 3.54 (m, 1H), 3.52 - 3.44 (m, 1H), 3.18 - 2.97 (m, 4H), 2.90 - 2.71 (m, 1H), 2.20 - 1.95 (m, 3H), 1.92 - 1.50 (m, 7H); $^{19}F$ NMR (282 MHz, DMSO-$d_6$) $\delta$ -110.18, -133.98, -141.52, -172.18. The chiral analysis conditions for compound **4a'** were as follows: column: Optichiral C9-3, 3 × 100 mm, 3 μm; mobile phase A: supercritical carbon dioxide fluid; mobile phase B: methanol (20 mmol/L ammonia); flow rate: 2 mL/min; isocratic gradient elution with 50% phase B over 2 minutes; detector: UV 230 nm; retention time: 0.836 minutes; *dr* > 40:1.

Step 5:

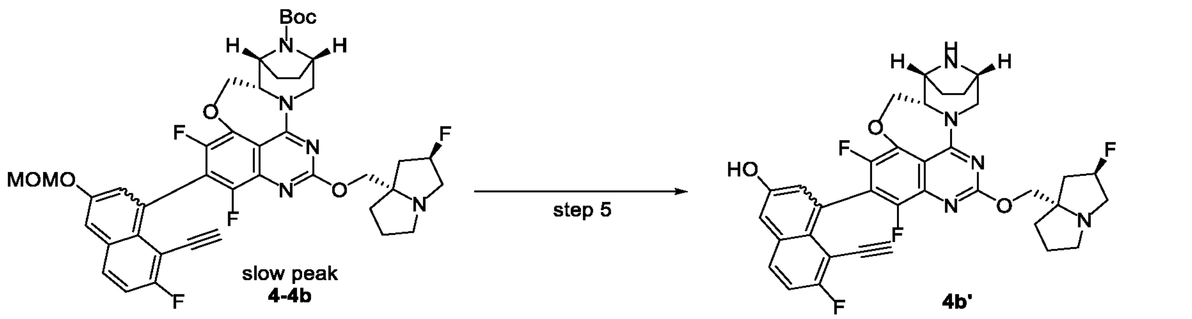


slow peak
4-4b

4b'

Compound **4b'** (white solid, 9.3 mg, yield of 32%) could be obtained using the same method above. MS (ESI, m/z): 646.0 $[M + H]^+$; $^1H$ NMR (300 MHz, DMSO-$d_6$ + $D_2O$) $\delta$ 8.02 - 7.94 (m, 1H), 7.53 - 7.45 (m, 1H), 7.43 (d, *J* = 2.6 Hz, 1H), 7.16 (d, *J* = 2.6 Hz, 1H), 5.69 - 5.43 (m, 1H), 5.13 - 5.02 (m, 1H), 4.77 - 4.67 (m, 1H), 4.61 - 4.40 (m, 4H), 4.31 - 4.20 (m, 2H), 4.07 - 4.02 (m, 1H), 3.88 - 3.72 (m, 3H), 3.54 - 3.43 (m, 1H), 3.36 - 3.23 (m, 1H), 2.62 - 2.54 (m, 1H), 2.47 - 2.41 (m, 1H), 2.38 - 2.26 (m, 1H), 2.25 - 1.87 (m, 7H); $^{19}F$ NMR (377 MHz, DMSO-$d_6$) $\delta$ -110.00, -133.12, -139.43, -172.80. The chiral analysis conditions for compound **4b'** were as follows: column: Optichiral C9-3, 3 × 100 mm, 3 μm; mobile phase A: supercritical carbon dioxide fluid; mobile phase B: methanol (20 mmol/L ammonia); flow rate: 2 mL/min; isocratic gradient elution with 50% phase B over 2 minutes; detector: UV 230 nm; retention time: 1.233 minutes; *dr* > 13:1.

**Example 5**

(*S* or *R*)-4-((5a*S*,6*S*,9*R*)-1,3-Difluoro-13-(((2*R*,7a*S*)-2-fluorotetrahydro-1*H*-pyrrolizin-7a(5*H*)-yl)methoxy)-5a,6,7,8,9,10-hexahydro-5*H*-6,9-epiminoazepino[2',1':3,4][1,4]oxazepino[5,6,7-*de*]quinazolin-2-yl)-5-ethylnaphthalen-2-ol dihydrochloride 5a; (*R* or *S*)-4-((5a*S*,6*S*,9*R*)-1,3-difluoro-13-(((2*R*,7a*S*)-2-fluorotetrahydro-1*H*-pyrrolizin-7a(5*H*)-yl)methoxy)-5a,6,7,8,9,10-hexahydro-5*H*-6,9-epiminoazepino[2',1':3,4][1,4]oxazepino[5,6,7-*de*]quinazolin-2-yl)-5-ethylnaphthalen-2-ol dihydrochloride **5b**

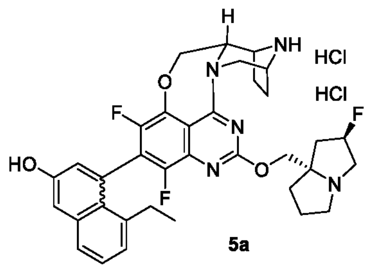

**5a**

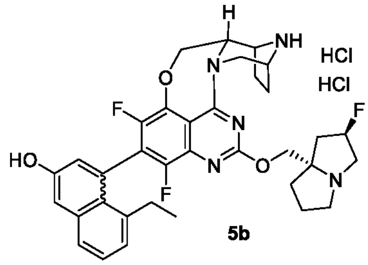

**5b**

The synthetic route is as follows:

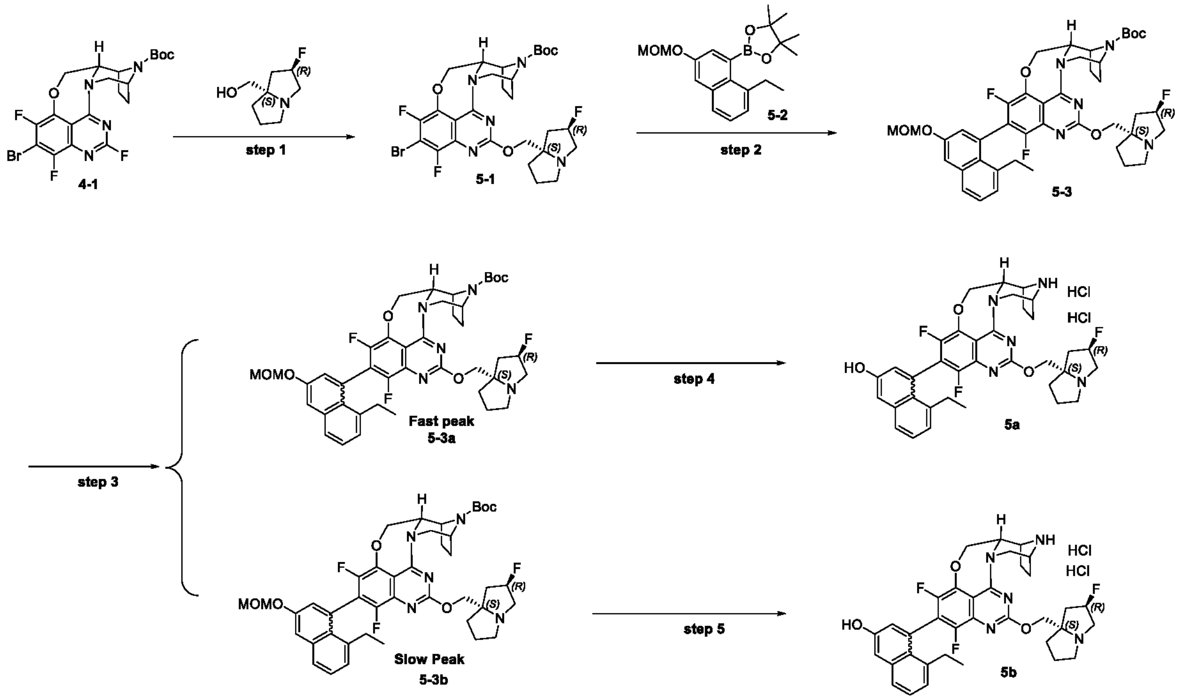


**Step 1:**

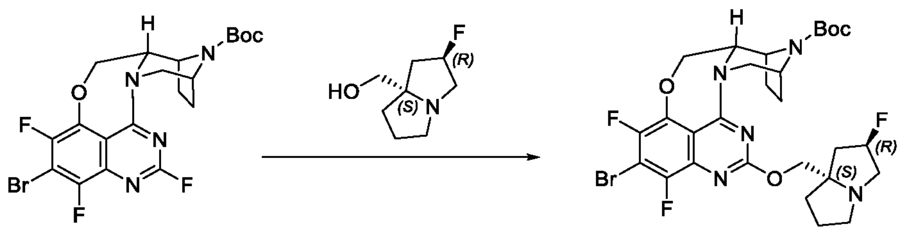

**4-1** **5-1**

Under nitrogen atmosphere at 25°C with stirring, compound **4-1** (320 mg, 0.60 mmol, 1.0 eq), triethylenediamine (13.74 mg, 0.12 mmol, 0.2 eq), cesium carbonate (399.16 mg, 1.21 mmol, 2.0 eq), (2*R*,7a*S*)-2-fluorotetrahydro-1*H*-pyrrolizin-7a(5*H*)-methanol (121.9 mg, 0.728 mmol, 1.2 eq), and *N,N*-dimethylformamide (4 mL) were sequentially added to a reaction flask. The mixture was reacted at 60°C for 16 hours, with the reaction progress monitored by LC-MS and TLC. After the reaction was completed, the reaction mixture was concentrated. The resulting crude product was purified by reverse-phase chromatography (C18 column) and eluted with a mobile phase of 10% to 90% acetonitrile/water (0.1% ammonium bicarbonate) over 20 minutes, with detection at UV 254/220 nm. The product obtained was compound **5-1** (white solid, 280 mg, yield of 68%). MS (ESI, m/z): 640.0/642.0 $[M + H]^{+}$.

**Step 2:**

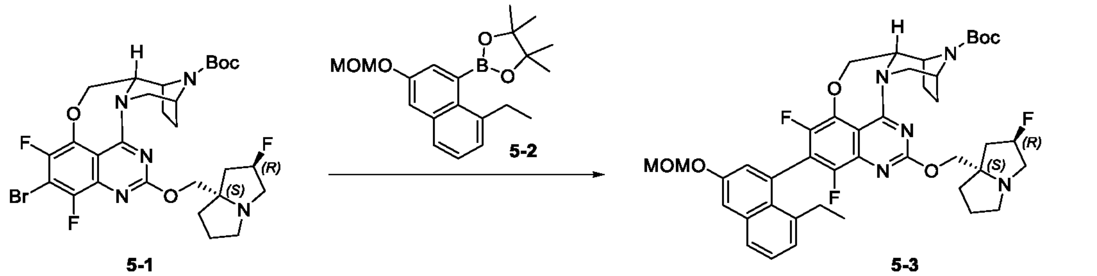

5-1 5-2 5-3

Compound **5-2** was synthesized with reference to patent WO2019179515A1.

Under nitrogen atmosphere at 25°C with stirring, compound **5-1** (80 mg, 0.12 mmol, 1.0 eq), compound **5-2** (54.50 mg, 0.15 mmol, 1.2 eq), potassium phosphate (56.34 mg, 0.25 mmol, 2.0 eq), 3-(*tert*-butyl)-4-(2,6-dimethoxyphenyl)-2,3-dihydrobenzo[D][1,3]oxaphosphole (8.7 mg, 0.02 mmol, 0.2 eq), tris(dibenzylideneacetone)dipalladium (12.15 mg, 0.01 mmol, 0.1 eq), toluene (1 mL), and water (0.2 mL) were sequentially added to a 25 mL Schlenk tube. The resulting mixture was reacted at 80°C for 16 hours, with the reaction progress monitored by LC-MS and TLC. After the reaction was completed, the reaction mixture was cooled to room temperature, then concentrated under reduced pressure to obtain the crude product. The resulting crude product was purified by silica gel column chromatography and subjected to a gradient elution with a mobile phase of 0% to 10% methanol/dichloromethane. The collected fraction was subjected to rotary evaporation under reduced pressure to remove the solvent, yielding compound **5-3** (white solid, 100 mg, yield of 97%). MS (ESI, m/z): 776.3 $[M + H]^{+}$.

**Step 3:**

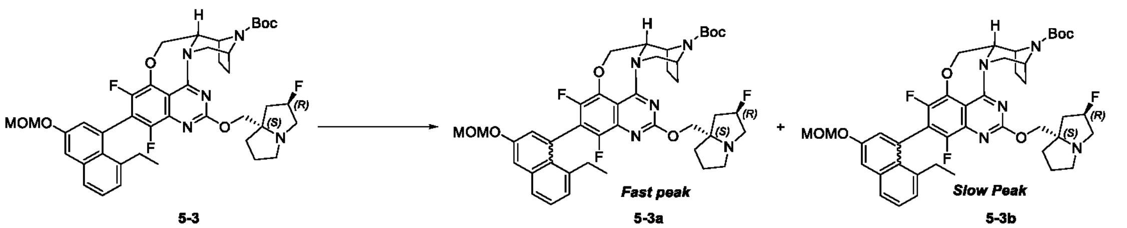


5-3 5-3a 5-3b

The compound **5-3** (100 mg) obtained from step **2** was subjected to chiral resolution using supercritical fluid chromatography (SFC): chiral column: NB_CHIRALPAK AD-H, 3 × 25 cm, 5 μm; mobile phase A: supercritical carbon dioxide, mobile phase B: isopropanol; flow rate: 75 mL/min; elution with 55% mobile phase B; detector: UV 224/292 nm, resulting in two products. The product with a shorter retention time (2.95 minutes) was compound **5-3a** (white solid, 40 mg, yield of 40%), MS (ESI, m/z): 776.3 $[M + H]^{+}$. The product with a longer retention time (6.50 minutes) was compound **5-3b** (white solid, 40 mg, yield of 40%), MS (ESI, m/z): 776.3 $[M + H]^{+}$.

**Step 4:**

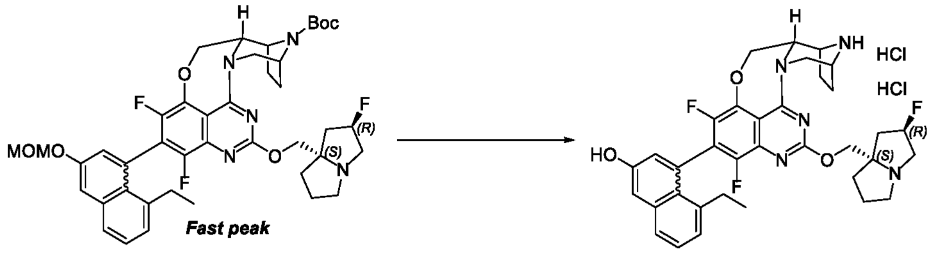


5-3a 5a

With stirring at 0°C, a solution of hydrogen chloride in 1,4-dioxane (4 M, 1 mL) was slowly added to a solution of compound **5-3a** (40 mg, 0.04 mmol, 1.00 eq) in methanol (1 mL). The mixture was reacted at 25°C for 1 hour, with the reaction progress monitored by LC-MS and TLC. After the reaction was completed, the mixture was concentrated under reduced pressure to obtain the crude product. The resulting crude product was purified by reverse-phase chromatography (C18 column) and eluted with a mobile phase of 0% to 30% acetonitrile/water (0.1% hydrochloric acid) over 20 minutes, with detection at UV 254/220 nm. The product obtained was compound **5a** (yellow solid, 30.0 mg, yield of 85%). MS (ESI, *m/z*): 632.3 $[M + H]^+$; $^1H$ NMR (400 MHz, DMSO-$d_6$ + $D_2O$) $\delta$ 7.74 - 7.65 (m, 1H), 7.44 - 7.38 (m, 1H), 7.34 (d, *J* = 2.6 Hz, 1H), 7.21 - 7.14 (m, 1H), 6.97 (d, *J* = 2.6 Hz, 1H), 5.70 - 5.47 (m, 1H), 5.12 - 5.02 (m, 1H), 4.78 - 4.67 (m, 1H), 4.66 - 4.45 (m, 4H), 4.37 - 4.24 (m, 2H), 3.91 - 3.84 (m, 1H), 3.83 - 3.76 (m, 2H), 3.62 - 3.54 (m, 1H), 3.37 - 3.24 (m, 1H), 2.67 - 2.55 (m, 1H), 2.51 - 2.29 (m, 4H), 2.26 - 1.86 (m, 7H), 0.95 - 0.81 (m, 3H); $^{19}F$ NMR (377 MHz, DMSO-$d_6$) $\delta$ -132.33, -139.34, -172.64. The chiral analysis conditions for compound **5a** were as follows: N-CHIRALPAK IC-3 (Lot No. IC3SCK-VK002), 3.0 × 100 mm, 3 μm; mobile phase A: supercritical carbon dioxide fluid; mobile phase B: methanol (20 mmol/L ammonia); flow rate: 2 mL/min; isocratic gradient elution with 50% phase B over 12 minutes; detector: UV 220 nm; retention time: 3.304 minutes; *dr* > 40:1.

**Step 5:**

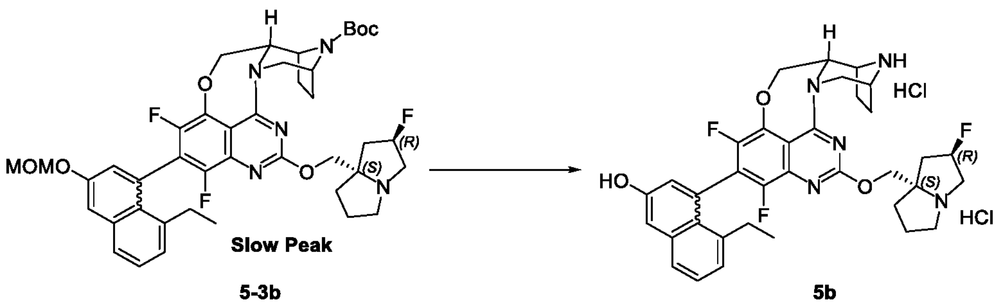


5-3b 5b

Compound **5b** (yellow solid, 32.0 mg, yield of 90%) could be obtained by using the same method as **step 4;** MS (ESI, *m/z):* 632.3 $[M + H]^+$; $^1H$ NMR (400 MHz, DMSO-$d_6$ + $D_2O$) $\delta$ 7.72 - 7.68 (m, 1H), 7.44 - 7.38 (m, 1H), 7.34 (d, *J* = 2.7 Hz, 1H), 7.19 - 7.14 (m, 1H), 6.98 (d, *J* = 2.6 Hz, 1H), 5.71 - 5.50 (m, 1H), 5.14 - 5.02 (m, 1H), 4.79 - 4.67 (m, 1H), 4.65 - 4.48 (m, 4H), 4.35 - 4.23 (m, 2H), 3.90 - 3.74 (m, 3H), 3.61 - 3.54 (m, 1H), 3.35 - 3.26 (m, 1H), 2.72 - 2.55 (m, 1H), 2.50 - 2.29 (m, 4H), 2.21 - 1.89 (m, 7H), 0.91 - 0.80 (m, 3H); $^{19}F$ NMR (377 MHz, DMSO-$d_6$) $\delta$ -132.26, -139.60, -172.72. The chiral analysis conditions for compound **5b** were as follows: N-CHIRALPAK IC-3 (Lot No.IC3SCK-VK002), 3.0 × 100 mm, 3 μm; mobile phase A: supercritical carbon dioxide fluid; mobile phase B: methanol (20 mmol/L ammonia); flow rate: 2 mL/min; isocratic gradient elution with 50% phase B over 12 minutes; detector: UV 220 nm; retention time: 2.199 minutes; *dr* > 40:1.

## Example 6

(*S* or *R*)-4'-((1*R*,5*S*)-3,8-Diazabicyclo[3.2.1]octan-3-yl)-4-chloro-2'-(((2*R*,7a*S*)-2-fluorotetrahydro-1*H*-pyrrolizin-7*a*(5*H*)-yl)methoxy)-6-hydroxy-5',8'-dihydro-6'*H*-spiro[indene-1,7'-quinazolin]-3(2*H*)-one dihydrochloride **6a;** (*R* or*S*)-4'-((1*R*,5*S*)-3,8-diazabicyclo[3.2.1]octan-3-yl)-4-chloro-2'-(((2*R*,7a*S*)-2-fluorotetrahydro-1*H*-pyrrolizin-7*a*(5*H*)-yl)methoxy)-6-hydroxy-5',8'-dihydro-6'*H*-spiro[indene-1,7'-quinazolin]-3(2*H*)-one dihydrochloride **6b**

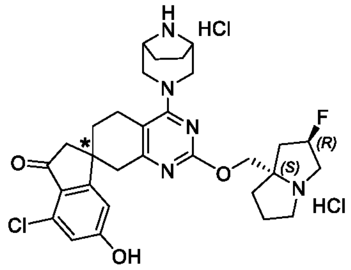

6a

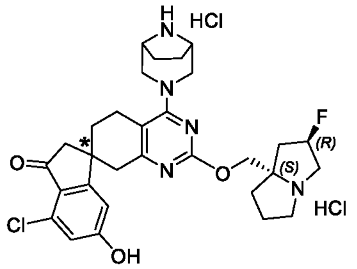

6b

The synthetic route is as follows:

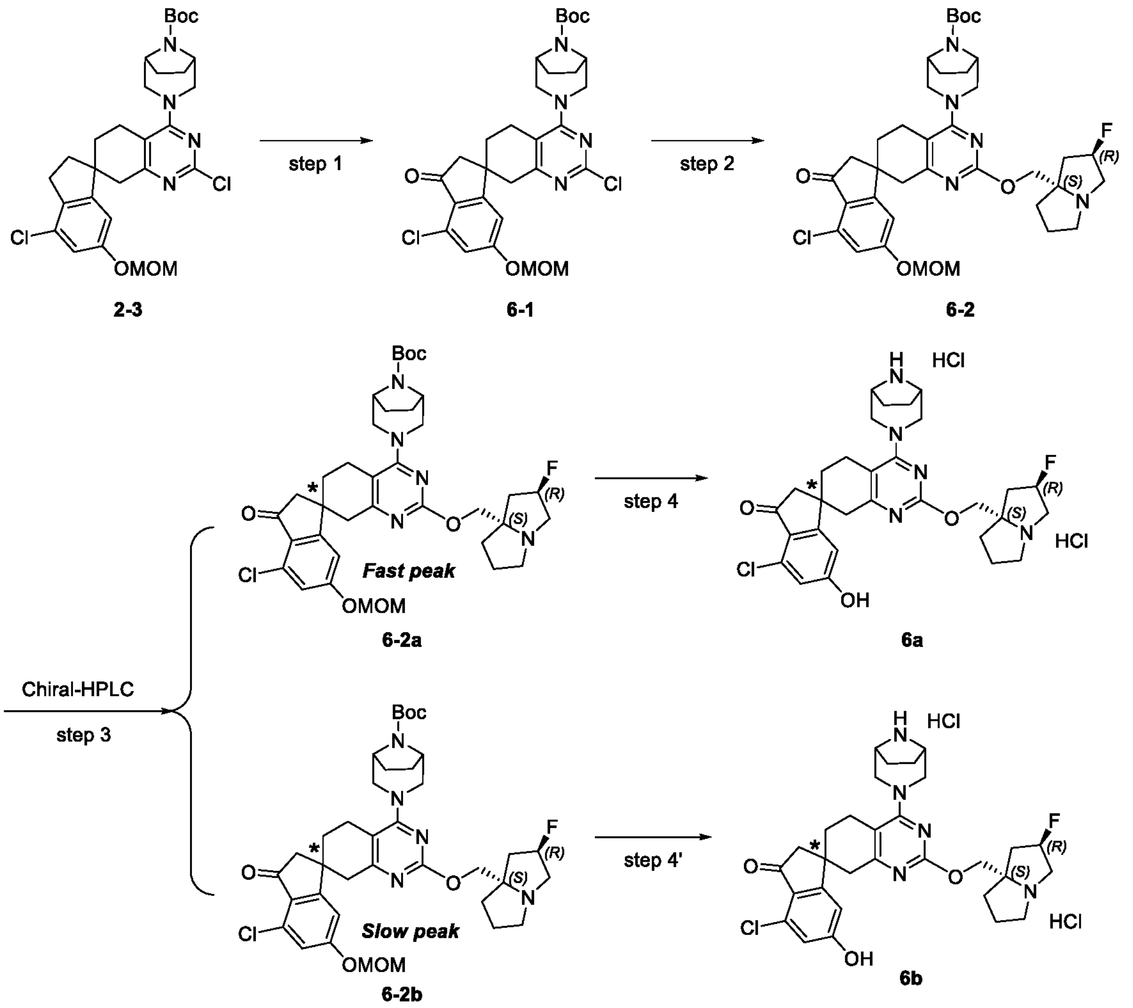


2-3

6-1

6-2

6-2a

6a

6-2b

6b

**Step 1:**

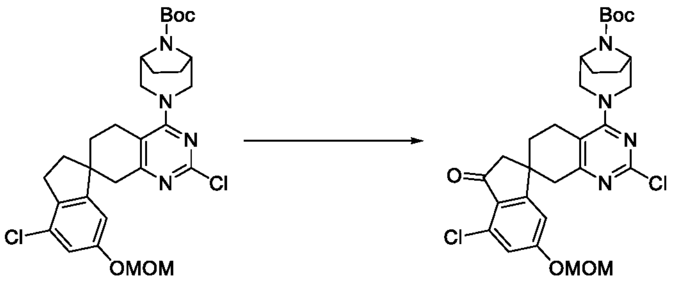

2-3

6-1

With stirring at 0°C, compound **2-3** (250 mg, 0.413 mmol, 1 eq), dichloromethane (4 mL), chromium trioxide (173.74 mg, 1.652 mmol, 4 eq), and *tert*-butyl hydroperoxide solution (70%, 2 mL) were sequentially added to a 25 mL single-neck flask. The mixture was reacted with stirring at 20°C for 16 hours, with the reaction progress monitored by LC-MS and TLC. After the reaction was completed, the mixture was filtered, and the filter cake was washed with dichloromethane (15 mL × 3). The combined filtrate was washed with water (40 mL), and the aqueous phase was extracted with dichloromethane (30 mL × 3). All organic phases were combined, dried over anhydrous sodium sulfate, and filtered to remove the desiccant. The filtrate was concentrated under reduced pressure to obtain the crude product. The crude product was purified by silica gel column chromatography and subjected to a gradient elution with a mobile phase of 0% to 60% ethyl acetate/petroleum ether. The collected fraction was subjected to rotary evaporation under reduced pressure to remove the solvent, yielding compound **6-1** (yellow solid, 140 mg, yield of 52%). MS(ESI, m/z): 589.0/591.0/593.0 $[M + H]^+$; $^1H$ NMR (400 MHz, $CDCl_3$) $\delta$ 7.07 - 7.04 (m, 1H), 6.93 - 6.88 (m, 1H), 5.24 - 5.17 (m, 2H), 4.42 - 4.23 (m, 2H), 4.16 - 4.06 (m, 1H), 3.72 - 3.64 (m, 1H), 3.50 - 3.44 (m, 4H), 3.22 - 3.11 (m, 2H), 2.96 - 2.87 (m, 1H), 2.76 - 2.55 (m, 5H), 2.01 - 1.91 (m, 5H), 1.49 (s, 9H).

**Step 2:**

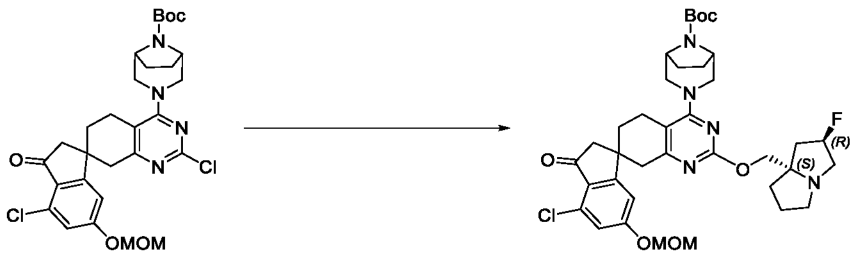

6-1

6-2

Under nitrogen atmosphere at 25°C with stirring, compound **6-1** (135 mg, 0.218 mmol, 1.0 eq), [(2*R*,7a*S*)-2-fluoro-hexahydropyrrolizin-7a-yl]methanol (145.83 mg, 0.872 mmol, 1.2 eq), 1,4-dioxane (2 mL), and a solution of potassium *tert*-butoxide in tetrahydrofuran (1 M, 1.09 mL, 1.09 mmol, 1.5 eq) were sequentially added to a 250 mL three-neck flask. The mixture was reacted with stirring under nitrogen atmosphere at 70°C for 3 hours. The reaction was monitored by LC-MS and TLC. After the reaction was completed, the reaction mixture was quenched with water (25 mL). The resulting mixture was extracted with ethyl acetate (30 mL × 3). The organic phases were combined, dried over anhydrous sodium sulfate, and filtered to remove the desiccant. The filtrate was concentrated under reduced pressure to obtain the crude product. The crude product was purified by reverse-phase chromatography (C18 column) and eluted with a mobile phase of 20% to 95% acetonitrile/water (10 mmol/L ammonium bicarbonate) over 25 minutes, with detection at UV 254/220 nm. The collected fraction was subjected to rotary evaporation under reduced pressure to remove the solvent, yielding compound **6-2** (white solid, 73 mg, yield of 46%). MS(ESI, m/z): 712.2/714.2 $[M + H]^+$.

**Step 3:**

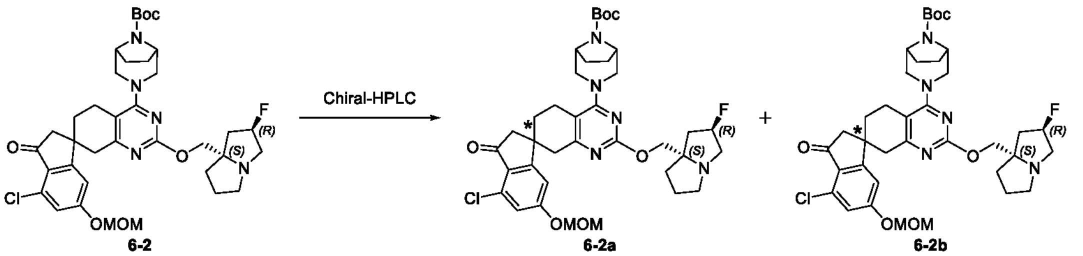


6-2 6-2a 6-2b

The compound **6-2** (35 mg) obtained from **step 2** was subjected to chiral resolution by preparative supercritical liquid chromatography: chiral column CHIRAL ART Cellulose-SB, 2 × 25 cm, 5 μm; mobile phase A: *n*-hexane (10 mmol/L ammonia in methanol), mobile phase B: ethanol; flow rate: 20 mL/min; elution with 20% phase B over 18 minutes; detector: UV 220/203 nm, resulting in two products. The product with a shorter retention time (10.61 minutes) was compound **6-2a** (white solid, 14 mg, yield of 39%), MS (ESI, m/z): 712.2/714.2 $[M + H]^+$. The product with a longer retention time (14.59 minutes) was compound **6-2b** (white solid, 12 mg, yield of 34%), MS (ESI, m/z): 712.2/714.2 $[M + H]^+$.

**Step 4:**

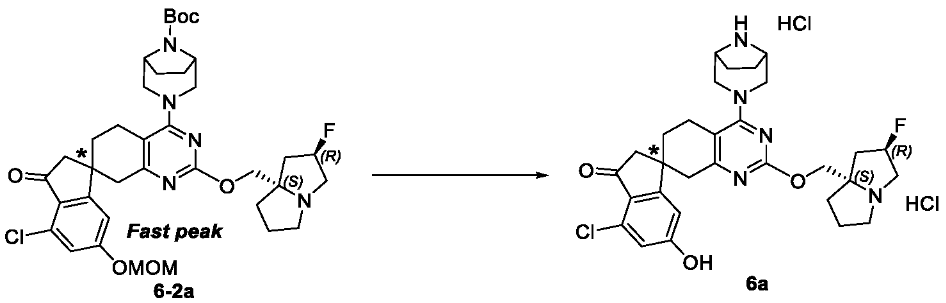


6-2a 6a

With stirring at 0°C, compound **6-2a** (12 mg, 0.016 mmol, 1 eq), methanol (0.5 mL), and a solution of hydrochloric acid in 1,4-dioxane (4 M, 0.5 mL) were sequentially added to a 25 mL single-neck flask. The reaction mixture was reacted with stirring at 25°C for 1.5 hours. The reaction progress was monitored by LC-MS and TLC. After the reaction was completed, the reaction mixture was concentrated under reduced pressure to obtain the crude product. The crude product was purified by reverse-phase chromatography (C18 column): mobile phase A: water (0.1% hydrochloric acid); mobile phase B: acetonitrile, elution with 5% to 95% phase B over 30 minutes; detector UV 220/254 nm. The collected fraction was subjected to rotary evaporation under reduced pressure to remove the solvent, yielding compound **6a** (yellow solid, 3.4 mg, yield of 32%). MS(ESI,m / z): 568.1/569.9 $[M + H]^+$; $^1H$ NMR (400 MHz, DMSO-$d_6$ + $D_2O$) $\delta$ 7.10 - 6.87 (m, 2H), 5.72 - 5.46 (m, 1H), 4.66 - 4.42 (m, 3H), 4.20 - 4.01 (m, 3H), 3.94 - 3.71 (m, 4H), 3.39 (s, 2H), 3.31 - 3.22 (m, 1H), 3.16 - 3.05 (m, 1H), 2.98 - 2.85 (m, 1H), 2.86 - 2.71 (m, 2H), 2.72 - 2.55 (m, 2H), 2.48 - 2.44 (m, 1H), 2.30 - 2.10 (m, 3H), 2.10 - 1.88 (m, 5H), 1.89 - 1.73 (m, 2H). The chiral analysis conditions for compound **6a** were as follows: N-CHIRALPAK IC-3 (Lot No. IC30CS-VF008), 4.6 × 100 mm, 3 μm; mobile phase A: supercritical carbon dioxide fluid; mobile phase B: methanol (20 mmol/L ammonia); flow rate: 2 mL/min; isocratic gradient elution with 50% phase B over 6 minutes; detector: UV 220 nm; retention time: 3.795 minutes; *dr* > 40:1.

**Step 4':**

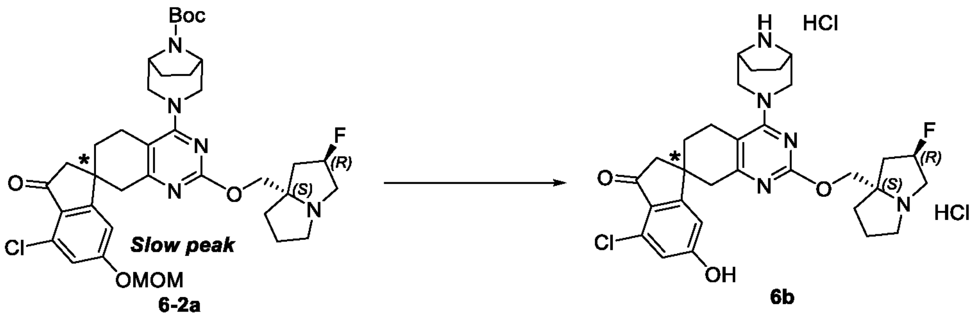


6-2a 6b

Compound **6b** (yellow solid, 3.3 mg, yield of 31%) was obtained by using the same method as **step 4.** MS(ESI, *m/z):* 568.2/569.9 [M + H]$^+$; $^1$H NMR (400 MHz, DMSO-$d_6$ + $D_2O$) $\delta$ 7.06 - 6.96 (m, 1H), 6.96 - 6.88 (m, 1H), 5.71 - 5.47 (m, 1H), 4.64 - 4.37 (m, 3H), 4.19 - 4.00 (m, 3H), 3.94 - 3.73 (m, 4H), 3.40 - 3.37 (m, 2H), 3.32 - 3.22 (m, 1H), 3.14 - 3.05 (m, 1H), 2.97 - 2.85 (m, 1H), 2.85 - 2.71 (m, 2H), 2.72 - 2.56 (m, 2H), 2.48 - 2.37 (m, 1H), 2.31 - 2.09 (m, 3H), 2.09 - 1.88 (m, 5H), 1.88 - 1.74 (m, 2H). The chiral analysis conditions for compound **6b** were as follows: N-CHIRALPAK IC-3 (Lot No.IC30CS-VF008), 4.6 × 100 mm, 3 μm; mobile phase A: supercritical carbon dioxide fluid; mobile phase B: methanol (20 mmol/L ammonia); flow rate: 2 mL/min; isocratic gradient elution with 50% phase B over 6 minutes; detector: UV 220 nm; retention time: 4.358 minutes; *dr* > 40:1.

## Example 7

*(1R* or 1*S*)-4'-((1*R*,5*S*)-3,8-Diazabicyclo[3.2.1]octan-3-yl)-4-chloro-2'-(((2*R*,7a*S*)-2-fluorotetrahydro-1*H*-pyrrolizin-7*a* (5*H*)-yl)methoxy)-3-methyl-2,3,5',8'-tetrahydro-6'*H*-spiro[indene-1,7'-quinazolin]-6-ol dihydrochloride **7a;** (1*R* or 1*S*, 3*R* or 3*S*)-4'-((1*R*,5*S*)-3,8-diazabicyclo[3.2.1]octan-3-yl)-4-chloro-2'-(((2*R*,7a*S*)-2-fluorotetrahydro-1*H*-pyrrolizin-7a(5*H*)-yl)methoxy)-3-methyl-2,3,5',8'-tetrahydro-6'*H*-spiro[indene-1,7'-quinazolin]-6-ol dihydrochloride **7aa;** (1*R* or 1*S,* 3*S* or 3*R*)-4'-((1*R*,5*S*)-3,8-diazabicyclo[3.2.1]octan-3-yl)-4-chloro-2'-(((2*R*,7a*S*)-2-fluorotetrahydro-1*H*-pyrrolizin-7a(5*H*)-yl)methoxy)-3-methyl-2,3,5',8'-tetrahydro-6'*H*-spiro[indene-1,7'-quinazolin]-6-ol dihydrochloride **7ab**

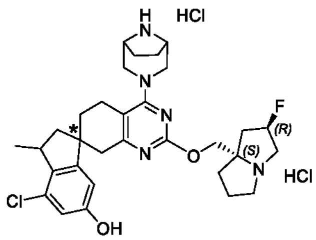

7a

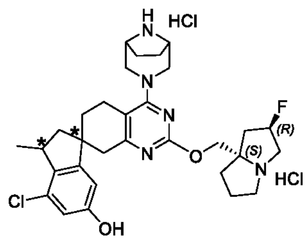

7aa

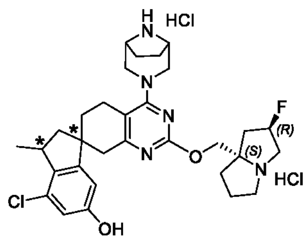

7ab

The synthetic route is as follows:

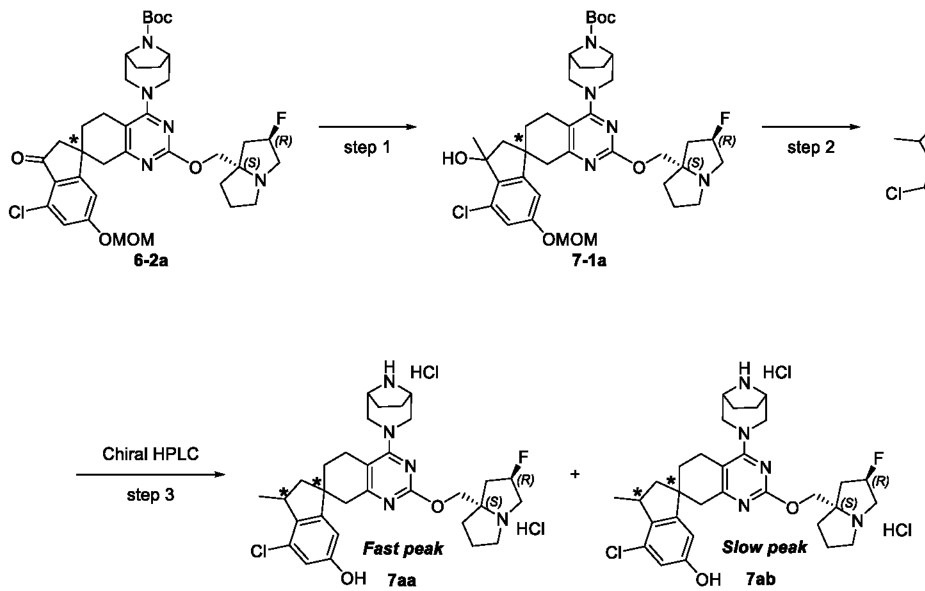


6-2a 7-1a 7a 7aa 7ab

Step 1:

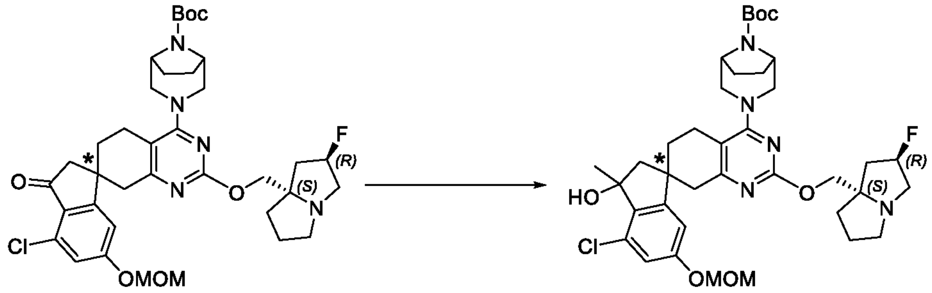

6-2a 7-1a

Under nitrogen atmosphere at 0°C with stirring, a solution of lanthanum(III) chloride bis(lithium chloride) complex in tetrahydrofuran (0.6 M, 111.15 μL, 0.067 mmol, 1 eq) was slowly added to a solution of compound **6-2a** (50 mg, 0.067 mmol, 1 eq) in anhydrous tetrahydrofuran (0.8 mL). After the mixture had reacted for 1 hour, a solution of methylmagnesium bromide in 2-methyltetrahydrofuran (3 M, 88.92 μL, 0.268 mmol, 4 eq) was added dropwise. After the addition was completed, the reaction was allowed to proceed for an additional hour under nitrogen atmosphere at 0°C with stirring, and the reaction progress was monitored by LC-MS and TLC. After the reaction was completed, the reaction mixture was quenched with saturated ammonium chloride solution (20 mL) at 0°C. After quenching, the mixture was extracted with dichloromethane (3 × 20 mL). The organic phases were combined, dried over anhydrous sodium sulfate, and filtered to remove the desiccant. The filtrate was concentrated under reduced pressure to obtain the crude product. The resulting crude product was purified by reverse-phase chromatography (C18 column), eluting with a gradient of 50% to 95% methanol/water (10 mmol/L ammonium bicarbonate) over 20 minutes, with detection at UV 254/220 nm. The product obtained was compound **7-1a** (white solid, 49.0 mg, yield of 98%). MS(ESI, *m/z):* 728.5/730.5 $[M + H]^+$.

**Step 2:**

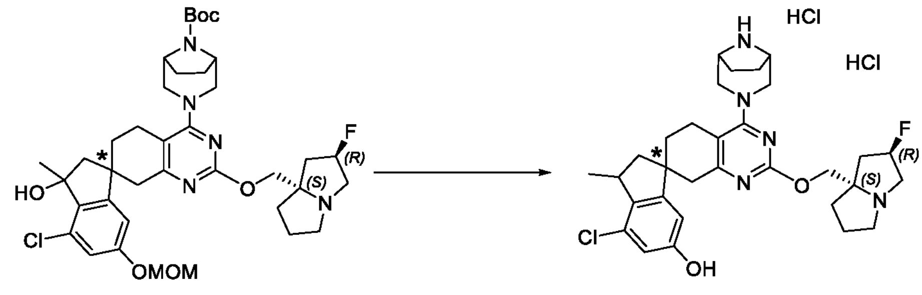

7-1a 7a

With stirring at 0°C, triethylsilane (20 mg, 0.165 mmol, 5 eq) was slowly added dropwise to a solution of compound **7-1a** (25 mg, 0.033 mmol, 1 eq) in trifluoroacetic acid (0.5 mL). The mixture was reacted with stirring at 25°C for 2 hours, with the reaction progress monitored by LC-MS and TLC. After the reaction was completed, the reaction mixture was subjected to rotary evaporation under reduced pressure to remove the solvent to obtain the crude product. The crude product was purified by reverse-phase chromatography (C18 column), eluting with a gradient of 5% to 95% acetonitrile/-water (0.1% hydrochloric acid) over 25 minutes, with detection at UV 254/220 nm. The product obtained was compound **7a** (white solid, 21.0 mg, yield of 98%). MS(ESI, *m*/*z*): 568.2/570.2 $[M+H]^+$, $^1H$ NMR (400 MHz, $CD_3OD$) $\delta$ 6.74 - 6.63 (m, 1H), 6.63 - 6.51 (m, 1H), 5.72 - 5.48 (m, 1H), 4.80 - 4.64 (m, 2H), 4.58 - 4.37 (m, 1H), 4.31 - 4.13 (m, 2H), 4.04 - 3.89 (m, 2H), 3.90 - 3.76 (m, 2H), 3.66 - 3.51 (m, 1H), 3.50 - 3.35 (m, 2H), 3.09 - 2.58 (m, 6H), 2.53 - 1.70 (m, 13H), 1.48 - 1.34 (m, 3H); $^{19}F$ NMR (377 MHz, $CD_3OD$) $\delta$ -174.27.

**Step 3:**

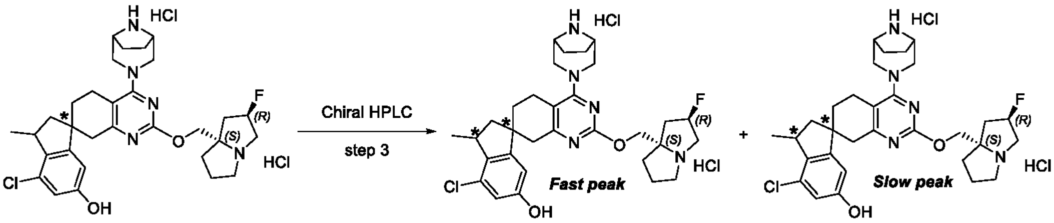


7a 7aa 7ab

The compound **7a** (17 mg, 0.026 mmol) was subjected to chiral resolution by high-performance liquid chromatography: chiral column CHIRALPAK IG, 2 × 25 cm, 5 μm; mobile phase A: n-hexane (10 mmol/L ammonia), mobile phase B: ethanol; flow rate: 25 mL/min; elution with 30% phase B over 33 minutes; detector: UV 210/290 nm, resulting in two products. The product with a shorter retention time (7.99 minutes) was further purified by reverse-phase chromatography (C18 column), eluting with a gradient of 5% to 95% acetonitrile/water (0.1% hydrochloric acid) over 10 minutes, with detection at UV 220/254 nm. The product obtained was product **7aa** (white solid, 3.8 mg, yield of 22%). MS (ESI, *m*/*z*): 568.2/569.9 $[M + H]^+$, $^1H$ NMR (400 MHz, $CD_3OD$) $\delta$ 6.72 - 6.67 (m, 1H), 6.62 - 6.54 (m, 1H), 5.75 - 5.47 (m, 1H), 5.14 - 4.98 (m, 1H), 4.82 - 4.66 (m, 2H), 4.65 - 4.47 (m, 1H), 4.38 - 4.12 (m, 2H), 4.03 - 3.78 (m, 4H), 3.75 - 3.55 (m, 1H), 3.52 - 3.35 (m, 2H), 3.19 - 2.86 (m, 3H), 2.84 - 2.54 (m, 3H), 2.54 - 1.91 (m, 10H), 1.91-1.70 (m, 2H), 1.49 - 1.32 (m, 3H), $^{19}F$ NMR (377 MHz, $CD_3OD$) $\delta$ -174.26, -174.32. The product with a longer retention time (10.18 minutes) was also purified by reverse-phase chromatography (C18 column), eluting with a gradient of 5% to 95% acetonitrile/water (0.1% hydrochloric acid) over 10 minutes, with detection at UV 220/254 nm. The product obtained was product **7ab** (white solid, 3.3 mg, yield of 19%). MS (ESI, *m*/*z*): 568.2/567.0 $[M + H]^+$, $^1H$ NMR (400 MHz, $CD_3OD$) $\delta$ 6.71 - 6.65 (m, 1H), 6.60 - 6.52 (m, 1H), 5.69 - 5.50 (m, 1H), 4.85 - 4.68 (m, 3H), 4.67 - 4.46 (m, 1H), 4.39 - 4.11 (m, 2H), 4.09 - 3.76 (m, 4H), 3.76 - 3.55 (m, 1H), 3.55 - 3.35 (m, 2H), 3.09 - 2.55 (m, 6H), 2.54 - 2.00 (m, 10H), 2.00 - 1.76 (m, 2H), 1.50 - 1.36 (m, 3H); $^{19}F$ NMR (377 MHz, $CD_3OD$) $\delta$ -174.25, -174.28.

The chiral analysis conditions for compound **7aa** were as follows: CHIRALPAK IG-3, 4.6 × 50 mm, 3 μm; mobile phase A: *n*-hexane (0.1% ethylenediamine); mobile phase B: ethanol; flow rate: 1.67 mL/min; isocratic gradient elution with 30% phase B over 7 minutes; detector: UV 290 nm; retention time: 1.964 minutes; *dr* > 40:1.

The chiral analysis conditions for compound **7ab** were as follows: CHIRALPAK IG-3, 4.6 × 50 mm, 3 μm; mobile phase A: *n*-hexane (0.1% ethylenediamine); mobile phase B: ethanol; flow rate: 1.67 mL/min; isocratic gradient elution with 30% phase B over 7 minutes; detector: UV 290 nm; retention time: 4.664 minutes; *dr* > 40:1.

**Example 8**

(1*S* or 1*R*, 8'*S* or 8'*R*)-4'-((1*R*,5*S*)-3,8-Diazabicyclo[3.2.1]octan-3-yl)-4-chloro-8'-fluoro-2'-(((2*R*,7a*S*)-2-fluorotetrahydro-1*H*-pyrrolizin-7a(5*H*)-yl)methoxy)-2,3,5',8'-tetrahydro-6'*H*-spiro[indene-1,7'-quinazolin]-6-ol dihydrochloride **8a;** (1*R* or 1*S*, 8'*R* or 8'*S*)-4'-((1*R*,5*S*)-3,8-diazabicyclo[3.2.1]octan-3-yl)-4-chloro-8'-fluoro-2'-(((2*R*,7a*S*)-2-fluorotetrahydro-1*H*-pyrrolizin-7a(5*H*)-yl)methoxy)-2,3,5',8'-tetrahydro-6'*H*-spiro[indene-1,7'-quinazolin]-6-ol dihydrochloride **8b;** (1*S* or 1*R,* 8'*R* or 8'*S*)-4'-((1*R*,5*S*)-3,8-diazabicyclo[3.2.1]octan-3-yl)-4-chloro-8'-fluoro-2'-(((2*R*,7a*S*)-2-fluorotetrahydro-1*H*-pyrrolizin-7a(5*H*)-yl)methoxy)-2,3,5',8'-tetrahydro-6'*H*-spiro[indene-1,7'-quinazolin]-6-ol dihydrochloride **8c;** (1*R* or 1*S*, 8'S or 8'*R*)-4'-((1*R*,5*S*)-3,8-diazabicyclo[3.2.1]octan-3-yl)-4-chloro-8'-fluoro-2'-(((2*R*,7a*S*)-2-fluorotetrahydro-1*H*-pyrrolizin-7a(5*H*)-yl)methoxy)-2,3,5',8'-tetrahydro-6'*H*-spiro[indene-1,7'-quinazolin]-6-ol dihydrochloride **8d**

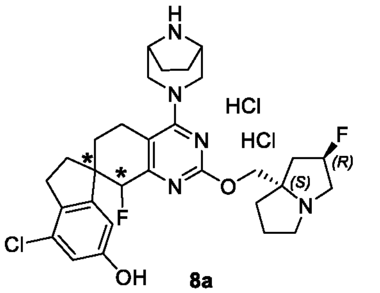

**8a**

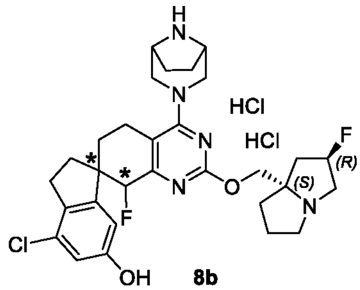

**8b**

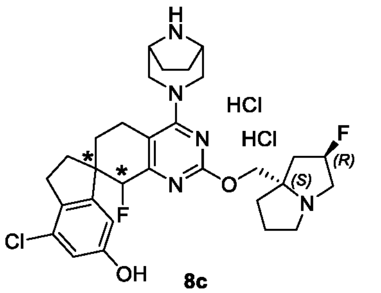

**8c**

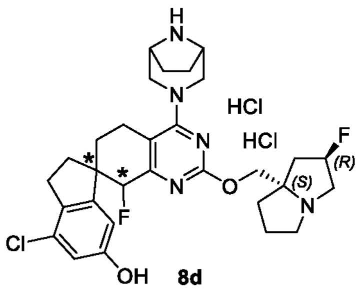

**8d**

The synthetic route is as follows:

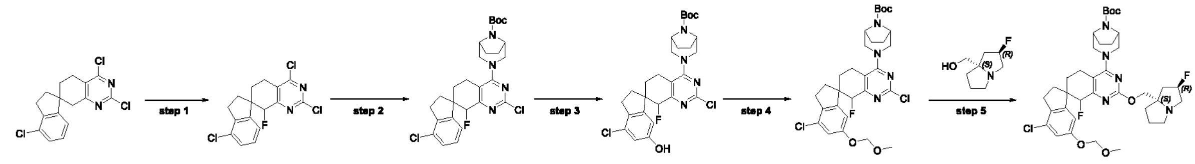


1-8 8-1 8-2 8-3 8-4 8-5

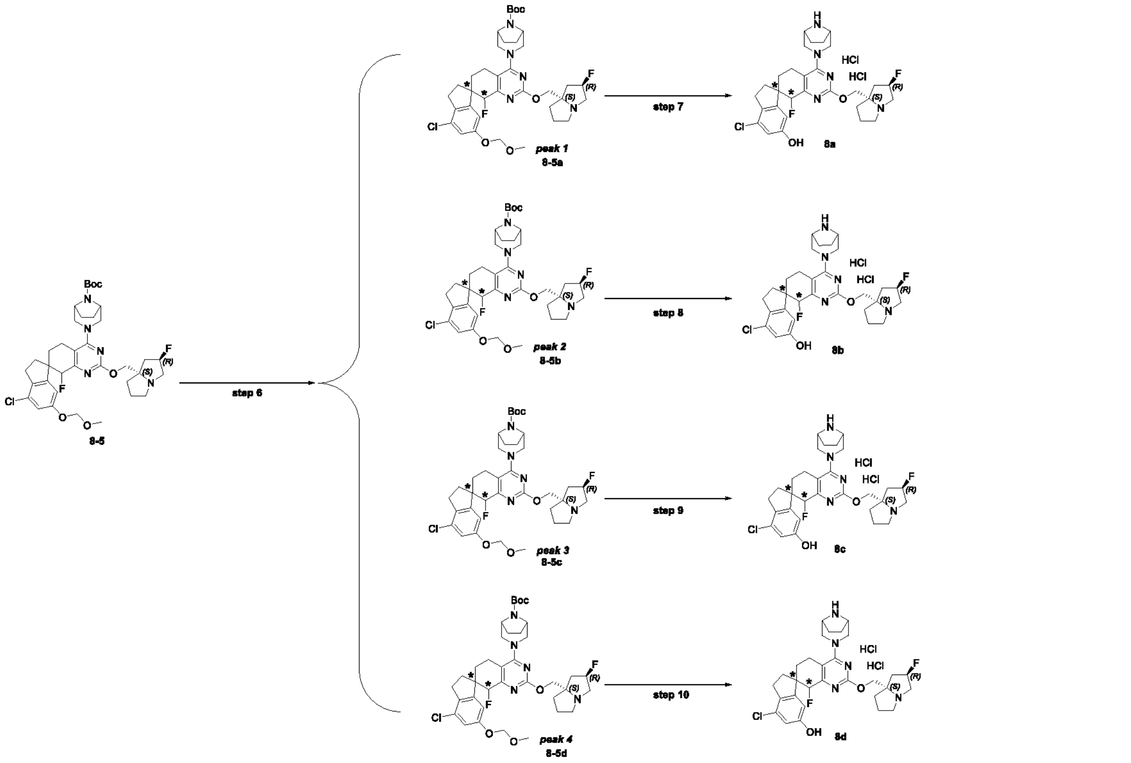


8-5
peak 1 8-5a 8a
peak 2 8-5b 8b
peak 3 8-5c 8c
peak 4 8-5d 8d

**Step 1:**

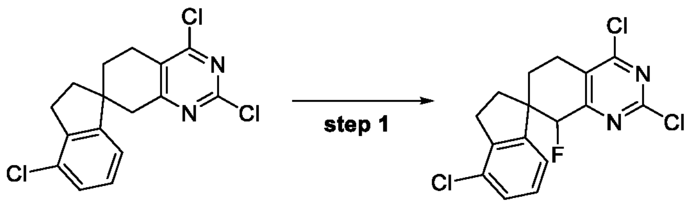


1-8 8-1

Under nitrogen atmosphere at -78°C with stirring, a solution of lithium diisopropylamide in tetrahydrofuran (1 M, 5.96 mL, 5.958 mmol, 1.42 eq) was slowly added dropwise to a solution of compound **1-8** (1.5 g, 4.196 mmol, 1.0 eq) in anhydrous tetrahydrofuran (10 mL). The mixture was reacted with stirring at -78°C for 30 minutes. At the same temperature, a solution of *N*-fluorobenzenesulfonimide (1.88 g, 5.665 mmol, 1.35 eq) in anhydrous tetrahydrofuran (5 mL) was slowly added dropwise to the reaction mixture. The mixture was stirred at -78°C for 5 minutes, then slowly warmed to -40°C and reacted at that temperature for 1 hour. The reaction progress was monitored by LC-MS and TLC. After the reaction was completed, the reaction was quenched by adding saturated sodium bicarbonate solution (20 mL). The resulting mixture was extracted with ethyl acetate (30 mL × 3). The organic phases were combined, dried over anhydrous sodium sulfate, and filtered to remove the desiccant. The filtrate was concentrated under reduced pressure to obtain the crude product. The crude product was purified by silica gel column chromatography and subjected to a gradient elution with a mobile phase of 0% to 25% ethyl acetate/petroleum ether. The collected fraction was subjected to rotary evaporation under reduced pressure to remove the solvent, yielding compound **8-1** (white solid, 1.4 g, yield of 90%). MS (ESI, m/z):

357.0/359.0/361.0 [M + H]+; $^{1}$H NMR (400 MHz, $CDCl_3$) $\delta$ 7.28 - 7.23 (m, 1H), 7.16 - 7.11 (m, 1H), 6.76 - 6.66 (m, 1H), 5.43- 5.27 (m, 1H), 3.15 - 3.00 (m, 2H), 2.97 - 2.76 (m, 2H), 2.38 - 2.29 (m, 1H), 2.18 - 2.02 (m, 3H).

**Step 2:**

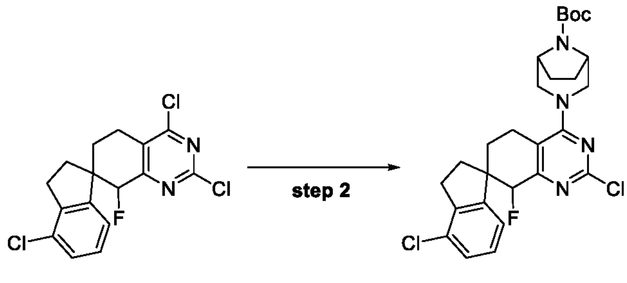


**8-1** **8-2**

Under nitrogen atmosphere at 25°C with stirring, compound **8-1** (789 mg, 2.096 mmol, 1.0 eq), *tert*-butyl 3,8-diazabicyclo[3.2.1]octane-8-carboxylate (468.36 mg, 2.096 mmol, 1.0 eq), *N,N*-diisopropylethylamine (1.15 mL, 3.0 eq), and dimethyl sulfoxide (9 mL) were sequentially added to a reaction flask. The mixture was reacted at 25°C for 1 hour, with the reaction progress monitored by LC-MS and TLC. After the reaction was completed, the reaction mixture was added with saturated brine (10 mL). The resulting mixture was extracted with ethyl acetate (20 mL × 3). The organic phases were combined, dried over anhydrous sodium sulfate, and filtered to remove the desiccant. The filtrate was concentrated under reduced pressure to obtain the crude product. The crude product was purified by silica gel column chromatography and subjected to a gradient elution with a mobile phase of 0% to 20% ethyl acetate/petroleum ether. The collected fraction was subjected to rotary evaporation under reduced pressure to remove the solvent, yielding compound **8-2** (pale yellow solid, 987 mg, yield of 83%). MS (ESI, m/z): 533.2/535.2/537.2 [M + H]+.

Step 3:

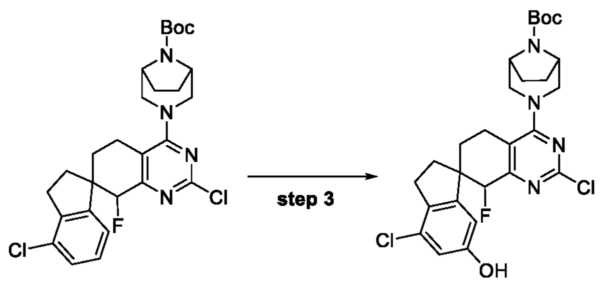


**8-2** **8-3**

Under nitrogen atmosphere at 25°C with stirring, compound **8-2** (985 mg, 1.754 mmol, 1.0 eq), chloro(1,5-cyclooctadiene)iridium(I) dimer (124.02 mg, 0.175 mmol, 0.1 eq), 4,4'-di-*tert*-butyl-2,2'-dipyridine (99.11 mg, 0.351 mmol, 0.2 eq), bis(pinacolato)diboron (1.88 g, 7.033 mmol, 4.0 eq), pinacolborane (107.16 μL, 0.702 mmol, 0.4 eq), and 1,4-dioxane (10 mL) were sequentially added to a 40 mL reaction flask. The mixture was reacted at 120°C for 2 hours, with the reaction progress monitored by LC-MS and TLC. After the reaction was completed, the reaction mixture was cooled to room temperature and concentrated under reduced pressure to obtain the crude intermediate. With stirring at 0°C, a mixed solvent of methanol/tetrahydrofuran (1/1, 20 mL) was added to the crude intermediate, followed by the portion-wise addition of urea hydrogen peroxide (1.74 g, 17.57 mmol, 10.0 eq). The mixture was reacted at 25°C for 2 hours, with the reaction progress monitored by LC-MS and TLC. After the reaction was completed, the mixture was concentrated under reduced pressure to obtain the crude product. The resulting crude product was purified by reverse-phase chromatography (C18 column) and eluted with a mobile phase of 5% to 95% methanol/water (0.1% ammonium bicarbonate) over 30 minutes, with detection at UV 254/220 nm. The product obtained was compound **8-3** (pale yellow solid, 564 mg, yield of 55%). MS (ESI, m/z): 549.2/551.2/553.2 [M + H]+.

**Step 4:**

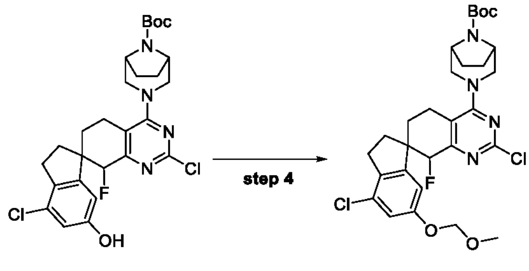


8-3 8-4

Under nitrogen atmosphere at 0°C with stirring, compound **8-3** (550 mg, 0.951 mmol, 1.0 eq), *N,N*-diisopropylethylamine (871.78 μL, 4.755 mmol, 5.0 eq), dichloromethane (7 mL), and chloromethyl methyl ether (188.33 μL, 2.853 mmol, 3.0 eq) were sequentially added to a reaction flask. The mixture was reacted at 20°C for 1 hour, with the reaction progress monitored by LC-MS and TLC. After the reaction was completed, the reaction mixture was concentrated under reduced pressure to obtain the crude product. The crude product was purified by silica gel column chromatography and subjected to a gradient elution with a mobile phase of 0% to 20% ethyl acetate/petroleum ether. The collected fraction was subjected to rotary evaporation under reduced pressure to remove the solvent, yielding compound **8-4** (white solid, 466 mg, yield of 78%). MS (ESI, m/z): 593.2/595.2/597.2 $[M + H]^+$.

**Step 5:**

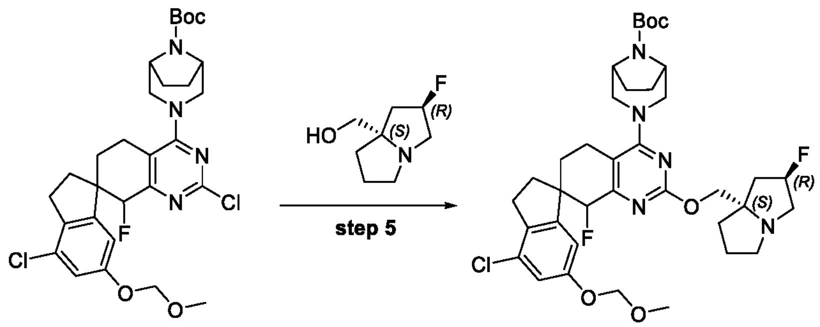


8-4 8-5

Under nitrogen atmosphere at 0°C with stirring, a solution of potassium tert-butoxide in tetrahydrofuran (1 M, 3.52 mL, 3.520 mmol, 5.0 eq) was slowly added to a solution of compound **8-4** (440 mg, 0.704 mmol, 1.0 eq) and (2*R*,7a*S*)-2-fluorotetrahydro-1*H*-pyrrolizin-7a(5*H*)-methanol (590.12 mg, 3.521 mmol, 5.0 eq) in 1,4-dioxane (5 mL). The mixture was reacted at 60°C for 1 hour, with the reaction progress monitored by LC-MS and TLC. After the reaction was completed, the reaction mixture was added with saturated brine (10 mL). The resulting mixture was extracted with ethyl acetate (10 mL × 3). The organic phases were combined, dried over anhydrous sodium sulfate, and filtered to remove the desiccant. The filtrate was concentrated under reduced pressure to obtain the crude product. The resulting crude product was purified by silica gel column chromatography and subjected to a gradient elution with a mobile phase of 0% to 6% methanol/dichloromethane. The collected fraction was subjected to rotary evaporation under reduced pressure to remove the solvent, yielding the crude product of compound **8-5.** The resulting crude product was further purified by reverse-phase chromatography (C18 column) and eluted with a mobile phase of 40% to 95% methanol/water (10 mmol/L ammonia water) over 30 minutes, with detection at UV 254/220 nm. The collected fraction was subjected to rotary evaporation under reduced pressure to remove the solvent, yielding compound **8-5** (white solid, 231 mg, yield of 44%). MS (ESI, m/z): 716.3/718.3 [M + H]+.

**Step 6:**

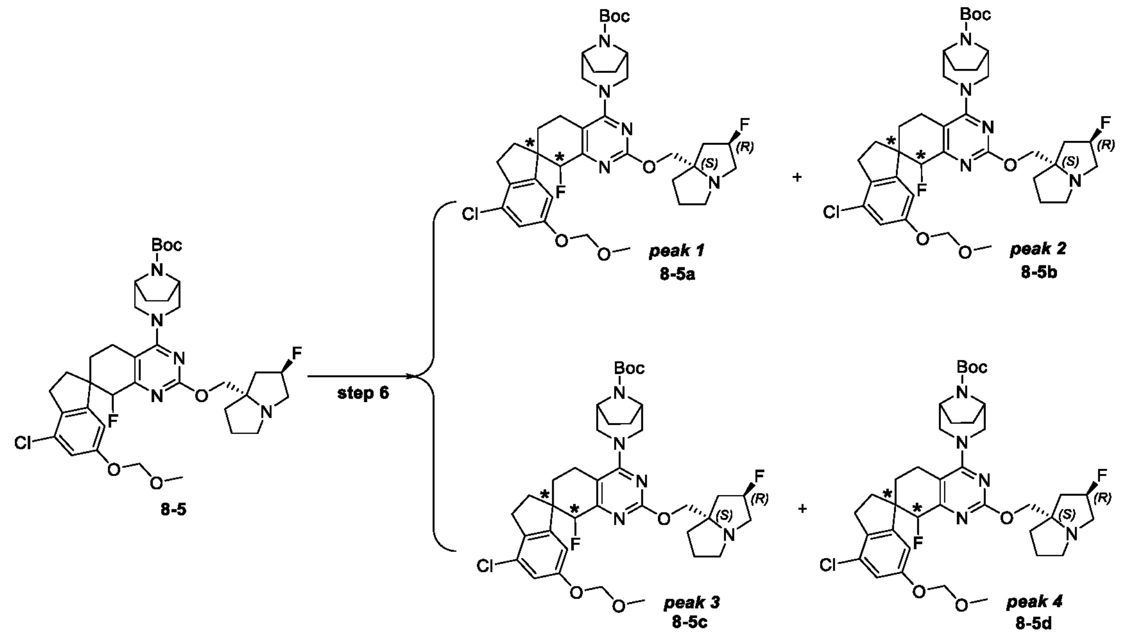


The compound **8-5** (231 mg) obtained from **step 5** was subjected to two rounds of chiral resolution using preparative chiral high-performance liquid chromatography, resulting in four isomers.

The conditions for the first round of chiral resolution were: Column: CHIRALPAK IE, 2 × 25 cm, 5 μm; mobile phase A: n-hexane: methyl tert-butyl ether = 1:1 (0.5%, 2 M, ammonia in methanol), mobile phase B: ethanol; flow rate: 20 mL/min; elution with 20% mobile phase B; detector UV 212/287 nm. The product corresponding to retention time RT1 = 7.165 minutes was compound **8-5a** (white solid, 40 mg, yield of 17%), MS (ESI, m/z): 716.3/718.3 $[M + H]^+$. The product corresponding to retention time RT2 = 8.985 minutes was a mixture of compounds **8-5b** and **8-5c** (white solid, 100 mg). The product corresponding to retention time RT3 = 14.34 minutes was compound **8-5d** (white solid, 52 mg, yield of 23%), MS (ESI, m/z): 716.3/718.3 $[M + H]^+$.

The mixture of compounds **8-5b** and **8-5c** (100 mg) was subjected to a second round of chiral resolution: Column: CHIRAL ART Cellulose-SB, 2 × 25 cm, 5 μm; mobile phase A: *n*-hexane (10 mmol/L, ammonia in methanol), mobile phase B: isopropanol; flow rate: 20 mL/min; elution with 10% mobile phase B; detector UV 210/288 nm. The product with a shorter retention time (17.0 minutes) was compound **8-5b** (white solid, 35 mg, yield of 15%), MS (ESI, m/z): 716.3/718.3 $[M + H]^+$. The product with a longer retention time (24.0 minutes) was compound **8-5c** (white solid, 53 mg, yield of 23%), MS (ESI, m/z): 716.3/718.3 $[M + H]^+$.

**Step** 7:

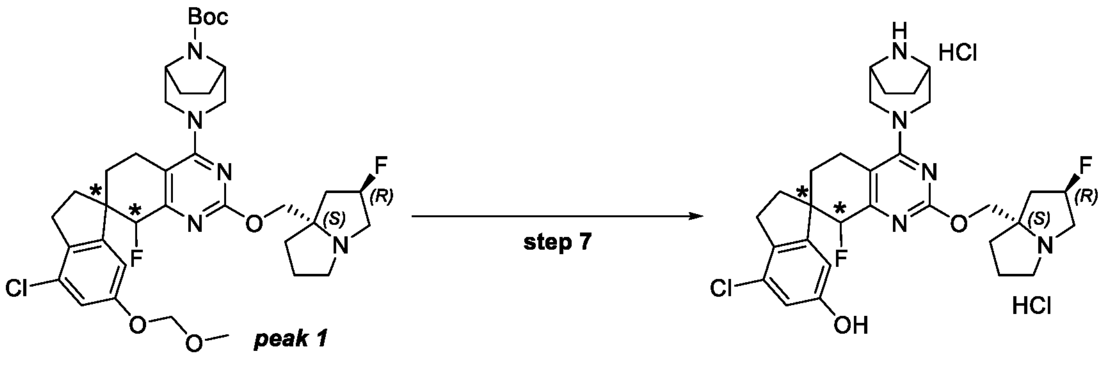


**8-5a** **8a**

With stirring at 0°C, compound **8-5a** (37 mg, 0.049 mmol, 1 eq), methanol (0.5 mL), and a solution of hydrochloric acid in 1,4-dioxane (4 M, 0.5 mL) were sequentially added to a 25 mL single-neck flask. The resulting mixture was reacted with stirring at 25°C for 1.5 hours. The reaction progress was monitored by LC-MS and TLC. After the reaction was completed, the reaction mixture was concentrated under reduced pressure to obtain the crude product. The crude product was purified by reverse-phase chromatography (C18 column) and eluted with a mobile phase of 5% to 95% acetonitrile/-water (0.1% hydrochloric acid) over 20 minutes, with detection at UV 220/254 nm. The collected fraction was subjected to rotary evaporation under reduced pressure to remove the solvent, yielding compound **8a** (white solid, 20.4 mg, yield of 64%). MS(ESI, m/z): 572.3/574.3 $[M + H]^+$; $^1H$ NMR (400 MHz, DMSO-$d_6$ + $D_2O$) $\delta$ 6.79 - 6.69 (m, 1H), 6.59 - 6.48 (m, 1H), 5.67 - 5.45 (m, 1H), 5.42 - 5.24 (m, 1H), 4.54 - 4.43 (m, 2H), 4.17 - 4.06 (m, 3H), 4.02 - 3.93 (m, 1H), 3.86 - 3.70 (m, 3H), 3.60 - 3.53 (m, 1H), 3.44 - 3.41 (m, 1H), 3.33 - 3.20 (m, 1H), 2.89 - 2.73 (m, 3H), 2.61 - 2.52 (m, 2H), 2.47 - 2.42 (m, 1H), 2.35 - 2.11 (m, 4H), 2.10 - 1.78 (m, 8H); $^{19}F$ NMR (377 MHz, DMSO-$d_6$ + $D_2O$) $\delta$ -172.75, -184.78.

**Step 8:**

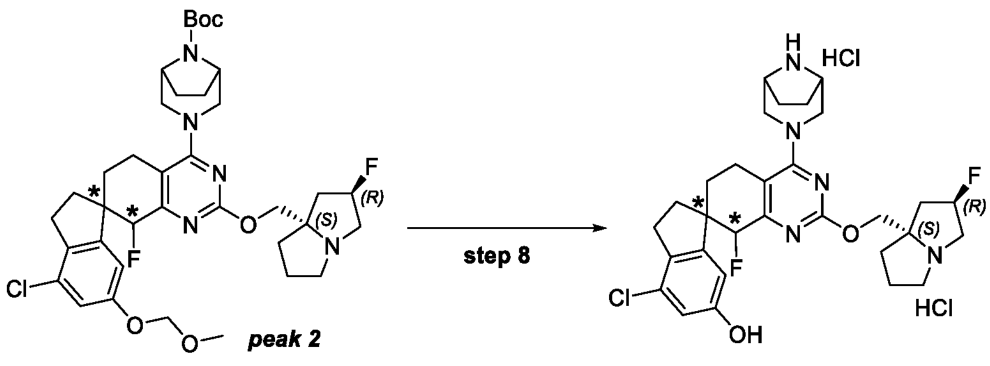


**8-5b** **8b**

Compound **8b** (white solid, 17 mg, yield of 48%) could be obtained using the same method described in **step 7** of this example. MS(ESI, m/z): 572.3/574.3 $[M + H]^+$; $^1H$ NMR (400 MHz, $CD_3OD$) $\delta$ 6.78 - 6.70 (m, 1H), 6.67 - 6.58 (m, 1H), 5.68 - 5.47 (m, 2H), 4.97 - 4.92 (m, 1H), 4.83 - 4.76 (m, 2H), 4.65 - 4.56 (m, 1H), 4.30 - 4.19 (m, 2H), 4.02 - 3.82 (m, 4H), 3.72 - 3.63 (m, 1H), 3.51 - 3.39 (m, 1H), 3.10 - 2.98 (m, 1H), 2.97 - 2.87 (m, 2H), 2.84 - 2.56 (m, 3H), 2.54 - 2.28 (m, 4H), 2.27 - 1.91 (m, 8H); $^{19}F$ NMR (377 MHz, $CD_3OD$) $\delta$ -174.20, -193.42.

**Step 9:**

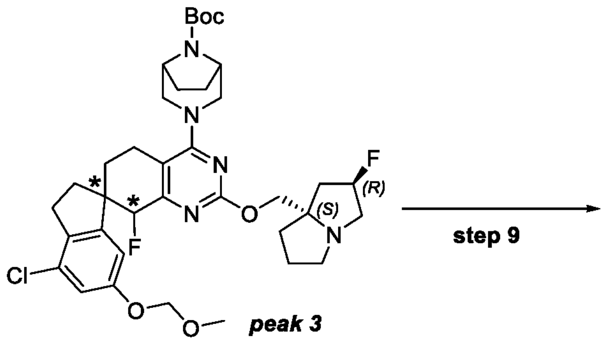


8-5c 8c

Compound **8c** (yellow solid, 25 mg, yield of 57%) could be obtained using the same method described in **step 7** of this example. MS(ESI, m/z): 572.3/574.3 [M + H]$^+$; $^1$H NMR (400 MHz, $CD_3OD$) $\delta$ 6.75 - 6.73 (m, 1H), 6.65 - 6.62 (m, 1H), 5.71 - 5.51 (m, 1H), 5.25 - 5.07 (m, 1H), 5.05 - 4.98 (m, 1H), 4.92 - 4.90 (m, 1H), 4.79 - 4.71 (m, 1H), 4.58 - 4.46 (m, 1H), 4.33 - 4.20 (m, 2H), 4.02 - 3.90 (m, 3H), 3.89 - 3.83 (m, 1H), 3.68 - 3.59 (m, 1H), 3.51 - 3.41 (m, 1H), 3.05 - 2.91 (m, 3H), 2.86 - 2.59 (m, 3H), 2.52 - 2.42 (m, 1H), 2.41 - 2.02 (m, 10H), 1.94 - 1.84 (m, 1H); $^{19}$F NMR (377 MHz, $CD_3OD$) $\delta$ -174.02, -174.84.

**Step 10:**

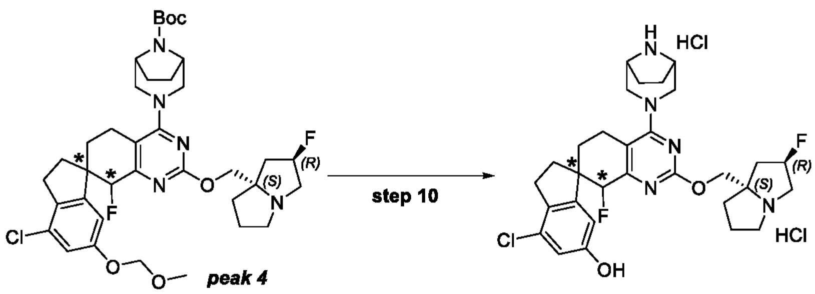


8-5d 8d

Compound **8d** (yellow solid, 34.9 mg, yield of 83%) could be obtained using the same method described in **step 7** of this example. MS(ESI, m/z): 572.3/574.3 [M + H]$^+$; $^1$H NMR (400 MHz, DMSO-$d_6$ + $D_2O$) $\delta$ 6.78 - 6.73 (m, 1H), 6.67 - 6.58 (m, 1H), 5.66 - 5.49 (m, 1H), 5.08 - 4.91 (m, 1H), 4.56 - 4.43 (m, 2H), 4.39 - 4.26 (m, 1H), 4.17 - 4.06 (m, 2H), 4.00 - 3.90 (m, 1H), 3.87 - 3.82 (m, 1H), 3.81 - 3.72 (m, 2H), 3.70 - 3.64 (m, 1H), 3.45 - 3.36 (m, 1H), 3.34 - 3.22 (m, 1H), 2.96 - 2.76 (m, 3H), 2.72 - 2.55 (m, 2H), 2.48 - 2.44 (m, 1H), 2.34 - 2.25 (m, 1H), 2.24 - 1.96 (m, 8H), 1.95 - 1.84 (m, 2H), 1.79 - 1.68 (m, 1H); $^{19}$F NMR (377 MHz, DMSO-$d_6$ + $D_2O$) $\delta$ -170.35, -172.72.

**Example 9**

(*S* or *R*)-4'-((1*R*,5*S*)-3,8-Diazabicyclo[3.2.1]octan-3-yl)-4-chloro-8',8'-difluoro-2'-(((2*R*,7*aS*)-2-fluorotetrahydro-1*H*-pyrrolizin-7a(5*H*)-yl)methoxy)-2,3,5',8'-tetrahydro-6'*H*-spiro[indene-1,7'-quinazolin]-6-ol dihydrochloride **9a;** (*R* or *S*)-4'-((1*R*,5*S*)-3,8-diazabicyclo[3.2.1]octan-3-yl)-4-chloro-8',8'-difluoro-2'-(((2*R*,7*aS*)-2-fluorotetrahydro-1*H*-pyrrolizin-7a(5*H*)-yl)methoxy)-2,3,5',8'-tetrahydro-6'*H*-spiro[indene-1,7'-quinazolin]-6-ol dihydrochloride **9b**

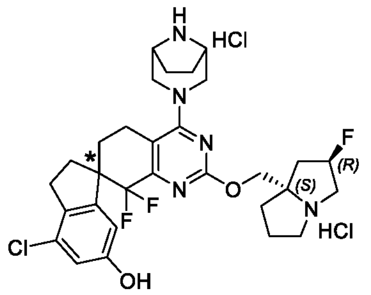

9a

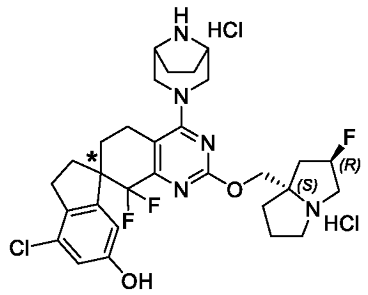

9b

The synthetic route is as follows:

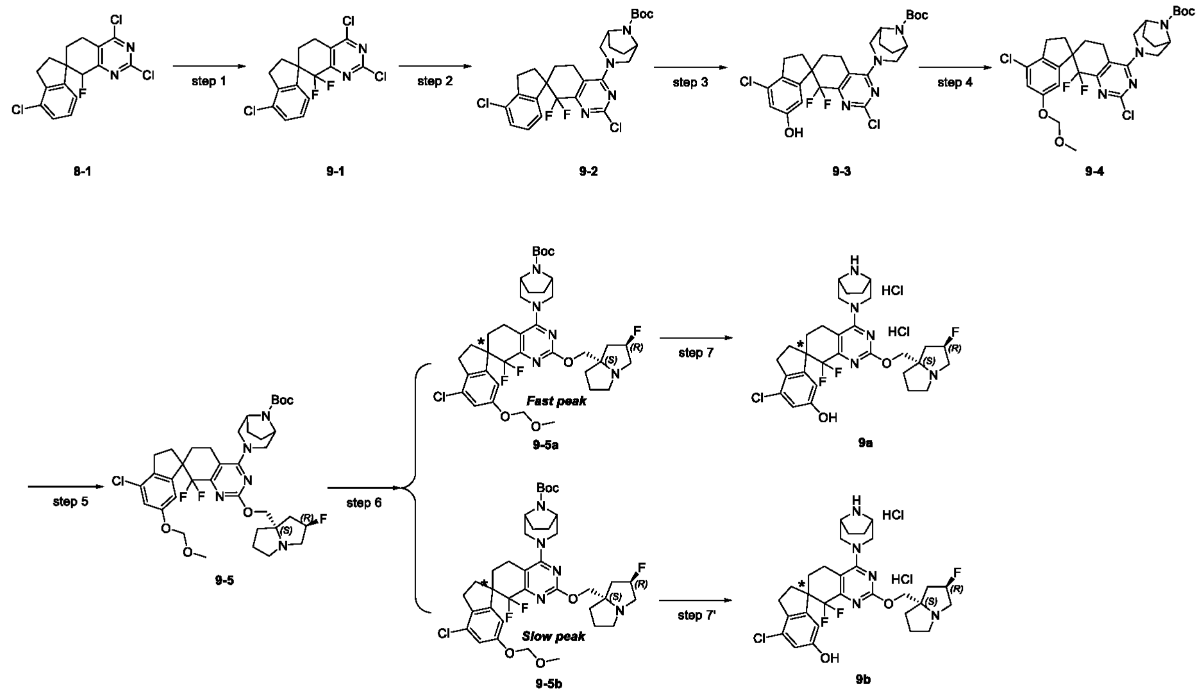


Step 1:

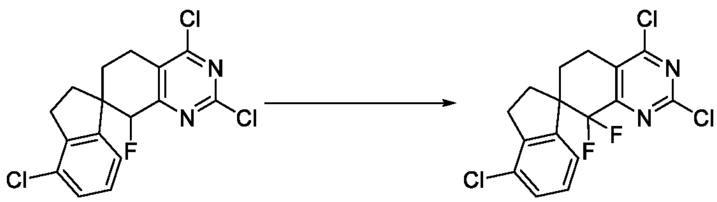

8-1 9-1

Under nitrogen atmosphere at -70°C with stirring, a solution of lithium diisopropylamide in tetrahydrofuran (1.0 M, 1.89 mL, 1.886 mmol, 1.42 eq) was slowly added dropwise to a solution of compound **8-1** (500 mg, 1.328 mmol, 1.0 eq) in anhydrous tetrahydrofuran (5 mL). The mixture was reacted with stirring at -70°C under nitrogen atmosphere for 0.5 hours, and then a solution of *N*-fluorobenzenesulfonimide (595.16 mg, 1.793 mmol, 1.35 eq) in anhydrous tetrahydrofuran (5 mL) was slowly added dropwise to the mixture. The mixture was reacted with stirring under nitrogen atmosphere at -40°C for 1 hour. The reaction progress was monitored by LC-MS and TLC. After the reaction was completed, the mixture was cooled to 0°C, and 10 mL of saturated sodium bicarbonate aqueous solution was added to quench the reaction. The mixture was extracted with ethyl acetate (3 × 20 mL), and the organic phases were combined. The organic phases were washed with

saturated brine, then dried over anhydrous sodium sulfate, and filtered to remove the desiccant. The filtrate was subjected to rotary evaporation under reduced pressure to remove the solvent, yielding the crude product. The crude product was purified by silica gel column chromatography and subjected to a gradient elution with a mobile phase of 0% to 20% ethyl acetate/petroleum ether. The collected fraction was subjected to rotary evaporation under reduced pressure to remove the solvent, yielding compound **9-1** (yellow solid, 310 mg, yield of 59%). MS (ESI, *m/z):* 374.9/376.9/378.9 $[M + H]^+$; $^1H$ NMR (300 MHz, $CDCl_3$) $\delta$ 7.35 - 7.28 (m, 1H), 7.24 - 7.16 (m, 1H), 7.15 - 7.06 (m, 1H), 3.19 - 2.99 (m, 2H), 3.00 - 2.80 (m, 2H), 2.65 - 2.50 (m, 1H), 2.34 - 2.18 (m, 1H), 2.17 - 2.00 (m, 2H); $^{19}F$ NMR (282 MHz, $CDCl_3$) $\delta$ -96.37, -97.36, -117.36, -118.36.

**Step 2:**

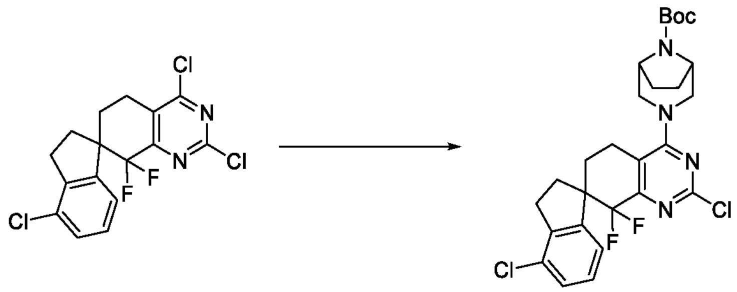

9-1 9-2

Under nitrogen atmosphere at 25°C with stirring, compound **9-1** (250 mg, 0.632 mmol, 1.0 eq), dimethyl sulfoxide (4 mL), *N,N*-diisopropylethylamine (258.07 mg, 1.896 mmol, 3.0 eq), and 8-*tert*-butoxycarbonyl-3,8-diazabicyclo[3.2.1] octane (141.30 mg, 0.632 mmol, 1.0 eq) were sequentially added to a 25 mL Schlenk tube. The mixture was reacted with stirring at 25°C under nitrogen atmosphere for 1 hour, with the reaction progress monitored by LC-MS and TLC. After the reaction was completed, the reaction mixture was cooled to 0°C, and ice water (30 mL) was added to quench the reaction. The mixture was extracted with ethyl acetate (3 × 20 mL). The organic phases were combined, washed with saturated brine, then dried over anhydrous sodium sulfate, and filtered to remove the desiccant. The filtrate was concentrated to obtain the crude product. The crude product was purified by silica gel column chromatography and subjected to a gradient elution with a mobile phase of 0% to 20% ethyl acetate/petroleum ether. The collected fraction was subjected to rotary evaporation under reduced pressure to remove the solvent, yielding compound **9-2** (white solid, 360 mg, yield of 98%). MS (ESI, m/z): 551.2/553.2 $[M + H]^+$; $^1H$ NMR (400 MHz, $CDCl_3$) $\delta$ 7.32 - 7.27 (m, 1H), 7.21 - 7.12 (m, 1H), 7.12 - 7.02 (m, 1H), 4.49 - 4.19 (m, 2H), 4.17 - 3.97 (m, 1H), 3.86 - 3.72 (m, 1H), 3.53 - 3.31 (m, 1H), 3.33 - 2.91 (m, 3H), 2.86 - 2.70 (m, 1H), 2.72 - 2.56 (m, 1H), 2.53 - 2.28 (m, 2H), 2.20 - 2.06 (m, 1H), 2.03 - 1.85 (m, 4H), 1.82 - 1.68 (m, 1H), 1.49 (s, 9H).

**Step 3:**

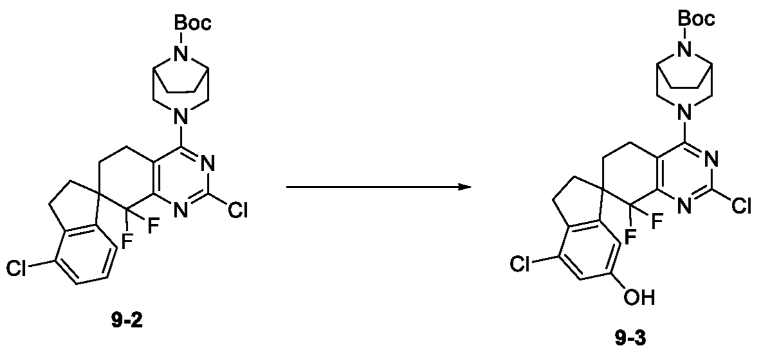

9-2 9-3

Under nitrogen atmosphere at 25°C with stirring, compound **9-2** (330 mg, 0.568 mmol, 1.0 eq), bis(pinacolato) diboron (607.84 mg, 2.272 mmol, 4.0 eq), chloro(1,5-cyclooctadiene)iridium(I) dimer (40.20 mg, 0.0057 mmol, 0.1 eq), 4,4'-di-*tert*-butyl-2,2'-bipyridine (32.12 mg, 0.114 mmol, 0.2 eq), pinacolborane (30.63 mg, 0.227 mmol, 0.4 eq), and 1,4-dioxane (3 mL) were sequentially added to a 10 mL single-neck flask. The mixture was reacted with stirring under nitrogen atmosphere at 120°C for 2 hours. The reaction progress was monitored by LC-MS and TLC. After the reaction was

completed, the reaction mixture was subjected to rotary evaporation under reduced pressure to remove the solvent, yielding the crude intermediate. With stirring at 0°C, tetrahydrofuran (2 mL), methanol (2 mL), and urea hydrogen peroxide (88.17 mg, 0.890 mmol, 10 eq) were sequentially added to the crude intermediate. The mixture was reacted with stirring at 25°C for 1 hour. The reaction progress was monitored by LC-MS and TLC. After the reaction was completed, the mixture was concentrated to obtain the crude product. The resulting crude product was purified by reverse-phase chromatography (C18 column) and eluted with a mobile phase of 50% to 95% methanol/water (10 mmol/L ammonium bicarbonate) over 25 minutes, with detection at UV 254/220 nm. The product obtained was compound **9-3** (white solid, 170 mg, yield of 50%). MS (ESI, m/z): 567.1/568.8/570.4 [M + H]+.

**Step 4:**

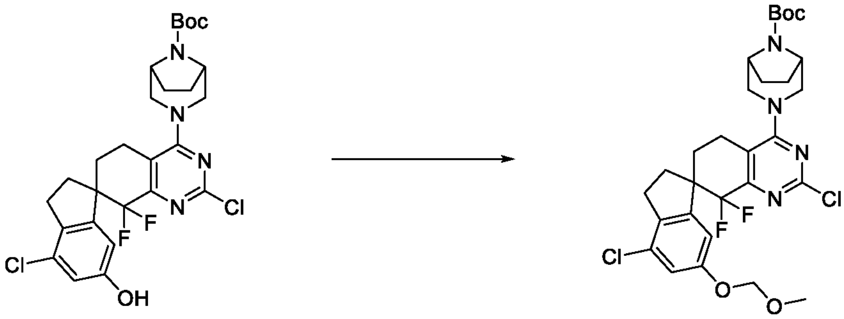

9-3 9-4

Under nitrogen atmosphere at 0°C with stirring, *N,N*-diisopropylethylamine (116.16 mg, 0.855 mmol, 3.0 eq) and chloromethyl methyl ether (39.83 mg, 0.570 mmol, 2.0 eq) were sequentially added dropwise to a solution of compound **9-3** (170 mg, 0.285 mmol, 1.00 eq) in dichloromethane (2 mL). The mixture was reacted with stirring under nitrogen atmosphere at 25°C for 2 hours. The reaction progress was monitored by LC-MS and TLC. After the reaction was completed, the reaction mixture was cooled to 0°C, and 10 mL of water was added at 0°C to quench the reaction. The mixture was extracted with dichloromethane (3 × 10 mL). The organic phases were combined, washed with saturated brine (20 mL), then dried over anhydrous sodium sulfate, and filtered to remove the desiccant. The filtrate was subjected to rotary evaporation under reduced pressure to remove the solvent, yielding the crude product. The crude product was purified by silica gel column chromatography and subjected to a gradient elution with a mobile phase of 0% to 50% ethyl acetate/petroleum ether. The collected fraction was subjected to rotary evaporation under reduced pressure to remove the solvent, yielding compound **9-4** (white solid, 180 mg, yield of 98%). MS (ESI, m/z): 611.2/613.2 [M + H]+.

**Step 5:**

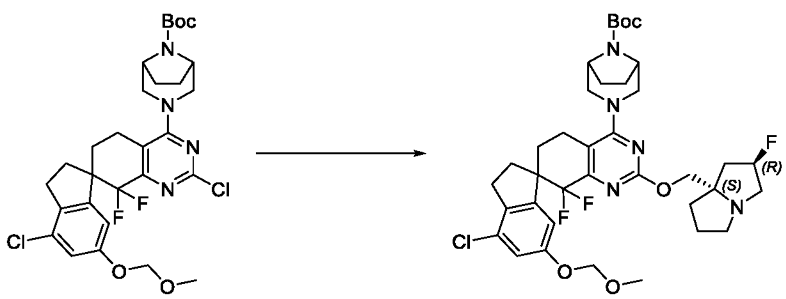

9-4 9-5

Under nitrogen atmosphere at 25°C with stirring, (2*R*,7a*S*)-2-fluorotetrahydro-1*H*-pyrrolizin-7*a*(5*H*)-methanol (49.99 mg, 0.299 mmol, 1.2 eq) and a solution of potassium *tert*-butoxide in tetrahydrofuran (1 M, 0.373 mL, 0.373 mmol, 1.5 eq) were sequentially added to a solution of compound **9-4** (160 mg, 0.249 mmol, 1.00 eq) in 1,4-dioxane (2 mL). The resulting mixture was reacted with stirring at 60°C under nitrogen atmosphere for 1 hour. The reaction progress was monitored by LC-MS and TLC. After the reaction was completed, the reaction mixture was cooled to 0°C, and 10 mL of water was added to quench the reaction. The mixture was extracted with dichloromethane (20 mL × 3). The organic

phases were combined, washed with saturated brine (20 mL), then dried over anhydrous sodium sulfate, and filtered to remove the desiccant. The filtrate was subjected to rotary evaporation under reduced pressure to remove the solvent, yielding the crude product. The resulting crude product was purified by silica gel column chromatography and subjected to a gradient elution with a mobile phase of 0% to 10% methanol/dichloromethane. The collected fraction was subjected to rotary evaporation under reduced pressure to remove the solvent, yielding compound **9-5** (white solid, 125 mg, yield of 65%). MS (ESI, m/z): 734.3/736.3 $[M + H]^+$.

**Step 6:**

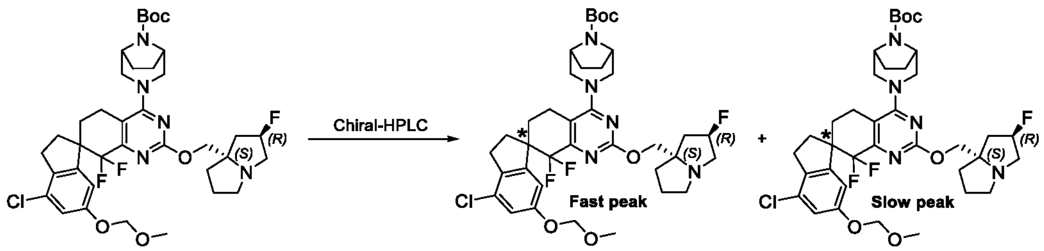


9-5 9-5a 9-5b

The compound **9-5** (120 mg) obtained from **step 5** was subjected to chiral resolution by preparative chiral high-performance liquid chromatography under the following conditions: chiral column CHIRAL ART Cellulose-SB, 2 × 25 cm, 5 μm; mobile phase A: n-hexane (0.5%, 2 M ammonia in methanol), mobile phase B: ethanol; flow rate: 20 mL/min; elution with 20% phase B over 9 minutes; detector: UV 212/288 nm, resulting in two products. The compound with a shorter retention time (3.95 minutes) was compound **9-5a** (white solid, 55 mg, yield of 46%), MS (ESI, m/z): 734.3/736.3 $[M + H]^+$. The compound with a longer retention time (5.99 minutes) was compound **9-5b** (white solid, 50 mg, yield of 42%), MS (ESI, m/z): 734.3/736.3 $[M + H]^+$.

**Step 7:**

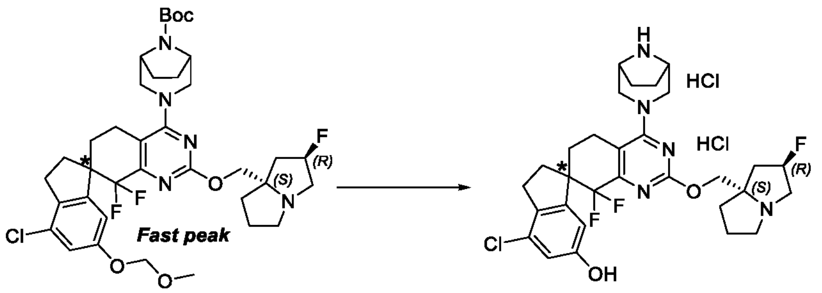


9-5a 9a

With stirring at 0°C, a solution of hydrochloric acid in 1,4-dioxane (4 M, 1.5 mL) was added dropwise to a solution of compound **9-5a** (55 mg, 0.071 mmol, 1.00 eq) in methanol (1.5 mL). The mixture was reacted with stirring at room temperature for 1 hour, with the reaction progress monitored by LC-MS and TLC. After the reaction was completed, the reaction mixture was concentrated to obtain the crude product. The resulting crude product was purified by reverse-phase chromatography (C18 column) and eluted with a mobile phase of 5% to 95% acetonitrile/methanol (v/v = 1/1): water (0.1% hydrochloric acid) over 30 minutes, with detection at UV 254/220 nm. The product obtained was compound **9a** (white solid, 36.0 mg, yield of 75%). MS (ESI, m/z): 590.2/591.5 $[M + H]^+$, $^1H$ NMR (400 MHz, DMSO-$d_6$ + $D_2O$) $\delta$ 6.79 (d, $J$ = 2.0 Hz, 1H), 6.66 - 6.56 (m, 1H), 5.69 - 5.42 (m, 1H), 4.56 - 4.39 (m, 2H), 4.28 - 4.17 (m, 1H), 4.16 - 4.05 (m, 2H), 3.98 - 3.90 (m, 1H), 3.89 - 3.67 (m, 3H), 3.65 - 3.56 (m, 1H), 3.42 - 3.35 (m, 1H), 3.33 - 3.23 (m, 1H), 2.95 - 2.73 (m, 3H), 2.70 - 2.55 (m, 2H), 2.48 - 2.41 (m, 1H), 2.34 - 2.09 (m, 6H), 2.08 - 1.81 (m, 6H), $^{19}F$ NMR (377 MHz, DMSO-$d_6$ + $D_2O$) $\delta$ -96.226, -96.957, - 112.281, -113.042, -172.817, -172.858. The chiral analysis conditions for compound **9a** were as follows: Lux Cellulose-2, 4.6 × 100 mm, 3 μm; mobile phase A: supercritical carbon dioxide fluid; mobile phase B: methanol (0.1% diethylamine); flow rate: 2 mL/min; isocratic gradient elution with 50% phase B over 7.5 minutes; detector: UV 220 nm; retention time: 5.228 minutes; *dr* > 40:1.

**Step 7':**

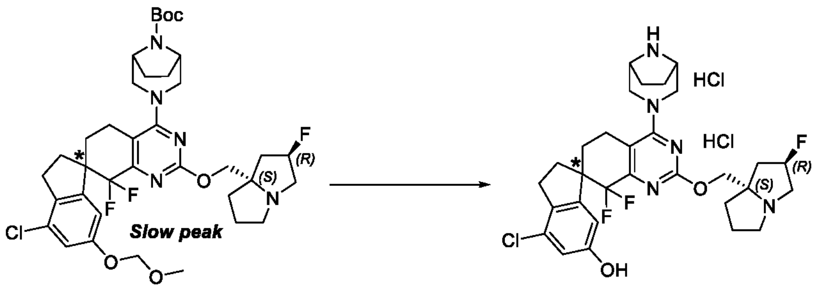


**9-5b** **9b**

Compound **9b** (white solid, 38.0 mg, yield of 88%) was obtained by using the same method as **step 7.** MS (ESI, m/z): 590.1/591.9 $[M + H]^+$, $^1H$ NMR (400 MHz, DMSO-$d_6$ + $D_2O$) $\delta$ 6.79 (d, $J$ = 2.0 Hz, 1H), 6.62 (d, $J$= 1.8 Hz, 1H), 5.70 - 5.43 (m, 1H), 4.57 - 4.39 (m, 2H), 4.26 - 4.16 (m, 1H), 4.16 - 4.05 (m, 2H), 4.00 - 3.90 (m, 1H), 3.90 - 3.67 (m, 3H), 3.67 - 3.56 (m, 1H), 3.46 - 3.46 (m, 1H), 3.36 - 3.20 (m, 1H), 2.95 - 2.74 (m, 3H), 2.71 - 2.55 (m, 2H), 2.49 - 2.42 (m, 1H), 2.37 - 2.09 (m, 6H), 2.09 - 1.81 (m, 6H), $^{19}F$ NMR (377 MHz, DMSO-$d_6$ + $D_2O$) $\delta$ -96.640, -97.390, -111.876, -112.605, -172.779, -172.826. The chiral analysis conditions for compound **9b** were as follows: Lux Cellulose-2, 4.6 × 100 mm, 3 μm; mobile phase A: supercritical carbon dioxide fluid; mobile phase B: methanol (0.1% diethylamine); flow rate: 2 mL/min; isocratic gradient elution with 50% phase B over 7.5 minutes; detector: UV 220 nm; retention time: 4.017 minutes; *dr* > 40:1.

## Example 10

(*S* or *R*)-6-((5a*S*,6*S*,9*R*)-1,3-Difluoro-13-(((2*R*,7a*S*)-2-fluorotetrahydro-1*H*-pyrrolizin-7a(5*H*)-yl)methoxy)-5a,6,7,8,9,10-hexahydro-5*H*-6,9-epiminoazepino[2', 1':3,4][1,4]oxazepino[5,6,7-*de*]quinazolin-2-yl)-4-methyl-5-(trifluoromethyl)pyridin-2-amine **10a'**; (*R* or *S*)-6-((5a*S*,6*S*,9*R*)-1,3-difluoro-13-(((2*R*,7a*S*)-2-fluorotetrahydro-1*H*-pyrrolizin-7a(5*H*)-yl)methoxy)-5a,6,7,8,9,10-hexahydro-5*H*-6,9-epiminoazepino[2', 1':3,4][1,4]oxazepino[5,6,7-*de*]quinazolin-2-yl)-4-methyl-5-(trifluoromethyl)pyridin-2-amine **10b'**

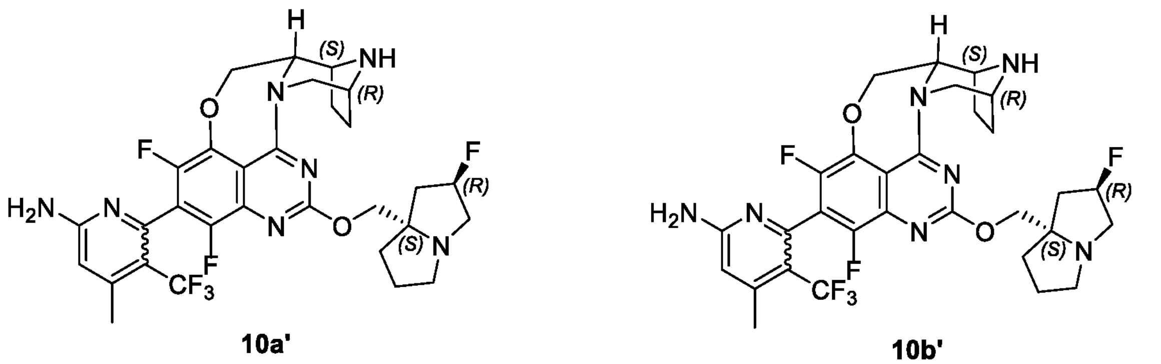

**10a'** **10b'**

The synthetic route is as follows:

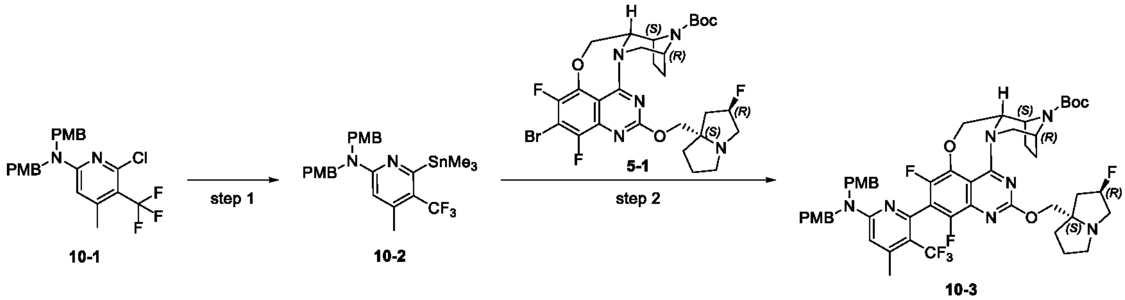


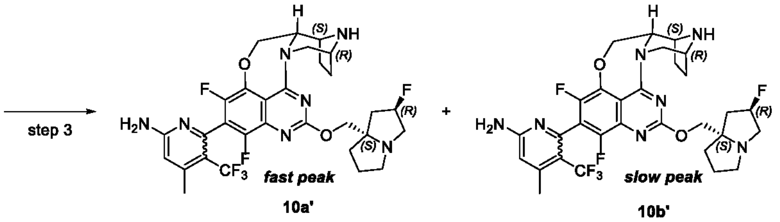


Step 1:

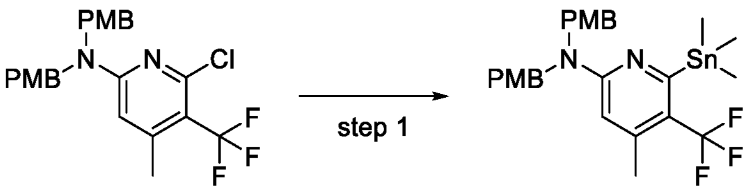


10-1 10-2

Compound **10-1** was synthesized with reference to patent WO2022035790A1.

Under nitrogen atmosphere at 25°C with stirring, compound **10-1** (1.2 g, 1 eq), hexamethylditin (1.8 eq), [1,1'-bis(diphenylphosphino)ferrocene]palladium(II) dichloride dichloromethane complex (0.1 eq), and 1,4-dioxane (12 mL) were sequentially added to a reaction flask. The resulting mixture was reacted with stirring at 100°C under nitrogen atmosphere for 20 hours. The reaction progress was monitored by LC-MS and TLC. After the reaction was completed, the crude product was purified by reverse-phase chromatography (C18 column) and eluted with a mobile phase of 5% to 95% acetonitrile/water (0.1% ammonium bicarbonate) over 25 minutes, with detection at UV 254/200 nm. The product obtained was compound **10-2** (yellow oil, 280 mg, yield of 18%). MS (ESI, m/z): 577.1/579.1/581.1 $[M + H]^+$; $^1H$ NMR (400 MHz, $CD_3OD$) $\delta$ 7.00 - 6.88 (m, 4H), 6.72 - 6.60 (m, 4H), 6.14 (s, 1H), 4.56 (s, 4H), 3.57 (s, 6H), 2.13 - 2.02 (m, 3H), 0.07 (s, 9H); $^{19}F$ NMR (377 MHz, $CD_3OD$) $\delta$ -56.55.

**Step 2:**

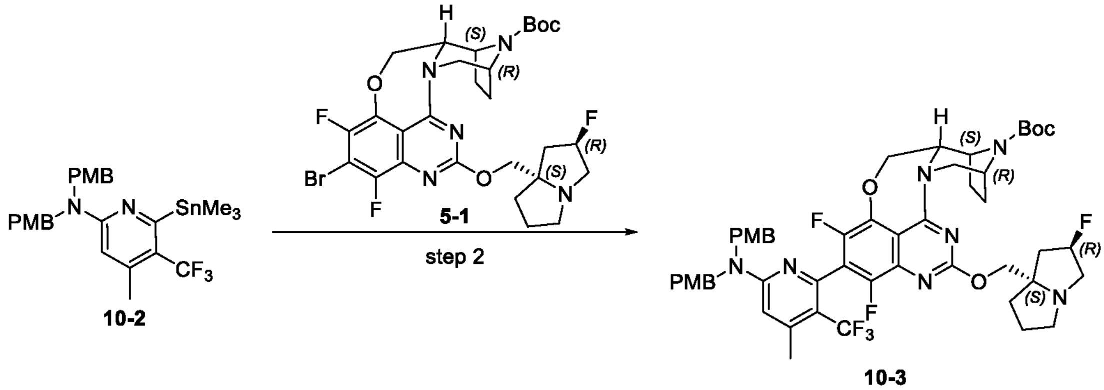

10-2 5-1 10-3

Under nitrogen atmosphere at 25°C with stirring, compound **10-2** (122 mg, 0.200 mmol, 1.5 eq), compound **5-1** (90 mg, 0.13 mmol, 1 eq), tetrakis(triphenylphosphine)palladium (283 mg, 0.24 mmol, 0.5 eq), cuprous iodide (13 mg, 0.07 mmol, 0.5 eq), a solution of lithium chloride in tetrahydrofuran (0.67 mL, 0.33 mmol, 2.5 eq, 0.5 M), and *N,N*-dimethylformamide (2 mL) were sequentially added to a reaction flask. The resulting mixture was reacted with stirring at 100°C under nitrogen atmosphere for 16 hours. The reaction progress was monitored by LC-MS and TLC. After the reaction was completed, the crude product was purified by reverse-phase chromatography (C18 column) and eluted with a mobile phase of 5% to 95% acetonitrile/water (0.1% ammonium bicarbonate) over 25 minutes, with detection at UV 254/220 nm. The product obtained was compound **10-3** (white solid, 68 mg, yield of 49%). MS (ESI, m/z): 976.3 [M + H]+.

**Step 3:**

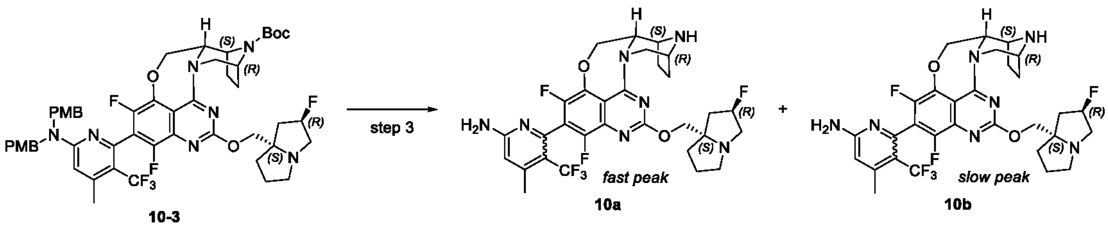

10-3 10a 10b

With stirring at 0°C, trifluoroacetic acid (2 mL) was added dropwise to a solution of compound **10-3** (68 mg, 0.07 mmol, 1 eq) in anisole (2 mL). The resulting mixture was reacted with stirring at 100°C for 1.5 hours. The reaction progress was monitored by LC-MS and TLC. After the reaction was completed, the reaction mixture was subjected to rotary evaporation under reduced pressure to remove the solvent, yielding the crude product. The crude product was purified by high-performance liquid chromatography,: column: YMC-Actus Triart C18 ExRS, 30 × 150 mm, 5 μm; mobile phase A: water (10 mmol/L ammonium bicarbonate); mobile phase B: acetonitrile; flow rate: 60 mL/min; elution with a gradient of 25% to 45% mobile phase B over 13 minutes; detector: 220 nm. The product with a shorter retention time (10.13 minutes) was compound **10a** (11 mg, white solid, yield of 24%); MS (ESI, m/z): 636.2 $[M + H]^+$; $^1H$ NMR (400 MHz, DMSO-$d_6$) $\delta$ 6.87 (s, 2H), 6.49 (s, 1H), 5.39 - 5.16 (m, 1H), 4.87 - 4.77 (m, 1H), 4.62 - 4.50 (m, 1H), 4.34 - 4.24 (m, 1H), 4.08 - 3.98 (m, 2H), 3.98 - 3.91 (m, 1H), 3.62 - 3.54 (m, 1H), 3.47 - 3.42 (m, 1H), 3.14 - 2.97 (m, 4H), 2.86 - 2.66 (m, 2H), 2.40 - 2.32 (m, 3H), 2.14 - 1.94 (m, 3H), 1.89 - 1.61 (m, 6H), 1.59 - 1.47 (m, 1H); $^{19}F$ NMR (377 MHz, DMSO-$d_6$) $\delta$ -53.51, - 135.87, -143.64, -172.14. The product with a longer retention time (10.98 minutes) was compound **10b** (5 mg, white solid, yield of 11%); MS (ESI, m/z): 636.2 $[M + H]^+$; $^1H$ NMR (300 MHz, DMSO-$d_6$) $\delta$ 6.87 (s, 2H), 6.50 (s, 1H), 5.42 - 5.16 (m, 1H), 4.78 - 4.69 (m, 1H), 4.60 - 4.47 (m, 1H), 4.42 - 4.29 (m, 1H), 4.14 - 4.04 (m, 1H), 4.00 - 3.89 (m, 2H), 3.65 - 3.56 (m, 1H), 3.50 - 3.44 (m, 1H), 3.13 - 2.98 (m, 4H), 2.89 - 2.65 (m, 2H), 2.44 - 2.32 (m, 3H), 2.17 - 2.10 (m, 1H), 2.09 - 1.93 (m, 2H), 1.88 - 1.52 (m, 7H); $^{19}F$ NMR (282 MHz, DMSO-$d_6$) $\delta$ -53.51, - 135.81, -143.67, -172.10.

**Example 11**

5-Cyclopropyl-4-((2*R* or 2*S*, 5a*S*,6*S*,9*R*)-1,3-difluoro-13-(((2*R*,7a*S*)-2-fluorotetrahydro-1*H*-pyrrolizin-7a(5*H*)-yl)methoxy)-5a,6,7,8,9,10-hexahydro-5*H*-6,9-epiminoazepino[2',1':3,4][1,4]oxazepino[5,6,7-*de*]quinazolin-2-yl)naphthalen-2-ol dihydrochloride **11a;** 5-cyclopropyl-4-((2*S* or 2*R*, 5a*S*,6*S*,9*R*)-1,3-difluoro-13-(((2*R*,7a*S*)-2-fluorotetrahydro-1*H*-pyrrolizin-7a(5*H*)-yl)methoxy)-5a,6,7,8,9,10-hexahydro-5*H*-6,9-epiminoazepino[2',1':3,4][1,4]oxazepino[5,6,7-*de*]quinazolin-2-yl)naphthalen-2-ol dihydrochloride **11b**

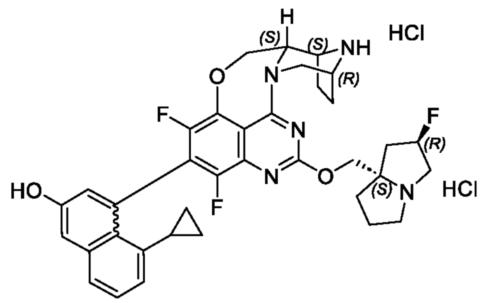

**11a**

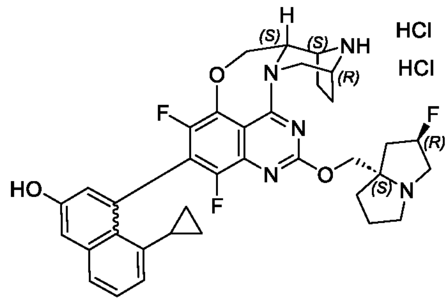

**11b**

The synthetic route is as follows:

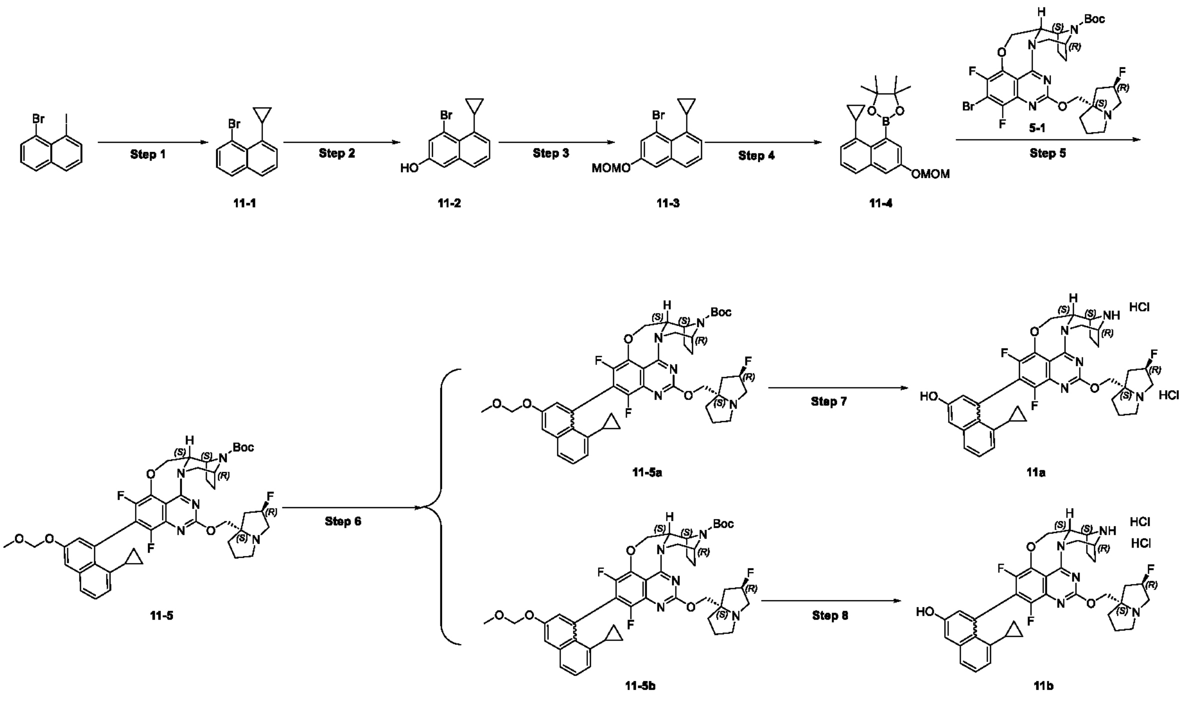


**Step 1:**

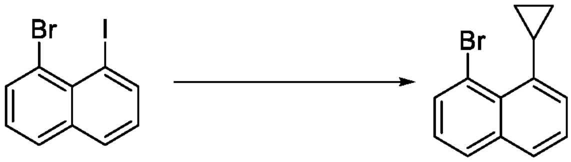

11-1

Under nitrogen atmosphere at 25°C with stirring, compound 1-bromo-8-iodonaphthalene (6.00 g, 17.119 mmol, 1 eq), cyclopropylboronic acid (1.55 g, 17.119 mmol, 1 eq), potassium phosphate (7.65 g, 34.238 mmol, 2 eq), [1,1'-bis(diphenylphosphino)ferrocene]palladium(II) dichloride dichloromethane complex (1.47 g, 1.712 mmol, 0.1 eq), toluene (60 mL), and water (12 mL) were sequentially added to a reaction flask. The resulting mixture was stirred at 60°C under nitrogen atmosphere for 6 hours, with the reaction progress monitored by TLC. After the reaction was completed, the reaction mixture was cooled to room temperature, and 20 mL of saturated ammonium chloride aqueous solution was added to dilute the reaction mixture. The resulting mixture was extracted with ethyl acetate (20 mL × 3). The organic phases were combined, dried over anhydrous sodium sulfate, and filtered to remove the desiccant. The filtrate was concentrated under reduced pressure to obtain the crude product. The crude product was purified by silica gel column chromatography and subjected to a gradient elution with a mobile phase of 0% to 10% ethyl acetate/petroleum ether. The collected fraction was concentrated under reduced pressure to remove the solvent, yielding compound 11-1 (pale yellow solid, 2.4 g, yield of 53%). $^1H$ NMR (300 MHz, $CDCl_3$) δ 7.93 - 7.87 (m, 1H), 7.84 - 7.78 (m, 1H), 7.74 - 7.69 (m, 1H), 7.52 - 7.47 (m, 1H), 7.43 - 7.36 (m, 1H), 7.28 - 7.22 (m, 1H), 3.08 - 2.94 (m, 1H), 1.18 - 1.08 (m, 2H), 0.93 - 0.84 (m, 2H).

**Step 2:**

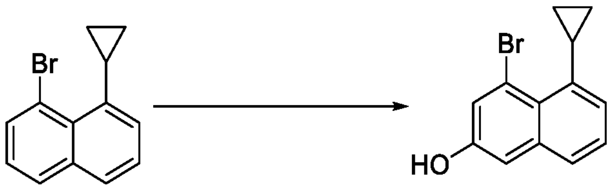

11-1 11-2

Under nitrogen atmosphere at 25°C with stirring, bis(pinacolato)diboron (1.34 g, 7.496 mmol, 1.3 eq), 4,4'-di-*tert*-butyl-2,2'-bipyridine (330 mg, 1.153 mmol, 0.2 eq), and chloro(1,5-cyclooctadiene)iridium(I) dimer (410 mg, 0.577 mmol, 0.1 eq) were sequentially added to a solution of compound **11-1** (1.5 g, 5.776 mmol, 1 eq) in heptane (20 mL). The mixture was stirred at 80°C for 2 hours, with the reaction progress monitored by TLC. After the reaction was completed, the reaction mixture was concentrated under reduced pressure to remove the solvent, yielding a mixture. The resulting mixture was dissolved in tetrahydrofuran (10 mL), and then water (5 mL), acetic acid (30 mL), and hydrogen peroxide (30%, 15 mL) were slowly added dropwise to the mixture at 0°C with stirring. The mixture was stirred at 0°C for 30 minutes, with the reaction progress monitored by TLC. After the reaction was completed, saturated sodium bicarbonate solution was slowly added to adjust the pH of the reaction mixture to 8 at 0°C with stirring. The mixture was extracted with ethyl acetate (100 mL × 3). The organic phases were combined, dried over anhydrous sodium sulfate, and filtered to remove the desiccant. The filtrate was concentrated under reduced pressure to obtain the crude product. The crude product was purified by silica gel column chromatography and subjected to a gradient elution with a mobile phase of 0% to 20% methyl tert-butyl ether/petroleum ether. The collected fraction was subjected to rotary evaporation under reduced pressure to remove the solvent, yielding compound **11-2** (yellow oily liquid, 700 mg, yield of 45%). $^1H$ NMR (400 MHz, DMSO-$d_6$) δ 10.05 (s, 1H), 7.65 - 7.58 (m, 1H), 7.50 (d, *J*= 2.6 Hz, 1H), 7.33 - 7.27 (m, 1H), 7.24 - 7.17 (m, 2H), 2.83 (m, 1H), 1.08 - 1.01 (m, 2H), 0.83 - 0.77 (m, 2H).

**Step 3:**

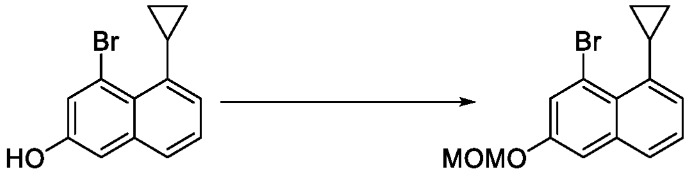

11-2 11-3

Under nitrogen atmosphere at 0°C with stirring, *N,N*-diisopropylethylamine (860.56 μL, 4.694 mmol, 2 eq) and chloromethyl methyl ether (298.32 mg, 3.521 mmol, 1.5 eq) were slowly added to a solution of compound **11-2** (650 mg, 2.347 mmol, 1 eq) in dichloromethane (10 mL). The mixture was stirred at 25°C for 2 hours, with the reaction progress monitored by LC-MS and TLC. After the reaction was completed, the mixture was concentrated under reduced pressure to obtain the crude product. The crude product was purified by silica gel column chromatography and subjected to a gradient elution with a mobile phase of 0% to 12% ethyl acetate/petroleum ether. The collected fraction was subjected to rotary evaporation under reduced pressure to remove the solvent, yielding compound **11-3** (yellow oil, 660 mg, yield of 88%). $^1H$ NMR (300 MHz, $CDCl_3$) $\delta$ 7.68 (d, $J$ = 2.6 Hz, 1H), 7.63 - 7.57 (m, 1H), 7.38 (d, $J$ = 2.6 Hz, 1H), 7.36 - 7.31 (m, 2H), 5.28 (s, 2H), 3.53 (s, 3H), 2.99 - 2.90 (m, 1H), 1.16 - 1.07 (m, 2H), 0.94 - 0.83 (m, 2H).

**Step 4:**

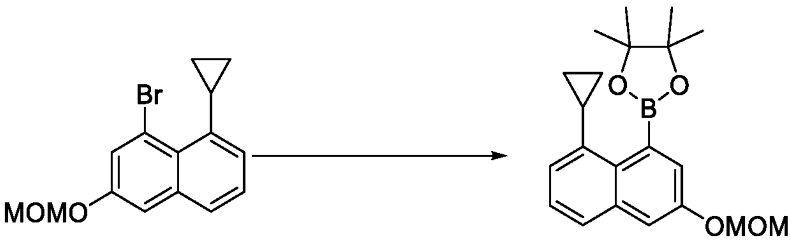

11-3 11-4

Under nitrogen atmosphere at -78°C with stirring, a solution of *n*-butyllithium in *n*-hexane (2.5 M, 0.59 mL, 1.485 mmol, 1.6 eq) was slowly added dropwise to a solution of compound **11-3** (300 mg, 0.928 mmol, 1 eq) in anhydrous tetrahydrofuran (4 mL). The mixture was reacted with stirring at -78°C under nitrogen atmosphere for 1 hour. At the same temperature, a solution of 2-isopropoxy-4,4,5,5-tetramethyl-1,3,2-dioxaborolane (327.07 mg, 1.670 mmol, 1.8 eq) in anhydrous tetrahydrofuran (1 mL) was slowly added dropwise to the reaction mixture. The mixture was stirred at -78°C for an additional 1 hour, then allowed to warm to room temperature and stirred at room temperature for 30 minutes, with the reaction progress monitored by LC-MS and TLC. After the reaction was completed, the reaction was quenched by adding 50 mL of saturated ammonium chloride aqueous solution at 0°C. The mixture was extracted with ethyl acetate (60 mL × 3). The organic phases were combined, dried over anhydrous sodium sulfate, and filtered to remove the desiccant. The filtrate was concentrated under reduced pressure to obtain the crude product. The crude product was purified by silica gel column chromatography and subjected to a gradient elution with a mobile phase of 0% to 12% ethyl acetate/petroleum ether. The collected fraction was subjected to rotary evaporation under reduced pressure to remove the solvent, yielding compound **11-4** (colorless oily liquid, 290 mg, yield of 79%). MS(ESI, m/z): 355.1 $[M + H]^+$; $^1H$ NMR (400 MHz, $CDCl_3$) $\delta$ 7.61 - 7.56 (m, 1H), 7.44 - 7.39 (m, 2H), 7.35 - 7.29 (m, 1H), 7.23 - 7.19 (m, 1H), 5.29 (s, 2H), 3.51 (s, 3H), 2.72 - 2.57 (m, 1H), 1.40 (s, 12H), 1.05 - 0.97 (m, 2H), 0.69 - 0.59 (m, 2H).

**Step 5:**

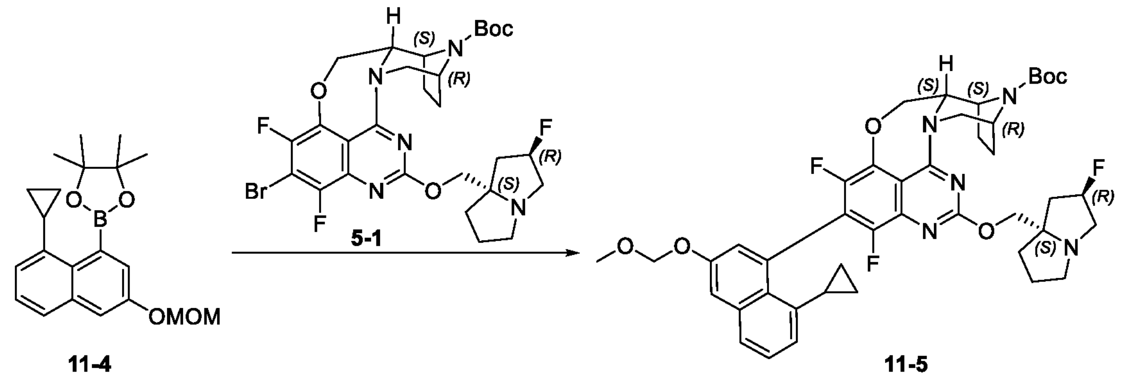

5-1

11-4

11-5

Under nitrogen atmosphere at 25°C, compound **5-1** (200 mg, 0.297 mmol, 1.0 eq), compound **11-4** (143.8 mg, 0.386 mmol, 1.3 eq), tris(dibenzylideneacetone)dipalladium (28.59 mg, 0.03 mmol, 0.1 eq), 4-(anthracen-9-yl)-3-(*tert*-butyl)-2,3-dihydrobenzo[d] [1, 3]oxaphosphole (20.63 mg, 0.06 mmol, 0.2 eq), potassium phosphate (132.56 mg, 0.594 mmol, 2.0 eq), toluene (4.0 mL), and water (0.8 mL) were sequentially added to a reaction flask. The mixture was stirred at 80°C for 3 hours, with the reaction progress monitored by LC-MS and TLC. After the reaction was completed, the reaction mixture was cooled to 25°C. The reaction mixture was concentrated under reduced pressure to remove residual solvent, yielding the crude product. The crude product was purified by silica gel column chromatography and subjected to a gradient elution with a mobile phase of 0% to 10% methanol/dichloromethane. The collected fraction was subjected to rotary evaporation under reduced pressure to remove the solvent, yielding compound **11-5** (white solid, 180 mg, yield of 73%). MS (ESI, m/z): 788.4 $[M + H]^+$.

**Step 6:**

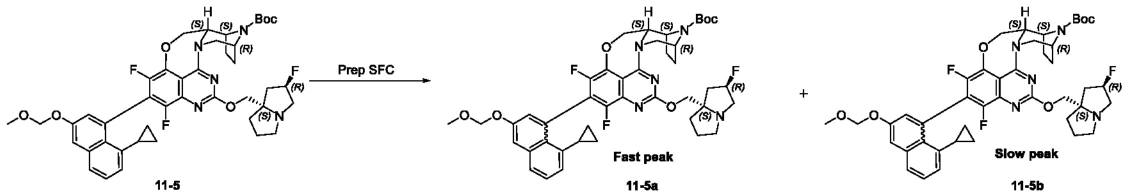


Fast peak

Slow peak

11-5

11-5a

11-5b

The compound **11-5** (180 mg) obtained from **step 5** was subjected to chiral resolution using supercritical fluid chromatography: chiral column: CHIRALPAK ID, 2 × 25 cm, 5 μm; mobile phase A: n-hexane/methyl tert-butyl ether (1/1) (0.5%, 2 M ammonia in methanol), mobile phase B: ethanol; flow rate: 20 mL/min; elution with 30% mobile phase B; detector: UV 226 nm, resulting in two products. The product with a shorter retention time (5.20 minutes) was compound **11-5a** (white solid, 62 mg, yield of 44%); compound **11-5a:** MS (ESI, *m/z):* 788.4 $[M + H]^+$. The product with a longer retention time (8.19 minutes) was compound **11-5b** (white solid, 57 mg, yield of 44%); compound **11-5b:** MS (ESI, *m/z):* 788.4 $[M + H]^+$.

**Step 7:**

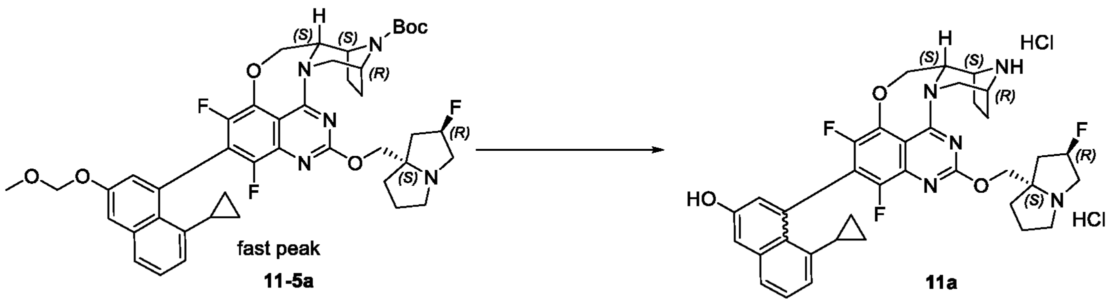

fast peak

11-5a

11a

With stirring at 0°C, a solution of hydrochloric acid in 1,4-dioxane (4 M, 2 mL) was added dropwise to a solution of compound **11-5a** (67 mg, 0.081 mmol, 1.00 eq) in methanol (2 mL). The mixture was reacted at room temperature for 1.5 hours, with the reaction progress monitored by LC-MS. After the reaction was completed, the reaction mixture was concentrated to obtain the crude product. The crude product was purified by reverse-phase flash chromatography (C18 column) and eluted with a mobile phase of 5% to 95% acetonitrile/water (0.1% hydrochloric acid) over 25 minutes, with detection at UV 254 nm. The product obtained was compound 11a (white solid, 41 mg, yield of 69%). MS (ESI, m/z):644.3 $[M + H]^+$, $^1H$ NMR (400 MHz, DMSO-$d_6$) $\delta$ 11.36 (s, 1H), 10.38 - 9.72 (m, 3H), 7.69 (d, *J* = 8.0 Hz, 1H), 7.37 - 7.31 (m, 2H), 7.13 (d, *J* = 7.2 Hz, 1H), 7.01 (d, *J* = 2.4 Hz, 1H), 5.65 - 5.48 (m, 1H), 4.99 (d, *J* = 14.0 Hz, 1H), 4.75 - 4.68 (m, 1H), 4.65 - 4.54 (m, 4H), 4.30 - 4.22 (m, 2H), 3.80 - 3.70 (m, 3H), 3.65 (d, *J* = 14.0 Hz, 1H), 3.35 - 3.22 (m, 1H), 2.64 - 2.52 (m, 1H), 2.49 - 2.45 (m, 1H), 2.37 - 2.27 (m, 1H), 2.22 - 1.88 (m, 7H), 1.58 - 1.46 (m, 1H), 0.55 - 0.44 (m, 2H), 0.34 - 0.27 (m, 1H), 0.13 - 0.07 (m, 1H); $^{19}F$ NMR (377 MHz, DMSO-$d_6$) $\delta$ -132.06, -139.03, -172.75.

**Step 8:**

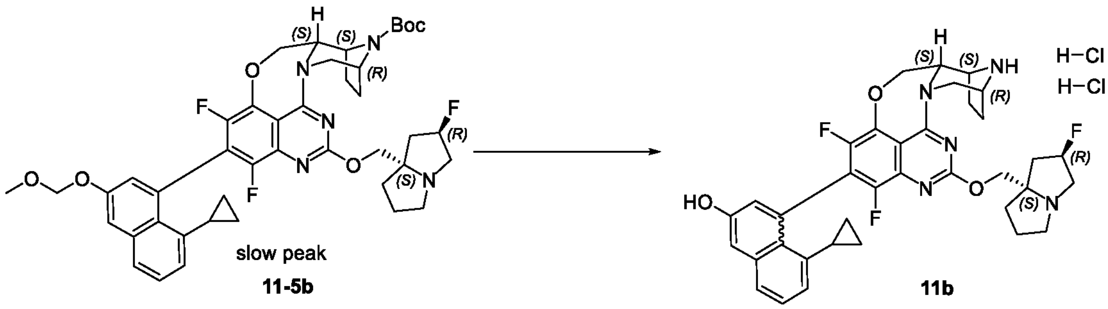

slow peak
**11-5b**

**11b**

With stirring at 0°C, a solution of hydrochloric acid in 1,4-dioxane (4 M, 2 mL) was added dropwise to a solution of compound **11-5b** (57 mg, 0.069 mmol, 1.00 eq) in methanol (2 mL). The mixture was reacted at room temperature for 1.5 hours, with the reaction progress monitored by LC-MS and TLC. After the reaction was completed, the reaction mixture was concentrated to obtain the crude product. The crude product was purified by reverse-phase flash chromatography (C18 column) and eluted with a mobile phase of 5% to 95% acetonitrile/water (0.1% hydrochloric acid) over 25 minutes, with detection at UV 254 nm. The product obtained was compound **11b** (white solid, 33.1 mg, yield of 65%). MS (ESI, m/z):644.3 $[M + H]^+$, $^1H$ NMR (400 MHz, DMSO-$d_6$) $\delta$ 11.54 (s, 1H), 10.47 - 9.79 (m, 3H), 7.69 (d, *J* = 8.0 Hz, 1H), 7.38 - 7.29 (m, 2H), 7.14 (d, *J* = 7.2 Hz, 1H), 7.01 (d, *J* = 2.4 Hz, 1H), 5.68 - 5.46 (m, 1H), 5.04 (d, *J* = 14.0 Hz, 1H), 4.81 - 4.69 (m, 1H), 4.66 - 4.53 (m, 4H), 4.33 - 4.22 (m, 2H), 3.86 - 3.78 (m, 3H), 3.66 (d, *J* = 14.0 Hz, 1H), 3.33 - 3.22 (m, 1H), 2.64 - 2.53 (m, 1H), 2.49 - 2.42 (m, 1H), 2.37 - 2.27 (m, 1H), 2.23 - 1.83 (m, 7H), 1.62 - 1.50 (m, 1H), 0.64 - 0.54 (m, 1H), 0.50 - 0.43 (m, 1H), 0.34 - 0.20 (m, 2H); $^{19}F$ NMR (377 MHz, DMSO-$d_6$) $\delta$ -132.34, -138.67, -172.64.

**Example 12**

4-((2*R* or 2*S*, 5a*S*,6*S*,9*R*)-1,3-Difluoro-13-(((2*R*,7a*S*)-2-fluorotetrahydro-1*H*-pyrrolizin-7a(5*H*)-yl)methoxy)-5a,6,7,8,9,10-hexahydro-5*H*-6,9-epiminoazepino[2',1':3,4][1,4]oxazepino[5,6,7-*de*]quinazolin-2-yl)-5-(2-methoxyethyl)naphthalen-2-ol dihydrochloride **12a;** 4-((2*S* or 2*R*, 5a*S*,6*S*,9*R*)-1,3-difluoro-13-(((2*R*,7a*S*)-2-fluorotetrahydro-1*H*-pyrrolizin-7a(5*H*)-yl)methoxy)-5a,6,7,8,9,10-hexahydro-5*H*-6,9-epiminoazepino[2',1':3,4][1,4]oxazepino[5,6,7-*de*]quinazolin-2-yl)-5-(2-methoxyethyl)naphthalen-2-ol dihydrochloride **12b**

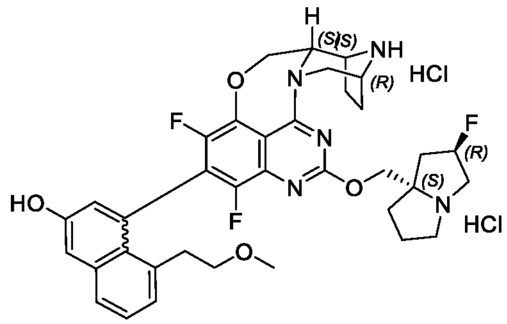

12a

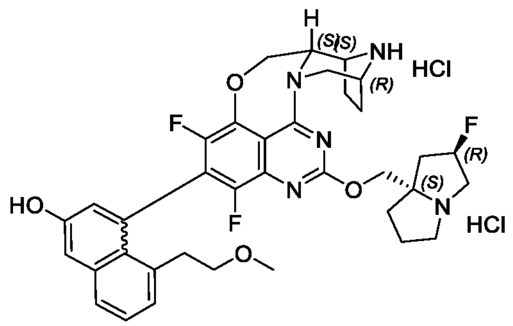

12b

The synthetic route is as follows:

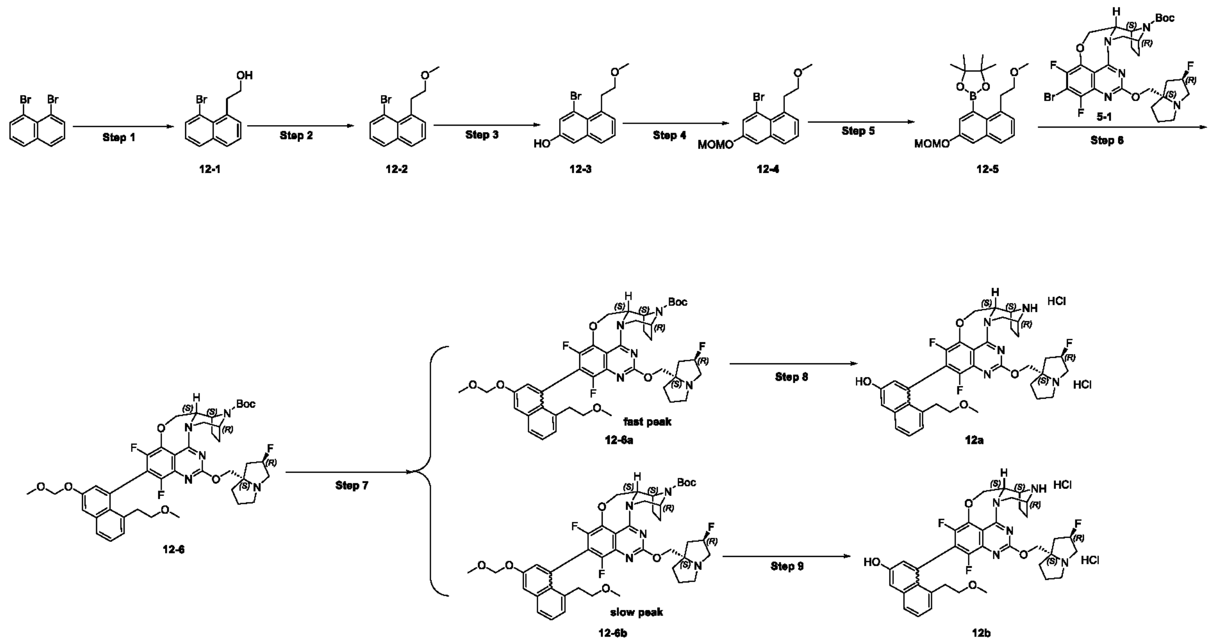


**Step 1:**

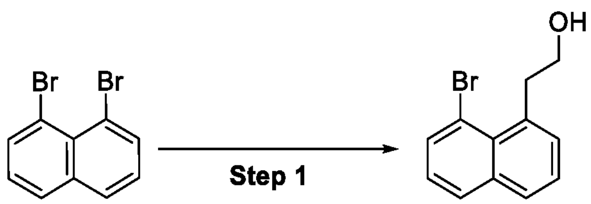


12-1

Under nitrogen atmosphere at -78°C with stirring, a solution of n-butyllithium in *n*-hexane (2.5 M, 31.9 mL, 79.729 mmol, 1.2 eq) was slowly added dropwise to a solution of 1,8-dibromonaphthalene (20 g, 66.441 mmol, 1.0 eq) in anhydrous tetrahydrofuran (200 mL). The mixture was reacted with stirring at -78°C under nitrogen atmosphere for 0.5 hours, and then a solution of ethylene oxide in anhydrous tetrahydrofuran (2.5 M, 265.8 mL, 664.410 mmol, 10 eq) was slowly added dropwise to the mixture. The mixture was reacted with stirring under nitrogen atmosphere at 0°C for 0.5 hours. The reaction was monitored by LC-MS and TLC. After the reaction was completed, at 0°C, the reaction mixture was added with water (500 mL) to quench the reaction. The mixture was extracted with ethyl acetate (1 L × 3). The organic phases were combined, washed with saturated brine (500 mL), dried over anhydrous sodium sulfate, and filtered to

remove the desiccant. The filtrate was subjected to rotary evaporation under reduced pressure to remove the solvent, yielding the crude product. The resulting crude product was purified by reverse-phase chromatography (C18 column) and eluted with a mobile phase of 40% to 95% acetonitrile/water (10 mmol/L ammonium bicarbonate aqueous solution) over 30 minutes. The collected fraction was subjected to rotary evaporation under reduced pressure to remove the solvent, yielding compound 12-1 (white solid, 9.5 g, yield of 54%). $^1H$ NMR (300 MHz, $CDCl_3$) $\delta$ 7.91 - 7.76 (m, 3H), 7.51 - 7.40 (m, 2H), 7.29 - 7.24 (m, 1H), 4.10 - 3.99 (m, 2H), 3.94 - 3.83 (m, 2H).

**Step 2:**

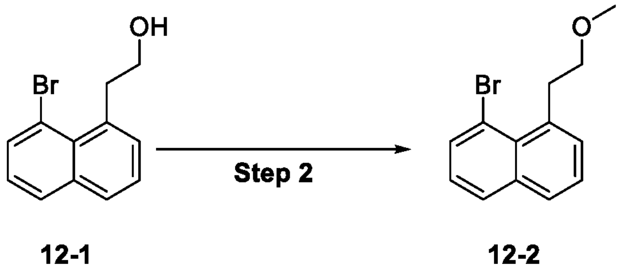


**12-1** **12-2**

Under nitrogen atmosphere at 0°C with stirring, sodium hydride (60%, 1.51 g, 37.83 mmol, 2.0 eq) was added to a solution of compound **12-1** (5 g, 18.915 mmol, 1.0 eq) in anhydrous tetrahydrofuran (50 mL) in batches. After the addition was completed, the mixture was stirred at 0°C under nitrogen atmosphere for 30 minutes, then iodomethane (4.24 g, 28.372 mmol, 1.5 eq) was added dropwise thereto. After the addition was completed, the mixture was reacted with stirring at 25°C under nitrogen atmosphere for 1 hour, with the reaction progress monitored by TLC. After the reaction was completed, the reaction mixture was carefully poured into ice water (250 mL) to quench the reaction. The mixture was then extracted with ethyl acetate (250 mL × 3). The organic phases were combined, dried over anhydrous sodium sulfate, and filtered to remove the desiccant. The filtrate was concentrated under reduced pressure to obtain a crude product. The crude product was purified by silica gel column chromatography and subjected to a gradient elution with a mobile phase of 0% to 10% ethyl acetate/petroleum ether. The collected fraction was subjected to rotary evaporation under reduced pressure to remove the solvent, yielding compound **12-2** (orange liquid, 5.1 g, yield of 97%). $^1H$ NMR (400 MHz, $CDCl_3$) $\delta$ 7.88 - 7.72 (m, 3H), 7.48 - 7.37 (m, 2H), 7.25 - 7.18 (m, 1H), 3.89 - 3.82 (m, 2H), 3.79 - 3.72 (m, 2H), 3.39 (s, 3H).

**Step 3:**

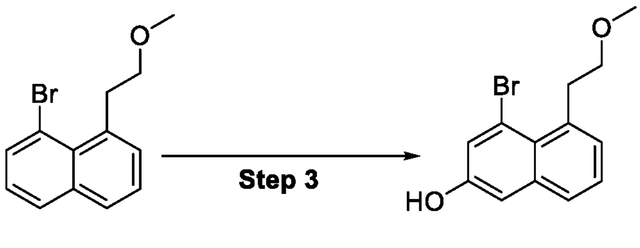


**12-2** **12-3**

Under nitrogen atmosphere at 25°C with stirring, compound **12-2** (2.6 g, 9.315 mmol, 1.0 eq), n-hexane (26 mL), bis(pinacolato)diboron (3.24 g, 12.110 mmol, 1.3 eq), bis(1,5-cyclooctadiene)di-methoxyiridium(I) dimer (0.65 g, 0.931 mmol, 0.1 eq), and 4,4'-di-*tert*-butyl-2,2'-bipyridine (0.53 g, 1.863 mmol, 0.2 eq) were sequentially added to a 100 mL three-neck flask. The mixture was reacted with stirring under nitrogen atmosphere at 60°C for 2 hours. The reaction progress was monitored by LC-MS and TLC. After the reaction was completed, the reaction mixture was concentrated. With stirring at 0°C, tetrahydrofuran (8 mL), water (4 mL), acetic acid (12 mL), and hydrogen peroxide (6 mL) were sequentially added to the resulting mixture. The mixture was reacted with stirring at 0°C for 0.5 hours. The reaction progress was monitored by LC-MS and TLC. After the reaction was completed, saturated sodium bicarbonate solution (100 mL) was added at 0°C with stirring. The resulting mixture was extracted with ethyl acetate (100 mL × 3). The organic phases were combined, dried, and filtered to remove the desiccant. The filtrate was concentrated under reduced pressure

to obtain a crude product. The crude product was purified by silica gel column chromatography and subjected to a gradient elution with a mobile phase of 0% to 30% ethyl acetate/petroleum ether. The collected fraction was subjected to rotary evaporation under reduced pressure to remove the solvent, yielding compound **12-3** (white solid, 870 mg, yield of 32%). MS (ESI, m/z): 278.9/280.9 $[M + H]^+$, $^1H$ NMR (400 MHz, $CDCl_3$) $\delta$ 7.60 - 7.53 (m, 1H), 7.52 (d, *J* = 2.7 Hz, 1H), 7.34 - 7.27 (m, 2H), 7.13 (d, *J* = 2.7 Hz, 1H), 3.83 - 3.77 (m, 2H), 3.76 - 3.70 (m, 2H), 3.39 (s, 3H).

**Step 4:**

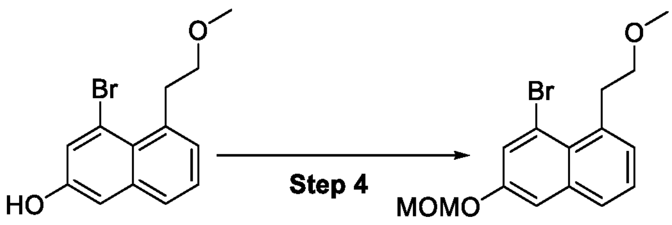


**12-3** **12-4**

Under nitrogen atmosphere at 0°C with stirring, compound 12-3 (850 mg, 2.872 mmol, 1.00 eq), dichloromethane (9 mL), *N,N*-diisopropylethylamine (390.75 mg, 2.872 mmol, 1.0 eq), and chloromethyl methyl ether (301.48 mg, 4.308 mmol, 1.5 eq) were sequentially added to a 50 mL three-neck flask. The mixture was reacted with stirring under nitrogen atmosphere at 25°C for 1 hour. The reaction progress was monitored by LC-MS and TLC. After the reaction was completed, the reaction mixture was added with water (50 mL) at 0°C to quench the reaction. The mixture was extracted with dichloromethane (50 mL $\times$ 3). The organic phases were combined, washed with saturated brine (100 mL), dried over anhydrous sodium sulfate, and filtered to remove the desiccant. The filtrate was subjected to rotary evaporation under reduced pressure to remove the solvent, yielding the crude product. The resulting crude product was purified by silica gel column chromatography and subjected to a gradient elution with a mobile phase of 0% to 10% ethyl acetate/petroleum ether. The collected fraction was subjected to rotary evaporation under reduced pressure to remove the solvent, yielding compound **12-4** (pale yellow solid, 780 mg, yield of 79%). $^1H$ NMR (400 MHz, $CDCl_3$) $\delta$ 7.67 - 7.61 (m, 2H), 7.40 - 7.28 (m, 3H), 5.26 (s, 2H), 3.84 - 3.77 (m, 2H), 3.76 - 3.70 (m, 2H), 3.51 (s, 3H), 3.38 (s, 3H).

**Step 5:**

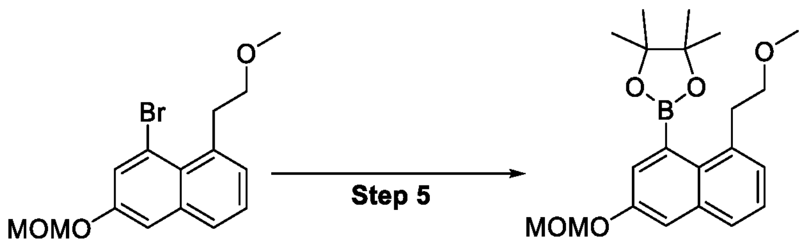


**12-4** **12-5**

Under nitrogen atmosphere at -78°C with stirring, a solution of compound **12-4** (400 mg, 1.169 mmol, 1.00 eq) in anhydrous tetrahydrofuran (4 mL) was added to a 25 mL Schlenk tube, followed by a dropwise addition of a solution of n-butyllithium in n-hexane (2.5 M, 0.61 mL, 1.52 mmol, 1.3 eq). The mixture was stirred at -78°C for 0.5 hours. Subsequently, under nitrogen atmosphere at -78°C with stirring, a solution of 2-isopropoxy-4,4,5,5-tetramethyl-1,3,2-dioxaborolane (411.94 mg, 2.104 mmol, 1.8 eq) in anhydrous tetrahydrofuran (1 mL) was slowly added dropwise to the reaction mixture. The mixture was reacted with stirring at -78°C under nitrogen atmosphere for 1 hour, and then reacted at 25°C under nitrogen atmosphere for an additional 0.5 hours. The reaction progress was monitored by LC-MS and TLC. After the reaction was completed, with stirring at 0°C, the reaction mixture was added with saturated ammonium chloride solution (50 mL) to quench the reaction. The mixture was extracted with ethyl acetate (50 mL $\times$ 3). The organic phases were combined, washed with saturated brine (100 mL), dried over anhydrous sodium sulfate, and filtered to remove the

desiccant. The filtrate was subjected to rotary evaporation under reduced pressure to remove the solvent, yielding the crude product. The crude product was purified by silica gel column chromatography and subjected to a gradient elution with a mobile phase of 0% to 10% methyl *tert*-butyl ether/petroleum ether. The collected fraction was subjected to rotary evaporation under reduced pressure to remove the solvent, yielding compound **12-5** (colorless oil, 440 mg, yield of 97%). $^1H$ NMR (400 MHz, $CDCl_3$) $\delta$ 7.64 - 7.58 (m, 1H), 7.40 (d, *J* = 2.7 Hz, 1H), 7.37 - 7.32 (m, 2H), 7.30 - 7.25 (m, 1H), 5.28 (s, 2H), 3.68 (t, *J* = 7.5 Hz, 2H), 3.50 (s, 3H), 3.43 (t, *J* = 7.5 Hz, 2H), 3.34 (s, 3H), 1.45 (s, 12H).

**Step 6:**

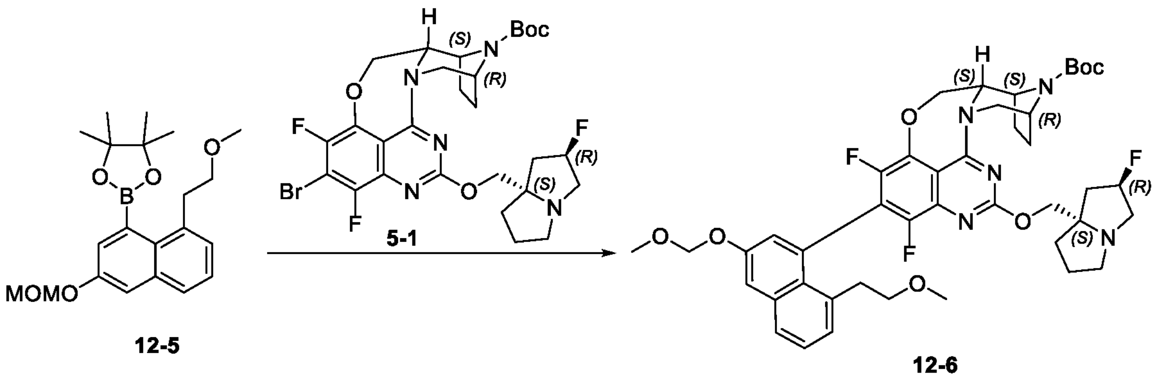


Under nitrogen atmosphere at 25°C, compound **5-1** (150 mg, 0.22 mmol, 1.0 eq), compound **12-5** (92.90 mg, 0.24 mmol, 1.1 eq), tris(dibenzylideneacetone)dipalladium (21.45 mg, 0.022 mmol, 0.1 eq), 4-(anthracen-9-yl)-3-(*tert*-butyl)-2,3-dihydrobenzo[*d*][1,3]oxaphosphole (15.47 mg, 0.044 mmol, 0.2 eq), potassium phosphate (149.13 mg, 0.66 mmol, 3.0 eq), toluene (2.0 mL), and water (0.4 mL) were sequentially added to a reaction flask. The mixture was stirred at 80°C for 12 hours, with the reaction progress monitored by LC-MS and TLC. After the reaction was completed, the reaction mixture was cooled to 25°C. The reaction mixture was concentrated under reduced pressure to remove residual solvent, yielding the crude product. The crude product was purified by silica gel column chromatography and subjected to a gradient elution with a mobile phase of 0% to 10% methanol/dichloromethane. The collected fraction was subjected to rotary evaporation under reduced pressure to remove the solvent, yielding compound **12-6** (white solid, 140 mg, yield of 74%). MS (ESI, m/z): 806.4 $[M + H]^+$.

**Step 7:**

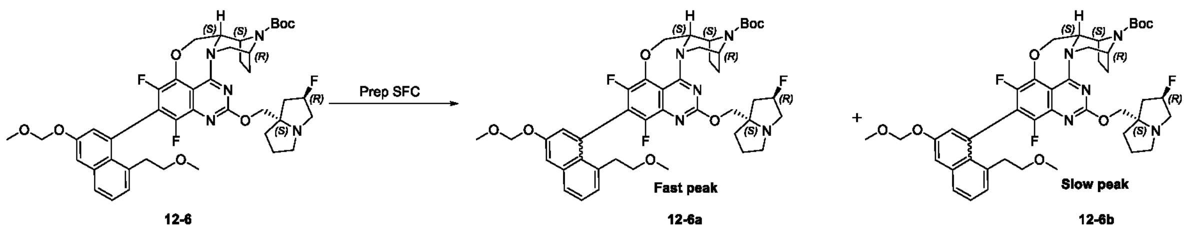


The compound **12-6** (140 mg) obtained from **step 6** was subjected to chiral resolution using supercritical fluid chromatography: chiral column: CHIRALPAK ID, 3 × 25 cm, 5 μm; mobile phase A: *n*-hexane/methyl *tert*-butyl ether (1/1) (0.5%, 2 M ammonia in methanol), mobile phase B: ethanol; flow rate: 40 mL/min; elution with 10% mobile phase B; detector: UV 228 nm, resulting in two products. The product with a shorter retention time (7.65 minutes) was compound 12-6a (white solid, 55 mg, yield of 40%); compound **12-6a:** MS (ESI, *m*/*z*): 806.4 $[M + H]^+$. The product with a longer retention time (9.65 minutes) was compound **12-6b** (white solid, 62 mg, yield of 44%); compound **12-6b:** MS (ESI, *m*/*z):* 806.4 $[M + H]^+$.

**Step 8:**

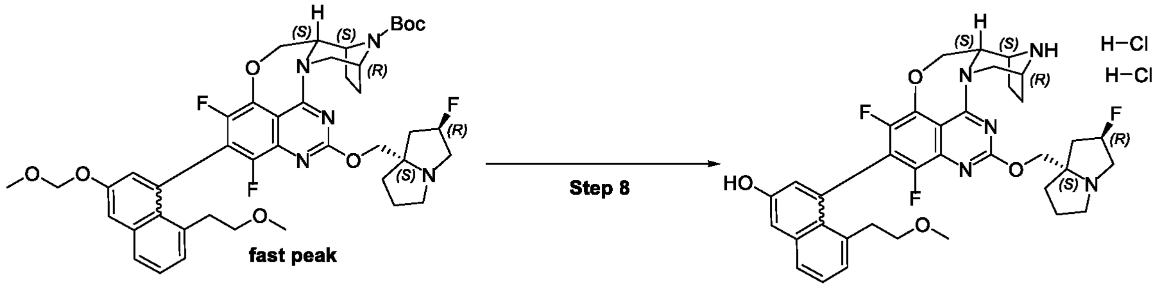


12-6a 12a

With stirring at 0°C, a solution of hydrochloric acid in 1,4-dioxane (4 M, 2 mL) was added dropwise to a solution of compound **12-6a** (55 mg, 0.065 mmol, 1.00 eq) in methanol (2 mL). The mixture was reacted at room temperature for 1.5 hours, with the reaction progress monitored by LC-MS and TLC. After the reaction was completed, the reaction mixture was concentrated to obtain the crude product. The crude product was purified by reverse-phase flash chromatography (C18 column) and eluted with a mobile phase of 5% to 95% acetonitrile/water (0.1% hydrochloric acid) over 25 minutes, with detection at UV 254 nm. The product obtained was compound **12a** (yellow solid, 29 mg, yield of 59%). MS (ESI, m/z): 662.3 $[M+H]^+$, $^1$H NMR (400 MHz, $CD_3OD$-$d_4$) $\delta$ 7.68 (d, $J$ = 8.4 Hz, 1H), 7.40 - 7.33 (m, 1H), 7.33 - 7.29 (m, 1H), 7.21 - 7.14 (m, 1H), 7.04 - 6.94 (m, 1H), 5.67 - 5.33 (m, 2H), 4.84 - 4.53 (m, 6H), 4.46 - 4.33 (m, 2H), 4.09 - 3.82 (m, 3H), 3.80 - 3.63 (m, 1H), 3.53 - 3.41 (m, 1H), 3.28 - 3.24 (m, 1H), 3.18 - 3.05 (m, 3H), 2.79 - 2.57 (m, 4H), 2.53 - 2.44 (m, 1H), 2.43 - 2.30 (m, 3H), 2.29 - 2.08 (m, 4H); $^{19}$F NMR (377 MHz, $CD_3OD$-$d_4$) $\delta$ -134.56, -137.61, -174.26.

**Step 9:**

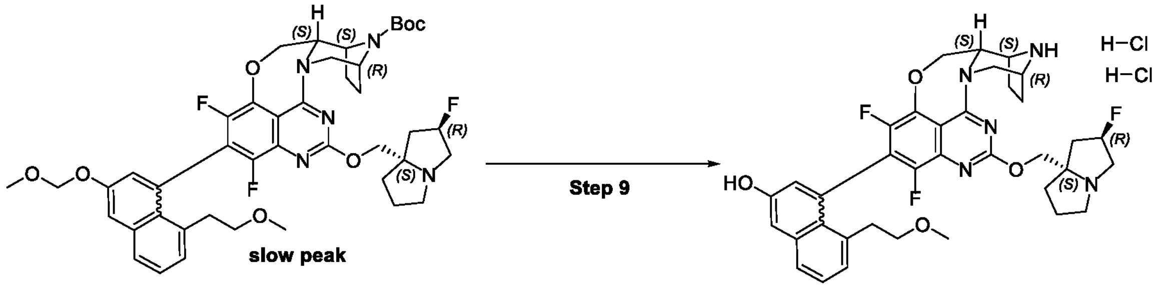


12-6b 12b

With stirring at 0°C, a solution of hydrochloric acid in 1,4-dioxane (4 M, 2 mL) was added dropwise to a solution of compound **12-6b** (55 mg, 0.065 mmol, 1.00 eq) in methanol (2 mL). The mixture was reacted at room temperature for 1.5 hours, with the reaction progress monitored by LC-MS and TLC. After the reaction was completed, the reaction mixture was concentrated to obtain the crude product. The crude product was purified by reverse-phase flash chromatography (C18 column) and eluted with a mobile phase of 5% to 95% acetonitrile/water (0.1% hydrochloric acid) over 25 minutes, with detection at UV 254 nm. The product obtained was compound **12b** (yellow solid, 25.9 mg, yield of 46%). MS (ESI, m/z): 662.3 $[M+H]^+$, $^1$H NMR (400 MHz, $CD_3OD$-$d_4$) $\delta$ 7.69 (d, $J$ = 8.4 Hz, 1H), 7.43 - 7.27 (m, 2H), 7.20 - 7.16 (m, 1H), 7.03 - 6.94 (m, 1H), 5.64 - 5.51 (m, 1H), 5.45 - 5.31 (m, 1H), 4.84 - 4.73 (m, 4H), 4.70 -4.50 (m, 2H), 4.43 - 4.36 (m, 2H), 4.05 - 3.84 (m, 3H), 3.76 - 3.64 (m, 1H), 3.49 - 3.42 (m, 1H), 3.28 - 3.25 (m, 1H), 3.17 - 3.10 (m, 3H), 2.78 - 2.58 (m, 4H), 2.53 - 2.43 (m, 1H), 2.39 - 2.30 (m, 3H), 2.24 - 2.14 (m, 4H); $^{19}$F NMR (376 MHz, $CD_3OD$-$d_4$) $\delta$ -134.36, -138.03, -174.28.

**Example 13**

4-((2*S* or 2*R*, 6a*S*,7*S*,10*R*)-1,3-Difluoro-14-(((2*R*,7a*S*)-2-fluorotetrahydro-1*H*-pyrrolizin-7a(5*H*)-yl)methoxy)-5,6,6a,7,8,9,10,11-octahydro-7,10-epiminoazepino[1',2':5,6][1,5]oxazocino[4,3,2-*de*]quinazolin-2-yl)-5-ethylnaphthalen-2-ol **13a';** 4-((2*R* or 2*S*, 6a*S*,7*S*,10*R*)-1,3-difluoro-14-(((2*R*,7a*S*)-2-fluorotetrahydro-1H-pyrrolizin-7a(5*H*)-yl)methoxy)-5,6,6a,7,8,9,10,11-octahydro-7,10-epiminoazepino[1',2':5,6][1,5]oxazocino[4,3,2-*de*]quinazolin-2-yl)-5-ethylnaphthalen-2-ol dihydrochloride **13b**

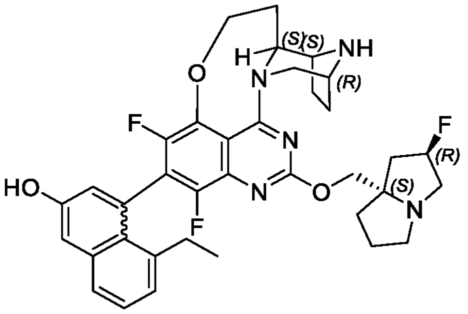

**13a'**

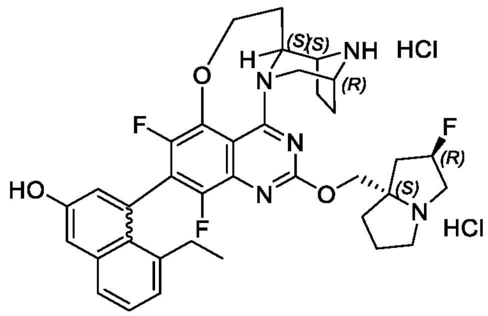

**13b**

The synthetic route is as follows:

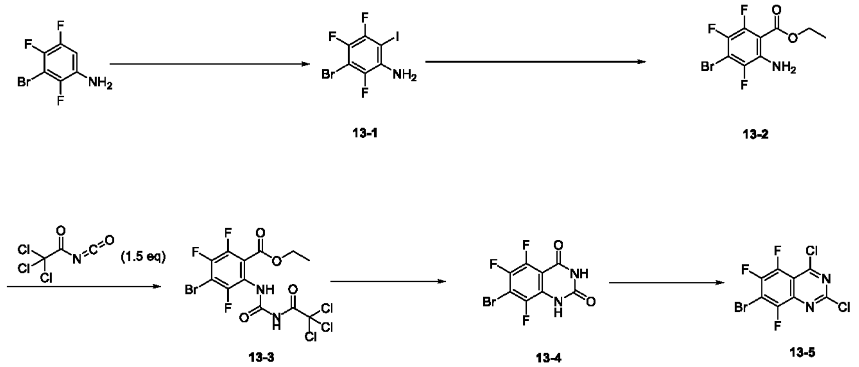


13-1 13-2 13-3 13-4 13-5

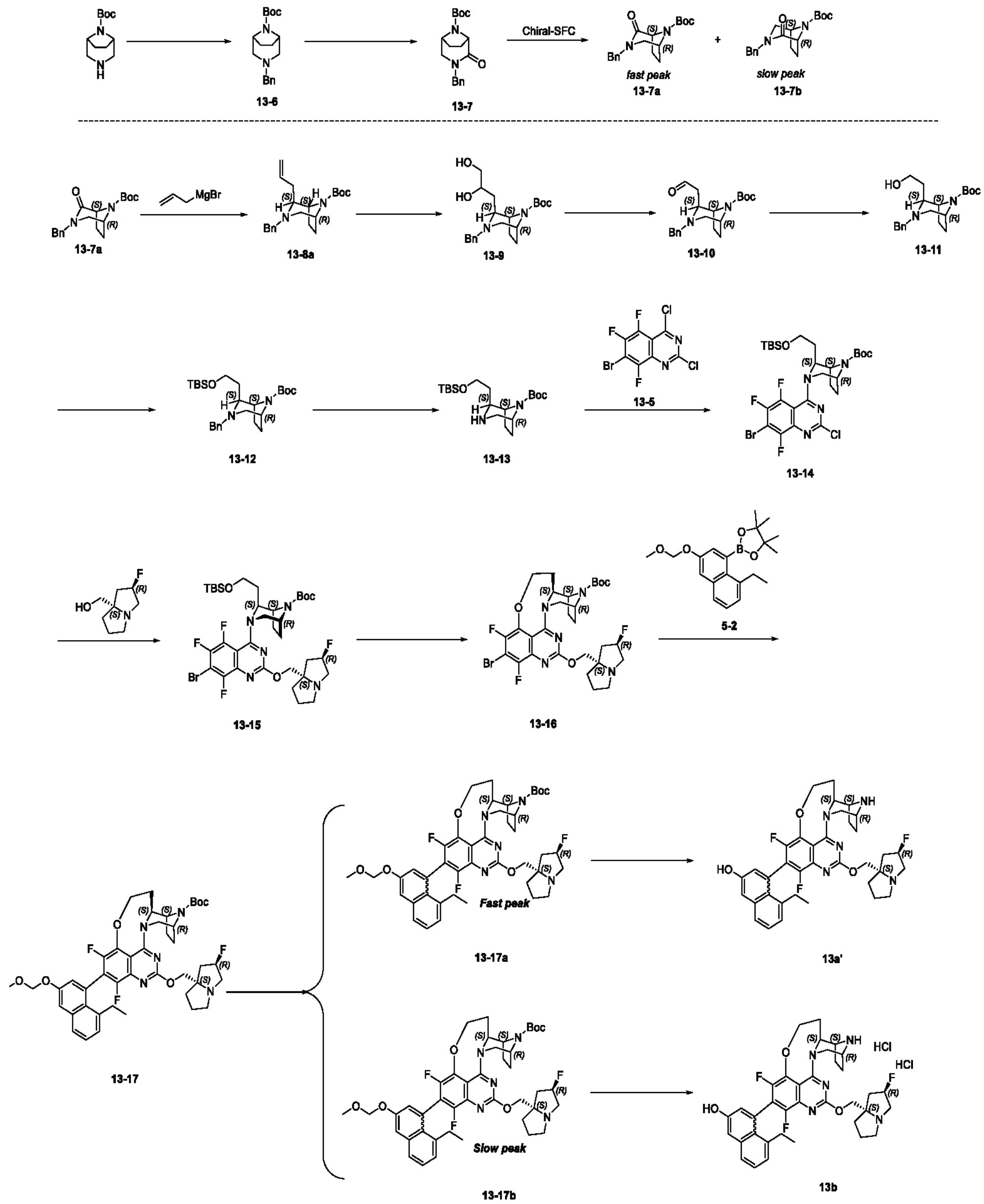


**Step 1:**

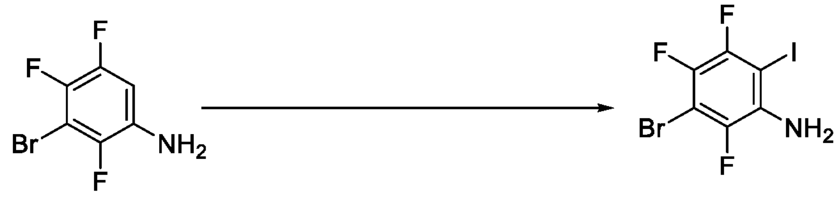

**13-1**

With stirring at 25°C, *N*-iodosuccinimide (29.87 g, 126.108 mmol, 1.5 eq) was added in batches to a solution of compound 3-bromo-2,4,5-trifluoroaniline (25 g, 105.09 mmol, 1 eq) and p-toluenesulfonic acid monohydrate (2.1 g, 10.506 mmol, 0.1 eq) in acetonitrile (300 mL). The mixture was reacted with stirring at 60°C under nitrogen atmosphere for 3 hours. The reaction progress was monitored by LC-MS and TLC. After the reaction was completed, the mixture was

cooled to room temperature and concentrated under reduced pressure to remove excess solvent. After the solvent was removed, the remaining solid was dissolved in 500 mL of ethyl acetate, and the resulting mixed solution was washed with saturated sodium thiosulfate solution (500 mL × 3). The organic phase was dried over anhydrous sodium sulfate, and then filtered to remove the desiccant. The filtrate was subjected to rotary evaporation under reduced pressure to obtain a crude product. The crude product was purified by silica gel column chromatography and subjected to a gradient elution with a mobile phase of 0% to 10% ethyl acetate/petroleum ether. The collected fraction was subjected to rotary evaporation under reduced pressure to remove the solvent, yielding compound **13-1** as a brown solid (30 g, yield of 73%). $^{1}$H-NMR (400 MHz, $CDCl_3$) $\delta$ 4.20 (s, 2H); $^{19}$F-NMR (377 MHz, $CDCl_3$) $\delta$ -117.85, -128.47, -141.77.

**Step 2:**

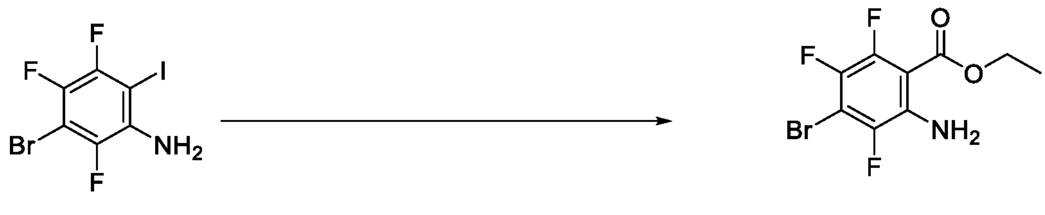

**13-1** **13-2**

Under nitrogen atmosphere at 25°C with stirring, bis(triphenylphosphine)palladium(II) chloride (3.39 g, 4.59 mmol, 0.1 eq) and triethylamine (24.17 mL, 165.22 mmol, 3.6 eq) were sequentially added to a solution of compound **13-1** (17 g, 45.895 mmol, 1 eq) in ethanol (500 mL). The mixture was reacted at 80°C under a carbon monoxide atmosphere at 5 atm for 16 hours. The reaction progress was monitored by LC-MS and TLC. After the reaction was completed, the mixture was cooled to room temperature and concentrated under reduced pressure to remove excess solvent, yielding the crude product. The crude product was purified by silica gel column chromatography and subjected to a gradient elution with a mobile phase of 0% to 10% ethyl acetate/petroleum ether. The collected fraction was subjected to rotary evaporation under reduced pressure to remove the solvent, yielding compound **13-2** as a brown solid (10.5 g, yield of 72%). $^{1}$H-NMR (400 MHz, $CDCl_3$) $\delta$ 5.67 (s, 2H), 4.50 - 4.35 (m, 2H), 1.44 - 1.36 (m, 3H); $^{19}$F-NMR (377 MHz, $CDCl_3$) $\delta$ -132.94, -135.73, -147.14.

**Step 3:**

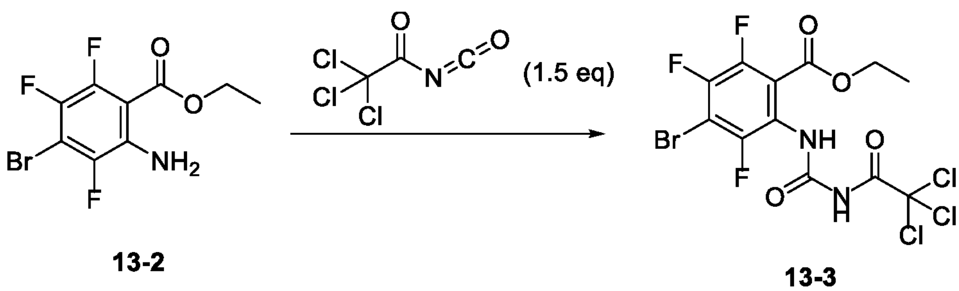


**13-2** **13-3**

Under nitrogen atmosphere at 0°C with stirring, trichloroacetyl isocyanate (4.74 g, 23.904 mmol, 1.5 eq) was slowly added dropwise to a solution of compound **13-2** (5 g, 15.936 mmol, 1 eq) in tetrahydrofuran (50 mL). The mixture was reacted at 25°C for 0.5 hours. The reaction progress was monitored by LC-MS and TLC. After the reaction was completed, the mixture was concentrated under reduced pressure to remove excess solvent, yielding the crude product. The resulting crude product was slurried with methyl *tert*-butyl ether (50 mL) and purified to obtain compound **13-3** as a brown solid (5.7 g, yield of 69%). MS(ESI, m/z): 484.8/486.8.

**Step 4:**

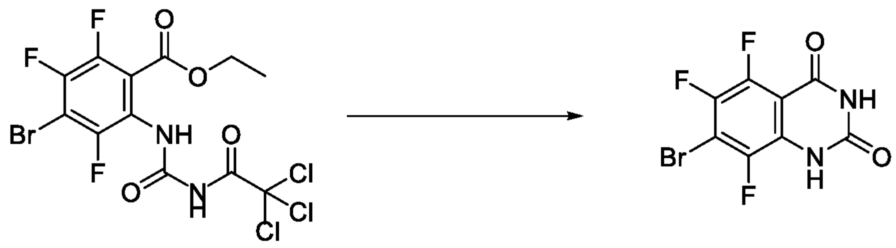

13-3 13-4

With stirring at 0°C, a solution of ammonia in methanol (7 M, 10 mL) was slowly added dropwise to a solution of compound 13-3 (5.7 g, 11.13 mmol, 1 eq) in methanol (100 mL). The mixture was reacted at 25°C for 1 hour. The reaction progress was monitored by LC-MS and TLC. After the reaction was completed, the mixture was concentrated under reduced pressure to remove excess solvent, yielding the crude product. The resulting crude product was slurried with methyl *tert-butyl* ether (50 mL) and purified to obtain compound **13-4** (brown solid, 3.18 g, yield of 91%). $^{1}$H-NMR (400 MHz, DMSO-$d_6$) $\delta$ 11.62 (s, 1H), 11.58 (s, 1H); $^{19}$F-NMR (377 MHz, DMSO) $\delta$ -125.47, -139.98, -143.15.

**Step 5:**

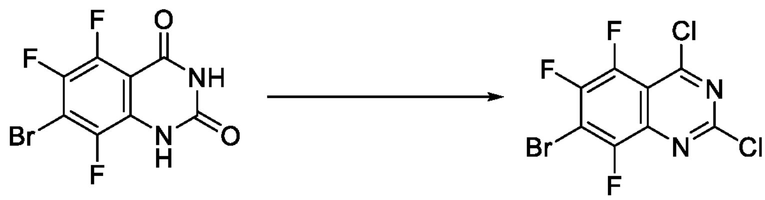

13-4 13-5

Under nitrogen atmosphere at 0°C with stirring, triethylamine (1 mL) was slowly added dropwise to a solution of compound **13-4** (1 g, 3.22 mmol, 1 eq) in phosphorus oxychloride (15 mL). The mixture was reacted at 100°C for 16 hours. The reaction progress was monitored by LC-MS and TLC. After the reaction was completed, the mixture was concentrated under reduced pressure to remove excess solvent, yielding the crude product. The crude product was purified by silica gel column chromatography and subjected to a gradient elution with a mobile phase of 0% to 10% ethyl acetate/petroleum ether. The collected fraction was subjected to rotary evaporation under reduced pressure to remove the solvent, yielding compound **13-4** as a brown solid (850 mg, yield of 75%). $^{19}$F-NMR (377 MHz, DMSO) $\delta$ -115.15, -124.03, -137.27.

**Step 6:**

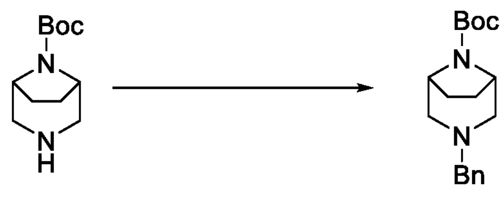

13-6

Under nitrogen atmosphere at 0°C with stirring, benzyl bromide (26.59 g, 147.67 mmol, 1.1 eq) was slowly added dropwise to a solution of compound 8-*tert*-butoxycarbonyl-3,8-diazabicyclo[3.2.1]octane (30 g, 134.2 mmol, 1 eq) and anhydrous potassium carbonate (39.09 g, 268.496 mmol, 2 eq) in acetonitrile (300 mL). The mixture was reacted at 80°C for 6 hours. The reaction progress was monitored by LC-MS and TLC. After the reaction was completed, the reaction system was cooled to room temperature, then filtered to remove the excess potassium carbonate. The filtrate was concentrated under reduced pressure to obtain the crude product. The crude product was purified by silica gel column chromatography and subjected to a gradient elution with a mobile phase of 0% to 30% methyl tert-butyl ether/petroleum

ether. The collected fraction was subjected to rotary evaporation under reduced pressure to remove the solvent, yielding compound **13-6** (brown solid, 33 g, yield of 77%). MS(ESI, m/z): 303.4 [M+H]$^+$, $^1$H-NMR (400 MHz, $CDCl_3$) $\delta$ 7.30 (d, $J$ = 4.5 Hz, 4H), 7.25 - 7.19 (m, 1H), 4.15 - 4.11 (m, 2H), 3.47 (s, 2H), 2.62 - 2.58 (m, 2H), 2.29 - 2.25 (m, 2H), 1.99 - 1.74 (m, 4H), 1.46 (s, 9H).

**Step 7:**

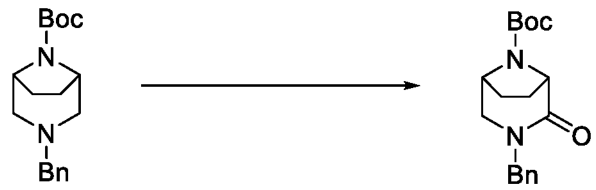

**13-6** **13-7**

With stirring at 0°C, iodine (221.57 g, 829.312 mmol, 8 eq) and sodium bicarbonate (91.67 g, 1.04 mol, 10 eq) were sequentially added to a mixed solution of compound **13-6** (33 g, 103.6 mmol, 1 eq) in water (330 mL) and dimethyl sulfoxide (825 mL). The resulting mixture was reacted at 25°C for 16 hours. The reaction progress was monitored by LC-MS and TLC. After the reaction was completed, the system was cooled to 0°C, then slowly added with 1 L of saturated sodium thiosulfate solution to quench the reaction. The mixture was extracted with ethyl acetate (500 mL × 3). The organic phases were combined, washed with saturated brine (1 L × 3), dried over anhydrous sodium sulfate, and filtered to remove the desiccant. The filtrate was subjected to rotary evaporation under reduced pressure to remove the solvent, yielding the crude product. The crude product was slurried with methyl tert-butyl ether/n-hexane (1/5, 200 mL) and purified, yielding compound **13-7** (white solid, 29 g, yield of 84%). MS(ESI, m/z): 261.1 [M-$^t$Bu+H]$^+$; $^1$H-NMR (400 MHz, $CDCl_3$) $\delta$ 7.33 - 7.24 (m, 3H), 7.21 - 7.15 (m, 2H), 4.71 - 4.32 (m, 4H), 3.72 - 3.51 (m, 1H), 2.89 - 2.74 (m, 1H), 2.26 - 2.10 (m, 3H), 1.67 - 1.55 (m, 1H), 1.44 (s, 9H).

**Step 8:**

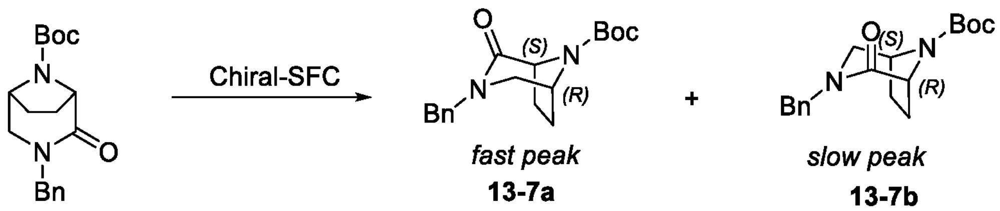


*fast peak* **13-7a** + *slow peak* **13-7b**

**13-7**

The compound **13-7** (28 g) obtained from **step 7** was subjected to chiral resolution using supercritical fluid chromatography: chiral column: CHIRALPAK IC, 5 × 25 cm, 5 μm; mobile phase A: supercritical carbon dioxide, mobile phase B: isopropanol (0.5%, 2 M ammonia in methanol); flow rate: 200 mL/min; column temperature: 35°C; elution with 40% mobile phase B; detector: UV 220 nm, resulting in two products. The product with a shorter retention time (5.26 minutes) was compound **13-7a** (white solid, 11 g, yield of 39%), MS(ESI, m/z): 261.1 [M-$^t$Bu+H]$^+$. The product with a longer retention time (7.92 minutes) was compound **13-7b** (white solid, 11 g, yield of 39%), MS(ESI, m/z): 261.1 [M-$^t$Bu+H]$^+$.

**Step 9:**

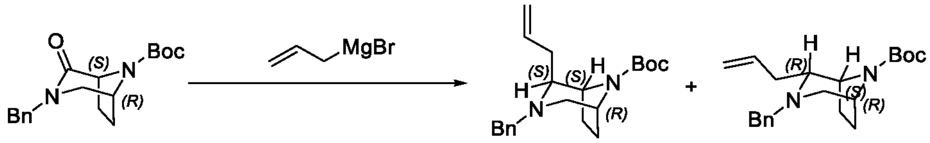

13-7a 13-8a 13-8b

Under nitrogen atmosphere at 0°C with stirring, 1,1,3,3-tetramethyldisiloxane (12.74 g, 90.075 mmol, 3 eq) was slowly added to a solution of compound **13-7a** (10 g, 30.025 mmol, 1 eq) and carbonylchlorobis(triphenylphosphine) iridium(I) (2.47 g, 3.00 mmol, 0.1 eq) in dichloromethane (100 mL). The mixture was reacted at 25°C for 1.5 hours. After the reaction mixture became clear, the system was cooled to -78°C, and 1 M allylmagnesium bromide (45 mL, 45.03 mmol, 1.5 eq) was slowly added dropwise thereto. After the addition was completed, the mixture was slowly warmed to room temperature and reacted for an additional 1 hour. The reaction progress was monitored by LC-MS and TLC. After the reaction was completed, the system was cooled to 0°C, then slowly added with 500 mL of saturated ammonium chloride solution to quench the reaction. The mixture was extracted with ethyl acetate (500 mL × 3). The organic phases were combined, washed with saturated brine (1 L × 1), dried over anhydrous sodium sulfate, and filtered to remove the desiccant. The filtrate was subjected to rotary evaporation under reduced pressure to remove the solvent, yielding the crude product. The resulting crude product was purified by silica gel column chromatography and subjected to a gradient elution with a mobile phase of 0% to 20% methyl *tert*-butyl ether/petroleum ether, yielding two compounds, respectively. Compound **13-8a** (colorless oily liquid, 7.26 g, yield of 67%). MS(ESI, m/z): 343.2 $[M+H]^+$;$^1$H NMR (400 MHz, $CDCl_3$) $\delta$ 7.37 - 7.27 (m, 4H), 7.25 - 7.20 (m, 1H), 5.84 (s, 1H), 5.17 - 4.95 (m, 2H), 4.42 - 4.00 (m, 2H), 3.68 (d, *J*= 13.4 Hz, 1H), 3.50 (d, *J* = 13.5 Hz, 1H), 2.73 - 2.50 (m, 2H), 2.37 - 2.29 (m, 1H), 2.29 - 2.19 (m, 2H), 1.96 - 1.63 (m, 4H), 1.46 (s, 9H). Compound **13-8b** (colorless oily liquid, 240 mg, 2%). MS(ESI, m/z): 343.2 $[M+H]^+$; $^1$H NMR (400 MHz, $CDCl_3$) $\delta$ 7.34 - 7.27 (m, 4H), 7.25 - 7.17 (m, 1H), 5.81 (s, 1H), 5.15 - 5.00 (m, 2H), 4.22 - 3.91 (m, 3H), 3.06 (d, *J*= 13.8 Hz, 1H), 2.69 - 2.40 (m, 3H), 2.37 - 2.13 (m, 1H), 2.11 - 1.90 (m, 2H), 1.88 - 1.63 (m, 3H), 1.45 (s, 9H).

**Step 10:**

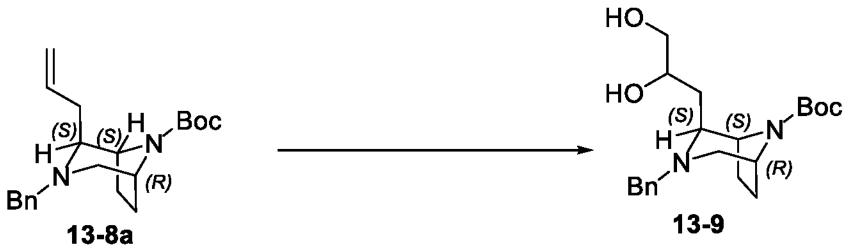

13-8a 13-9

With stirring at 0°C, potassium ferricyanide (4.39 g, 13.3 mmol, 2.4 eq), potassium osmate dihydrate (220 mg, 0.555 mmol, 0.1 eq), triethylenediamine (130 mg, 1.11 mmol, 0.2 eq), potassium carbonate (2.42 g, 16.64 mmol, 3 eq), methanesulfonamide (560 mg, 5.548 mmol, 1 eq), and water (10 mL) were sequentially added to a reaction flask. Then, a solution of compound **13-8a** (1.9 g, 5.548 mmol, 1 eq) in dimethoxyethane (5 mL)/tert-butanol (20 mL) was added dropwise to the mixture. After the addition was completed, the mixture was reacted at 0°C for an additional 2 hours. The reaction progress was monitored by LC-MS and TLC. After the reaction was completed, the system was cooled to 0°C, then slowly added with 100 mL of water for dilution. The mixture was extracted with chloroform/isopropanol (3/1, 100 mL × 3). The organic phases were combined, washed with saturated brine (200 mL × 1), dried over anhydrous sodium sulfate, and filtered to remove the desiccant. The filtrate was subjected to rotary evaporation under reduced pressure to remove the solvent, yielding the crude product. The resulting crude product was purified by silica gel column chromatography and subjected to a gradient elution with a mobile phase of 0% to 15% methanol/dichloromethane. The collected fraction was subjected to rotary evaporation under reduced pressure to remove the solvent, yielding compound **13-9** (dark yellow oily liquid, 2.1 g, yield of 95%). MS(ESI, m/z): 377.1 $[M+H]^+$.

**Step 11:**

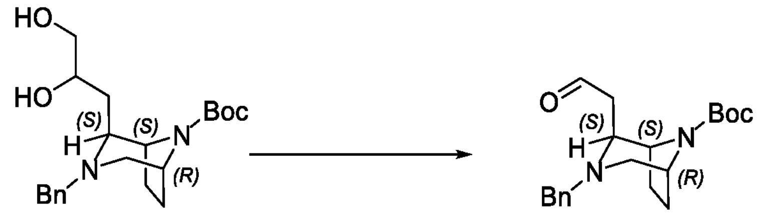

13-9 13-10

With stirring at 25°C, sodium periodate (5.68 g, 25.235 mmol, 5 eq) was added to a mixed solution of compound **13-9** (2 g, 5.047 mmol, 1 eq) in acetonitrile (32 mL) and water (8 mL). The mixture was reacted at 25°C for 0.5 hours. The reaction progress was monitored by LC-MS and TLC. After the reaction was completed, the mixture was filtered to remove the insoluble material, and the filtrate was extracted with chloroform/isopropanol (3/1, 50 mL × 3), concentrated under reduced pressure to remove the solvent, yielding the crude product **13-10** (pale yellow oily liquid, 1.7 g, yield of 93%), which was immediately used for the next step. MS(ESI, m/z): 345.1 $[M+H]^+$.

**Step 12:**

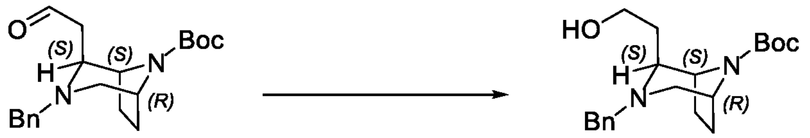

13-10 13-11

With stirring at 0°C, sodium borohydride (280 mg, 7.034 mmol, 1.5 eq) was added to a solution of compound **13-10** (1.7 g, 4.689 mmol, 1 eq) in methanol (20 mL). The mixture was reacted at 25°C for 1 hour. The reaction progress was monitored by LC-MS and TLC. After the reaction was completed, the mixture was concentrated under reduced pressure to remove the solvent. The resulting mixture was purified by silica gel column chromatography and subjected to a gradient elution with a mobile phase of 0% to 5% methanol/dichloromethane. The collected fraction was subjected to rotary evaporation under reduced pressure to remove the solvent, yielding compound **13-11** (pale yellow oily liquid, 1.2 g, yield of 63%). MS(ESI, m/z): 347.3 $[M+H]^+$. $^1H$ NMR (400 MHz, DMSO-$d_6$) $\delta$ 7.35 - 7.28 (m, 4H), 7.25 - 7.20 (m, 1H), 4.44 - 4.30 (m, 1H), 4.18 - 4.00 (m, 2H), 3.59 (d, $J$ = 13.7 Hz, 2H), 3.46 - 3.35 (m, 2H), 2.72 (d, $J$ = 10.0 Hz, 1H), 2.45 (d, $J$ = 10.8 Hz, 1H), 2.24 - 2.21 (m, 1H), 1.85 (s, 1H), 1.74 - 1.55 (m, 5H), 1.39 (s, 9H).

**Step 13:**

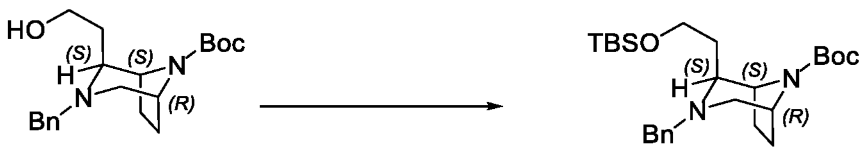

13-11 13-12

Under nitrogen atmosphere at 0°C with stirring, imidazole (196.49 mg, 2.742 mmol, 2 eq) and tert-butyldimethylsilyl chloride (326.26 mg, 2.056 mmol, 1.5 eq) were sequentially added to a solution of compound **13-11** (500 mg, 1.371 mmol, 1 eq) in dichloromethane (5 mL). The mixture was reacted at 25°C for 16 hours. The reaction progress was monitored by LC-MS and TLC. After the reaction was completed, the mixture was concentrated under reduced pressure to remove the solvent. The resulting mixture was purified by silica gel column chromatography and subjected to a gradient elution with a mobile phase of 0% to 20% ethyl acetate/petroleum ether. The collected fraction was subjected to rotary evaporation under reduced pressure to remove the solvent, yielding compound **13-12** (colorless oily liquid, 470 mg, yield of 70%). MS(ESI, m/z): 461.3 $[M+H]^+$,

**Step 14:**

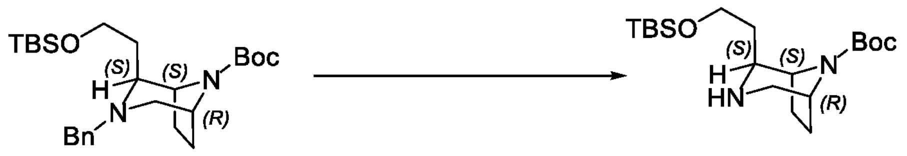

13-12

13-13

With stirring at 25°C, palladium hydroxide/carbon (95 mg, 20% palladium hydroxide) was added to a solution of compound **13-12** (470 mg, 0.969 mmol, 1 eq) in ethanol (10 mL). The mixture was reacted at 25°C under hydrogen atmosphere at 1 atm for 3 hours The reaction progress was monitored by LC-MS and TLC. After the reaction was completed, the mixture was filtered to remove the palladium hydroxide/carbon. The filtrate was concentrated under reduced pressure to obtain a crude product. The resulting crude product was purified by silica gel column chromatography and subjected to a gradient elution with a mobile phase of 0% to 10% methanol/dichloromethane. The collected fraction was subjected to rotary evaporation under reduced pressure to remove the solvent, yielding compound **13-13** as colorless oily liquid (370 mg, yield of 97%). MS(ESI, m/z): 371.2 $[M+H]^+$; $^1$H-NMR (400 MHz, $CDCl_3$) $\delta$ 4.29 - 3.95 (m, 2H), 3.84 - 3.62 (m, 2H), 3.18 - 3.03 (m, 1H), 2.97 - 2.75 (m, 1H), 2.59 - 2.45 (m, 1H), 2.02 - 1.93 (m, 1H), 1.90 - 1.75 (m, 4H), 1.74 - 1.66 (m, 1H), 1.46 (s, 9H), 0.88 (s, 9H), 0.05 (s, 6H).

**Step 15:**

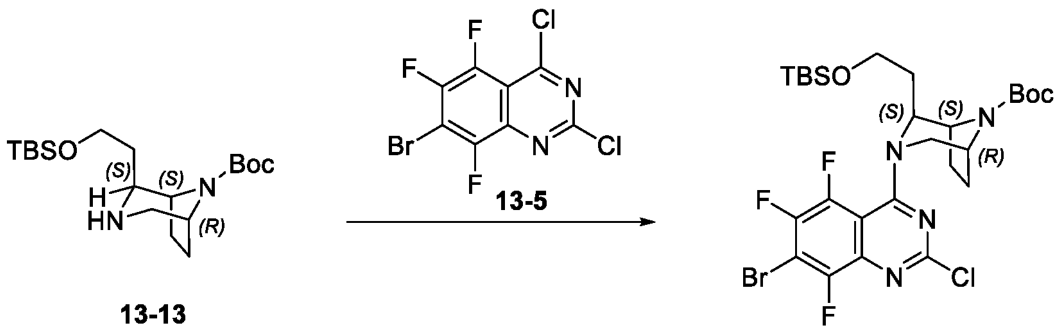

13-5

13-13

13-14

Under nitrogen atmosphere at 0°C with stirring, compound **13-13** (350 mg, 0.948 mmol, 1 eq) was added to a solution of compound **13-5** (331 mg, 0.948 mmol, 1 eq) and *N,N*-diisopropylethylamine (380.8 mg, 2.952 mmol, 3 eq) in anhydrous dichloromethane (5 mL). The mixture was reacted at 25°C for 3 hours The reaction progress was monitored by LC-MS and TLC. After the reaction was completed, the mixture was concentrated under reduced pressure to remove excess solvent, yielding the crude product. The crude product was purified by silica gel column chromatography and subjected to a gradient elution with a mobile phase of 0% to 20% ethyl acetate/petroleum ether. The collected fraction was subjected to rotary evaporation under reduced pressure to remove the solvent, yielding compound **13-14** (yellow solid, 580 mg, yield of 87%). MS(ESI, m/z): 665.1/667.1 $[M+H]^+$; $^1$H NMR (400 MHz, $CDCl_3$) $\delta$ 5.71 - 5.15 (m, 1H), 4.54 - 4.32 (m, 2H), 4.10 - 3.82 (m, 1H), 3.77 - 3.46 (m, 2H), 3.42 - 3.03 (m, 1H), 2.20 - 2.08 (m, 1H), 2.02 - 1.69 (m, 4H), 1.49 (s, 9H), 1.17 (s, 1H), 0.79 (s, 9H), -0.07 (s, 3H), -0.16 (s, 3H).

**Step 16:**

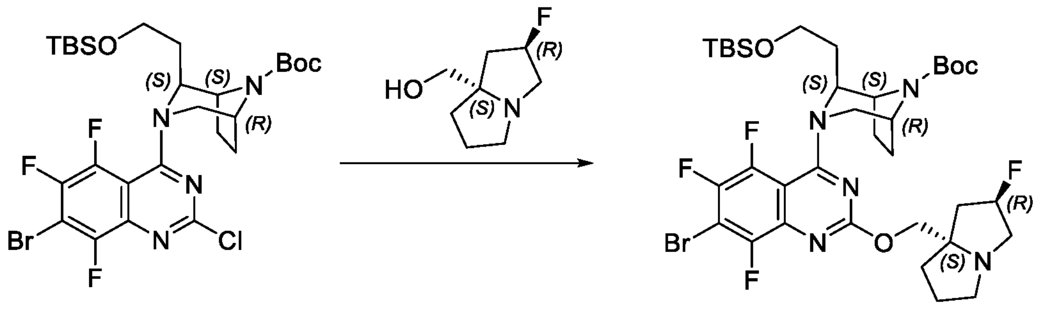

13-14 13-15

Under nitrogen atmosphere at 25°C with stirring, compound (2*R*,7a*S*)-2-fluorotetrahydro-1*H*-pyrrolizin-7a(5*H*)-methanol (151.45 mg, 0.904 mmol, 1.2 eq) was added to a solution of compound **13-14** (528 mg, 0.753 mmol, 1 eq), cesium carbonate (516.58 mg, 1.505 mmol, 2 eq), and triethylenediamine (17.79 mg, 0.151 mmol, 0.2 eq) in *N,N*-dimethylformamide (6 mL). The mixture was reacted at 60°C for 2.5 hours. The reaction progress was monitored by LC-MS and TLC. After the reaction was completed, the mixture was filtered to remove the insoluble material. The filtrate was concentrated under reduced pressure to obtain a crude product. The resulting crude product was purified by reverse-phase chromatography (C18 column): mobile phase A: water (0.1% ammonia water); mobile phase B: methanol; elution with 50% to 95% phase B over 30 minutes; detector: UV 254/220 nm. The product obtained was compound **13-15** as a white solid (325 mg, yield of 51%). MS(ESI, m/z): 788.0/790.0 $[M+H]^+$; $^1H$ NMR (400 MHz, $CDCl_3$) $\delta$ 5.43 - 5.15 (m, 1H), 4.44 - 4.03 (m, 4H), 3.96 - 3.76 (m, 1H), 3.71 - 3.47 (m, 2H), 3.43 - 3.11 (m, 3H), 3.07 - 2.91 (m, 1H), 2.39 - 2.10 (m, 4H), 2.05 - 1.69 (m, 7H), 1.64 (s, 4H), 1.48 (s, 9H), 0.76 (s, 9H), -0.09 (s, 3H), -0.17 (s, 3H).

**Step 17:**

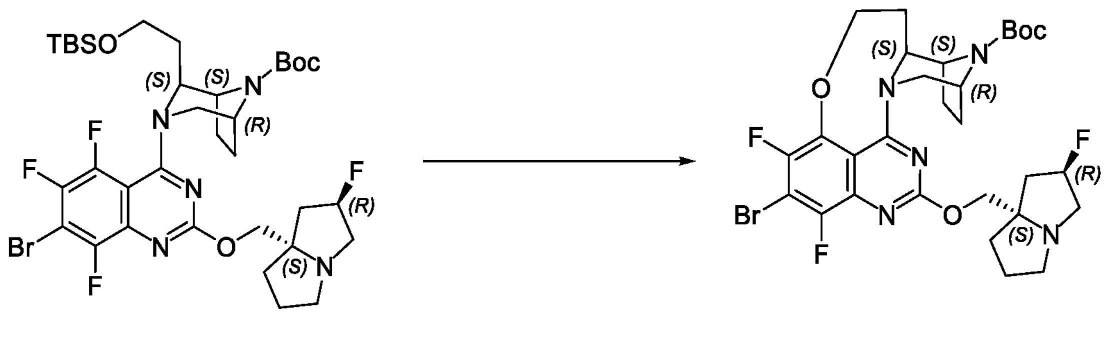

13-15 13-16

Under nitrogen atmosphere at 25°C with stirring, cesium fluoride (288.86 mg, 1.805 mmol, 5 eq) was added to a solution of compound **13-15** (300 mg, 0.361 mmol, 1 eq) in *N,N*-dimethylformamide (15 mL). The mixture was reacted at 80°C for 24 hours. The reaction progress was monitored by LC-MS and TLC. After the reaction was completed, the mixture was concentrated under reduced pressure to remove excess solvent, yielding the crude product. The resulting crude product was purified by silica gel column chromatography and subjected to a gradient elution with a mobile phase of 0% to 10% methanol/dichloromethane. The collected fraction was subjected to rotary evaporation under reduced pressure to remove the solvent, yielding compound **13-16** as a white solid (200 mg, yield of 80%). MS(ESI, m/z): 654.2/656.2 $[M+H]^+$; $^1H$ NMR (400 MHz, $CDCl_3$) $\delta$ 5.28 - 5.16 (m, 2H), 4.63 - 4.06 (m, 6H), 3.74 - 3.61 (m, 1H), 3.49 - 2.84 (m, 5H), 2.36 - 2.27 (m, 1H), 2.26 - 2.12 (m, 4H), 2.06 - 1.77 (m, 5H), 1.77 - 1.65 (m, 2H), 1.50 (s, 9H).

**Step 18:**

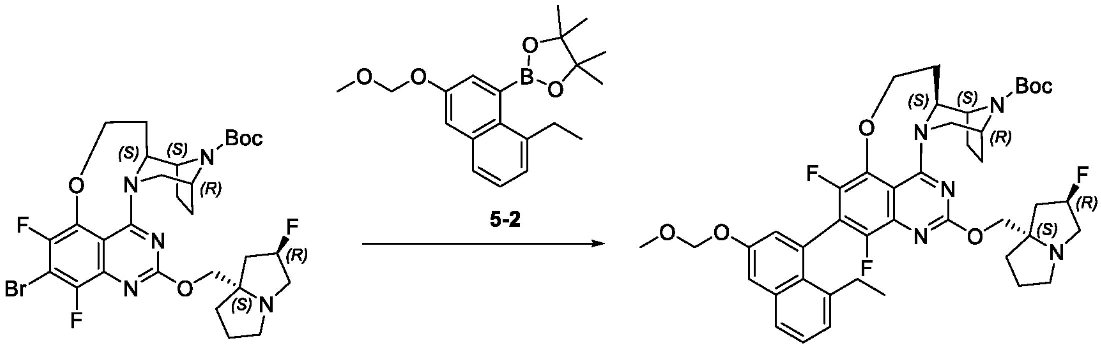

5-2

13-16

13-17

Under nitrogen atmosphere at 25°C with stirring, compound **13-16** (190 mg, 0.276 mmol, 1 eq), compound **5-2** (198.69 mg, 0.552 mmol, 2 eq), tris(dibenzylideneacetone)dipalladium (26.58 mg, 0.028 mmol, 0.1 eq), 3-(*tert*-butyl)-4-(2,6-dimethoxyphenyl) -2,3-dihydrobenzo[d][1,3]oxaphosphole (19.18 mg, 0.055 mmol, 0.2 eq), potassium phosphate (184.85 mg, 0.828 mmol, 3 eq), toluene (4 mL), and water (0.8 mL) were sequentially added to a reaction flask. The resulting mixture was reacted at 80°C for 3 hours. The reaction progress was monitored by LC-MS and TLC. After the reaction was completed, the mixture was concentrated under reduced pressure to remove excess solvent, yielding the crude product. The resulting crude product was purified by silica gel column chromatography and subjected to a gradient elution with a mobile phase of 0% to 10% methanol/dichloromethane. The collected fraction was subjected to rotary evaporation under reduced pressure to remove the solvent, yielding compound **13-17** (yellow solid, 180 mg, yield of 80%). MS(ESI, m/z): 790.8 $[M+H]^+$; $^1H$ NMR (400 MHz, $CDCl_3$) $\delta$ 7.69 (d, $J$ = 8.2 Hz, 1H), 7.54 - 7.49 (m, 1H), 7.44 - 7.37 (m, 1H), 7.24 - 7.20 (m, 1H), 7.14 - 7.08 (m, 1H), 5.28 - 5.21 (m, 1H), 4.63 - 3.98 (m, 6H), 3.90 - 3.74 (m, 1H), 3.40 - 3.16 (m, 3H), 3.04 (s, 1H), 2.58 - 2.42 (m, 2H), 2.38 - 2.12 (m, 5H), 2.11 - 1.84 (m, 7H), 1.72 - 1.55 (m, 4H), 1.50 (s, 9H), 0.99 - 0.95 (m, 3H), 0.88 (t, $J$ = 6.6 Hz, 3H).

**Step 19:**

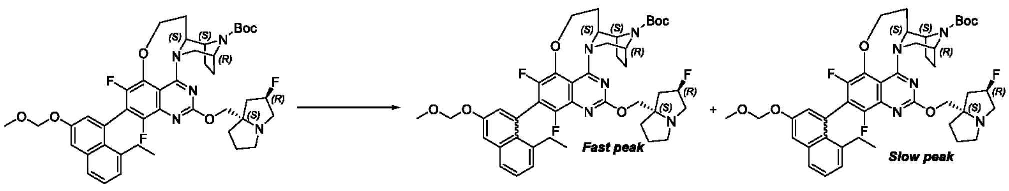


13-17 13-17a 13-17b

The compound **13-17** (180 mg) obtained from step **18** was subjected to chiral resolution by high-performance liquid chromatography: chiral column CHIRAL ART Cellulose-SC, 2 × 25 cm, 5 μm; mobile phase A: n-hexane/[methyl tert-butyl ether (2 M ammonia in methanol)] (50%/50%); mobile phase B: ethanol; flow rate: 20 mL/min; column temperature: 35°C; elution with 30% phase B; detector: UV224/294 nm, resulting in two products. The product with a shorter retention time (3.9 minutes) was compound **13-17a** (white solid, 35 mg, yield of 19%), MS (ESI, m/z): 790.8 $[M + H]^+$. The product with a longer retention time (4.9 minutes) was compound **13-17b** (white solid, 125 mg, yield of 69%), MS (ESI, m/z): 790.8 $[M + H]^+$.

**Step 20:**

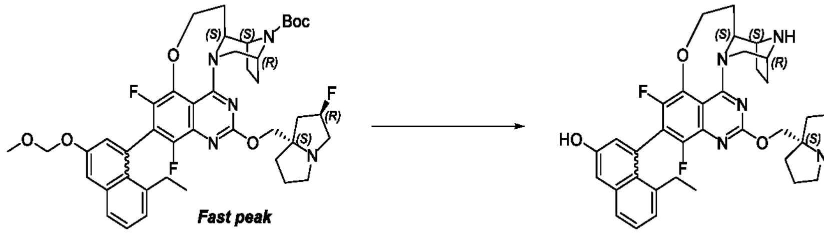


13-17a 13a'

With stirring at 0°C, a 4 M solution of hydrochloric acid in 1,4-dioxane (1 mL) was added to a solution of compound **13-17a** (30 mg, 0.036 mmol, 1 eq) in methanol (1 mL). The mixture was reacted at 25°C for 1 hour. The reaction progress was monitored by LC-MS and TLC. After the reaction was completed, the mixture was concentrated under reduced pressure to remove excess solvent, yielding the crude product. The resulting crude product was purified by high pressure liquid chromatography: (column: XBridge Prep OBD C18, 30 × 150 mm, 5 μm; mobile phase A: water (10 M ammonium bicarbonate), mobile phase B: acetonitrile; flow rate: 60 mL/min; elution with a gradient of 25% to 55% mobile phase B; detector: UV 220 nm). The collected fraction was subjected to rotary evaporation under reduced pressure to remove the solvent, yielding compound **13a'** as a white solid (6 mg, yield of 24%). MS(ESI, m/z): 646.2 $[M+H]^+$, $^1$H-NMR (400 MHz, $CD_3OD$) $\delta$ 7.64 - 7.58 (m, 1H), 7.38 - 7.31 (m, 1H), 7.29 - 7.24 (m, 1H), 7.17 - 7.11 (m, 1H), 6.97 - 6.89 (m, 1H), 5.20 - 5.12 (m, 1H), 4.63 - 4.43 (m, 2H), 4.33 - 4.23 (m, 2H), 4.20 - 4.12 (m, 1H), 3.73 - 3.67 (m, 1H), 3.66 - 3.61 (m, 1H), 3.51 - 3.45 (m, 1H), 3.27 - 3.18 (m, 3H), 3.07 - 2.98 (m, 1H), 2.62 - 2.53 (m, 1H), 2.52 - 2.42 (m, 2H), 2.41 - 2.11 (m, 3H), 2.10 - 1.94 (m, 3H), 1.93 - 1.81 (m, 2H), 1.81 - 1.71 (m, 2H), 1.41 - 1.33 (m, 1H), 0.92 (t, J = 7.4 Hz, 3H); $^{19}$F-NMR (377 MHz, $CD_3OD$) $\delta$ 136.03, -139.76, -173.60.

**Step 21:**

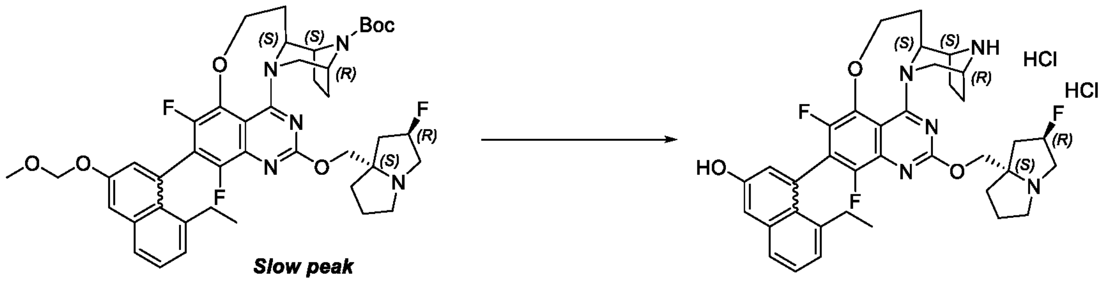


13-17b 13b

With stirring at 0°C, a 4 M solution of hydrochloric acid in 1,4-dioxane (2 mL) was added to a solution of compound **13-17b** (125 mg, 0.150 mmol, 1 eq) in methanol (2 mL). The mixture was reacted at 25°C for 1 hour. The reaction progress was monitored by LC-MS and TLC. After the reaction was completed, the mixture was concentrated under reduced pressure to remove excess solvent, yielding the crude product. The resulting crude product was purified by reverse-phase chromatography (C18 column): mobile phase A: water (0.1% hydrochloric acid); mobile phase B: acetonitrile; elution with 5% to 95% phase B over 25 minutes; detector: UV 254/220 nm. The product obtained was compound **13b** (yellow solid, 80 mg, yield of 72%). MS(ESI, m/z): 646.25 $[M+H]^+$; $^1$H-NMR (400 MHz, $CD_3OD$) $\delta$ 7.69 - 7.60 (m, 1H), 7.43 - 7.34 (m, 1H), 7.31 (d, *J* = 2.6 Hz, 1H), 7.22 - 7.14 (m, 1H), 6.96 (d, *J* = 2.6 Hz, 1H), 5.73 - 5.48 (m, 2H), 5.00 (d, *J* = 11.7 Hz, 1H), 4.84 - 4.71 (m, 3H), 4.58 - 4.47 (m, 1H), 4.46 - 4.39 (m, 1H), 4.35 (s, 1H), 4.27 - 4.16 (m, 1H), 4.07 - 3.89 (m, 2H), 3.88 - 3.75 (m, 2H), 3.53 - 3.42 (m, 1H), 2.85 - 2.20 (m, 10H), 2.20 - 2.08 (m, 3H), 2.07 - 1.93 (m, 1H), 1.03 - 0.84 (m, 3H); $^{19}$F-NMR (377 MHz, $CD_3OD$) $\delta$ -132.98, -137.59, -174.33.

**Example 14**

9-((2*R* or 2*S*, 5a*S*,6*S*,9*R*)-1,3-Difluoro-13-(((2*R*,7a*S*)-2-fluorotetrahydro-1*H*-pyrrolizin-7a(5*H*)-yl)methoxy)-5a,6,7,8,9,10-hexahydro-5*H*-6,9-epiminoazepino[2',1':3,4][1,4]oxazepino[5,6,7-*de*]quinazolin-2-yl)-2,3-dihydro-1*H*-cyclopenta[a]naphthalen-7-ol dihydrochloride **14a;** 9-((2*S* or 2*R*, 5a*S*,6*S*,9*R*)-1,3-difluoro-13-(((2*R*,7a*S*)-2-fluorotetrahydro-1*H*-pyrrolizin-7a(5*H*)-yl)methoxy)-5a,6,7,8,9,10-hexahydro-5*H*-6,9-epiminoazepino[2',1':3,4][1,4]oxazepino [5,6,7-de]quinazolin-2-yl)-2,3-dihydro-1*H*-cyclopenta[a]naphthalen-7-ol dihydrochloride **14b**

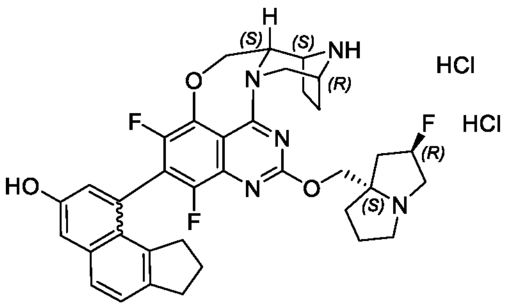

**14a**

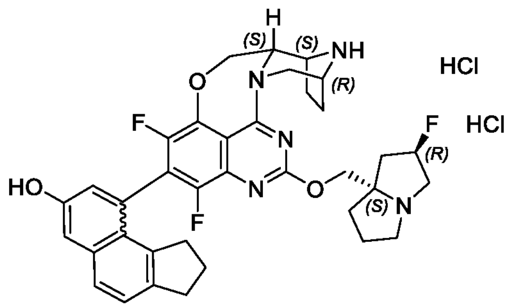

**14b**

The synthetic route is as follows:

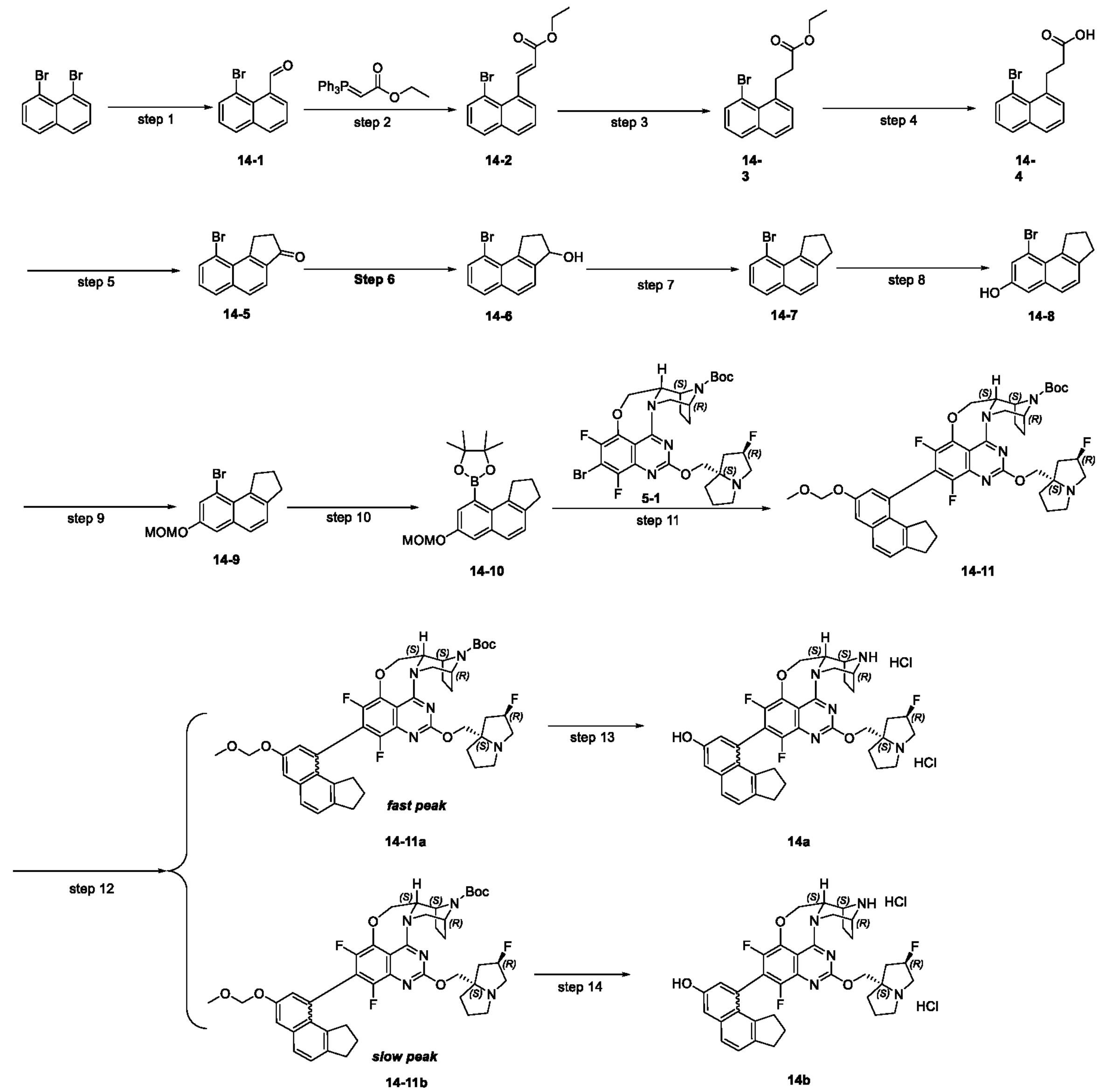


## Step 1:

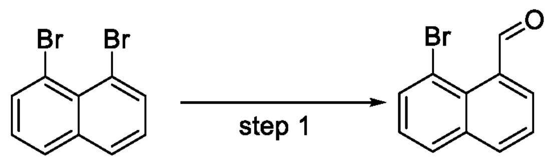


14-1

Under nitrogen atmosphere at -78°C with stirring, *n*-butyllithium (2.5 M in *n*-hexane, 20.93 mL) was slowly added dropwise to a solution of 1,8-dibromonaphthalene (15 g, 49.831 mmol, 1.0 eq) in anhydrous tetrahydrofuran (150 mL). The mixture was stirred at -78°C for 1 hour. *N,N*-Dimethylformamide (4.26 mL, 52.323 mmol, 1.05 eq) was added dropwise to the reaction system, and the mixture was reacted at -78°C for an additional 1 hour, then allowed to naturally warm to 25°C and reacted for 1 hour. The reaction progress was monitored by TLC. After the reaction was completed, the reaction mixture was poured into saturated ammonium chloride solution (400 mL) at 0°C to quench the reaction. The mixture was extracted with ethyl acetate (400 mL × 3), and the organic phase was dried and concentrated to obtain a crude product.

The crude product was purified by silica gel column chromatography and subjected to a gradient elution with a mobile phase of 0% to 5% ethyl acetate/petroleum ether. The collected fraction was concentrated under reduced pressure to remove the solvent, yielding compound **14-1** (white solid, 10.2 g, yield of 82%). $^1$H NMR (400 MHz, $CDCl_3$) $\delta$ 11.43 (s, 1H), 8.01 - 7.99 (m, 1H), 7.92 - 7.86 (m, 4H), 7.57 - 7.54 (m, 1H), 7.40 - 7.36 (m, 1H).

**Step 2:**

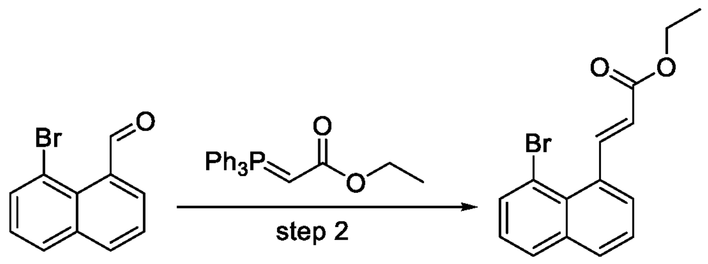


14-1 14-2

Under nitrogen atmosphere at 25°C with stirring, compound **14-1** (7.2 g, 29.096 mmol, 1.0 eq), ethoxycarbonylmethylene triphenylphosphorane (11.74 g, 32.006 mmol, 1.1 eq), and toluene (100.0 mL) were sequentially added to a reaction flask. The resulting mixture was reacted with stirring at 100°C under nitrogen atmosphere for 2 hours. The reaction progress was monitored by TLC. After the reaction was completed, the reaction mixture was cooled to room temperature and concentrated under reduced pressure to remove excess reagents, yielding the crude product. The crude product was purified by silica gel column chromatography and subjected to a gradient elution with a mobile phase of 0% to 20% ethyl acetate/petroleum ether. The collected fraction was concentrated under reduced pressure to remove the solvent, yielding compound **14-2** (yellow oil substance, 5.6 g, yield of 60%). $^1$H NMR (400 MHz, $CDCl_3$) $\delta$ 9.07 (d, $J$ = 16.0 Hz, 1H), 7.90 - 7.84 (m, 2H), 7.82 (d, $J$ = 8.0 Hz, 1H), 7.56 - 7.51 (m, 1H), 7.49 - 7.42 (m, 1H), 7.32 - 7.28 (m, 1H), 6.14 (d, $J$ = 16.0 Hz, 1H), 4.34 - 4.26 (m, 2H), 1.41 - 1.33 (m, 3H).

**Step 3:**

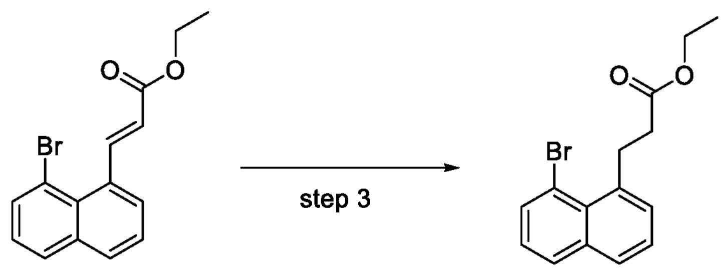


14-2 14-3

With stirring at room temperature, compound **14-2** (5 g, 15.565 mmol, 1.0 eq), wet palladium on carbon (10% palladium content, 500 mg), ethanol (50 mL), and water (5 mL) were sequentially added to a reaction flask. The mixture was reacted under a hydrogen atmosphere (10 atm) at 25°C for 2 hours, with the reaction progress monitored by TLC. After the reaction was completed, the mixture was filtered, and the filtrate was concentrated to obtain the crude product. The crude product was purified by silica gel column chromatography and subjected to a gradient elution with a mobile phase of 0% to 20% ethyl acetate/petroleum ether. The collected fraction was concentrated under reduced pressure to remove the solvent, yielding compound **14-3** (colorless oil, 2.3 g, yield of 46%). $^1$H NMR (400 MHz, $CDCl_3$) $\delta$ 7.88 - 7.79 (m, 2H), 7.78 - 7.73 (m, 1H), 7.47 - 7.36 (m, 2H), 7.26 - 7.22 (m, 1H), 4.19 - 4.10 (m, 2H), 3.91 - 3.83 (m, 2H), 2.79 - 2.75 (m, 2H), 1.25 - 1.22 (m, 3H).

**Step 4:**

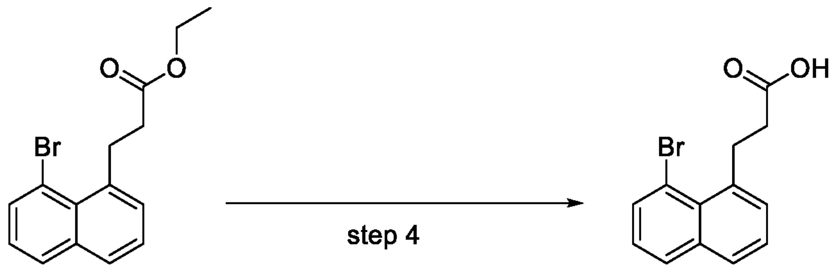


**14-3** **14-4**

With stirring at 25°C, compound **14-3** (2.1 g, 6.494 mmol, 1.0 eq), methanol (15 mL), and tetrahydrofuran (5 mL) were sequentially added to a reaction flask. Then, a solution of lithium hydroxide (327.46 mg, 12.988 mmol, 2.0 eq) in water (5 mL) was added dropwise to the above system. The resulting mixture was stirred at 25°C for 3 hours, with the reaction progress monitored by TLC. After the reaction was completed, the reaction mixture was concentrated to obtain the crude product. The pH of the crude product was adjusted to 4 with hydrochloric acid aqueous solution (1 M), and the mixture was filtered. The collected filter cake was dried to obtain compound **14-4** (white solid, 1.6 g, yield of 83%). $^1$H NMR (400 MHz, DMSO-$d_6$) $\delta$ 8.02 - 7.96 (m, 1H), 7.94 - 7.86 (m, 2H), 7.55 - 7.46 (m, 2H), 7.39 - 7.33 (m, 1H), 3.75 - 3.68 (m, 2H), 2.65 - 2.60 (m, 2H).

**Step 5:**

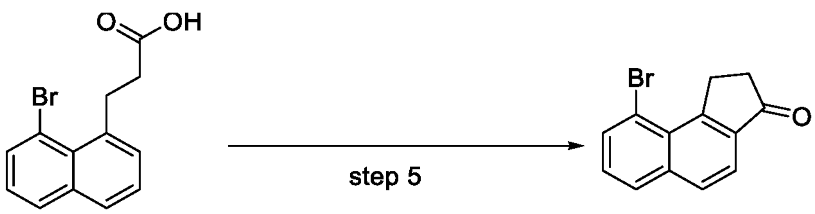


**14-4** **14-5**

With stirring at 25°C, compound **14-4** (1.3 g, 4.424 mmol, 1.0 eq) and polyphosphoric acid (40 g) were sequentially added to a reaction flask. The mixture was stirred at 80°C for 3 hours, with the reaction progress monitored by LC-MS and TLC. After the reaction was completed, the reaction mixture was quenched by pouring into water (50 mL) and extracted with ethyl acetate (50 mL × 3). The organic phase was dried and concentrated to obtain a crude product. The crude product was purified by silica gel column chromatography and subjected to a gradient elution with a mobile phase of 0% to 50% ethyl acetate/petroleum ether. The collected fraction was concentrated under reduced pressure to remove the solvent, yielding compound **14-5** (colorless oil, 800 mg, yield of 66%). MS (ESI, m/z): 261.0/263.0 $[M + H]^+$; $^1$H NMR (400 MHz, DMSO-$d_6$) $\delta$ 8.13 - 8.08 (m, 1H), 8.03 - 7.97 (m, 2H), 7.67 (d, $J$ = 8.0 Hz, 1H), 7.59 - 7.53 (m, 1H), 3.95 - 3.87 (m, 2H), 2.76 - 2.69 (m, 2H).

**Step 6:**

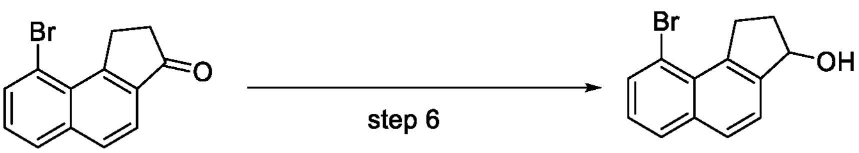


**14-5** **14-6**

Under nitrogen atmosphere at -78°C with stirring, diisobutylaluminum hydride (1 M, 3 mL, 1.2 eq) was added dropwise to a solution of compound **14-5** (700 mg, 2.547 mmol, 1.0 eq) in anhydrous tetrahydrofuran (7.0 mL). The mixture was stirred at -78°C under nitrogen atmosphere for 2 hours, with the reaction progress monitored by TLC. After the reaction was completed, the reaction mixture was quenched by pouring into saturated ammonium chloride aqueous solution (20 mL) and extracted with ethyl acetate (20 mL × 3). The organic phase was dried and concentrated to obtain a crude product. The crude product was purified by silica gel column chromatography and subjected to a gradient elution with a mobile phase of 0% to 50% ethyl acetate/petroleum ether. The collected fraction was concentrated under reduced pressure to remove the solvent, yielding compound **14-6** (white solid, 650 mg, yield of 92%). $^1$H NMR (400 MHz, DMSO-$d_6$) $\delta$ 7.96 (d, $J$ = 8.0 Hz, 1H), 7.90 - 7.80 (m, 2H), 7.58 (d, $J$ = 8.0 Hz, 1H), 7.35 - 7.29 (m, 1H), 5.37 (d, $J$ = 8.0 Hz, 1H), 5.20 - 5.10 (m, 1H), 3.96 - 3.84 (m, 1H), 3.57 - 3.46 (m, 1H), 2.48 - 2.37 (m, 1H), 1.93 - 1.81 (m, 1H).

**Step 7:**

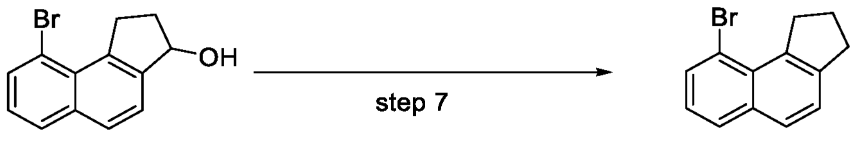


**14-6** **14-7**

With stirring at 0°C, compound **14-6** (640 mg, 2.311 mmol, 1.0 eq), triethylsilane (565.63 mg, 4.622 mmol, 2.0 eq) and trifluoroacetic acid (7.0 mL) were sequentially added to a reaction flask. The mixture was stirred at 0°C for 2 hours, with the reaction progress monitored by TLC. After the reaction was completed, the reaction mixture was concentrated under reduced pressure to remove excess reagents, yielding the crude product. The crude product was purified by silica gel column chromatography and subjected to a gradient elution with a mobile phase of 0% to 1% ethyl acetate/petroleum ether. The collected fraction was concentrated under reduced pressure to remove the solvent, yielding compound **14-7** (white solid, 600 mg, yield of 95%). $^1$H NMR (400 MHz, DMSO-$d_6$) $\delta$ 7.95 - 7.90 (m, 1H), 7.83 - 7.79 (m, 2H), 7.49 (d, J = 8.0 Hz, 1H), 7.30 - 7.24 (m, 1H), 3.80 - 3.73 (m, 2H), 3.05 - 2.97 (m, 2H), 2.15 - 2.06 (m, 2H).

**Step 8:**

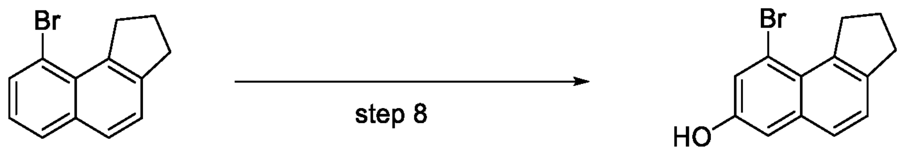


**14-7** **14-8**

Under nitrogen atmosphere at 25°C with stirring, compound **14-7** (300 mg, 1.153 mmol, 1.0 eq), chloro(1,5-cyclooctadiene)iridium(I) dimer (80.47 mg, 0.115 mmol, 0.1 eq), 4,4'-di-tert-butyl-2,2'-dipyridine (32.58 mg, 0.115 mmol, 0.1 eq), bis(pinacolato)diboron (369.91 mg, 1.384 mmol, 1.2 eq), and n-hexane (3 mL) were sequentially added to a reaction flask. The resulting mixture was reacted at 60°C for 2 hours, with the reaction progress monitored by TLC. After the reaction was completed, the reaction mixture was cooled to room temperature and concentrated under reduced pressure to obtain the crude intermediate. With stirring at 0°C, a solvent mixture of tetrahydrofuran/water (2/1, 9 mL) was added to the crude intermediate. Then, hydrogen peroxide (30%, 9 mL) and acetic acid (4.5 mL) were added dropwise sequentially. The mixture was reacted at 0°C for 0.5 hours, with the reaction progress monitored by TLC. After the reaction was completed, the pH was adjusted to 8 using a saturated sodium carbonate aqueous solution. The mixture was extracted with ethyl acetate (20 mL × 3), and the organic phase was dried and concentrated to obtain a crude product. The crude product was purified by silica gel column chromatography and subjected to a gradient elution with a mobile phase of 0% to 50% ethyl acetate/petroleum ether. The collected fraction was concentrated under reduced pressure to remove the solvent, yielding compound **14-8** (white solid, 250 mg, yield of 78%). $^1$H NMR (400 MHz, DMSO-$d_6$) $\delta$ 9.90 (s, 1H), 7.57 (d, $J$ = 8.0 Hz, 1H), 7.40 (d, $J$ = 4.0 Hz, 1H), 7.34 (d, $J$ = 8.0 Hz, 1H), 7.16 (d, $J$ = 4.0 Hz, 1H), 3.73 - 3.64 (m, 2H), 2.97 - 2.90 (m, 2H), 2.12 - 2.01 (m, 2H).

**Step 9:**

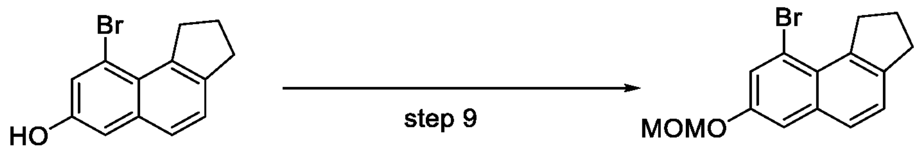


**14-8** **14-9**

Under nitrogen atmosphere at 0°C, compound **14-8** (250 mg, 0.903 mmol, 1.0 eq), *N,N*-diisopropylethylamine (349.97 mg, 2.709 mmol, 3.0 eq), and dichloromethane (3.0 mL) were sequentially added to a reaction flask. Chloromethyl methyl ether (126.32 mg, 1.806 mmol, 2.0 eq) was then slowly added dropwise to the above system, and the mixture was stirred at 25°C for 1 hour, with the reaction progress monitored by TLC. After the reaction was completed, the reaction mixture was quenched by pouring into ice water (20 mL), and extracted with dichloromethane (20 mL × 3). The organic phase was dried and concentrated to obtain a crude product. The crude product was purified by silica gel column chromatography and subjected to a gradient elution with a mobile phase of 0% to 20% ethyl acetate/petroleum ether. The collected fraction was concentrated under reduced pressure to remove the solvent, yielding compound **14-9** (yellow solid, 220 mg, yield of 75%). $^1$H NMR (400 MHz, DMSO-$d_6$) $\delta$ 7.70 (d, *J* = 8.0 Hz, 1H), 7.57 (d, *J* = 4.0 Hz, 1H), 7.50 (d, *J* = 4.0 Hz, 1H), 7.43 (d, *J* = 8.0 Hz, 1H), 5.30 (s, 2H), 3.76 - 3.68 (m, 2H), 3.42 (s, 3H), 3.00 - 2.93 (m, 2H), 2.15 - 2.03 (m, 2H).

**Step 10:**

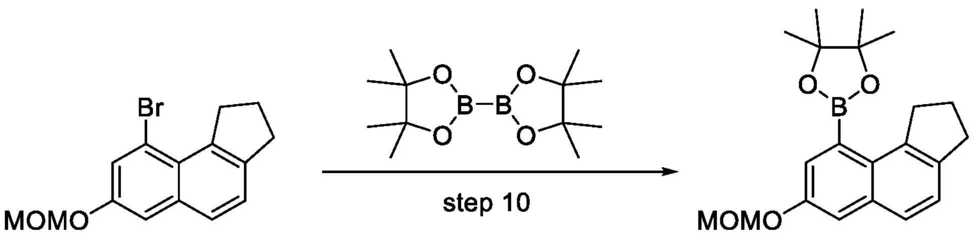


**14-9** **14-10**

Under nitrogen atmosphere at 25°C, compound **14-9** (200 mg, 0.619 mmol, 1.0 eq), bis(pinacolato)diboron (214.93 mg, 0.805 mmol, 1.3 eq), [1,1'-bis(diphenylphosphino)ferrocene]dichloropalladium(II) dichloromethane complex (53.04 mg, 0.062 mmol, 0.1 eq), potassium acetate (191.69 mg, 1.857 mmol, 3.0 eq), and 1,4-dioxane (2.0 mL) were sequentially added to a reaction flask. The mixture was stirred at 100°C for 2 hours, with the reaction progress monitored by TLC. After the reaction was completed, the reaction mixture was cooled to 25°C. The reaction mixture was concentrated under reduced pressure to remove excess reagents, yielding the crude product. The crude product was purified by silica gel column chromatography and subjected to a gradient elution with a mobile phase of 0% to 20% ethyl acetate/petroleum ether. The collected fraction was concentrated under reduced pressure to remove the solvent, yielding compound **14-10** (white solid, 160 mg, yield of 69%). $^1$H NMR (400 MHz, $CDCl_3$) $\delta$ 7.56 (d, *J* = 8.0 Hz, 1H), 7.45 - 7.41 (m, 2H), 7.34 (d, *J* = 8.0 Hz, 1H), 5.28 (s, 2H), 3.50 (s, 3H), 3.33 - 3.27 (m, 2H), 3.08 - 3.02 (m, 2H), 2.22 - 2.12 (m, 2H), 1.43 (s, 12H).

**Step 11:**

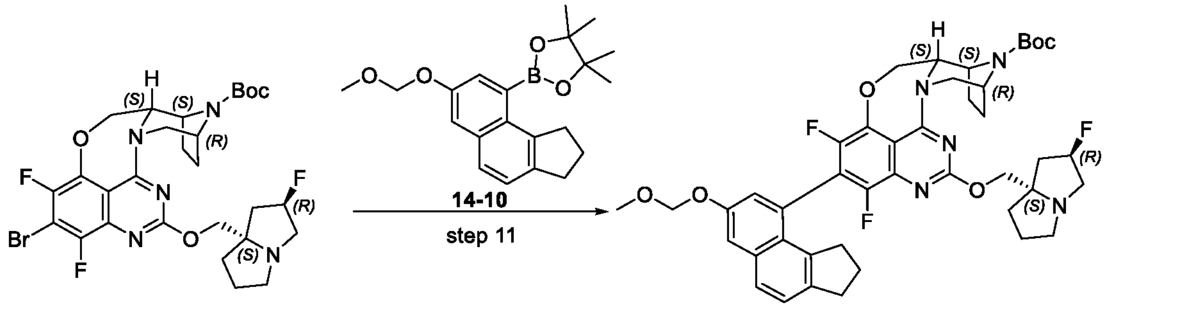


5-1 14-11

Under nitrogen atmosphere at 25°C with stirring, compound **5-1** (25 mg, 0.037 mmol, 1.0 eq), compound **14-10** (13.83 mg, 0.037 mmol, 1.0 eq), 3-(*tert*-butyl)-4-(2,6-dimethoxyphenyl)-2,3-dihydrobenzo[*d*][1,3]oxaphosphole (2.58 mg, 0.007 mmol, 0.2 eq), tris(dibenzylideneacetone)dipalladium(0) (3.57 mg, 0.004 mmol, 0.1 eq), potassium phosphate (16.57 mg, 0.074 mmol, 2.0 eq), water (0.1 mL), and toluene (0.5 mL) were sequentially added to a reaction flask. The resulting mixture was reacted with stirring at 80°C under nitrogen atmosphere for 16 hours. The reaction progress was monitored by LC-MS and TLC. After the reaction was completed, the reaction mixture was filtered, and the filtrate was concentrated under reduced pressure to remove the solvent, yielding the crude product. The crude product was purified by silica gel column chromatography and subjected to a gradient elution with a mobile phase of 0% to 10% methanol/dichloromethane. The collected fraction was concentrated under reduced pressure to remove the solvent, yielding compound **14-11** (white solid, 30 mg, yield of 95%). MS (ESI, m/z): 788.3 $[M + H]^+$.

**Step 12:**

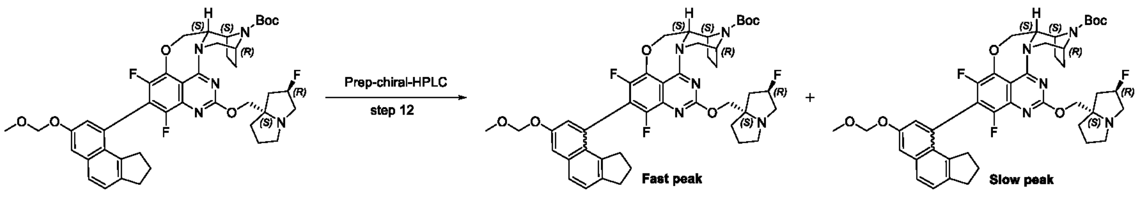


14-11 14-11a 14-11b

The compound **14-11** (30 mg) obtained from **step 11** of the present example was subjected to chiral resolution using high-performance liquid chromatography: chiral column: CHIRALPAK ID, 2 × 25 cm, 5 μm; mobile phase A: n-hexane/methyl tert-butyl ether (1/1) (0.5%, 2 M ammonia in methanol), mobile phase B: methanol; flow rate: 20 mL/min; elution with 20% mobile phase B over 8 minutes; detector: UV 222 nm, resulting in two products. The product with a shorter retention time (5.00 minutes) was compound **14-11a** (white solid, 13 mg, yield of 43%); compound **14-11a:** MS (ESI, *m*/*z*): 788.3 $[M + H]^+$. The product with a longer retention time (6.35 minutes) was compound **14-11b** (white solid, 12 mg, yield of 40%); compound **14-11b:** MS (ESI, *m*/*z):* 788.3 $[M + H]^+$.

**Step 13:**

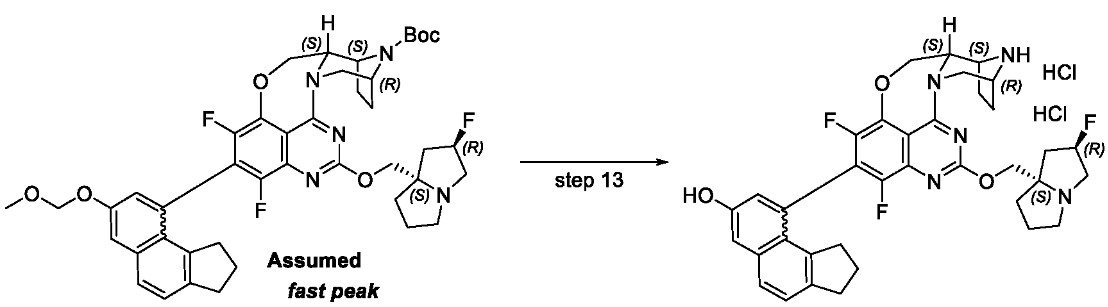


14-11a 14a

With stirring at 0°C, a solution of hydrochloric acid in 1,4-dioxane (4 M, 1 mL) was added dropwise to a solution of compound **14-11a** (13 mg, 0.016 mmol, 1.00 eq) in methanol (1 mL). The mixture was reacted at room temperature for 1 hour, with the reaction progress monitored by LC-MS. After the reaction was completed, the reaction mixture was concentrated to obtain the crude product. The crude product was purified by reverse-phase flash chromatography (C18 column) and eluted with a mobile phase of 5% to 95% acetonitrile/water (0.1% hydrochloric acid) over 25 minutes, with detection at UV 254 nm. The product obtained was compound **14a** (yellow solid, 4.8 mg, yield of 42%). MS (ESI, m/z): 644.3 $[M + H]^+$, $^1H$ NMR (400 MHz, $CD_3OD$) $\delta$ 7.61 (d, $J$ = 8.4 Hz, 1H), 7.36 (d, $J$ = 8.4 Hz, 1H), 7.32 - 7.28 (m, 1H), 7.01 - 6.94 (m, 1H), 5.64 - 5.37 (m, 2H), 4.83 - 4.71 (m, 2H), 4.69 - 4.57 (m, 2H), 4.46 - 4.27 (m, 2H), 4.07 - 3.83 (m, 3H), 3.75 - 3.62 (m, 1H), 3.49 - 3.41 (m, 1H), 2.99 - 2.87 (m, 2H), 2.80 - 2.58 (m, 3H), 2.56 - 2.28 (m, 6H), 2.26 - 2.08 (m, 4H), 1.96 - 1.79 (m, 2H); $^{19}F$ NMR (377 MHz, $CD_3OD$) $\delta$ -135.06, -137.29, -174.17.

**Step 14:**

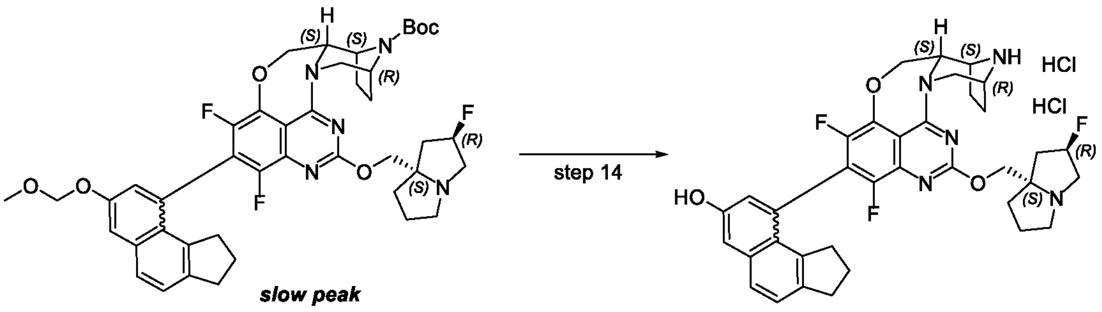


**14-11b** **14b**

With stirring at 0°C, a solution of hydrochloric acid in 1,4-dioxane (4 M, 1 mL) was added dropwise to a solution of compound **14-11b** (12 mg, 0.014 mmol, 1.00 eq) in methanol (1 mL). The mixture was reacted at room temperature for 1 hour, with the reaction progress monitored by LC-MS. After the reaction was completed, the reaction mixture was concentrated to obtain the crude product. The crude product was purified by reverse-phase flash chromatography (C18 column) and eluted with a mobile phase of 5% to 95% acetonitrile/water (0.1% hydrochloric acid) over 25 minutes, with detection at UV 254 nm. The product obtained was compound **14b** (yellow solid, 2.6 mg, yield of 24%). MS (ESI, m/z): 644.3 $[M + H]^+$, $^1H$ NMR (400 MHz, $CD_3OD$) $\delta$ 7.63 (d, $J$ = 8.4 Hz, 1H), 7.39 (d, $J$ = 8.4 Hz, 1H), 7.33 (d, $J$ = 2.4 Hz, 1H), 6.99 (d, $J$ = 2.4 Hz, 1H), 5.69 - 5.52 (m, 1H), 5.43 - 5.35 (m, 1H), 4.81 - 4.54 (m, 4H), 4.44 - 4.37 (m, 2H), 4.10 - 3.84 (m, 4H), 3.75 - 3.69 (m, 1H), 3.53 - 3.44 (m, 1H), 2.99 - 2.90 (m, 2H), 2.82 - 2.57 (m, 2H), 2.56 - 2.45 (m, 3H), 2.42 - 2.31 (m, 3H), 2.26 - 2.14 (m, 4H), 2.00 - 1.87 (m, 2H); $^{19}F$ NMR (377 MHz, $CD_3OD$) $\delta$ -134.64, -137.87, -174.21.

**Example 15**

(2*S*,6*R*,7a*S*)-7*a*-((((2*R* or 2*S*, 5a*S*,6*S*,9*R*)-2-(8-Ethyl-3-hydroxynaphthalen-1-yl)-1,3-difluoro-*5*a,6,7,8,9,10-hexahydro-5*H*-6,9-epiminoazepino[2',1':3,4][1,4]oxazepino[5,6,7-*de*]quinazolin-13-yl)oxy)methyl)-6-fluorohexahydro-1*H*-pyrrolizin-2-ol dihydrochloride **15a**; (2*S*,6*R*,7a*S*)-7a-((((2*S* or 2*R*, 5a*S*,6*S*,9*R*)-2-(8-ethyl-3-hydroxynaphthalen-1-yl)-1,3-difluoro-5a,6,7,8,9,10-hexahydro-5*H*-6,9-epiminoazepino[2',1':3,4][1,4]oxazepino[5,6,7-*de*]quinazolin-13-yl)oxy) methyl)-6-fluorohexahydro-1*H*-pyrrolizin-2-ol dihydrochloride **15b**

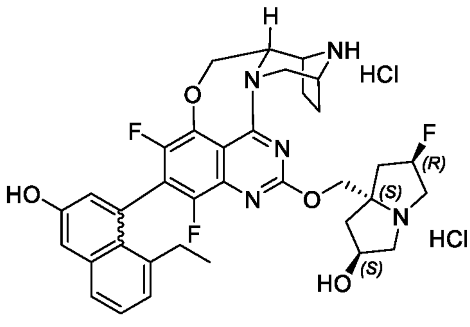

**15a**

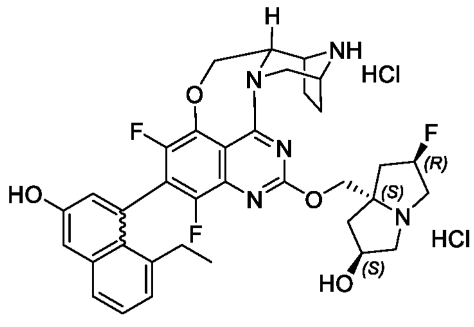

**15b**

The synthetic route is as follows:

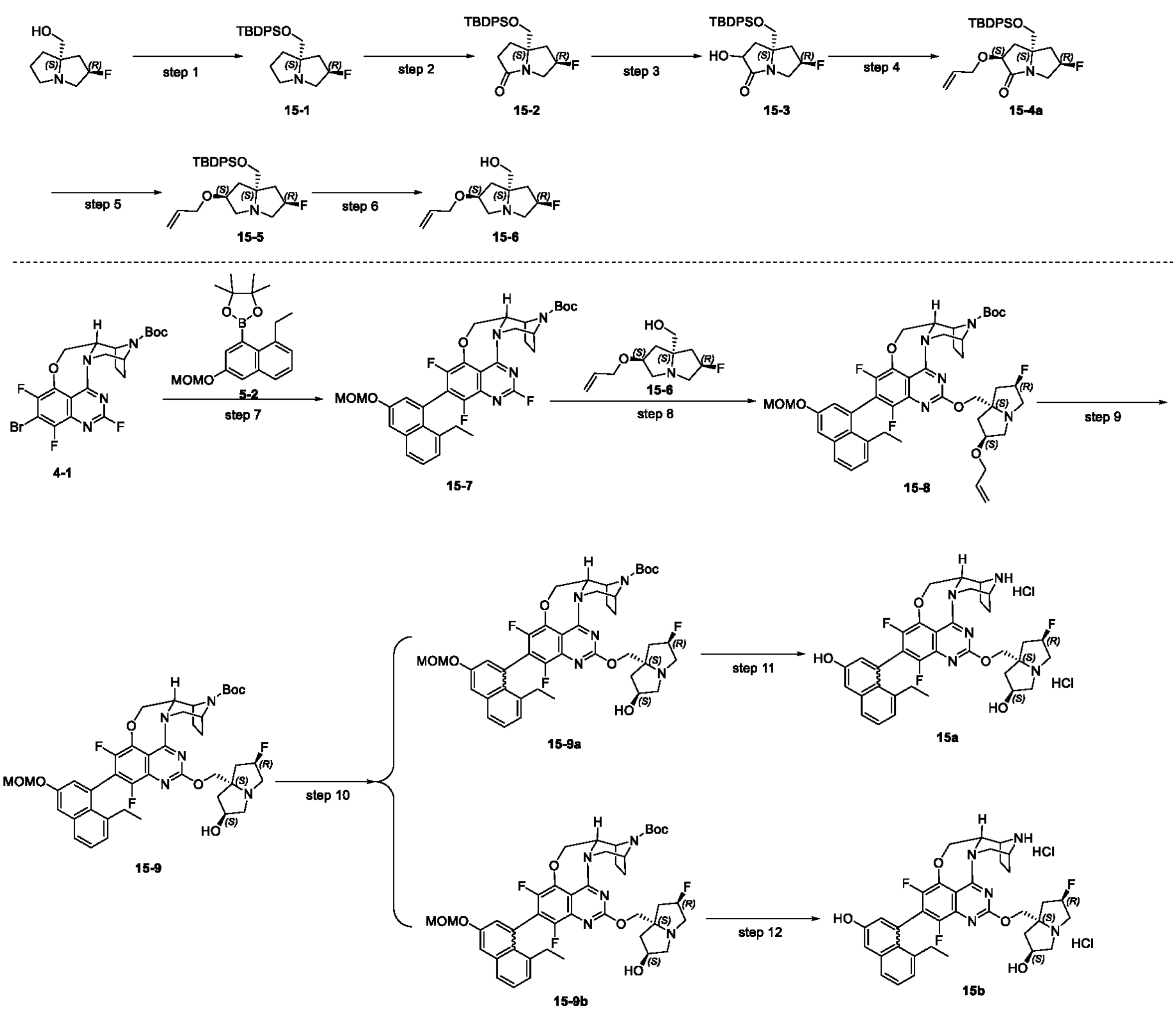


**Step 1**:

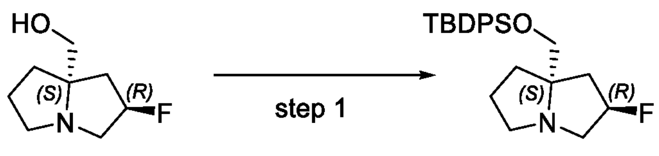

**15-1**

Under nitrogen atmosphere at 0°C with stirring, compound (2*R*,7a*S*)-2-fluorotetrahydro-1*H*-pyrrolizin-7a(5*H*)-methanol (30 g, 179.0 mmol, 1.0 eq), imidazole (15.4 g, 214.8 mmol, 1.2 eq), and dichloromethane (300 mL) were sequentially added to a reaction flask. Then, *tert*-butyldiphenylchlorosilane (67.3 g, 232.6 mmol, 1.3 eq) was slowly added to the mixture. The resulting mixture was reacted with stirring at 25°C under nitrogen atmosphere for 2 hours, with the reaction progress monitored by LC-MS and TLC. After the reaction was completed, the reaction mixture was quenched by pouring into saturated sodium bicarbonate solution, and extracted with dichloromethane (300 mL × 3). The organic phases were combined, dried over anhydrous sodium sulfate, and filtered to remove the desiccant. The filtrate was concentrated under reduced pressure to obtain the crude product. The crude product was purified by silica gel column chromatography and subjected to a gradient elution with a mobile phase of 0% to 10% methanol/dichloromethane. The collected fraction was concentrated under reduced pressure to remove the solvent, yielding compound **15-1** (colorless oil, 63 g, yield of 84%). MS (ESI, m/z): 398.2 $[M + H]^+$; $^1H$ NMR (300 MHz, $CDCl_3$) $\delta$ 7.77 - 7.64 (m, 4H), 7.50 - 7.35 (m, 6H), 5.35 - 5.09 (m, 1H), 3.49 (d, *J* = 9.6 Hz, 1H), 3.38 (d, *J* = 9.6 Hz, 1H), 3.27 - 2.99 (m, 3H), 2.98 - 2.83 (m, 1H), 2.26 - 2.14 (m, 1H), 2.13 - 1.94 (m, 2H), 1.95 - 1.63 (m, 3H), 1.09 (s, 9H).

**Step 2:**

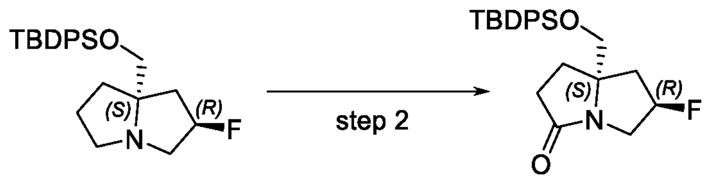


**15-1** **15-2**

With stirring at 0°C, a solution of ruthenium trichloride hydrate (4.42 g, 18.6 mmol, 0.2 eq) in water (400 mL) and sodium periodate (104.9 g, 465.9 mmol, 5.0 eq) were added to a solution of compound **15-1** (39 g, 93.2 mmol, 1.0 eq) in carbon tetrachloride (400 mL). The mixture was reacted with stirring at 25°C for 1 hour, with the reaction progress monitored by LC-MS and TLC. After the reaction was completed, the reaction mixture was diluted with water (500 mL) and extracted with dichloromethane (500 mL × 3). The organic phases were combined, dried over anhydrous sodium sulfate, and filtered to remove the desiccant. The filtrate was concentrated under reduced pressure to obtain a crude product. The crude product was purified by silica gel column chromatography and subjected to a gradient elution with a mobile phase of 0% to 60% petroleum ether/methyl tert-butyl ether. The collected fraction was concentrated under reduced pressure to remove the solvent, yielding compound **15-2** (white solid, 19 g, yield of 47%). MS (ESI, *m*/*z*): 412.2 $[M + H]^+$; $^1H$ NMR (400 MHz, $CDCl_3$) $\delta$ 7.66 - 7.59 (m, 4H), 7.48 - 7.37 (m, 6H), 5.38 - 5.15 (m, 1H), 4.21 - 4.08 (m, 1H), 3.63 - 3.53 (m, 1H), 3.49 - 3.39 (m, 1H), 3.17 - 3.00 (m, 1H), 2.79 - 2.66 (m, 1H), 2.44 - 2.34 (m, 1H), 2.33 - 2.21 (m, 1H), 2.21 - 2.10 (m, 1H), 2.05 - 1.92 (m, 2H), 1.04 (s, 9H).

**Step 3:**

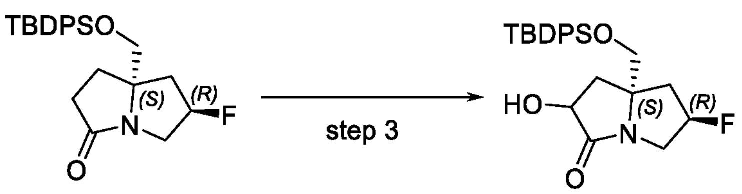


**15-2** **15-3**

Under nitrogen atmosphere at -78°C with stirring, compound **15-2** (5 g, 11.541 mmol, 1.0 eq) and 18-crown-6 (6.42 g, 23.082 mmol, 2.0 eq) were dissolved in 50 mL of tetrahydrofuran. Then, a solution of potassium *tert*-butoxide in tetrahydrofuran (1 M, 15 mL) was added dropwise to the mixture. The resulting mixture was reacted with stirring at -78°C under nitrogen atmosphere for 1 hour. Then, 3-phenyl-2-phenylsulfonyl-1,2-oxaziridine (4.76 g, 17.312 mmol, 1.5 eq) was added thereto. The mixture was reacted at -78°C for 1 hour, with the reaction progress monitored by TLC and LC-MS. After the reaction was completed, the reaction mixture was poured into 100 mL of water and extracted with ethyl acetate (100 mL × 3). The organic phase was concentrated to obtain a crude product. The crude product was purified by reverse-phase

chromatography (C18 column) and eluted with a mobile phase of 5% to 95% acetonitrile/water (0.1% ammonia water) over 25 minutes, with detection at UV 254/220 nm. The product obtained was compound **15-3** (yellow oil, 4.5 g, yield of 86%). MS (ESI, *m/z*): 428.2 [M + H]$^+$; $^1$H NMR (400 MHz, $CDCl_3$) $\delta$ 7.65 - 7.61 (m, 4H), 7.46 - 7.39 (m, 6H), 5.36 - 5.20 (m, 1H), 4.91 (s, 1H), 4.39 - 4.33 (m, 1H), 4.13 - 4.01 (m, 1H), 3.77 - 3.70 (m, 1H), 3.48 - 3.40 (m, 1H), 3.25 - 3.08 (m, 1H), 2.26 - 2.22 (m, 1H), 2.16 - 2.09 (m, 1H), 2.00 - 1.98 (m, 1H), 1.94 - 1.88 (m, 1H), 1.06 (s, 9H).

**Step 4:**

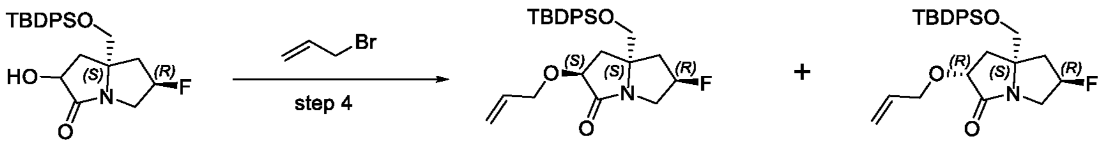


**15-3** **15-4a** **15-4b**

Under nitrogen atmosphere at 0°C with stirring, compound **15-3** (1.7 g, 3.777 mmol, 1.0 eq), 3-bromopropene (625.26 mg, 4.910 mmol, 1.3 eq), and 20 mL of *N,N*-dimethylformamide were sequentially added to a reaction flask. Once fully dissolved, sodium hydride (60%, 226.6 mg, 5.665 mmol, 1.5 eq) was added thereto in batches. The resulting mixture was reacted at 0°C for 1 hour, with the reaction progress monitored by TLC and LC-MS. After the reaction was completed, the reaction mixture was poured into 100 mL of saturated ammonium chloride aqueous solution and then extracted with ethyl acetate (100 mL × 3). The organic phase was concentrated to obtain a crude product. The crude product was purified by silica gel column chromatography and subjected to a gradient elution with a mobile phase of 0% to 50% methyl *tert*-butyl ether/petroleum ether, yielding two isomers, **15-4a** and **15-4b**. Compound **15-4a** (pale yellow oil, 1.28 g, yield of 68%). MS (ESI, m/z): 468.2 [M + H]$^+$; $^1$H NMR (400 MHz, $CD_3OD$) $\delta$ 7.68 - 7.59 (m, 4H), 7.48 - 7.34 (m, 6H), 5.83 - 5.68 (m, 1H), 5.50 - 5.27 (m, 1H), 5.19 - 5.02 (m, 2H), 4.16 - 3.88 (m, 5H), 3.60 (d, *J* = 10.1 Hz, 1H), 3.43 - 3.23 (m, 2H), 2.71 - 2.50 (m, 1H), 2.19 - 1.93 (m, 3H), 1.05 (s, 9H); $^{19}$F NMR (282 MHz, $CD_3OD$) $\delta$ -173.50. Compound **15-4b** (pale yellow oil, 220 mg, yield of 12%). MS (ESI, *m/z*):468.2 [M + H]$^+$; $^1$H NMR (400 MHz, $CDCl_3$) $\delta$ 7.64 - 7.59 (m, 4H), 7.49 - 7.36 (m, 6H), 6.00 - 5.88 (m, 1H), 5.33 - 5.11 (m, 3H), 4.62 - 4.56 (m, 1H), 4.42 - 4.35 (m, 1H), 4.27 - 4.13 (m, 2H), 3.48 (d, *J* = 10.4 Hz, 1H), 3.35 (d, *J* = 10.4 Hz, 1H), 3.08 - 2.93 (m, 1H), 2.69 - 2.60 (m, 1H), 2.18 - 1.95 (m, 3H), 1.04 (s, 9H); $^{19}$F NMR (376 MHz, $CDCl_3$) $\delta$ -173.11.

**Step 5**:

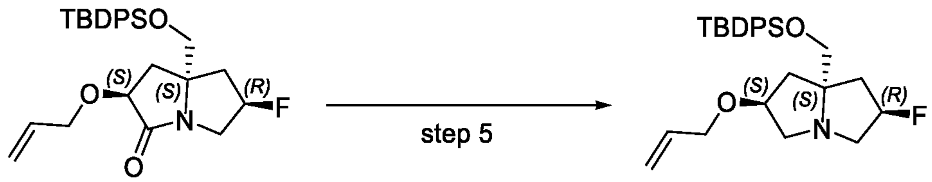


**15-4a** **15-5**

Under nitrogen atmosphere at 25°C with stirring, compound **15-4a** (950 mg, 1.93 mmol, 1.0 eq), carbonylchlorobis(triphenylphosphine)iridium(I) (158.5 mg, 0.193 mmol, 0.1 eq), and dichloromethane (10 mL) were sequentially added to the reaction flask. Then, 1,1,3,3-tetramethyldisiloxane (1.09 g, 7.720 mmol, 4.0 eq) was added thereto. The resulting mixture was reacted with stirring at 25°C for 30 minutes, after which sodium borohydride (307.39 mg, 7.72 mmol, 4.0 eq) and boron trifluoride etherate (1.15 g, 7.72 mmol, 4.0 eq) were added to the reaction mixture. The resulting mixture was reacted with stirring at 25°C for an additional 2 hours, with the reaction progress monitored by LC-MS and TLC. After the reaction was completed, 100 mL of water was added to the reaction mixture to quench the reaction. The mixture was then extracted with ethyl acetate (100 mL × 3), and the organic phases were combined. The organic phases were washed with saturated brine (100 mL × 3), then dried over anhydrous sodium sulfate, and filtered to remove the desiccant. The filtrate was concentrated under reduced pressure to obtain the crude product. The resulting crude product was purified by reverse-phase chromatography (C18 column) and eluted with a mobile phase of 5% to 95% methanol/water (0.1% ammonia water) over 25 minutes, with detection at UV 254/220 nm. The product obtained was compound **15-5** (pale yellow

oil, 480 mg, yield of 52%). MS (ESI, *m*/*z*): 454.2 [M + H]$^+$, $^1$H NMR (400 MHz, $CDCl_3$) $\delta$ 7.70 - 7.63 (m, 4H), 7.43 - 7.33 (m, 6H), 5.87 - 5.75 (m, 1H), 5.28 - 5.06 (m, 3H), 4.19 - 4.10 (m, 1H), 3.93 - 3.81 (m, 2H), 3.70 (d, *J* = 9.2 Hz, 1H), 3.59 - 3.44 (m, 1H), 3.40 - 3.22 (m, 1H), 3.20 - 3.13 (m, 1H), 3.13 - 3.00 (m, 2H), 2.27 - 1.96 (m, 4H), 1.06 (s, 9H).

**Step 6:**

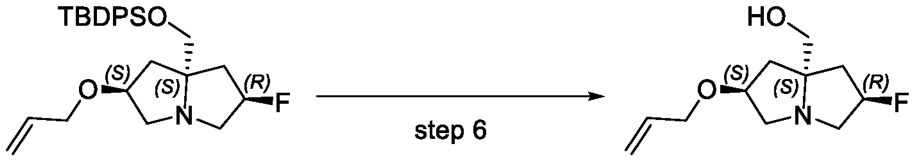


15-5 15-6

With stirring at 25°C, a solution of tetrabutylammonium fluoride in tetrahydrofuran (1 M, 2 mL) was added dropwise to a solution of compound **15-5** (480 mg, 1.005 mmol, 1.0 eq) in tetrahydrofuran (5 mL). The mixture was reacted with stirring at 60°C for 2 hours, with the reaction progress monitored by LC-MS and TLC. After the reaction was completed, the reaction mixture was concentrated to obtain the crude product. The resulting crude product was purified by silica gel column chromatography and subjected to a gradient elution with a mobile phase of 0% to 10% ammonia in methanol/dichloromethane. The collected fraction was concentrated under reduced pressure to remove the solvent, yielding compound **15-6** (colorless oil, 240 mg, yield of 95%). MS (ESI, *m*/*z*): 216.1 [M + H]$^+$; $^1$H NMR (400 MHz, $CDCl_3$) $\delta$ 5.96 - 5.85 (m, 1H), 5.34 - 5.15 (m, 3H), 4.20 - 4.13 (m, 1H), 4.03 - 3.92 (m, 2H), 3.63 (d, *J* = 10.8 Hz, 1H), 3.46 - 3.38 (m, 1H), 3.28 (d, *J* = 10.8 Hz, 1H), 3.24 - 3.19 (m, 1H), 3.18 - 3.06 (m, 2H), 2.17 - 2.04 (m, 4H).

**Step 7:**

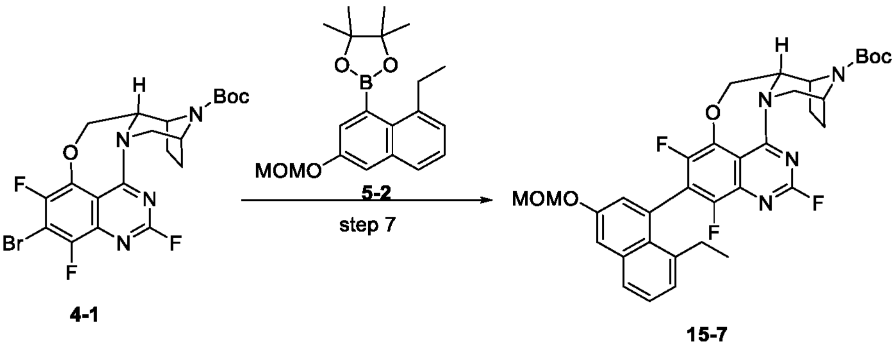


4-1

15-7

Under nitrogen atmosphere at 25°C with stirring, compound **4-1** (2 g, 3.790 mmol, 1.00 eq), compound **5-2** (1.64 g, 4.548 mmol, 1.2 eq), 3-(*tert*-butyl)-4-(2,6-dimethoxyphenyl)-2,3-dihydrobenzo[*d*][1,3]oxaphosphole (263.6 mg, 0.758 mmol, 0.2 eq), tris(dibenzylideneacetone)dipalladium(0) (365.34 mg, 0.379 mmol, 0.1 eq), potassium phosphate (1.69 g, 7.580 mmol, 2.0 eq), water (4 mL), and toluene (20 mL) were sequentially added to a reaction flask. The resulting mixture was reacted with stirring at 80°C under nitrogen atmosphere for 2 hours. The reaction progress was monitored by LC-MS and TLC. After the reaction was completed, the reaction mixture was filtered, and the filtrate was concentrated under reduced pressure to remove the solvent, yielding the crude product. The crude product was purified by silica gel column chromatography and subjected to a gradient elution with a mobile phase of 0% to 50% ethyl acetate/petroleum ether. The collected fraction was concentrated under reduced pressure to remove the solvent, yielding compound 15-7 (white solid, 2 g, yield of 78%). MS (ESI, m/z): 637.3 [M + H]$^+$; $^1$H NMR (400 MHz, $CDCl_3$) $\delta$ 7.73 - 7.69 (m, 1H), 7.55 (d, *J*= 2.8 Hz, 1H), 7.44 - 7.38 (m, 1H), 7.25 - 7.20 (m, 1H), 7.11 - 7.05 (m, 1H), 5.33 - 5.28 (m, 3H), 4.61 - 4.54 (m, 1H), 4.37 - 4.16 (m, 4H), 3.53 - 3.51 (m, 3H), 3.33 - 3.24 (m, 1H), 2.55 - 2.39 (m, 2H), 2.04 - 1.87 (m, 4H), 1.52 (s, 9H), 1.02 - 0.93 (m, 3H).

**Step 8:**

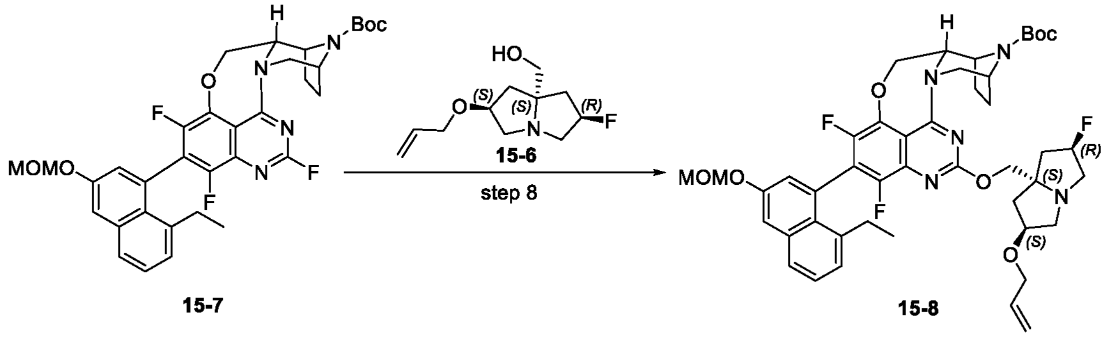


15-7 15-8

Under nitrogen atmosphere at 25°C with stirring, triethylenediamine (6.17 mg, 0.05 mmol, 0.2 eq), compound **15-7** (175 mg, 0.261 mmol, 1.0 eq), cesium carbonate (179.11 mg, 0.522 mmol, 2.0 eq), compound **15-6** (65.09 mg, 0.287 mmol, 1.1 eq), and N,N-dimethylformamide (3 mL) were sequentially added to a reaction flask. The resulting mixture was reacted with stirring at 80°C under nitrogen atmosphere for 2 hours, with the reaction progress monitored by LC-MS and TLC. After the reaction was completed, the crude product was purified by reverse-phase chromatography (C18 column) and eluted with a mobile phase of 5% to 95% methanol/water (0.1% ammonium bicarbonate aqueous solution) over 25 minutes, with detection at UV254/220 nm. The product obtained was compound **15-8** (off-white solid, 150 mg, yield of 65%). MS (ESI, *m/z*): 832.4 $[M + H]^+$.

**Step 9:**

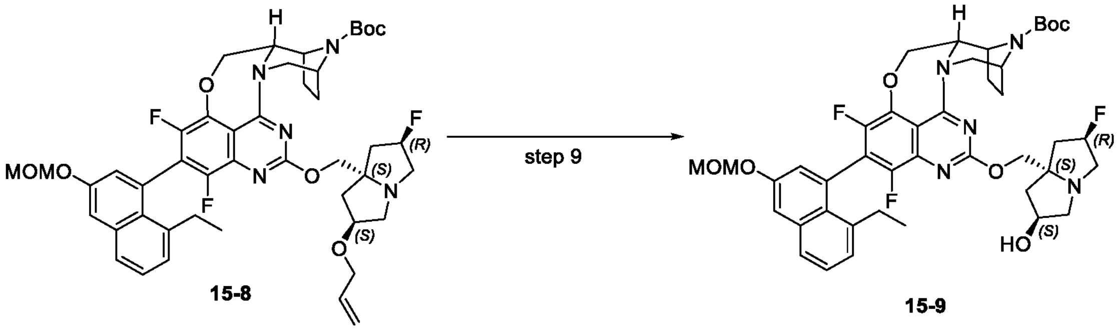


15-8 15-9

Under nitrogen atmosphere at 25°C with stirring, compound **15-8** (150 mg, 0.18 mmol, 1.00 eq), 1,3-dimethylbarbituric acid (56.16 mg, 0.36 mmol, 2.00 eq), tetrakis(triphenylphosphine)palladium (20.79 mg, 0.018 mmol, 0.1 eq), and dichloromethane (2 mL) were sequentially added to the reaction flask. The mixture was reacted at room temperature for 16 hours, with the reaction progress monitored by LC-MS and TLC. After the reaction was completed, the reaction mixture was concentrated to obtain the crude product. The crude product was purified by reverse-phase flash chromatography (C18 column) and eluted with a mobile phase of 5% to 95% methanol/water (0.1% ammonium bicarbonate) over 25 minutes, with detection at UV 254 nm. The product obtained was compound **15-9** (white solid, 108 mg, yield of 75%). MS (ESI, m/z): 792.4 $[M + H]^+$.

**Step 10:**

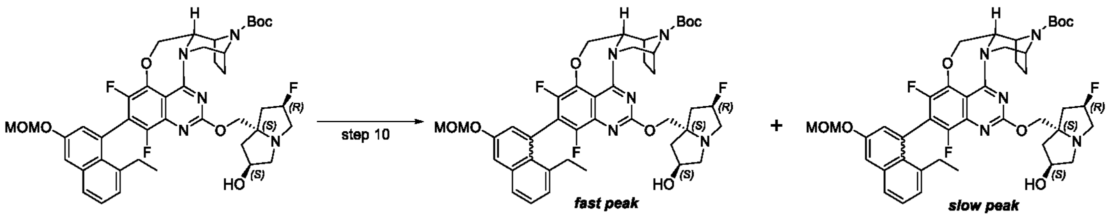


15-9 15-9a 15-9b

The compound **15-9** (108 mg) obtained from **step 9** of the present example was subjected to chiral resolution using high-performance liquid chromatography: chiral column: CHIRALPAK IE, 2 × 25 cm, 5 μm; mobile phase A: n-hexane (0.1%, 2 M ammonia in methanol), mobile phase B: ethanol; flow rate: 20 mL/min; elution with 40% mobile phase B over 21 minutes; detector: UV226/206 nm, resulting in two compounds. The product with a shorter retention time (11.53 minutes) was compound **15-9a** (white solid, 50 mg, yield of 46%), MS (ESI, m/z): 792.4 $[M + H]^+$. The product with a longer retention time (16.70 minutes) was compound **15-9b** (white solid, 53 mg, yield of 49%), MS (ESI, m/z): 792.4 $[M + H]^+$.

**Step 11:**

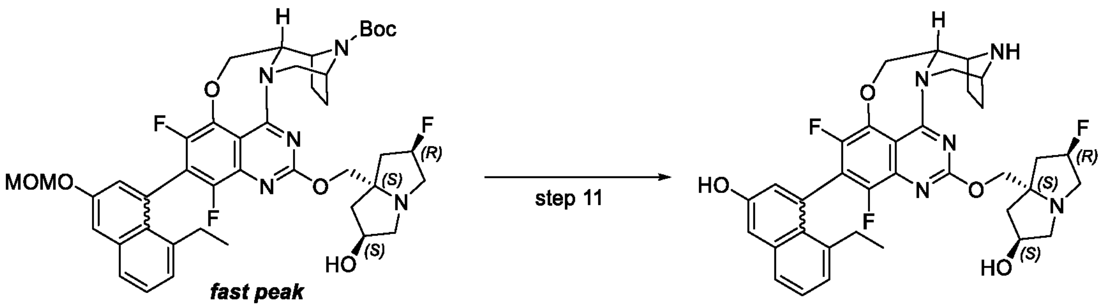


15-9a 15a

With stirring at 0°C, a solution of hydrochloric acid in 1,4-dioxane (4 M, 2 mL) was added dropwise to a solution of compound **15-9a** (50 mg, 0.060 mmol, 1.00 eq) in methanol (2 mL). The mixture was reacted at room temperature for 2 hours, with the reaction progress monitored by LC-MS. After the reaction was completed, the reaction mixture was concentrated to obtain the crude product. The crude product was purified by reverse-phase flash chromatography (C18 column) and eluted with a mobile phase of 5% to 95% acetonitrile/water (0.1% hydrochloric acid) over 25 minutes, with detection at UV 254 nm. The product obtained was compound **15a** (yellow solid, 18 mg, yield of 40%). MS (ESI, m/z): 648.3 $[M + H]^+$, $^1$H NMR (300 MHz, DMSO-$d_6$ +$D_2O$) $\delta$ 7.70 (d, *J* = 8.1 Hz, 1H), 7.44 - 7.37 (m, 1H), 7.34 (d, *J* = 2.7 Hz, 1H), 7.17 (d, *J* = 7.2 Hz, 1H), 6.98 (d, *J* = *2.7* Hz, 1H), 5.78 - 5.54 (m, 1H), 5.04 (d, *J* = 14.1 Hz, 1H), 4.84 - 4.53 (m, 6H), 4.36 - 4.21 (m, 2H), 3.99 - 3.82 (m, 1H), 3.77 - 3.72 (m, 2H), 3.62 - 3.56 (m, 1H), 3.46 - 3.33 (m, 1H), 2.79 - 2.58 (m, 2H), 2.43 - 2.30 (m, 4H), 2.16 - 1.90 (m, 4H), 0.92 - 0.78 (m, 3H); $^{19}$F NMR (282 MHz, DMSO-$d_6$) $\delta$ -132.31, -139.59, -174.60.

**Step 12:**

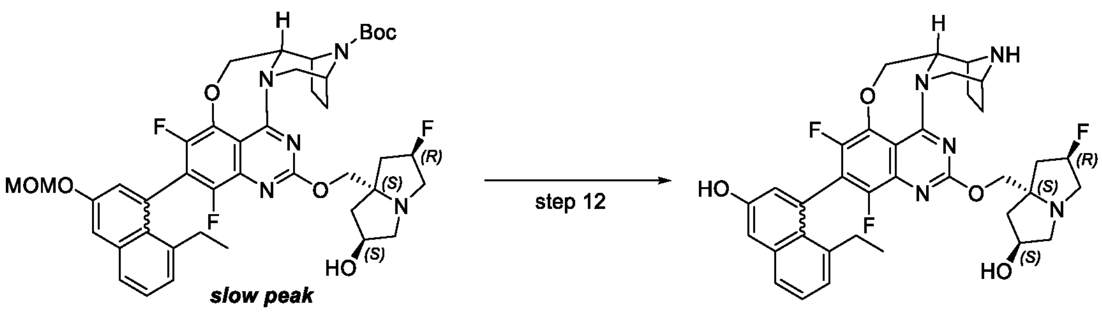


15-9b 15b

With stirring at 0°C, a solution of hydrochloric acid in 1,4-dioxane (4 M, 2 mL) was added dropwise to a solution of compound **15-9b** (53 mg, 0.063 mmol, 1.00 eq) in methanol (2 mL). The mixture was reacted at room temperature for 2 hours, with the reaction progress monitored by LC-MS. After the reaction was completed, the reaction mixture was concentrated to obtain the crude product. The crude product was purified by reverse-phase flash chromatography (C18 column) and eluted with a mobile phase of 5% to 95% acetonitrile/water (0.1% hydrochloric acid) over 25 minutes, with detection at UV 254 nm. The product obtained was compound **15b** (yellow solid, 24 mg, yield of 40%). MS (ESI, m/z): 648.3 $[M + H]^+$, $^1H$ NMR (300 MHz, DMSO-$d_6$ +$D_2O$) $\delta$ 7.69 (d, $J$ = 8.1 Hz, 1H), 7.44 - 7.37 (m, 1H), 7.34 (d, $J$ = 2.7 Hz, 1H), 7.17 (d, $J$ = 7.2 Hz, 1H), 6.97 (d, $J$ = 2.7 Hz, 1H), 5.73 - 5.56 (m, 1H), 5.05 - 4.95 (m, 1H), 4.82 - 4.50 (m, 6H), 4.29 (d, $J$ = 5.1 Hz, 2H), 3.98 - 3.84 (m, 1H), 3.77 - 3.63 (m, 3H), 3.46 - 3.34 (m, 1H), 2.79 - 2.59 (m, 1H), 2.48 - 2.28 (m, 5H), 2.20 - 1.88 (m, 4H), 0.92 - 0.84 (m, 3H); $^{19}F$ NMR (282 MHz, DMSO-$d_6$) $\delta$ -132.39, -139.31, -174.51.

**Example 16**

4-((2*R* or 2*S*, 5a*S*,6*S*,9*R*)-1,3-Difluoro-13-(((2*R*,6*S*,7a*S*)-2-fluoro-6-methoxytetrahydro-1*H*-pyrrolizin-7*a*(5*H*)-yl)methoxy)-5a,6,7,8,9,10-hexahydro-5*H*-6,9-epiminoazepino[2',1':3,4][1,4]oxazepino[5,6,7-*de*]quinazolin-2-yl)-5-ethylnaphthalen-2-ol dihydrochloride **16a**; 4-((2*S* or 2*R*, 5*aS*,6*S*,9*R*)-1,3-difluoro-13-(((2*R*,6*S*,7*aS*)-2-fluoro-6-methoxytetrahydro-1*H*-pyrrolizin-7*a*(5*H*)-yl)methoxy)-5a,6,7,8,9,10-hexahydro-5*H*-6,9-epiminoazepino[2',1':3,4][1,4]oxazepino [5,6,7-*de*]quinazolin-2-yl)-5-ethylnaphthalen-2-ol dihydrochloride **16b**

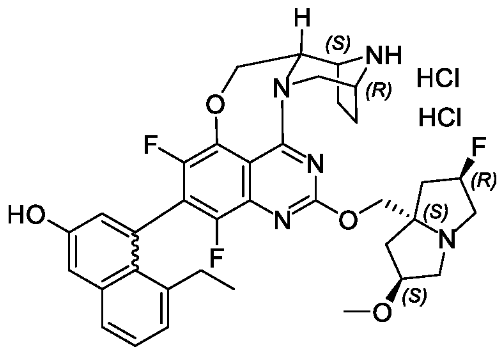

**16a**

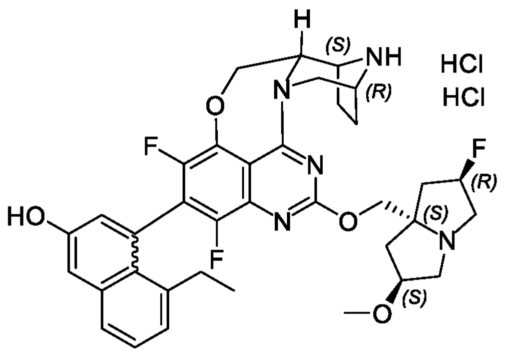

**16b**

The synthetic route is as follows:

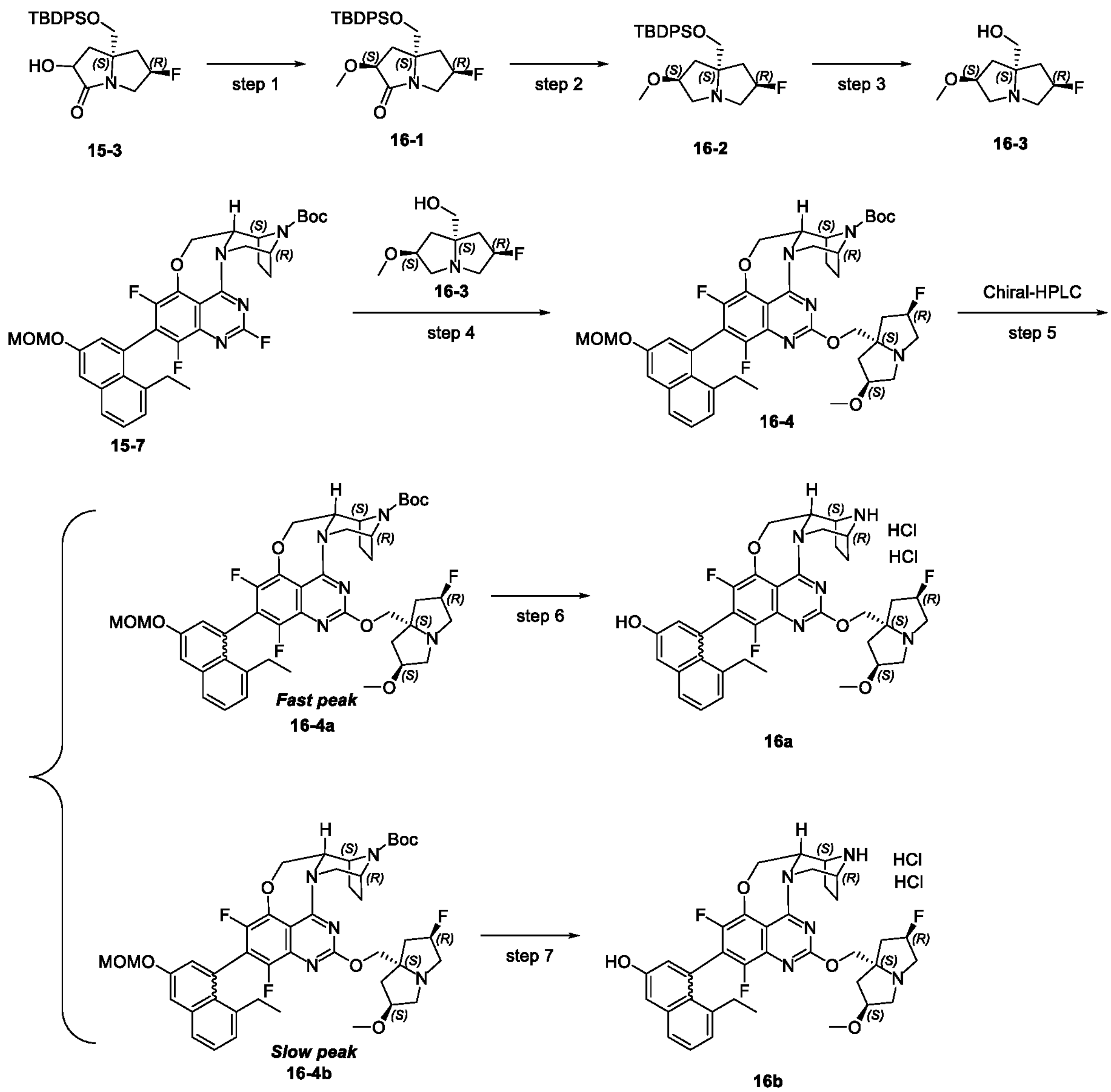


15-3 16-1 16-2 16-3

15-7 16-3 16-4

16-4a 16a

16-4b 16b

**Step 1:**

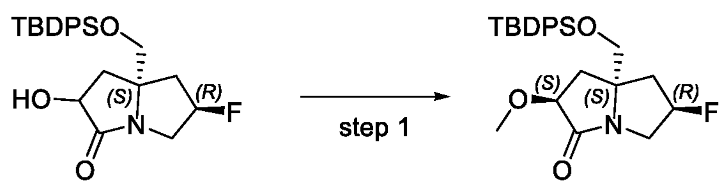


15-3 16-1

Under nitrogen atmosphere at 0°C, compound **15-3** (1.26 g, 2.80 mmol, 1 eq) and iodomethane (0.24 mL, 3.64 mmol, 1.3 eq) were dissolved in *N,N*-dimethylformamide (15 mL). Under nitrogen atmosphere at 0°C with stirring, sodium hydride (60% dispersed in mineral oil, 167.95 mg, 4.20 mmol, 1.5 eq) was added to the mixture in batches. The resulting mixture was reacted with stirring at 0°C under nitrogen atmosphere for 1 hour. The reaction progress was monitored by LC-MS and TLC. After the reaction was completed, at 0°C, the reaction mixture was added dropwise to saturated ammonium chloride solution (60 mL). The mixture was extracted with ethyl acetate (40 mL × 3). The organic phases were combined, washed with saturated brine (60 mL), then dried over anhydrous sodium sulfate, and filtered to remove the desiccant. The filtrate was concentrated under reduced pressure to obtain the crude product. The crude product was purified by silica gel

column chromatography and subjected to a gradient elution with a mobile phase of 0% to 20% methyl tert-butyl ether/petroleum ether. The collected fraction was concentrated under reduced pressure to remove the solvent, yielding compound **16-1** (colorless oil, 870 mg, yield of 67%). MS (ESI, m/z): 442.2 $[M + H]^+$; $^1$H NMR (400 MHz, $CDCl_3$) $\delta$ 7.69 - 7.55 (m, 4H), 7.46 - 7.37 (m, 6H), 5.49 - 5.30 (m, 1H), 4.17 - 4.03 (m, 1H), 3.98 - 3.90 (m, 1H), 3.91 - 3.81 (m, 1H), 3.55 - 3.47 (m, 1H), 3.43 - 3.40 (m, 3H), 3.40 - 3.27 (m, 1H), 2.68 - 2.47 (m, 1H), 2.15 - 2.05 (m, 1H), 2.02 - 1.86 (m, 2H), 1.04 (s, 9H); $^{19}$F NMR (377 MHz, $CDCl_3$) $\delta$ -171.77.

**Step 2:**

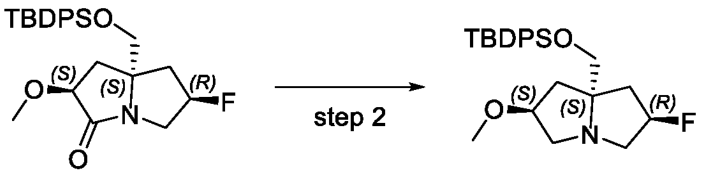


**16-1** **16-2**

Under nitrogen atmosphere at 0°C with stirring, borane-dimethyl sulfide (1 M, 0.97 mL, 9.7 mmol, 5 eq) was added dropwise to a solution of compound **16-1** (900 mg, 1.94 mmol, 1 eq) in tetrahydrofuran (10 mL). The resulting mixture was reacted with stirring at 25°C under nitrogen atmosphere for 2 hours. The reaction progress was monitored by LC-MS and TLC. After the reaction was completed, methanol (15 mL) was slowly added dropwise to the reaction mixture at 0°C with stirring. The mixture was then refluxed at 60°C for 30 minutes with stirring. Subsequently, the mixture was concentrated under reduced pressure to remove the solvent, yielding the crude product. The crude product was purified by silica gel column chromatography and subjected to a gradient elution with a mobile phase of 0% to 35% methyl tert-butyl ether/petroleum ether. The collected fraction was concentrated under reduced pressure to remove the solvent, yielding compound **16-2** (colorless oil, 840 mg, yield of 96%). MS (ESI, m/z): 428.1 $[M + H]^+$; $^1$H NMR (400 MHz, $CDCl_3$) $\delta$ 7.74 - 7.63 (m, 4H), 7.47 - 7.31 (m, 6H), 5.28 - 5.09 (m, 1H), 4.03 - 3.91 (m, 1H), 3.70 - 3.62 (m, 1H), 3.53 - 3.43 (m, 1H), 3.33 - 3.22 (m, 1H), 3.18 (s, 3H), 3.17 - 2.98 (m, 3H), 2.26 - 2.06 (m, 3H), 2.04 - 1.95 (m, 1H), 1.06 (s, 9H); $^{19}$F NMR (377 MHz, $CDCl_3$) $\delta$ -172.93.

**Step 3**:

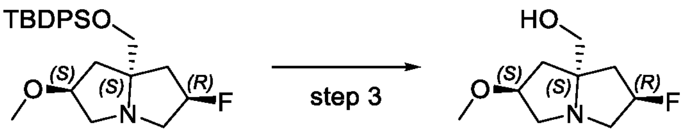


**16-2** **16-3**

With stirring at 0°C, a solution of tetrabutylammonium fluoride in tetrahydrofuran (1 M, 3.73 mL, 3.73 mmol, 2 eq) was added dropwise to a solution of compound **16-2** (840 mg, 1.87 mmol, 1 eq) in tetrahydrofuran (10 mL). The resulting mixture was reacted with stirring at 60°C for 1 hour. The reaction progress was monitored by LC-MS and TLC. After the reaction was completed, the reaction mixture was concentrated under reduced pressure to remove the solvent, yielding the crude product. The resulting crude product was purified by silica gel column chromatography and subjected to a gradient elution with a mobile phase of 0% to 7% methanol/dichloromethane. The collected fraction was concentrated under reduced pressure to remove the solvent, yielding compound **16-3** (colorless oil, 210 mg, yield of 56%). MS (ESI, m/z): 190.3 $[M + H]^+$.

**Step 4:**

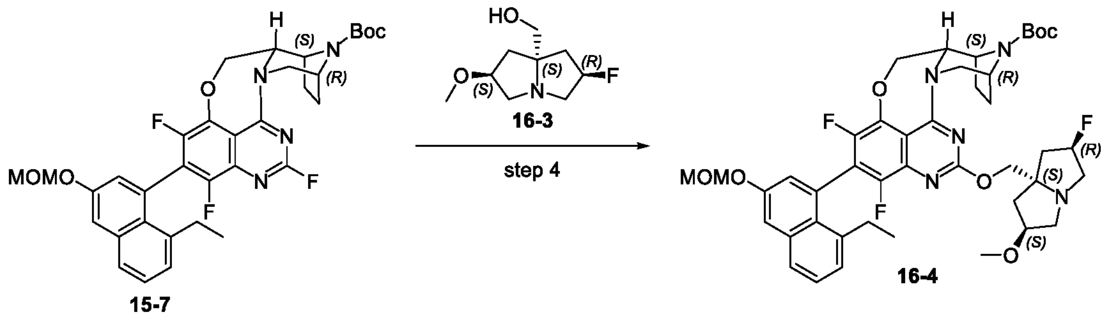


Under nitrogen atmosphere at 25°C with stirring, triethylenediamine (4.02 mg, 0.036 mmol, 0.20 eq), compound **15-7** (120 mg, 0.18 mmol, 1 eq), and cesium carbonate (122.82 mg, 0.36 mmol, 2 eq) were dispersed in *N,N*-dimethylformamide (1 mL). Then, under nitrogen atmosphere at 25°C with stirring, a solution of compound **16-3** (42.80 mg, 0.22 mmol, 1.2 eq) in N,N-dimethylformamide (1 mL) was added dropwise to the mixture. The resulting mixture was reacted with stirring at 80°C under nitrogen atmosphere for 2 hours. The reaction progress was monitored by LC-MS and TLC. After the reaction was completed, the reaction mixture was cooled to room temperature and filtered. The filter cake was washed with dichloromethane (2 mL × 3), and the filtrate was subjected to rotary evaporation under reduced pressure to obtain a crude product. The crude product was further purified by reverse-phase chromatography (C18 column) and eluted with a mobile phase of 40% to 95% methanol/water (0.1% ammonia water) over 30 minutes, with detection at UV 254/220 nm. The collected fraction was subjected to rotary evaporation under reduced pressure to remove the solvent, yielding the crude product. The crude product was further purified by preparative thin-layer chromatography using dichloromethane/methanol (15/1) as an eluent, yielding compound **16-4** (white solid, 108 mg, yield of 71%). MS (ESI, m/z): 806.4 $[M + H]^+$.

**Step 5:**

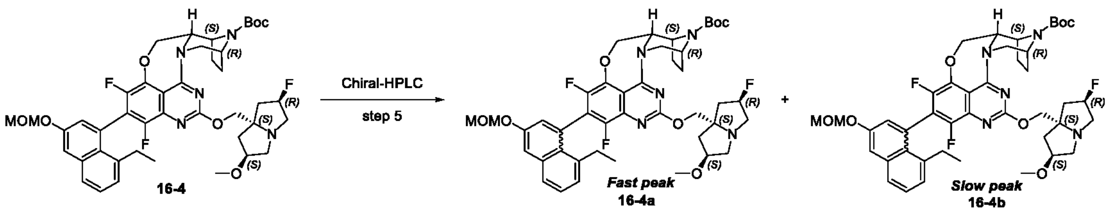


The compound **16-4** (105 mg) obtained from **step 4** of the present example was subjected to chiral resolution using preparative supercritical fluid chromatography: chiral column: CHIRALPAK ID, 2 × 25 cm, 5 μm; mobile phase A: *n*-hexane/methyl tert-butyl ether (2/1) (0.5%, 2 M ammonia in methanol), mobile phase B: methanol; flow rate: 20 mL/min; elution with 10% mobile phase B over 16 minutes; detector: UV 224/292 nm, resulting in two compounds. The product with a shorter retention time (8.61 minutes) was compound **16-4a** (white solid, 38 mg, yield of 36%), MS (ESI, *m/z*): 806.4 $[M + H]^+$. The product with a longer retention time (12.68 minutes) was compound **16-4b** (white solid, 34 mg, yield of 32%), MS (ESI, *m/z*): 806.4 [M + H]+.

**Step 6:**

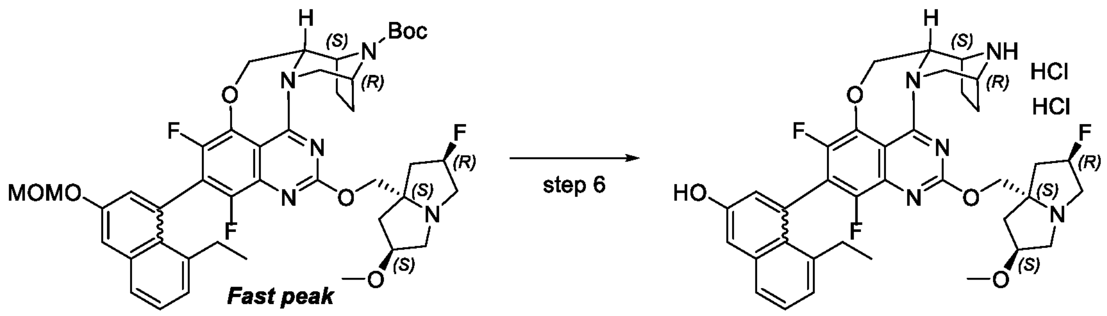


*Fast peak*
**16-4a**

**16a**

With stirring at 0°C, a solution of hydrochloric acid in 1,4-dioxane (4 M, 1 mL) was added dropwise to a solution of compound **16-4a** (38 mg, 0.047 mmol, 1 eq) in methanol (1 mL). The resulting mixture was reacted with stirring at 25°C for 1.5 hours. The reaction progress was monitored by LC-MS and TLC. After the reaction was completed, the reaction mixture was concentrated under reduced pressure to remove the solvent, yielding the crude product. The crude product was purified by reverse-phase chromatography (C18 column) and eluted with a mobile phase of 40% to 95% methanol/-water (0.1% hydrochloric acid) over 30 minutes, with detection at UV 254/220 nm. The product obtained was compound **16b** (yellow solid, 20.5 mg, yield of 59%). MS (ESI, *m/z*): 662.3 $[M + H]^+$; $^1$H NMR (400 MHz, DMSO-$d_6$ +$D_2O$) $\delta$ 7.76 - 7.65 (m, 1H), 7.44 -7.36 (m, 1H), 7.36 - 7.29 (m, 1H), 7.19 - 7.10 (m, 1H), 7.03 - 6.94 (m, 1H), 5.78 - 5.53 (m, 1H), 5.07 - 4.92 (m, 1H), 4.81 - 4.69 (m, 1H), 4.69 - 4.54 (m, 4H), 4.34 - 4.21 (m, 3H), 4.01 - 3.86 (m, 2H), 3.76 - 3.59 (m, 2H), 3.40 - 3.33 (m, 1H), 3.25 (s, 3H), 2.72 - 2.53 (m, 3H), 2.42 - 2.29 (m, 3H), 2.16 - 1.85 (m, 4H), 0.85 (t, *J* = 7.2 Hz, 3H); $^{19}$F NMR (377 MHz, DMSO-$d_6$ +$D_2O$) $\delta$ -132.33, -139.56, -174.51.

**Step 7:**

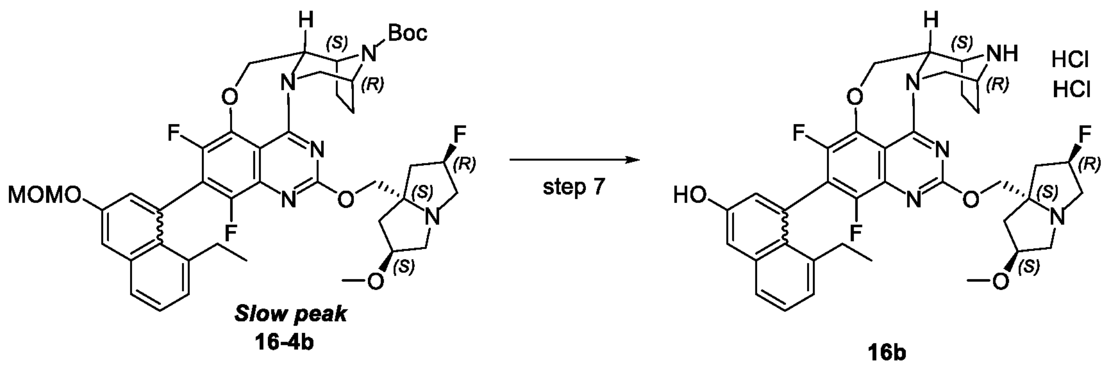


*Slow peak*
**16-4b**

**16b**

Compound **16b** was obtained using the same method as described in **step 6** of this example (yellow solid, 29 mg, yield of 93%). MS (ESI, *m/z):* 662.3 $[M + H]^+$; $^1$H NMR (400 MHz, DMSO-$d_6$ +$D_2O$) $\delta$ 7.73 - 7.66 (m, 1H), 7.43 - 7.35 (m, 1H), 7.35 - 7.31 (m, 1H), 7.18 - 7.13 (m, 1H), 6.99 - 6.94 (m, 1H), 5.75 - 5.51 (m, 1H), 5.05 - 4.90 (m, 1H), 4.80 - 4.50 (m, 5H), 4.36 - 4.19 (m, 3H), 4.01 - 3.84 (m, 2H), 3.77 - 3.58 (m, 2H), 3.40 - 3.33 (m, 1H), 3.26 (s, 3H), 2.69 - 2.53 (m, 3H), 2.45 - 2.29 (m, 3H), 2.22 - 1.85 (m, 4H), 0.88 (t, *J*= 7.2 Hz, 3H); $^{19}$F NMR (377 MHz, DMSO) $\delta$ -132.45, -139.30, -174.49.

**Example 17**

(5*aS*,6*S*,9*R*)-1,3-Difluoro-13-(((2*R*,7*aS*)-2-fluorotetrahydro-1*H*-pyrrolizin-7*a*(5*H*-yl)methoxy)-2-(5-methoxy-2-(1*H*-pyrazol-3-yl)phenyl)-5a,6,7,8,9,10-hexahydro-5*H*-6,9-epiminoazepino[2',1':3,4][1,4]oxazepino[5,6,7-*de*]quinazoline dihydrochloride **17**;

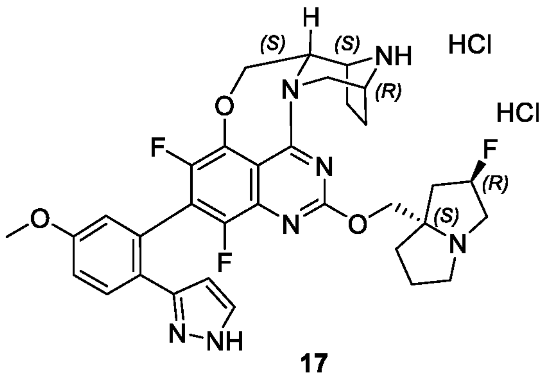

**17**

The synthetic route is as follows:

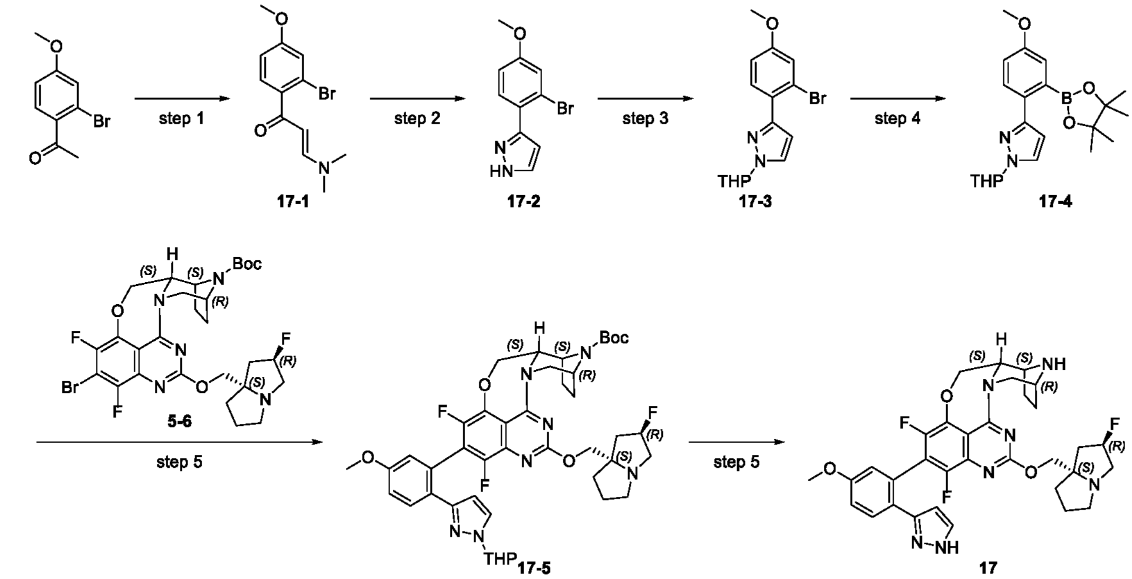


**Step 1:**

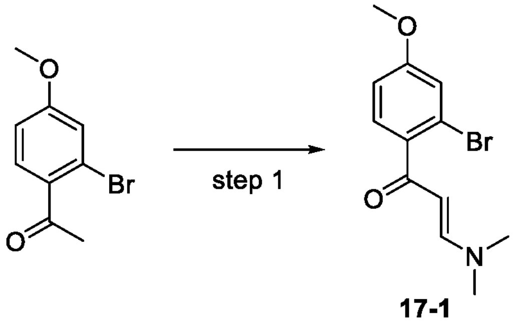


**17-1**

With stirring at 25°C, 1-(2-bromo-4-methoxy-phenyl)ethanone (5 g, 21.83 mmol, 1 eq) was dissolved in toluene (20.0 mL). With stirring at 25°C, 1,1-dimethoxy-*N,N*-dimethyl-methylamine (3.64 g, 30.5 mmol, 1.4 eq) was added to the mixture. The resulting mixture was reacted with stirring at 110°C for 16 hours with the reaction progress monitored by LC-MS and TLC. After the reaction was completed, the reaction mixture was quenched with water (50 mL). The mixture was extracted with ethyl acetate (2 × 50 mL). The organic phases were combined, then dried over anhydrous sodium sulfate, and filtered to remove the desiccant. The filtrate was subjected to rotary evaporation under reduced pressure to remove the solvent, yielding the crude product. The crude product was purified by silica gel column chromatography and subjected to a gradient elution with a mobile phase of 0% to 100% ethyl acetate/petroleum ether. The collected fraction was subjected

to rotary evaporation under reduced pressure to remove the solvent, yielding compound **17-1** (pale yellow solid, 2.5 g, yield of 40%). MS (ESI, m/z): 284.0/286.0 $[M + H]^+$. $^1H$ NMR (400 MHz, $CDCl_3$) $\delta$ 7.36 (d, *J* = 8.4 Hz, 2H), 7.13 (d, *J* = 2.4 Hz, 1H), 6.87 (d, *J* = 8.4 Hz, 1H), 5.40 (d, *J* = 12.8 Hz, 1H), 3.83 (s, 3H), 3.03 - 3.01 (m, 6H).

**Step 2:**

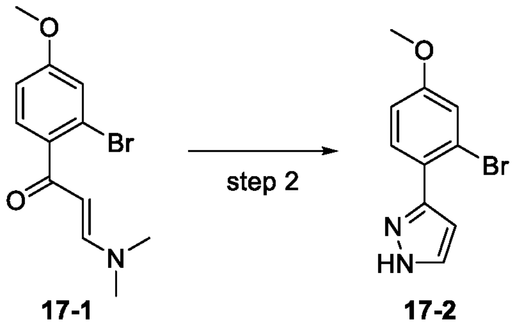


**17-1** **17-2**

With stirring at 25°C, compound **17-1** (2.3 g, 8.1 mmol, 1 eq) was dissolved in ethanol (20 mL). With stirring at 25°C, hydrazine hydrate (98%, 1.1 mL, 20 mmol, 2.4 eq) was added to the mixture. The resulting mixture was reacted with stirring at 100°C for 2 hours. The reaction progress was monitored by TLC and LC-MS After the reaction was completed, the reaction mixture was subjected to rotary evaporation under reduced pressure to remove the solvent, yielding the crude product. The crude product was diluted with water (20 mL) and the aqueous phase was extracted with dichloromethane (20 mL × 3). The organic phases were combined, dried over anhydrous magnesium sulfate, and filtered to remove the desiccant. The filtrate was subjected to rotary evaporation under reduced pressure to remove the solvent, yielding compound **17-2** (pale yellow oil, 2 g, yield of 98%). MS (ESI, m/z): 252.9/254.9 $[M + H]^+$; $^1H$ NMR ($CDCl_3$) $\delta$ 7.61 (d, *J* = 2.1 Hz, 1H), 7.49 (d, *J* = 8.6 Hz, 1H), 7.21 (d, *J* = 2.6 Hz, 1H), 6.89 (dd, *J* = 8.6, 2.6 Hz, 1H), 6.65 (d, *J* = 2.1 Hz, 1H), 3.86 (s, 3H).

**Step 3:**

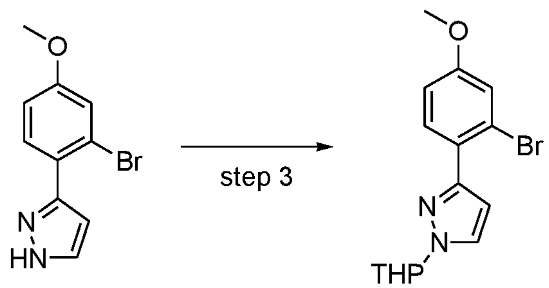


**17-2** **17-3**

With stirring at 25°C, compound **17-2** (1 g, 3.95 mmol, 1 eq) and 3,4-dihydro-2*H*-pyran (0.4 g, 5 mmol, 1.26 eq) were dissolved in tetrahydrofuran (20 mL). Then, with stirring at 25°C, *p*-toluenesulfonic acid (68 mg, 0.395 mmol, 0.1 eq) was added to the mixture. The resulting mixture was reacted with stirring at 70°C for 16 hours. The reaction progress was monitored by LC-MS and TLC. After the reaction was completed, the reaction was quenched by pouring the reaction mixture into saturated sodium bicarbonate solution (100 mL) at 0°C with stirring. The mixture was extracted with ethyl acetate (50 mL × 3). The organic phases were combined, then dried over anhydrous sodium sulfate, and filtered to remove the desiccant. The filtrate was subjected to rotary evaporation under reduced pressure to remove the solvent, yielding the crude product. The crude product was purified by silica gel column chromatography and subjected to a gradient elution with a mobile phase of 0% to 24% ethyl acetate/petroleum ether. The collected fraction was subjected to rotary evaporation under reduced pressure to remove the solvent, yielding compound **17-3** (colorless oil, 1.2 g, yield of 90%). MS (ESI, *m*/*z*): 337.1/339.1 $[M + H]^+$; $^1H$NMR ($CDCl_3$) $\delta$ 7.68 - 7.63 (m, 2H), 7.18 (d, *J* = 2.6 Hz, 1H), 6.90 (dd, *J* = 8.6, 2.6 Hz, 1H), 6.75 (d, *J* = 2.4 Hz, 1H), 5.44 (dd, *J* = 9.1, 3.2 Hz, 1H), 3.82 (s, 3H), 1.75 - 1.49 (m, 8H).

**Step 4:**

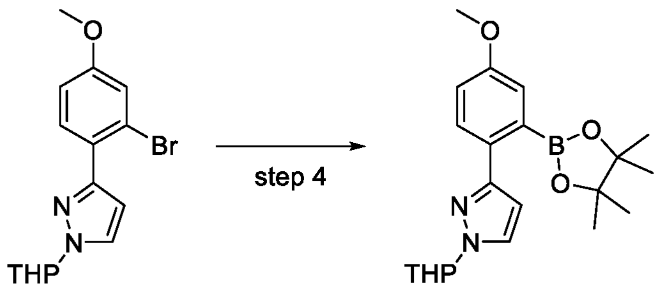


**17-3** **17-4**

Under argon atmosphere at 25°C with stirring, compound **17-3** (1 g, 2.97 mmol, 1 eq), bis(pinacolato)diboron (900 mg, 3.55 mmol, 1.2 eq), potassium acetate (580 mg, 5.91 mmol, 2 eq), and [1,1'-bis(diphenylphosphino)ferrocene] dichloropalladium(II) (220 mg, 0.3 mmol, 0.1 eq) were dissolved in 1,4-dioxane (20 mL). The mixture was reacted with stirring under argon atmosphere at 100°C for 2 hours. The reaction progress was monitored by LC-MS and TLC. After the reaction was completed, the reaction mixture was cooled to 25°C. The reaction mixture was filtered through diatomite, and the filter cake was washed with ethyl acetate (100 mL). The filtrate was subjected to rotary evaporation under reduced pressure to remove the solvent, yielding approximately 2.2 g of a brown oily crude product. The crude product was purified by silica gel column chromatography and subjected to a gradient elution with a mobile phase of 0% to 45% ethyl acetate/petroleum ether. The collected fraction was subjected to rotary evaporation under reduced pressure to remove the solvent, yielding compound **17-4** (colorless oil, 420 mg, yield of 47%). MS (ESI, *m/z*): 385.1 $[M + H]^+$.

**Step 5:**

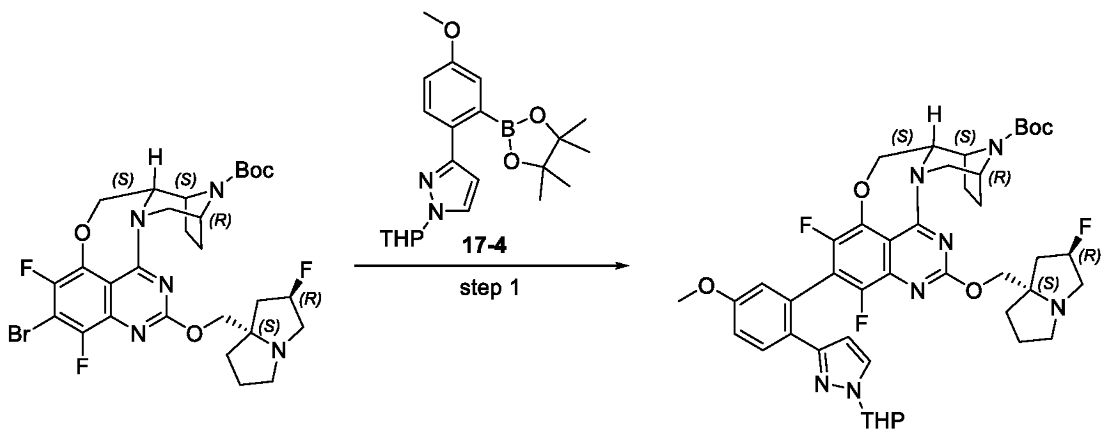


**5-1** **17-5**

Under nitrogen atmosphere at 25°C with stirring, compound **5-1** (50 mg, 0.078 mmol, 1.0 eq), compound **17-4** (39 mg, 0.101 mmol, 1.3 eq), tris(dibenzylideneacetone)dipalladium (7.15 mg, 0.008 mmol, 0.1 eq), 3-(tert-butyl)-4-(2,6-dimethoxyphenyl)-2,3-dihydrobenzo[d][1,3]oxaphosphole (5.15 mg, 0.016 mmol, 0.2 eq), and potassium phosphate (33.14 mg, 0.156 mmol, 2.0 eq) were dissolved in 0.5 mL of toluene and 0.1 mL of water. The mixture was reacted at 80°C for 16 hours, with the reaction progress monitored by TLC and LC-MS. After the reaction was completed, the reaction mixture was quenched with water (5 mL). The mixture was extracted with dichloromethane ($3 \times$ mL). The organic phases were combined, then dried over anhydrous sodium sulfate, and filtered to remove the desiccant. The filtrate was subjected to rotary evaporation under reduced pressure to remove the solvent, yielding the crude product. The crude product was purified by reverse-phase chromatography (C18 column) and eluted with a mobile phase of 5% to 95% methanol/water (0.1% ammonium bicarbonate solution) over 25 minutes, with detection at UV 254/220 nm. The product obtained was compound **17-5** (yellow oil, 15 mg, yield of 23%). MS (ESI, *m/z*): 818.4 $[M + H]^+$.

**Step 6:**

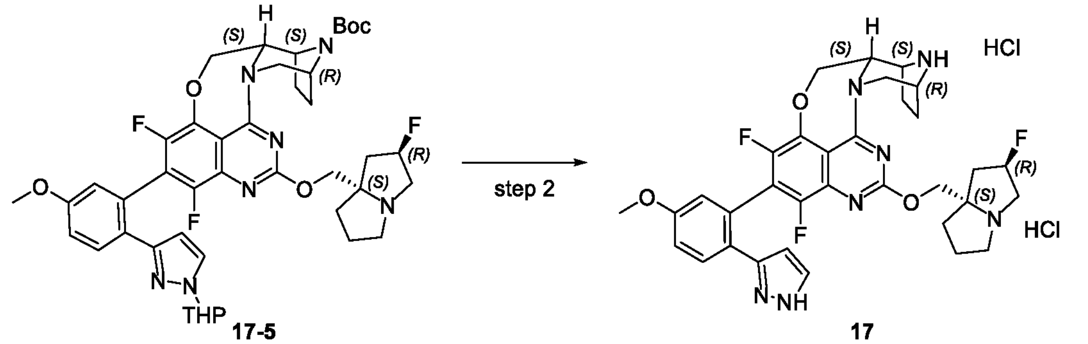


**17-5** **17**

Under nitrogen atmosphere at 0°C with stirring, compound **17-5** (15 mg, 0.015 mmol, 1.0 eq) and trimethylsilane (10.55 mg, 0.090 mmol, 5 eq) were dissolved in 0.6 mL of dichloromethane, and trifluoroacetic acid (0.2 mL) was slowly added dropwise thereto. The mixture was reacted at 25°C for 1 hour, with the reaction progress monitored by TLC and LC-MS. After the reaction was completed, the crude product was purified by reverse-phase chromatography (C18 column) and eluted with a mobile phase of 5% to 95% acetonitrile/water (0.1% hydrochloric acid) over 25 minutes, with detection at UV254/220 nm. The product obtained was compound **17** (yellow solid, 9.6 mg, yield of 74%). MS (ESI, *m/z):* 634.3 $[M + H]^+$. $^1H$ NMR (300 MHz, DMSO-$d_6$+$D_2O$) $\delta$ 7.81 - 7.71 (m, 1H), 7.53 - 7.48 (m, 1H), 7.22 - 7.13 (m, 1H), 6.97 - 6.89 (m, 1H), 5.88 - 5.78 (m, 1H), 5.71 - 5.40 (m, 1H), 5.09 - 4.87 (m, 1H), 4.70 - 4.42 (m, 6H), 4.33 - 4.20 (m, 2H), 3.87 - 3.72 (m, 5H), 3.64 - 3.60 (m, 1H), 3.35 - 3.21 (m, 1H), 2.66 - 2.56 (m, 1H), 2.45 (s, 1H), 2.37 - 2.25 (m, 1H), 2.24 - 2.11 (m, 2H), 2.11 - 1.82 (m, 5H). $^{19}F$ NMR (282 MHz, DMSO$d_6$) $\delta$ -132.55, -139.52, -172.73.

**Example 18**

4-((2*R* or 2*S*, 5a*S*,6*S*,9*R*)-1,3-Difluoro-13-(((2*R*,6*S*,7a*S*)-2-fluoro-6-(methoxymethyl)tetrahydro-1*H*-pyrrolizin-7*a*(5*H*)-yl)methoxy)-5a,6,7,8,9,10-hexahydro-5*H*-6,9-epiminoazepino[2',1':3,4][1,4]oxazepino[5,6,7-*de*]quinazolin-2-yl)-5-ethylnaphthalen-2-ol dihydrochloride **18a**; 4-((2*S* or 2*R*, 5a*S*,6*S*,9*R*)-1,3-difluoro-13-(((2*R*,6*S*,7a*S*)-2-fluoro-6-(methoxymethyl)tetrahydro-1*H*-pyrrolizin-7*a*(5*H*)-yl)methoxy)-5a,6,7,8,9,10-hexahydro-5*H*-6,9-epiminoazepino[2',1':3,4][1,4]oxazepino[5,6,7-*de*]quinazolin-2-yl)-5-ethylnaphthalen-2-ol dihydrochloride **18b**

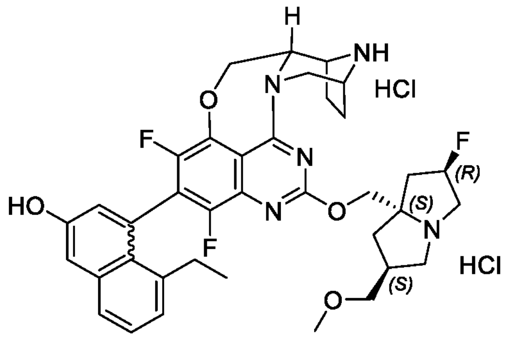

**18a**

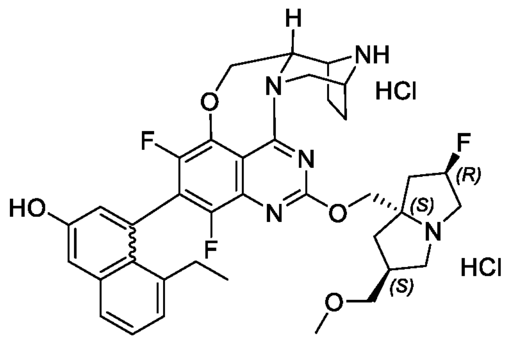

**18b**

Synthetic route

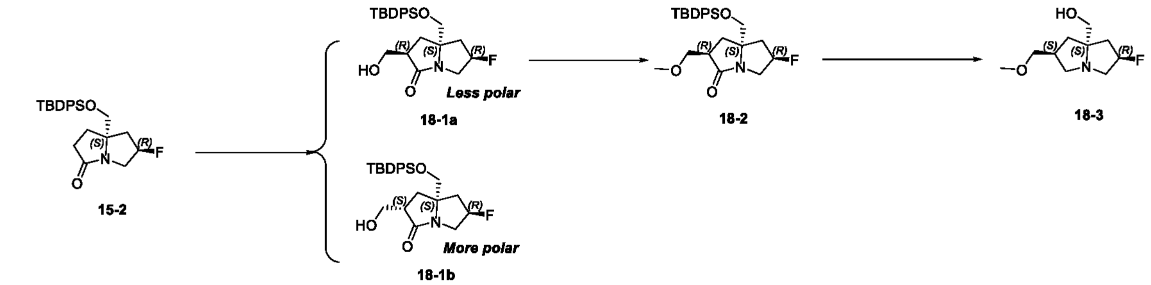


15-2, 18-1a, 18-1b, 18-2, 18-3

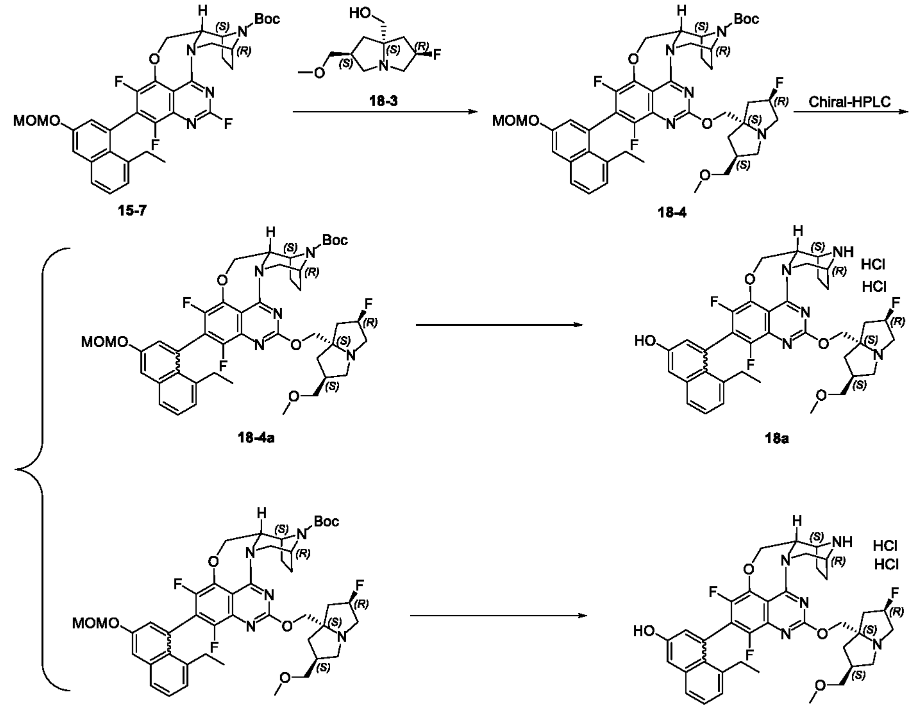


15-7, 18-3, 18-4, 18-4a, 18a, 18-4b, 18b

**Step 1:**

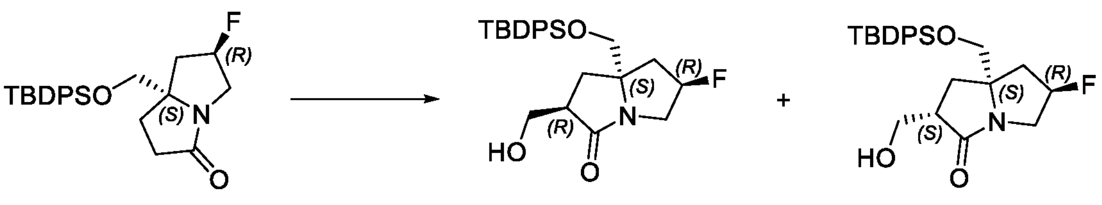

15-2, 18-1a, 18-1b

Under nitrogen atmosphere at -78°C with stirring, compound **15-2** (16 g, 36.930 mmol, 1.0 eq), tetrahydrofuran (150 mL), and hexamethylphosphoramide (25 mL) were sequentially added to a reaction flask. After the reactants were dissolved with stirring, a solution of lithium diisopropylamide in tetrahydrofuran (1 M, 46.5 mL, 1.5 eq) was added dropwise thereto. The resulting mixture was reacted with stirring at -78°C under nitrogen atmosphere for 30 minutes. Under nitrogen atmosphere at -78°C with stirring, paraformaldehyde (3.5 g, 73.860 mmol, 2.0 eq) was added to the reaction system, and the mixture was then slowly warmed to 25°C. The mixture was reacted with stirring at 25°C under nitrogen atmosphere for 2.5 hours, with the reaction progress monitored by LC-MS and TLC. After the reaction was completed, at 0°C, the reaction mixture was slowly added with saturated ammonium chloride aqueous solution (500 mL) to quench the reaction. The mixture was extracted with ethyl acetate (500 mL × 3). The organic phases were combined, dried over anhydrous sodium

sulfate, and filtered to remove the desiccant. The filtrate was concentrated under reduced pressure to remove the solvent, yielding the crude product. The resulting crude product was purified by silica gel column chromatography and subjected to a gradient elution with a mobile phase of 0% to 100% petroleum ether/methyl *tert*-butyl ether. The collected fraction was concentrated under reduced pressure to obtain two compounds. The less polar product was compound **18-1a** (yellow oil, 5.5 g, yield of 32%), MS (ESI, m/z): 442.2 $[M + H]^+$. $^1H$ NMR (400 MHz, $CDCl_3$) $\delta$ 7.68 - 7.55 (m, 4H), 7.50 - 7.35 (m, 6H), 5.34 - 5.17 (m, 1H), 4.23 - 4.06 (m, 1H), 3.88 - 3.74 (m, 1H), 3.70 - 3.62 (m, 1H), 3.59 (d, *J* = 10.4 Hz, 1H), 3.44 (d, *J* = 10.4 Hz, 1H), 3.19 - 3.07 (m, 1H), 3.06 - 2.94 (m, 1H), 2.33 - 2.16 (m, 2H), 2.07 - 1.93 (m, 1H), 1.88 - 1.79 (m, 1H), 1.05 (s, 9H); $^{19}F$ NMR (377 MHz, $CDCl_3$) $\delta$ - 173.94. The more polar crude product was further purified by reverse-phase chromatography (C18 column) and eluted with a mobile phase of 30% to 75% acetonitrile/water (0.05% trifluoroacetic acid) over 30 minutes, with detection at UV 220/254 nm. The product obtained was compound **18-1b** (yellow oil, 1.5 g, yield of 9.3%). MS (ESI, m/z): 442.2 $[M + H]^+$, $^1H$ NMR (400 MHz, $CDCl_3$) $\delta$ 7.65 - 7.57 (m, 4H), 7.49 - 7.37 (m, 6H), 5.47 - 5.28 (m, 1H), 4.15 - 4.03 (m, 1H), 3.82 - 3.76 (m, 1H), 3.71 - 3.62 (m, 2H), 3.45 - 3.40 (m, 1H), 3.40 - 3.26 (m, 1H), 2.91 - 2.81 (m, 1H), 2.63 - 2.45 (m, 1H), 2.21 - 2.16 (m, 1H), 1.97 - 1.85 (m, 1H), 1.85 - 1.78 (m, 1H), 1.05 (s, 9H); $^{19}F$ NMR (377 MHz, $CDCl_3$) $\delta$ -169.64.

**Step 2:**

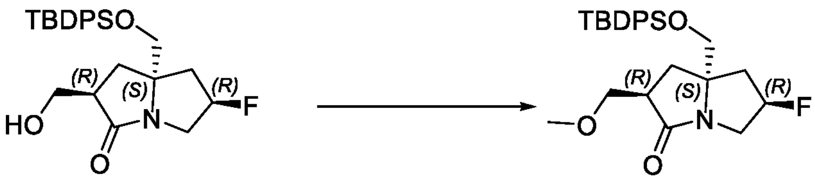

18-1a 18-2

Under nitrogen atmosphere at 0°C with stirring, sodium hydride (60%, 99.81 mg, 2.496 mmol, 2.0 eq) was added in batches to a solution of compound **18-1a** (580 mg, 1.248 mmol, 1.0 eq) and iodomethane (372.83 mg, 2.496 mmol, 2.0 eq) in *N,N*-dimethylformamide (6 mL). The resulting mixture was reacted at 25°C for 1 hour, with the reaction progress monitored by LC-MS and TLC. After the reaction was completed, the reaction mixture was quenched by pouring into saturated ammonium chloride solution and extracted with ethyl acetate (20 mL × 3). The organic phases were combined, dried over anhydrous sodium sulfate, and filtered to remove the desiccant. The filtrate was concentrated under reduced pressure to obtain a crude product. The crude product was purified by silica gel column chromatography and subjected to a gradient elution with a mobile phase of 0% to 30% ethyl acetate/petroleum ether. The collected fraction was concentrated under reduced pressure to remove the solvent, yielding compound **18-2** (colorless oil, 540 mg, yield of 90%). MS (ESI, m/z): 442.2 $[M + H]^+$; $^1H$ NMR (400 MHz, $CDCl_3$) $\delta$ 7.66 - 7.56 (m, 4H), 7.48 - 7.36 (m, 6H), 5.35 - 5.17 (m, 1H), 4.21 - 4.07 (m, 1H), 3.73 - 3.68 (m, 1H), 3.57 (d, *J* = 10.4 Hz, 1H), 3.50 - 3.45 (m, 1H), 3.42 (d, *J* = 10.4 Hz, 1H), 3.33 (s, 3H), 3.19 - 2.99 (m, 2H), 2.37 - 2.18 (m, 2H), 2.07 - 1.88 (m, 2H), 1.04 (s, 9H); $^{19}F$ NMR (377 MHz, $CDCl_3$) $\delta$ -173.49.

**Step 3:**

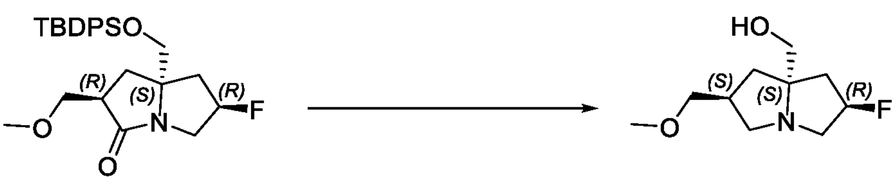

18-2 18-3

Under nitrogen atmosphere at 0°C with stirring, a solution of lithium aluminum hydride in tetrahydrofuran (1 M, 1.7 mL, 1.689 mmol, 1.5 eq) was slowly added dropwise to a solution of compound **18-2** (540 mg, 1.126 mmol, 1.0 eq) in anhydrous tetrahydrofuran (5 mL). The resulting mixture was reacted with stirring at 60°C under nitrogen atmosphere for 2 hours, with the reaction progress monitored by LC-MS and TLC. After the reaction was completed, the reaction mixture was quenched at 0°C by slowly adding water (0.5 mL), 20% sodium hydroxide aqueous solution (0.5 mL), and water (1.5 mL) sequentially with stirring. The mixture was filtered to remove the insoluble material, and the filter cake was washed with

methanol/tetrahydrofuran (10/1, 10 mL × 3). The filtrates were combined and then concentrated under reduced pressure to obtain a crude product. The crude product was purified by silica gel column chromatography and subjected to a gradient elution with a mobile phase of 0% to 10% 7 M ammonia in methanol/dichloromethane. The collected fraction was concentrated under reduced pressure to remove the solvent, yielding compound **18-3** (off-white solid, 280 mg, yield of 69%). MS (ESI, m/z): 204.1 $[M + H]^+$; $^1H$ NMR (400 MHz, $CDCl_3$) $\delta$ 5.24 - 5.03 (m, 1H), 3.32 - 3.26 (m, 3H), 3.25 (s, 3H), 3.22 (s, 1H), 3.18 - 3.13 (m, 1H), 3.13 - 3.06 (m, 1H), 3.02 - 2.83 (m, 1H), 2.70 - 2.61 (m, 1H), 2.52 - 2.37 (m, 1H), 2.07 - 1.80 (m, 3H), 1.66 - 1.56 (m, 1H); $^{19}F$ NMR (377 MHz, $CDCl_3$) $\delta$ -171.74.

**Step 4**:

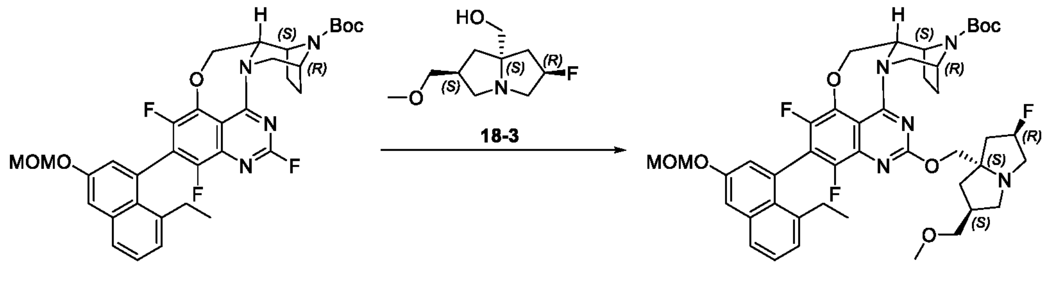


15-7 18-4

Under nitrogen atmosphere at 25°C with stirring, triethylenediamine (5.29 mg, 0.045 mmol, 0.2 eq) and cesium carbonate (153.53 mg, 0.448 mmol, 2.0 eq) were sequentially added to a solution of compound **15-7** (150 mg, 0.224 mmol, 1.0 eq) and compound **18-3** (47.89 mg, 0.224 mmol, 1.0 eq) in *N,N*-dimethylformamide (1.5 mL). The resulting mixture was reacted at 80°C for 2 hours, with the reaction progress monitored by LC-MS and TLC. After the reaction was completed, the reaction mixture was quenched by pouring into saturated ammonium chloride solution and extracted with ethyl acetate (20 mL × 3). The organic phases were combined, dried over anhydrous sodium sulfate, and filtered to remove the desiccant. The filtrate was concentrated under reduced pressure to obtain a crude product. The crude product was purified by silica gel column chromatography and subjected to a gradient elution with a mobile phase of 0% to 10% methanol/dichloromethane. The collected fraction was concentrated under reduced pressure to remove the solvent, yielding compound **18-4** (pale yellow solid, 140 mg, yield of 73%). MS (ESI, *m/z*): 820.3 $[M + H]^+$; $^1H$ NMR (400 MHz, $CDCl_3$) $\delta$ 7.74 - 7.66 (m, 1H), 7.52 (d, *J* = 2.8 Hz, 1H), 7.43 - 7.38 (m, 1H), 7.23 - 7.19 (m, 1H), 7.13 - 7.03 (m, 1H), 5.45 - 5.18 (m, 3H), 5.15 - 4.85 (m, 1H), 4.60 - 3.97 (m, 7H), 3.56 - 3.49 (m, 3H), 3.43 (s, 1H), 3.39 (d, *J* = 6.4 Hz, 2H), 3.32 (s, 3H), 3.30 - 3.05 (m, 3H), 2.87 - 2.57 (m, 2H), 2.56 - 2.41 (m, 2H), 2.42 - 2.10 (m, 3H), 2.08 - 1.80 (m, 5H), 1.78 - 1.70 (m, 3H), 1.52 (s, 9H).

**Step 5:**

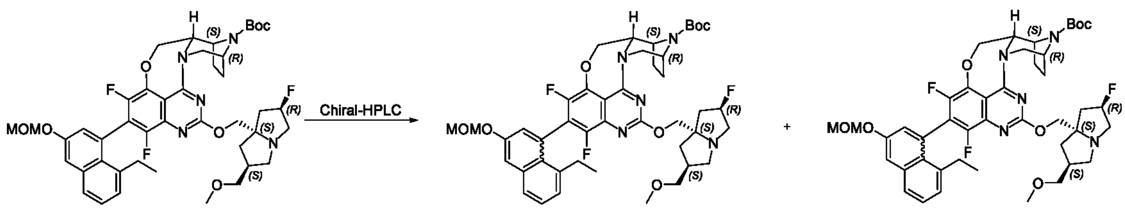


18-4 18-4a 18-4b

The compound 18-4 (140 mg) obtained from step 4 of the present example was subjected to chiral resolution using high-performance liquid chromatography: chiral column: CHIRALPAK IC, 2 × 25 cm, 5 μm; mobile phase A: n-hexane: methyl tert-butyl ether (1/1) (0.5%, 2 M ammonia in methanol), mobile phase B: methanol; flow rate: 20 mL/min; elution with 10% mobile phase B over 8.5 minutes; detector: UV 216/226 nm, resulting in two compounds. The product with a shorter retention time (5.11 minutes) was compound **18-4a** (white solid, 42 mg, yield of 30%), MS (ESI, *m/z*): 820.3 $[M + H]^+$. The product with a longer retention time (6.505 minutes) was compound **18-4b** (white solid, 42 mg, yield of 30%), MS (ESI, *m/z):* 820.3 $[M + H]^+$.

**Step 6:**

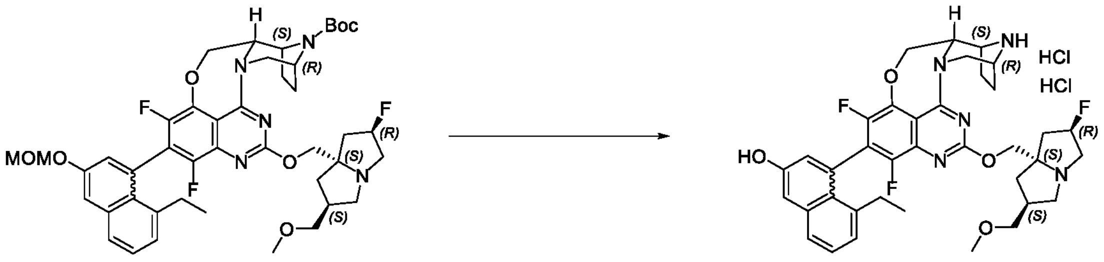

18-4a

18a

With stirring at 0°C, a solution of hydrochloric acid in 1,4-dioxane (4 M, 1 mL) was added dropwise to a solution of compound **18-4a** (42 mg, 0.049 mmol, 1.0 eq) in methanol (1 mL). The mixture was reacted with stirring at 25°C for 1 hour, with the reaction progress monitored by LC-MS. After the reaction was completed, the reaction mixture was subjected to rotary evaporation under reduced pressure to remove the solvent. The crude product was purified by reverse-phase chromatography (C18 column) and eluted with a mobile phase of 5% to 95% methanol/water (0.1% hydrochloric acid) over 30 minutes, with detection at UV 254/220 nm. This process yielded compound **18a** (white solid, 30.2 mg, yield of 82%). MS (ESI, m/z): 676.3 $[M + H]^+$; $^1H$ NMR (400 MHz, DMSO-$d_6$ +$D_2O$) $\delta$ 7.73 - 7.64 (m, 1H), 7.43 - 7.35 (m, 1H), 7.33 (d, $J$ = 2.6 Hz, 1H), 7.18 -7.11 (m, 1H), 6.97 (d, $J$ = 2.6 Hz, 1H), 5.70 - 5.50 (m, 1H), 5.11 - 4.98 (m, 1H), 4.82 - 4.68 (m, 1H), 4.67 - 4.53 (m, 4H), 4.33 - 4.21 (m, 2H), 3.97 - 3.82 (m, 2H), 3.80 - 3.56 (m, 2H), 3.44 - 3.41 (m, 1H), 3.40 - 3.33 (m, 1H), 3.26 (s, 3H), 3.11 - 3.01 (m, 1H), 2.82 - 2.69 (m, 1H), 2.60 - 2.55 (m, 1H), 2.47 - 2.29 (m, 4H), 2.17 - 1.87 (m, 5H), 0.85 (t, $J$ = 7.6 Hz, 3H); $^{19}F$ NMR (377 MHz, DMSO-$d_6$ +$D_2O$) $\delta$ -132.25, -139.52,-173.12.

**Step 7:**

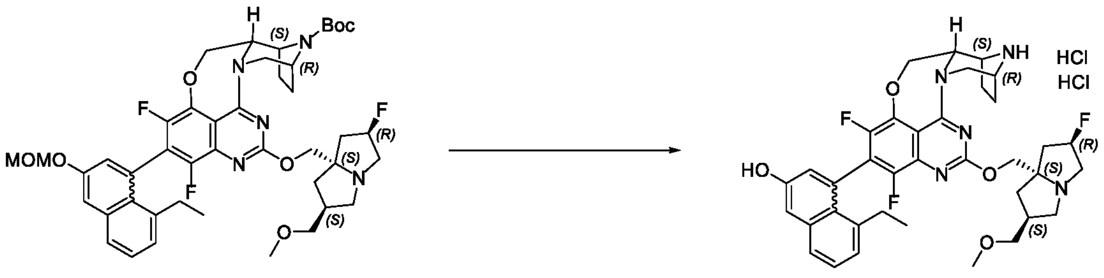

18-4b

18b

With stirring at 0°C, a solution of hydrochloric acid in 1,4-dioxane (4 M, 1 mL) was added dropwise to a solution of compound **18-4b** (42 mg, 0.049 mmol, 1.0 eq) in methanol (1 mL). The mixture was reacted with stirring at 25°C for 1 hour, with the reaction progress monitored by LC-MS. After the reaction was completed, the reaction mixture was subjected to rotary evaporation under reduced pressure to remove the solvent, yielding the crude product. The resulting crude product was purified by reverse-phase chromatography (C18 column) and eluted with a mobile phase of 5% to 95% methanol/-water (0.1% hydrochloric acid) over 30 minutes, with detection at UV 254/220 nm. The product obtained was compound **18b** (white solid, 25.0 mg, yield of 67.11%). MS (ESI, *m/z):* 676.3 $[M + H]^+$; $^1H$ NMR (400 MHz, DMSO-$d_6$+$D_2O$) $\delta$ 7.73 - 7.65 (m, 1H), 7.43 - 7.36 (m, 1H), 7.33 (d, $J$ = 2.6 Hz, 1H), 7.20 - 7.14 (m, 1H), 6.96 (d, $J$ = 2.6 Hz, 1H), 5.76 - 5.43 (m, 1H), 5.10 - 4.96 (m, 1H), 4.80 - 4.68 (m, 1H), 4.66 - 4.48 (m, 4H), 4.34 - 4.22 (m, 2H), 3.97 - 3.82 (m, 2H), 3.80 - 3.55 (m, 2H), 3.44 - 3.41 (m, 1H), 3.40 - 3.33 (m, 1H), 3.26 (s, 3H), 3.10 - 3.01 (m, 1H), 2.81 - 2.70 (m, 1H), 2.58 - 2.54 (m, 1H), 2.47 - 2.36 (m, 4H), 2.20 - 2.10 (m, 1H), 2.09 - 1.87 (m, 4H), 0.88 (t, $J$= 7.6 Hz, 3H); $^{19}F$ NMR (376 MHz, DMSO-$d_6$ +$D_2O$) $\delta$ -132.32, -139.22, -173.09.

**Example 19**

4-((5a*S*,6*S*,9*R*)-1,3-Difluoro-13-(((2*R*,7a*S*)-2-fluorotetrahydro-1*H*-pyrrolizin-7*a*(5*H*)-yl)methoxy)-5*a*,6,7,8,9,10-hexahydro-5*H*-6,9-epiminoazepino[2',1':3,4][1,4]oxazepino[5,6,7-*de*]quinazolin-2-yl)-7-fluorobenzo[*d*]thiazol-2-amine dihydrochloride **19**

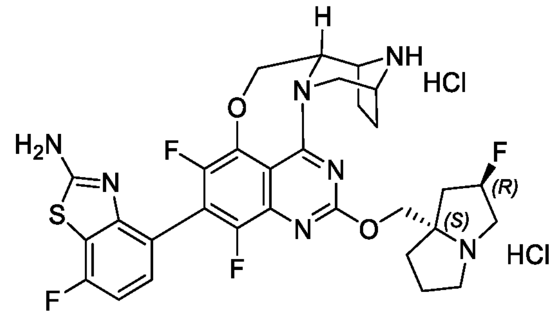

**19**

Synthetic route

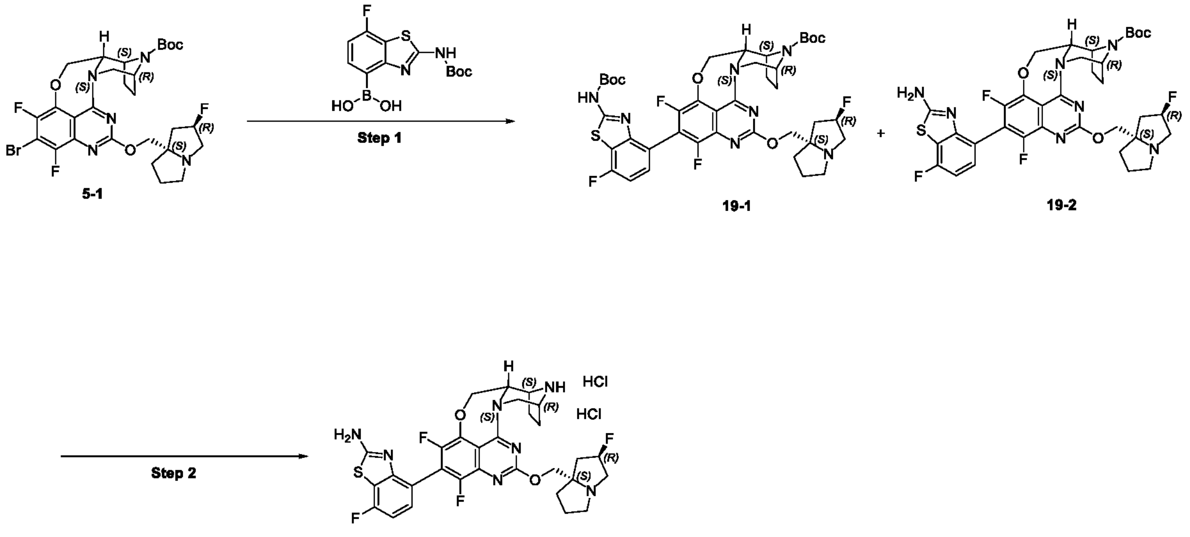


5-1 19-1 19-2

19

**Step 1:**

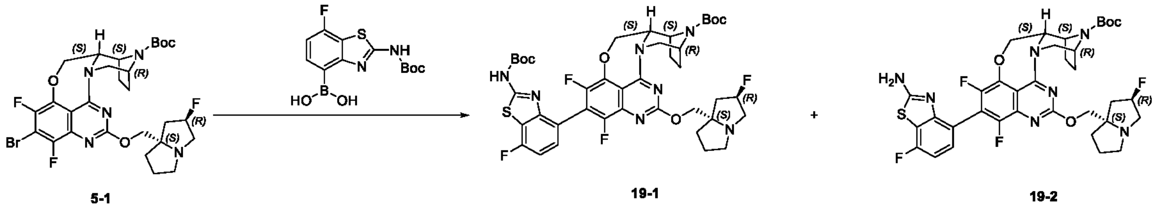

5-1 19-1 19-2

Under nitrogen atmosphere at 25°C with stirring, [2-(tert-butoxycarbonylamino)-7-fluoro-1,3-benzothiazol-4-yl] boronic acid (14.62 mg, 0.045 mmol, 1.5 eq), cesium carbonate (20.3 mg, 0.06 mmol, 2.0 eq), and tetrakis(triphenylphosphine)palladium (3.61 mg, 0.003 mmol, 0.1 eq) were sequentially added to a solution of compound **5-1** (20.0 mg, 0.03 mmol, 1.0 eq) in 1,4-dioxane/water (5/1, 1.2 mL). The reaction mixture was reacted at 100°C for 16 hour, with the reaction progress monitored by LC-MS and TLC. After the reaction was completed, the reaction mixture was cooled to 25°C. The reaction mixture was concentrated, then purified by silica gel column chromatography, and subjected to a gradient elution with a mobile phase of 0% to 10% methanol/dichloromethane. The collected fraction was subjected to rotary evaporation under reduced pressure to remove the solvent, yielding compound **19-1** (white solid, 8 mg, yield of 31%), MS (ESI, *m*/*z*): 828.0 $[M + H]^+$, and compound **19-2** (white solid, 10 mg, yield of 44%), MS (ESI, m/z): 728.0 $[M + H]^+$.

**Step 2:**

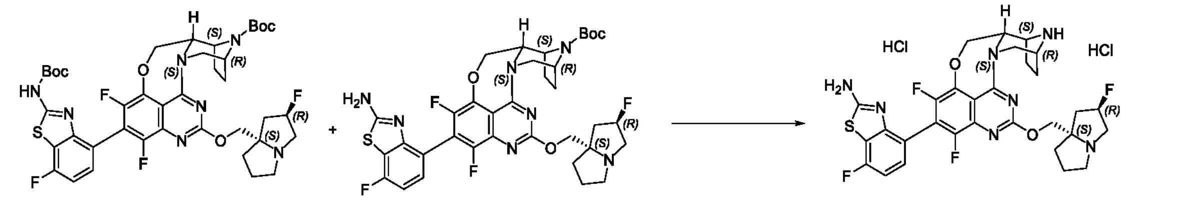

19-1 19-2 19

With stirring at 0°C, a solution of hydrochloric acid in dioxane (4 M, 1.0 mL) was slowly added dropwise to a solution of compound **19-1** (8.0 mg, 0.01 mmol, 1.0 eq) and compound **19-2** (10.0 mg, 0.01 mmol, 1.0 eq) in methanol (1.0 mL). The mixture was reacted with stirring at 25°C for 1 hour, with the reaction progress monitored by LC-MS. After the reaction was completed, the reaction mixture was concentrated to obtain the crude product. The crude product was purified by high-performance liquid chromatography under the following preparation conditions: reverse-phase column XSelect CSH Prep C18 OBD Column, 19 × 250 mm, 5 μm; mobile phase A: water (0.1% hydrochloric acid), mobile phase B: acetonitrile; flow rate: 25 mL/min; elution with a gradient of 5% to 95% mobile phase B over 30 minutes; detector: UV 254/220 nm. The product obtained was compound **19** (yellow solid, 4.10 mg, yield of 27%). MS (ESI, m/z): 628.05 $[M + H]^+$; $^1H$ NMR (400 MHz, $CD_3OD$) $\delta$ 7.65 - 7.52 (m, 1H), 7.37 - 7.21 (m, 1H), 5.68 - 5.54 (m, 2H), 4.71 - 4.68 (m, 1H), 4.48 - 4.29 (m, 3H), 4.13 - 3.85 (m, 4H), 3.83 - 3.72 (m, 1H), 3.51 - 3.42 (m, 1H), 2.90 - 2.46 (m, 4H), 2.43 - 2.31 (m, 3H), 2.31 - 2.06 (m, SH); $^{19}F$ NMR (377 MHz, $CD_3OD$) $\delta$ -113.73, -134.44, -136.41, -174.19.

**Example 20**

4-((2*R* or 2*S*, 5*aS*,6*S*,9*R*)-1,3-Difluoro-13-(((6'*R*,7*a*'*S*)-6'-fluorotetrahydrospiro[cyclopropane-1,3'-pyrrolizin]-7*a*'(5'*H*) methoxy)-5a,6,7,8,9,10-hexahydro-5*H*-[6,9]epiminoazepino[2',1':3,4][1,4]oxazepino[5,6,7-*de*]quinazolin-2-yl)-5-ethylnaphthalen-2-ol dihydrochloride 20a; 4-((2S or 2*R*, 5*aS,6S*,9*R*)-1,3-difluoro-13 -(((6'*R*,7a'*S*)-6'-fluorotetrahydrospiro [cyclopropane-1,3'-pyrrolizin]-7a'(5'*H*)methoxy)-5a,6,7,8,9,10-hexahydro-5*H*-[6,9]epiminoazepino[2',1':3,4][1,4]oxazepino[5,6,7-*de*]quinazolin-2-yl)-5-ethylnaphthalen-2-ol dihydrochloride 20b

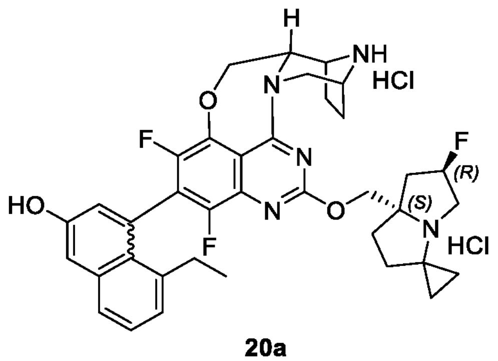

20a

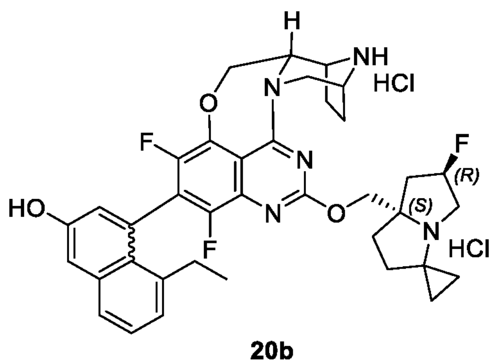

20b

Synthetic route

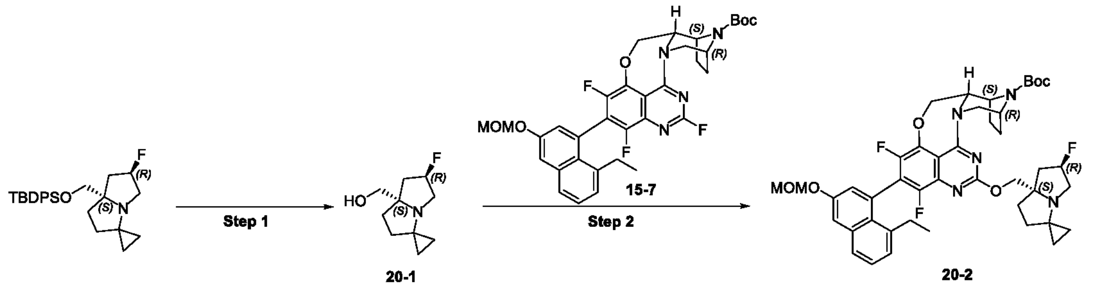


20-1 20-2

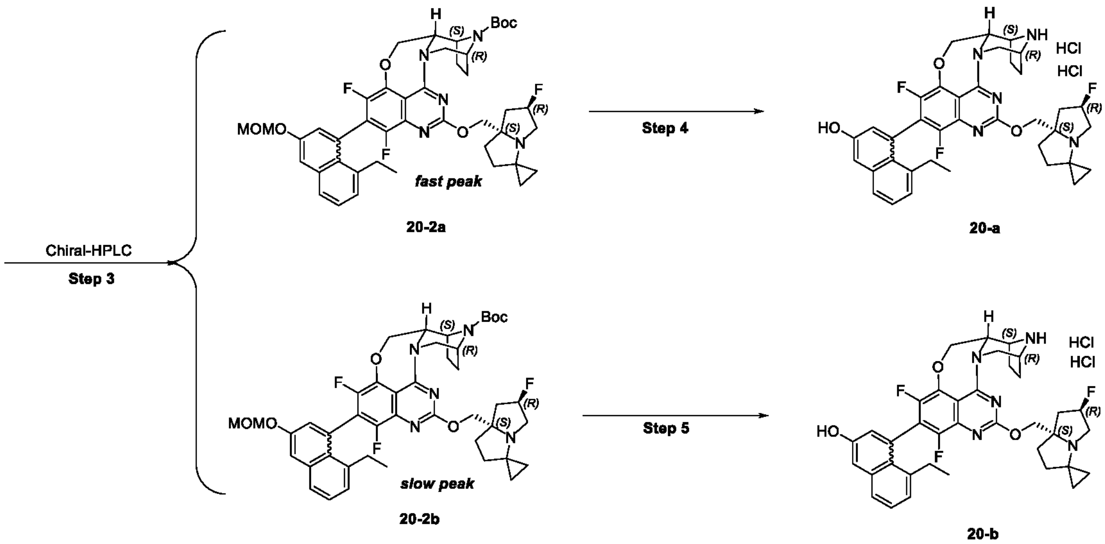


20-2a 20-a

20-2b 20-b

**Step 1:**

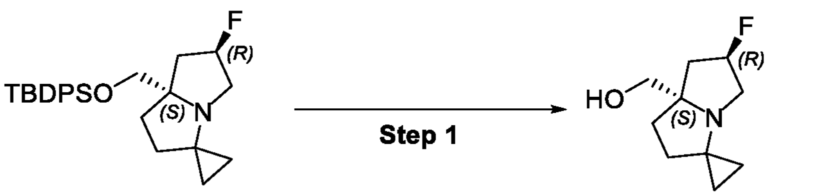


SPH 20-1

Under nitrogen atmosphere at 25°C with stirring, a solution of tetrabutylammonium fluoride in tetrahydrofuran (1 M, 3 mL, 2.0 eq) was added to a solution of compound **SPH** (670 mg, 1.502 mmol, 1.0 eq) in tetrahydrofuran (5 mL). The mixture was reacted with stirring at 25°C for 16 hours, with the reaction progress monitored by LC-MS and TLC. After the reaction was completed, the reaction mixture was concentrated under reduced pressure to obtain the crude product. The resulting crude product was purified by silica gel column chromatography and subjected to a gradient elution with a mobile phase of 0% to 10% ammonia in methanol/dichloromethane. The collected fraction was concentrated under reduced pressure to remove the solvent, yielding compound **20-1** (pale yellow solid, 290 mg, yield of 99%). MS (ESI, *m/z*):186.2 [M + H]$^+$; $^1$H NMR (300 MHz, $CDCl_3$) $\delta$ 5.26 - 5.00 (m, 1H), 3.52 - 3.12 (m, 4H), 2.50 - 2.30 (m, 2H), 2.18 - 1.96 (m, 2H), 1.87 - 1.74 (m, 1H), 1.39 - 1.26 (m, 1H), 0.94 - 0.83 (m, 1H), 0.71 - 0.60 (m, 2H), 0.51 - 0.41 (m, 1H).

**Step 2:**

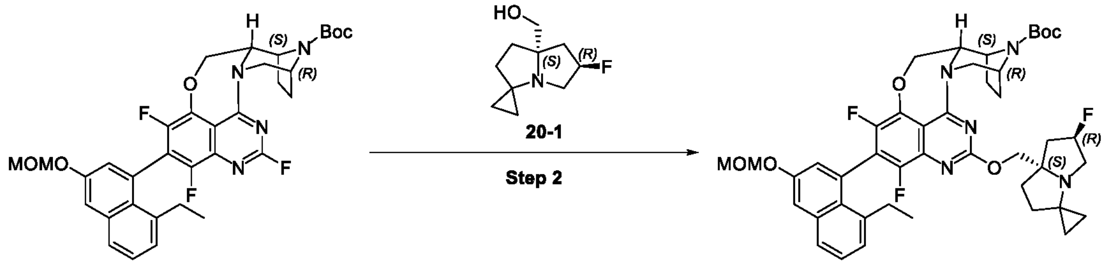


15-7 20-2

Under nitrogen atmosphere at 25°C with stirring, compound 15-7 (150 mg, 0.224 mmol, 1.0 eq), triethylenediamine (5.29 mg, 0.045 mmol, 0.2 eq), cesium carbonate (153.23 mg, 0.448 mmol, 2.0 eq), compound **20-1** (48.01 mg, 0.246 mmol, 1.1 eq), and *N,N*-dimethylformamide (2 mL) were sequentially added to a reaction flask. The resulting mixture was reacted with stirring at 80°C under nitrogen atmosphere for 5 hours, with the reaction progress monitored by LC-MS. After the reaction was completed, the reaction mixture was cooled to room temperature and filtered. The filter cake was washed with dichloromethane (2 mL × 3). The filtrates were combined and subjected to rotary evaporation under reduced pressure to obtain a crude product. The crude product was purified by reverse-phase chromatography (C18 column) and eluted with a mobile phase of 5% to 95% methanol/water (0.1% ammonia water) over 25 minutes, with detection at UV 254/220 nm. The product obtained was compound **20-2** (white solid, 95 mg, yield of 50%). MS (ESI, *m/z):* 802.5 [M + H]$^+$.

**Step 3:**

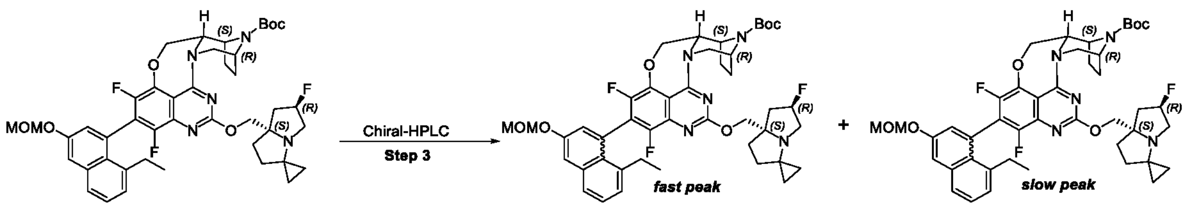


20-2 20-2a 20-2b

The compound **20-2** (95 mg) obtained from **step 2** of the present example was subjected to chiral resolution using high-performance liquid chromatography under the following conditions: chiral column: CHIRALPAK ID, 2 × 25 cm, 5 μm; mobile phase A: *n*-hexane/methyl *tert*-butyl ether (1/1) (0.5%, 2 M ammonia in methanol), mobile phase B: methanol; flow rate: 20 mL/min; elution with 15% mobile phase B over 9.5 minutes; detector: UV 226/292 nm. Two compounds were obtained. The product with a shorter retention time (4.17 minutes) was compound **20-2a** (pale yellow solid, 37 mg, yield of 39%), MS (ESI, *m/z):* 802.4[M + H]$^+$. The product with a longer retention time (6.78 minutes) was compound **20-2b** (pale yellow solid, 35 mg, yield of 37%), MS (ESI, *m/z):* 802.4[M + H]$^+$.

**Step 4:**

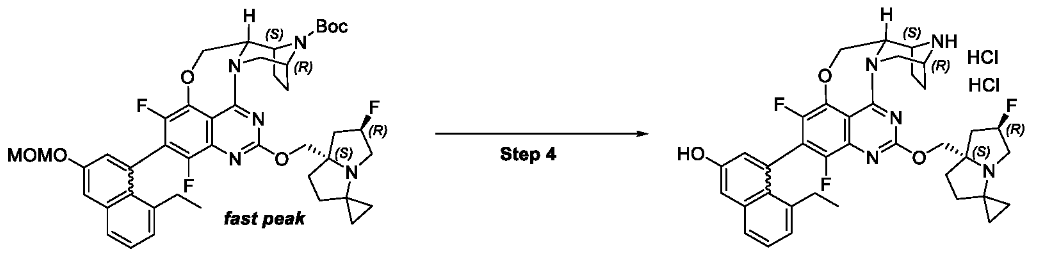


20-2a

20a

With stirring at 0°C, a solution of hydrochloric acid in 1,4-dioxane (4 M, 1 mL) was added dropwise to a solution of compound **20-2a** (37 mg, 0.044 mmol, 1.0 eq) in methanol (1 mL). The mixture was reacted at room temperature for 1 hour, with the reaction progress monitored by LC-MS. After the reaction was completed, the reaction mixture was concentrated to obtain the crude product. The crude product was purified by high-performance liquid chromatography: column Xselect CSH C18 OBD, 30 × 150 mm, 5 μm; mobile phase A: water (0.1% hydrochloric acid), mobile phase B: acetonitrile; flow rate: 60 mL/min; elution with 9% to 24% mobile phase B over 10 minutes; detector: UV 220/254 nm. The product obtained was compound **20a** (yellow solid, 15.5 mg, yield of 48%). MS (ESI, m/z): 658.4 $[M + H]^+$; $^1$H NMR (400 MHz, DMSO-$d_6$ +$D_2O$) $\delta$ 7.72 - 7.67 (m, 1H), 7.44 - 7.37 (m, 1H), 7.34 (d, *J* = 2.6 Hz, 1H), 7.18 - 7.13 (m, 1H), 6.97 (d, *J* = 2.6 Hz, 1H), 5.62 - 5.43 (m, 1H), 5.11 - 5.02 (m, 1H), 4.78 - 4.51 (m, 5H), 4.35 - 4.23 (m, 2H), 4.02 - 3.83 (m, 1H), 3.80 - 3.67 (m, 1H), 3.60 (d, *J* = 14.2 Hz, 1H), 2.74 - 2.54 (m, 3H), 2.49 - 2.40 (m, 1H), 2.40 - 2.32 (m, 2H), 2.30 - 2.19 (m, 1H), 2.18 - 2.06 (m, 1H), 2.06 - 1.90 (m, 3H), 1.87 - 1.78 (m, 1H), 1.53 - 1.44 (m, 1H), 1.25 - 1.16 (m, 1H), 1.06 - 0.91 (m, 2H), 0.88 - 0.81 (m, 3H); $^{19}$F NMR (377 MHz, DMSO-$d_6$) $\delta$ -132.30, -139.61, -172.83.

**Step 5:**

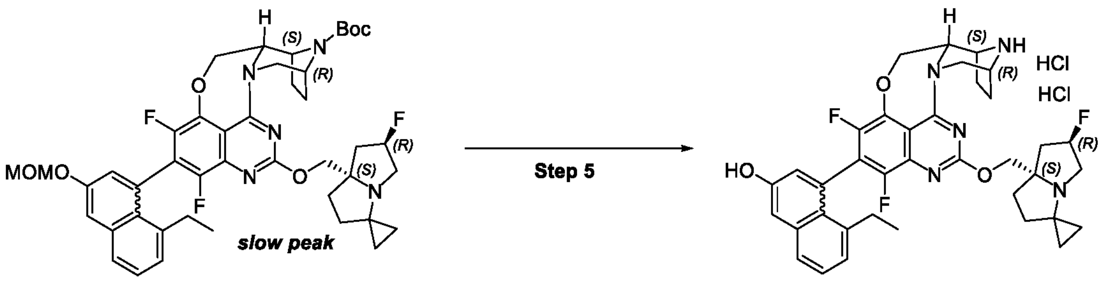


20-2b

20b

With stirring at 0°C, a solution of hydrochloric acid in 1,4-dioxane (4 M, 0.5 mL) was added dropwise to a solution of compound **20-2b** (35 mg, 0.044 mmol, 1.00 eq) in methanol (0.5 mL). The mixture was reacted at room temperature for 1 hour, with the reaction progress monitored by LC-MS. After the reaction was completed, the reaction mixture was concentrated to obtain the crude product. The crude product was purified by reverse-phase flash chromatography (C18 column) and eluted with a mobile phase of 5% to 95% acetonitrile/water (0.1% hydrochloric acid) over 30 minutes, with detection at UV 254 nm. The product obtained was compound **21b** (yellow solid, 17.6 mg, yield of 57%). MS (ESI, m/z): 658.4 $[M + H]^+$, $^1$H NMR (400 MHz, DMSO-$d_6$ +$D_2O$) $\delta$ 7.71 - 7.66 (m, 1H), 7.42 - 7.36 (m, 1H), 7.33 (d, *J* = 2.6 Hz, 1H), 7.19 - 7.13 (m, 1H), 6.98 (d, *J* = 2.6 Hz, 1H), 5.61 - 5.42 (m, 1H), 5.08 - 4.99 (m, 1H), 4.78 - 4.54 (m, 5H), 4.34 - 4.25 (m, 2H), 4.02 - 3.85 (m, 1H), 3.77 - 3.62 (m, 2H), 2.73 - 2.53 (m, 3H), 2.47 - 2.36 (m, 3H), 2.28 - 2.12 (m, 2H), 2.10 - 1.90 (m, 3H), 1.86 - 1.77 (m, 1H), 1.65 - 1.53 (m, 1H), 1.26 - 1.15 (m, 1H), 1.06 - 0.97 (m, 1H), 0.96 - 0.91 (m, 1H), 0.91 - 0.85 (m, 3H); $^{19}$F NMR (377 MHz, DMSO-$d_6$) $\delta$ -132.32, -139.41, -172.75.

**Example 21**

4-((2*R* or 2*S*, 5a*S*,6*S*,9*R*)-1,3-Difluoro-13-(((2*R*,6*R*,7a*S*)-2-fluoro-6-(hydroxymethyl)tetrahydro-1*H*-pyrrolizin-7*a*(5*H*)-yl)methoxy)-5a,6,7,8,9,10-hexahydro-5*H*-6,9-epiminoazepino[2',1':3,4][1,4]oxazepino[5,6,7-*de*]quinazolin-2-yl)-5-ethylnaphthalen-2-ol dihydrochloride **21a**; 4-((2*S* or 2*R*, 5a*S*,6*S*,9*R*)-1,3-difluoro-13-(((2*R*,6*R*,7a*S*)-2-fluoro-6-(hydroxymethyl)tetrahydro-1*H*-pyrrolizin-7*a*(5*H*)-yl)methoxy)-5a,6,7,8,9,10-hexahydro-5*H*-6,9-epiminoazepino[2',1':3,4][1,4]oxazepino[5,6,7-*de*]quinazolin-2-yl)-5-ethylnaphthalen-2-ol dihydrochloride **21b**

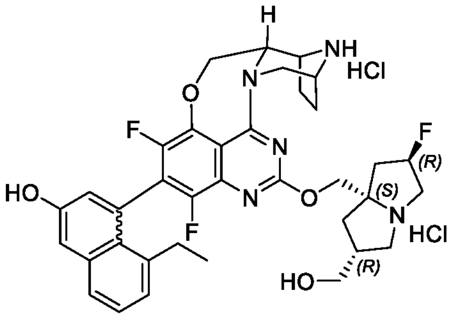

**21a**

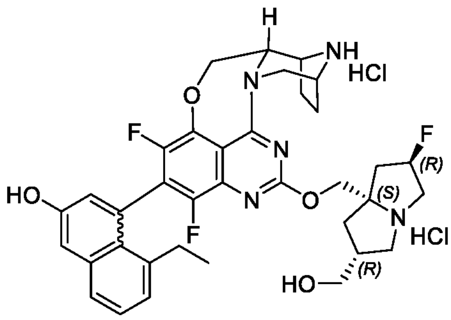

**21b**

The synthetic route is as follows:

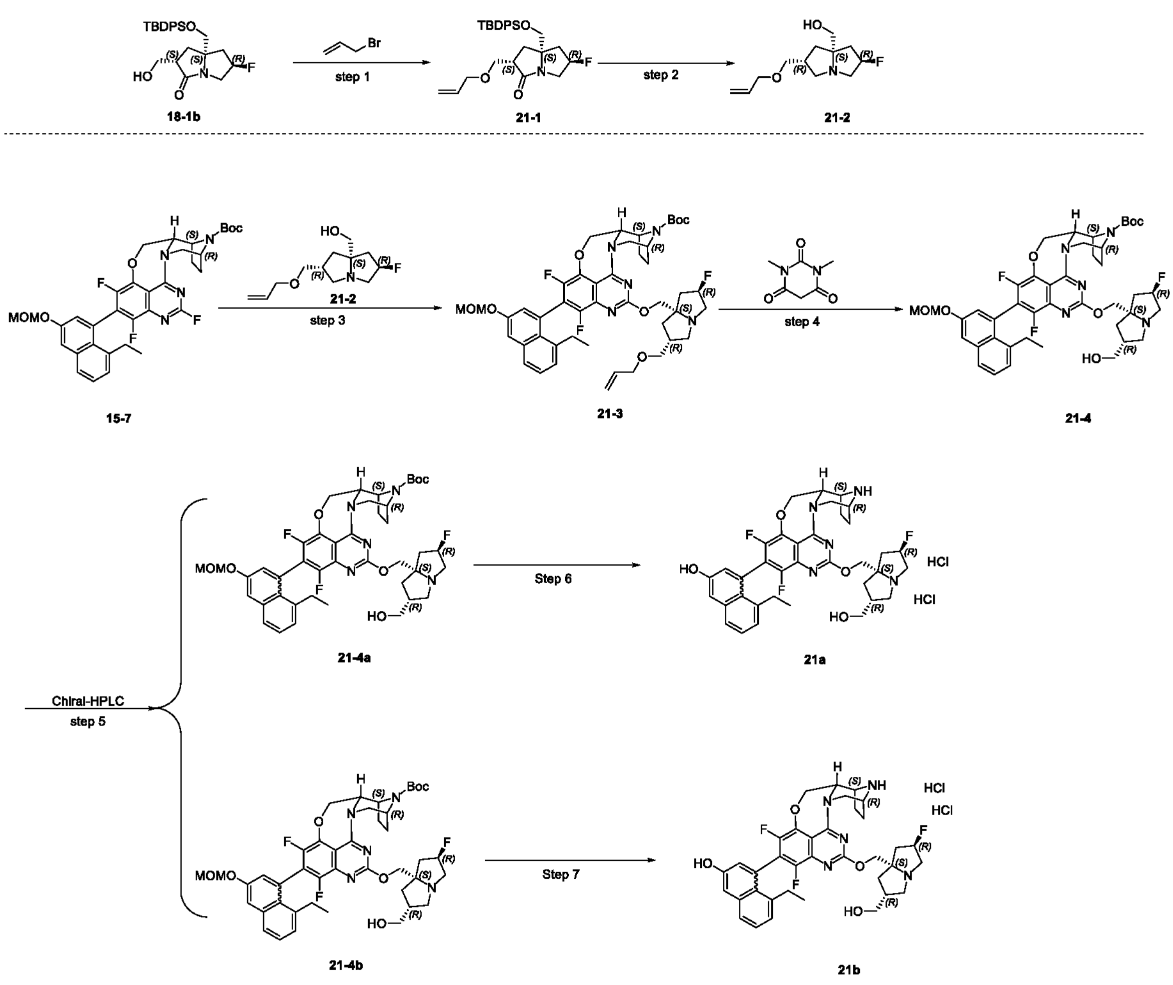

**Step 1:**

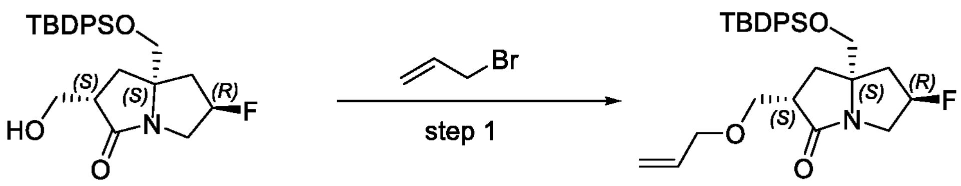


**18-1b** **21-1**

Under nitrogen atmosphere at 0°C with stirring, compound **18-1b** (700 mg, 1.506 mmol, 1.0 eq), allyl bromide (325.99 mg, 2.560 mmol, 2.0 eq), and anhydrous *N,N*-dimethylformamide (10 mL) were sequentially added to a reaction flask. Sodium hydride (60% dispersed in mineral oil, 102.39 mg, 2.560 mmol, 2.0 eq) was then added thereto in batches. The resulting mixture was reacted with stirring at 0°C under nitrogen atmosphere for 1 hour, with the reaction progress monitored by LC-MS and TLC. After the reaction was completed, the reaction mixture was quenched by pouring into saturated ammonium chloride aqueous solution (100 mL) and extracted with ethyl acetate (100 mL × 3). The organic phases were combined, dried over anhydrous sodium sulfate, and filtered to remove the desiccant. The filtrate was concentrated under reduced pressure to obtain a crude product. The crude product was purified by silica gel column chromatography and subjected to a gradient elution with a mobile phase of 0% to 50% ethyl acetate/petroleum ether. The collected fraction was concentrated under reduced pressure to remove the solvent, yielding compound **21-1** (colorless oil, 600 mg, yield of 78%). MS (ESI, m/z): 482.2[M + H]$^+$; $^1$H NMR (400 MHz, $CDCl_3$) $\delta$ 7.66 - 7.57 (m, 4H), 7.48 - 7.35 (m, 6H), 5.76 - 5.64 (m, 1H), 5.48 - 5.31 (m, 1H), 5.17 - 5.01 (m, 2H), 4.17 - 4.04 (m, 1H), 3.87 - 3.81 (m, 2H), 3.74 (d, *J* = 10.4, 1H), 3.62 - 3.50 (m, 2H), 3.46 - 3.30 (m, 2H), 2.90 - 2.82 (m, 1H), 2.66 - 2.52 (m, 1H), 2.20 - 2.10 (m, 1H), 1.98 - 1.81 (m, 2H), 1.04 (s, 9H).

**Step 2:**

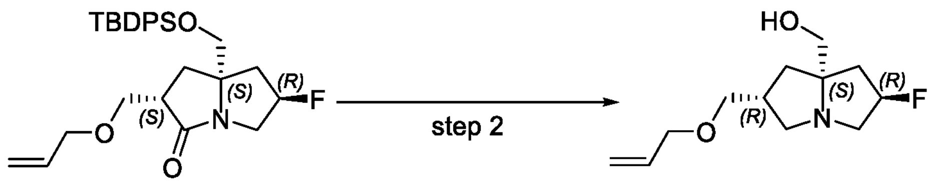


**21-1** **21-2**

Under nitrogen atmosphere at 0°C with stirring, a solution of lithium aluminum hydride in tetrahydrofuran (1 M, 2 mL, 1.5 eq) was slowly added dropwise to a solution of compound **21-2** (400 mg, 0.789 mmol, 1.0 eq) in anhydrous tetrahydrofuran (6 mL). The mixture was reacted with stirring at 60°C for 16 hours, with the reaction progress monitored by LC-MS and TLC. After the reaction was completed, the reaction mixture was cooled to 0°C, and then water (0.5 mL), 20% sodium hydroxide (0.5 mL), and water (1.5 mL) were sequentially and slowly added dropwise thereto. The resulting mixture was filtered, and the filtrate was concentrated under reduced pressure to obtain a crude product. The resulting crude product was purified by silica gel column chromatography and subjected to a gradient elution with a mobile phase of 0% to 10% ammonia in methanol/dichloromethane. The collected fraction was concentrated under reduced pressure to remove the solvent, yielding compound **21-2** (colorless oil, 80 mg, yield of 42%). MS (ESI, *m/z*): 230.1 [M + H]$^+$; $^1$H NMR (400 MHz, $CDCl_3$) $\delta$ 5.95 - 5.84 (m, 1H), 5.29 - 5.07 (m, 3H), 3.99 - 3.94 (m, 2H), 3.47 - 3.41 (m, 1H), 3.38 - 3.31 (m, 2H), 3.28 - 3.22 (m, 1H), 3.17 - 3.06 (m, 2H), 2.97 - 2.90 (m, 1H), 2.86 - 2.72 (m, 1H), 2.29 - 2.14 (m, 2H), 2.09 - 1.98 (m, 2H), 1.66 - 1.56 (m, 1H).

**Step 3:**

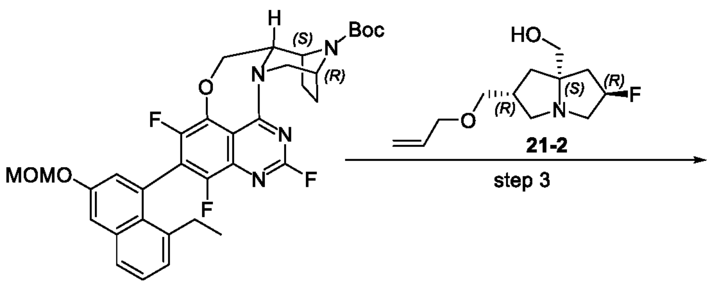


15-7 21-3

Under nitrogen atmosphere at 25°C with stirring, triethylenediamine (5.29 mg, 0.045 mmol, 0.2 eq), compound **15-7** (150 mg, 0.224 mmol, 1.0 eq), cesium carbonate (153.53 mg, 0.448 mmol, 2.0 eq), compound **21-2** (59.42 mg, 0.224 mmol, 1.0 eq), andN,N-dimethylformamide (1.5 mL) were sequentially added to a reaction flask. The resulting mixture was reacted with stirring at 80°C under nitrogen atmosphere for 5 hours, with the reaction progress monitored by LC-MS and TLC. After the reaction was completed, the crude product was purified by reverse-phase chromatography (C18 column) and eluted with a mobile phase of 5% to 95% methanol/water (0.1% ammonium bicarbonate aqueous solution) over 25 minutes, with detection at UV254/220 nm. The product obtained was compound **21-3** (white solid, 80 mg, yield of 40%). MS (ESI, *m/z*): 846.4 $[M + H]^+$.

**Step 4:**

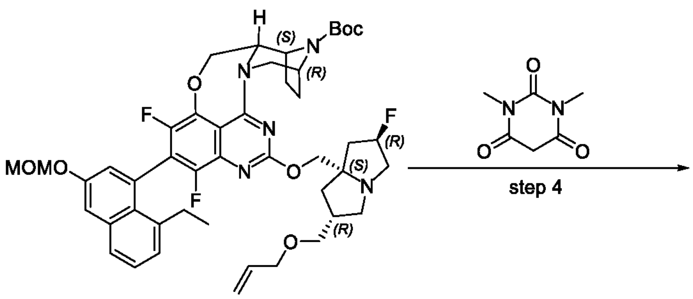


21-3 21-4

Under nitrogen atmosphere at 25°C with stirring, compound **21-3** (80 mg, 0.084 mmol, 1.0 eq), 1,3-dimethylbarbituric acid (41.53 mg, 0.252 mmol, 3.0 eq), tetrakis(triphenylphosphine)palladium (20.49 mg, 0.017 mmol, 0.2 eq), and dichloromethane (1 mL) were sequentially added to the reaction flask. The mixture was reacted at room temperature for 16 hours, with the reaction progress monitored by LC-MS and TLC. After the reaction was completed, the reaction mixture was concentrated to obtain the crude product. The crude product was purified by reverse-phase flash chromatography (C18 column) and eluted with a mobile phase of 5% to 95% methanol/water (0.1% ammonium bicarbonate) over 25 minutes, with detection at UV 254 nm. The product obtained was compound **21-4** (white solid, 45 mg, yield of 63%). MS (ESI, *m/z*): 806.4 $[M + H]^+$.

**Step 5:**

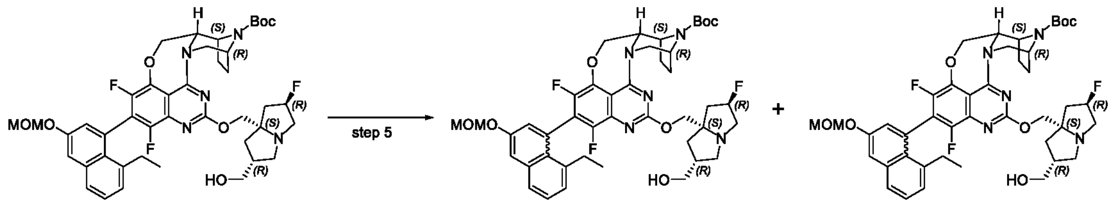


*fast peak* *slow peak*

**21-4** **21-4a** **21-4b**

The compound **21-4** (45 mg) obtained from **step 4** of the present example was subjected to chiral resolution using high-performance liquid chromatography. Chiral column: CHIRALPAK ID, 2 × 25 cm, 5 μm; mobile phase A: *n*-hexane/methyl *tert*-butyl ether (1/1) (0.5%, 2 M ammonia in methanol), mobile phase B: methanol; flow rate: 20 mL/min; elution with 10% mobile phase B over 9 minutes; detector: UV 226/292 nm. Two compounds were obtained. The product with a shorter retention time (5.05 minutes) was compound **21-4a** (white solid, 15 mg, yield of 39%). MS (ESI, m/z): 806.4 $[M + H]^+$. The product with a longer retention time (6.47 minutes) was compound **21-4b** (white solid, 12 mg, yield of 31%). MS (ESI, *m/z):* 806.4 $[M + H]^+$.

**Step 6:**

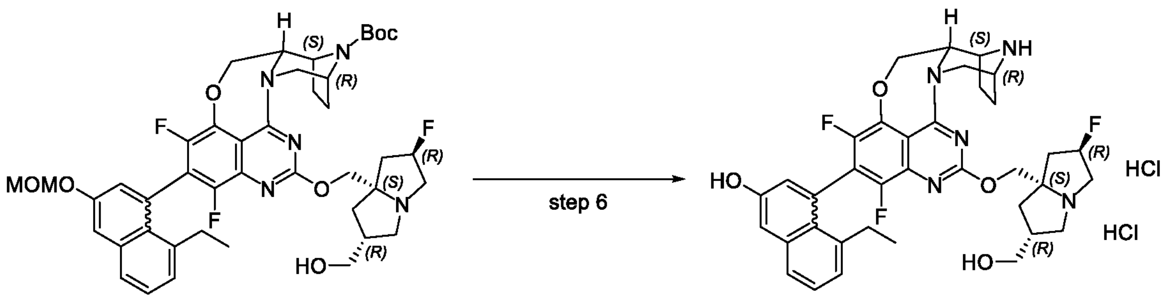


**21-4a** **21a**

With stirring at 0°C, a solution of hydrochloric acid in 1,4-dioxane (4 M, 0.5 mL) was added dropwise to a solution of compound **21-4a** (15 mg, 0.013 mmol, 1.00 eq) in methanol (0.5 mL). The mixture was reacted at room temperature for 1 hour, with the reaction progress monitored by LC-MS. After the reaction was completed, the reaction mixture was concentrated to obtain the crude product. The crude product was purified by reverse-phase flash chromatography (C18 column) and eluted with a mobile phase of 5% to 95% acetonitrile/water (0.1% hydrochloric acid) over 25 minutes, with detection at UV 254 nm. The product obtained was compound **21a** (yellow solid, 9 mg, yield of 80%). MS (ESI, *m/z*): 662.3 $[M + H]^+$, $^1H$ NMR (400 MHz, $CD_3OD$) $\delta$ 7.67 - 7.61 (m, 1H), 7.39 - 7.34 (m, 1H), 7.33 - 7.29 (m, 1H), 7.20 - 7.15 (m, 1H), 7.02 - 6.90 (m, 1H), 5.66 - 5.40 (m, 2H), 4.81 - 4.57 (m, 4H), 4.47 - 4.33 (m, 2H), 4.24 - 4.03 (m, 1H), 3.92 - 3.80 (m, 1H), 3.79 - 3.59 (m, 5H), 3.15 - 3.02 (m, 1H), 2.97 - 2.75 (m, 2H), 2.70 - 2.53 (m, 1H), 2.48 - 2.37 (m, 3H), 2.37 - 2.10 (m, 5H), 1.01 - 0.88 (m, 3H); $^{19}F$ NMR (377 MHz, $CD_3OD$) $\delta$ -134.82, -136.70, -172.47.

**Step** 7:

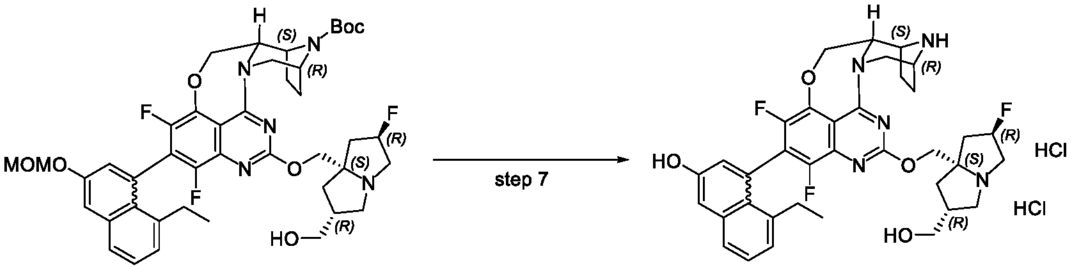


21-4b 21b

With stirring at 0°C, a solution of hydrochloric acid in 1,4-dioxane (4 M, 0.5 mL) was added dropwise to a solution of compound **21-4b** (12 mg, 0.014 mmol, 1.00 eq) in methanol (0.5 mL). The mixture was reacted at room temperature for 1 hour, with the reaction progress monitored by LC-MS. After the reaction was completed, the reaction mixture was concentrated to obtain the crude product. The crude product was purified by reverse-phase flash chromatography (C18 column) and eluted with a mobile phase of 5% to 95% acetonitrile/water (0.1% hydrochloric acid) over 30 minutes, with detection at UV 254 nm. The product obtained was compound **21b** (yellow solid, 8 mg, yield of 73%). MS (ESI, m/z): 662.3 $[M+H]^+$, $^1H$ NMR (400 MHz, $CD_3OD$) $\delta$ 7.67 - 7.62 (m, 1H), 7.40 - 7.35 (m, 1H), 7.31 (d, $J$ = 2.6 Hz, 1H), 7.20 - 7.15 (m, 1H), 6.95 - 6.91 (m, 1H), 5.66 - 5.32 (m, 2H), 4.84 - 4.64 (m, 4H), 4.62 - 4.52 (m, 1H), 4.43 - 4.34 (m, 2H), 4.22 - 4.04 (m, 1H), 3.93 - 3.79 (m, 1H), 3.78 - 3.57 (m, 5H), 3.18 - 3.01 (m, 1H), 2.94 - 2.74 (m, 1H), 2.69 - 2.52 (m, 1H), 2.51 - 2.34 (m, 4H), 2.32 - 2.12 (m, 4H), 1.02 - 0.94 (m, 3H); $^{19}F$ NMR (376 MHz, $CD_3OD$) $\delta$ -134.58, -137.14, -172.48.

**Example 22**

1-((5a*S*,6*S*,9*R*)-1,3-Difluoro-13-(((2*R*,7a*S*)-2-fluorotetrahydro-1*H*-pyrrolizin-7a(5*H*)-yl)methoxy)-5a,6,7,8,9,10-hexahydro-5*H*-6,9-epiminoazepino[2',1':3,4][1,4]oxazepino[5,6,7-*de*]quinazolin-2-yl)isoquinolin-3-amine **22**

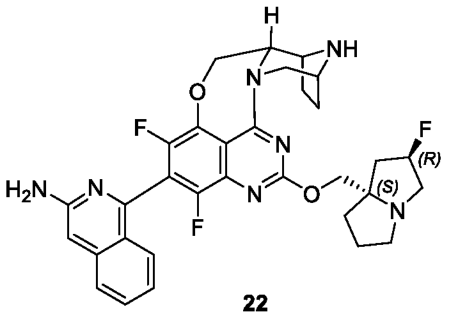

**22**

The synthetic route is as follows:

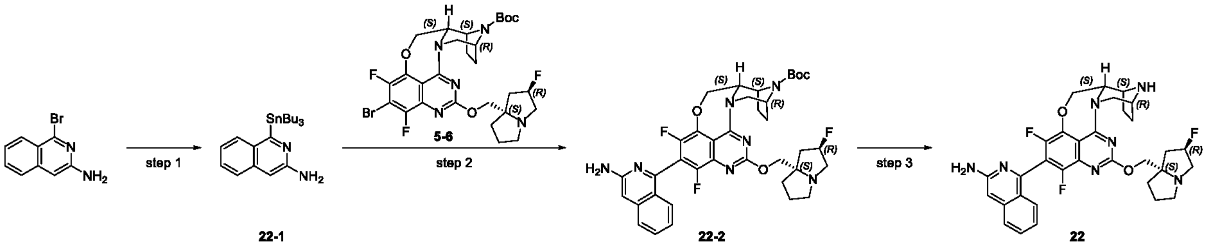


22-1 22-2 22

**Step 1:**

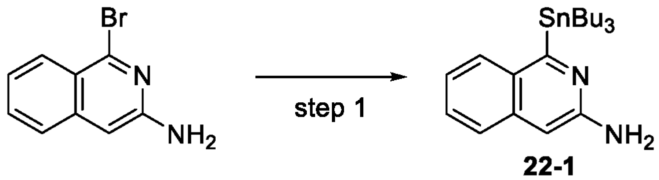


22-1

Under nitrogen atmosphere at 25°C with stirring, 1-bromoisoquinolin-3-amine (901 mg, 4.04 mmol, 1.0 eq), hexabutylditin (5.6 g, 9.70 mmol, 2.4 eq), tris(dibenzylideneacetone)dipalladium (370 mg, 0.404 mmol, 0.1 eq), tricyclohexylphosphine (339 mg, 1.21 mmol, 0.3 eq), lithium chloride (1.02 g, 24.2 mmol, 6.0 eq), and 1,4-dioxane (20 mL) were sequentially added to a reaction flask. The resulting mixture was reacted with stirring at 100°C under nitrogen atmosphere for 20 hours. The reaction progress was monitored by LC-MS and TLC. After the reaction was completed, the reaction mixture was cooled to 25°C. With stirring at 25°C, the reaction mixture was poured into an aqueous solution of potassium fluoride (100 mL) to quench the reaction. The resulting mixture was extracted with ethyl acetate (100 mL × 3). The organic phases were combined, dried over anhydrous sodium sulfate, and filtered to remove the desiccant. The filtrate was subjected to rotary evaporation under reduced pressure to remove the solvent, yielding the crude product. The crude product was purified by silica gel column chromatography and subjected to a gradient elution with a mobile phase of 0% to 100% ethyl acetate/petroleum ether. The collected fraction was subjected to rotary evaporation under reduced pressure to remove the solvent, yielding compound **22-1** (yellow oil, 780 mg, yield of 44%). MS (ESI, m/z): 431.3/433.3/435.2 $[M + H]^+$; $^1H$ NMR (400 MHz, $CDCl_3$) $\delta$ 7.73 (d, *J* = 8.4 Hz, 1H), 7.54 - 7.41 (m, 2H), 7.24 (tt, *J* = 14.1, 12.8, 4.6 Hz, 2H), 6.60 (s, 1H), 1.63 - 1.54 (m, 12H), 1.39 - 1.31 (m, 6H), 0.88 (t, *J* = 7.3 Hz, 9H).

**Step 2:**

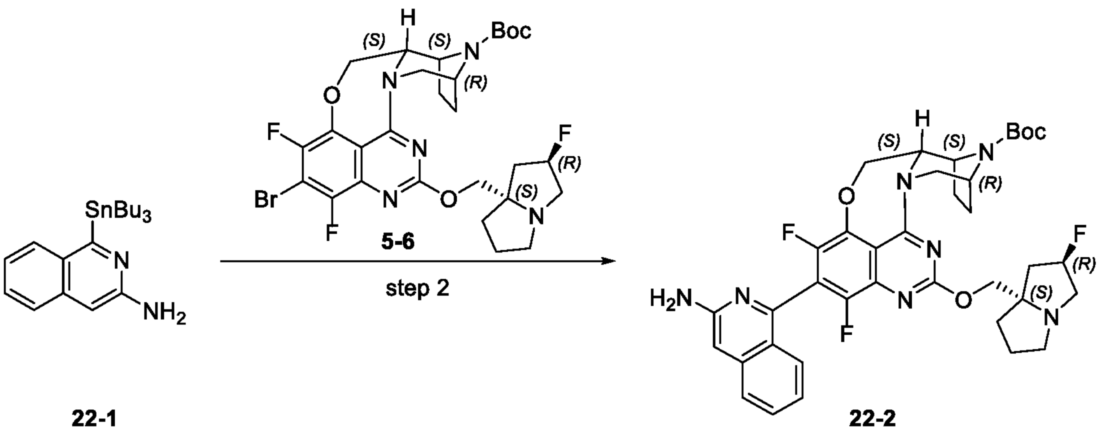


22-1

22-2

Under nitrogen atmosphere at 25°C with stirring, compound **22-1** (81.2 mg, 0.18 mmol, 1.5 eq), cuprous iodide (11.9 mg, 0.059 mmol, 0.5 eq), tetrakis(triphenylphosphine)palladium (72.2 mg, 0.059 mmol, 0.5 eq), and a solution of lithium chloride in tetrahydrofuran (0.5 M, 0.59 mL, 0.28 mmol, 2.5 eq) were added sequentially to a solution of compound **5-6** (80 mg, 0.12 mmol, 1 eq) in *N,N*-dimethylformamide (1 mL). The resulting mixture was reacted with stirring at 100°C under nitrogen atmosphere for 16 hours. The reaction progress was monitored by LC-MS and TLC. After the reaction was completed, the reaction mixture was cooled to 25°C and filtered to remove the insoluble material. The filter cake was washed with methanol, and the filtrate was purified by reverse-phase chromatography (C18 column) and eluted with a mobile phase of 5% to 95% acetonitrile/water (0.1% ammonium bicarbonate solution) over 25 minutes; detector: UV 254/220 nm. The product obtained was compound **22-2** (yellow solid, 83 mg, yield of 94%). MS (ESI, m/z): 704.7 $[M + H]^+$; $^1H$ NMR (400 MHz, $CDCl_3$) $\delta$ 7.71 - 7.57 (m, 1H), 7.57 - 7.40 (m, 2H), 7.21 - 7.07 (m, 1H), 6.91 - 6.82 (m, 1H), 5.40 - 5.19 (m, 1H), 5.14 - 4.77 (m, 1H), 4.65 - 3.93 (m, 10H), 3.42 - 3.13 (m, 4H), 3.07 - 2.89 (m, 1H), 2.40 - 1.61 (m, 14H).

**Step 3:**

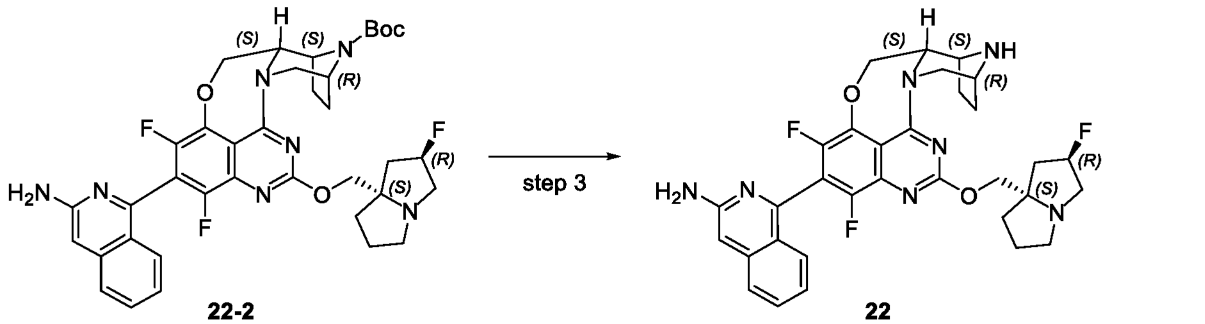


**22-2** **22**

With stirring at 25°C, compound **22-2** (83 mg, 0.11 mmol, 1 eq) was dissolved in methanol (1 mL). The mixture was then cooled to 0°C, and with stirring at 0°C, a solution of hydrogen chloride in 1,4-dioxane (4 M, 1 mL) was added dropwise to the mixture. The resulting mixture was reacted with stirring at 25°C for 1 hour, with the reaction progress monitored by LC-MS and TLC. After the reaction was completed, the reaction mixture was subjected to rotary evaporation under reduced pressure to remove the solvent, yielding the crude product. The crude product was purified by high-performance liquid chromatography: XBridge Shield RP18 OBD Column,30 × 150 mm, 5 μm; mobile phase A: water (10 mmol/L ammonium bicarbonate + 0.1% ammonia water), mobile phase B: methanol; flow rate: 60 mL/min; elution with a gradient of 48% to 67% mobile phase B over 7 minutes; detector: UV 220/254 nm. The product obtained was compound **22** (yellow solid, 35 mg, 46%). MS (ESI, m/z): 604.35 $[M + H]^+$; $^1$H NMR (400 MHz, DMSO-$d_6$) $\delta$ 7.67 - 7.61 (m, 1H), 7.52 - 7.45 (m, 1H), 7.38 - 7.29 (m, 1H), 7.15 - 7.06 (m, 1H), 6.85 - 6.71 (m, 1H), 6.22 - 6.06 (m, 2H), 5.37 - 5.18 (m, 1H), 4.92 - 4.78 (m, 1H), 4.66 - 4.55 (m, 1H), 4.47 - 4.30 (m, 1H), 4.14 - 3.89 (m, 3H), 3.64 - 3.53 (m, 1H), 3.52 - 3.44 (m, 1H), 3.15 - 3.04 (m, 3H), 3.04 - 2.97 (m, 1H), 2.87 - 2.78 (m, 1H), 2.78 - 2.69 (m, 1H), 2.19 - 2.09 (m, 1H), 2.09 - 1.93 (m, 2H), 1.91 - 1.64 (m, 6H), 1.62 - 1.47 (m, 1H); $^{19}$F NMR (376 MHz, DMSO) $\delta$ -134.12, -134.26, -142.53, -142.60, -172.11, -172.14.

**Example 23**

4-((2*R* or 2*S*, 5aS,6S,9R)-1,3-Difluoro-13-(((2*R*,6*S*,7*aS*)-2-fluoro-6-(hydroxymethyl)tetrahydro-1*H*-pyrrolizin-7a(5*H*)-yl) methoxy)-5a,6,7,8,9,10-hexahydro-5*H*-6,9-epiminoazepino[2',1':3,4][1,4]oxazepino[5,6,7-*de*]quinazolin-2-yl)-5-ethylnaphthalen-2-ol dihydrochloride **23a**; 4-((2*S* or 2*R*, 5*aS*,6*S*,9*R*)-1,3-difluoro-13-(((2*R*,6*S*,7*aS*)-2-fluoro-6-(hydroxymethyl)tetrahydro-1*H*-pyrrolizin-7*a*(5*H*)-yl)methoxy)-5*a*,6,7,8,9,10-hexahydro-5*H*-6,9-epiminoazepino[2',1':3,4][1,4]oxazepino[5,6,7-*de*]quinazolin-2-yl)-5-ethylnaphthalen-2-ol dihydrochloride 23b

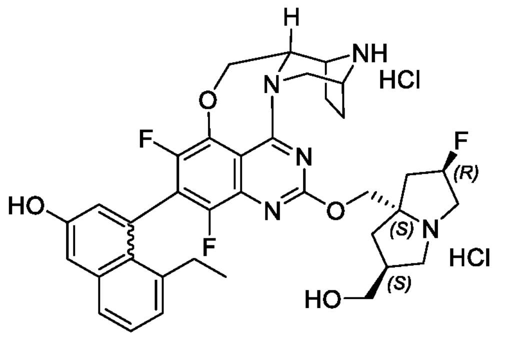

**23a**

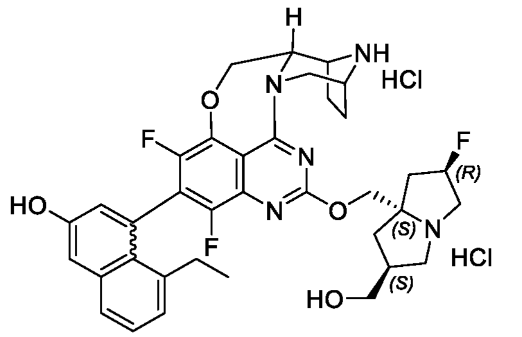

**23b**

Synthetic route

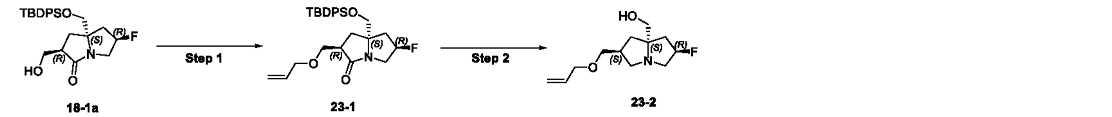


18-1a 23-1 23-2

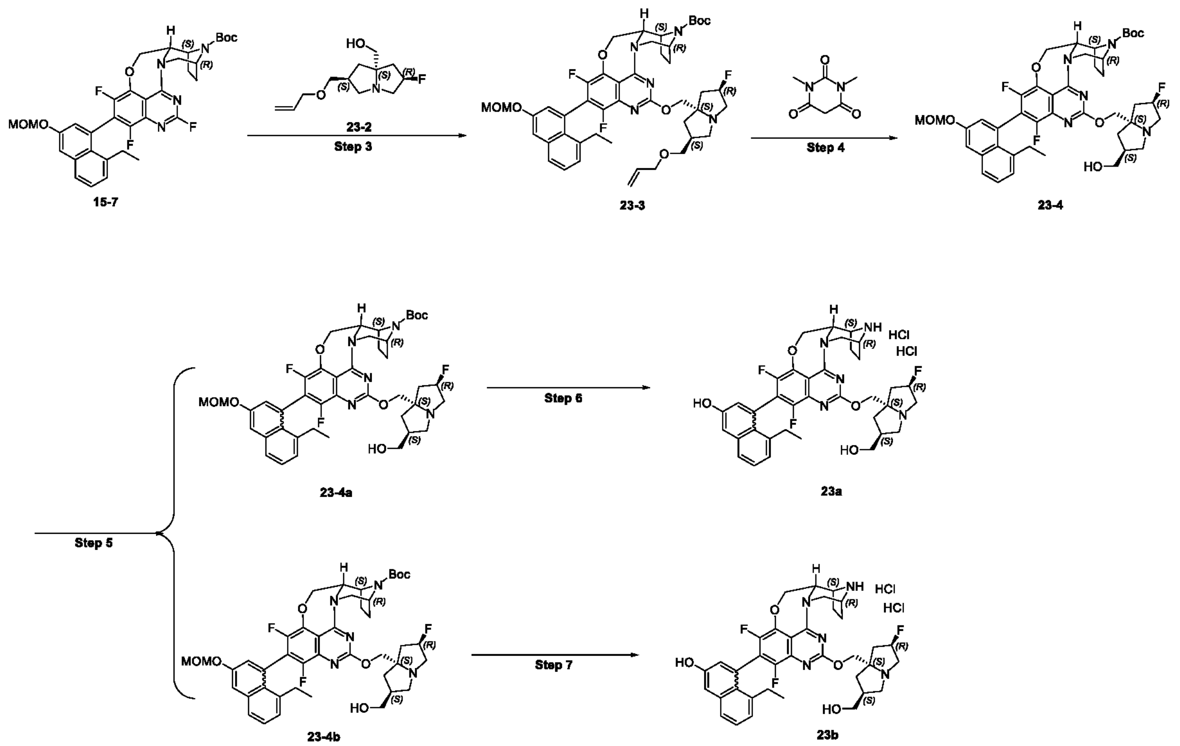


15-7 23-2 23-3 23-4 23-4a 23a 23-4b 23b

**Step 1:**

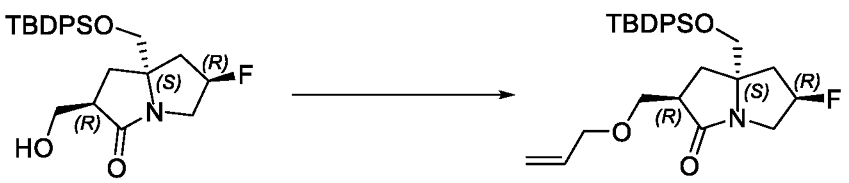

18-1a 23-1

Under nitrogen atmosphere at 0°C with stirring, compound **18-1a** (2.8 g, 6.02 mmol, 1.0 eq), allyl bromide (0.92 g, 7.22 mmol, 1.2 eq), and *N,N*-dimethylformamide (40 mL) were sequentially added to a 250 mL three-necked flask. Sodium hydride (60% dispersed in mineral oil, 0.28 g, 7.22 mmol, 1.2 eq) was then added to the reaction system in batches. The resulting mixture was reacted with stirring at 0°C under nitrogen atmosphere for 1 hour. The reaction progress was monitored by LC-MS and TLC. After the reaction was completed, the reaction mixture was poured into saturated ammonium chloride solution (500 mL) to quench the reaction. The mixture was extracted with ethyl acetate (500 mL $\times$ 3). The organic phases were combined, then dried over anhydrous sodium sulfate, and filtered to remove the desiccant. The filtrate was concentrated under reduced pressure to remove the solvent, yielding the crude product. The crude product was purified by silica gel column chromatography and subjected to a gradient elution with a mobile phase of 0% to 40% methyl tert-butyl ether/petroleum ether. The collected fraction was concentrated under reduced pressure to remove the solvent, yielding compound **23-1** (yellow oil, 1.57 g, yield of 51%). MS (ESI, m/z): 482.2 $[M + H]^+$; $^1H$ NMR (300 MHz, $CDCl_3$) $\delta$ 7.73 - 7.57 (m, 4H), 7.55 - 7.35 (m, 6H), 6.02 - 5.74 (m, 1H), 5.47 - 5.08 (m, 3H), 4.24 - 4.09 (m, 1H), 4.06 - 3.95 (m, 2H), 3.86 - 3.75 (m, 1H), 3.64 - 3.40 (m, 3H), 3.25 - 3.00 (m, 2H), 2.43 - 2.20 (m, 2H), 2.12 - 1.90 (m, 2H), 1.07 (s, 9H).

**Step 2:**

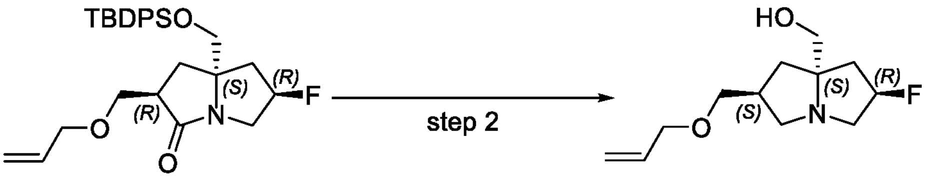


**23-1** **23-2**

Under nitrogen atmosphere at 0°C with stirring, a solution of lithium aluminum hydride in tetrahydrofuran (1 M, 3.55 mL, 1.5 eq) was added dropwise to a solution of compound **23-1** (1.2 g, 2.367 mmol, 1.0 eq) in tetrahydrofuran (20 mL). The mixture was reacted with stirring at 60°C for 2 hours, with the reaction progress monitored by LC-MS and TLC. After the reaction was completed, the reaction mixture was cooled to 0°C, and water (1 mL), 20% sodium hydroxide (1 mL), and water (3 mL) were sequentially added dropwise. The resulting mixture was filtered, and the filtrate was concentrated under reduced pressure to obtain a crude product. The resulting crude product was purified by silica gel column chromatography and subjected to a gradient elution with a mobile phase of 0% to 10% ammonia in methanol/dichloromethane. The collected fraction was concentrated under reduced pressure to remove the solvent, yielding compound **23-2** (colorless oil, 400 mg, yield of 70%). MS (ESI, *m/z*): 230.2 $[M + H]^+$; $^1H$ NMR (400 MHz, $CDCl_3$) $\delta$ 5.95 - 5.83 (m, 1H), 5.32 - 5.14 (m, 3H), 4.00 - 3.92 (m, 2H), 3.46 - 3.38 (m, 3H), 3.34 (d, *J* = 10.4 Hz, 1H), 3.27 (d, *J* = 10.4 Hz, 1H), 3.25 - 3.14 (m, 1H), 3.09 - 2.93 (m, 1H), 2.78 - 2.72 (m, 1H), 2.63 - 2.46 (m, 1H), 2.13 - 2.08 (m, 1H), 2.08 - 2.00 (m, 1H), 1.99 - 1.90 (m, 1H), 1.79 - 1.65 (m, 1H).

**Step 3:**

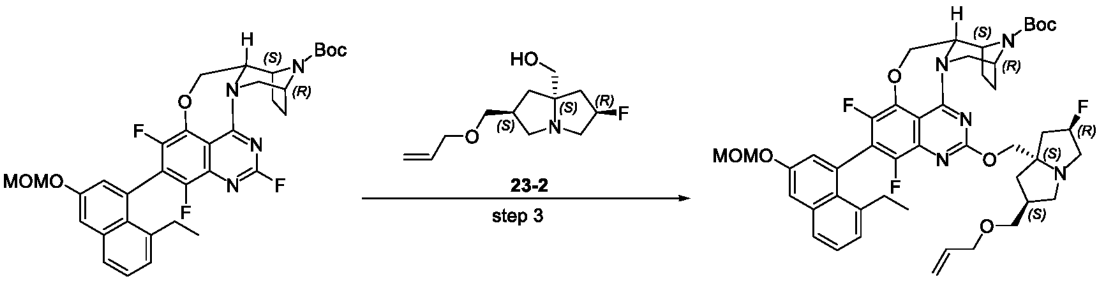


**15-7** **23-3**

Under nitrogen atmosphere at 25°C with stirring, triethylenediamine (7.05 mg, 0.060 mmol, 0.2 eq) and cesium carbonate (204.70 mg, 0.596 mmol, 2 eq) were sequentially added to a solution of compound **15-7** (200 mg, 0.298 mmol, 1.0 eq) and compound **23-2** (42.80 mg, 0.215 mmol, 1.2 eq) in *N,N*-dimethylformamide (4 mL). The resulting mixture was reacted at 80°C for 2 hours, with the reaction progress monitored by LC-MS. After the reaction was completed, the system was cooled to room temperature, and water (20 mL) was added to dilute the reaction mixture. The mixture was then extracted with ethyl acetate (20 mL × 3). The organic phases were combined, washed with saturated brine (30 mL), then dried over anhydrous sodium sulfate, and filtered to remove the desiccant. The filtrate was concentrated under reduced pressure to obtain a crude product. The resulting crude product was purified by silica gel column chromatography and subjected to a gradient elution with a mobile phase of 0% to 7% methanol/dichloromethane. The collected fraction was concentrated under reduced pressure to remove the solvent, yielding compound **23-2** (white solid, 155 mg, yield of 59%). MS (ESI, m/z): 846.4 $[M + H]^+$.

**Step 4:**

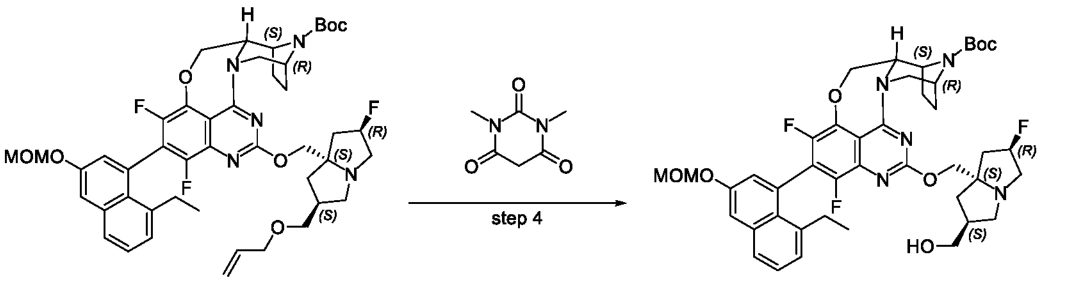


23-3 23-4

Under nitrogen atmosphere at 25°C with stirring, compound **23-3** (150 mg, 0.168 mmol, 1.0 eq), 1,3-dimethylbarbituric acid (83.06 mg, 0.504 mmol, 3.0 eq), tetrakis(triphenylphosphine)palladium (40.98 mg, 0.034 mmol, 0.2 eq), and dichloromethane (3 mL) were sequentially added to a reaction flask. The mixture was reacted at room temperature for 16 hours, with the reaction progress monitored by LC-MS and TLC. After the reaction was completed, the reaction mixture was concentrated to obtain the crude product. The crude product was purified by reverse-phase flash chromatography (C18 column) and eluted with a mobile phase of 5% to 95% methanol/water (0.1% ammonium bicarbonate) over 25 minutes, with detection at UV 254 nm. The product obtained was compound **23-4** (white solid, 125 mg, yield of 88%). MS (ESI, m/z): 806.4 $[M + H]^+$.

**Step 5:**

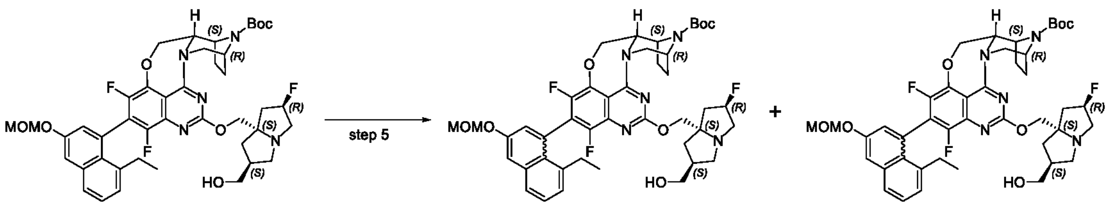


23-4 *fast peak* 23-4a *slow peak* 23-4b

The compound **23-4** (125 mg) obtained from **step 4** of the present example was subjected to chiral resolution using high-performance liquid chromatography. Chiral column: CHIRALPAK ID, 2 × 25 cm, 5 μm; mobile phase A: *n*-hexane/methyl *tert*-butyl ether (1/1) (0.5%, 2 M ammonia in methanol), mobile phase B: methanol; flow rate: 20 mL/min; elution with 10% mobile phase B over 12.7 minutes; detector: UV 226/292 nm. Two compounds were obtained. The product with a shorter retention time (8.40 minutes) was compound **23-4a** (white solid, 44 mg, yield of 34.8%), MS (ESI, m/z): 806.4 $[M + H]^+$. The product with a longer retention time (6.47 minutes) was compound **23-4b** (white solid, 43 mg, yield of 34%), MS (ESI, m/z): 806.4 $[M + H]^+$.

**Step 6:**

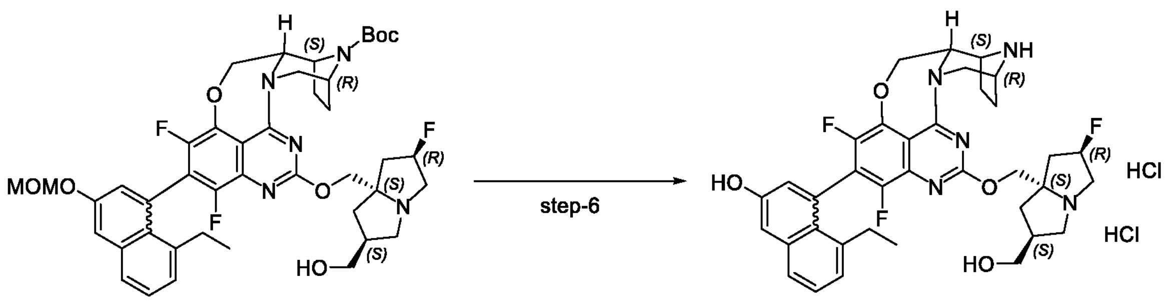


23-4a 23a

With stirring at 0°C, a solution of hydrochloric acid in 1,4-dioxane (4 M, 1.5 mL) was added dropwise to a solution

of compound **23-4a** (44 mg, 0.052 mmol, 1.0 eq) in methanol (1.5 mL). The mixture was reacted at room temperature for 1 hour, with the reaction progress monitored by LC-MS. After the reaction was completed, the reaction mixture was concentrated to obtain the crude product. The crude product was purified by high-performance liquid chromatography: Sunfire prep C18 column, 30 × 150 mm, 5 μm; mobile phase A: water (0.05% hydrochloric acid), mobile phase B: methanol; flow rate: 60 mL/min; elution with 26% to 41% mobile phase B over 9 minutes; detector: UV 220/254 nm. The product obtained was compound **23a** (yellow solid, 18.4 mg, yield of 47%). MS (ESI, m/z): 662.3 $[M + H]^+$; $^1H$ NMR (400 MHz, DMSO-$d_6$+$D_2O$) $\delta$ 7.73 - 7.66 (m, 1H), 7.43 - 7.36 (m, 1H), 7.33 (d, $J$ = 2.8 Hz, 1H), 7.18 - 7.12 (m, 1H), 6.97 (d, $J$ = 2.8 Hz, 1H), 5.69 - 5.51 (m, 1H), 5.09 - 5.01 (m, 1H), 4.76 - 4.69 (m, 1H), 4.65 - 4.53 (m, 4H), 4.33 - 4.24 (m, 2H), 3.96 - 3.80 (m, 2H), 3.79 - 3.59 (m, 2H), 3.55 - 3.49 (m, 1H), 3.43 - 3.41 (m, 1H), 3.12 - 3.02 (m, 1H), 2.70 - 2.55 (m, 2H), 2.48 - 2.42 (m, 1H), 2.41 - 2.32 (m, 3H), 2.17 - 1.90 (m, 5H), 0.90 - 0.78 (m, 3H); $^{19}F$ NMR (377 MHz, DMSO-$d_6$ +$D_2O$) $\delta$ -132.25, -139.61, -172.75.

**Step 7:**

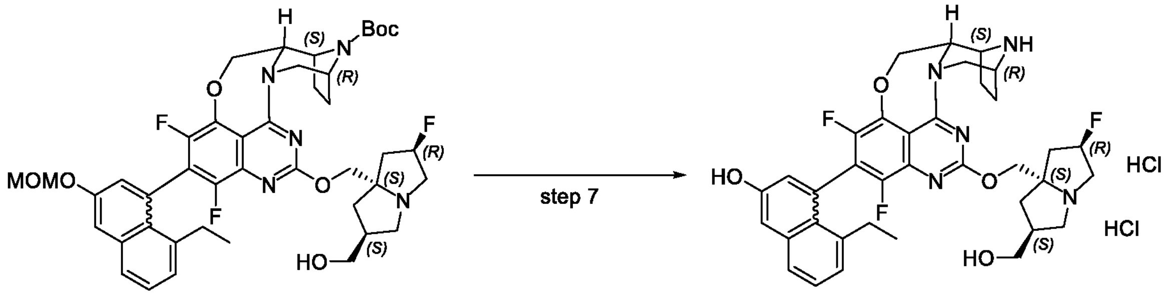


**23-4b** **23b**

With stirring at 0°C, a solution of hydrochloric acid in 1,4-dioxane (4 M, 1.5 mL) was added dropwise to a solution of compound **23-4b** (43 mg, 0.051 mmol, 1.0 eq) in methanol (1.5 mL). The mixture was reacted at room temperature for 1 hour, with the reaction progress monitored by LC-MS. After the reaction was completed, the reaction mixture was concentrated to obtain the crude product. The crude product was purified by reverse-phase flash chromatography (C18 column) and eluted with a mobile phase of 5% to 95% acetonitrile/water (0.1% hydrochloric acid) over 30 minutes, with detection at UV 254 nm. The product obtained was compound **23b** (yellow solid, 29.2 mg, yield of 76%). MS (ESI, m/z): 662.3 $[M + H]^+$, $^1H$ NMR (400 MHz, DMSO-$d_6$) $\delta$ 11.51 (s, 1H), 10.56 - 9.73 (m, 3H), 7.71 - 7.65 (m, 1H), 7.42 - 7.35 (m, 1H), 7.32 (d, $J$ = 2.6 Hz, 1H), 7.19 - 7.11 (m, 1H), 6.97 (d, $J$ = 2.6 Hz, 1H), 5.68 - 5.49 (m, 1H), 5.02 (d, $J$ = 14.0 Hz, 1H), 4.80 - 4.72 (m, 1H), 4.66 - 4.55 (m, 4H), 4.41 - 4.25 (m, 4H), 3.94 - 3.80 (m, 2H), 3.73 - 3.61 (m, 2H), 3.57 - 3.48 (m, 1H), 3.47 - 3.39 (m, 1H), 3.13 - 3.00 (m, 1H), 2.66 - 2.53 (m, 2H), 2.47 - 2.34 (m, 3H), 2.20 - 2.11 (m, 1H), 2.09 - 1.86 (m, 4H), 0.94 - 0.84 (m, 3H); $^{19}F$ NMR (376 MHz, DMSO-$d_6$) $\delta$ -132.33, -139.33, -172.66.

**Example 24**

3-((5a*S*,6*S*,9*R*)-1,3-Difluoro-13-(((2*R*,7a*S*)-2-fluorotetrahydro-1*H*-pyrrolizin-7a(5*H*)-yl)methoxy)-5*a*,6,7,8,9,10-hexahydro-5*H*-6,9-epiminoazepino[2',1':3,4][1,4]oxazepino[5,6,7-*de*]quinazolin-2-yl)-4-(1H-pyrazol-3-yl)phenol dihydrochloride **24**

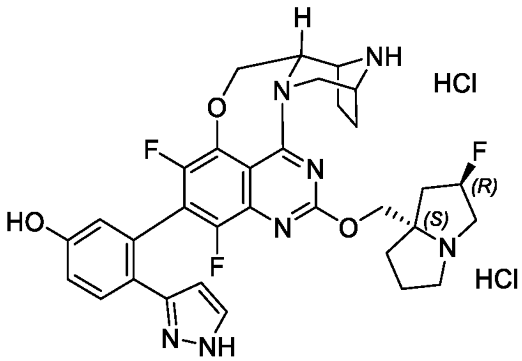

**24**

The synthetic route is as follows:

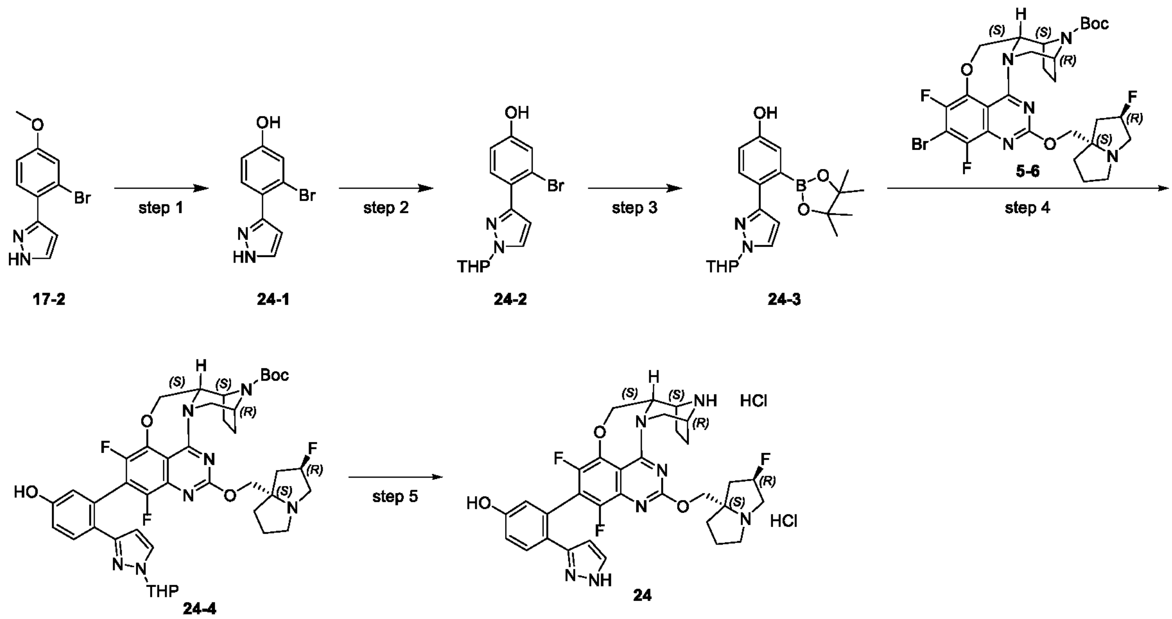


**Step 1:**

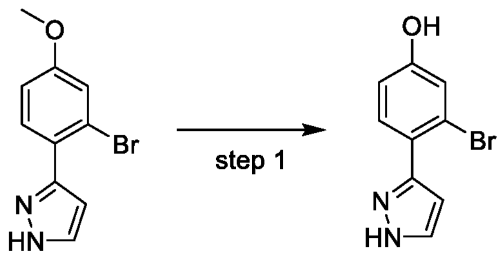


**17-2** **24-1**

With stirring at 25°C, (2*S*)-2-amino-4-methylthio-butanoic acid (1.2 g, 8.0 mmol, 2 eq), compound **17-2** (1 g, 4 mmol, 1 eq), and methanesulfonic acid (20 mL) were sequentially added to a reaction flask. The resulting mixture was reacted with stirring at 50°C for 48 hours. The reaction progress was monitored by LC-MS and TLC. After the reaction was completed, the reaction mixture was cooled to 0°C. Subsequently, the reaction was quenched by pouring the reaction mixture into ice water (100 mL) at 0°C with stirring. The pH of the mixture was adjusted to 7 using 5 M sodium hydroxide aqueous solution with stirring at 0°C. The mixture was then extracted with ethyl acetate (200 mL × 3), then dried over

anhydrous sodium sulfate, and filtered to remove the desiccant. The filtrate was subjected to rotary evaporation under reduced pressure to obtain a crude product. The crude product was purified by silica gel column chromatography and subjected to a gradient elution with a mobile phase of 0% to 50% ethyl acetate/petroleum ether. The collected fraction was subjected to rotary evaporation under reduced pressure to remove the solvent, yielding compound **24-1** (white solid, 750 mg, yield of 79%). MS (ESI, m/z): 238.9/240.9 $[M + H]^+$; $^1H$ NMR (400 MHz, DMSO-$d_6$) $\delta$ 9.99 (s, 1H), 7.68 (s, 1H), 7.44 (d, *J* = 8.5 Hz, 1H), 7.10 (d, *J* = 2.5 Hz, 1H), 6.85 (dd, *J* = 8.5, 2.5 Hz, 1H), 6.54 (d, *J* = 2.2 Hz, 1H).

**Step 2:**

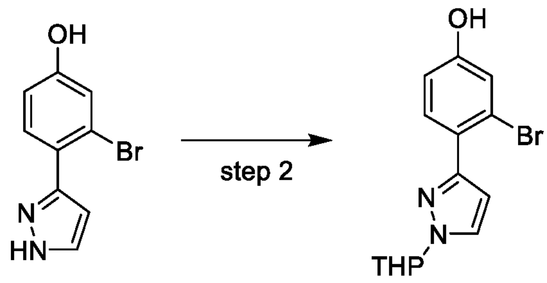


**24-1** **24-2**

With stirring at 25°C, compound **24-1** (750 mg, 3.14 mmol, 1 eq) and 3,4-dihydro-2*H*-pyran (300 mg, 3.57 mmol, 1.13 eq) were dissolved in tetrahydrofuran (20 mL). With stirring at 25°C, *p*-toluenesulfonic acid (60 mg, 0.349 mmol, 0.1 eq) was added to the mixture. The resulting mixture was reacted with stirring at 70°C for 16 hours. The reaction progress was monitored by LC-MS and TLC. After the reaction was completed, the reaction mixture was cooled to 25°C. The reaction was quenched by pouring the reaction mixture into saturated sodium bicarbonate solution (50 mL) at 25°C with stirring. The mixture was extracted with ethyl acetate (50 mL × 3). The organic phases were combined and dried over anhydrous sodium sulfate. The filtrate was subjected to rotary evaporation under reduced pressure to remove the solvent, yielding the crude product. The crude product was purified by silica gel column chromatography and subjected to a gradient elution with a mobile phase of 0% to 50% ethyl acetate/petroleum ether. The collected fraction was subjected to rotary evaporation under reduced pressure to remove the solvent, yielding compound **24-2** (colorless oil, 960 mg, yield of 95%). MS (ESI, m/z): 323.0/325.0 $[M + H]^+$; $^1H$ NMR ($CDCl_3$) $\delta$ 7.67 (d, *J* = 2.5 Hz, 1H), 7.50 (d, *J* = 8.4 Hz, 1H), 7.12 (d, *J* = 2.5 Hz, 1H), 6.78 - 6.70 (m, 2H), 5.53 - 5.44 (m, 1H), 4.12 (q, *J* = 4.2, 3.6 Hz, 1H), 3.74 (td, *J* = 11.2, 2.9 Hz, 1H), 2.15 (ddd, *J* = 9.8, 6.1, 3.8 Hz, 2H), 1.81 - 1.52 (m, 4H).

**Step 3:**

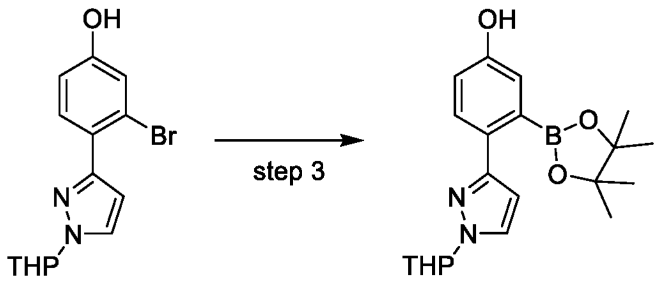


**24-2** **24-3**

Under nitrogen atmosphere at 25°C with stirring, compound **24-2** (950 mg, 2.94 mmol, 1 eq), bis(pinacolato) diboron (1 g, 3.94 mmol, 1.34 eq), 1,1'-bis(diphenylphosphino)ferrocene palladium(II) chloride (220 mg, 0.301 mmol, 0.1 eq), and 1,4-dioxane (10 mL) were added sequentially to a reaction flask. The mixture was reacted with stirring under nitrogen atmosphere at 100°C for 4 hours. The reaction progress was monitored by LC-MS and TLC. After the reaction was completed, the reaction mixture was cooled to 25°C. The resulting mixture was filtered through diatomite, and the filter cake was washed with ethyl acetate (100 mL). The filtrate was subjected to rotary evaporation under reduced pressure to

remove the solvent, yielding the crude product. The crude product was purified by silica gel column chromatography and subjected to a gradient elution with a mobile phase of 0% to 69% ethyl acetate/petroleum ether. The collected fraction was subjected to rotary evaporation under reduced pressure to remove the solvent, yielding compound **24-3** (brown solid, 460 mg, yield of 54%). MS (ESI, m/z): 371.2 [M + H]+.

**Step 4:**

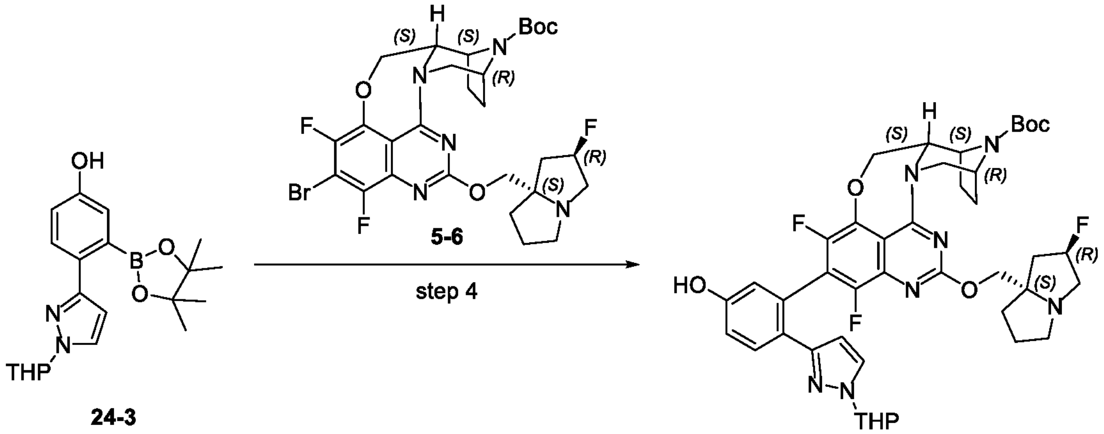


24-3

24-4

Under nitrogen atmosphere at 25°C with stirring, compound **5-6** (50.0 mg, 0.074 mmol, 1.0 eq), compound **24-3** (115.6 mg, 0.30 mmol, 4.0 eq), potassium phosphate (33.14 mg, 0.148 mmol, 2 eq), 3-(*tert*-butyl)-4-(2,6-dimethoxyphenyl)-2,3-dihydrobenzo[D][1,3]oxaphosphole (5.16 mg, 0.015 mmol, 0.2 eq), tris(dibenzylideneacetone)dipalladium (7.2 mg, 0.007 mmol, 0.1 eq), toluene (1 mL), and water (0.2 mL) were sequentially added to a reaction flask. The mixture was reacted with stirring under nitrogen atmosphere at 80°C for 2 hours. The reaction progress was monitored by LC-MS and TLC. After the reaction was completed, the reaction mixture was cooled to 25°C. With stirring at 25°C, the reaction mixture was diluted by adding water (5 mL). The resulting mixture was extracted with ethyl acetate (5 mL) and dichloromethane (5 mL × 2). The organic phases were combined, then dried over anhydrous sodium sulfate, and filtered to remove the desiccant. The filtrate was subjected to rotary evaporation under reduced pressure to remove the solvent, yielding the crude product. The resulting crude product was purified by silica gel column chromatography and subjected to a gradient elution with a mobile phase of 0% to 15% dichloromethane/a solution of ammonia in methanol (7 M). The collected fraction was subjected to rotary evaporation under reduced pressure to remove the solvent, yielding compound **24-4** (white solid, 50 mg, yield of 87%). MS (ESI, m/z): 804.4 [M+H]+.

**Step 5:**

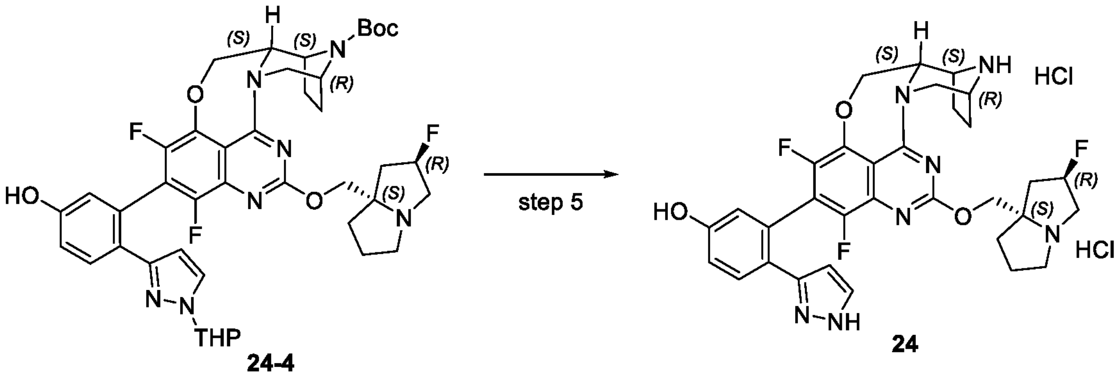


24-4

24

With stirring at 25°C, compound **24-4** (50 mg, 0.059 mmol, 1 eq) was dissolved in dichloromethane (1 mL). The reaction mixture was then cooled to 0°C, and trifluoroacetic acid (0.3 mL) and riethylsilyl hydride (36.2 mg, 0.30 mmol, 5 eq)

were added dropwise to the reaction mixture. The mixture was reacted with stirring at 25°C for 1 hour, with the reaction progress monitored by LC-MS and TLC. After the reaction was completed, the reaction mixture was subjected to rotary evaporation under reduced pressure to remove the solvent, yielding the crude product. The crude product was purified by high-performance liquid chromatography: Xselect CSH C18 OBD Column, 30 × 150 mm, 5 μm; mobile phase A: water (0.05% hydrochloric acid), mobile phase B: methanol; flow rate: 60 mL/min; elution with a gradient of 12% to 19% mobile phase B over 7 minutes; detector: UV 220/254 nm. The product obtained was compound **24** (yellow solid, 22.5 mg, yield of 60%). MS (ESI, m/z): 620.3 $[M+H]^+$; $^1H$ NMR (400 MHz, $CD_3OD$) $\delta$ 8.05 - 7.79 (m, 1H), 7.71 - 7.57 (m, 1H), 7.19 - 7.08 (m, 1H), 7.07 - 6.90 (m, 1H), 6.33 - 6.09 (m, 1H), 5.70 - 5.49 (m, 1H), 5.49 - 5.30 (m, 1H), 4.84 - 4.69 (m, 3H), 4.69 - 4.51 (m, 2H), 4.50 - 4.28 (m, 2H), 4.11 - 3.82 (m, 3H), 3.81 - 3.60 (m, 1H), 3.54 - 3.40 (m, 1H), 2.84 - 2.55 (m, 2H), 2.55 - 2.41 (m, 1H), 2.41 - 2.04 (m, 7H); $^{19}F$ NMR (400 MHz, $CD_3OD$) $\delta$ 135.24, 135.48, 135.58, 135.77, 137.54, 138.15, 174.19.

**Example 25**

4-((2*R* or 2*S*)-13-(((2*R*,6*R*, 7*aS*)-2-(((2*R*,6*S*)-2,6-Dimethylmorpholino)methyl)-6-fluorotetrahydro-1*H*-pyrrolizin-7*a*(5*H*-yl)methoxy)-1,3-difluoro-5*a*,6,7,8,9,10-hexahydro-5*H*-6,9-epiminoazepino[2',1':3,4][1,4]oxazepino[5,6,7-*de*]quinazolin-2-yl)-5-ethylnaphthalen-2-ol trihydrochloride **25a;** 4-((2S or 2*R*)-13-(((2*R*,6*R*,7*aS*)-2-(((2*R*,6*S*)-2,6-dimethylmorpholino)methyl)-6-fluorotetrahydro-1*H*-pyrrolizin-7*a*(5*H*)-yl)methoxy)-1,3-difluoro-5a,6,7,8,9,10-hexahydro-5*H*-6,9-epiminoazepino[2',1':3,4][1,4]oxazepino[5,6,7-*de*]quinazolin-2-yl)-5-ethylnaphthalen-2-ol trihydrochloride **25b**

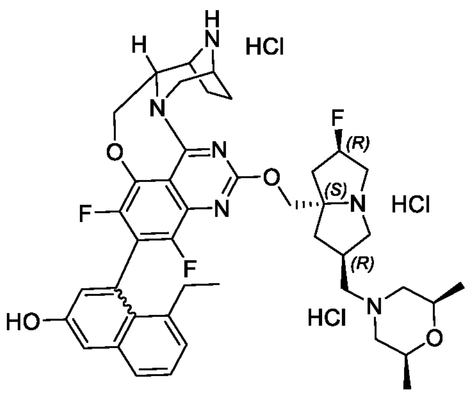

**25a**

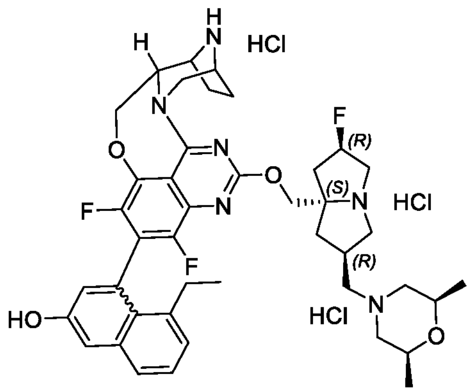

**25b**

The synthetic route is as follows:

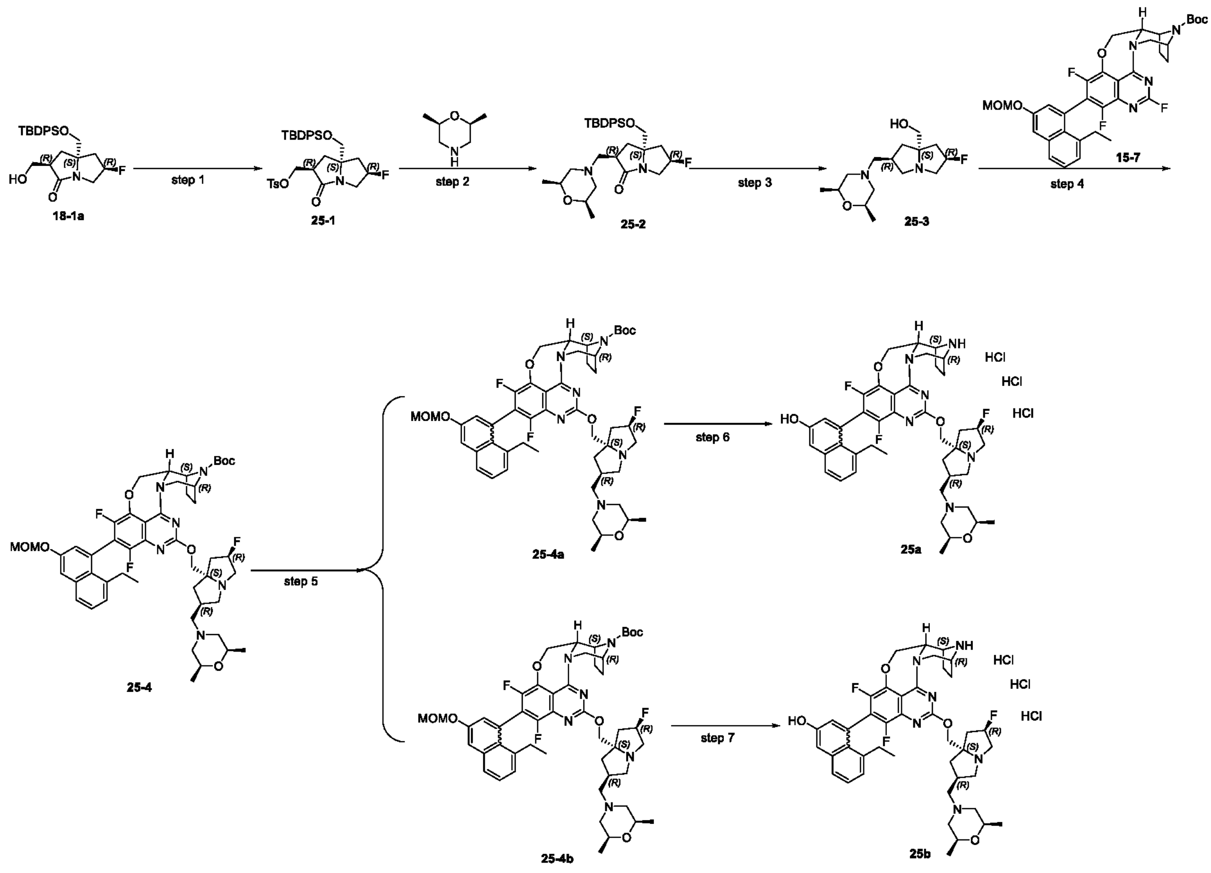


**Step 1:**

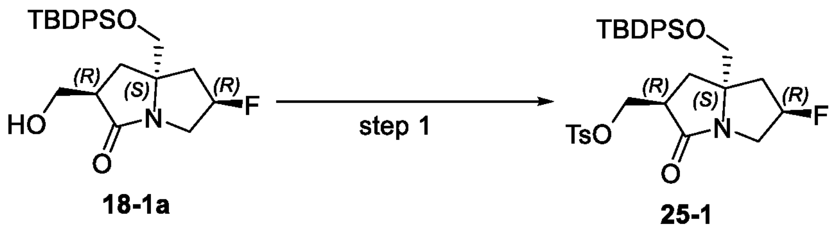


Under nitrogen atmosphere at 0°C with stirring, compound **18-1a** (1.5 g, 3.227 mmol, 1.0 eq), 4-dimethylaminopyridine (62.24 mg, 0.484 mmol, 0.15 eq), triethylamine (687.42 mg, 6.454 mmol, 2.0 eq), and dichloromethane (15 mL) were sequentially added to a reaction flask. Then, *p*-toluenesulfonyl chloride (777.03 mg, 3.872 mmol, 1.2 eq) was slowly added to the mixture. The resulting mixture was reacted with stirring at 25°C under nitrogen atmosphere for 16 hours, with the reaction progress monitored by LC-MS and TLC. After the reaction was completed, the reaction mixture was quenched by pouring into saturated sodium bicarbonate solution, and extracted with dichloromethane (30 mL × 3). The organic phases were combined, dried over anhydrous sodium sulfate, and filtered to remove the desiccant. The filtrate was concentrated under reduced pressure to obtain the crude product. The crude product was purified by silica gel column chromatography and subjected to a gradient elution with a mobile phase of 0% to 50% ethyl acetate/petroleum ether. The collected fraction was concentrated under reduced pressure to remove the solvent, yielding compound **25-1** (colorless oil, 1.77 g, yield of 87%). MS (ESI, m/z): 596.2 [M + H]$^+$; $^1$H NMR (400 MHz, $CDCl_3$) $\delta$ 7.80 - 7.75 (m, 2H), 7.63 - 7.57 (m, 4H), 7.48 - 7.36 (m, 6H), 7.36 - 7.31 (m, 2H), 5.29 - 5.13 (m, 1H), 4.36 - 4.30 (m, 1H), 4.13 - 3.99 (m, 2H), 3.53 (d, *J* = 10.4 Hz, 1H), 3.39 (d, *J* = 10.4 Hz, 1H), 3.24 - 3.14 (m, 1H), 3.08 - 2.93 (m, 1H), 2.49 - 2.40 (m, 4H), 2.26 - 2.10 (m, 1H), 2.05 - 1.87 (m, 2H), 1.03 (s, 9H).

**Step 2:**

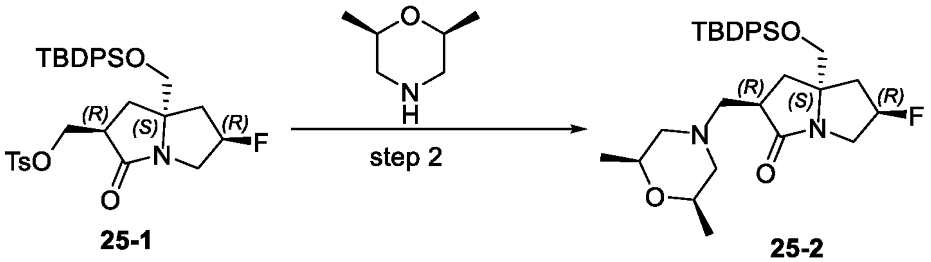


**25-1** **25-2**

Under nitrogen atmosphere at 25°C with stirring, compound **25-1** (1.77 g, 2.822 mmol, 1.0 eq), cis-2,6-dimethylmorpholine (684.33 mg, 5.644 mmol, 2.0 eq), potassium carbonate (1231.74 mg, 8.466 mmol, 3.0 eq), and acetonitrile (18 mL) were sequentially added to a reaction flask. The mixture was reacted with stirring at 80°C for 12 hours, with the reaction progress monitored by LC-MS and TLC. After the reaction was completed, the reaction mixture was filtered. The filtrate was concentrated to obtain the crude product. The crude product was purified by silica gel column chromatography and subjected to a gradient elution with a mobile phase of 0% to 50% methyl *tert*-butyl ether/petroleum ether. The collected fraction was concentrated under reduced pressure to remove the solvent, yielding compound **25-2** (colorless oil, 1.32 g, yield of 47%). MS (ESI, *m/z*): 539.3 [M + H]$^+$; $^1$H NMR (400 MHz, $CDCl_3$) $\delta$ 7.66 - 7.60 (m, 4H), 7.48 - 7.35 (m, 6H), 5.31 - 5.16 (m, 1H), 4.20 - 4.12 (m, 1H), 3.68 - 3.55 (m, 3H), 3.43 (d, *J* = 10.4 Hz, 1H), 3.17 - 3.01 (m, 2H), 2.90 - 2.78 (m, 1H), 2.71 - 2.55 (m, 2H), 2.45 - 2.32 (m, 2H), 2.31 - 2.14 (m, 1H), 2.04 - 1.79 (m, 3H), 1.77 - 1.66 (m, 1H), 1.16 - 1.10 (m, 6H), 1.04 (s, 9H).

**Step 3:**

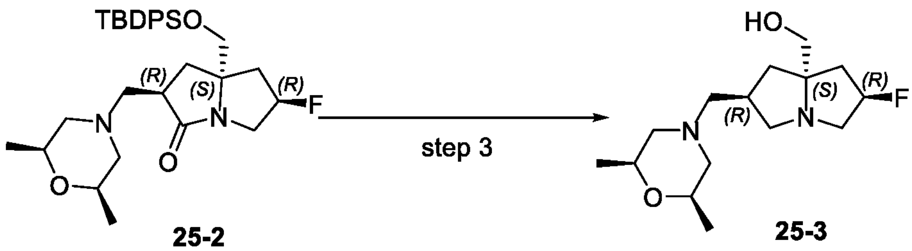


**25-2** **25-3**

Under nitrogen atmosphere at 0°C with stirring, a solution of lithium aluminum hydride in tetrahydrofuran (1 M, 1.8 mL, 1.5 eq) was slowly added dropwise to a solution of compound 25-2 (700 mg, 1.234 mmol, 1.0 eq) in anhydrous tetrahydrofuran (7 mL). The mixture was reacted with stirring at 60°C for 2 hours, with the reaction progress monitored by LC-MS and TLC. After the reaction was completed, the reaction mixture was cooled to 0°C, and then water (0.5 mL), 20% sodium hydroxide (0.5 mL), and water (1.5 mL) were sequentially and slowly added dropwise thereto. The resulting mixture was filtered, and the filtrate was concentrated under reduced pressure to obtain a crude product. The resulting crude product was purified by silica gel column chromatography and subjected to a gradient elution with a mobile phase of 0% to 10% ammonia in methanol/dichloromethane. The collected fraction was concentrated under reduced pressure to remove the solvent, yielding compound **25-3** (colorless oil, 290 mg, yield of 77%). MS (ESI, *m/z*): 287.2 [M + H]$^+$. $^1$H NMR (400 MHz, $CDCl_3$) $\delta$ 5.32 - 5.17 (m, 1H), 3.69 - 3.59 (m, 2H), 3.47 - 3.41 (m, 1H), 3.35 (d, *J* = 10.4 Hz, 1H), 3.27 (d, *J* = 10.4 Hz, 1H), 3.24 - 3.17 (m, 1H), 3.09 - 2.93 (m, 1H), 2.76 - 2.63 (m, 3H), 2.56 - 2.42 (m, 1H), 2.37 - 2.28 (m, 2H), 2.14 - 2.05 (m, 2H), 2.02 - 1.93 (m, 2H), 1.72 - 1.63 (m,2H), 1.18 - 1.11 (m, 6H).

**Step 4:**

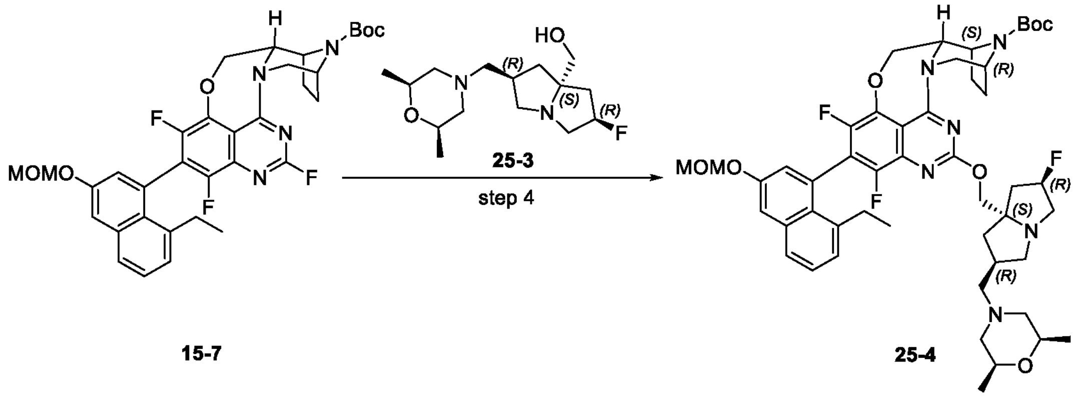


15-7 25-4

Under nitrogen atmosphere at 25°C with stirring, triethylenediamine (5.29 mg, 0.045 mmol, 0.2 eq), compound **15-7** (150 mg, 0.224 mmol, 1.0 eq), cesium carbonate (153.53 mg, 0.448 mmol, 2.0 eq), compound **25-3** (67.47 mg, 0.024 mmol, 1.0 eq), and *N*,*N*-dimethylformamide (1.5 mL) were sequentially added to a reaction flask. The resulting mixture was reacted with stirring at 80°C under nitrogen atmosphere for 2 hours, with the reaction progress monitored by LC-MS and TLC. After the reaction was completed, the crude product was purified by reverse-phase chromatography (C18 column) and eluted with a mobile phase of 5% to 95% methanol/water (0.1% ammonium bicarbonate aqueous solution) over 25 minutes, with detection at UV254/220 nm. The product obtained was compound **25-4** (yellow solid, 180 mg, yield of 84%). MS (ESI, m/z): 903.4 [M + H]$^+$.

**Step 5:**

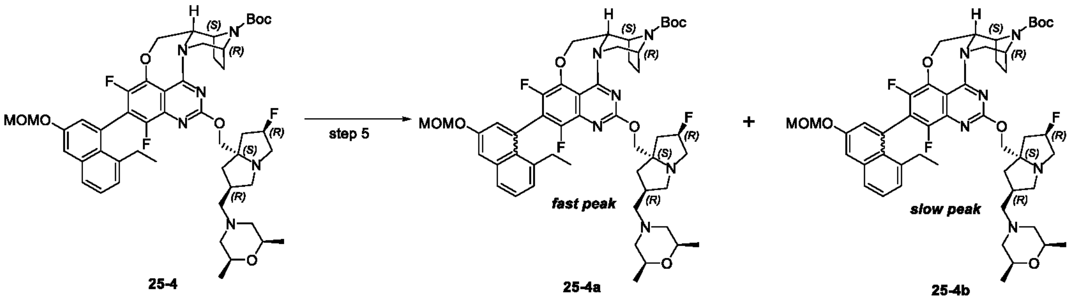


25-4 25-4a 25-4b

The compound **25-4** (180 mg) obtained from **step 4** of the present example was subjected to chiral resolution using high-performance liquid chromatography. Chiral column: CHIRALPAK IC, 2 × 25 cm, 5 μm; mobile phase A: *n*-hexane/methyl *tert*-butyl ether (1/1) (0.5%, 2 M ammonia in methanol), mobile phase B: methanol; flow rate: 20 mL/min; elution with 10% mobile phase B over 15 minutes; detector: UV 224/250 nm. Two compounds were obtained. The product with a shorter retention time (8.145 minutes) was compound **25-4a** (white solid, 65 mg, yield of 36%), MS (ESI, m/z): 903.4 [M + H]$^+$. The product with a longer retention time (11.32 minutes) was compound **25-4b** (white solid, 70 mg, yield of 39%), MS (ESI, m/z): 903.4 [M + H]$^+$.

**Step 6:**

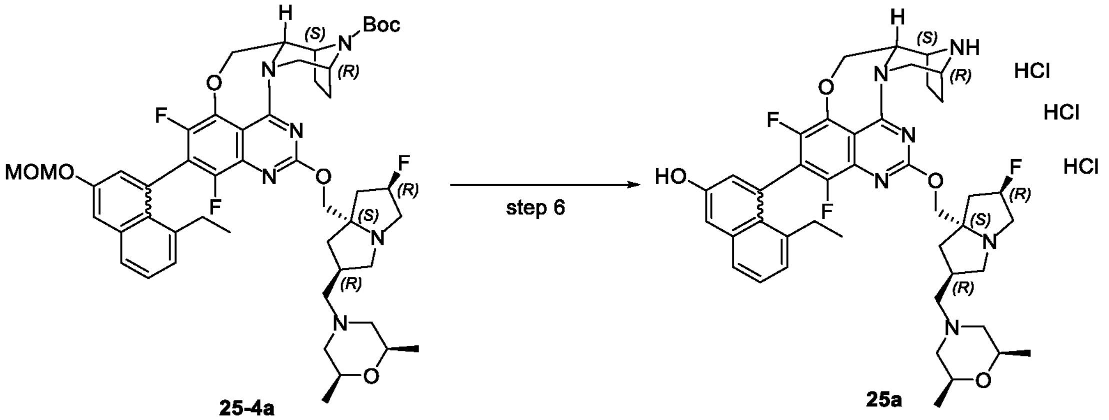


25-4a 25a

With stirring at 0°C, a solution of hydrochloric acid in 1,4-dioxane (4 M, 2 mL) was added dropwise to a solution of compound **25-4a** (65 mg, 0.072 mmol, 1.00 eq) in methanol (2 mL). The mixture was reacted at room temperature for 2 hours, with the reaction progress monitored by LC-MS. After the reaction was completed, the reaction mixture was concentrated to obtain the crude product. The crude product was purified by reverse-phase flash chromatography (C18 column) and eluted with a mobile phase of 5% to 95% acetonitrile/water (0.1% hydrochloric acid) over 25 minutes, with detection at UV 254 nm. The product obtained was compound **25a** (yellow solid, 37.3 mg, yield of 67%). MS (ESI, m/z): 759.4 $[M + H]^+$; $^1H$ NMR (400 MHz, DMSO-$d_6$ +$D_2O$) $\delta$ 7.72 - 7.66 (m, 1H), 7.42 - 7.36 (m, 1H), 7.33 (d, $J$ = 2.6 Hz, 1H), 7.17 - 7.13 (m, 1H), 6.98 (d, $J$ = 2.6 Hz, 1H), 5.73 - 5.54 (m, 1H), 5.16 - 5.05 (m, 1H), 4.81 - 4.69 (m, 1H), 4.68 - 4.57 (m, 4H), 4.36 - 4.24 (m, 2H), 4.23 - 4.13 (m, 1H), 4.11 - 3.98 (m, 2H), 3.97 - 3.85 (m, 1H), 3.83 - 3.62 (m, 2H), 3.46 - 3.38 (m, 3H), 3.35 - 3.18 (m, 2H), 3.15 - 2.98 (m, 2H), 2.83 - 2.73 (m, 1H), 2.70 - 2.56 (m, 3H), 2.42 - 2.31 (m, 2H), 2.19 - 1.88 (m, 5H), 1.12 (d, $J$ = 6.4 Hz, 6H), 0.90 - 0.80 (m, 3H); $^{19}F$ NMR (377 MHz, DMSO-$d_6$) $\delta$ -132.14, - 139.50, -173.10.

**Step 7:**

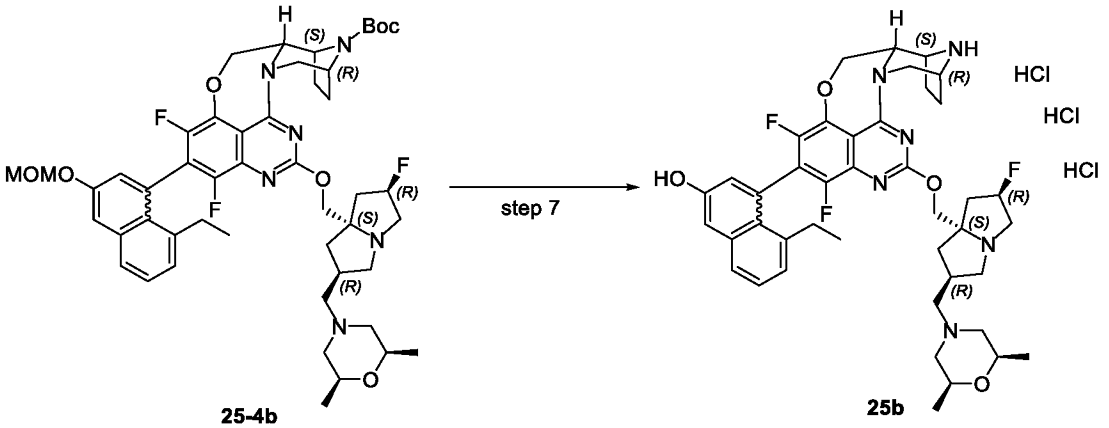


25-4b 25b

With stirring at 0°C, a solution of hydrochloric acid in 1,4-dioxane (4 M, 2 mL) was added dropwise to a solution of compound **25-4b** (70 mg, 0.077 mmol, 1.00 eq) in methanol (2 mL). The mixture was reacted at room temperature for 2 hours, with the reaction progress monitored by LC-MS. After the reaction was completed, the reaction mixture was concentrated to obtain the crude product. The crude product was purified by reverse-phase flash chromatography (C18 column) and eluted with a mobile phase of 5% to 95% acetonitrile/water (0.1% hydrochloric acid) over 30 minutes, with detection at UV 254 nm. The product obtained was compound **25b** (yellow solid, 56 mg, yield of 85%). MS (ESI, m/z): 759.4 $[M + H]^+$; $^1H$ NMR (400 MHz, DMSO-$d_6$ +$D_2O$) $\delta$ 7.72 - 7.66 (m, 1H), 7.41 - 7.36 (m, 1H), 7.33 (d, $J$ = 2.4 Hz, 1H), 7.19 - 7.12 (m, 1H), 6.97 (d, $J$ = 2.4 Hz, 1H), 5.72 - 5.54 (m, 1H), 5.15 - 5.04 (m, 1H), 4.79 - 4.72 (m, 1H), 4.68 - 4.55 (m, 4H), 4.34 - 4.24 (m, 2H), 4.23 - 4.14 (m, 1H), 4.13 - 3.99 (m, 2H), 3.98 - 3.84 (m, 1H), 3.82 - 3.62 (m, 2H), 3.46 - 3.41 (m, 3H), 3.36 - 3.21 (m, 2H), 3.16 - 2.99 (m, 2H), 2.82 - 2.73 (m, 1H), 2.70 - 2.55 (m, 3H), 2.47 - 2.37 (m, 2H), 2.19 - 2.11 (m, 1H), 2.09 - 1.89 (m, 4H), 1.11 (d, $J$ = 6.4 Hz, 6H), 0.93 - 0.84 (m, 3H); $^{19}F$ NMR (377 MHz, DMSO-$d_6$) $\delta$ -132.16, -139.21, -172.98.

**Example 26**

6-((2*S* or 2*R*, 6a*S*,7*S*,10*R*)-1,3-Difluoro-14-(((2*R*,7a*S*)-2-fluorotetrahydro-1*H*-pyrrolizin-7a(5*H*)-yl)methoxy)-5,6,6a,7,8,9,10,11-octahydro-7,10-epiminoazepino[1',2':5,6][1,5]oxazocino[4,3,2-de]quinazolin-2-yl)-4-methyl-5-(trifluoromethyl)pyridin-2-amine **26a'**; 6-((2*R* or 2*S*, 6a*S*,7*S*,10*R*)-1,3-difluoro-14-(((2*R*,7a*S*)-2-fluorotetrahydro-1*H*-pyrrolizin-7a(5*H*)-yl)methoxy)-5,6,6a,7,8,9,10,11-octahydro-7,10-epiminoazepino[1',2':5,6][1,5]oxazocino[4,3,2-*de*]quinazolin-2-yl)-4-methyl-5-(trifluoromethyl)pyridin-2-amine **26b'**

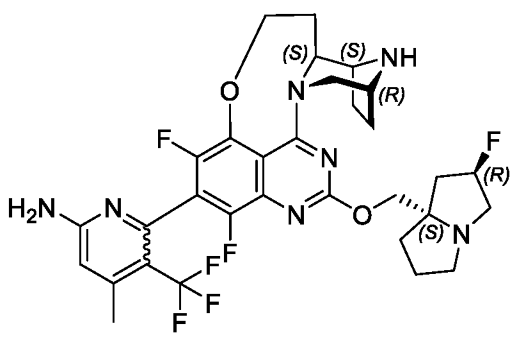

**26a'**

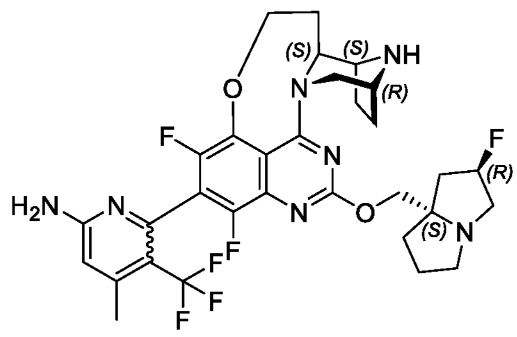

**26b'**

The synthetic route is as follows:

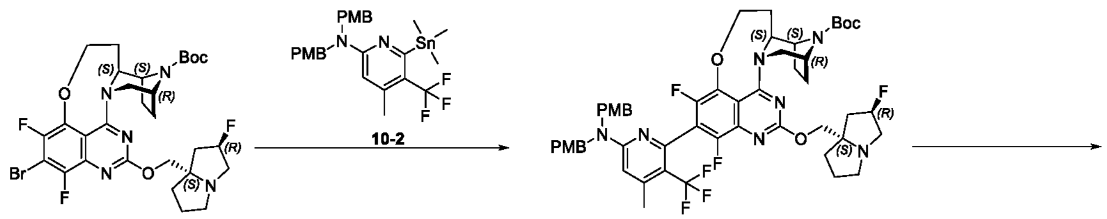


13-16

26-1

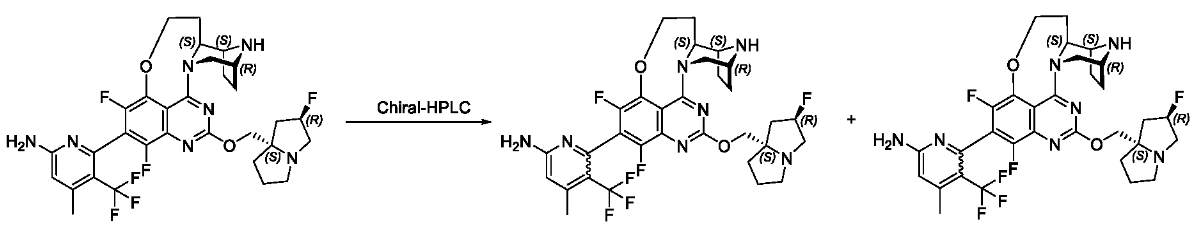


26

26a'

26b'

**Step 1:**

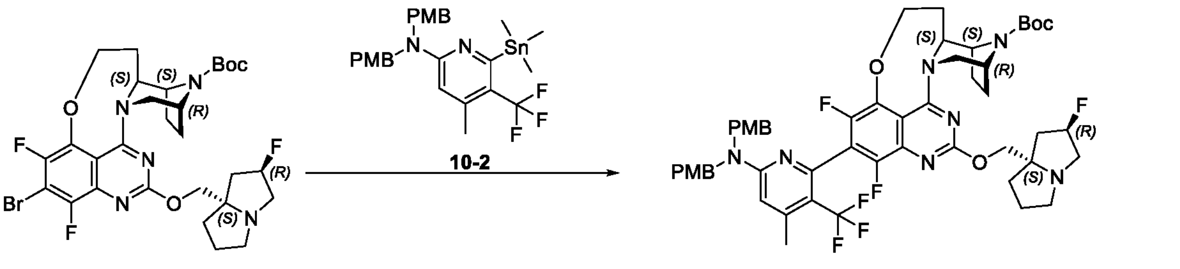


**13-16** **26-1**

Under nitrogen atmosphere at 25°C with stirring, tetrakis(triphenylphosphine)palladium (176.5 mg, 0.145 mmol, 0.5 eq), cuprous iodide (29.1 mg, 0.145 mmol, 0.5 eq), and a solution of anhydrous lithium chloride in tetrahydrofuran (0.5 M, 1.45 mL, 0.725 mmol, 2.5 eq) were sequentially added to a solution of compound 13-16 (200 mg, 0.29 mmol, 1 eq) and compound **10-2** (265.5 mg, 0.435 mmol, 1.5 eq) in anhydrous *N*,*N*-dimethylformamide (4 mL). The resulting mixture was reacted at 100°C for 16 hours, with the reaction progress monitored by LC-MS and TLC. After the reaction was completed, the reaction mixture was cooled to room temperature, initially purified by reverse-phase chromatography (C18 column), and eluted with a mobile phase of 30% to 95% acetonitrile/water (0.1% ammonia water) over 30 minutes, with detection at UV254/220 nm. The crude product of the compound was obtained. The crude product obtained was further purified by silica gel column chromatography and subjected to a gradient elution with a mobile phase of 0% to 8% methanol/dichloromethane. The collected fraction was concentrated under reduced pressure to remove the solvent, yielding compound **26-1** (white solid, 120 mg, yield of 39%). MS (ESI, *m*/*z*): 990.4 $[M + H]^+$; $^1H$ NMR (400 MHz, $CDCl_3$) $\delta$ 7.18 - 7.08 (m, 4H), 6.91 - 6.76 (m, 4H), 6.39 (d, *J* = 5.5 Hz, 1H), 5.57 - 5.20 (m, 2H), 4.93 - 4.59 (m, 5H), 4.55 - 4.42 (m, 2H), 4.39 - 4.03 (m, 3H), 3.80 (d, *J* = 1.5 Hz, 7H), 3.58 - 3.35 (m, 1H), 3.31 - 3.24 (m, 1H), 3.22 - 3.06 (m, 1H), 2.54 - 2.33 (m, 5H), 2.28 - 2.03 (m, 5H), 1.97 - 1.71 (m, 5H), 1.55 - 1.41 (m, 11H).

**Step 2:**

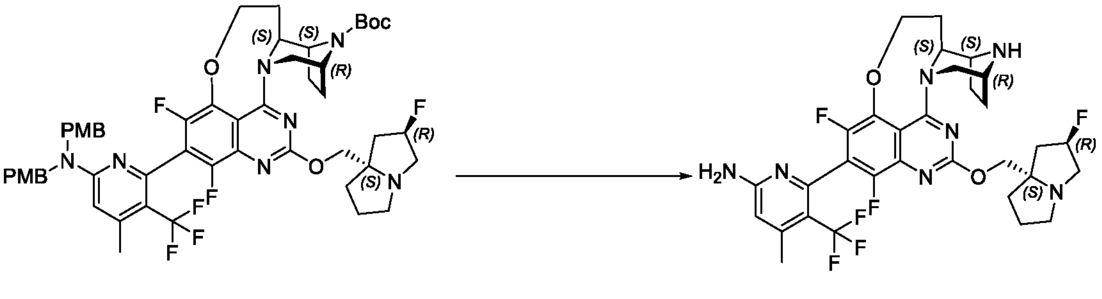

**26-1** **26**

With stirring at 25°C, trifluoroacetic acid (2 mL) was slowly added dropwise to a solution of compound **26-1** (120 mg, 0.115 mmol, 1.0 eq) in anisole (2 mL). The mixture was reacted at 100°C for 1 hour, with the reaction progress monitored by LC-MS. After the reaction was completed, the mixture was cooled to room temperature. The mixture was concentrated under reduced pressure to remove excess solvent, yielding the crude product. The resulting crude product was purified by reverse-phase chromatography (C18 column) and eluted with a mobile phase of 20% to 95% methanol/-water (0.1% ammonia water) over 30 minutes, with detection at UV 254/220 nm. The product obtained was compound **26** (white solid, 65 mg, yield of 82%). MS (ESI, *m/z):* 650.2 $[M + H]^+$.

**Step 3:**

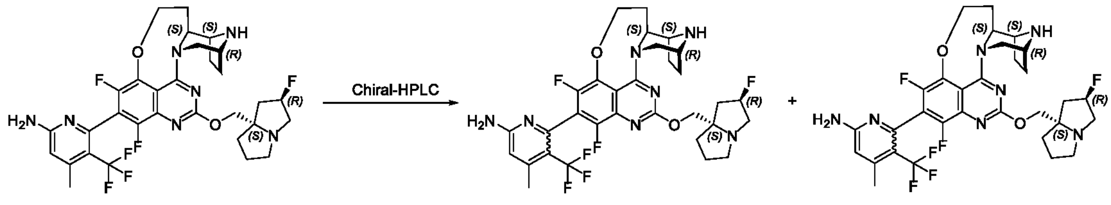


26 26a 26b

The compound **26** (65 mg) obtained from **step 2** of the present example was subjected to chiral resolution using preparative chiral high-performance liquid chromatography: chiral column: CHIRALPAK ID, 2 × 25 cm, 5 μm; mobile phase A: *n*-hexane/methyl *tert*-butyl ether=1/1 (0.5%, 2 M ammonia in methanol), mobile phase B: methanol; flow rate: 20 mL/min; elution with 10% mobile phase B over 23 minutes; detector: UV 216/244 nm, resulting in two products. The product with a shorter retention time (7.73 minutes) was compound **26a** (white solid, 30 mg, yield of 46%). MS (ESI, *m*/*z*): 650.3 [M + H]$^+$; $^1$H NMR (400 MHz, DMSO-$d_6$) $\delta$ 6.82 (s, 2H), 6.49 (s, 1H), 5.38 - 5.18 (m, 1H), 4.98 - 4.86 (m, 1H), 4.53 - 4.41 (m, 1H), 4.26 - 4.11 (m, 1H), 4.04 (d, *J* = 10.3 Hz, 1H), 3.93 (d, *J* = 10.3 Hz, 1H), 3.62 - 3.46 (m, 2H), 3.34 - 3.38 (m, 1H), 3.19 - 3.04 (m, 3H), 3.04 - 2.99 (m, 1H), 2.88 - 2.79 (m, 1H), 2.57 - 2.51 (m, 1H), 2.36 (d, *J* = 2.3 Hz, 3H), 2.18 - 1.70 (m, 8H), 1.68 - 1.52 (m, 2H), 1.51 - 1.42 (m, 1H), 1.31 - 1.23 (m, 1H); $^{19}$F NMR (377 MHz, DMSO-$d_6$) $\delta$ -53.80, -138.16, -142.25, -172.08. The product with a longer retention time (13.39 minutes) was compound **26b** (white solid, 18 mg, yield of 28%). MS (ESI, *m*/*z*): 650.3 [M + H]$^+$; $^1$H NMR (400 MHz, DMSO-$d_6$) $\delta$ 6.83 (s, 2H), 6.49 (s, 1H), 5.41 - 5.18 (m, 1H), 4.97 (d, *J* = 13.0 Hz, 1H), 4.52 - 4.39 (m, 1H), 4.24 - 4.06 (m, 2H), 4.01 - 3.88 (m, 1H), 3.62 (s, 1H), 3.57 - 3.51 (m, 1H), 3.45 (s, 1H), 3.22 - 3.14 (m, 1H), 3.14 - 2.96 (m, 3H), 2.91 - 2.73 (m, 1H), 2.36 (s, 3H), 2.21 - 1.89 (m, 4H), 1.89 - 1.45 (m, 6H), 1.38 - 1.15 (m, 3H); $^{19}$F NMR (377 MHz, DMSO-$d_6$) $\delta$ -53.25, -137.17, -141.21, -172.13.

## Example 27

4-((2*R* or 2*S*, 5*aS*,6*S*,9*R*)-1,3-Difluoro-13-(((2*R*,6*R*,7*aS*)-2-fluoro-6-(piperidin-1-ylmethyl)tetrahydro-1*H*-pyrrolizin-7*a*(5*H*)-yl)methoxy)-5*a*,6,7,8,9,10-hexahydro-5*H*-6,9-epiminoazepino[2',1':3,4][1,4]oxazepino[5,6,7-*de*]quinazolin-2-yl)-5-ethylnaphthalen-2-ol trihydrochloride **27a;** 4-((2*S* or 2*R*, 5*aS*,6*S*,9*R*)-1,3-difluoro-13-(((2*R*,6*R*,7*aS*)-2-fluoro-6-(piperidin-1-ylmethyl)tetrahydro-1*H*-pyrrolizin-7*a*(5*H*)-yl)methoxy)-5*a*,6,7,8,9,10-hexahydro-5*H*-6,9-epiminoazepino[2',1':3,4][1,4]oxazepino[5,6,7-de]quinazolin-2-yl)-5-ethylnaphthalen-2-ol trihydrochloride 27b

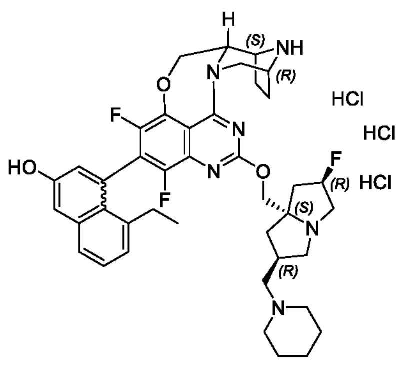

27a

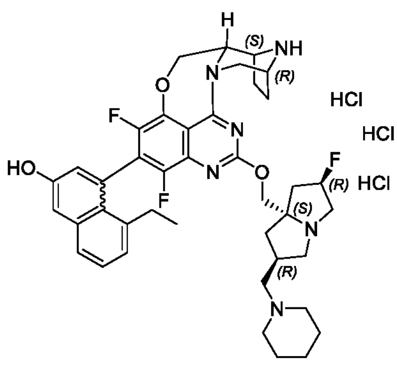

27b

The synthetic route is as follows:

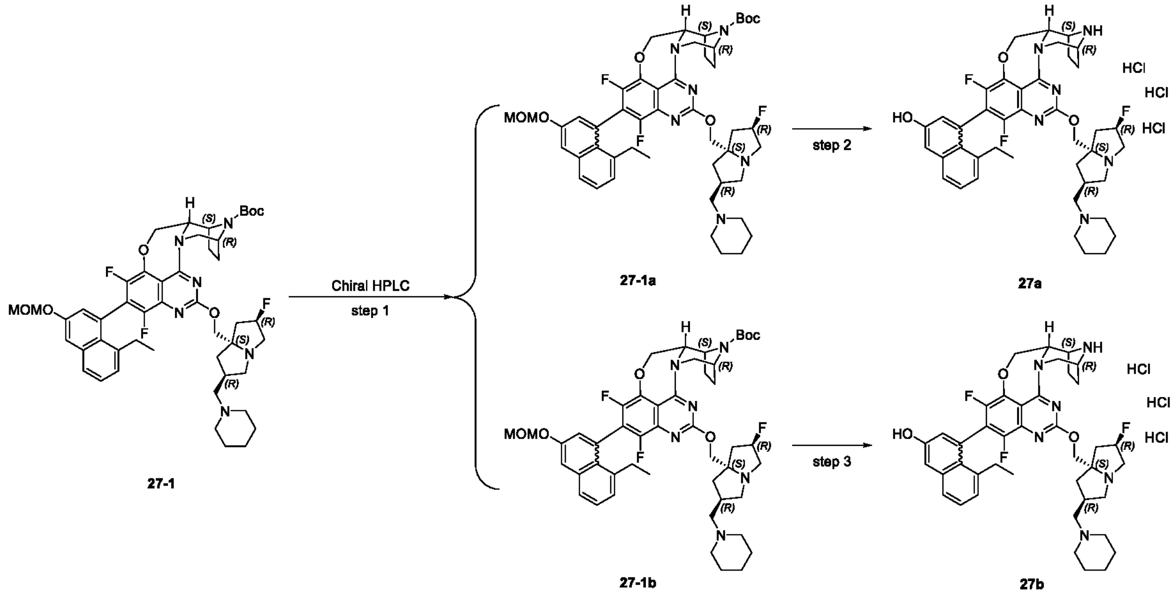


27-1 27-1a 27a 27-1b 27b

**Step 1:**

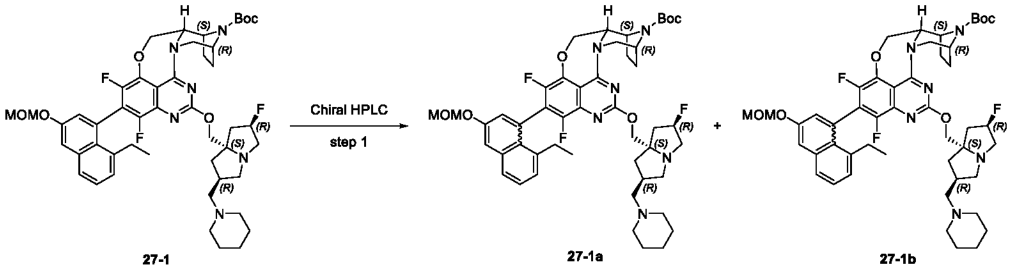


27-1 27-1a 27-1b

Compound **27-1** (150 mg) was synthesized with reference to **Example 25.**

Compound **27-1** (150 mg) was subjected to chiral resolution using high-performance liquid chromatography. Chiral column: CHIRALPAK IE, 2 × 25 cm, 5 μm; mobile phase A: *n*-hexane/methyl *tert*-butyl ether (1/1) (10 mmol/L ammonia in methanol), mobile phase B: methanol; flow rate: 20 mL/min; elution with 10% mobile phase B over 21 minutes; detector: UV 226/220 nm. Two compounds were obtained. The product with a shorter retention time (8.532 minutes) was compound **27-1a** (white solid, 65 mg, yield of 43%), MS (ESI, m/z): 873.4 $[M + H]^+$. The product with a longer retention time (11.97 minutes) was compound **27-1b** (white solid, 48 mg, yield of 32%), MS (ESI, m/z): 873.4 $[M + H]^+$.

**Step 2:**

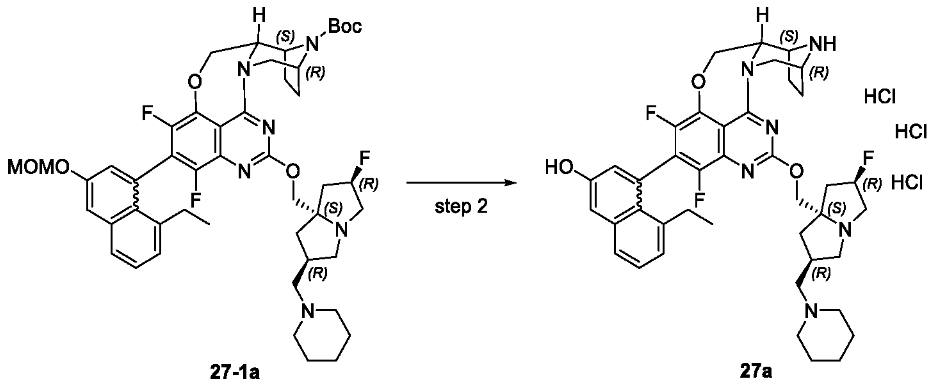

27-1a

27a

With stirring at 0°C, a solution of hydrochloric acid in 1,4-dioxane (4 M, 1 mL) was added dropwise to a solution of compound **27-1a** (65 mg, 0.074 mmol, 1.00 eq) in methanol (1 mL). The mixture was reacted at room temperature for 1 hour, with the reaction progress monitored by LC-MS. After the reaction was completed, the reaction mixture was concentrated to obtain the crude product. The crude product was purified by reverse-phase flash chromatography (C18 column) and eluted with a mobile phase of 5% to 95% acetonitrile/water (0.1% hydrochloric acid) over 25 minutes, with detection at UV 220 nm. The product obtained was compound **27a** (yellow solid, 24.5 mg, yield of 40%). MS (ESI, *m/z):* 729.4 $[M + H]^+$; $^1H$ NMR (400 MHz, DMSO-$d_6$) $\delta$ 11.79 (s, 1H), 10.80 (s, 1H), 10.43 - 10.29 (m, 1H), 10.14 (s, 1H), 10.01 - 9.88 (m, 1H), 7.73 - 7.63 (m, 1H), 7.42 - 7.35 (m, 1H), 7.32 (d, *J* = 2.6 Hz, 1H), 7.17 - 7.10 (m, 1H), 7.00 (d, *J* = 2.6 Hz, 1H), 5.72 - 5.49 (m, 1H), 5.09 (d, *J* = 13.9 Hz, 1H), 4.78 - 4.71 (m, 1H), 4.68 - 4.56 (m, 4H), 4.33 - 4.21 (m, 2H), 4.15 (s, 1H), 3.94 - 3.83 (m, 1H), 3.82 - 3.71 (m, 3H), 3.44 - 3.37 (m, 2H), 3.30 - 3.22 (m, 1H), 3.21 - 3.13 (m, 1H), 3.10 - 2.98 (m, 2H), 2.91 - 2.78 (m, 2H), 2.78 - 2.69 (m, 1H), 2.59 - 2.55 (m, 1H), 2.40 - 2.33 (m, 2H), 2.17 - 2.06 (m, 1H), 2.04 - 1.81 (m, 6H), 1.80 - 1.63 (m, 3H), 1.44 - 1.30 (m, 1H), 0.90 - 0.80 (m, 3H); $^{19}F$ NMR (377 MHz, DMSO-$d_6$) $\delta$ -132.18, -139.58, -172.95.

**Step 3:**

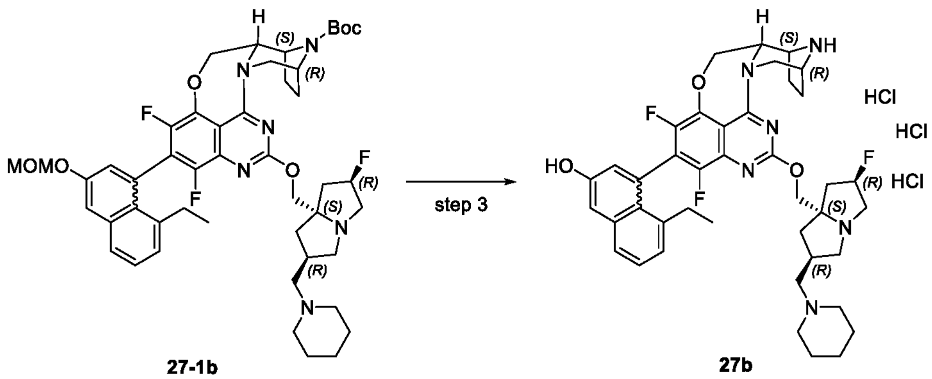

27-1b

27b

Referring to the same method as **step 2** of this example, compound **27b** (yellow solid, 29.1 mg, yield of 65%) was obtained from compound **27-1b.** MS (ESI, *m/z):* 729.4 $[M + H]^+$; $^1H$ NMR (400 MHz, DMSO-$d_6$ + $D_2O$) $\delta$ 7.72 - 7.67 (m, 1H), 7.46 - 7.37 (m, 1H), 7.34 (d, *J* = 2.7 Hz, 1H), 7.22 - 7.15 (m, 1H), 6.97 - 6.90 (m, 1H), 5.78 - 5.53 (m, 1H), 5.18 - 4.99 (m, 1H), 4.78 - 4.69 (m, 1H), 4.65 - 4.56 (m, 3H), 4.52 - 4.42 (m, 1H), 4.32 - 4.24 (m, 2H), 4.15 - 4.04 (m, 1H), 3.98 - 3.85 (m, 1H), 3.83 - 3.75 (m, 1H), 3.57 - 3.50 (m, 1H), 3.50 - 3.39 (m, 2H), 3.31 - 3.16 (m, 2H), 3.11 - 2.97 (m, 2H), 2.95 - 2.81 (m, 2H), 2.75 - 2.65 (m, 1H), 2.62 - 2.57 (m, 1H), 2.45 - 2.33 (m, 3H), 2.20 - 2.09 (m, 1H), 2.08 - 1.89 (m, 4H), 1.86 - 1.63 (m, 5H), 1.49 - 1.33 (m, 1H), 0.95 - 0.79 (m, 3H); $^{19}F$ NMR (377 MHz, DMSO-$d_6$) $\delta$ -132.24, -139.21, -173.00.

**Example 28**

4-((2*R* or 2*S*, 5*aS*,6*S*,9*R*)-1,3-Difluoro-13-(((2*R*,6*R*,7*aS*)-2-fluoro-6-(morpholinomethyl)tetrahydro-1*H*-pyrrolizin-7*a*(5*H*)-yl)methoxy)-5a,6,7,8,9,10-hexahydro-5*H*-6,9-epiminoazepino[2',1':3,4][1,4]oxazepino[5,6,7-*de*]quinazolin-2-yl)-5-ethylnaphthalen-2-ol trihydrochloride **28a;** 4-((2S or 2R, 5*aS*,6*S*,9*R*)-1,3-difluoro-13-(((2*R*,6*R*,7*aS*)-2-fluoro-6-(morpholinomethyl)tetrahydro-1*H*-pyrrolizin-7*a*(5*H*)-yl)methoxy)-5a,6,7,8,9,10-hexahydro-5*H*-6,9-epiminoazepino[2',1':3,4][1,4]oxazepino[5,6,7-*de*]quinazolin-2-yl)-5-ethylnaphthalen-2-ol trihydrochloride **28b**

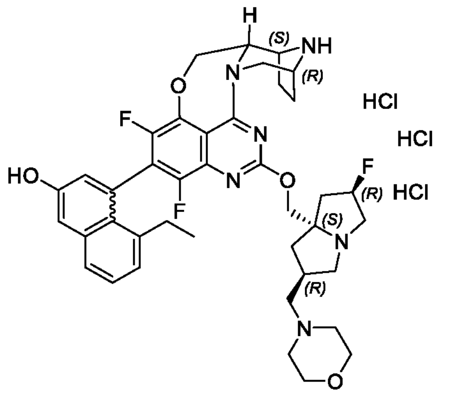

**28a**

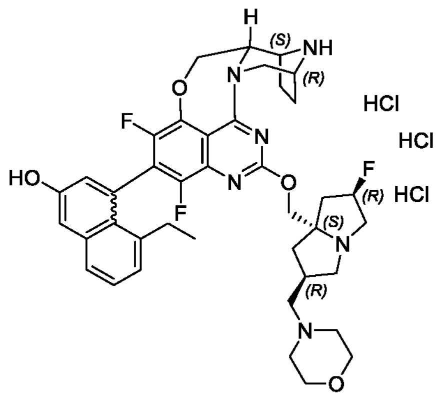

**28b**

The synthetic route is as follows:

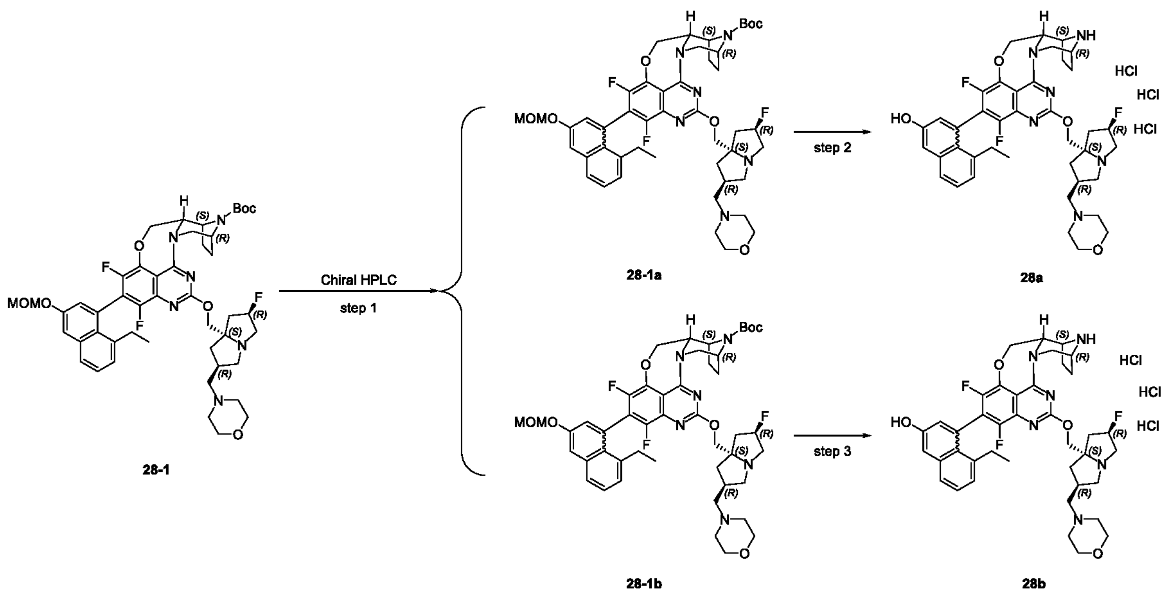


Step 1:

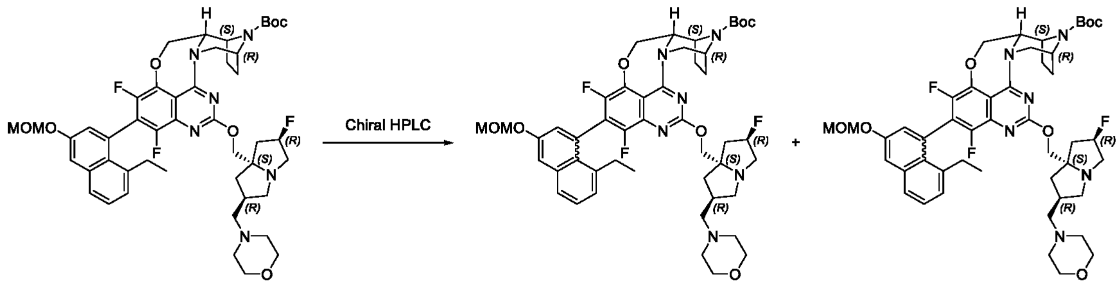


28-1 28-1a 28-1b

Compound **28-1** (120 mg) was synthesized with reference to **Example 25.**

Compound **28-1** (120 mg) was subjected to chiral resolution using high-performance liquid chromatography. Chiral column: CHIRALPAK IE, 2 × 25 cm, 5 μm; mobile phase A: *n*-hexane/methyl *tert*-butyl ether (1/1) (10 mmol/L ammonia in methanol), mobile phase B: methanol; flow rate: 20 mL/min; elution with 10% mobile phase B over 20 minutes; detector: UV 226/220 nm. Two compounds were obtained. The product with a shorter retention time (11.44 minutes) was compound **28-1a** (white solid, 42 mg, yield of 35%), MS (ESI, m/z): 875.4 $[M + H]^+$. The product with a longer retention time (16.06 minutes) was compound **28-1b** (white solid, 38 mg, yield of 31%), MS (ESI, m/z): 875.4 $[M + H]^+$.

**Step 2:**

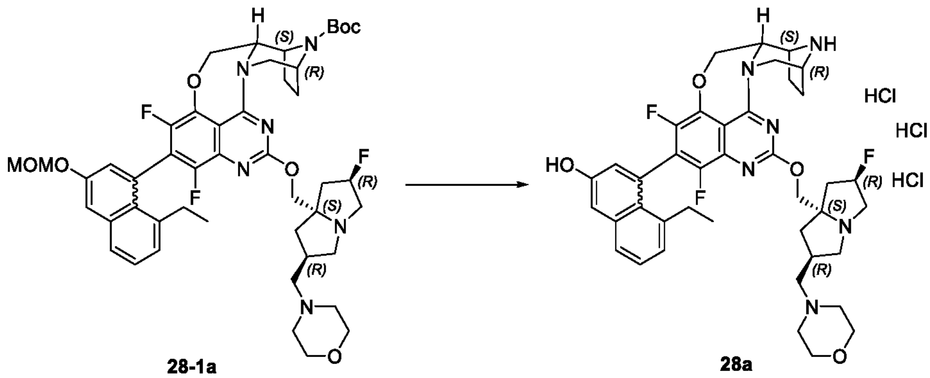

28-1a 28a

With stirring at 0°C, a solution of hydrochloric acid in 1,4-dioxane (4 M, 1 mL) was added dropwise to a solution of compound **28-1a** (42 mg, 0.048 mmol, 1.00 eq) in methanol (1 mL). The mixture was reacted at room temperature for 1 hour, with the reaction progress monitored by LC-MS. After the reaction was completed, the reaction mixture was concentrated to obtain the crude product. The crude product was purified by reverse-phase flash chromatography (C18 column) and eluted with a mobile phase of 5% to 95% acetonitrile/water (0.1% hydrochloric acid) over 25 minutes, with detection at UV 254 nm. The product obtained was compound **28a** (yellow solid, 13.2 mg, yield of 32%). MS (ESI, *m*/*z*): 731.3 $[M + H]^+$; $^1$H NMR (400 MHz, DMSO-$d_6$) δ 11.81 (s, 1H), 11.63 (s, 1H), 10.37 (d, *J* = 9.9 Hz, 1H),10.28 - 10.01 (m, 1H), 9.94 (s, 1H), 7.72 - 7.63 (m, 1H), 7.42 - 7.35 (m, 1H), 7.32 (d, *J* = 2.6 Hz, 1H), 7.20 - 7.10 (m, 1H), 7.00 (d, *J* = 2.6 Hz, 1H), 5.75 - 5.50 (m, 1H), 5.10 (d, *J* = 13.9 Hz, 1H), 4.74 (d, *J* = 11.3 Hz, 1H), 4.68 - 4.55 (m, 4H), 4.30 - 4.23 (m, 2H), 4.18 - 4.13 (m, 1H), 3.90 - 3.86 (m, 2H), 3.85 - 3.74 (m, 2H), 3.68 (d, *J* = 14.0 Hz, 2H), 3.55 - 3.21 (m, 5H), 3.17 - 2.95 (m, 5H), 2.82 - 2.72 (m, 1H), 2.57 (s, 1H), 2.41 - 2.30 (m, 2H), 2.17 - 1.87 (m, 6H), 0.92 - 0.80 (m, 3H); $^{19}$F NMR (377 MHz, DMSO-$d_6$) δ -132.18, -139.55, -173.01.

**Step 3:**

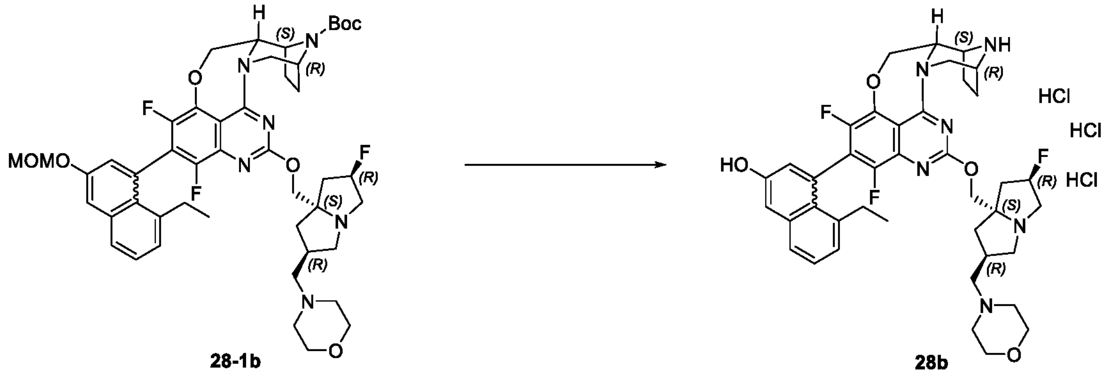

28-1b 28b

Referring to the same method as **step 2** of this example, compound **28b** (yellow solid, 22.5 mg, yield of 63%) was obtained from compound **28-1b.** MS (ESI, *m/z*): 731.3 $[M + H]^+$; $^1H$ NMR (400 MHz, DMSO-$d_6$+$D_2O$) $\delta$ 7.72 - 7.63 (m, 1H), 7.43 - 7.36 (m, 1H), 7.33 (d, *J* = 2.6 Hz, 1H), 7.19 - 7.12 (m, 1H), 6.96 (d, *J* = 2.7 Hz, 1H), 5.75 - 5.55 (m, 1H), 5.16 - 5.03 (m, 1H), 4.79 - 4.68 (m, 1H), 4.65 - 4.56 (m, 3H), 4.56 - 4.44 (m, 1H), 4.33 - 4.22 (m, 2H), 4.19 - 4.06 (m, 1H), 4.03 - 3.76 (m, 6H), 3.74 - 3.66 (m, 1H), 3.61 - 3.61 (m, 2H), 3.42 - 3.23 (m, 3H), 3.18 - 2.97 (m, 4H), 2.81 - 2.65 (m, 1H), 2.58 (s, 1H), 2.45 - 2.36 (m, 2H), 2.20 - 1.87 (m, 5H), 0.94 - 0.78 (m, 3H); $^{19}F$ NMR (377 MHz, DMSO-$d_6$) $\delta$ -132.23, -139.25, -173.00, -173.05.

**Example 29**

4-((2*S* or 2*R*, 5a*S*,6*S*,9*R*)-1,3-Difluoro-13-(((2*R*,6*R*,7a*S*)-2-fluoro-6-((4-methylpiperazin-1-yl)methyl)tetrahydro-1*H*-pyrrolizin-7*a*(5*H*)-yl)methoxy)-5*a*,6, 7,9,10-hexahydro-5*H*-6,9-epiminoazepino[2',1':3,4][1,4]oxazepino[5,6,7-*de*]quinazolin-2-yl)-5-ethylnaphthalen-2-ol tetrahydrochloride **29a;** 4-((2*R* or 2*S*, 5a*S*,6*S*,9*R*)-1,3-difluoro-13-(((2*R*,6*R*,7*aS)*-2-fluoro-6-((4-methylpiperazin-1-yl)methyl)tetrahydro-1*H*-pyrrolizin-7*a*(5*H*-yl)methoxy)-5*a*,6,7,9,10-hexahydro-5*H*-6,9-epiminoazepino[2',1':3,4][1,4]oxazepino[5,6,7-*de*]quinazolin-2-yl)-5-ethylnaphthalen-2-ol tetrahydrochloride **29b**

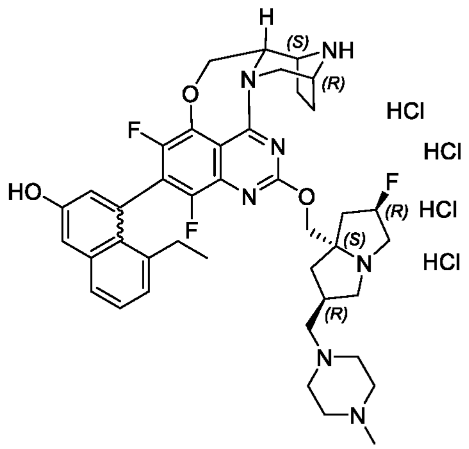

**29a**

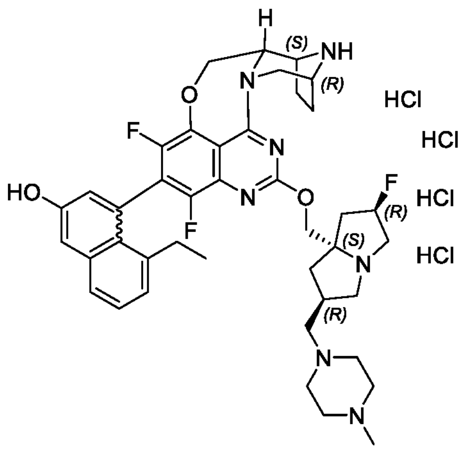

**29b**

The synthetic route is as follows:

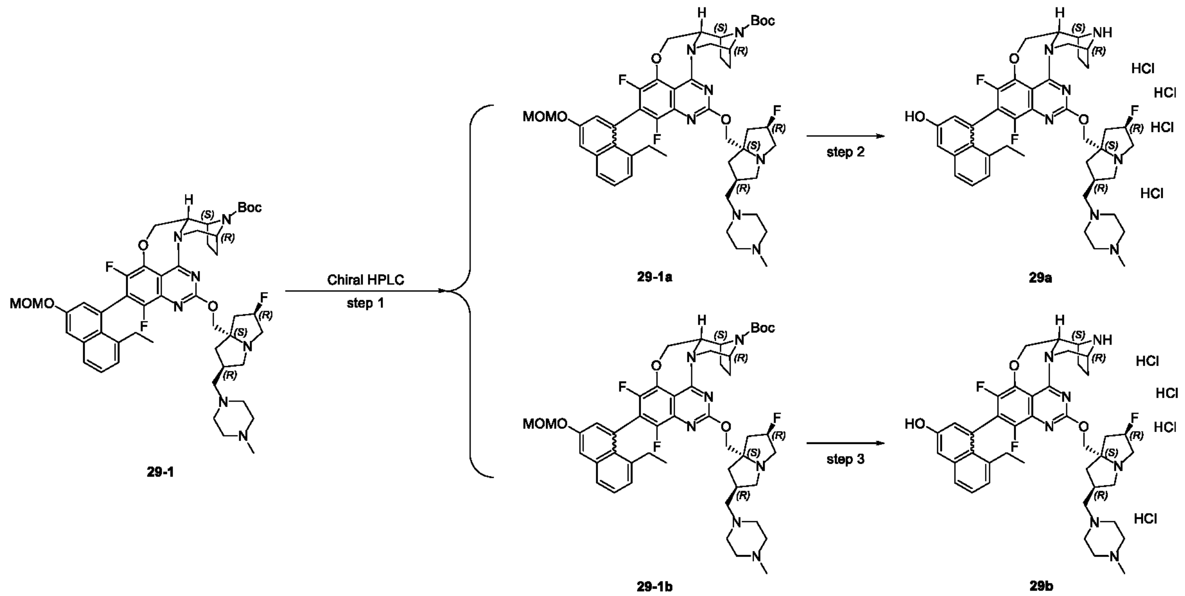


29-1 29-1a 29a 29-1b 29b

Step 1:

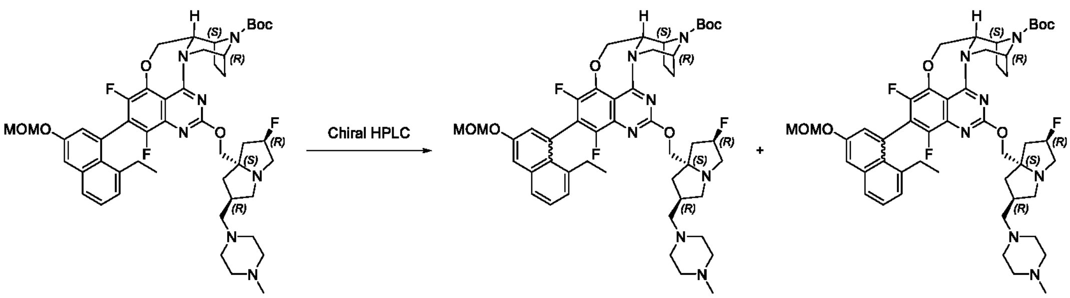


29-1 29-1a 29-1b

Compound **29-1** (90 mg) was synthesized with reference to **Example 25.**

Compound **29-1** (90 mg) was subj ected to chiral resolution using high-performance liquid chromatography. Chiral column: CHIRALPAK IE, 2 × 25 cm, 5 μm; mobile phase A: n-hexane (10 mmol/L ammonia), mobile phase B: ethanol; flow rate: 20 mL/min; elution with 40% mobile phase B over 20.5 minutes; detector: UV 226/292 nm. Two compounds were obtained. The product with a shorter retention time (13.16 minutes) was compound **29-1a** (white solid, 40 mg, yield of 44%), MS (ESI, m/z): 888.5 $[M + H]^+$. The product with a longer retention time (14.77 minutes) was compound **29-1b** (white solid, 34 mg, yield of 37%), MS (ESI, m/z): 888.5 [M + H]+.

**Step 2:**

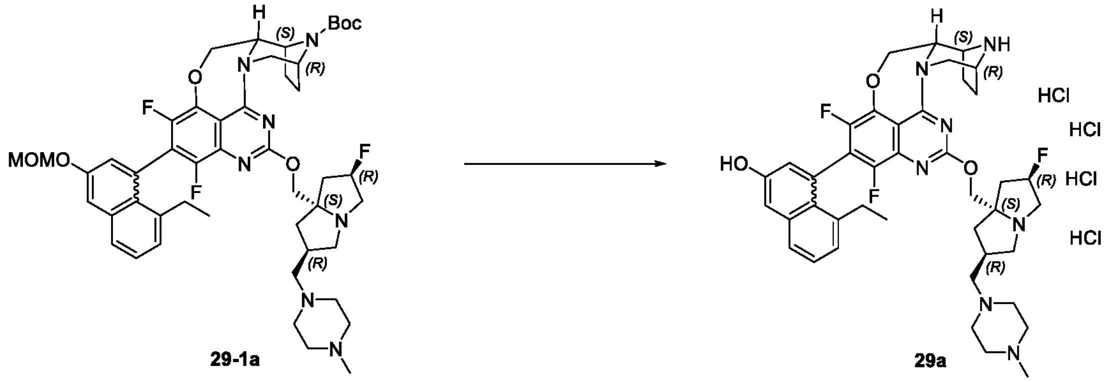

29-1a 29a

With stirring at 0°C, a solution of hydrochloric acid in 1,4-dioxane (4 M, 1 mL) was added dropwise to a solution of compound **29-1a** (40 mg, 0.043 mmol, 1.00 eq) in methanol (1 mL). The mixture was reacted at room temperature for 1 hour, with the reaction progress monitored by LC-MS. After the reaction was completed, the reaction mixture was concentrated to obtain the crude product. The crude product was purified by reverse-phase flash chromatography (C18 column) and eluted with a mobile phase of 5% to 95% acetonitrile/water (0.1% hydrochloric acid) over 25 minutes, with detection at UV 254 nm. The product obtained was compound **29a** (yellow solid, 6.6 mg, yield of 17%). MS (ESI, *m/z):* 744.4 $[M + H]^+$; $^1H$ NMR (400 MHz, $CD_3OD$) $\delta$ 7.68 - 7.61 (m, 1H), 7.42 - 7.35 (m, 1H), 7.34 - 7.29 (m, 1H), 7.21 - 7.14 (m, 1H), 7.01 - 6.93 (m, 1H), 5.77 - 5.47 (m, 2H), 5.10 - 4.98 (m, 1H), 4.83 - 4.75 (m, 4H), 4.74 - 4.62 (m, 2H), 4.46 (s, 1H), 4.42 - 4.35 (m, 1H), 4.32 - 4.21 (m, 1H), 4.08 - 3.90 (m, 2H), 3.88 - 3.52 (m, 7H), 3.30 - 3.16 (m, 4H), 3.03 - 2.96 (m, 3H), 2.96 - 2.86 (m, 1H), 2.82 - 2.63 (m, 2H), 2.49 - 2.37 (m, 2H), 2.36 - 2.05 (m, 5H), 1.02 - 0.86 (m, 3H); $^{19}F$ NMR (376 MHz, $CD_3OD$) $\delta$ -134.71, -136.37, -174.53.

**Step 3:**

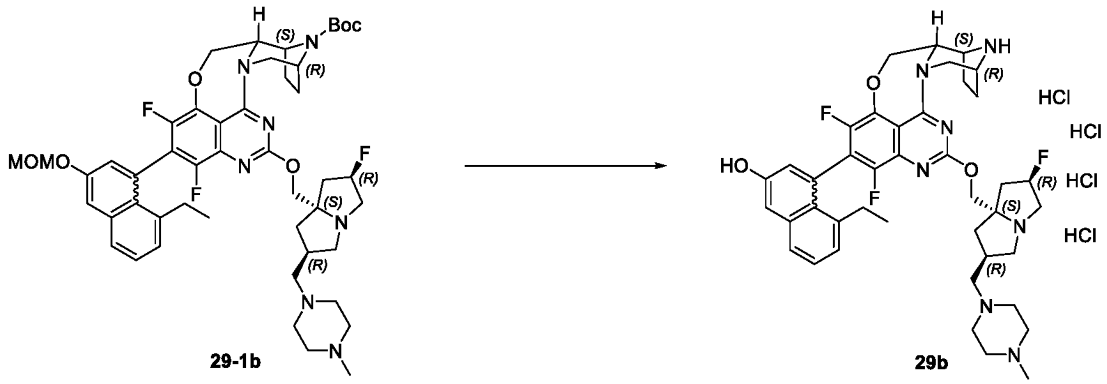

29-1b 29b

Referring to the same method as **step 2** of this example, compound **29b** (yellow solid, 10 mg, yield of 39%) was obtained from compound **29-1b.** MS (ESI, *m/z):* 744.4 $[M + H]^+$; $^1H$ NMR (400 MHz, $CD_3OD$) $\delta$ 7.68 - 7.61 (m, 1H), 7.42 - 7.35 (m, 1H), 7.34 - 7.28 (m, 1H), 7.23 - 7.15 (m, 1H), 7.00 - 6.91 (m, 1H), 5.74 - 5.44 (m, 2H), 5.06 - 4.94 (m, 1H), 4.83 - 4.76 (m, 4H), 4.74 - 4.60 (m, 2H), 4.53 - 4.36 (m, 2H), 4.34 - 4.20 (m, 1H), 4.10 - 3.87 (m, 2H), 3.87 - 3.53 (m, 7H), 3.30 - 3.14 (m, 4H), 3.05 - 2.97 (m, 3H), 2.96 - 2.84 (m, 1H), 2.79 - 2.58 (m, 2H), 2.54 - 2.44 (m, 2H), 2.42 - 2.31 (m, 1H), 2.30 - 2.08 (m, 4H), 1.02 - 0.91 (m, 3H); $^{19}F$ NMR (376 MHz, $CD_3OD$) $\delta$ -134.60, -134.68, -136.29, -136.65,-174.65, -174.66.

**Effect example A**

1. Experiment obj ective

To detect the inhibitory capability of small molecule compounds on the binding activity between KRAS_WT and SOS1 using a drug screening system based on the binding of KRAS_WT and SOS1 interaction.

2. Experimental materials and instruments

The experimental materials and instruments are listed in Table 1:

Table 1

| Reagent | Brand | Catalog No. |
|---|---|---|
| KRAS-WT/SOS1 binding kits | Cisbio | 63ADK000CB15PEH |
| GTP | Sigma | V900868 |
| Consumable | Brand | Catalog No. |
| Topseal A | PerkinElmer | E5341 |
| 384-Well Polypropylene microplate | labcyte | PP-0200 |
| 96-Well Plates | Nunc | 249944 |
| 384-well plates | Corning | CLS4514 |
| Instrument | Brand | Catalog No. |
| Envision | Perkin Elmer | 2104 |
| Centrifuge | Eppendorf | 5810R |
| Multi-channel pipettes | Eppendorf/Sartorius | / |
| Echo | Labcyte | / |

3 Experimental methods

3.1. Experimental steps

a) BI-2852 was used as a positive control, with its stock solution as the first dilution point, followed by a 3-fold dilution, making a total of 10 + 0 dilutions. Similarly, the first dilution point of the test compounds was also their respective stock solutions, followed by a 3-fold dilution, making a total of 11 + 0 dilutions. Using Echo, 0.2 μL of the gradient-diluted compound solutions were transferred into a 384-well plate, with each compound tested in duplicate. The final DMSO concentration was 1%. The plate was centrifuged at 1000 rpm for 1 minute. The final concentrations of the positive controls were 100, 33.33, 11.11, 3.70, 1.23, 0.412, 0.137, 0.046, 0.015, 0.005, and 0 μM. The final concentrations of the test compounds were 200, 66.67, 22.22, 7.41, 2.47, 0.27, 0.091, 0.03, 0.0152, 0.01, and 0 μM.
b) KRAS_WT from the kit was prepared with GTP at a final concentration of 10 μM in the dilution buffer, and 5 μL was transferred into the 384-well reaction plate. The plate was centrifuged at 1000 rpm for 1 minute.
c) Subsequently, 5 μL of the SOS1 mixture was transferred into the 384-well reaction plate. The plate was centrifuged at 1000 rpm for 1 minute and incubated at 25°C for 15 minutes.
d) 10 μL of the detection mixture was transferred into the 384-well reaction plate. The plate was centrifuged at 1000 rpm for 1 minute and incubated overnight at 4°C.
e) The excitation wavelength at 665 nm and emission wavelength at 615 nm were read using an Envision multimode plate reader. The 665/615 ratio signal intensity was used to characterize the enzyme activity.
f) The raw data was analyzed.

3.2 Experimental data processing method:

The $IC_{50}$ of the compounds was fitted using a nonlinear regression equation with GraphPad Prism 8. The results are presented in Table 2:

Negative control: DMSO
Positive control: 100 μM BI-2852

The following nonlinear fitting formula was used to obtain the $IC_{50}$ (half-maximal inhibitory concentration) of the compounds:

$$Y = Bottom + (Top - Bottom) / (1 + 10^((LogIC_{50} - X) * HillSlope))$$

X: logarithm of the compound concentration
Y: 665/615 Ratio

Table 2

| Compound No. | GTP-KRAS-G12D:SOS1 $IC_{50}$ (nM) | GTP-KRAS-G12D:CRAF $IC_{50}$ (nM) | GTP-KRAS-WT:SOS1 $IC_{50}$ (nM) |
|---|---|---|---|
| **1a** | 45.62 | 333.4 | 4100 |
| **2a** | 16.74 | 32.49 | 253.6 |
| **3** | 11.89 | 12.25 | 79.63 |
| **4b’** | 18.31 | 8.216 | 5.596 |
| **5a** | 14.54 | 5.807 | 11.36 |
| **5b** | 2801 | \ | \ |
| **6a** | 313.7 | \ | \ |
| **6b** | \ | \ | \ |
| **7a** | 21.54 | 23.98 | 245.7 |
| **7aa** | 9.947 | 16.37 | 232.8 |
| **7ab** | 17.44 | 84.83 | 582.3 |
| **8a** | 13.48 | 19.54 | 61.21 |
| **8b** | \ | \ | \ |
| **8c** | 4013 | \ | \ |
| **8d** | 115 | 491.4 | 4746 |
| **9a** | 15.18 | 46.82 | 559.4 |
| **9b** | \ | \ | \ |
| **10a’** | 700.3 | | |
| **10b’** | 11.65 | | |
| **11b** | 18.53 | | |
| **12b** | 21.3 | | |
| **13a’** | 28.16 | | |
| **13b** | 8933 | | |
| **14b** | 15.32 | | |
| **15a** | 2674 | | |
| **15b** | 6.541 | | |
| **16b** | 7.55 | | |
| **17** | 8307 | | |
| **18a** | 1937 | | |
| **18b** | 7.5 | | |
| **19** | 12.66 | | |
| **20b** | 8.806 | | |

(continued)

| Compound No. | GTP-KRAS-G12D:SOS1 $IC_{50}$ (nM) | GTP-KRAS-G12D:CRAF $IC_{50}$ (nM) | GTP-KRAS-WT:SOS1 $IC_{50}$ (nM) |
|---|---|---|---|
| **21a** | 7218 | | |
| **21b** | 7.213 | | |
| **22** | 13.4 | | |
| **23a** | 3689 | | |
| **23b** | 5.749 | | |
| **24** | 2065 | | |
| **25b** | 6.72 | | |
| **26a** | 246.9 | | |
| **26b** | 15.7 | | |
| **27b** | 17.24 | | |
| **28a** | 5303 | | |
| **28b** | 15.17 | | |
| **29b** | **15.7** | | |

**Effect example B: *In vivo* bioavailability experiment in mice**

Experimental reagents: acetonitrile, an HPLC-grade reagent, was produced by Honeywell; methanol, an HPLC-grade reagent, was supplied by Merck; formic acid (HCOOH), an HPLC-grade reagent, was produced by J&K Scientific; DMSO and Solutol were commercially available products; analytical-grade water was prepared from deionized water using a Milli-Q water purification system.

Experimental Instruments: Liquid chromatography-mass spectrometry (LC/MS/MS) analysis system composed of Waters ACQUITY UPLC coupled with a Sciex QTRAP® 6500+ mass spectrometer.

Experimental Animals: ICR mice, weighing 20 grams, provided by Sino-British SIPPR Lab Animal Ltd, Shanghai.

Experimental procedure:

1. Preparation of compound stock solution:

The compound to be tested was accurately weighed and dissolved in DMSO to prepare a stock solution with a concentration of 15 mg/mL.

2. Preparation of dosing solutions:

The compound stock solution was accurately measured and diluted with 0.9% saline and Solutol in a ratio of 8.8:1 to a concentration of 0.3 mg/mL. The solution was clear and transparent, used for intravenous administration. Additionally, the compound was accurately weighed and mixed with 0.5% CMC-Na to a concentration of 0.5 mg/mL, used for oral gavage administration.

Intravenous group: Three ICR mice, weighing 20 g ± 2 g, were intravenously administered the intravenous dosing solution. The dosing volume was 10 mL/kg, with a dose of 3 mg/kg. Blood samples (0.03 mL) were collected from the fundus vein of the mice at pre-dose and at 2, 15, 30, 60, 120, 240, 480, and 1440 minutes post-dose.

Oral gavage group: Three ICR mice, weighing 20 g ± 2 g, were administered the oral gavage dosing solution. The dosing volume was 20 mL/kg, with doses of 10 mg/kg and 30 mg/kg. Blood samples (0.03 mL) were collected from the fundus vein of the mice at pre-dose and at 5, 15, 30, 60, 120, 240, 480, and 1440 minutes post-dose.

The blood samples were centrifuged at 6800 g for 6 minutes at 2-8°C. The plasma was collected and stored in centrifuge tubes at -70°C for later analysis.

3. Plasma sample processing

3.1 Preparation of standard curve

The concentration range of the standard curve working solution was 20, 10, 4, 2, 1, 0.2, 0.1, 0.02 μg/mL.
A 19 μL aliquot of blank mouse plasma was taken, and 1 μL of the standard curve working solution was added to prepare a series of concentration samples at 1, 0.5, 0.2, 0.1, 0.05, 0.01, 0.005, and 0.001 μg/mL. The mixture was vortexed and mixed well, and 400 μL of methanol containing an internal standard (Warfarin, 100 ng/mL) was added to precipitate the proteins. The mixture was vortexed and shaken for 1 minute, centrifuged at 18000 g for 7 minutes at 4°C, and the supernatant was transferred to a 96-well plate for injection.

3.2 QC sample processing

The concentration range of the QC working solution was: Low: 0.06 μg/mL; Middle: 8 μg/mL; High: 16 μg/mL.
A 19 μL aliquot of blank mouse plasma was taken, and 1 μL of the standard curve working solution was added to prepare a series of concentration samples at 0.003, 0.4, and 0.8 μg/mL. The mixture was vortexed and mixed well, and 400 μL of methanol containing an internal standard (Warfarin, 100 ng/mL) was added to precipitate the proteins. The mixture was vortexed and shaken for 1 minute, centrifuged at 18000 g for 7 minutes at 4°C, and the supernatant was transferred to a 96-well plate for injection.

3.3 Plasma sample processing

A 20 μL aliquot of the plasma sample was mixed with 400 μL of methanol containing an internal standard (Warfarin, 100 ng/mL) to precipitate the proteins. The mixture was vortexed for 10 minutes, centrifuged at 18000 g for 7 minutes at 4°C, and 400 μL of the supernatant was transferred to a 96-well plate for a 10 μL injection.

4. Sample determination method

4.1 Instruments

Liquid chromatography system: Acquity UPLC system (including a binary solvent manager, sample manager, column heater, and degasser) from Waters Corporation, USA.
MS/MS system: QTRAP® 6500+ triple quadrupole mass spectrometer equipped with an electrospray ionization (ESI) source from Sciex Corporation, USA.
Data acquisition: Analyst 1.7.3 software from Sciex Corporation, USA.

4.2 Liquid chromatography conditions

Analytical column: BEH C18 column, 1.7 μm, 50 × 2.1 mm I.D., from Waters Corporation, USA.
Flow rate: 0.60 mL/min; injection volume: 10 μL; column temperature: 40°C. The gradient elution program used was as follows:

Table 3

| Time (min) | B%<br>0.1% Formic acid in ACN |
|---|---|
| 0.00 | 10 |
| 0.60 | 90 |
| 1.10 | 90 |
| 1.11 | 10 |
| 1.40 | 10 |

4.3 Mass spectrometry conditions

The ion source was an electrospray ionization source (Turbo Ionspray, ESI); the ion spray voltage was 5500V; the temperature was 500°C; the ion source gas 1 ($N_2$) pressure was 50 psi; the ion source gas 2 ($N_2$) pressure was 50 psi; the

curtain gas (N2) pressure was 20 psi; the collision gas pressure (CAD) was Medium; detection was performed in positive ion mode; the scan type was multiple reaction monitoring (MRM).

5. Experimental results as shown in Table 4

Table 4. Pharmacokinetic parameters of compounds 13a', 18b, and 25b in mice

| Parameter (unit) | Compound 13a' | | Compound 18b | | Compound 25b | |
|---|---|---|---|---|---|---|
| | Intravenous injection | Oral | Intravenous injection | Oral | Intravenous injection | Oral |
| Dose (mg/mL) | 3 | 30 | 3 | 30 | 3 | 30 |
| Maximum plasma concentration (ng/mL) | 3563.93 ± 329.57 | 781.43 ± 409.22 | 7632.72 ± 827.93 | 508.76 ± 182.77 | 22039.3 ± 5780.49 | 863.75 ± 450.55 |
| Area under curve (h) * (ng/mL) | 2062.35 ± 270.21 | 3091.66 ± 81.29 | 1944.73 ± 138.59 | 1904.95 ± 930.87 | 7940.26 ± 1436.49 | 2365.19 ± 1119.64 |
| Half-life (h) | 15.93 ± 9.48 | 4.68 ± 0.68 | 11.81 ± 1.98 | 3.57 ± 0.93 | 12.21 ± 4.12 | 3.81 ± 0.13 |
| Mean residence time (h) | 4.73 ± 0.46 | 4.53 ± 0.78 | 1.41 ±0.07 | 3.77 ± 0.45 | 1.01 ±0.06 | 3.15 ± 0.6 |
| Clearance (L/kg * h) | 0.39 ± 0.08 | | 1.47 ± 0.12 | | 0.38 ± 0.078 | |
| Steady-state volume of distribution (L/kg) | 5.33 ± 1.89 | | 4.98 ± 0.49 | | 0.84 ± 0.275 | |
| Bioavailability (%) | | 15 ± 0.39 | | 9.8 ± 4.79 | | 2.98 ± 1.41 |

**Effect example C: Evaluation of antitumor effect on Panc-1 (KRASG12D pancreatic cancer cell) CDX model**

Female Nu/Nu nude mice, approximately 8 to 10 weeks old, (Beijing Vital River Laboratory Animal Technology Co., Ltd) were used in these studies. The animals were maintained in individually ventilated cages with a 12-hour light/dark cycle. Food and water were available ad libitum. Panc-1 tumor cells were maintained *in vitro* at 37°C in an atmosphere containing 5% $CO_2$ using DMED medium supplemented with 10% fetal bovine serum. Prior to tumor inoculation, cells in the exponential growth phase were harvested and quantified using a cell counter. Panc-1 tumor cells ($1 \times 10^7$) in 0.2 mL PBS were subcutaneously inoculated into the right flank of each mouse. Randomization was initiated when the average tumor size reached approximately 154.21 $mm^3$. A total of 9 tumor-bearing mice were enrolled in the study, and these mice were allocated into 2 treatment groups (3 mice per group). Additionally, 3 non-tumor-bearing mice were assigned to the untreated group.

The animals were treated twice daily via intravenous injection (I.V.) with either a vehicle (10% HP-β-CD) or the vehicle containing the indicated dose of the compound, for approximately 28 days (QD × 28 I.V), or until ethical endpoints were reached (BWL > 20%; median tumor volume (MTV) > 2000 $mm^3$; individual TV > 3000 $mm^3$; clinical signs of discomfort). The clinical signs of discomfort were characterized, but not limited to the following: severe dehydration, hypothermia, abnormal/labored breathing, lethargy, evident pain, diarrhea, skin lesions, neurological symptoms, impaired mobility (inability to eat or drink) due to significant ascites and abdominal enlargement, inability to stand, persistent prone or lateral recumbency, signs of muscle atrophy, paralyzed gait, clonic convulsions, tonic convulsions, and continuous bleeding from body orifices. Drug administration and tumor and body weight measurements were conducted in a laminar flow cabinet.

Tumor volume was measured in two dimensions using calipers twice weekly after randomization, and the volume was calculated in $mm^3$ using the formula: $V = (L \times W \times W) \times 0.5$, where V is the tumor volume, L is the tumor length (the longest tumor dimension), and W is the tumor width (the longest tumor dimension perpendicular to L). Results were presented as median tumor volume per group (in $mm^3$ ± interquartile range). Tumor growth inhibition (TGI%) was calculated using the formula: TGI% = [1 - (Ti/Ci)] × 100, where Ti is the median tumor volume in the treatment group on the measurement day, and Ci is the median tumor volume in the control group on the measurement day.

Table 5 illustrates the results of this study.

Table 5: Panc-1 KRASG12D in Nu/Nu

| Drug | Dose (mg/kg QD IV) | TGI (%) Day 28 | BWL (%) Day 14 | BWL (%) Day 28 |
|---|---|---|---|---|
| Vehicle | 0 | n/a | -0.29 | -1.22 |
| Compound 5a | 10 | 96 | -0.77 | -1.58 |

## Claims

**1.** A compound of formula I, formula II, formula III, or formula IV, a pharmaceutically acceptable salt thereof, a solvate thereof, a stereoisomer thereof, a tautomer thereof, a prodrug thereof, a metabolite thereof, or an isotopic compound thereof:

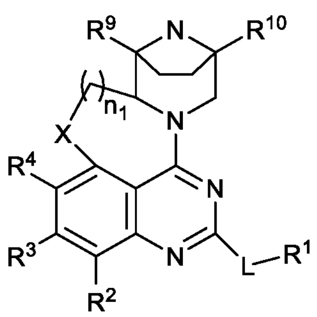

I ,

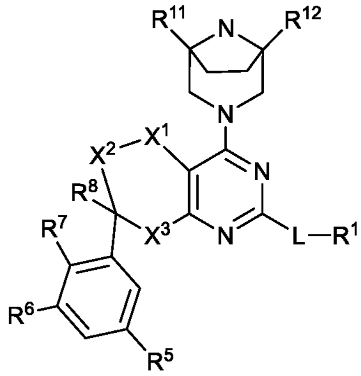

II ,

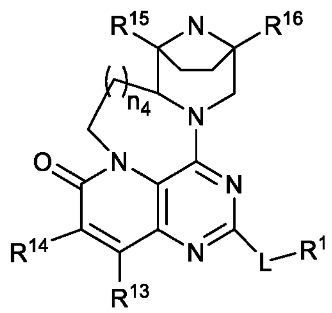

III ,

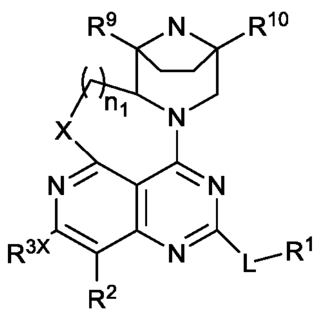

IV ,

wherein formula I, II, or III satisfies the following situation 1 or situation 2:

**situation 1:**

X is N, O, or S;

n1 and n4 are each independently 1, 2, 3, or 4;

each L is independently $-O-(CR^{L-1}R^{L-2})_{n2}-*$, $-(CR^{L-3}R^{L-4})_{n3}-*$, or

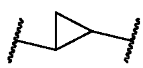

;

* represents one end connected to $R^1$;

n2 and n3 are each independently 0, 1, 2, 3, or 4;

$R^{L-1}$, $R^{L-2}$, $R^{L-3}$, and $R^{L-4}$ are each independently H, $C_1$-$C_6$ alkyl, $C_1$-$C_6$ alkoxy, $C_1$-$C_6$ alkyl substituted by one or more $R^{L-1-1}$, or halogen;

each $R^{L-1-1}$ is independently halogen or $C_1$-$C_6$ alkoxy;
each $R^1$ is 4- to 10-membered heterocycloalkyl substituted by one or more $R^{1-1}$; heteroatoms in the 4- to 10-membered heterocycloalkyl of the 4- to 10-membered heterocycloalkyl substituted by one or more $R^{1-1}$ are independently 1, 2, or 3 kinds of N, O, or S, and the number of heteroatoms is 1, 2, or 3;
each $R^{1-1}$ is independently halogen;
$R^2$ and $R^{13}$ are each independently H or halogen;
$R^3$ and $R^{14}$ are each independently $C_6$-$C_{10}$ aryl, $C_6$-$C_{10}$ aryl substituted by one or more $R^{3-1}$, 5- to 10-membered heteroaryl, or 5- to 10-membered heteroaryl substituted by one or more $R^{3-2}$; heteroatoms in the 5- to 10-membered heteroaryl and the 5- to 10-membered heteroaryl substituted by one or more $R^{3-2}$ are independently 1, 2, or 3 kinds of N, O, or S, and the number of heteroatoms is 1, 2, or 3;
$R^{3-1}$ and $R^{3-2}$ are each independently OH, halogen, $C_1$-$C_6$ alkyl, $C_1$-$C_6$ alkyl substituted by one or more $R^{3-1-1}$, $C_2$-$C_6$ alkynyl, 3- to 8-membered cycloalkyl, -S-C($R^{3-1-2}$)$_3$, -S($R^{3-1-3}$)$_5$, amino, $C_1$-$C_6$ alkyl, or 5- to 10-membered heteroaryl;
alternatively, any two adjacent $R^{3-1}$, together with the carbon atom to which they are attached, form a 5- to 10-membered heteroaryl group or a 5- to 10-membered heteroaryl group substituted by one or more $R^{3-1-4}$; heteroatoms in the 5- to 10-membered heteroaryl group and the 5- to 10-membered heteroaryl group substituted by one or more $R^{3-1-4}$ are independently 1, 2, or 3 kinds of N, O, or S, and the number of heteroatoms is 1, 2, or 3;
each $R^{3-1-1}$ is independently oxo (=O), OH, $C_1$-$C_6$ alkoxy, or halogen;
$R^{3-1-2}$ and $R^{3-1-3}$ are each independently halogen;
each $R^{3-1-4}$ is independently $C_1$-$C_6$ alkyl;
$R^4$ is H, OH, $C_1$-$C_6$ alkyl, $C_1$-$C_6$ alkoxy, $C_1$-$C_6$ alkyl substituted by one or more $R^{4-1}$, cyano, or halogen;
each $R^{4-1}$ is independently halogen;
$X^1$ is C($R^{1a}R^{1b}$) or O;
$X^2$ is C($R^{2a}R^{2b}$) or O;
$X^3$ is C($R^{3a}R^{3b}$) or O;
$R^{1a}$, $R^{1b}$, $R^{2a}$, $R^{2b}$, $R^{3a}$, and $R^{3b}$ are each independently H, $C_1$-$C_6$ alkyl, or halogen;
$R^5$ is H or OH;
$R^6$ is H, $C_1$-$C_6$ alkyl, $C_1$-$C_6$ alkoxy, 3- to 8-membered cycloalkyl, halogen, or $C_1$-$C_6$ alkyl substituted by one or more $R^{6-1}$;
each $R^{6-1}$ is independently halogen;
$R^7$ and $R^8$ are connected to form ring A, and ring A is a 5- to 6-membered saturated or unsaturated monocyclic, spirocyclic, or fused carbocyclic ring, a 5- to 6-membered saturated or unsaturated monocyclic, spirocyclic, or fused carbocyclic ring substituted by one or more $R^{7-1}$, a 5- to 6-membered saturated or unsaturated monocyclic, spirocyclic, or fused heterocyclic ring with 1, 2, or 3 heteroatoms selected from 1, 2, or 3 kinds of N, O, and S, or a 5- to 6-membered saturated or unsaturated monocyclic, spirocyclic, or fused heterocyclic ring with 1, 2, or 3 heteroatoms selected from 1, 2, or 3 kinds of N, O, and S substituted by one or more $R^{7-2}$;
$R^{7-1}$ and $R^{7-2}$ are each independently $C_1$-$C_6$ alkyl, oxo, or halogen;
$R^9$, $R^{10}$, $R^{11}$, $R^{12}$, $R^{15}$, and $R^{16}$ are each independently H, $C_1$-$C_6$ alkyl, or halogen;

**situation 2:**

X is N, O, or S;
n1 and n4 are independently 1, 2, 3, or 4;
each L is independently -O-($CR^{L-1}R^{L-2}$)$_{n2}$-*, -($CR^{L-3}R^{L-4}$)$_{n3}$-*, or

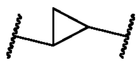;

* represents one end connected to $R^1$;
n2 and n3 are each independently 0, 1, 2, 3, or 4;
$R^{L-1}$, $R^{L-2}$, $R^{L-3}$, and $R^{L-4}$ are each independently H, $C_1$-$C_6$ alkyl, $C_1$-$C_6$ alkoxy, $C_1$-$C_6$ alkyl substituted by one or more $R^{L-1-1}$, or halogen;
each $R^{L-1-1}$ is independently halogen or $C_1$-$C_6$ alkoxy;
each $R^1$ is 4- to 10-membered heterocycloalkyl substituted by one or more $R^{1-1}$; heteroatoms in the 4- to 10-membered heterocycloalkyl of the 4- to 10-membered heterocycloalkyl substituted by one or more $R^{1-1}$ are

independently 1, 2, or 3 kinds of N, O, or S, and the number of heteroatoms is 1, 2, or 3;
each $R^{1-1}$ is independently halogen, hydroxyl, -O-$C_1$-$C_6$ alkyl, $C_1$-$C_6$ alkyl, or $C_1$-$C_6$ alkyl substituted by one or more $R^{1-1-1}$;
each $R^{1-1-1}$ is independently hydroxyl, -O-$C_1$-$C_6$ alkyl, 4- to 10-membered heterocycloalkyl, or 4- to 10-membered heterocycloalkyl substituted by one or more $R^{1-1-1-1}$; heteroatoms in the 4-to 10-membered heterocycloalkyl and the 4- to 10-membered heterocycloalkyl substituted by one or more $R^{1-1-1-1}$ are independently 1, 2, or 3 kinds of N, O, or S, and the number of heteroatoms is 1, 2, or 3;
each $R^{1-1-1-1}$ is independently $C_1$-$C_6$ alkyl;
$R^2$ and $R^{13}$ are each independently H or halogen;
$R^3$ is

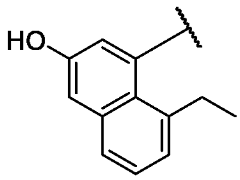 , 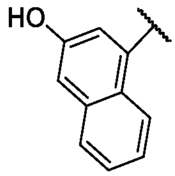 , 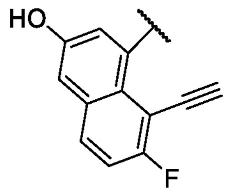 , 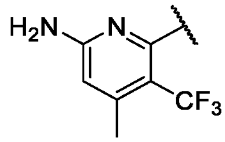 ,

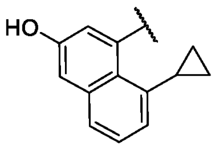 , 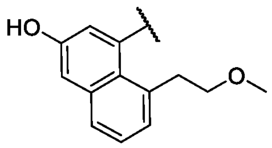 , 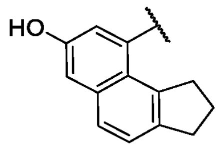 , 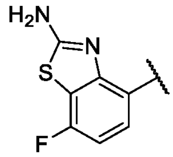 ,

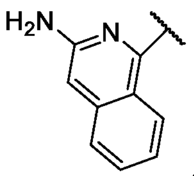 , or 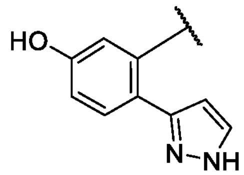 ;

when $R^3$ is

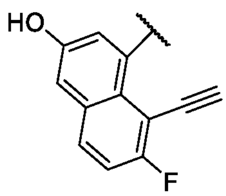 or 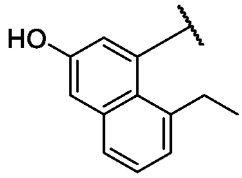 ,

R4 is F;
each $R^{14}$ is independently $C_6$-$C_{10}$ aryl, $C_6$-$C_{10}$ aryl substituted by one or more $R^{3-1}$, 5- to 10-membered heteroaryl, or 5- to 10-membered heteroaryl substituted by one or more $R^{3-2}$; heteroatoms in the 5- to 10-membered heteroaryl and the 5- to 10-membered heteroaryl substituted by one or more $R^{3-2}$ are independently 1, 2, or 3 kinds of N, O, or S, and the number of heteroatoms is 1, 2, or 3;
$R^{3-1}$ and $R^{3-2}$ are each independently OH, halogen, $C_1$-$C_6$ alkyl, $C_1$-$C_6$ alkyl substituted by one or more $R^{3-1-1}$, $C_2$-$C_6$ alkynyl, 3- to 8-membered cycloalkyl, -S-C($R^{3-1-2}$)$_3$, -S($R^{3-1-3}$)$_5$, amino, $C_1$-$C_6$ alkyl, 5- to 10-membered heteroaryl, 5- to 10-membered heteroaryl substituted by one or more $R^{3-1-4}$, or -O-$C_1$-$C_6$ alkyl; heteroatoms in the 5- to 10-membered heteroaryl and the 5- to 10-membered heteroaryl substituted by one or more $R^{3-1-4}$ are independently 1, 2, or 3 kinds of N, O, or S, and the number of heteroatoms is 1, 2, or 3;
alternatively, any two adjacent $R^{3-1}$, together with the carbon atom to which they are attached, form a 5- to 6-membered carbocyclic ring, a 5- to 6-membered carbocyclic ring substituted by one or more $R^{3-1-4}$ a 5- to 6-membered heterocyclic ring, or a 5- to 6-membered heterocyclic ring substituted by one or more $R^{3-1-4}$; heteroatoms in the 5- to 6-membered heterocyclic ring and the 5- to 6-membered heterocyclic ring substituted by one or more $R^{3-1-4}$ are independently 1, 2, or 3 kinds of N, O, or S, and the number of heteroatoms is 1, 2, or 3;

each $R^{3-1-1}$ is independently oxo (=O), OH, $C_1$-$C_6$ alkoxy, or halogen;
$R^{3-1-2}$ and $R^{3-1-3}$ are each independently halogen;
each $R^{3-1-4}$ is independently $C_1$-$C_6$ alkyl;
$R^4$ is H, OH, $C_1$-$C_6$ alkyl, $C_1$-$C_6$ alkoxy, $C_1$-$C_6$ alkyl substituted by one or more $R^{4-1}$, cyano, or F;
each $R^{4-1}$ is independently halogen;
$X^1$ is $C(R^{1a}R^{1b})$ or O;
$X^2$ is $C(R^{2a}R^{2b})$ or O;
$X^3$ is $C(R^{3a}R^{3b})$ or O;
$R^{1a}$, $R^{1b}$, $R^{2a}$ , $R^{2b}$, $R^{3a}$, and $R^{3b}$ are each independently H, $C_1$-$C_6$ alkyl, or halogen;
$R^5$ is H or OH;
$R^6$ is H, $C_1$-$C_6$ alkyl, $C_1$-$C_6$ alkoxy, 3- to 8-membered cycloalkyl, halogen, or $C_1$-$C_6$ alkyl substituted by one or more $R^{6-1}$;
each $R^{6-1}$ is independently halogen;
$R^7$ and $R^8$ are connected to form ring A, and ring A is a 5- to 6-membered saturated or unsaturated monocyclic, spirocyclic, or fused carbocyclic ring, a 5- to 6-membered saturated or unsaturated monocyclic, spirocyclic, or fused carbocyclic ring substituted by one or more $R^{7-1}$, a 5- to 6-membered saturated or unsaturated monocyclic, spirocyclic, or fused heterocyclic ring with 1, 2, or 3 heteroatoms selected from 1, 2, or 3 kinds of N, O, and S, or a 5- to 6-membered saturated or unsaturated monocyclic, spirocyclic, or fused heterocyclic ring with 1, 2, or 3 heteroatoms selected from 1, 2, or 3 kinds of N, O, and S substituted by one or more $R^{7-2}$;
$R^{7-1}$ and $R^{7-2}$ are each independently $C_1$-$C_6$ alkyl, oxo, or halogen;
$R^9$, $R^{10}$, $R^{11}$, $R^{12}$, $R^{15}$, and $R^{16}$ are each independently H, $C_1$-$C_6$ alkyl, or halogen;
in formula IV,
X is N, O, or S;
each n1 is independently 1, 2, 3, or 4;
each L is independently $-O-(CR^{L-1}R^{L-2})_{n2}-*$, $-(CR^{L-3}R^{L-4})_{n3}-*$, or

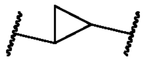
;

* represents one end connected to $R^1$;
n2 and n3 are each independently 0, 1, 2, 3, or 4;
$R^{L-1}$, $R^{L-2}$, $R^{L-3}$, and $R^{L-4}$ are each independently H, $C_1$-$C_6$ alkyl, $C_1$-$C_6$ alkoxy, $C_1$-$C_6$ alkyl substituted by one or more $R^{L-1-1}$, or halogen;
each $R^{L-1-1}$ is independently halogen or $C_1$-$C_6$ alkoxy;
each $R^1$ is 4- to 10-membered heterocycloalkyl substituted by one or more $R^{1-1}$; heteroatoms in the 4- to 10-membered heterocycloalkyl of the 4- to 10-membered heterocycloalkyl substituted by one or more $R^{1-1}$ are independently 1, 2, or 3 kinds of N, O, or S, and the number of heteroatoms is 1, 2, or 3;
each $R^{1-1}$ is independently halogen, hydroxyl, $-O-C_1$-$C_6$ alkyl, $C_1$-$C_6$ alkyl, or $C_1$-$C_6$ alkyl substituted by one or more $R^{1-1-1}$;
each $R^{1-1-1}$ is independently hydroxyl, $-O-C_1$-$C_6$ alkyl, 4- to 10-membered heterocycloalkyl, or 4- to 10-membered heterocycloalkyl substituted by one or more $R^{1-1-1-1}$; heteroatoms in the 4-to 10-membered heterocycloalkyl and the 4- to 10-membered heterocycloalkyl substituted by one or more $R^{1-1-1-1}$ are independently 1, 2, or 3 kinds of N, O, or S, and the number of heteroatoms is 1, 2, or 3;
each $R^{1-1-1-1}$ is independently $C_1$-$C_6$ alkyl;
$R^2$ and $R^{13}$ are each independently H or halogen;
$R^{3X}$ is 5- to 10-membered heteroaryl or 5- to 10-membered heteroaryl substituted by one or more $R^{3X-1}$; heteroatoms in the 5- to 10-membered heteroaryl and the 5- to 10-membered heteroaryl substituted by one or more $R^{3X-1}$ are independently 1, 2, or 3 kinds of N, O, or S, and the number of heteroatoms is 1, 2, or 3;
each $R^{3X-1}$ is independently $C_1$-$C_6$ alkyl, amino, or $C_1$-$C_6$ alkyl substituted by one or more halogens;
$R^9$ and $R^{10}$ are each independently H, $C_1$-$C_6$ alkyl, or halogen.

2. The compound of formula I, formula II, formula III, or formula IV, the pharmaceutically acceptable salt thereof, the solvate thereof, the stereoisomer thereof, the tautomer thereof, the prodrug thereof, the metabolite thereof, or the isotopic compound thereof according to claim 1, wherein
in the situation 1, the compound of formula I, formula II, or formula III, the pharmaceutically acceptable salt thereof, the solvate thereof, the stereoisomer thereof, the tautomer thereof, the prodrug thereof, the metabolite thereof, or the

isotopic compound thereof satisfies one or more of the following conditions:

(1) each $R^1$ is also 11-membered heterocycloalkyl substituted by one or more $R^{1-1}$; heteroatoms in the 11-membered heterocycloalkyl of the 11-membered heterocycloalkyl substituted by one or more $R^{1-1}$ are independently 1, 2, or 3 kinds of N, O, or S, and the number of heteroatoms is 1, 2, or 3;
(2) each $R^{1-1}$ is also independently hydroxyl, -O-$C_1$-$C_6$ alkyl, $C_1$-$C_6$ alkyl, or $C_1$-$C_6$ alkyl substituted by one or more $R^{1-1-1}$;

each $R^{1-1-1}$ is independently hydroxyl, -O-$C_1$-$C_6$ alkyl, 4- to 10-membered heterocycloalkyl, or 4- to 10-membered heterocycloalkyl substituted by one or more $R^{1-1-1-1}$; heteroatoms in the 4-to 10-membered heterocycloalkyl and the 4- to 10-membered heterocycloalkyl substituted by one or more $R^{1-1-1-1}$ are independently 1, 2, or 3 kinds of N, O, or S, and the number of heteroatoms is 1, 2, or 3;
each $R^{1-1-1-1}$ is independently $C_1$-$C_6$ alkyl;

(3) $R^{3-1}$ and $R^{3-2}$ are also each independently 5- to 10-membered heteroaryl substituted by one or more $R^{3-1-4}$ or -O-$C_1$-$C_6$ alkyl; heteroatoms in the "5- to 10-membered heteroaryl substituted by one or more $R^{3-1-4}$" are independently 1, 2, or 3 kinds of N, O, or S, and the number of heteroatoms is 1, 2, or 3;

alternatively, any two adjacent $R^{3-1}$, together with the carbon atom to which they are attached, form a 5- to 6-membered carbocyclic ring, a 5- to 6-membered carbocyclic ring substituted by one or more $R^{3-1-4}$ a 5- to 6-membered heterocyclic ring, or a 5- to 6-membered heterocyclic ring substituted by one or more $R^{3-1-4}$; heteroatoms in the "5- to 6-membered heterocyclic ring" and the "5- to 6-membered heterocyclic ring substituted by one or more $R^{3-1-4}$" are independently 1, 2, or 3 kinds of N, O, or S, and the number of heteroatoms is 1, 2, or 3.

**3.** The compound of formula I, formula II, formula III, or formula IV, the pharmaceutically acceptable salt thereof, the solvate thereof, the stereoisomer thereof, the tautomer thereof, the prodrug thereof, the metabolite thereof, or the isotopic compound thereof according to claim 1 or 2, wherein the compound of formula I, formula II, or formula III, the pharmaceutically acceptable salt thereof, the solvate thereof, the stereoisomer thereof, the tautomer thereof, the prodrug thereof, the metabolite thereof, or the isotopic compound thereof satisfies one or more of the following conditions:

(1) X is O;
(2) n1 is 1 or 2; preferably 1;
(3) n2 and n3 are each independently 1 or 2;
(4) $R^{L-1}$, $R^{L-2}$, $R^{L-3}$, and $R^{L-4}$ are each independently H, $C_1$-$C_6$ alkyl substituted by one or more $R^{L-1-1}$, or halogen;
(5) each $R^{L-1-1}$ is independently $C_1$-$C_6$ alkoxy;
(6) $R^2$ is halogen;
(7) $R^3$ is $C_6$-$C_{10}$ aryl substituted by one or more $R^{3-1}$ or 5- to 10-membered heteroaryl substituted by one or more $R^{3-2}$; preferably $C_6$-$C_{10}$ aryl substituted by one or more $R^{3-1}$;
(8) each $R^{3-1}$ is independently 5- to 10-membered heteroaryl, -O-$C_1$-$C_6$ alkyl, $C_1$-$C_6$ alkyl substituted by one or more $R^{3-1-1}$, 3- to 8-membered cycloalkyl, OH, halogen, $C_1$-$C_6$ alkyl, or $C_2$-$C_6$ alkynyl; preferably OH, halogen, $C_1$-$C_6$ alkyl, or $C_2$-$C_6$ alkynyl;
(9) $R^4$ is H, halogen, cyano, OH, $C_1$-$C_6$ alkoxy, or $C_1$-$C_6$ alkyl substituted by one or more $R^{4-1}$;
(10) $X^1$ is $C(R^{1a}R^{1b})$;
(11) $X^2$ is $C(R^{2a}R^{2b})$;
(12) $X^3$ is $C(R^{3a}R^{3b})$;
(13) $R^{1a}$, $R^{1b}$, $R^{2a}$, $R^{2b}$, $R^{3a}$, and $R^{3b}$ are each independently H or halogen;
(14) $R^5$ is OH;
(15) $R^6$ is H, halogen, $C_1$-$C_6$ alkyl, $C_1$-$C_6$ alkyl substituted by one or more $R^{6-1}$, or 3- to 8-membered cycloalkyl;
(16) ring A is a 5- to 6-membered saturated or unsaturated monocyclic, spirocyclic, or fused carbocyclic ring, a 5- to 6-membered saturated or unsaturated monocyclic, spirocyclic, or fused carbocyclic ring substituted by one or more $R^{7-1}$, or a 5- to 6-membered saturated or unsaturated monocyclic, spirocyclic, or fused heterocyclic ring with 1, 2, or 3 heteroatoms selected from 1, 2, or 3 kinds of N, O, and S;
(17) $R^9$, $R^{10}$, $R^{11}$, and $R^{12}$ are each independently H or $C_1$-$C_6$ alkyl;
(18) each $R^{3-2}$ is independently $C_1$-$C_6$ alkyl, amino, halogen, or $C_1$-$C_6$ alkyl substituted by one or more $R^{3-1-1}$;
(19) each $R^{3-1-1}$ is independently $C_1$-$C_6$ alkoxy or halogen.

**4.** The compound of formula I, formula II, formula III, or formula IV, the pharmaceutically acceptable salt thereof, the

solvate thereof, the stereoisomer thereof, the tautomer thereof, the prodrug thereof, the metabolite thereof, or the isotopic compound thereof according to claim 1 or 2, wherein the compound of formula I, formula II, or formula III, the pharmaceutically acceptable salt thereof, the solvate thereof, the stereoisomer thereof, the tautomer thereof, the prodrug thereof, the metabolite thereof, or the isotopic compound thereof satisfies one or more of the following conditions:

(1) $R^{L-1}$, $R^{L-2}$, $R^{L-3}$, and $R^{L-4}$ are each independently H;
(2) $R^4$ is H or halogen;
(3) $R^{1a}$, $R^{1b}$, $R^{2a}$, $R^{2b}$, $R^{3a}$, and $R^{3b}$ are each independently H;
(4) $R^6$ is halogen;
(5) ring A is a 5- to 6-membered saturated or unsaturated monocyclic carbocyclic ring.

**5.** The compound of formula I, formula II, formula III, or formula IV, the pharmaceutically acceptable salt thereof, the solvate thereof, the stereoisomer thereof, the tautomer thereof, the prodrug thereof, the metabolite thereof, or the isotopic compound thereof according to claim 1, wherein

in formula I, X is O;
n1 is 1;
$R^1$ is 4- to 10-membered heterocycloalkyl substituted by one or more $R^{1-1}$;
each $R^{1-1}$ is independently halogen;
$R^2$ is halogen;
$R^4$ is H or halogen;
$R^3$ is $C_6$-$C_{10}$ aryl substituted by one or more $R^{3-1}$;
each $R^{3-1}$ is independently OH, halogen, $C_1$-$C_6$ alkyl, or $C_2$-$C_6$ alkynyl;
L is $-O-(CR^{L-1}R^{L-2})_{n2}-*$;
$R^{L-1}$ or $R^{L-2}$ are each independently H;
n2 is 1;
$R^9$ and $R^{10}$ are each independently H;
in formula II, $X^1$ is $C(R^{1a}R^{1b})$ or O;
$X^2$ is $C(R^{2a}R^{2b})$ or O;
$X^3$ is $C(R^{3a}R^{3b})$ or O;
$R^{1a}$, $R^{1b}$, $R^{2a}$, $R^{2b}$, $R^{3a}$, and $R^{3b}$ are each independently H or halogen;
L is $-O-(CR^{L-1}R^{L-2})_{n2}-*$, $-(CR^{L-3}R^{L-4})_{n3}-*$, or

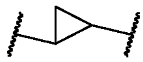;

* represents one end connected to $R^1$;
n2 and n3 are each independently 1 or 2;
$R^{L-1}$, $R^{L-2}$, $R^{L-3}$, and $R^{L-4}$ are each independently H, $C_1$-$C_6$ alkyl substituted by one or more $R^{L-1-1}$, or halogen;
each $R^{L-1-1}$ is independently $C_1$-$C_6$ alkoxy;
$R^1$ is 4- to 10-membered heterocycloalkyl substituted by one or more $R^{1-1}$;
each $R^{1-1}$ is independently halogen;
$R^5$ is H or OH;
$R^6$ is H, $C_1$-$C_6$ alkyl, $C_1$-$C_6$ alkoxy, 3- to 8-membered cycloalkyl, halogen, or $C_1$-$C_6$ alkyl substituted by one or more $R^{6-1}$;
each $R^{6-1}$ is independently halogen;
$R^7$ and $R^8$ are connected to form ring A, and ring A is a 5- to 6-membered saturated or unsaturated monocyclic, spirocyclic, or fused carbocyclic ring, a 5- to 6-membered saturated or unsaturated monocyclic, spirocyclic, or fused carbocyclic ring substituted by one or more $R^{7-1}$, or a 5- to 6-membered saturated or unsaturated monocyclic, spirocyclic, or fused heterocyclic ring with 1, 2, or 3 heteroatoms selected from 1, 2, or 3 kinds of N, O, and S;
each $R^{7-1}$ is independently $C_1$-$C_6$ alkyl, oxo, or halogen;
$R^{11}$ and $R^{12}$ are each independently H, $C_1$-$C_6$ alkyl, or halogen.

**6.** The compound of formula I, formula II, formula III, or formula IV, the pharmaceutically acceptable salt thereof, the solvate thereof, the stereoisomer thereof, the tautomer thereof, the prodrug thereof, the metabolite thereof, or the

isotopic compound thereof according to claim 5, wherein

$X^1$ is $C(R^{1a}R^{1b})$;
$X^2$ is $C(R^{2a}R^{2b})$;
$X^3$ is $C(R^{3a}R^{3b})$;
$R^{1a}$, $R^{1b}$, $R^{2a}$, $R^{2b}$, $R^{3a}$, and $R^{3b}$ are each independently H or halogen;
$R^1$ is 4- to 10-membered heterocycloalkyl substituted by one or more $R^{1-1}$;
each $R^{1-1}$ is independently halogen;
L is $-O-(CR^{L-1}R^{L-2})_{n2}-*$;
$R^{L-1}$ or $R^{L-2}$ are each independently H;
n2 is 1;
$R^5$ is OH;
$R^6$ is halogen;
ring A is a 5-membered saturated monocyclic carbocyclic ring;
$R^{11}$ and $R^{12}$ are each independently H.

**7.** The compound of formula I, formula II, formula III, or formula IV, the pharmaceutically acceptable salt thereof, the solvate thereof, the stereoisomer thereof, the tautomer thereof, the prodrug thereof, the metabolite thereof, or the isotopic compound thereof according to any one of claims 1 to 6, wherein the compound of formula I, formula II, formula III, or formula IV, the pharmaceutically acceptable salt thereof, the solvate thereof, the stereoisomer thereof, the tautomer thereof, the prodrug thereof, the metabolite thereof, or the isotopic compound thereof satisfies one or more of the following conditions:

(1) in $R^1$, the "4- to 10-membered heterocycloalkyl" in the "4- to 10-membered heterocycloalkyl substituted by one or more $R^{1-1}$" is 8- to 10-membered heterocycloalkyl containing an N atom, and may also be bicyclo[3.3.0] heterooctyl containing an N atom, for example,

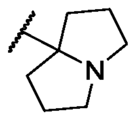
;

(2) in $R^4$, $R^6$, $R^{3-1}$, $R^{L-1}$, $R^{L-2}$, $R^{L-3}$, $R^{L-4}$, $R^{7-1}$, $R^{7-2}$, $R^9$, $R^{10}$, $R^{11}$, $R^{12}$, $R^{1-1}$, $R^{1-1-1}$, $R^{1-1-1-1}$, $R^{3-1-4}$, $R^{1a}$, $R^{1b}$, $R^{2a}$, $R^{2b}$, $R^{3a}$, $R^{3b}$, $R^{15}$, $R^{16}$, $R^{3X-1}$, and $R^{3-2}$ , each "$C_1$-$C_6$ alkyl" in the "$C_1$-$C_6$ alkyl", "$C_1$-$C_6$ alkyl substituted by one or more $R^{4-1}$", "$C_1$-$C_6$ alkyl substituted by one or more $R^{3-1-1}$", "$C_1$-$C_6$ alkyl substituted by one or more $R^{L-1-1}$", "-O-$C_1$-$C_6$ alkyl", "$C_1$-$C_6$ alkyl substituted by one or more $R^{1-1-1}$", "5- to 10-membered heteroaryl substituted by $C_1$-$C_6$ alkyl", "$C_1$-$C_6$ alkyl substituted by one or more $R^{6-1}$", and "$C_1$-$C_6$ alkyl substituted by one or more halogens" is independently methyl, ethyl, n-propyl, isopropyl, n-butyl, isobutyl, or tert-butyl; preferably methyl or ethyl;
(3) in $R^{1a}$, $R^{1b}$, $R^{2a}$, $R^{2b}$, $R^{3a}$, $R^{3b}$, $R^{1-1}$, $R^2$, $R^4$, $R^6$, $R^{3-1}$, $R^{3-1-1}$, $R^{3-1-2}$, $R^{3-1-3}$, $R^{4-1}$, $R^{6-1}$ , $R^{L-1}$, $R^{L-2}$, $R^{L-3}$, $R^{L-4}$, $R^{L-1-1}$, $R^{7-1}$, $R^{7-2}$, $R^9$, $R^{10}$, $R^{11}$, $R^{13}$, $R^{3-2}$, $R^{15}$, $R^{16}$, $R^{3X-1}$, and $R^{12}$, each halogen is independently fluorine, chlorine, bromine, or iodine, preferably fluorine or chlorine;
(4) in $R^4$, $R^6$, $R^{3-1-1}$, $R^{L-1}$, $R^{L-2}$, $R^{L-3}$, $R^{L-4}$, and $R^{L-1-1}$, each "$C_1$-$C_6$ alkoxy" is independently methoxy, ethoxy, *n*-propoxy, isopropoxy, *n*-butoxy, isobutoxy, or *tert*-butoxy; preferably methoxy or ethoxy;
(5) in $R^6$, $R^{3-2}$, and $R^{3-1}$, each 3- to 8-membered cycloalkyl is independently cyclopropyl, cyclobutyl, cyclopentyl, or cyclohexyl, preferably cyclopropyl or cyclobutyl;
(6) in $R^{3-1}$ and $R^{3-2}$, the "$C_2$-$C_6$ alkynyl" is $C_2$-$C_4$ alkynyl, preferably ethynyl;
(7) in $R^3$ and $R^{14}$, each "$C_6$-$C_{10}$ aryl" in the "$C_6$-$C_{10}$ aryl" and "$C_6$-$C_{10}$ aryl substituted by one or more $R^{3-1}$" is independently phenyl or naphthyl, preferably naphthyl;
(8) in $R^3$, $R^{3-1}$, $R^{3-2}$, and $R^1$, each "5- to 10-membered heteroaryl" in the "5- to 10-membered heteroaryl", 5- to 10-membered heteroaryl substituted by $C_1$-$C_6$ alkyl, and "5- to 10-membered heteroaryl substituted by one or more $R^{3-2}$" is independently 9- to 10-membered heteroaryl;
(9) in $R^{1-1-1}$, the 4- to 10-membered heterocycloalkyl in the 4- to 10-membered heterocycloalkyl and the "4- to 10-membered heterocycloalkyl substituted by one or more $R^{1-1-1-1}$" is independently 5- to 6-membered monocyclic heterocycloalkyl, heteroatoms are N and/or O, and the number is 1 or 2; the 4- to 10-membered heterocycloalkyl may also be piperidinyl, piperazinyl, or morpholinyl, and further may be

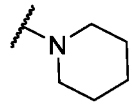, 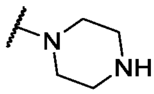, or 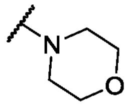;

(10) in $R^{1-1-1}$, two $R^{1-1-1}$ attached to the same carbon atom, together with the carbon atom to which they are attached, form a 3- to 8-membered cycloalkyl group, which is cyclopropyl, cyclobutyl, cyclopentyl, or cyclohexyl;

(11) in $R^{3X}$, each "5- to 10-membered heteroaryl" in the "5- to 10-membered heteroaryl" and the "5- to 10-membered heteroaryl substituted by one or more $R^{3X-1}$" is independently pyridyl;

(12) when $R^3$ and $R^{14}$ are each independently "$C_6$-$C_{10}$ aryl substituted by one or more $R^{3-1}$", and any two adjacent $R^{3-1}$, together with the carbon atom to which they are attached, form a 5- to 10-membered heteroaryl group or a "5- to 10-membered heteroaryl group substituted by one or more $R^{3-1-4}$", then the $R^3$ and $R^{14}$ are each independently

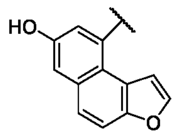

**8.** The compound of formula I, formula II, formula III, or formula IV, the pharmaceutically acceptable salt thereof, the solvate thereof, the stereoisomer thereof, the tautomer thereof, the prodrug thereof, the metabolite thereof, or the isotopic compound thereof according to claim 1 or 2, wherein the compound of formula I, formula II, formula III, or formula IV, the pharmaceutically acceptable salt thereof, the solvate thereof, the stereoisomer thereof, the tautomer thereof, the prodrug thereof, the metabolite thereof, or the isotopic compound thereof satisfies one or more of the following conditions:

(1) -L-$R^1$ is

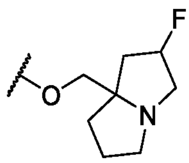, 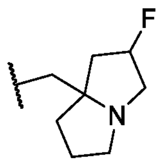, 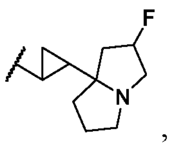, 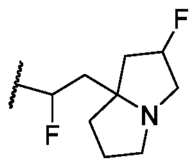, 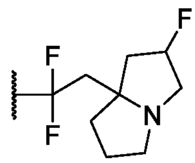,

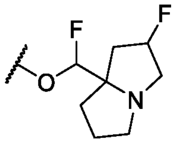, 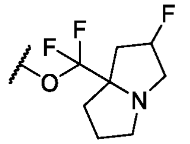, 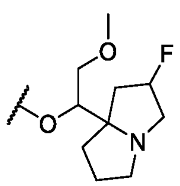, 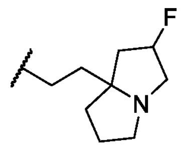, 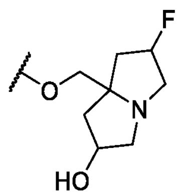,

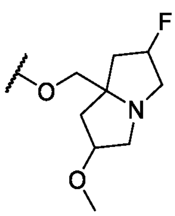, 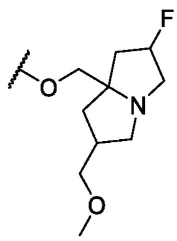, 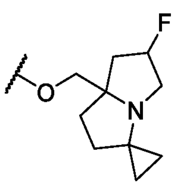, 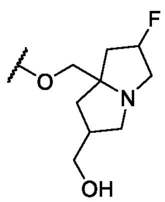, 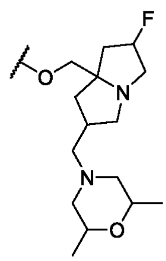, 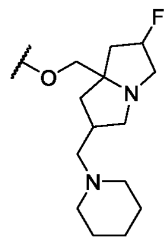,

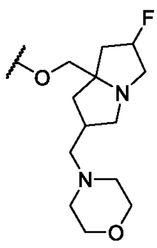, 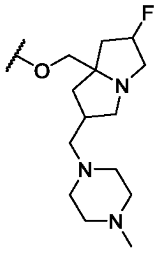, 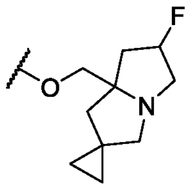, 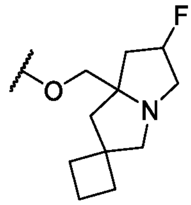, or 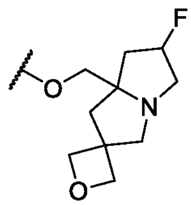,

preferably

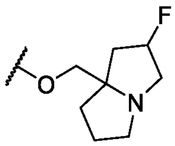, 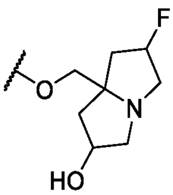, 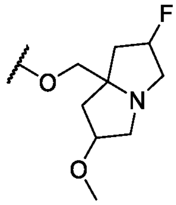, 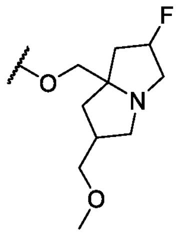, 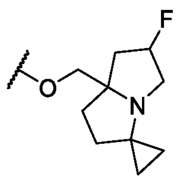,

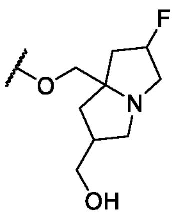, 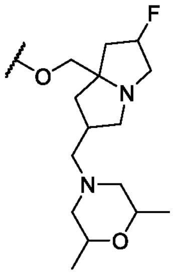, 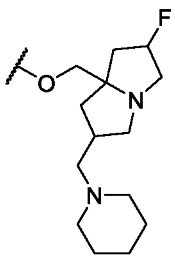, 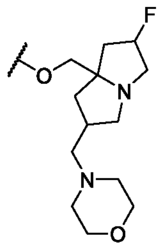, or 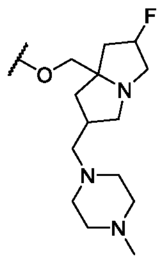,

more preferably

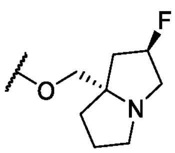, 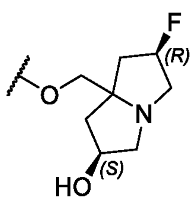, 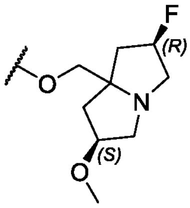, 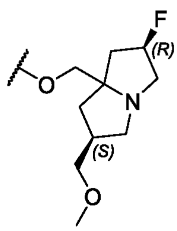, 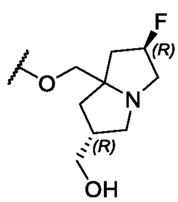,

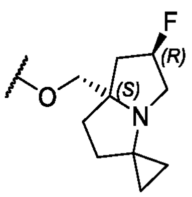, 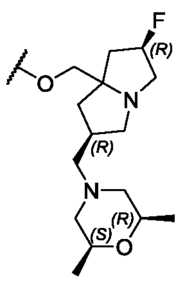, 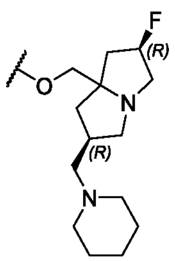, 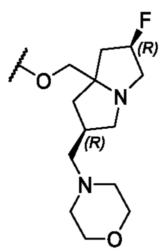, or 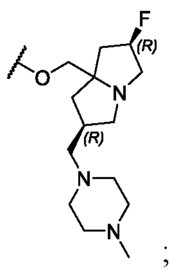;

(2) $R^2$ is fluorine;
(3) $R^3$ is

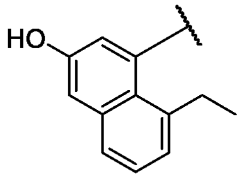 , 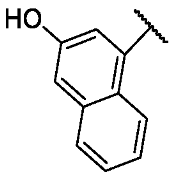 , 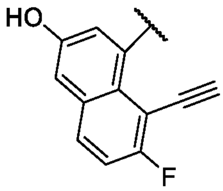 , 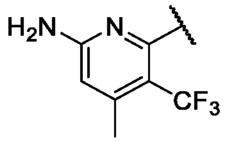 ,

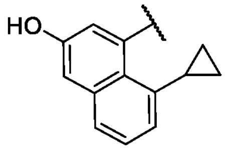 , 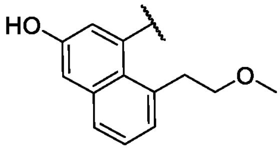 , 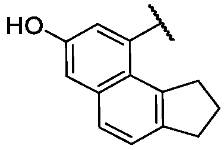 , 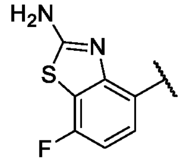 ,

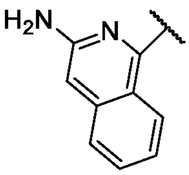 , or 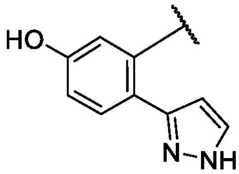 ,

preferably

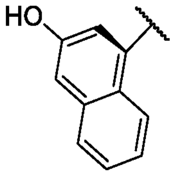 , 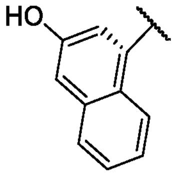 ,

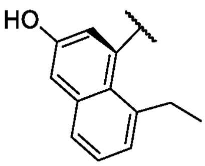 , 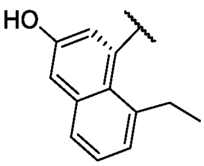 , 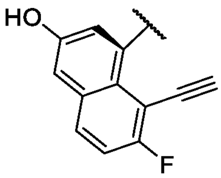 , 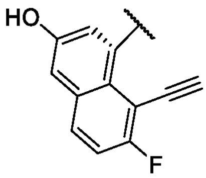 , 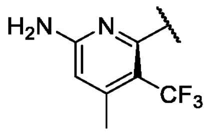 ,

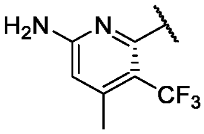 , 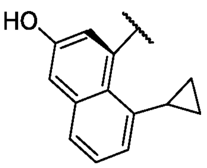 , 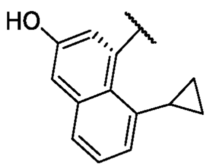 , 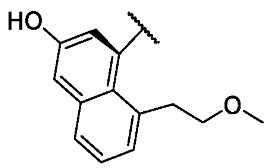 ,

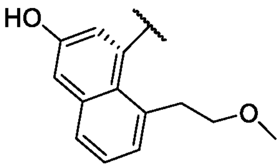 , 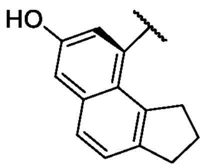 , 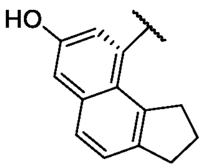 , 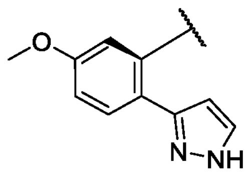 ,

or

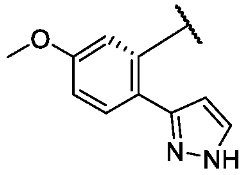;

(4) $R^4$ is H, fluorine, chlorine, cyano, trifluoromethyl, hydroxyl, methoxy, or ethoxy, preferably H or fluorine;
(5) $R^9$, $R^{10}$, $R^{11}$, and $R^{12}$ are each independently H or methyl, preferably H;
(6) $X^1$, $X^2$, and $X^3$ are each independently $CH_2$, O, CHF, or $CF_2$, preferably $CH_2$;
(7) $R^5$ is OH or H, preferably OH;
(8) $R^6$ is H, chlorine, fluorine, methyl, trifluoromethyl, or cyclopropyl, preferably chlorine;
(9) ring A is

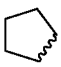, 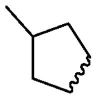, 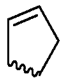, 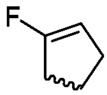, 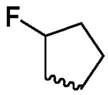, 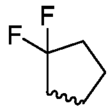, 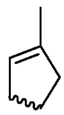, 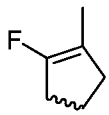,

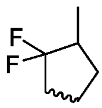, 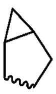, 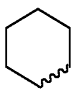, 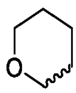, 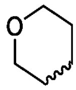, 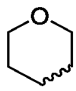, 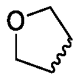, 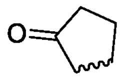, 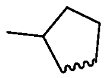, or 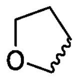,

preferably

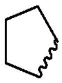, 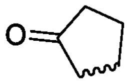, or 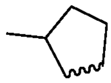,

for example,

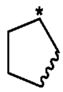, 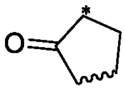, or 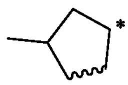;

(10) $R^{13}$ is fluorine;
(11) $R^{14}$ is

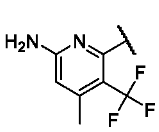, 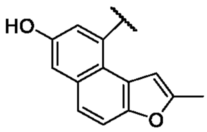, 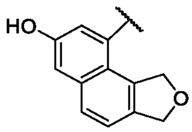, 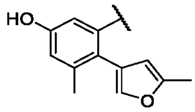, 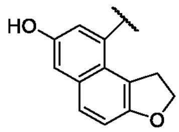,

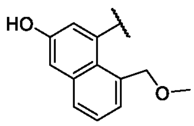, 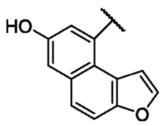, 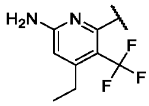, 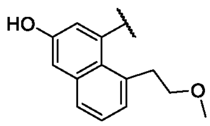, 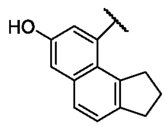,

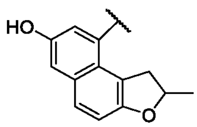, 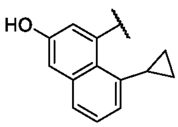, or 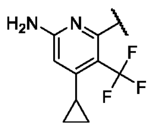;

(12) $R^{3X}$ is

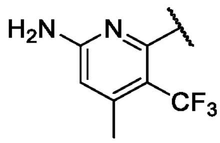

**9.** The compound of formula I, formula II, formula III, or formula IV, the pharmaceutically acceptable salt thereof, the solvate thereof, the stereoisomer thereof, the tautomer thereof, the prodrug thereof, the metabolite thereof, or the isotopic compound thereof according to claim 1 or 2, wherein the compound of formula I, formula II, or formula III, the pharmaceutically acceptable salt thereof, the solvate thereof, the stereoisomer thereof, the tautomer thereof, the prodrug thereof, the metabolite thereof, or the isotopic compound thereof is any one of the following schemes:

scheme **1:**

in the compound of formula I, formula II, or formula III, X is N, O, or S;
n1 and n4 are independently 1, 2, 3, or 4;
each L is independently -O-($CR^{L-1}R^{L-2}$)$_{n2}$-*, -($CR^{L-3}R^{L-4}$)$_{n3}$-*, or

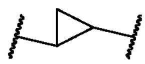;

* represents one end connected to $R^1$;
n2 and n3 are each independently 0, 1, 2, 3, or 4;
$R^{L-1}$, $R^{L-2}$, $R^{L-3}$, and $R^{L-4}$ are each independently H, $C_1$-$C_6$ alkyl, $C_1$-$C_6$ alkoxy, $C_1$-$C_6$ alkyl substituted by one or more $R^{L-1-1}$, or halogen;
each $R^{L-1-1}$ is independently halogen or $C_1$-$C_6$ alkoxy;
each $R^1$ is 4- to 10-membered heterocycloalkyl substituted by one or more $R^{1-1}$; heteroatoms in the 4- to 10-membered heterocycloalkyl of the 4- to 10-membered heterocycloalkyl substituted by one or more $R^{1-1}$ are independently 1, 2, or 3 kinds of N, O, or S, and the number of heteroatoms is 1, 2, or 3;
each $R^{1-1}$ is independently halogen;
$R^2$ and $R^{13}$ are each independently H or halogen;
$R^3$ and $R^{14}$ are each independently $C_6$-$C_{10}$ aryl, $C_6$-$C_{10}$ aryl substituted by one or more $R^{3-1}$, 5- to 10-membered heteroaryl, or 5- to 10-membered heteroaryl substituted by one or more $R^{3-2}$; heteroatoms in the 5- to 10-membered heteroaryl and the 5- to 10-membered heteroaryl substituted by one or more $R^{3-2}$ are independently 1, 2, or 3 kinds of N, O, or S, and the number of heteroatoms is 1, 2, or 3;
$R^{3-1}$ and $R^{3-2}$ are each independently OH, halogen, $C_1$-$C_6$ alkyl, $C_1$-$C_6$ alkyl substituted by one or more $R^{3-1-1}$, $C_2$-$C_6$ alkynyl, 3- to 8-membered cycloalkyl, -S-C($R^{3-1-2}$)$_3$, -S($R^{3-1-3}$)$_5$, amino, $C_1$-$C_6$ alkyl, or 5- to 10-membered heteroaryl;
alternatively, any two adjacent $R^{3-1}$, together with the carbon atom to which they are attached, form a 5- to 10-membered heteroaryl group or a 5- to 10-membered heteroaryl group substituted by one or more $R^{3-1-4}$; heteroatoms in the 5- to 10-membered heteroaryl group and the 5- to 10-membered heteroaryl group substituted by one or more $R^{3-1-4}$ are independently 1, 2, or 3 kinds of N, O, or S, and the number of heteroatoms is 1, 2, or 3;
each $R^{3-1-1}$ is independently oxo (=O), OH, $C_1$-$C_6$ alkoxy, or halogen;
$R^{3-1-2}$ and $R^{3-1-3}$ are each independently halogen;
each $R^{3-1-4}$ is independently $C_1$-$C_6$ alkyl;
$R^4$ is H, OH, $C_1$-$C_6$ alkyl, $C_1$-$C_6$ alkoxy, $C_1$-$C_6$ alkyl substituted by one or more $R^{4-1}$, cyano, or halogen;
each $R^{4-1}$ is independently halogen;
$X^1$ is C($R^{1a}R^{1b}$) or O;
$X^2$ is C($R^{2a}R^{2b}$) or O;
$X^3$ is C($R^{3a}R^{3b}$) or O;
$R^{1a}$, $R^{1b}$, $R^{2a}$, $R^{2b}$, $R^{3a}$, and $R^{3b}$ are each independently H, $C_1$-$C_6$ alkyl, or halogen;
$R^5$ is H or OH;
$R^6$ is H, $C_1$-$C_6$ alkyl, $C_1$-$C_6$ alkoxy, 3- to 8-membered cycloalkyl, halogen, or $C_1$-$C_6$ alkyl substituted by one or more $R^{6-1}$;
each $R^{6-1}$ is independently halogen;
$R^7$ and $R^8$ are connected to form ring A, and ring A is a 5- to 6-membered saturated or unsaturated monocyclic, spirocyclic, or fused carbocyclic ring, a 5- to 6-membered saturated or unsaturated monocyclic,

spirocyclic, or fused carbocyclic ring substituted by one or more $R^{7-1}$, a 5- to 6-membered saturated or unsaturated monocyclic, spirocyclic, or fused heterocyclic ring with 1, 2, or 3 heteroatoms selected from 1, 2, or 3 kinds of N, O, and S, or a 5- to 6-membered saturated or unsaturated monocyclic, spirocyclic, or fused heterocyclic ring with 1, 2, or 3 heteroatoms selected from 1, 2, or 3 kinds of N, O, and S substituted by one or more $R^{7-2}$;

$R^{7-1}$ and $R^{7-2}$ are each independently $C_1$-$C_6$ alkyl, oxo, or halogen;

$R^9$, $R^{10}$, $R^{11}$, $R^{12}$, $R^{15}$, and $R^{16}$ are each independently H, $C_1$-$C_6$ alkyl, or halogen;

scheme **2:**

in the compound of formula I, formula II, or formula III, X is N, O, or S;

n1 and n4 are independently 1, 2, 3, or 4;

each L is independently -O-($CR^{L-1}R^{L-2}$)$_{n2}$-*, -($CR^{L-3}R^{L-4}$)$_{n3}$-*, or

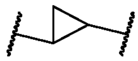
;

* represents one end connected to $R^1$;

n2 and n3 are each independently 0, 1, 2, 3, or 4;

$R^{L-1}$, $R^{L-2}$, $R^{L-3}$, and $R^{L-4}$ are each independently H, $C_1$-$C_6$ alkyl, $C_1$-$C_6$ alkoxy, $C_1$-$C_6$ alkyl substituted by one or more $R^{L-1-1}$, or halogen;

each $R^{L-1-1}$ is independently halogen or $C_1$-$C_6$ alkoxy;

each $R^1$ is 4- to 10-membered heterocycloalkyl substituted by one or more $R^{1-1}$; heteroatoms in the 4- to 10-membered heterocycloalkyl of the 4- to 10-membered heterocycloalkyl substituted by one or more $R^{1-1}$ are independently 1, 2, or 3 kinds of N, O, or S, and the number of heteroatoms is 1, 2, or 3;

each $R^{1-1}$ is independently halogen;

$R^2$ and $R^{13}$ are each independently H or halogen;

$R^3$ and $R^{14}$ are each independently $C_6$-$C_{10}$ aryl, $C_6$-$C_{10}$ aryl substituted by one or more $R^{3-1}$, 5- to 10-membered heteroaryl, or 5- to 10-membered heteroaryl substituted by one or more $R^{3-2}$; heteroatoms in the 5- to 10-membered heteroaryl and the 5- to 10-membered heteroaryl substituted by one or more $R^{3-2}$ are independently 1, 2, or 3 kinds of N, O, or S, and the number of heteroatoms is 1, 2, or 3;

$R^{3-1}$ and $R^{3-2}$ are each independently OH, halogen, $C_1$-$C_6$ alkyl, $C_1$-$C_6$ alkyl substituted by one or more $R^{3-1-1}$, $C_2$-$C_6$ alkynyl, 3- to 8-membered cycloalkyl, -S-C($R^{3-1-2}$)$_3$, -S($R^{3-1-3}$)$_5$, or amino;

alternatively, any two adjacent $R^{3-1}$, together with the carbon atom to which they are attached, form a 5- to 10-membered heteroaryl group or a 5- to 10-membered heteroaryl group substituted by one or more $R^{3-1-4}$; heteroatoms in the 5- to 10-membered heteroaryl group and the 5- to 10-membered heteroaryl group substituted by one or more $R^{3-1-4}$ are independently 1, 2, or 3 kinds of N, O, or S, and the number of heteroatoms is 1, 2, or 3;

each $R^{3-1-1}$ is independently oxo (=O), OH, $C_1$-$C_6$ alkoxy, or halogen;

$R^{3-1-2}$ and $R^{3-1-3}$ are each independently halogen;

each $R^{3-1-4}$ is independently $C_1$-$C_6$ alkyl;

$R^4$ is H, OH, $C_1$-$C_6$ alkyl, $C_1$-$C_6$ alkoxy, $C_1$-$C_6$ alkyl substituted by one or more $R^{4-1}$, cyano, or halogen;

each $R^{4-1}$ is independently halogen;

$X^1$ is C($R^{1a}R^{1b}$) or O;

$X^2$ is C($R^{2a}R^{2b}$) or O;

$X^3$ is C($R^{3a}R^{3b}$) or O;

$R^{1a}$, $R^{1b}$, $R^{2a}$, $R^{2b}$, $R^{3a}$, and $R^{3b}$ are each independently H, $C_1$-$C_6$ alkyl, or halogen;

$R^5$ is H or OH;

$R^6$ is H, $C_1$-$C_6$ alkyl, $C_1$-$C_6$ alkoxy, 3- to 8-membered cycloalkyl, halogen, or $C_1$-$C_6$ alkyl substituted by one or more $R^{6-1}$;

each $R^{6-1}$ is independently halogen;

$R^7$ and $R^8$ are connected to form ring A, and ring A is a 5- to 6-membered saturated or unsaturated monocyclic, spirocyclic, or fused carbocyclic ring, a 5- to 6-membered saturated or unsaturated monocyclic, spirocyclic, or fused carbocyclic ring substituted by one or more $R^{7-1}$, a 5- to 6-membered saturated or unsaturated monocyclic, spirocyclic, or fused heterocyclic ring with 1, 2, or 3 heteroatoms selected from 1, 2, or 3 kinds of N, O, and S, or a 5- to 6-membered saturated or unsaturated monocyclic, spirocyclic, or fused heterocyclic ring with 1, 2, or 3 heteroatoms selected from 1, 2, or 3 kinds of N, O, and S substituted by one or

more $R^{7-2}$;
$R^{7-1}$ and $R^{7-2}$ are each independently $C_1$-$C_6$ alkyl, oxo, or halogen;
$R^9$, $R^{10}$, $R^{11}$, $R^{12}$, $R^{15}$, and $R^{16}$ are each independently H, $C_1$-$C_6$ alkyl, or halogen;

scheme 3:
as in formula I or formula II:

wherein X is N, O, or S;
n1 is 1, 2, 3, or 4;
L is -O-$(CR^{L-1}R^{L-2})_{n2}$-*, -$(CR^{L-3}R^{L-4})_{n3}$-*, or

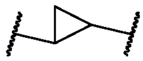;

* represents one end connected to $R^1$;
n2 and n3 are each independently 0, 1, 2, 3, or 4;
$R^{L-1}$, $R^{L-2}$, $R^{L-3}$, and $R^{L-4}$ are each independently H, $C_1$-$C_6$ alkyl, $C_1$-$C_6$ alkoxy, $C_1$-$C_6$ alkyl substituted by one or more $R^{L-1-1}$, or halogen;
each $R^{L-1-1}$ is independently halogen or $C_1$-$C_6$ alkoxy;
$R^1$ is 4- to 10-membered heterocycloalkyl substituted by one or more $R^{1-1}$; heteroatoms in the 4- to 10-membered heterocycloalkyl of the 4- to 10-membered heterocycloalkyl substituted by one or more $R^{1-1}$ are independently 1, 2, or 3 kinds of N, O, or S, and the number of heteroatoms is 1, 2, or 3;
each $R^{1-1}$ is independently halogen;
$R^2$ is H or halogen;
$R^3$ is $C_6$-$C_{10}$ aryl, $C_6$-$C_{10}$ aryl substituted by one or more $R^{3-1}$, or 5- to 10-membered heteroaryl; heteroatoms in the 5- to 10-membered heteroaryl are independently 1, 2, or 3 kinds of N, O, or S, and the number of heteroatoms is 1, 2, or 3;
each $R^{3-1}$ is independently OH, halogen, $C_1$-$C_6$ alkyl, $C_1$-$C_6$ alkyl substituted by one or more $R^{3-1-1}$, $C_2$-$C_6$ alkynyl, 3- to 8-membered cycloalkyl, -S-C$(R^{3-1-2})_3$, or -S$(R^{3-1-3})_5$;
each $R^{3-1-1}$ is independently oxo (=O), OH, $C_1$-$C_6$ alkoxy, or halogen;
$R^{3-1-2}$ and $R^{3-1-3}$ are each independently halogen;
$R^4$ is H, OH, $C_1$-$C_6$ alkyl, $C_1$-$C_6$ alkoxy, $C_1$-$C_6$ alkyl substituted by one or more $R^{4-1}$, cyano, or halogen;
each $R^{4-1}$ is independently halogen;
$X^1$ is $C(R^{1a}R^{1b})$ or O;
$X^2$ is $C(R^{2a}R^{2b})$ or O;
$X^3$ is $C(R^{3a}R^{3b})$ or O;
$R^{1a}$, $R^{1b}$, $R^{2a}$, $R^{2b}$, $R^{3a}$, and $R^{3b}$ are each independently H, $C_1$-$C_6$ alkyl, or halogen;
$R^5$ is H or OH;
$R^6$ is H, $C_1$-$C_6$ alkyl, $C_1$-$C_6$ alkoxy, 3- to 8-membered cycloalkyl, halogen, or $C_1$-$C_6$ alkyl substituted by one or more $R^{6-1}$;
each $R^{6-1}$ is independently halogen;
$R^7$ and $R^8$ are connected to form ring A, and ring A is a 5- to 6-membered saturated or unsaturated monocyclic, spirocyclic, or fused carbocyclic ring, a 5- to 6-membered saturated or unsaturated monocyclic, spirocyclic, or fused carbocyclic ring substituted by one or more $R^{7-1}$, a 5- to 6-membered saturated or unsaturated monocyclic, spirocyclic, or fused heterocyclic ring with 1, 2, or 3 heteroatoms selected from 1, 2, or 3 kinds of N, O, and S, or a 5- to 6-membered saturated or unsaturated monocyclic, spirocyclic, or fused heterocyclic ring with 1, 2, or 3 heteroatoms selected from 1, 2, or 3 kinds of N, O, and S substituted by one or more $R^{7-2}$;
$R^{7-1}$ and $R^{7-2}$ are each independently $C_1$-$C_6$ alkyl, oxo (=O), or halogen;
$R^9$, $R^{10}$, $R^{11}$, and $R^{12}$ are each independently H, $C_1$-$C_6$ alkyl, or halogen;

scheme 4:
as in formula I or formula II:

X is N, O, or S;
n1 is 1, 2, 3, or 4;
L is independently -O-$(CR^{L-1}R^{L-2})_{n2}$-*, -$(CR^{L-3}R^{L-4})_{n3}$-*, or

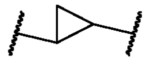;

* represents one end connected to $R^1$;
n2 and n3 are each independently 0, 1, 2, 3, or 4;
$R^{L-1}$, $R^{L-2}$, $R^{L-3}$, and $R^{L-4}$ are each independently H, $C_1$-$C_6$ alkyl, $C_1$-$C_6$ alkoxy, $C_1$-$C_6$ alkyl substituted by one or more $R^{L-1-1}$, or halogen;
each $R^{L-1-1}$ is independently halogen or $C_1$-$C_6$ alkoxy;
$R^1$ is 4- to 10-membered heterocycloalkyl substituted by one or more $R^{1-1}$; heteroatoms in the 4- to 10-membered heterocycloalkyl of the 4- to 10-membered heterocycloalkyl substituted by one or more $R^{1-1}$ are independently 1, 2, or 3 kinds of N, O, or S, and the number of heteroatoms is 1, 2, or 3;
each $R^{1-1}$ is independently halogen;
$R^2$ is H or halogen;
$R^3$ is $C_6$-$C_{10}$ aryl, $C_6$-$C_{10}$ aryl substituted by one or more $R^{3-1}$, or 5- to 10-membered heteroaryl; heteroatoms in the 5- to 10-membered heteroaryl are independently 1, 2, or 3 kinds of N, O, or S, and the number of heteroatoms is 1, 2, or 3;
each $R^{3-1}$ is independently OH, halogen, $C_1$-$C_6$ alkyl, $C_1$-$C_6$ alkyl substituted by one or more $R^{3-1-1}$, $C_2$-$C_6$ alkynyl, 3- to 8-membered cycloalkyl, -S-C$(R^{3-1-2})_3$, or -S$(R^{3-1-3})_5$;
each $R^{3-1-1}$ is independently oxo (=O), OH, $C_1$-$C_6$ alkoxy, or halogen;
$R^{3-1-2}$ and $R^{3-1-3}$ are each independently halogen;
$R^4$ is H, OH, $C_1$-$C_6$ alkyl, $C_1$-$C_6$ alkoxy, $C_1$-$C_6$ alkyl substituted by one or more $R^{4-1}$, cyano, or halogen;
each $R^{4-1}$ is independently halogen;
$X^1$ is C$(R^{1a}R^{1b})$ or O;
$X^2$ is C$(R^{2a}R^{2b})$ or O;
$X^3$ is C$(R^{3a}R^{3b})$ or O;
$R^{1a}$, $R^{1b}$, $R^{2a}$, $R^{2b}$, $R^{3a}$, and $R^{3b}$ are each independently H, $C_1$-$C_6$ alkyl, or halogen;
$R^5$ is H or OH;
$R^6$ is H, $C_1$-$C_6$ alkyl, $C_1$-$C_6$ alkoxy, 3- to 8-membered cycloalkyl, halogen, or $C_1$-$C_6$ alkyl substituted by one or more $R^{6-1}$;
each $R^{6-1}$ is independently halogen;
$R^7$ and $R^8$ are connected to form ring A, and ring A is a 5- to 6-membered saturated or unsaturated monocyclic, spirocyclic, or fused carbocyclic ring, a 5- to 6-membered saturated or unsaturated monocyclic, spirocyclic, or fused carbocyclic ring substituted by one or more $R^{7-1}$, a 5- to 6-membered saturated or unsaturated monocyclic, spirocyclic, or fused heterocyclic ring with 1, 2, or 3 heteroatoms selected from 1, 2, or 3 kinds of N, O, and S, or a 5- to 6-membered saturated or unsaturated monocyclic, spirocyclic, or fused heterocyclic ring with 1, 2, or 3 heteroatoms selected from 1, 2, or 3 kinds of N, O, and S substituted by one or more $R^{7-2}$;
$R^{7-1}$ and $R^{7-2}$ are each independently $C_1$-$C_6$ alkyl or halogen;
$R^9$, $R^{10}$, $R^{11}$, and $R^{12}$ are each independently H, $C_1$-$C_6$ alkyl, or halogen.

**10.** The compound of formula I, formula II, formula III, or formula IV, the pharmaceutically acceptable salt thereof, the solvate thereof, the stereoisomer thereof, the tautomer thereof, the prodrug thereof, the metabolite thereof, or the isotopic compound thereof according to claim 1 or 2, wherein the compound of formula I, formula II, formula III, or formula IV is any one of the following compounds:

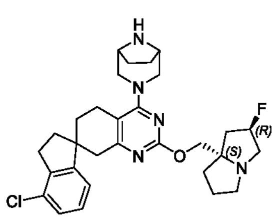
1'

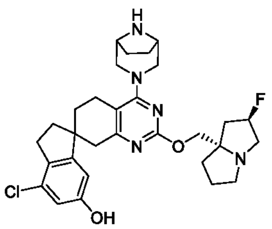
2'

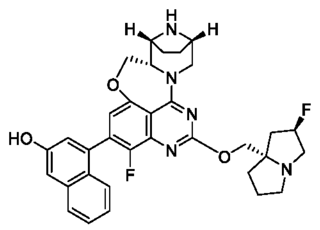
3

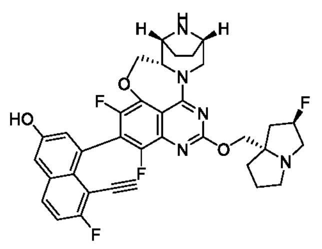
4'

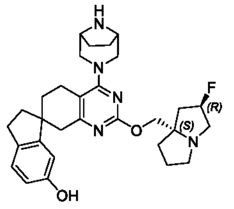
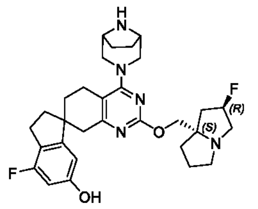
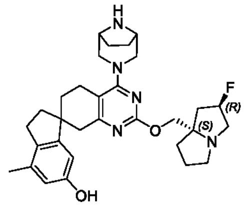

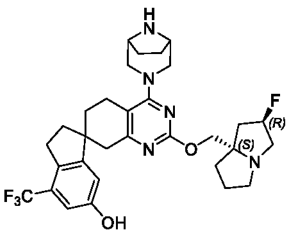
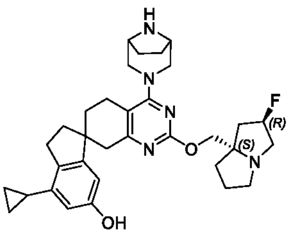
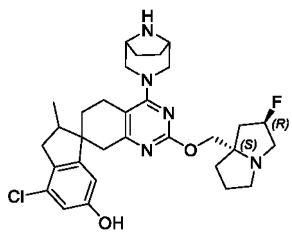

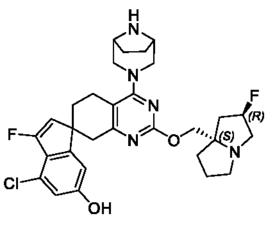
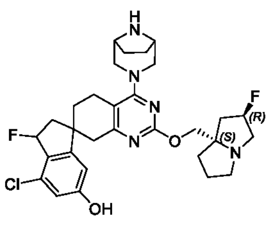
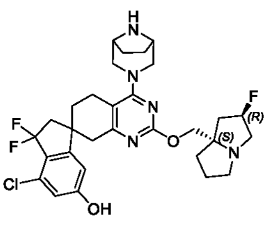
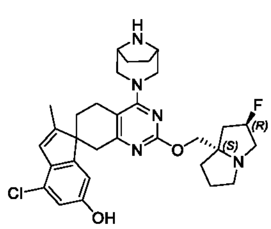

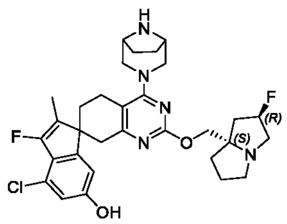
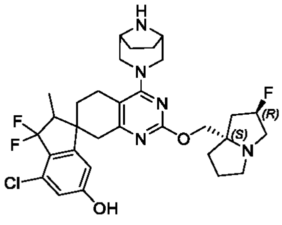
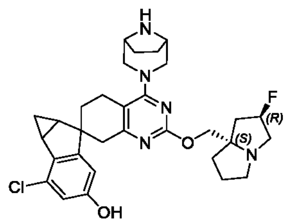
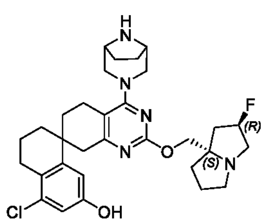

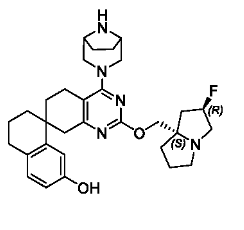
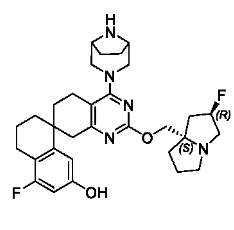
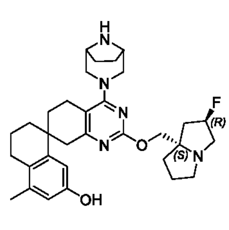
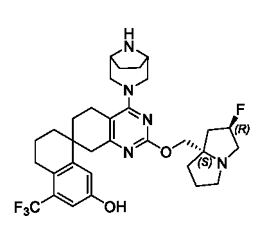

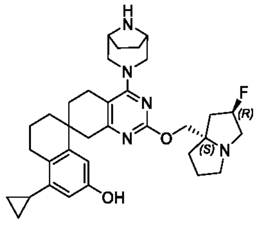
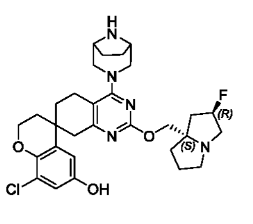
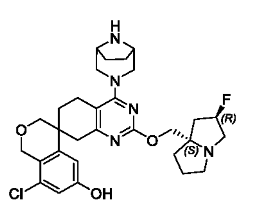
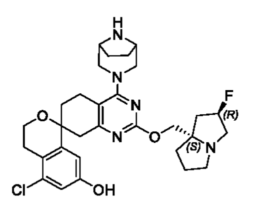

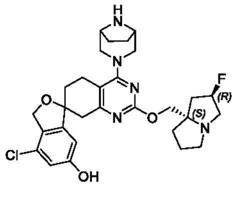
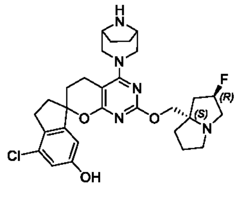
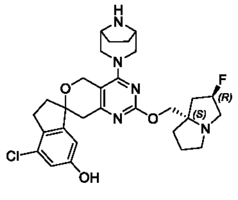
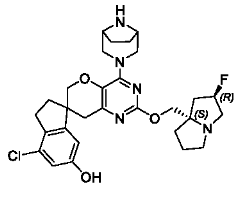

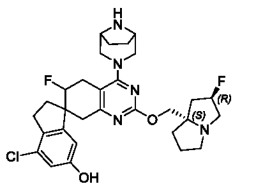
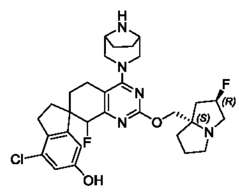
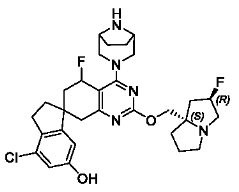
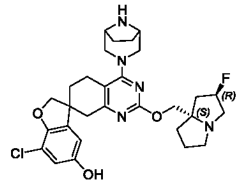
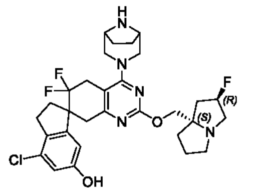
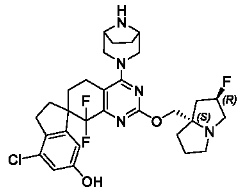
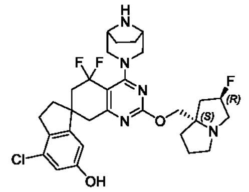
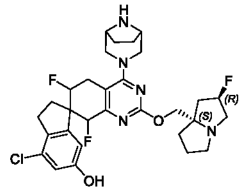
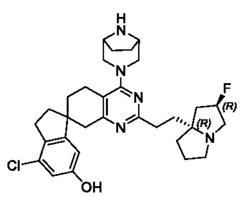
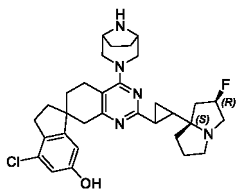
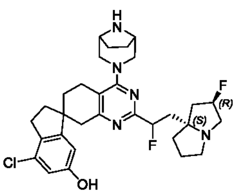
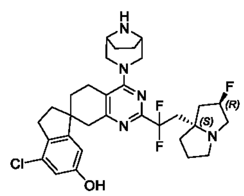
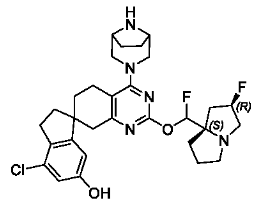
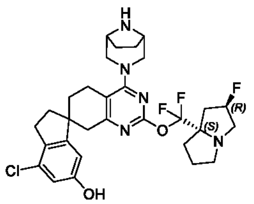
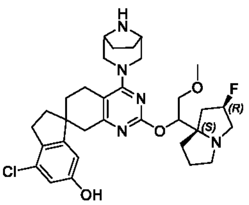
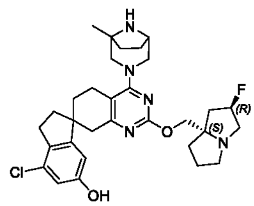
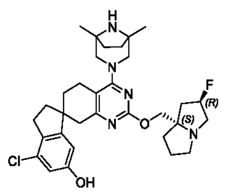
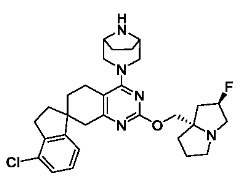
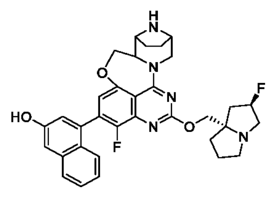
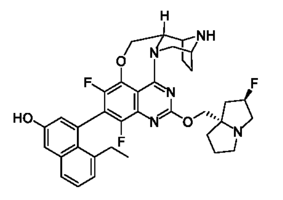
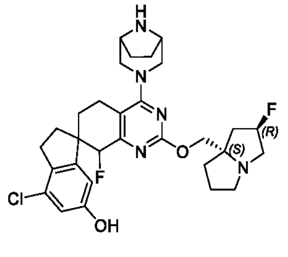
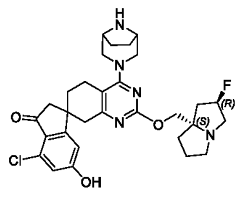
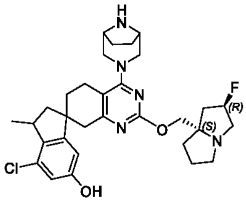
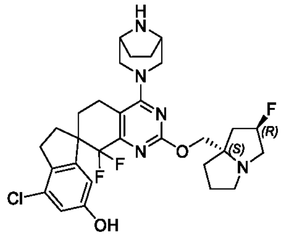
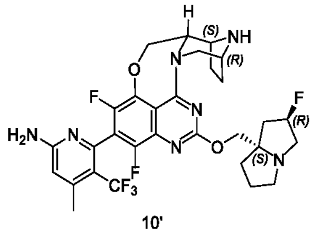

10'

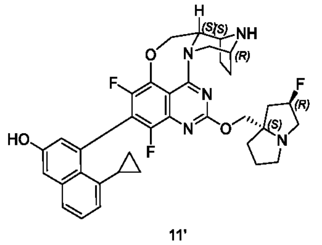

11'

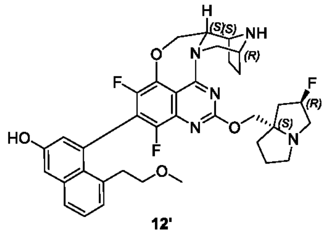

12'

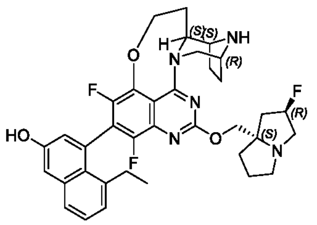

13'

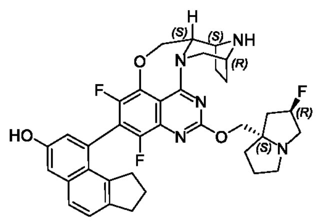

14'

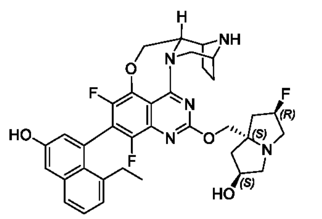

15'

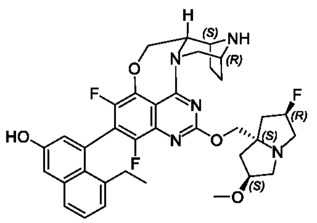

16'

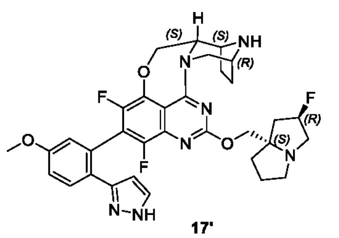

17'

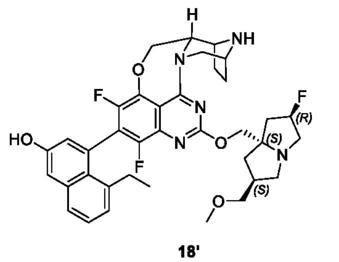

18'

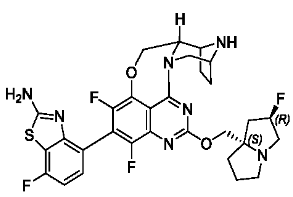

19'

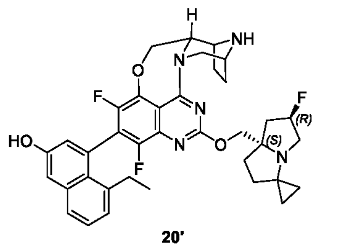

20'

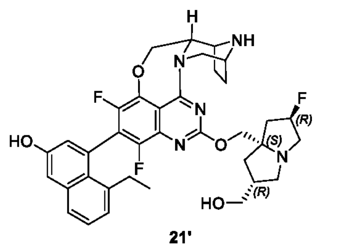

21'

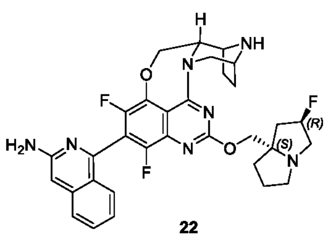

22

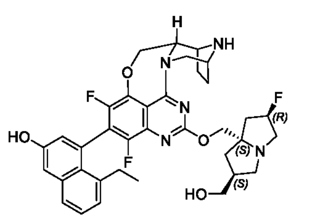

23'

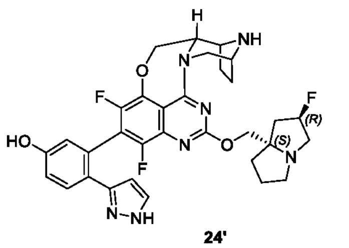

24'

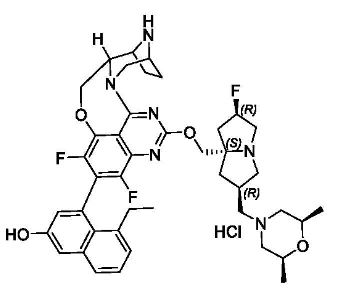

25'

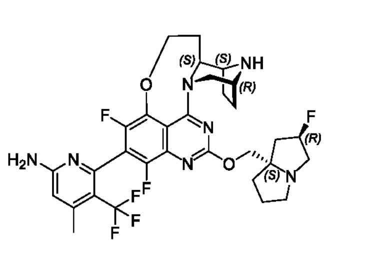

26'

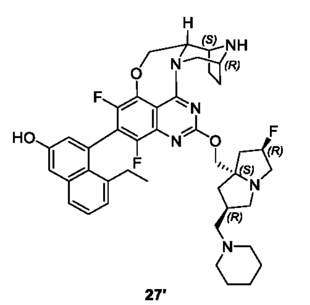

27'

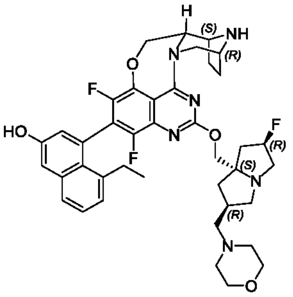

28'

or

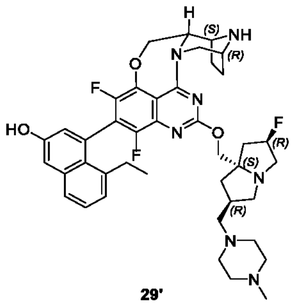

29'

11. The compound of formula I, formula II, formula III, or formula IV, the pharmaceutically acceptable salt thereof, the solvate thereof, the stereoisomer thereof, the tautomer thereof, the prodrug thereof, the metabolite thereof, or the isotopic compound thereof according to claim 1 or 2, wherein the stereoisomer of the compound of formula I, formula II, formula III, or formula IV is any one of the following compounds:

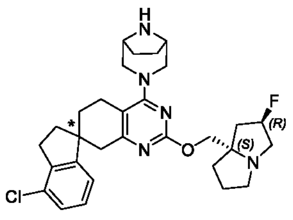

1a'

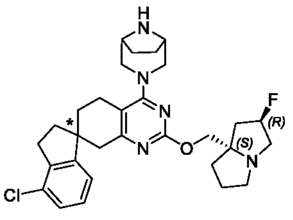

1b'

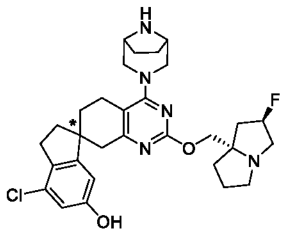

2a'

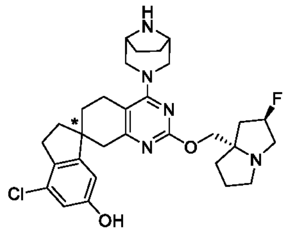

2b'

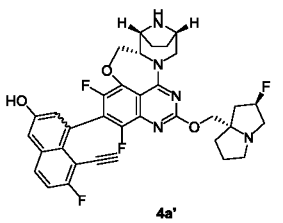

4a'

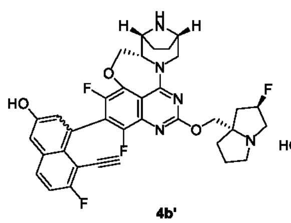

4b'

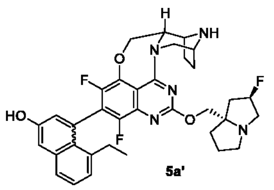

5a'

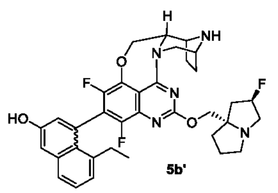

5b'

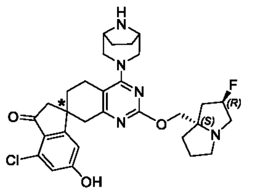

6a'

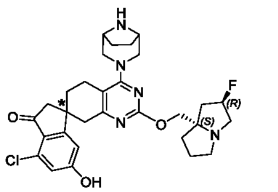

6b'

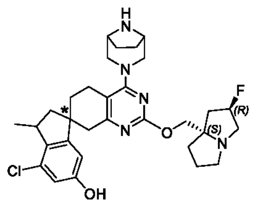

7a'

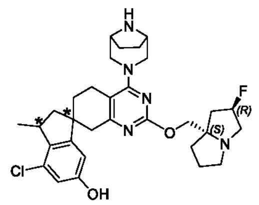

7aa'

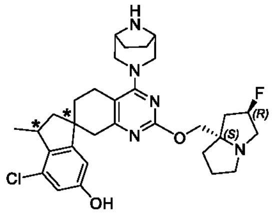

7ab'

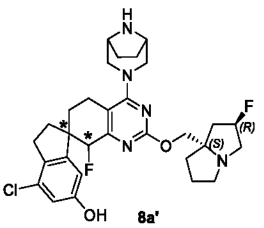

8a'

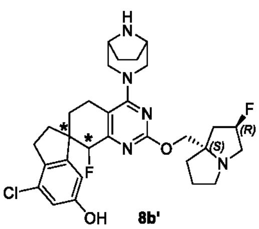

8b'

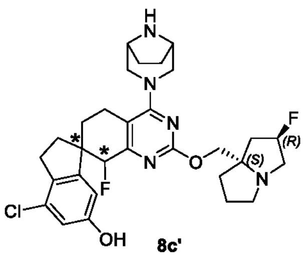

8c'

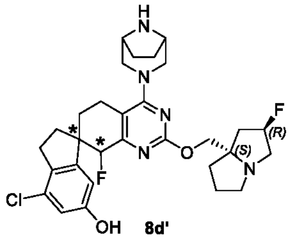
8d'

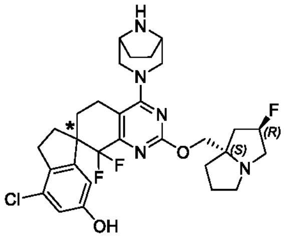
9a'

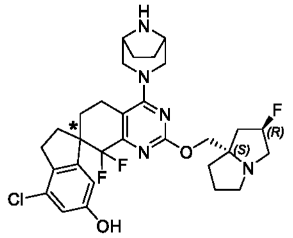
9b'

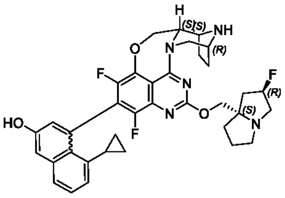
11a'

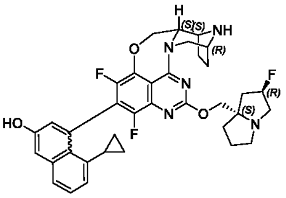
11b'

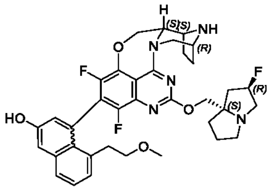
12a'

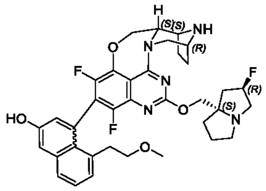
12b'

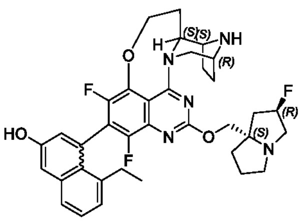
13a'

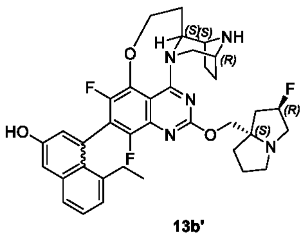
13b'

| Compound | Condition | Retention time |
|---|---|---|
| 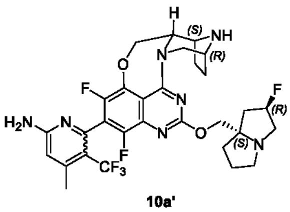 10a' | Chromatographic column YMC-Actus Triart C18 ExRS, 30 × 150 mm, 5 μm; mobile phase A: water (10 mmol/L ammonium bicarbonate), mobile phase B: acetonitrile; flow rate: 60 mL/min; elution with 25% to 45% phase B in 13 minutes; detector: 220 nm | 10.13 minutes |
| 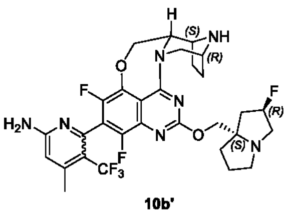 10b' | | 10.98 minutes |

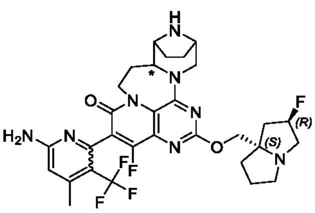

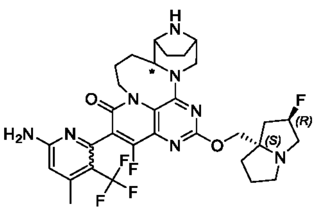

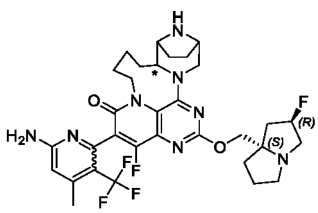

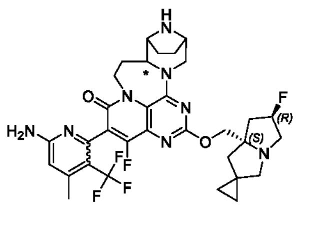
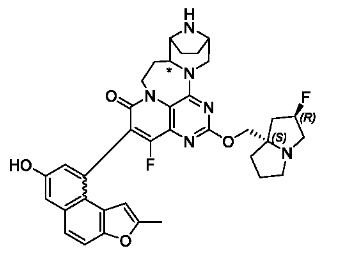
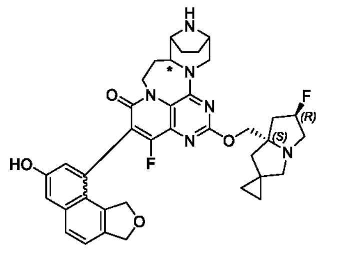

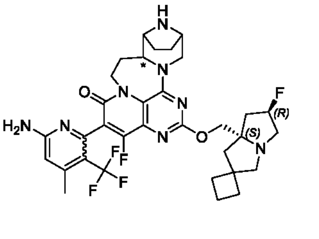
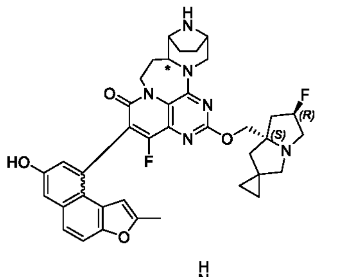
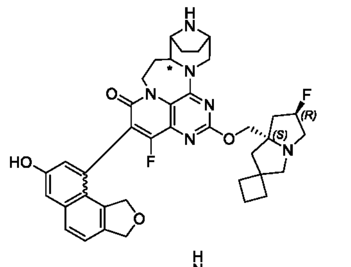

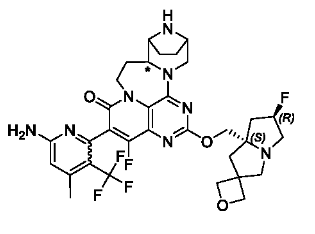
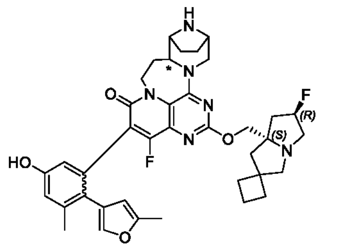
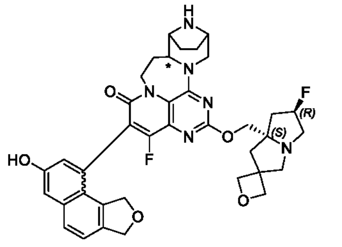

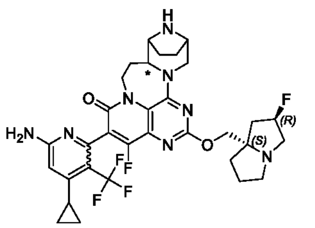
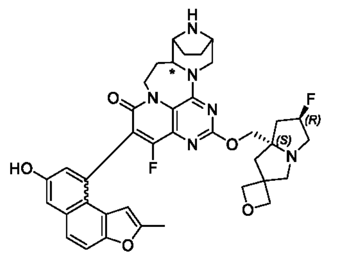
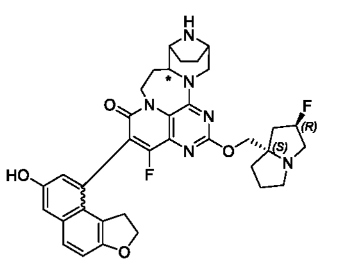

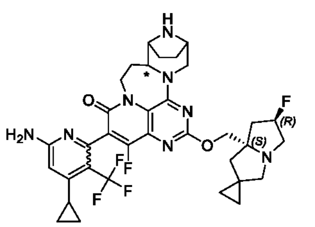
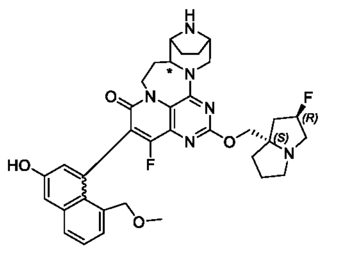
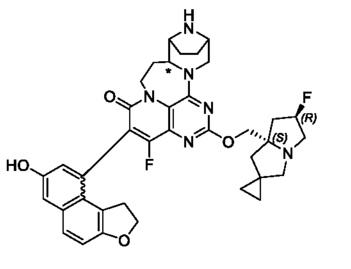

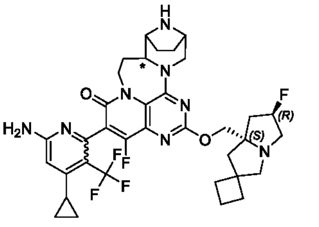
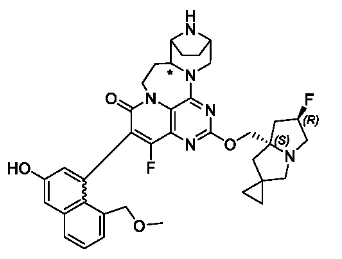
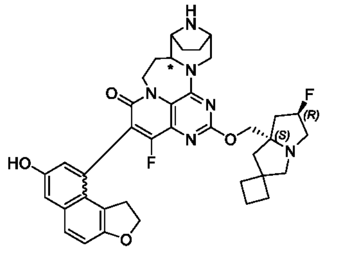

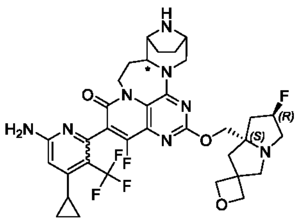
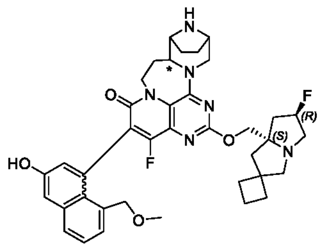
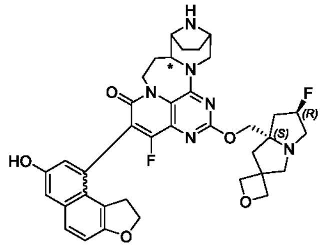
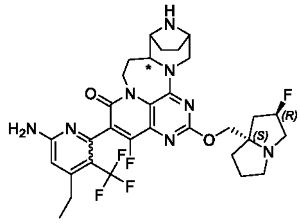
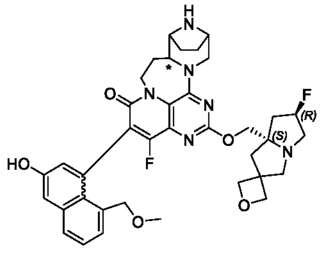
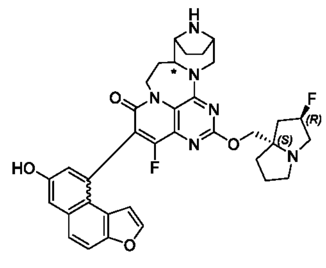
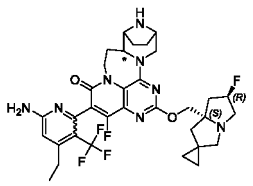
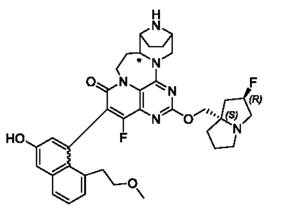
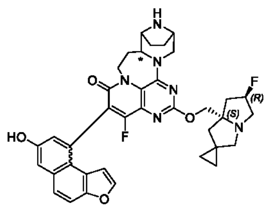
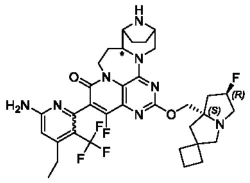
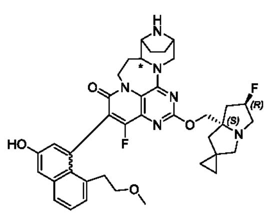
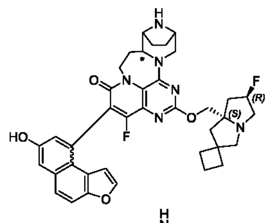
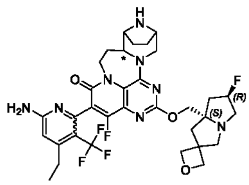
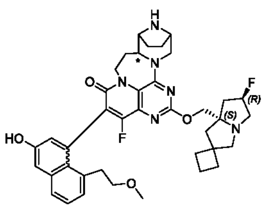
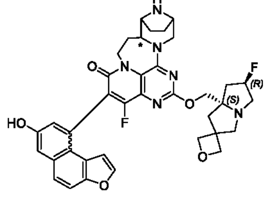
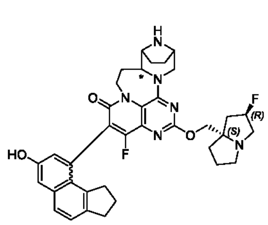
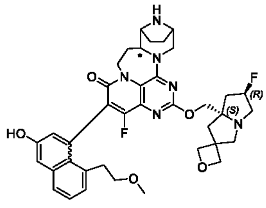
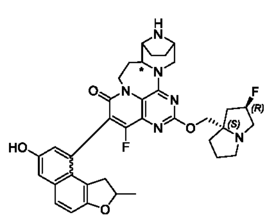

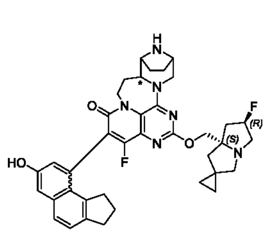
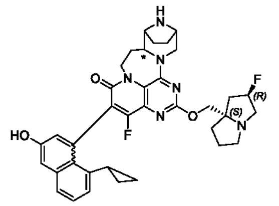
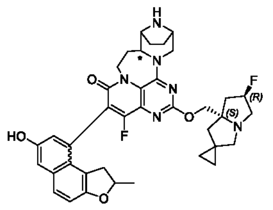
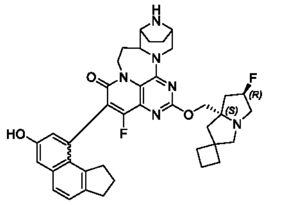
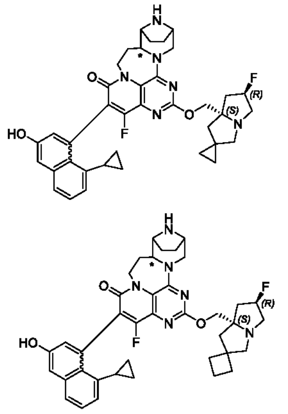
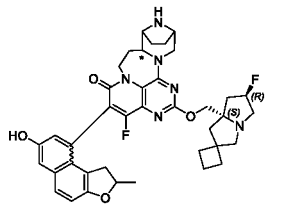
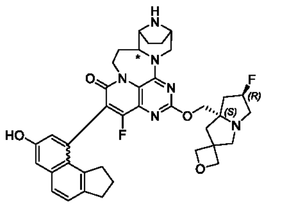
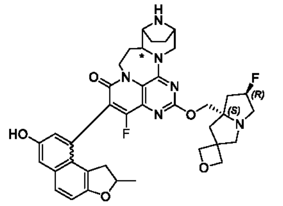
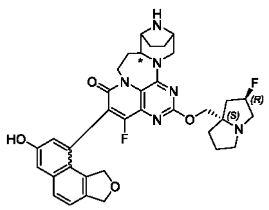
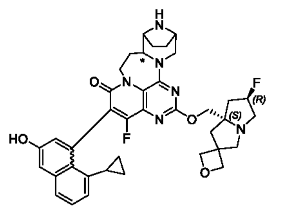
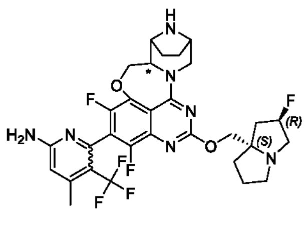
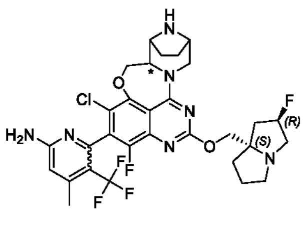
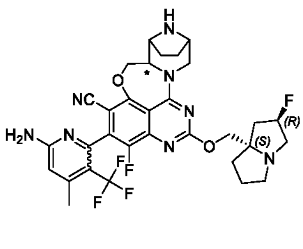
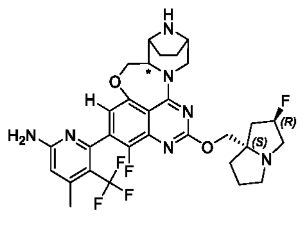
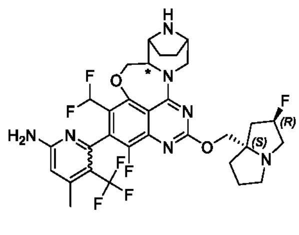
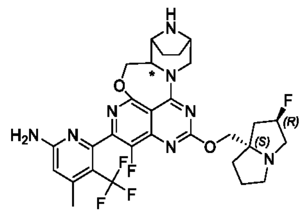

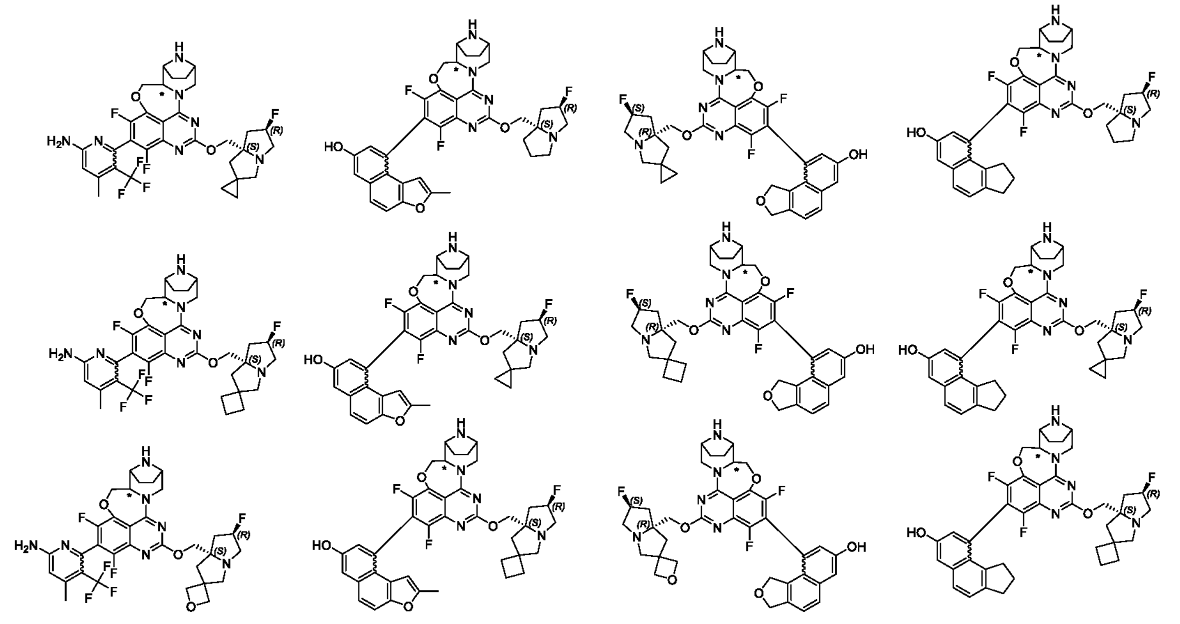

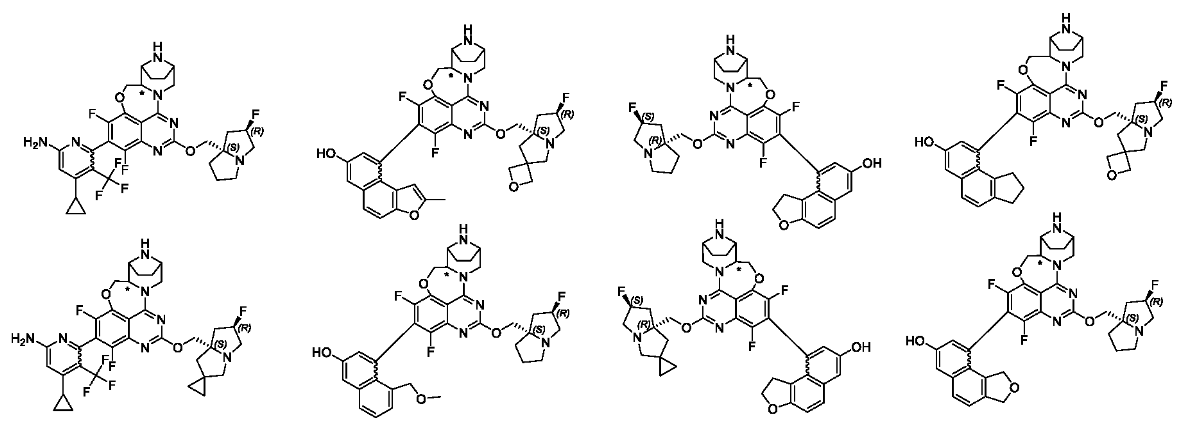

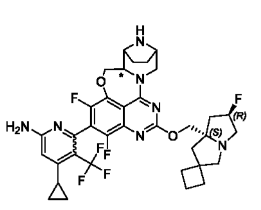

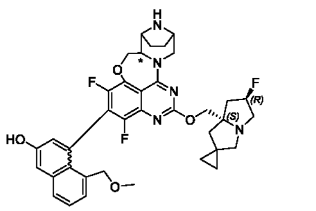

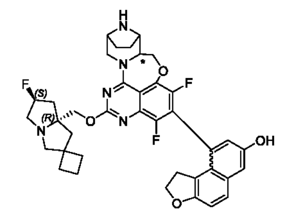

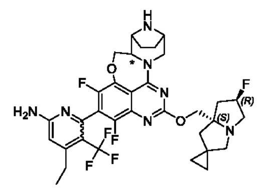

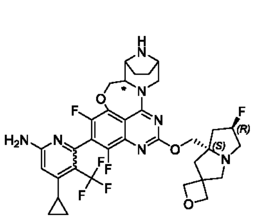
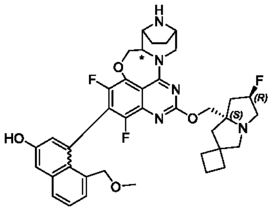
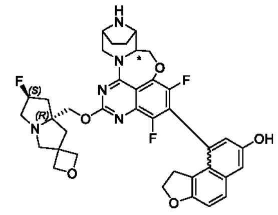
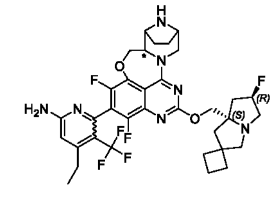
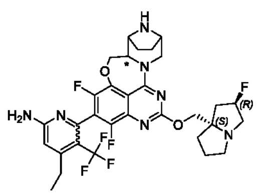
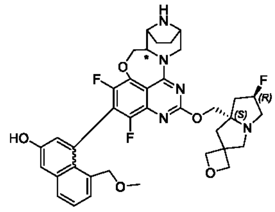
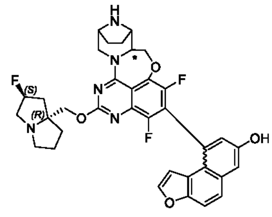
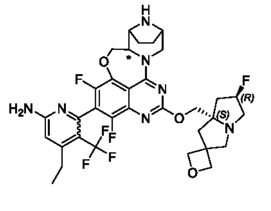
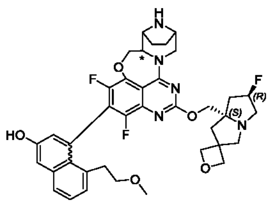
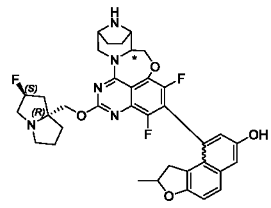
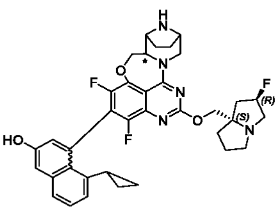
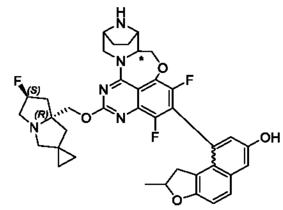
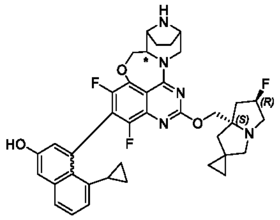
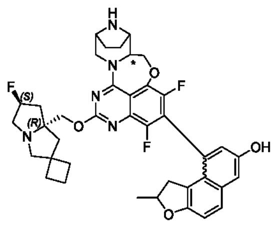
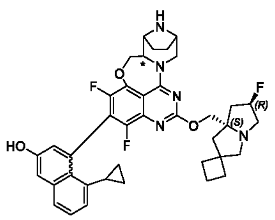
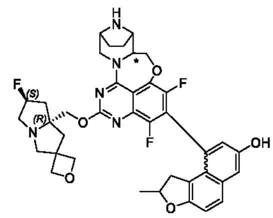
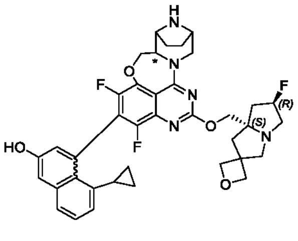
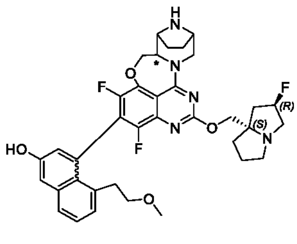
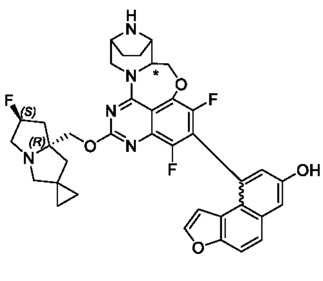
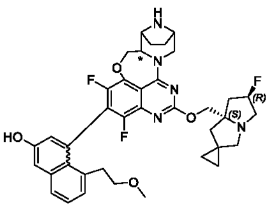
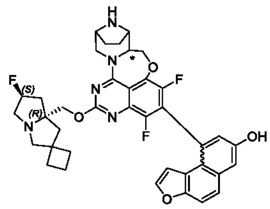
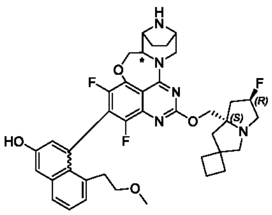
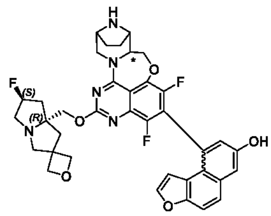

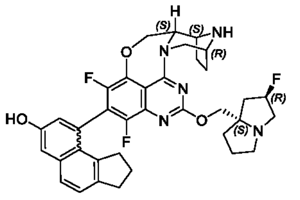
14a'

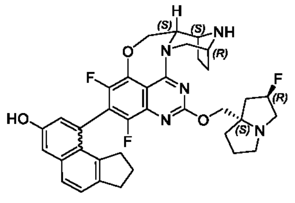
14b'

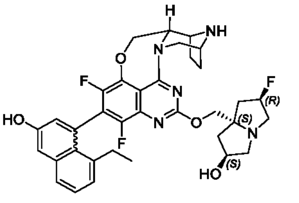
15a'

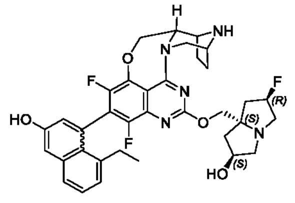
15b'

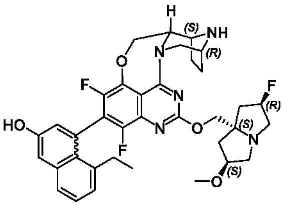
16a'

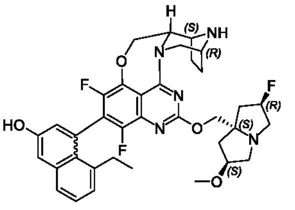
16b'

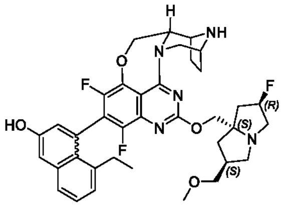
18a'

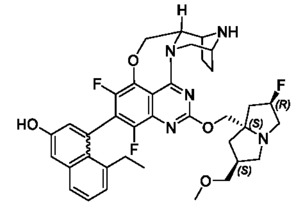
18b'

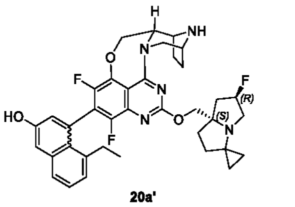
20a'

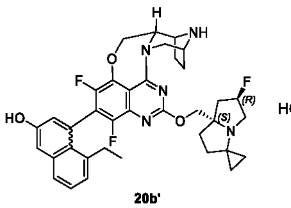
20b'

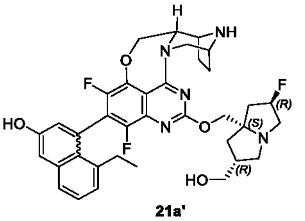
21a'

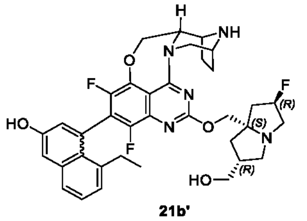
21b'

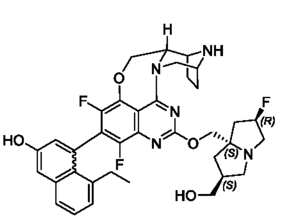
23a'

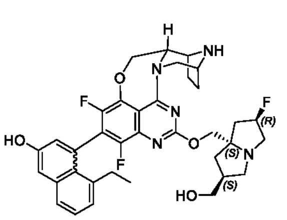
23b'

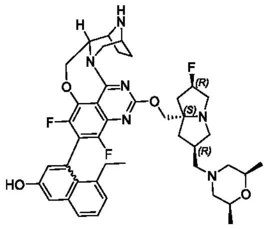
25a'

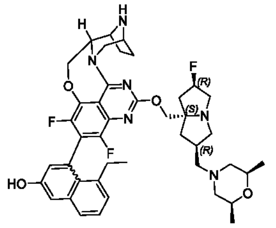
25b'

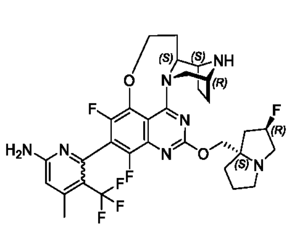
26a'

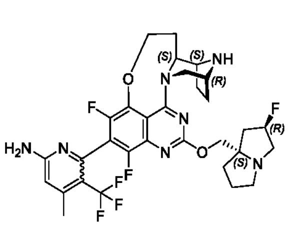
26b'

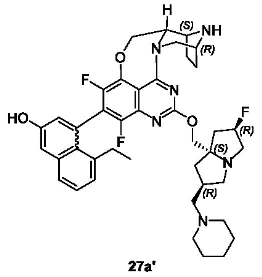
27a'

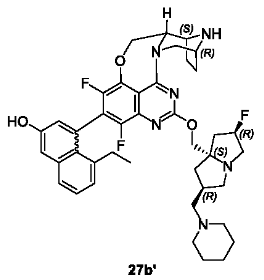
27b'

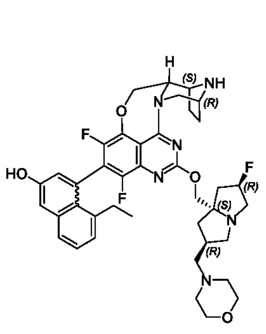
28a'

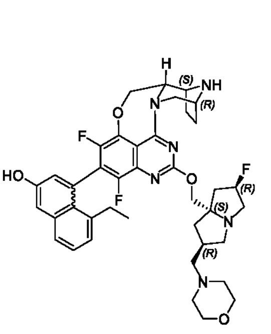
28b'

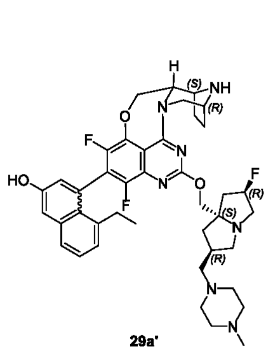
29a'

or

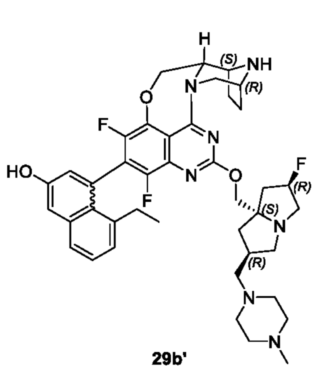
29b' ;

wherein "*" denotes a carbon atom in S configuration or a carbon atom in R configuration, and " " denotes " " or " ".

**12.** The compound of formula I, formula II, formula III, or formula IV, the pharmaceutically acceptable salt thereof, the solvate thereof, the stereoisomer thereof, the tautomer thereof, the prodrug thereof, the metabolite thereof, or the isotopic compound thereof according to claim 1 or 2, wherein the pharmaceutically acceptable salt of the compound of formula I, formula II, formula III, or formula IV is a hydrochloride salt of the compound of formula I, formula II, formula III, or formula IV;

and/or, the number of the pharmaceutically acceptable salts of the compound of formula I, formula II, formula III, or formula IV may be 1, 2, 3, 4, or 5;
the pharmaceutically acceptable salt of the compound of formula I, formula II, formula III, or formula IV is preferably any one of the following compounds:

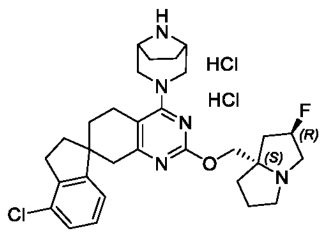

1"

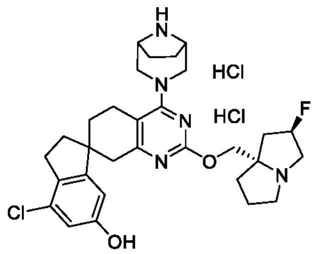

2"

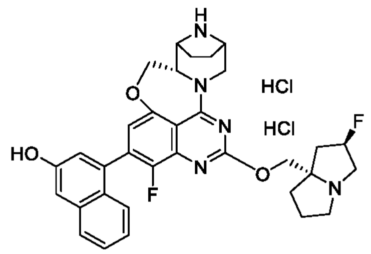

3

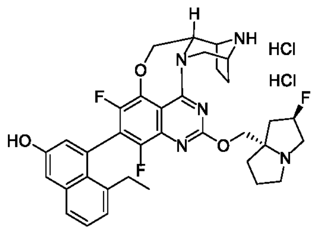

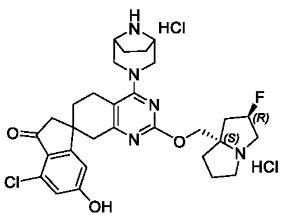

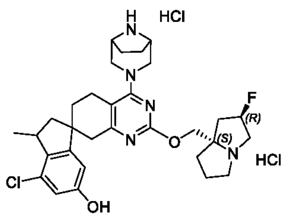

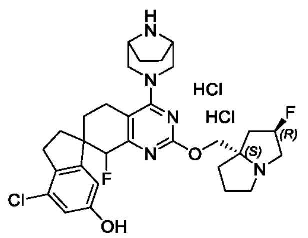

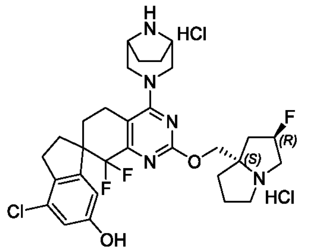

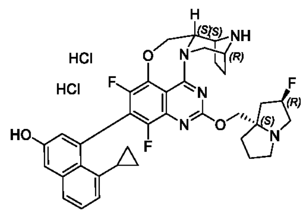

11"

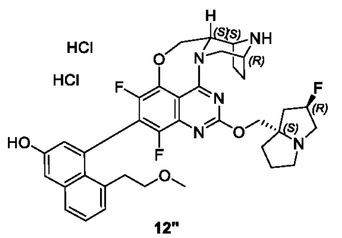

12"

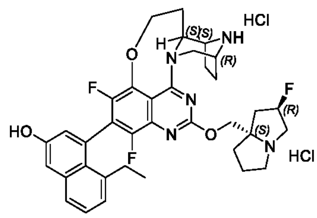

13"

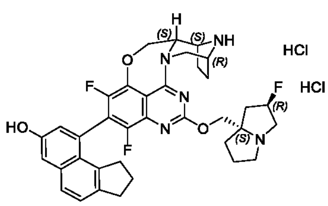

14"

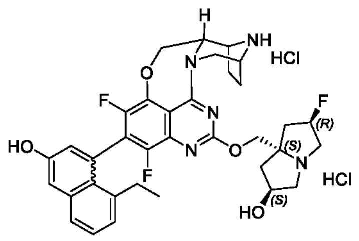
15''

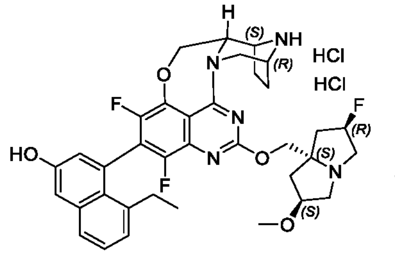
16''

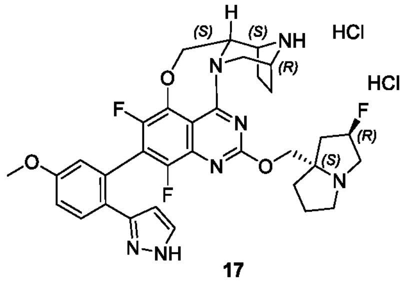
17

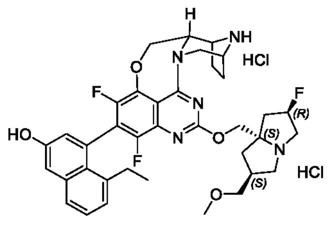
18''

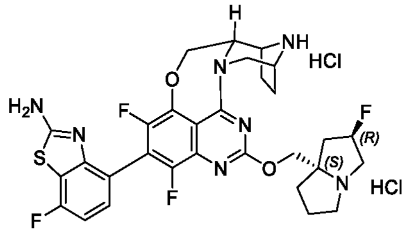
19

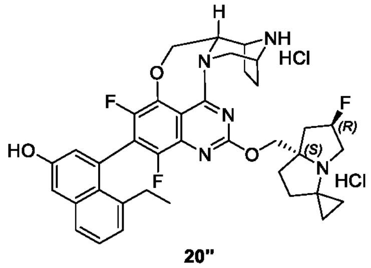
20''

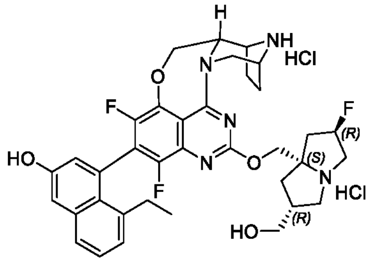
21''

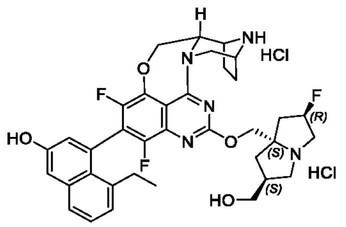
23''

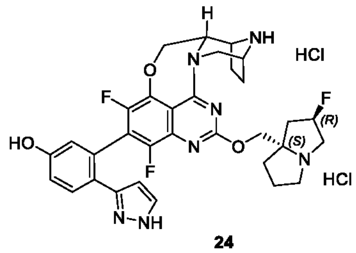
24

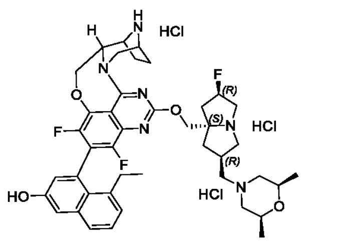
25''

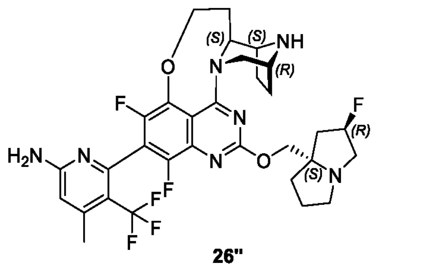
26''

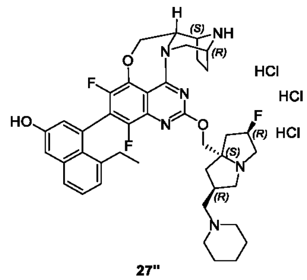
27''

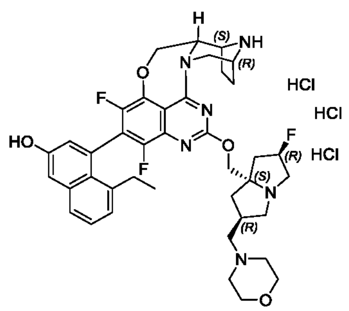
28''

or

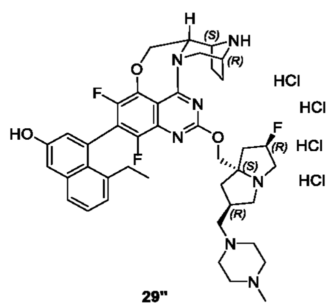
29''

**13.** The compound of formula I, formula II, or formula III, the pharmaceutically acceptable salt thereof, the solvate thereof, the stereoisomer thereof, the tautomer thereof, the prodrug thereof, the metabolite thereof, or the isotopic compound thereof according to claim 1 or 2, wherein the pharmaceutically acceptable salt of the compound of formula I, formula II, formula III, or formula IV is any one of the following compounds:

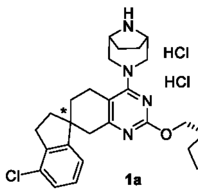
1a

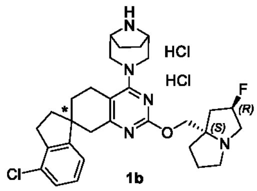
1b

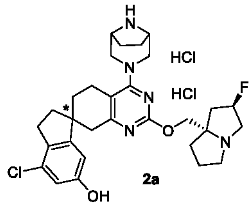
2a

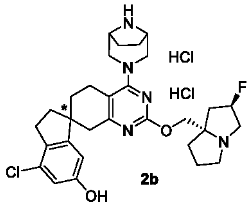
2b

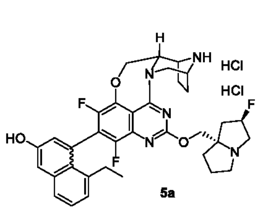
5a

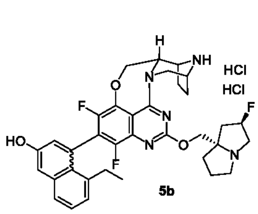
5b

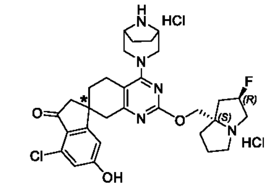
6a

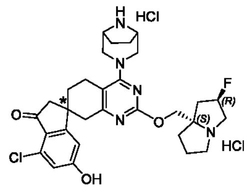
6b

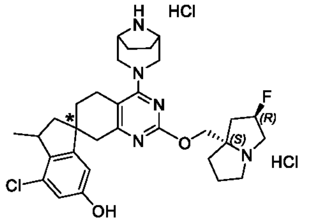
7a

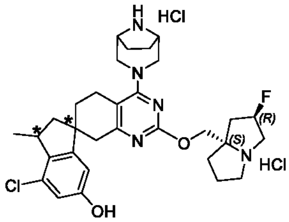
7aa

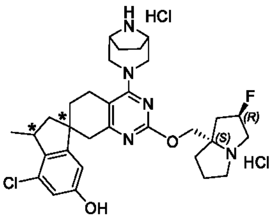
7ab

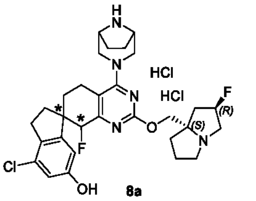
8a

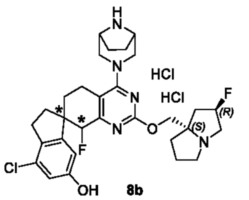
8b

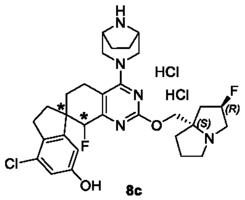
8c

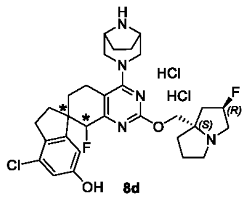
8d

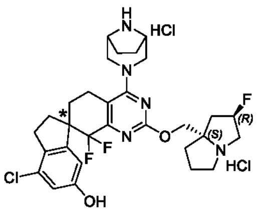
9a

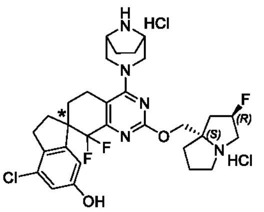
9b

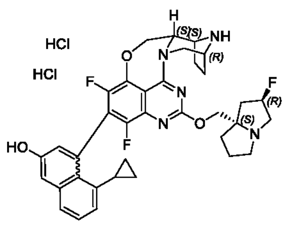
11a

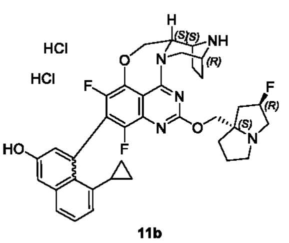
11b

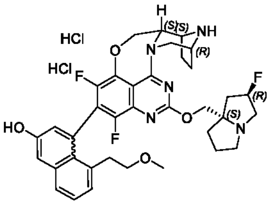
12a

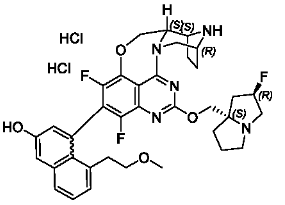
12b

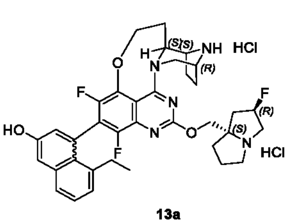
13a

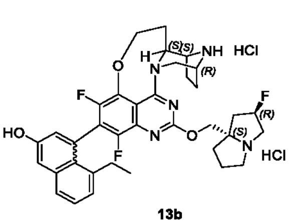
13b

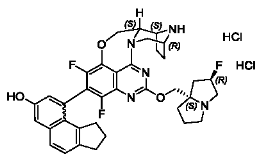
14a

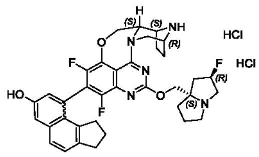
14b

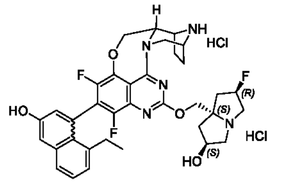
15a

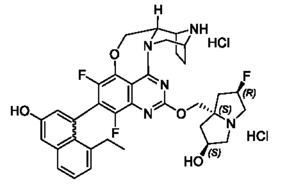
15b

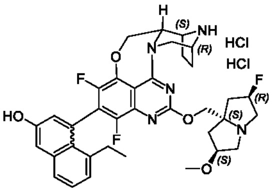
16a

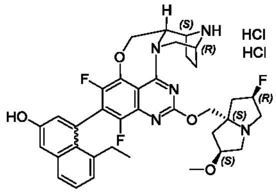
16b

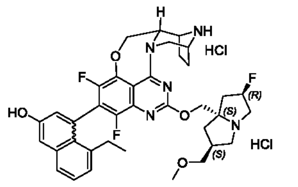
18a

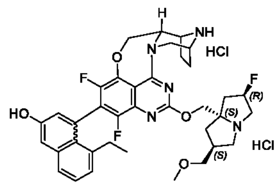
18b

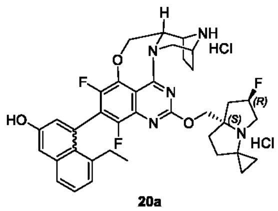
20a

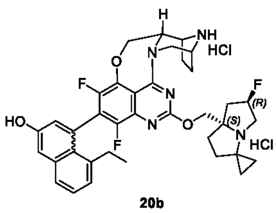
20b

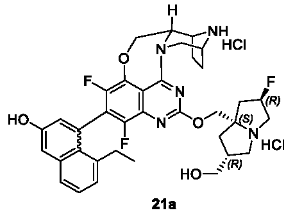
21a

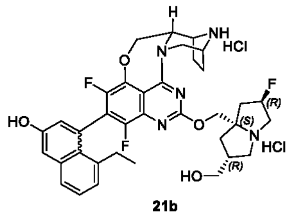
21b

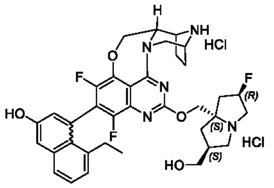
23a

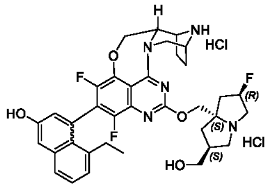
23b

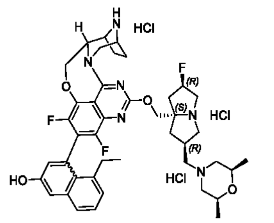
25a

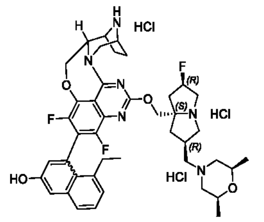
25b

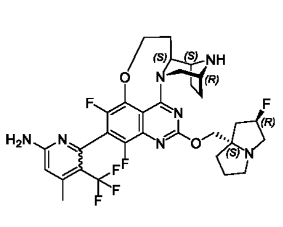
26a

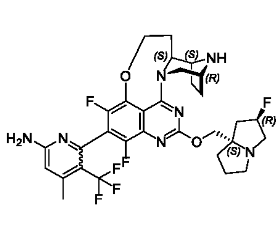
26b

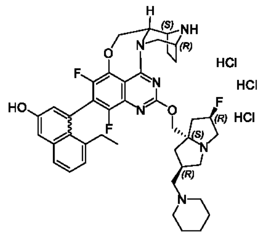
27a

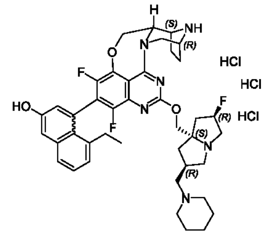
27b

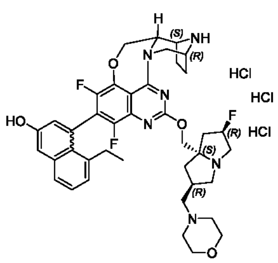
28a

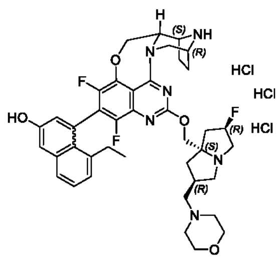
28b

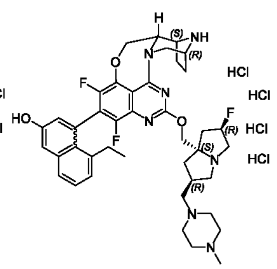
29a

or

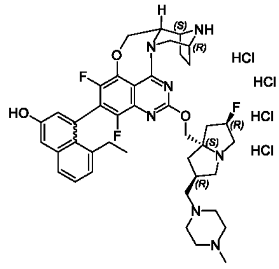
29b

;

wherein "*" denotes a carbon atom in S configuration or a carbon atom in R configuration, and " 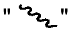 " denotes " 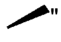 " or "  ".

**14.** Compounds shown below or pharmaceutically acceptable salts thereof

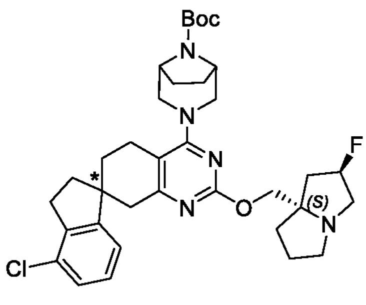

**1-10a**

,

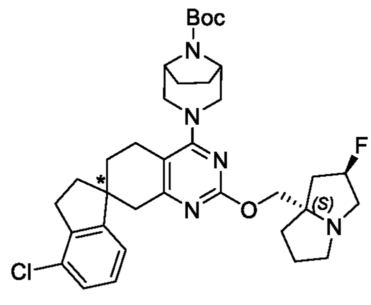

**1-10b**

,

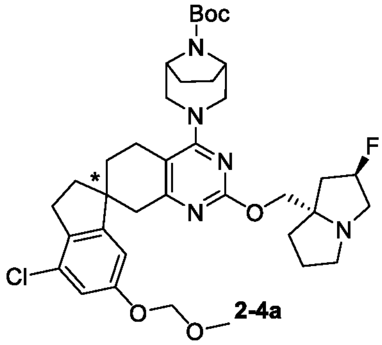

**2-4a**

,

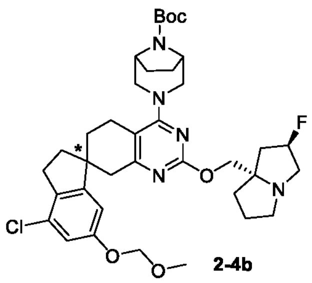

**2-4b**

,

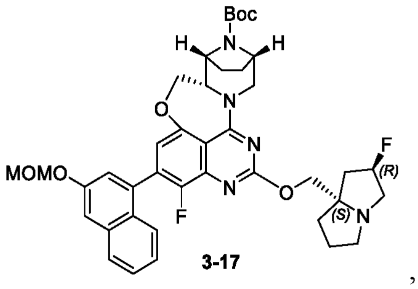

**3-17**

,

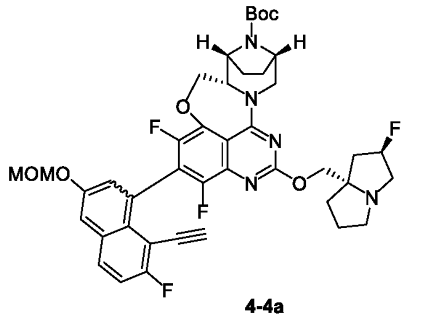

**4-4a**

,

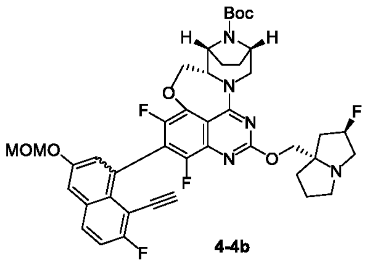

**4-4b**

,

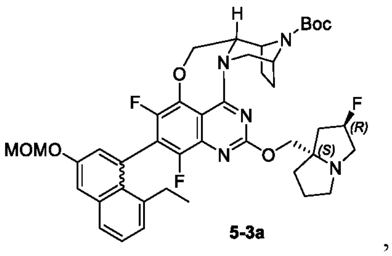

**5-3a**

,

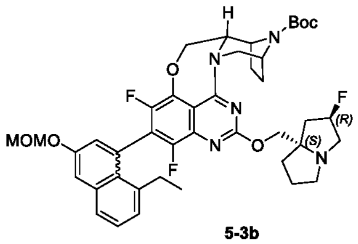

**5-3b**

,

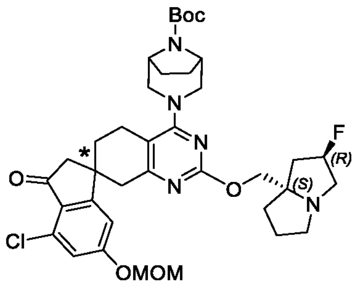

**6-2a**

,

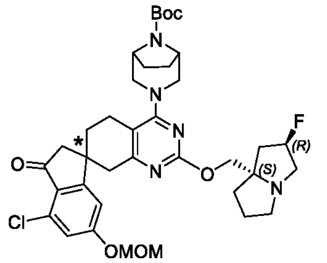

**6-2b**

,

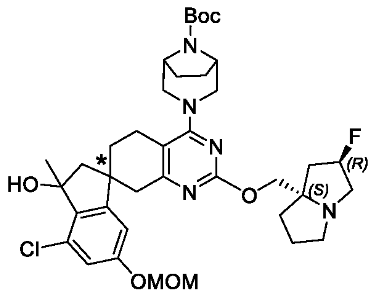

**7-1a**

,

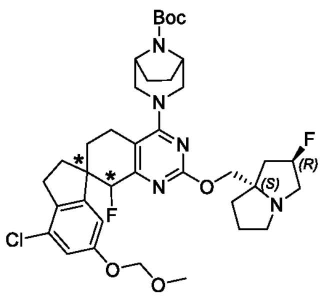

8-5a ,

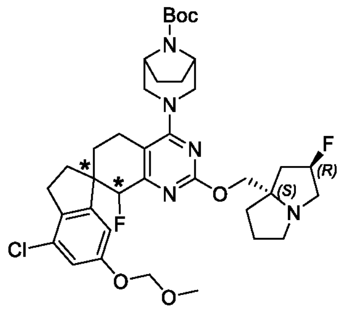

8-5b ,

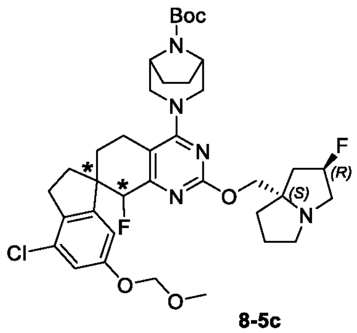

8-5c ,

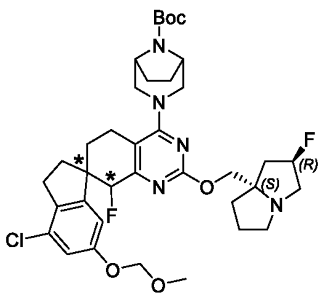

8-5d ,

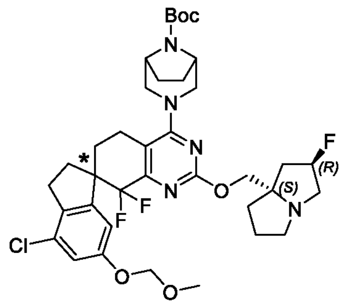

9-5a ,

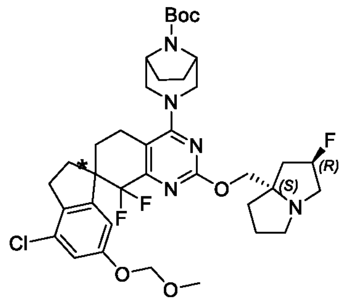

9-5b ,

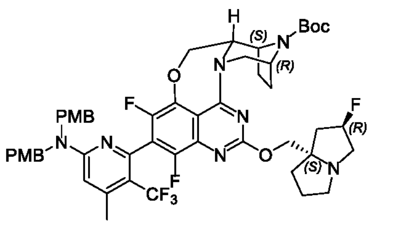

10-3 ,

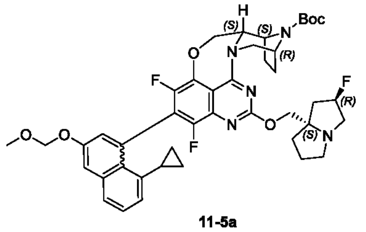

11-5a ,

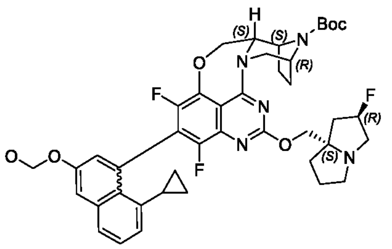

11-5b ,

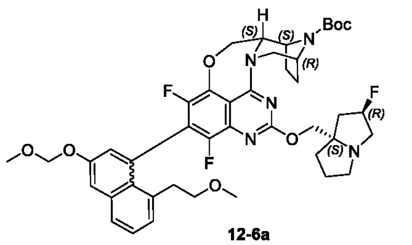

12-6a ,

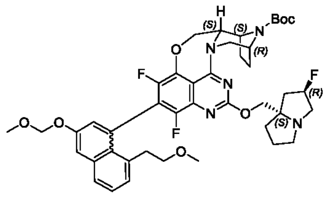

12-6b ,

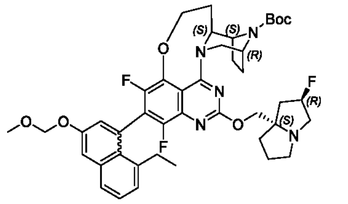

13-17a ,

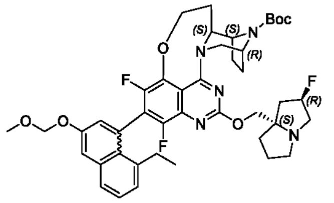

13-17b ,

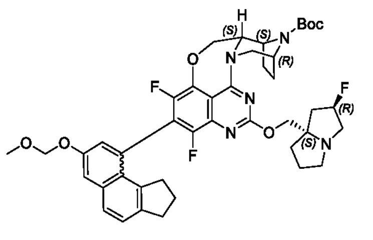

14-11a ,

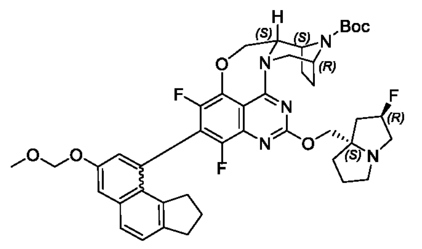

14-11b ,

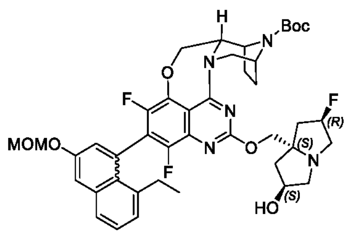
15-9a ,

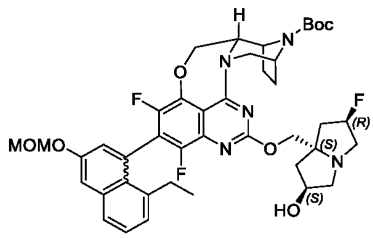
15-9b ,

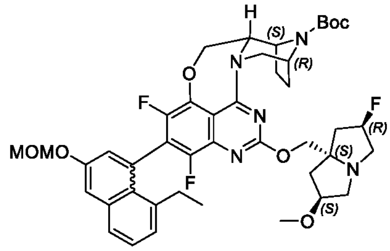
16-4a ,

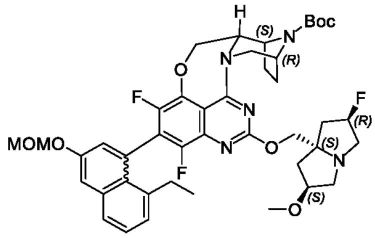
16-4b ,

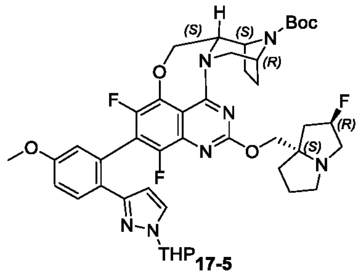
17-5 ,

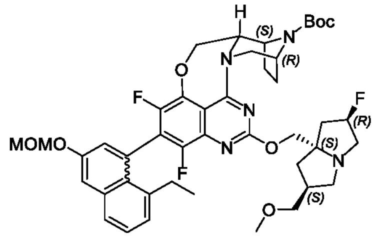
18-4a ,

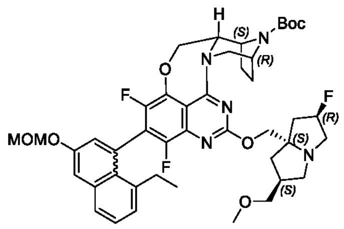
18-4b ,

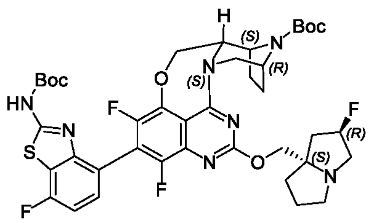
19-1

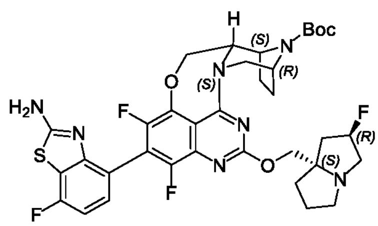
19-2 ,

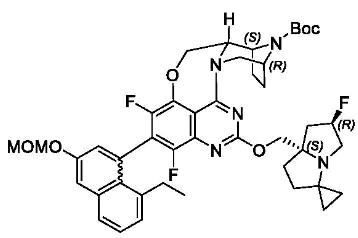
20-2a ,

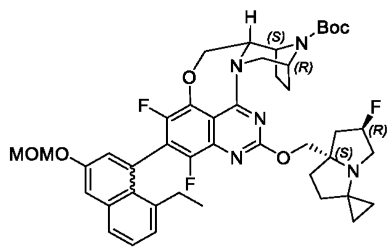
20-2b ,

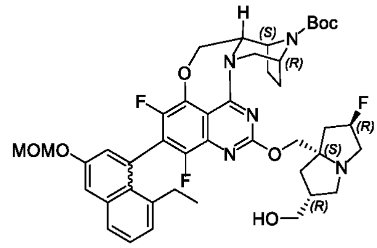
21-4a ,

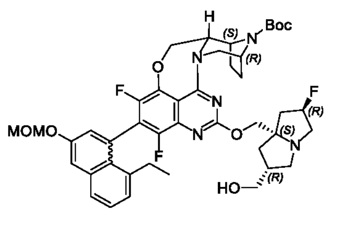
21-4b ,

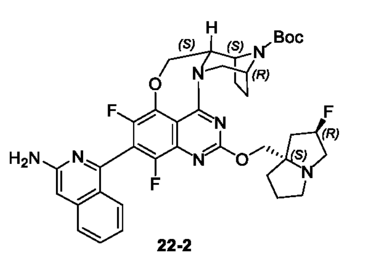
22-2 ,

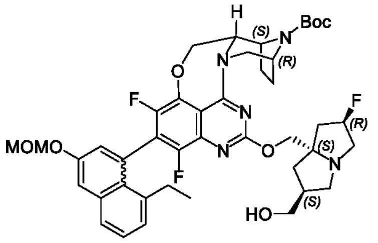
23-4a ,

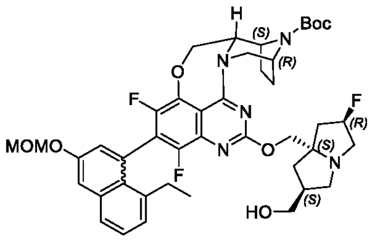

23-4b ,

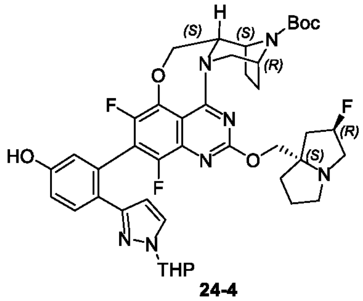

24-4 ,

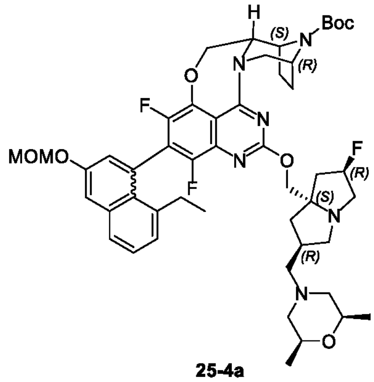

25-4a ,

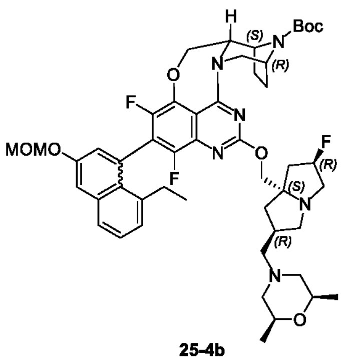

25-4b ,

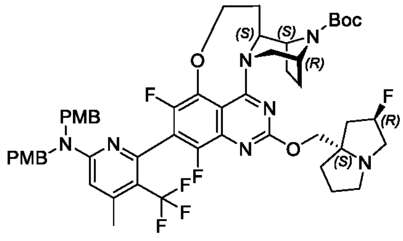

26-1 ,

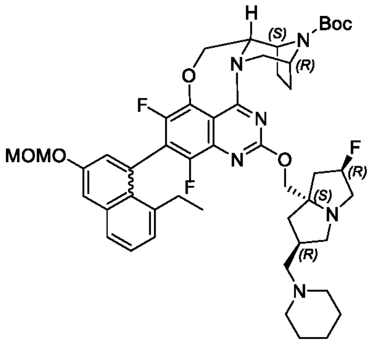

27-1a ,

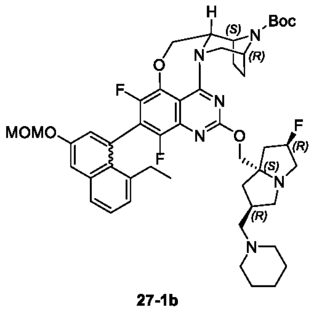

27-1b ,

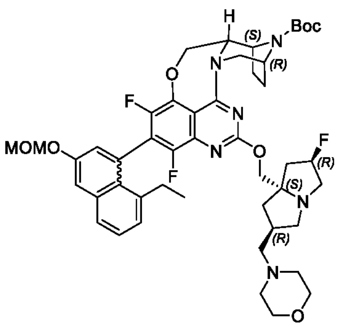

28-1a ,

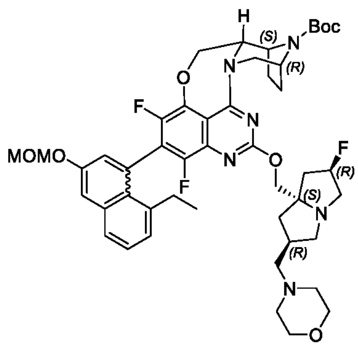

28-1b ,

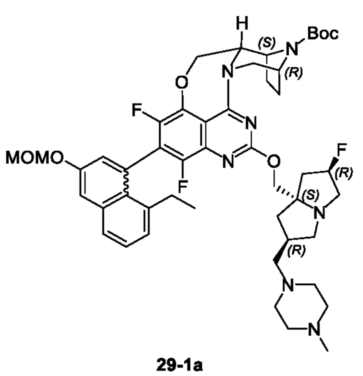

29-1a ,

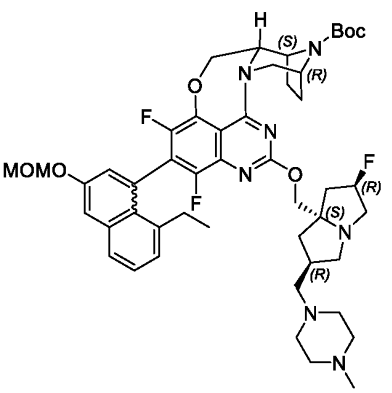

29-1b ,

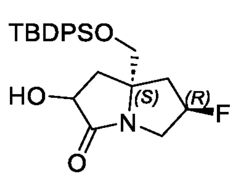

15-3 ,

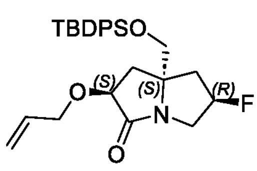

**15-4a** ,

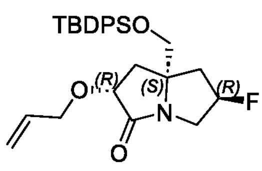

**15-4b** ,

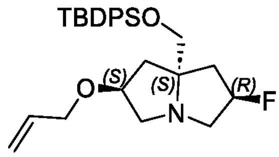

**15-5** ,

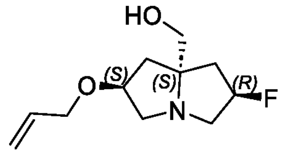

**15-6** ,

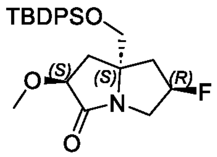

**16-1** ,

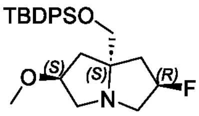

**16-2** ,

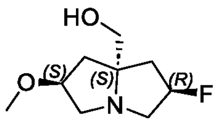

**16-3** ,

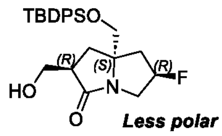

***Less polar***

**18-1a** ,

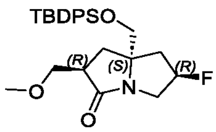

**18-2** ,

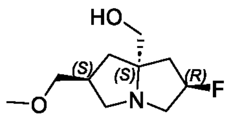

**18-3** ,

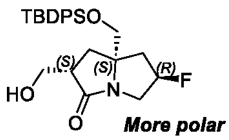

***More polar***

**18-1b** ,

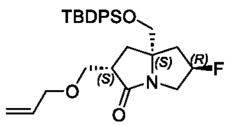

**21-1** ,

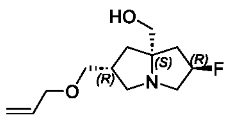

**21-2** ,

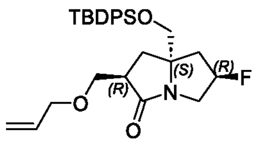

**23-1** ,

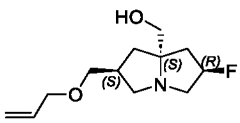

**23-2** ,

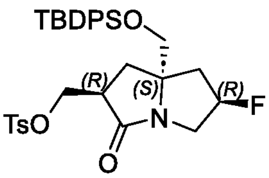

**25-1** ,

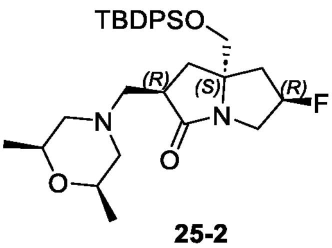

**25-2** ,

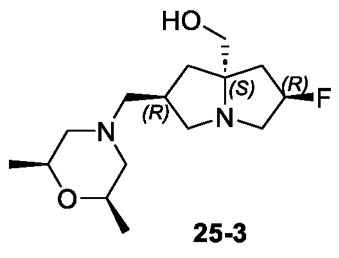

**25-3** ,

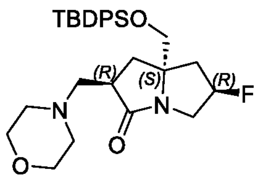

**28-2** ,

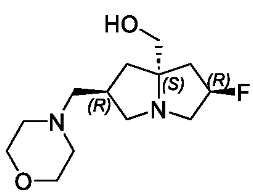

**28-3** ,

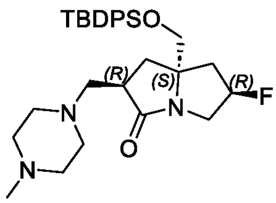

**29-2** ,

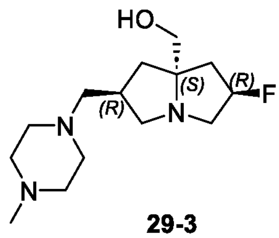

**29-3** ,

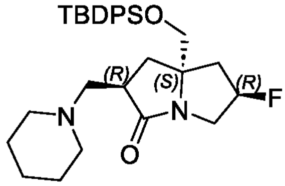

**27-2** ,

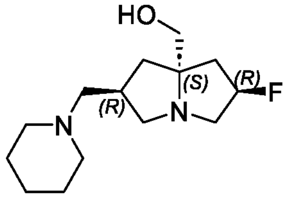

**27-3** ,

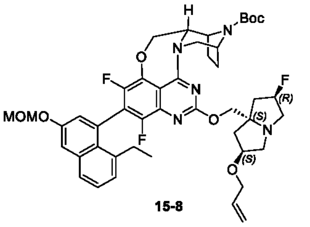

15-8 ,

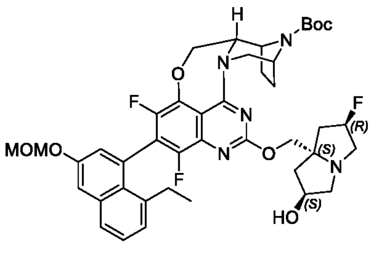

15-9 ,

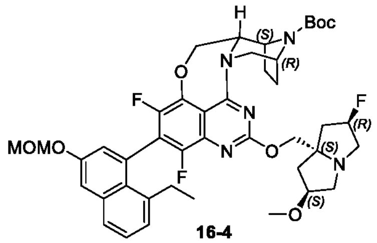

16-4 ,

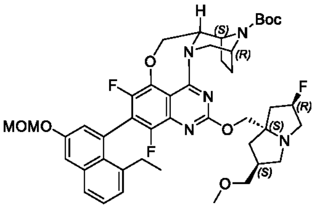

18-4 ,

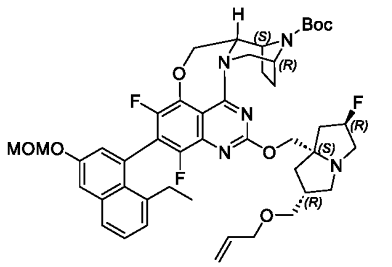

21-3 ,

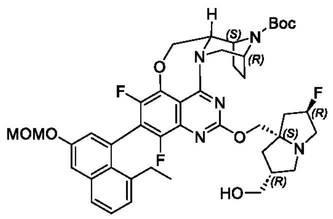

21-4 ,

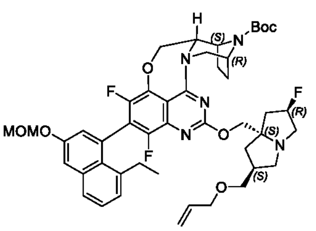

23-3 ,

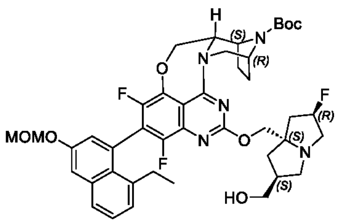

23-4 ,

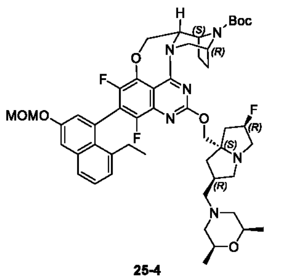

25-4 ,

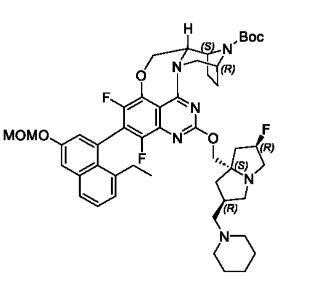

27-1 ,

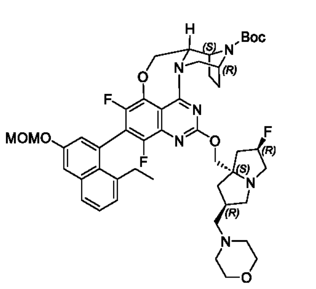

28-1 ,

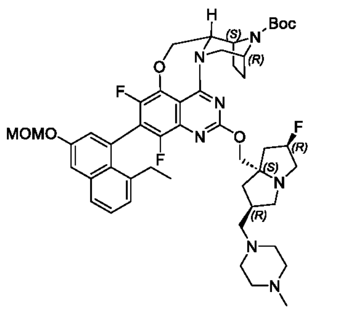

29-1 ,

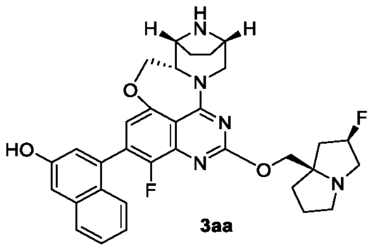

3aa

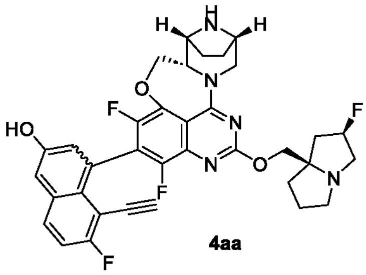

4aa

,

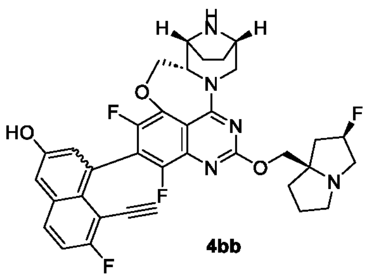

4bb

,

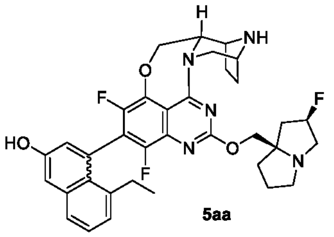

5aa

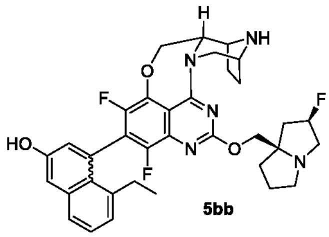

5bb

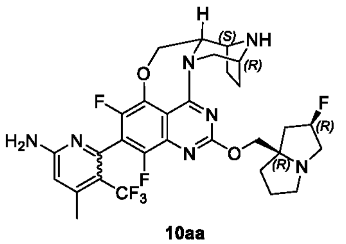

10aa

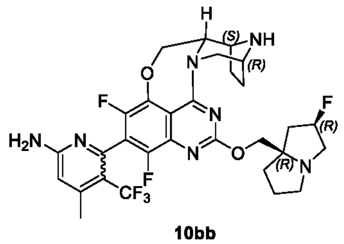

10bb

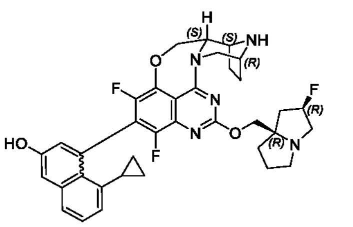

11aa

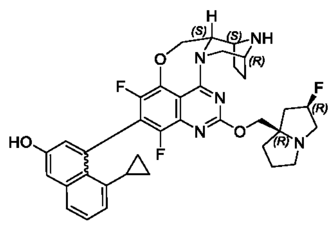

11bb

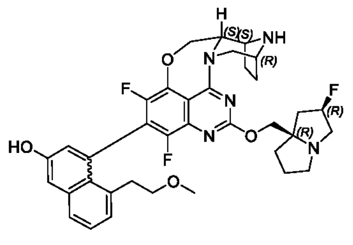

12aa

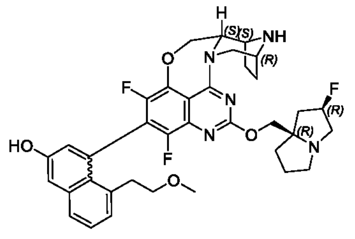

12bb

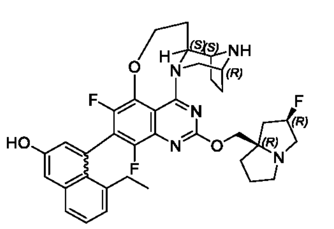

13aa

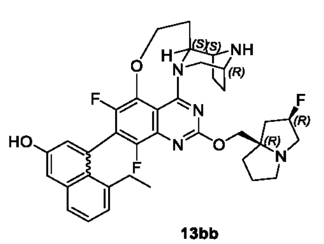

13bb

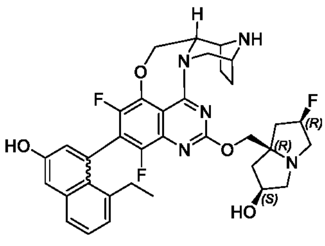

15aa

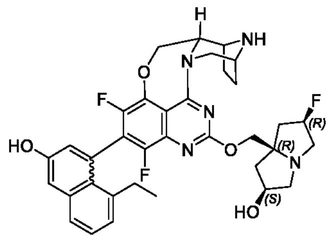

15bb

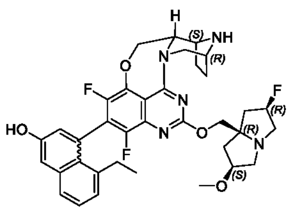

16aa

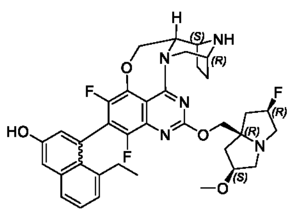

16bb

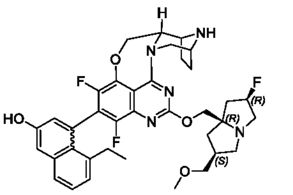

18aa

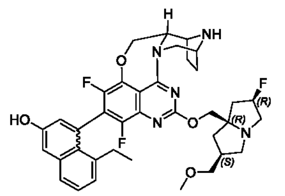

18bb

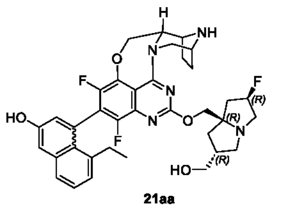

21aa

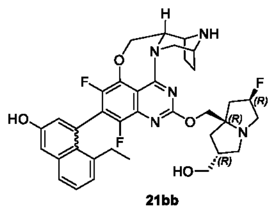

21bb

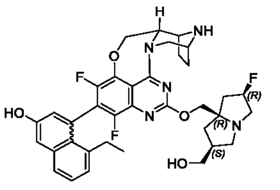

23aa

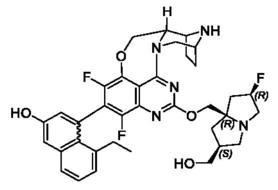

23bb

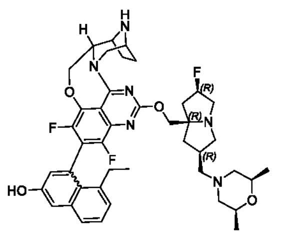

25aa

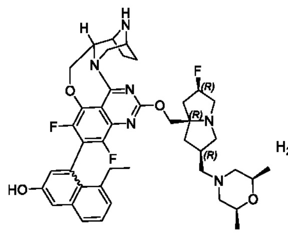

25bb

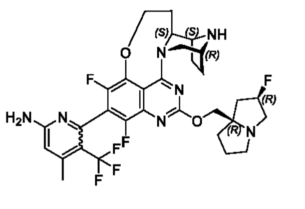

26aa

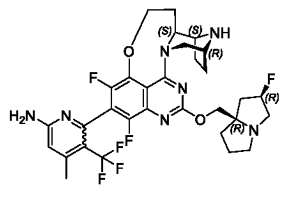

26bb

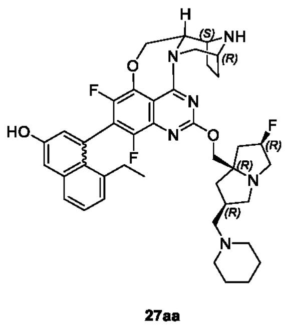

27aa

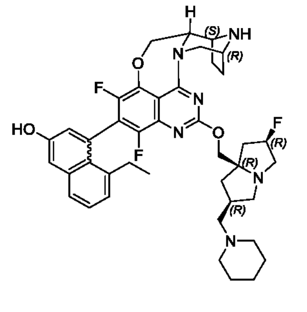

27bb

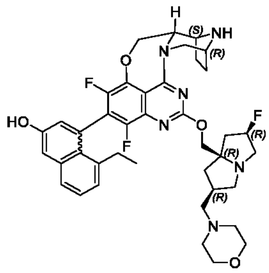

28aa

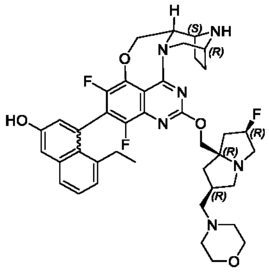

28bb

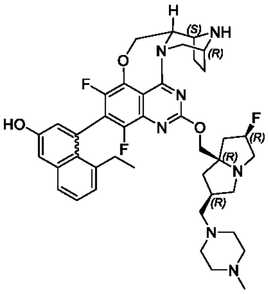

29aa

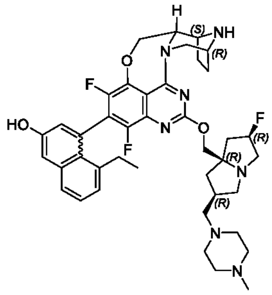

29bb

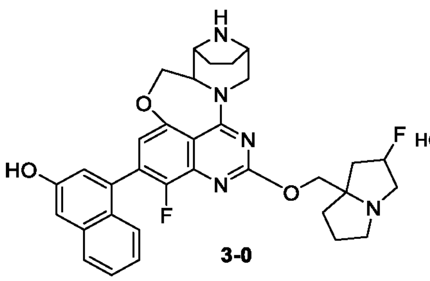

3-0

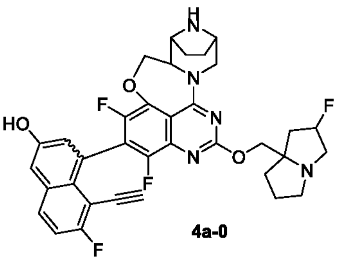

4a-0

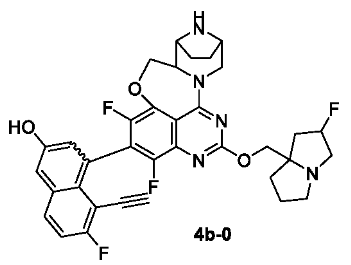

4b-0

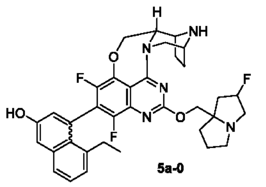

5a-0

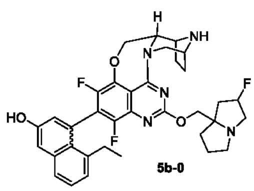

5b-0

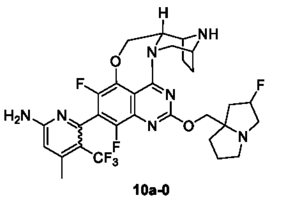

10a-0

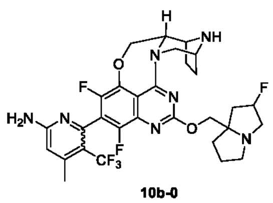

10b-0

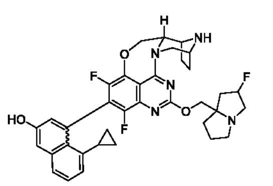

11a-0

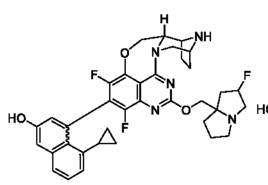

11b-0

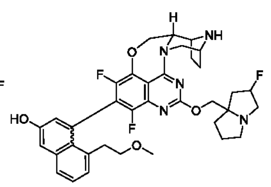

12a-0

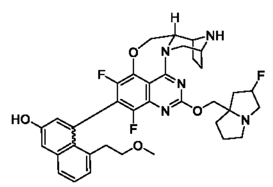

12b-0

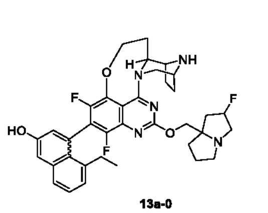

13a-0

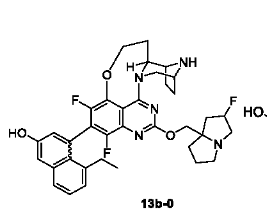

13b-0

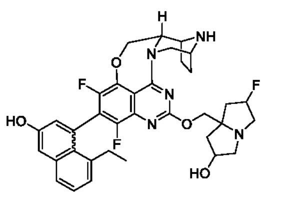

15a-0

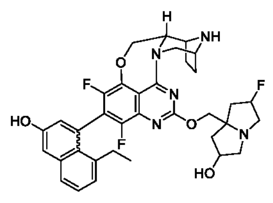

15b-0

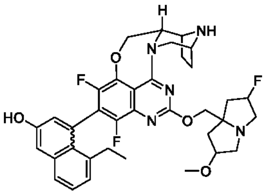

16a-0

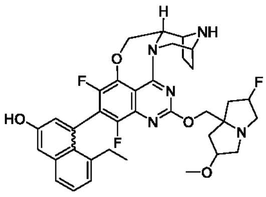

16b-0

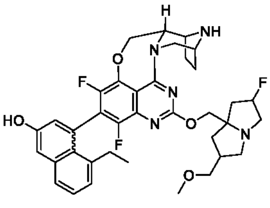

18a-0

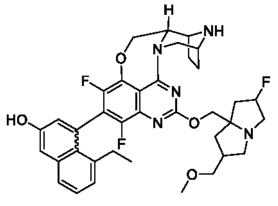

18b-0

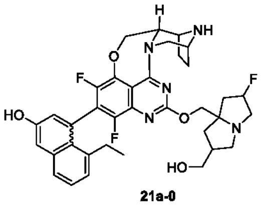

21a-0

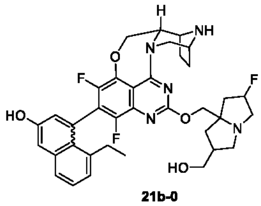

21b-0

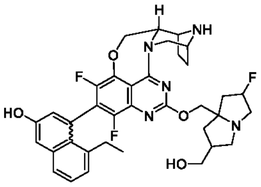

23a-0

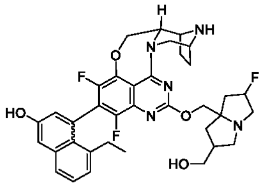

23b-0

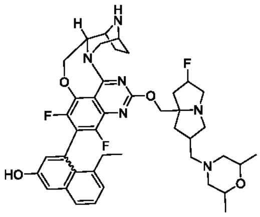

25a-0

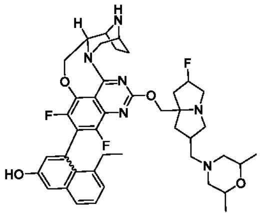

25b-0

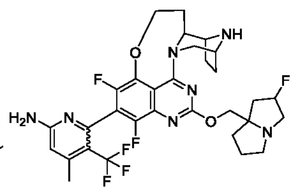

26a-0

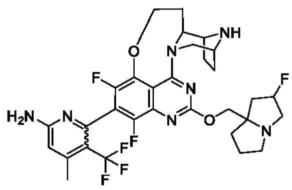

26b-0

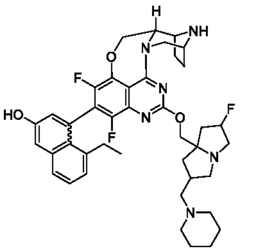

27a-0

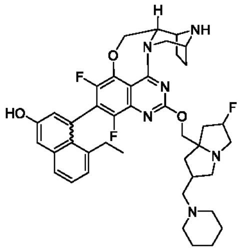

27b-0

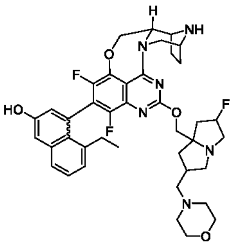

28a-0

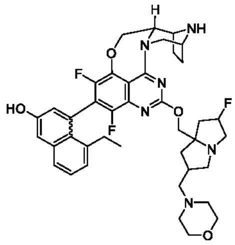

28b-0

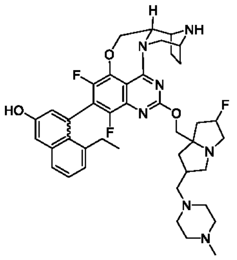

29a-0

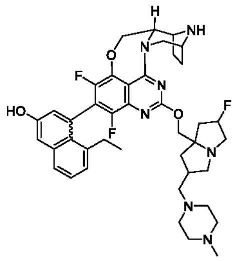

29b-0

wherein "*" denotes a carbon atom in S configuration or a carbon atom in R configuration, and " 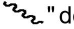 " denotes " 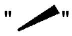 " or " 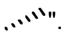 ".

**15.** A pharmaceutical composition comprising the compound of formula I, formula II, formula III, or formula IV, the pharmaceutically acceptable salt thereof, the solvate thereof, the stereoisomer thereof, the tautomer thereof, the prodrug thereof, the metabolite thereof, or the isotopic compound thereof according to any one of claims 1 to 13, and a pharmaceutical excipient.

**16.** A use of the compound of formula I, formula II, formula III, or formula IV, the pharmaceutically acceptable salt thereof, the solvate thereof, the stereoisomer thereof, the tautomer thereof, the prodrug thereof, the metabolite thereof, or the isotopic compound thereof according to any one of claims 1 to 13, or the pharmaceutical composition according to

claim 15 in the manufacture of an inhibitor of KRAS mutant protein or a medicament, and the medicament is used for the prevention or treatment of a cancer mediated by KRAS mutation;

the KRAS mutant protein is preferably KRAS G12D mutant protein;
the cancer mediated by KRAS mutant protein is selected from blood cancer, pancreatic cancer, MYH-associated polyposis, colorectal cancer, lung cancer, brain cancer, thyroid cancer, head and neck cancer, nasopharyngeal cancer, throat cancer, oral cancer, salivary gland cancer, esophageal cancer, gastric cancer, lung cancer, liver cancer, kidney cancer, pancreatic cancer, gallbladder cancer, cholangiocarcinoma, colorectal cancer, small intestine cancer, gastrointestinal stromal tumor, urothelial carcinoma, urethral cancer, bladder cancer, breast cancer, vaginal cancer, ovarian cancer, endometrial cancer, cervical cancer, fallopian tube cancer, testicular cancer, prostate cancer, hemangioma, leukemia, lymphoma, myeloma, skin cancer, lipoma, bone cancer, soft tissue sarcoma, neurofibroma, glioma, neuroblastoma, and glioblastoma; preferably selected from blood cancer, pancreatic cancer, MYH-associated polyposis, colorectal cancer, lung cancer, colorectal cancer, and non-small cell lung cancer; more preferably selected from blood cancer, pancreatic cancer, MYH-associated polyposis, colorectal cancer, or lung cancer.

**INTERNATIONAL SEARCH REPORT**

International application No. **PCT/CN2023/073901**

**A. CLASSIFICATION OF SUBJECT MATTER**

C07D519/00(2006.01)i;C07D498/22(2006.01)i;C07D403/14(2006.01)i;C07D413/14(2006.01)i;C07D491/107(2006.01)i; A61P35/00(2006.01)i;A61K31/553(2006.01)i

According to International Patent Classification (IPC) or to both national classification and IPC

**B. FIELDS SEARCHED**

Minimum documentation searched (classification system followed by classification symbols)

IPC：C07D A61P A61K

Documentation searched other than minimum documentation to the extent that such documents are included in the fields searched

Electronic data base consulted during the international search (name of data base and, where practicable, search terms used)

CNABS; CNTXT; VEN; WOTXT; EPTXT; USTXT; STN; CNKI; ISI web of science: 上海医药集团股份有限公司, 张朝欣, 毛海斌, 夏广新, 付玉红, 柯樱, 喹唑啉, 螺环, 三并杂环, 四环氮氧杂, KRAS, KRas, 癌症, 肿瘤, +quinazolin+, +spirocyclic+, tri+heterocyclic, tetracyclic oxazepinyl, cancer, tumor, tumour, structural formula (I), structural formula (I)I

**C. DOCUMENTS CONSIDERED TO BE RELEVANT**

| Category* | Citation of document, with indication, where appropriate, of the relevant passages | Relevant to claim No. |
|---|---|---|
| PX | WO 2023001141 A1 (SHANGHAI ZION PHARMA CO., LTD.) 26 January 2023 (2023-01-26)<br>claims 86-97 | 1-5, 7-16 |
| PX | WO 2022173678 A1 (GENENTECH, INC.) 18 August 2022 (2022-08-18)<br>description, page 19, paragraph [0083] to page 99, paragraph [0253] | 1-5, 7-16 |
| X | CN 113087700 A (SUZHOU ASCENTAGE PHARMA CO., LTD.) 09 July 2021 (2021-07-09)<br>claims 1-67 and 123-141, and description, page 18, paragraph [0161] | 1-16 |
| PX | CN 115557974 A (SHANGHAI DE NOVO PHARMATECH CO., LTD.) 03 January 2023 (2023-01-03)<br>claims 1-22 | 1-5, 7-16 |
| PX | WO 2022268051 A1 (JIANGSU HENGRUI MEDICINE CO., LTD.) 29 December 2022 (2022-12-29)<br>claims 1-22 | 1-5, 7-16 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **19 April 2023** | **15 May 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2023/073901

C. DOCUMENTS CONSIDERED TO BE RELEVANT

| Category* | Citation of document, with indication, where appropriate, of the relevant passages | Relevant to claim No. |
|---|---|---|
| PX | WO 2022199587 A1 (MEDSHINE DISCOVERY INC.) 29 September 2022 (2022-09-29) claims 1-25 | 1-5, 7-16 |
| PX | WO 2022194245 A1 (GENFLEET THERAPEUTICS (SHANGHAI) INC.) 22 September 2022 (2022-09-22) claims 1-15 | 1-5, 7-16 |
| PX | WO 2022188729 A1 (JACOBIO PHARMACEUTICALS CO., LTD.) 15 September 2022 (2022-09-15) claims 1-34 | 1-5, 7-16 |
| PX | CN 114805311 A (SUZHOU ASCENTAGE PHARMA CO., LTD.) 29 July 2022 (2022-07-29) claims 1-145 | 1-16 |
| PX | WO 2022206723 A1 (ZHEJIANG HISUN PHARMACEUTICAL CO., LTD.) 06 October 2022 (2022-10-06) claims 1-24 | 1-5, 7-16 |
| A | CN 112745335 A (WUHAN YUXIANG PHARMACEUTICAL TECHNOLOGY CO., LTD.) 04 May 2021 (2021-05-04) claims 1-15, and description, page 6, paragraph [0044] | 1-16 |
| A | WO 2021093758 A1 (SICHUAN HAISCO PHARMACEUTICAL CO., LTD.) 20 May 2021 (2021-05-20) entire document | 1-16 |

Form PCT/ISA/210 (second sheet) (July 2022)

**INTERNATIONAL SEARCH REPORT**
**Information on patent family members**

International application No.
**PCT/CN2023/073901**

| Patent document cited in search report | Publication date (day/month/year) | Patent family member(s) | Publication date (day/month/year) |
|---|---|---|---|
| WO 2023001141 A1 | 26 January 2023 | None | |
| WO 2022173678 A1 | 18 August 2022 | US 2022281893 A1 | 08 September 2022 |
| | | TW 202241912 A | 01 November 2022 |
| CN 113087700 A | 09 July 2021 | WO 2021139748 A1 | 15 July 2021 |
| | | US 2021230174 A1 | 29 July 2021 |
| | | TW 202140458 A | 01 November 2021 |
| | | US 11312724 B2 | 26 April 2022 |
| | | TW 770760 B1 | 11 July 2022 |
| CN 115557974 A | 03 January 2023 | WO 2023274383 A1 | 05 January 2023 |
| WO 2022268051 A1 | 29 December 2022 | None | |
| WO 2022199587 A1 | 29 September 2022 | None | |
| WO 2022194245 A1 | 22 September 2022 | None | |
| WO 2022188729 A1 | 15 September 2022 | None | |
| CN 114805311 A | 29 July 2022 | WO 2022156761 A1 | 28 July 2022 |
| WO 2022206723 A1 | 06 October 2022 | None | |
| CN 112745335 A | 04 May 2021 | None | |
| WO 2021093758 A1 | 20 May 2021 | TW 202128672 A | 01 August 2021 |
| | | TW 760919 B1 | 11 April 2022 |
| | | CN 114630832 A | 14 June 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2022101147120
- CN 2022106541481
- CN 2022108381203
- CN 2022111251811
- CN 2022112045597
- WO 2019179515 A1